# EUROPEAN PATENT SPECIFICATION

(11) **EP 4 100 410 B1**
(45) Date of publication and mention of the grant of the patent: **22.04.2026**
(21) Application number: 21709200.6
(22) Date of filing: 03.02.2021
(51) Int. Cl.: C07D 487/10, A61K 31/496, A61K 31/497, A61K 31/498, A61K 31/4985, A61K 31/501, A61K 31/506, A61K 31/513, A61K 31/517, A61K 31/5386, A61P 25/14, A61P 25/16, A61P 25/18, A61P 25/30, C07D 519/00

(54) **MUSCARINIC RECEPTOR 4 ANTAGONISTS AND METHODS OF USE**
MUSCARINISCHE REZEPTOR-4-ANTAGONISTEN UND VERFAHREN ZUR VERWENDUNG
ANTAGONISTES DU RÉCEPTEUR MUSCARINIQUE 4 ET PROCÉDÉS D'UTILISATION

(30) Priority: 05.02.2020 US 202062970465 P
(43) Date of publication of application: 14.12.2022
(73) Proprietor: Neurocrine Biosciences, Inc., San Diego, CA 92130 (US)
(72) Inventor: HARRIOTT, Nicole, San Diego, California 92130 (US); PAGANO, Nicholas, San Diego, California 92130 (US); LEY, Corinne Rose, San Diego, California 92130 (US)
(74) Representative: Mewburn Ellis LLP
(86) International application number: PCT/US2021/016465
(87) International publication number: WO 2021/158698

(56) References cited:
- EP-A1- 3 371 164
- WO-A1-2019/183636

## Description

### FIELD OF THE INVENTION

The present invention relates to certain compounds described herein, such as compounds of Formula **(la)** and pharmaceutical compositions thereof that modulate the activity of the muscarinic acetylcholine receptor M₄. Compounds of the present invention and pharmaceutical compositions thereof are directed to methods useful in the treatment or prophylaxis of a neurological disease, disorder, or symptom, such as, Tourette's syndrome (TS), Alzheimer's Disease (AD), schizophrenia, Lewy Body Dementia (LBD), cognitive deficits associated with schizophrenia, Parkinson's Disease, parkinsonism, tremor, dyskinesias, excessive daytime sleepiness, dystonia, chorea, levodopa induced dyskinesia, attention deficit hyperactivity disorder (ADHD), cerebral palsy, progressive supranuclear palsy (PSP), Multiple System Atrophy (MSA), Huntington's disease (HD), and chorea associate with Huntington's disease and conditions related thereto.

### BACKGROUND OF THE INVENTION

Muscarinic acetylcholine receptors are autonomic receptors that form G protein-receptor complexes in the cell membranes of certain neurons and other cell types (*e.g.*, endothelial cells of blood vessels). Muscarinic receptors are located postsynaptically at the parasympathetic neuroeffector junction, from where the receptors function to increase or decrease the activity of the effector cells. Extrapyramidal symptoms are observed in patients treated with antipsychotic therapeutics and in patients who have neuroleptic malignant syndrome, brain damage (*e.g.*, athetotic cerebral palsy), encephalitis, and meningitis. Drugs other than antipsychotics also cause extrapyramidal symptoms, for example antidopaminergic drugs (*e.g.*, the antiemetic metoclopramide and the antidepressant amoxapine) and selective serotonin reuptake inhibitors (SSR*), which indirectly decrease dopamine. Conditions associated with extrapyramidal symptoms include acute dystonic reactions, akathisia, pseudoparkinsonism, and tardive dyskinesia. Extrapyramidal symptoms caused by antipsychotic therapeutics are being treated with anticholinergic drugs that lack selectivity for any of the five muscarinic receptor subtypes (see, *e.g.,* Erosa-Rivero et al., Neuropharmacology 81:176-87 (2014)). Classical muscarinic receptor antagonists (*e.g.* atropine and scopolamine) and 3-quinuclidinyl benzilate (QNB) lack selectivity for human muscarinic acetylcholine receptors subtypes (*i.e.* M₁, M₂, M₃, M₄ and M₅) (see, *e.g.,* Bolden et al., J Pharmacol Exp Ther. 260(2):576-580 (1992)). Because anticholinergic drugs that effect multiple muscarinic receptors may cause distinct and in certain instances opposing effects, therapeutics that exhibit selectivity for particular receptors are desired. For example, M₄ antagonists inhibit striatal acetylcholine release and M₂ antagonists increase striatal acetylcholine release (see, *e.g.,* Quik et al., Nicotine & Tobacco Research 21(3):357-369 (2019)). In addition, the muscarinic receptor pan antagonist trihexyphenidyl (M₁ (Kᵢ = 1 nM), M₂ (Kᵢ = 20 nM), M₃ (Kᵢ = 10 nM), M₄ (Kᵢ = 10 nM) and M₅ (Kᵢ = 30 nM)) is thought to have use-limiting side effects, such as, cognitive impairment, tachycardia, and gastrointestinal tract function associated with antagonism of M₁, M₂, and M₃.

Despite the advances that have been made in this field, a need remains in the art for improved M₄ antagonists, including compounds, compositions, and methods related thereto. The present disclosure fulfills these and other needs, as evident in reference to the following disclosure.

### SUMMARY OF THE INVENTION

Any references in the description to methods of treatment refer to the compounds, pharmaceutical compositions and medicaments described herein in said methods of treatment.

One aspect of the present invention encompasses, *inter alia,* certain 2-azaspiro[3.3]heptane derivatives of Formula (la): 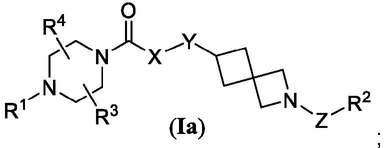 or a pharmaceutically acceptable salt thereof:
wherein:
X is O or NH;
Y is CH₂ or absent;
Z is C₁-C₄ alkylene or absent;
R¹ is C₆-C₁₀ aryl or 5-10 membered heteroaryl, each optionally substituted with one or more groups selected from: C₁-C₆ alkoxy, C₁-C₆ alkoxycarbonyl, C₁-C₆ alkyl, C₁-C₆ alkylamino, C₁-C₆ alkylcarbonyl, C₁-C₆ alkylcarbamoyl, C₁-C₆ alkylsulfanyl, C₂-C₆ dialkylamino, C₂-C₆ dialkyl carbamoyl, C₁-C₆ sulfinyl, C₃-C₁₀ cycloalkyl, C₁-C₆ haloalkoxy, C₁-C₆ haloalkyl, amino, carbamoyl, cyano, halogen, and nitro;
R² is selected from: C₆-C₁₀ aryl, C₁-C₆ alkyl, C₃-C₇ cycloalkyl, C₅-C₈ bicycloalkanyl, C₆-C₈ bicycloalkenyl, 5-10 membered heteroaryl, and heterocyclyl; each optionally substituted with one or more groups selected from: C₁-C₄ alkyl, C₁-C₆ alkoxy, carbamoyl, cyano, C₁-C₆ haloalkoxy, C₁-C₆ sulfonyl, and halogen; and
   a) R³ and R⁴ are each independently selected from H and C₁-C₄ alkyl; or
   b) R³ and R⁴ taken together form a bridging group selected from: CH₂, CH₂-CH₂, and CH₂-O-CH₂; and
   c) R³ and R⁴ are bonded to different ethylene groups of the piperazine ring;
or a compound selected from the group consisting of:
   (2R,6S)-N-[2-(cyclohex-1-en-1-ylmethyl)-2-azaspiro[3.3]heptan-6-yl]-2,6-dimethyl-4-[5-(trifluoromethyl)pyrimidin-2-yl]piperazine-1-carboxamide;
   (2R,6S)-2,6-dimethyl-N-{2-[(4-nitrophenyl)methyl]-2-azaspiro[3.3]heptan-6-yl}-4-[5-(trifluoromethyl)pyrimidin-2-yl]piperazine-1-carboxamide;
   (2R,6S)-N-{2-[(4-hydroxyphenyl)methyl]-2-azaspiro[3.3]heptan-6-yl}-2,6-dimethyl-4-[5-(trifluoromethyl)pyrimidin-2-yl]piperazine-1-carboxamide;
   methyl 4-[(6-{[(2R,6S)-2,6-dimethyl-4-[5-(trifluoromethyl)pyrimidin-2-yl]piperazine-1-carbonyl]amino}-2-azaspiro[3.3]heptan-2-yl)methyl]benzoate;
   (2R,6S)-N-{2-[(4-acetamidophenyl)methyl]-2-azaspiro[3.3]heptan-6-yl}-2,6-dimethyl-4-[5-(trifluoromethyl)pyrimidin-2-yl]piperazine-1-carboxamide;
   (2R,6S)-2,6-dimethyl-N-(2-{[4-(trifluoromethyl)phenyl]methyl}-2-azaspiro[3.3]heptan-6-yl)-4-[5-(trifluoromethyl)pyrimidin-2-yl]piperazine-1-carboxamide;
   (2R,6S)-N-{2-[(4-carbamimidoylphenyl)methyl]-2-azaspiro[3.3]heptan-6-yl}-2,6-dimethyl-4-[5-(trifluoromethyl)pyrimidin-2-yl]piperazine-1-carboxamide;
   (2R,6S)-N-[2-(adamantan-1-ylmethyl)-2-azaspiro[3.3]heptan-6-yl]-2,6-dimethyl-4-[5-(trifluoromethyl)pyrimidin-2-yl]piperazine-1-carboxamide;
   (2R,6S)-N-(2-{[4-(dimethylcarbamoyl)phenyl]methyl}-2-azaspiro[3.3]heptan-6-yl)-2,6-dimethyl-4-[5-(trifluoromethyl)pyrimidin-2-yl]piperazine-1-carboxamide;
   2-benzyl-6-(difluoromethyl)-2-azaspiro[3.3]heptan-6-yl (2R,6S)-2,6-dimethyl-4-[5-(trifluoromethyl)pyrimidin-2-yl]piperazine-1-carboxylate;
   2-benzyl-6-(difluoromethyl)-2-azaspiro[3.3]heptan-6-yl (2R,6S)-2,6-dimethyl-4-[5-(trifluoromethyl)pyrazin-2-yl]piperazine-1-carboxylate;
   2-benzyl-6-(difluoromethyl)-2-azaspiro[3.3]heptan-6-yl (2R,6S)-2,6-dimethyl-4-[6-(trifluoromethyl)pyridazin-3-yl]piperazine-1-carboxylate;
   2-benzyl-6-(difluoromethyl)-2-azaspiro[3.3]heptan-6-yl (2R,6R)-2,6-dimethyl-4-[5-(trifluoromethyl)pyrimidin-2-yl]piperazine-1-carboxylate;
   2-benzyl-6-(difluoromethyl)-2-azaspiro[3.3]heptan-6-yl (2R,6R)-2,6-dimethyl-4-[5-(trifluoromethyl)pyrazin-2-yl]piperazine-1-carboxylate;
   2-benzyl-6-(difluoromethyl)-2-azaspiro[3.3]heptan-6-yl (2R,5S)-2,5-dimethyl-4-[5-(trifluoromethyl)pyrimidin-2-yl]piperazine-1-carboxylate;
   2-benzyl-6-(difluoromethyl)-2-azaspiro[3.3]heptan-6-yl (2R,5S)-2,5-dimethyl-4-[5-(trifluoromethyl)pyrazin-2-yl]piperazine-1-carboxylate;
   2-benzyl-6-(difluoromethyl)-2-azaspiro[3.3]heptan-6-yl (2R)-4-(5-cyanopyrimidin-2-yl)-2-methylpiperazine-1-carboxylate;
   2-benzyl-6-(difluoromethyl)-2-azaspiro[3.3]heptan-6-yl (2R)-2-methyl-4-[5-(trifluoromethyl)pyrimidin-2-yl]piperazine-1-carboxylate;
   2-benzyl-6-(difluoromethyl)-2-azaspiro[3.3]heptan-6-yl (2R)-2-methyl-4-[5-(trifluoromethyl)pyrazin-2-yl]piperazine-1-carboxylate;
   2-benzyl-6-methyl-2-azaspiro[3.3]heptan-6-yl (2R,6S)-4-(5-cyanopyrimidin-2-yl)-2,6-dimethylpiperazine-1-carboxylate;
   2-benzyl-6-methyl-2-azaspiro[3.3]heptan-6-yl (2R,6S)-2,6-dimethyl-4-[5-(trifluoromethyl)pyrimidin-2-yl]piperazine-1-carboxylate;
   2-benzyl-6-methyl-2-azaspiro[3.3]heptan-6-yl (2R,6S)-2,6-dimethyl-4-[5-(trifluoromethyl)pyrazin-2-yl]piperazine-1-carboxylate;
   2-benzyl-6-methyl-2-azaspiro[3.3]heptan-6-yl (2R,6R)-4-(5-cyanopyrimidin-2-yl)-2,6-dimethylpiperazine-1-carboxylate;
   2-benzyl-6-methyl-2-azaspiro[3.3]heptan-6-yl (2R,6R)-2,6-dimethyl-4-[5-(trifluoromethyl)pyrimidin-2-yl]piperazine-1-carboxylate;
   2-benzyl-6-methyl-2-azaspiro[3.3]heptan-6-yl (2R,6R)-2,6-dimethyl-4-[5-(trifluoromethyl)pyrazin-2-yl]piperazine-1-carboxylate;
   2-benzyl-6-methyl-2-azaspiro[3.3]heptan-6-yl (2R,5S)-4-(5-cyanopyrimidin-2-yl)-2,5-dimethylpiperazine-1-carboxylate;
   2-benzyl-6-methyl-2-azaspiro[3.3]heptan-6-yl (2R,5S)-2,5-dimethyl-4-[5-(trifluoromethyl)pyrimidin-2-yl]piperazine-1-carboxylate;
   2-benzyl-6-methyl-2-azaspiro[3.3]heptan-6-yl (2R,5S)-2,5-dimethyl-4-[5-(trifluoromethyl)pyrazin-2-yl]piperazine-1-carboxylate;
   2-benzyl-2-azaspiro[3.3]heptan-6-yl (2R,6S)-4-(5-ethynylpyrimidin-2-yl)-2,6-dimethylpiperazine-1-carboxylate;
   2-benzyl-2-azaspiro[3.3]heptan-6-yl (2R,6S)-4-[5-(dimethylphosphoryl)pyridin-2-yl]-2,6-dimethylpiperazine-1-carboxylate; and
   2-benzyl-2-azaspiro[3.3]heptan-6-yl (2R,6S)-4-[5-(dimethylphosphoryl) pyrimidin-2-yl]-2,6-dimethylpiperazine-1-carboxylate;
   or a pharmaceutically acceptable salt thereof.

One aspect of the present invention relates to pharmaceutical products selected from: a pharmaceutical composition, a formulation, a unit dosage form, and a kit; each comprising a compound of the present invention or a pharmaceutically acceptable salt thereof.

One aspect of the present invention relates to pharmaceutical compositions comprising a compound of the present invention or a pharmaceutically acceptable salt thereof, and a pharmaceutically acceptable carrier.

One aspect of the present invention relates to methods for preparing a pharmaceutical composition comprising the step of admixing a compound according of the present invention or a pharmaceutically acceptable salt thereof, and a pharmaceutically acceptable carrier.

One aspect of the present invention relates to methods for antagonizing a muscarinic receptor 4 (M₄) of a cell comprising contacting the cell with the compound according of the present invention or a pharmaceutically acceptable salt thereof.

One aspect of the present invention relates to methods for treating or preventing a neurological disease, disorder, or symptom in an individual, comprising administering to the individual in need thereof, a therapeutically effective amount of a compound according of the present invention or a pharmaceutically acceptable salt thereof; a pharmaceutical product of the present invention; or a pharmaceutical composition of the present invention.

One aspect of the present invention relates to methods for treating or preventing a muscarinic receptor 4 (M₄) mediated disease, disorder, or symptom in an individual, comprising administering to said individual in need thereof, a therapeutically effective amount of a compound according of the present invention or a pharmaceutically acceptable salt thereof; a pharmaceutical product of the present invention; or a pharmaceutical composition of the present invention.

One aspect of the present invention relates to uses of a compound of the present invention or a pharmaceutically acceptable salt thereof in the manufacture of a medicament for treating or preventing a neurological disease, disorder, or symptom in an individual.

One aspect of the present invention relates to uses of a compound of the present invention or a pharmaceutically acceptable salt thereof in the manufacture of a medicament for treating or preventing a muscarinic receptor 4 (M₄) mediated disease, disorder, or symptom in an individual.

One aspect of the present invention relates to compounds of the present invention or a pharmaceutically acceptable salt thereof; pharmaceutical products of the present invention; or pharmaceutical compositions of the present invention; for use in a method of treatment or prophylaxis of the human or animal body by therapy.

One aspect of the present invention relates to compounds of the present invention or a pharmaceutically acceptable salt thereof; pharmaceutical products of the present invention; or pharmaceutical compositions of the present invention; for use in a method for treating or preventing a neurological disease, disorder, or symptom in an individual.

One aspect of the present invention relates to compounds of the present invention or a pharmaceutically acceptable salt thereof; pharmaceutical products of the present invention; or pharmaceutical compositions of the present invention; for use in a method for treating or preventing a muscarinic receptor 4 (M₄) mediated disease, disorder, or symptom in an individual.

These and other aspects of the invention disclosed herein will be set forth in greater detail as the patent disclosure proceeds.

### BRIEF DESCRIPTION OF THE FIGURES

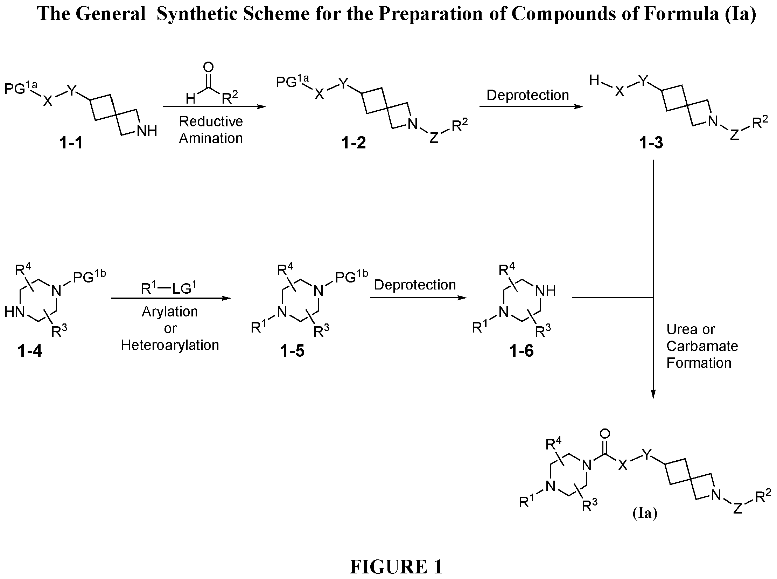

**Fig. 1** shows a general synthetic scheme for the preparation of carbamate and urea compounds of Formula **(la),** wherein PG^{1a} (*i.e.,* Protecting Group 1a) and PG^{1b} (*i.e.,* Protecting Group 1b) can independently be a variety of protecting groups known to one skilled in the art, such as, a *tert*-butoxycarbonyl (*i.e.,* BOC) group; LG¹ (*i.e.,* Leaving Group 1) can be, for example, Cl and Br; and X, Y, Z, R¹, R², R³, and R⁴ each have the same definitions as described herein, *supra* and *infra.* In this scheme, the R¹ group and Z-R² group are introduced prior to the formation of the carbamate or urea functionality.

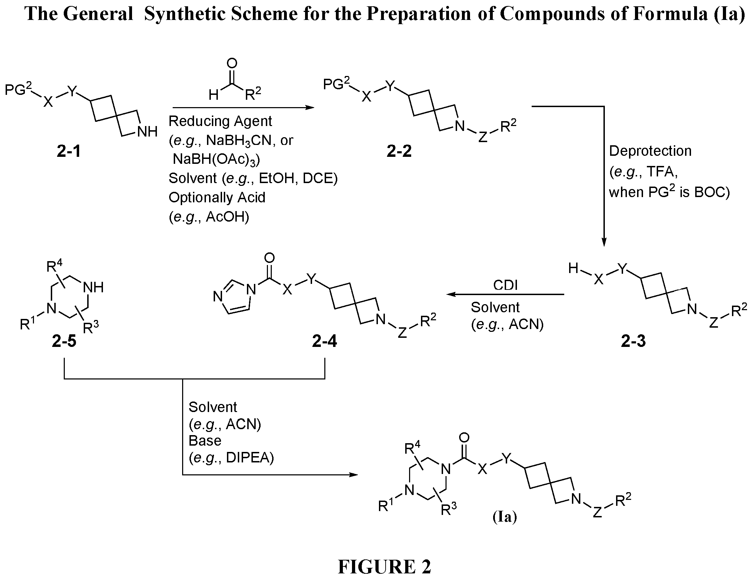

**Fig. 2** shows a general synthetic scheme for the preparation of compounds of Formula **(Ia),** wherein PG² (*i.e.,* Protecting Group 2) can be a variety of protecting groups known to one skilled in the art, such as, a *tert*-butoxycarbonyl (*i.e.,* BOC) group; and X, Y, Z, R¹, R², R³, and R⁴ each have the same definitions as described herein, *supra* and *infra.* Intermediate **2-1** in **Fig. 2** can be prepared according to any one of Examples 2, 3, 4, and 5. In this scheme, the R¹ group and Z-R² group are introduced prior to the formation of the carbamate or urea functionality.

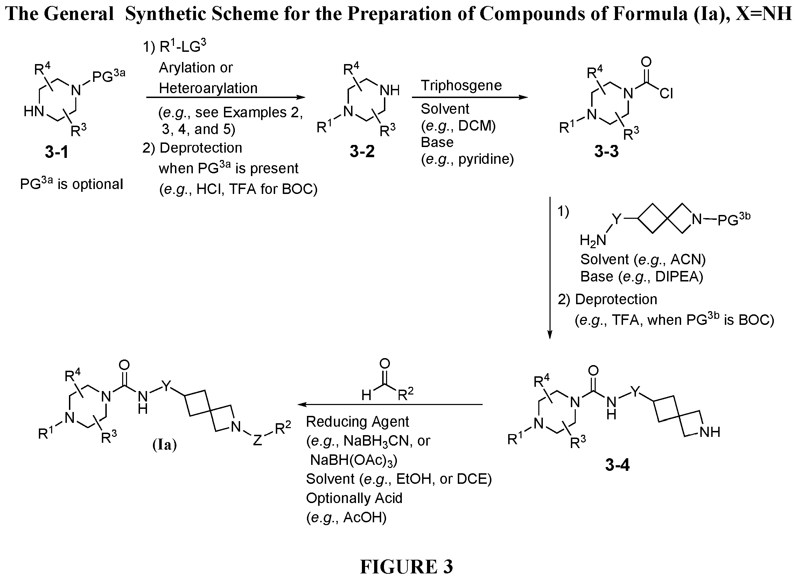

**Fig. 3** shows a general synthetic scheme for the preparation of compounds of Formula **(Ia),** wherein PG^{3a} (*i.e.,* Protecting Group 3a) and PG^{3b} (*i.e.,* Protecting Group 3b) can independently be a variety of protecting groups known to one skilled in the art, such as, a *tert*-butoxycarbonyl (*i.e.,* BOC) group; LG³ (*i.e.,* Leaving Group 3) can be, for example, Cl and Br; X=NH; and Y, Z, R¹, R², R³, and R⁴ each have the same definitions as described herein, *supra* and *infra.* In this scheme, the Z-R² group is introduced in the last step.

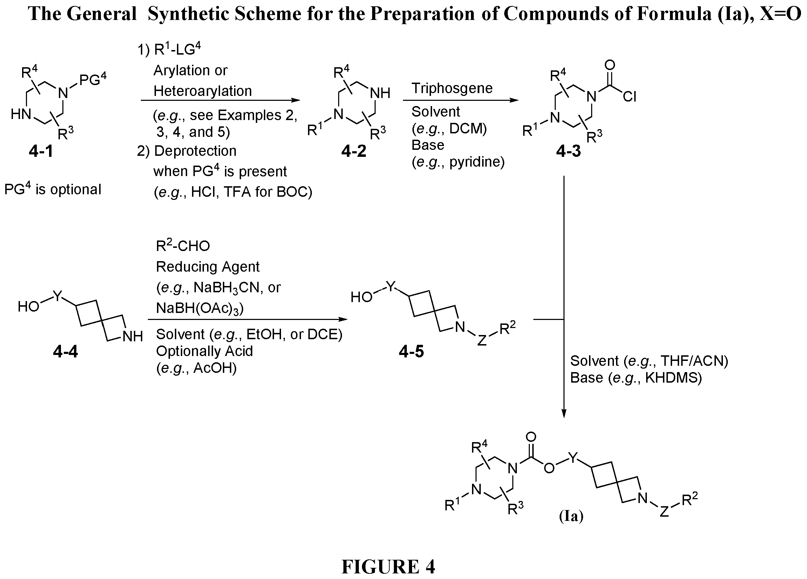

Fig. **4** shows a general synthetic scheme for the preparation of compounds of Formula **(Ia),** wherein PG⁴ (*i.e.,* Protecting Group 4) can be a variety of protecting groups known to one skilled in the art, such as, a *tert*-butoxycarbonyl (*i.e.,* BOC) group; LG⁴ (*i.e.,* Leaving Group 4) can be, for example, Cl and Br; X=O; and Y, Z, R¹, R², R³, and R⁴ each have the same definitions as described herein, *supra* and *infra.* In this scheme, the R¹ group and Z-R² group are each introduced prior to the formation of the carbamate functionality.

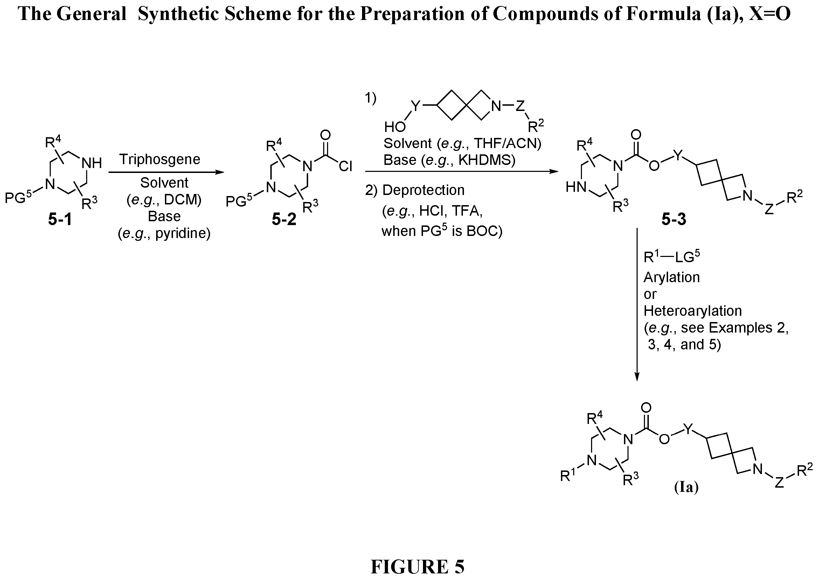

**Fig. 5** shows a general synthetic scheme for the preparation of compounds of Formula **(Ia),** wherein PG⁵ (*i.e.,* Protecting Group 5) can be a variety of protecting groups known to one skilled in the art, such as, a *tert*-butoxycarbonyl (*i.e.,* BOC) group; LG⁵ (*i.e.,* Leaving Group 5) can be, for example, Cl and Br; X=O; and Y, Z, R¹, R², R³, and R⁴ each have the same definitions as described herein, *supra* and *infra.* In this scheme, the R¹ group is introduced in the last step.

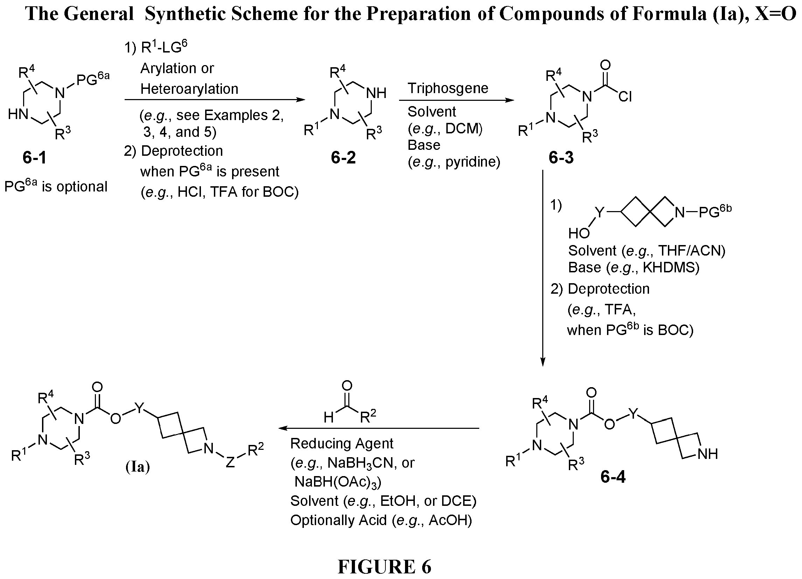

**Fig. 6** shows a general synthetic scheme for the preparation of compounds of Formula **(Ia),** wherein PG^{6a} (*i.e.,* Protecting Group 6a) and PG^{6b} (*i.e.,* Protecting Group 6b) can independently be a variety of protecting groups known to one skilled in the art, such as, a *tert*-butoxycarbonyl (*i.e.*, BOC) group; LG⁶ (*i.e.*, Leaving Group 6) can be, for example, Cl and Br; X=O; and Y, Z, R¹, R², R³, and R⁴ each have the same definitions as described herein, *supra* and *infra.* In this scheme, the Z-R² group is introduced in the last step.

### DETAILED DESCRIPTION OF THE INVENTION

### DEFINITIONS

For clarity and consistency, the following definitions will be used throughout this patent document.

As used herein, **"administering"** refers to providing a compound of the invention or other therapy, remedy or treatment to the individual in need of treatment in a form that can be introduced into that individual's body in a therapeutically useful form and therapeutically useful amount, including, but not limited to: oral dosage forms, such as, tablets, capsules, syrups, suspensions, and the like; injectable dosage forms, such as, IV, IM, IP, and the like; transdermal dosage forms, including creams, jellies, powders, and patches; buccal dosage forms; inhalation powders, sprays, suspensions, and the like; and rectal suppositories. A health care practitioner can directly provide a compound to an individual in the form of a sample or can indirectly provide a compound to an individual by providing an oral or written prescription for the compound. Also, for example, an individual can obtain a compound by themselves without the involvement of a health care practitioner. When the compound is administered to the individual, the body is transformed by the compound in some way. When a compound of the invention is provided in combination with one or more other agents, "administration" is understood to include the compound and other agents are administered at the same time or at different times. When the agents of a combination are administered at the same time, they can be administered together in a single composition or they can be administered separately. The preferred method of administration can vary depending on various factors, *e.g*., the components of the pharmaceutical formulation, the site of the disease, and the severity of the disease.

The term **"composition"** refers to a compound or crystalline form thereof, including but not limited to, salts, solvates, and hydrates of a compound of the present invention, in combination with at least one additional component, such as, a composition obtained/prepared during synthesis, preformulation, in-process testing (*e.g.,* TLC, HPLC, NMR samples), and the like.

The term **"hydrate"** as used herein refers to a compound of the invention or a salt thereof that further includes a stoichiometric or non-stoichiometric amount of water bound by non-covalent intermolecular forces.

The term "in **need of treatment"** and the term **"in need thereof"** when referring to treatment are used interchangeably to mean a judgment made by a caregiver (*e.g*. physician, nurse, nurse practitioner, *etc.* in the case of humans; veterinarian in the case of animals, including non-human mammals) that an individual or animal requires or will benefit from treatment. This judgment is made based on a variety of factors that are in the realm of a caregiver's expertise, but that includes the knowledge that the individual or animal is ill, or will become ill, as the result of a disease, condition or disorder that is treatable by the compounds of the invention. Accordingly, the compounds of the invention can be used in a protective or preventive manner; or compounds of the invention can be used to alleviate, inhibit, or ameliorate the disease, condition, or disorder.

The term **"individual"** or **"subject"** refers to any animal, including mammals, such as, mice, rats, other rodents, rabbits, dogs, cats, swine, cattle, sheep, horses, primates, and humans. In some embodiment "individual" refers to humans. In the context of a clinical trial or screening or activity experiment the subject may be a healthy volunteer or healthy participant without an underlying M₄ mediated disorder or condition or a volunteer or participant that has received a diagnosis for a disorder or condition in need of medical treatment as determined by a health care professional. In the context outside of a clinical trial a subject under the care of a health care professional who has received a diagnosis for a disorder or condition is typically described as a patient.

The term **"pediatric subject"** refers to a subject under the age of 21 years at the time of diagnosis or treatment. The term "pediatric" can be further divided into various subpopulations including: neonates (from birth through the first month of life); infants (1 month up to two years of age); children (two years of age up to 12 years of age); and adolescents (12 years of age through 21 years of age (up to, but not including, the twenty-second birthday)) see *e.g.,* Berhman et al., Textbook of Pediatrics, 15th Ed. Philadelphia: W.B. Saunders Company, 1996; Rudolph et al., Rudolph's Pediatrics, 21st Ed. New York: McGraw-Hill, 2002; and Avery et al., Pediatric Medicine, 2nd Ed. Baltimore: Williams & Wilkins; 1994.

The phrase **"pharmaceutically acceptable"** refers to compounds (and salts thereof), compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term **"pharmaceutical composition"** refers to a specific composition comprising at least one active ingredient; including but not limited to, salts, solvates, and hydrates of compounds of the present invention, whereby the composition is amenable to investigation for a specified, efficacious outcome in a mammal (for example, without limitation, a human). Those of ordinary skill in the art will understand and appreciate the techniques appropriate for determining whether an active ingredient has a desired efficacious outcome based upon the needs of the artisan.

The term **"prescribing"** refers to order, authorize, or recommend the use of a drug or other therapy, remedy, or treatment. In some embodiments, a health care provider orally advises, recommends, or authorizes the use of a compound, dosage regimen, or other treatment to an individual. The health care provider may or may not provide a written prescription for the compound, dosage regimen, or treatment. Further, the health care provider may or may not provide the compound or treatment to the individual. For example, the health care provider can advise the individual where to obtain the compound without providing the compound. In some embodiments, a health care provider can provide a written prescription for the compound, dosage regimen, or treatment to the individual. A prescription can be written on paper or recorded on electronic media. In addition, a prescription can be called in (oral) or faxed in (written) to a pharmacy or a dispensary. In some embodiments, a sample of the compound or treatment is given to the individual. As used herein, giving a sample of a compound constitutes an implicit prescription for the compound. Different health care systems around the world use different methods for prescribing and administering compounds or treatments, and these methods are encompassed by the disclosure herein. A health care provider can include, for example, a physician, nurse, nurse practitioner, or other health care professional who can prescribe or administer compounds (drugs) for the disorders disclosed herein. In addition, a health care provider can include anyone who can recommend, prescribe, administer, or prevent an individual from receiving a compound or drug, including, for example, an insurance provider.

The terms **"prevent", "preventing",** and **"prevention"** refer to the elimination or reduction of the occurrence or onset of one or more symptoms associated with a particular disorder. For example, the terms **"prevent", "preventing",** and **"prevention"** can refer to the administration of therapy on a prophylactic or preventative basis to an individual who may ultimately manifest at least one symptom of a disorder but who has not yet done so. Such individuals can be identified on the basis of risk factors that are known to correlate with the subsequent occurrence of the disease, such as the presence of a biomarker. Alternatively, prevention therapy can be administered as a prophylactic measure without prior identification of a risk factor. Delaying the onset of the at least one episode and/or symptom of a disorder can also be considered prevention or prophylaxis.

The term **"solvate"** refers to a solid form of a compound of the present invention (or a pharmaceutically acceptable salt thereof), which includes one or more molecules of a solvent in stoichiometric or non-stoichiometric amount. Wherein the solvent is water, the solvate is a hydrate. Alternatively, the solvent may be an organic solvent. The organic solvent includes, but is not limited to, methanol, ethanol, 1-propanol, 2-propanol, t-butanol, acetone, ethyl methyl ketone, 4-methyl-2-pentanone, cyclohexanone, acetonitrile, N,N-dimethylformamide, dimethylsulfoxide and ethyl acetate.

Processes for preparing a solvate of a compound of the present invention (or a pharmaceutically acceptable salt thereof) may include: (a) reaction of a compound of the present invention (or a pharmaceutically acceptable salt thereof) with a solvent; (b) precipitation of a complex from a solution of a compound of the present invention (or a pharmaceutically acceptable salt thereof) and a solvent; and (c) crystallization of a complex from a solution of a compound of the present invention (or a pharmaceutically acceptable salt thereof) and a solvent. The solvate may be in a crystalline form. Alternatively, the solvate may be in an amorphous form.

The terms **"treat", "treating",** and **"treatment"** refer to medical management of a disease, disorder, or condition of a subject (*e.g.,* patient) (see, *e.g.,* Stedman's Medical Dictionary). In general, an appropriate dose and treatment regimen provide the M₄ antagonist in an amount sufficient to provide therapeutic benefit. Therapeutic benefit for subjects to whom the M₄ antagonist compound(s) described herein are administered, includes, for example, an improved clinical outcome, wherein the object is to prevent or slow or retard (lessen) an undesired physiological change associated with the disease, or to prevent or slow or retard (lessen) the expansion or severity of such disease. The effectiveness of one or more M₄ antagonists may include beneficial or desired clinical results that comprise, but are not limited to, abatement, lessening, or alleviation of symptoms that result from or are associated with the disease to be treated; decreased occurrence of symptoms; improved quality of life; longer disease-free status (*i.e*., decreasing the likelihood or the propensity that a subject will present symptoms on the basis of which a diagnosis of a disease is made); diminishment of extent of disease; stabilized (*i.e.,* not worsening) state of disease; delay or slowing of disease progression; amelioration or palliation of the disease state; and remission (whether partial or total), whether detectable or undetectable; and/or overall survival.

The term **"therapeutically effective amount"** refers to the amount of the compound of the present invention or a pharmaceutically acceptable salt thereof, or an amount of a pharmaceutical composition comprising the compound of the invention or a pharmaceutically acceptable salt thereof, that elicits the biological or medicinal response in a tissue, system, animal, or human that is being sought by an individual, researcher, veterinarian, medical doctor, or other clinician or caregiver, which can include one or more of the following:
(1) preventing the disorder, for example, preventing a disease, condition, or disorder in an individual who may be predisposed to the disease, condition, or disorder but does not yet experience or display the relevant pathology or symptomatology;
(2) inhibiting the disorder, for example, inhibiting a disease, condition, or disorder in an individual who is experiencing or displaying the relevant pathology or symptomatology (*i.e.,* arresting further development of the pathology and/or symptomatology); and
(3) ameliorating the disorder, for example, ameliorating a disease, condition, or disorder in an individual who is experiencing or displaying the relevant pathology or symptomatology (*i.e.,* reversing the pathology and/or symptomatology).

### CHEMICAL GROUP, MOIETY OR RADICAL

The term **"amino"** refers to the group -NH₂.

The term **"C₆-C₁₀ aryl"** refers to a saturated ring system containing 6 to 10 carbon atoms that can contain a single ring or two fused rings and is aromatic, such as phenyl and naphthalenyl. When one or more substituents are present on the "aryl" ring, the substituent(s) can be bonded at any available ring carbon.

The term **"C₁-C₆ alkyl"** and **"C₁-C₄ alkyl"** refers to a saturated straight or branched carbon radical containing 1 to 6 carbons (*i.e.,* "C₁-C₆ alkyl") or 1 to 4 carbons (*i.e.,* "C₁-C₄ alkyl"). Some embodiments are 1 to 5 carbons (*i.e.,* C₁-C₅ alkyl), some embodiments are 1 to 4 carbons (*i.e.,* C₁-C₄ alkyl), some embodiments are 1 to 3 carbons (*i.e.,* C₁-C₃ alkyl), and some embodiments are 1 or 2 carbons. Examples of an alkyl group include: methyl, ethyl, *n-*propyl, isopropyl, *n*-butyl, *sec*-butyl, isobutyl, *tert-*butyl*,* pentyl, isopentyl, *tert*-pentyl, *neo*pentyl, 1-methylbutyl [*i.e.,* -CH(CH₃)CH₂CH₂CH₃], 2-methylbutyl [*i.e.,* -CH₂CH(CH₃)CH₂CH₃], *n*-hexyl and the like.

The term **"C₁-C₆ alkylamino"** refers to a radical consisting of one C₁-C₆ alkyl group bonded to an NH group, wherein C₁-C₆ alkyl has the same meaning as described herein. Some embodiments are "C1-C2 alkylamino". Some examples include methylamino, ethylamino, *n*-propylamino, isopropylamino, *n*-butylamino, *s*-butylamino, isobutylamino, *t-*butylamino, and the like.

The term **"C₁-C₆ alkylcarbamoyl"** refers to a radical consisting of a single C₁-C₆ alkyl group bonded to the nitrogen of a carbamoyl group, wherein carbamoyl and C₁-C₆ alkyl has the same definition as found herein. Some embodiments include C₁-C₄ alkylcarboxamide. Some embodiments include C₁-C₂ alkylcarboxamide. Examples include, *N-*methylcarboxamide, *N*-ethylcarboxamide, *N*-*n*-propylcarboxamide, *N*-isopropylcarboxamide, *N*-*n*-butylcarboxamide, *N-s*-butylcarboxamide, *N*-isobutylcarboxamide, *N-t-*butylcarboxamide, and the like.

The term **"C₁-C₄ alkylene"** refers to a straight or branched, saturated aliphatic, divalent radical having 1 to 4 carbon atoms. Some embodiments contain 1 to 3 carbons (*i.e.,* "C₁-C₃ alkylene"). Some embodiments contain 1 or 2 carbons (*i.e.,* "C₁-C₂ alkylene"). Some embodiments contain 1 carbon atom (*i.e.,* CH₂). Examples include, methylene (*i.e.,* CH₂), ethylene (*i.e*., CH₂CH₂), *n*-propylene (*i.e.,* CH₂CH₂CH₂), propane-1,1-diyl [*i.e.,* CH(CH₂CH₃)], propane-1,2-diyl [*i.e.,* CH₂CH(CH₃)], *n*-butylene (*i.e.,* CH₂CH₂CH₂CH₂), and the like.

The term **"C₁-C₆ alkoxy"** refers to a radical consisting of a C₁-C₆ alkyl group attached directly to an oxygen atom, wherein C₁-C₆ alkyl has the same definition as found herein. Some embodiments contain 1 to 5 carbons (*i.e.,* C₁-C₅ alkoxy). Some embodiments contain 1 to 4 carbons (*i.e.,* C₁-C₄ alkoxy). Some embodiments contain 1 to 3 carbons (*i.e.,* C₁-C₃ alkoxy). Some embodiments contain 1 or 2 carbons. Examples include methoxy, ethoxy, *n-*propoxy, isopropoxy, *n*-butoxy, *t*-butoxy, isobutoxy, *ses*-butoxy, and the like.

The term **"C₁-C₆ alkoxycarbonyl"** refers to a radical consisting of a single C₁-C₆ alkoxy group with the oxygen bonded to the carbon of a carbonyl group, wherein C₁-C₆ alkoxy has the same definition as found herein. Examples include methoxycarbonyl, ethoxycarbonyl, propoxycarbonyl, isopropoxycarbonyl, butoxycarbonyl, *sec*-butoxycarbonyl, isobutoxycarbonyl, *tert*-butoxycarbonyl, and the like.

The term **"C₁-C₆ alkylcarbonyl"** refers to a radical consisting of a C₁-C₆ alkyl group bonded directly to a carbonyl group, wherein C₁-C₆ alkyl has the same definition as found herein. Examples include acetyl, propionyl, butyryl, isobutyryl, pentanoyl, 2-methylbutanoyl, 3-methylbutanoyl, pivaloyl, and the like.

The term **"C₁-C₆ alkylsulfanyl"** or **"C₁-C₆ alkylthio"** refers to a radical consisting of a C₁-C₆ alkyl group bonded directly to a sulfur atom, wherein C₁-C₆ alkyl has the same definition as found herein. Examples include methylsulfanyl (*i.e.,* -S-CH₃), ethylsulfanyl (*i.e.,* -S-CH₂CH₃), *n*-propylsulfanyl (*i.e.,* -S-CH₂CH₂CH₃), isopropylsulfanyl, *n*-butylsulfanyl, *sec-*butylsulfanyl, isobutylsulfanyl, *t*-butylsulfanyl, and the like.

The term **"C₁-C₆ haloalkyl"** refers to a radical consisting of a C₁-C₆ alkyl group substituted with one or more halogens, wherein C₁-C₆ alkyl has the same definition as found herein. The C₁-C₆ haloalkyl may be fully substituted in which case it can be represented by the formula CₙL₂ₙ₊₁, wherein L is a halogen and "n" is 1, 2, 3, 4, 5, or 6. When more than one halogen is present then they may be the same or different and selected from: fluorine, chlorine, bromine, and iodine. In some embodiments, haloalkyl contains 1 to 5 carbons (*i.e.,* C₁-C₅ haloalkyl). In some embodiments, haloalkyl contains 1 to 4 carbons (*i.e.,* C₁-C₄ haloalkyl). In some embodiments, haloalkyl contains 1 to 3 carbons (*i.e.,* C₁-C₃ haloalkyl). In some embodiments, haloalkyl contains 1 or 2 carbons. Examples of haloalkyl groups include fluoromethyl, difluoromethyl, trifluoromethyl, chlorodifluoromethyl, 1-fluoroethyl, 2,2,2-trifluoroethyl, pentafluoroethyl, 4,4,4-trifluorobutyl, and the like.

The term **"C₁-C₆ alkylsulfinyl"** refers to a radical consisting a C₁-C₆ alkyl radical bonded to the sulfur of a sulfoxide radical of the formula: -S(=O)- wherein C₁-C₆ alkyl has the same definition as described herein. Examples include methylsulfinyl, ethylsulfinyl, *n-*propylsulfinyl, isopropylsulfinyl, *n*-butylsulfinyl, sec-butylsulfinyl, isobutylsulfinyl, t-butylsulfinyl, and the like.

The term **"carbonyl"** refers to the group -C(=O)-.

The term **"C₃-C₇ cycloalkyl"** refers to a saturated ring radical containing 3 to 7 carbons. Some embodiments contain 3 to 6 carbons. Some embodiments contain 3 to 5 carbons. Some embodiments contain 5 to 7 carbons. Some embodiments contain 3 to 4 carbons. Examples include cyclopropyl, cyclobutyl, cyclopentyl, cyclohexyl, and cycloheptyl.

The term **"C₂-C₆ dialkylamino"** refers to a radical consisting of an amino group substituted with two alkyl groups, the alkyl groups can be the same or different provided that two alkyl groups together do not exceed a total of 6 carbon atoms between the two alkyl groups. Some embodiments include C₂-C₄ dialkylamino. Some examples include dimethylamino, methylethylamino, diethylamino, methylpropylamino, methylbutylamino, methylpentylamino, methylisopropylamino, ethylpropylamino, ethylisopropylamino, dipropylamino, propylisopropylamino, and the like.

The term **"C₂-C₆ dialkylcarbamoyl"** refers to a radical consisting of two alkyl groups bonded to the nitrogen of a carbamoyl group and the two alkyl groups together do not exceed a total of 6 carbon atoms between the two alkyl groups. Some embodiments include C₂-C₄ dialkylamino carboxamide. Examples include, dimethylcarbamoyl, ethyl(methyl)carbamoyl, diethylcarbamoyl, methyl(propyl)carbamoyl, butyl(methyl)carbamoyl, and the like.

The term **"C₅-C₈ bicycloalkanyl"** refers to a cyclic alkyl system that is characterized by the presence of two atoms, termed "bridgehead atoms" that are connected to each other via one or more "bridging atoms". Examples include bicyclo[1.1.1]pentanyl, bicyclo[2.1.1]hexanyl, bicyclo[2.2.1]heptanyl, bicyclo[3.1.1]heptanyl, bicyclo[2.2.2]octanyl, bicyclo[3.2.1]octane, and the like.

The term **"C₆-C₈ bicycloalkenyl"** refers to a cyclic alkyl system that is characterized by the presence of two atoms, termed "bridgehead atoms" that are connected to each other via one or more "bridging atoms" and contains one double bond, provided a bridge head carbon is not part of the double bond (*i.e.,* the C₆-C₈ bicycloalkenyl groups complies with Bredt's rule). Examples include bicyclo[2.1.1]hex-2-enyl, bicyclo[2.2.1]hept-2-enyl, bicyclo[2.2.1]hept-5-enyl, bicyclo[3.1.1]hept-2-enyl, bicyclo[2.2.2]oct-2-enyl, bicyclo[3.2.1]oct-2-enyl, bicyclo[3.2.1]oct-3-enyl, bicyclo[3.2.1]oct-6-en-2-yl, and the like.

The term **"carbamoyl"** refers to the group -C(=O)NH₂.

The term **"cyano"** refers to the group -CN.

The term **"ethylene"** refers to the group -CH₂CH₂-.

The term **"halogen"** refers to fluoro, chloro, bromo, or iodo group. In some embodiments, halogen is fluoro, chloro, or bromo. In some embodiments, halogen is fluoro or chloro. In some embodiments, halogen is fluoro.

The term **"5-10 membered heteroaryl"** refers to an aromatic ring system containing 5 to 10 ring atoms in a single ring or two fused rings and having at least one ring group in the ring system selected from: O, S, N, and NH. Some embodiments are **"5-6 membered heteroaryl"** and refers to an aromatic ring containing 5 to 6 ring atoms in a single ring and has at least one ring group in the ring selected from: O, S, N, and NH. In some embodiments, **"5-10 membered heteroaryl"** refers to: furanyl, thiophenyl (*i.e.,* thienyl), pyrrolyl, imidazolyl, oxazolyl, thiazolyl, isoxazolyl, pyrazolyl, isothiazolyl, oxadiazolyl, triazolyl, tetrazolyl, thiadiazolyl, pyridinyl, pyrazinyl, pyrimidinyl, pyridazinyl, quinoxalinyl, triazinyl, benzofuranyl, 1*H*-indolyl, benzo[b]thiophenyl, and the like. In some embodiments, **"5-10 membered heteroaryl"** refers to: pyrazinyl, pyridazinyl, pyridinyl, pyrimidinyl, 1*H-*indolyl, quinoxalinyl, thiadiazolyl, and the like. It is understood, that when referring to the heteroaryl groups thiophenyl (thienyl), thiophen-2-yl (thien-2-yl), and thiophen-3-yl (thien-3-yl), they correspond to the following structures respectively:

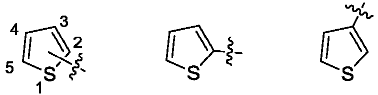

The term **"3-7 membered heterocyclyl"** refers to a non-aromatic ring system containing 3 to 7 ring atoms having one, two, or three ring groups in the ring system selected independently from: O, S, S(=O), S(=O)₂, and NH. In some embodiments, **"3-7 membered heterocyclyl"** refers to a non-aromatic ring radical containing 3 to 7 ring atoms having one or two ring groups in the ring system selected independently from: O, S, S(=O), S(=O)₂, and NH. In some embodiments, **"3-6 membered heterocyclyl"** refers to a non-aromatic ring radical containing 3 to 6 ring atoms having one or two ring groups in the ring system selected independently from: O, S, S(=O), S(=O)₂, and NH. In some embodiments, **"4-6 membered heterocyclyl"** refers to a non-aromatic ring radical containing 4 to 6 ring atoms having one or two ring groups in the ring system selected independently from: O, S, S(=O), S(=O)₂, and NH. In some embodiments, the one or two ring groups in the ring system are selected independently from: O and NH. Examples of a **"heterocyclyl"** group include: aziridinyl, azetidinyl, piperidinyl, morpholinyl, oxetanyl, piperazinyl, pyrrolidinyl, thiomorpholinyl, 1,1-dioxidothiomorpholinyl, oxolanyl (tetrahydrofuranyl), oxanyl (tetrahydropyranyl), and the like.

The term **"nitro"** refers to the group -NO₂.

### COMPOUNDS OF THE INVENTION

One aspect of the present invention encompasses, *inter alia,* certain 2-azaspiro[3.3]heptane compounds of Formula (**Ia**): 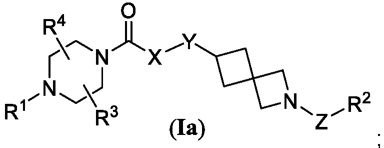 or a pharmaceutically acceptable salt thereof: wherein X, Y, Z, R¹, R², R³, and R⁴ all have the same definitions as described herein, *supra* and *infra.*

It is understood that R³ and R⁴ are bonded to different ethylene (*i.e.,* CH₂CH₂) groups of the piperazine ring. Accordingly, R³ and R⁴ are not bonded to the same carbon or to adjacent carbons, instead, R³ and R⁴ together with the piperazine to which they are bonded form the following rings systems, wherein:
a) R³ and R⁴ are each independently selected from H and C₁-C₄ alkyl (*i.e.,* Formulae **(Ia-1), (Ia-2),** and **(Ia-3));** or
b) R³ and R⁴ taken together form a bridging group selected from: CH₂ (*i.e.,* Formulae **(Ia-4), (Ia-5),** and **(Ia-6));** CH₂-CH₂ (*i.e.,* Formulae **(Ia-7), (Ia-8), (Ia-9);** and CH₂-O-CH₂ (*i.e.,* Formulae **(Ia-10),** and **(Ia-11)):** 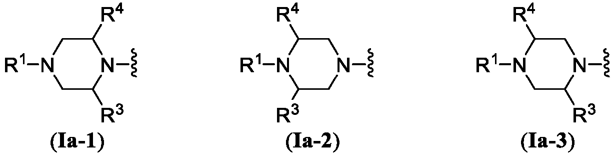 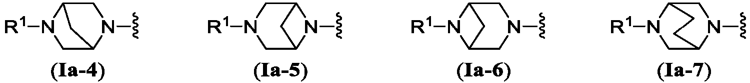 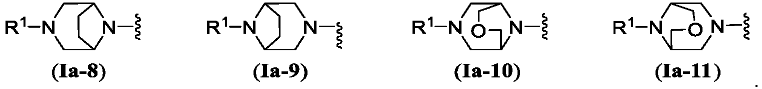 It is further understood that the remainder part of each of the Formulae **(Ia-1)** to **(Ia-11)** although not explicitly shown, refers to the following substructure: 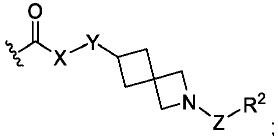 wherein the variables resulting from the combination of any one of Formulae **(Ia-1)** to **(Ia-11)** and the substructure have the same definitions as described herein *supra* and *infra,* an example for Formulae **(Ia-1)** is shown below:

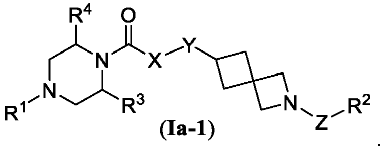

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination. All combinations of the embodiments pertaining to the chemical groups represented by the variables (*e.g*., X, Y, Z, R¹, R², R³, and R⁴) contained within the generic chemical formulae described herein, for example, Formulae **(Ia), (Ia-1), (Ia-2), (Ia-3), (Ia-4), (Ia-5), (Ia-6), (Ia-7), (Ia-8), (Ia-9), (Ia-10), (Ia-11), (IIa),** (**IIc**), (**IIe**), (**IIg**), (**IIi**), **(IIk),** (**IIIa**), (**IIIc**), and the formulae disclosed in the figures, are specifically embraced by the present invention just as if each and every combination was individually and explicitly recited, to the extent such combinations embrace compounds that result in stable compounds (*i.e.,* compounds that can be isolated, characterized, and tested for biological activity). In addition, all subcombinations of the chemical groups listed in the embodiments describing such variables, as well as all subcombinations of uses and medical indications described herein, are also specifically embraced by the present invention just as if each and every subcombination of chemical groups and subcombination of uses and medical indications was individually and explicitly recited herein.

As used herein, **"substituted"** indicates that at least one hydrogen atom of the chemical group is replaced by a non-hydrogen substituent or group, the non-hydrogen substituent or group can be monovalent or divalent. When the chemical group or substituent is divalent, then it is understood that this group is further substituted with another substituent or group. When a chemical group herein is **"substituted"** it may have up to the full valance of substitution; for example, a methyl group can be substituted by 1, 2, or 3 substituents, a methylene group can be substituted by 1 or 2 substituents, a phenyl group can be substituted by 1, 2, 3, 4, or 5 substituents, a naphthyl group can be substituted by 1, 2, 3, 4, 5, 6, or 7 substituents, and the like. Likewise, "substituted with one or more substituents" refers to the substitution of a group substituted with one substituent up to the total number of substituents physically allowed by the group. It is understood that "optionally substituted" as used herein refers to the group being either "unsubstituted" or "substituted" with a group. Accordingly, when a group is "optionally substituted with one or more substituents", it is understood that the group is either "unsubstituted" or "substituted" and when substituted, the group is substituted with one substituent up to the total number of substituents physically allowed by the group as described above. In some embodiments, a group can be "optionally substituted with one, two, three, or four substituents". In some embodiments, a group can be "optionally substituted with one, two, or three substituents". In some embodiments, a group can be "optionally substituted with one or two substituents". In some embodiments, a group can be "optionally substituted with one substituent". Further, when a group is substituted with more than one substituent, then the substituents can be identical, or they can be different.

It is understood and appreciated that compounds of Formula **(Ia)** and formulae related thereto may have one or more chiral centers and therefore can exist as enantiomers and/or diastereoisomers. Accordingly, it is understood that compounds of Formula (la) and the formulae used throughout this disclosure embrace all such enantiomers, diastereoisomers, and mixtures thereof, including but not limited to racemates, unless specifically stated or shown otherwise.

### The X Group

In some embodiments, X is O or NH.

In some embodiments, X is NH.

One aspect of the present invention pertains to compounds of Formula (**IIIa**) or a pharmaceutically acceptable salt thereof: 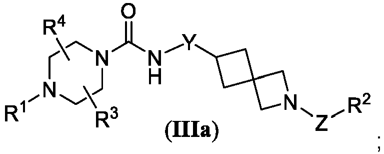 wherein: Y, Z, R¹, R², R³, and R⁴ all have the same definitions as described herein, *supra* and *infra.*

In some embodiments, X is O.

One aspect of the present invention pertains to compounds of Formula (**IIIc**) or a pharmaceutically acceptable salt thereof: 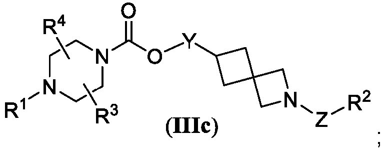 wherein: Y, Z, R¹, R², R³, and R⁴ all have the same definitions as described herein, *supra* and *infra.*

### The Y Group

In some embodiments, Y is CH₂ or absent.

In some embodiments, Y is CH₂.

In some embodiments, Y is absent.

### The Z Group

In some embodiments, Z is C₁-C₄ alkylene or absent.

In some embodiments, Z is C₁-C₄ alkylene.

In some embodiments, Z is CH₂.

In some embodiments, Z is absent.

### The R¹ Group

In some embodiments, R¹ is C₆-C₁₀ aryl or 5-10 membered heteroaryl, each optionally substituted with one or more groups selected from: C₁-C₆ alkoxy, C₁-C₆ alkoxycarbonyl, C₁-C₆ alkyl, C₁-C₆ alkylamino, C₁-C₆ alkylcarbonyl, C₁-C₆ alkylcarbamoyl, C₁-C₆ alkylsulfanyl, C₂-C₆ dialkylamino, C₂-C₆ dialkyl carbamoyl, C₁-C₆ sulfinyl, C₃-C₁₀ cycloalkyl, C₁-C₆ haloalkoxy, C₁-C₆ haloalkyl, amino, carbamoyl, cyano, halogen, and nitro.

In some embodiments, R¹ is C₆-C₁₀ aryl or 5-10 membered heteroaryl, each optionally substituted with one or more groups selected from: C₁-C₆ alkoxy, C₁-C₆ alkoxycarbonyl, C₁-C₆ alkyl, C₁-C₆ alkylcarbonyl, C₁-C₆ alkylsulfanyl, C₁-C₆ sulfinyl, C₁-C₆ haloalkoxy, C₁-C₆ haloalkyl, cyano, halogen, and nitro.

In some embodiments, R¹ is C₆-C₁₀ aryl or 5-10 membered heteroaryl, each optionally substituted with one, two, three, or four groups, wherein the groups can be selected from any of the lists of groups associated with R¹ as described herein, *supra* and *infra.* In some embodiments, R¹ is C₆-C₁₀ aryl or 5-10 membered heteroaryl, each optionally substituted with one, two, or three groups, wherein the groups can be selected from any of the lists of groups associated with R¹ as described herein, *supra* and *infra.* In some embodiments, R¹ is C₆-C₁₀ aryl or 5-10 membered heteroaryl, each optionally substituted with one or two groups, wherein the groups can be selected from any of the lists of groups associated with R¹ as described herein, *supra* and *infra.* In some embodiments, R¹ is C₆-C₁₀ aryl or 5-10 membered heteroaryl, each optionally substituted with one group, wherein the group can be selected from any of the lists of groups associated with R¹ as described herein, *supra* and *infra.*

In some embodiments, R¹ is C₆-C₁₀ aryl or 5-10 membered heteroaryl, each optionally substituted with one or more groups selected from: C₁-C₆ alkoxy, C₁-C₆ alkoxycarbonyl, C₁-C₆ alkyl, C₁-C₆ alkylcarbonyl, C₁-C₆ alkylsulfanyl, C₁-C₆ haloalkoxy, C₁-C₆ haloalkyl, cyano, halogen, and nitro.

In some embodiments, R¹ is C₆-C₁₀ aryl or 5-10 membered heteroaryl, each optionally substituted with one or more groups selected from: acetyl, bromo, chloro, cyano, difluoromethoxy, difluoromethyl, fluoro, methoxy, methoxycarbonyl, methyl, methylsulfanyl, nitro, trifluoromethoxy, and trifluoromethyl.

In some embodiments, R¹ is selected from: phenyl, pyrazinyl, pyridazinyl, pyridinyl, pyrimidinyl, quinoxalinyl, and thiadiazolyl, each optionally substituted with one or more groups selected from: C₁-C₆ alkoxy, C₁-C₆ alkoxycarbonyl, C₁-C₆ alkyl, C₁-C₆ alkylcarbonyl, C₁-C₆ alkylsulfanyl, C₁-C₆ haloalkoxy, C₁-C₆ haloalkyl, cyano, halogen, and nitro.

In some embodiments, R¹ is selected from: phenyl, pyrazinyl, pyridazinyl, pyridinyl, pyrimidinyl, quinoxalinyl, and thiadiazolyl, each optionally substituted with one or more groups selected from: acetyl, bromo, chloro, cyano, difluoromethoxy, difluoromethyl, fluoro, methoxy, methoxycarbonyl, methyl, methylsulfanyl, nitro, trifluoromethoxy, trifluoromethyl, and cyclopropyl.

In some embodiments, R¹ is selected from: phenyl, pyrazinyl, pyridazinyl, pyridinyl, pyrimidinyl, quinoxalinyl, and thiadiazolyl, each optionally substituted with one or more groups selected from: acetyl, bromo, chloro, cyano, difluoromethoxy, difluoromethyl, fluoro, methoxy, methoxycarbonyl, methyl, methylsulfanyl, nitro, trifluoromethoxy, and trifluoromethyl.

In some embodiments, R¹ is selected from: 1,3,4-thiadiazol-2-yl, phenyl, pyrazin-2-yl, pyridazin-3-yl, pyridin-2-yl, pyridin-3-yl, pyrimidin-2-yl, and quinoxalin-2-yl, each optionally substituted with one or more groups selected from: acetyl, bromo, chloro, cyano, difluoromethoxy, difluoromethyl, fluoro, methoxy, methoxycarbonyl, methyl, methylsulfanyl, nitro, trifluoromethoxy, and trifluoromethyl.

In some embodiments, R¹ is selected from: 2-cyano-4-(trifluoromethyl)phenyl, 3-(trifluoromethyl)pyridin-2-yl, 3,4,5-trifluorophenyl, 3,6-dimethylpyrazin-2-yl, 3-cyano-4-(trifluoromethoxy)phenyl, 3-cyano-4-fluorophenyl, 3-cyano-5-fluorophenyl, 4-(trifluoromethoxy)phenyl, 4-(trifluoromethyl)phenyl, 4-cyano-2,5-difluorophenyl, 4-cyano-2-fluorophenyl, 4-cyano-6-(trifluoromethyl)pyridin-3-yl, 4-methylpyrimidin-2-yl, 5-(difluoromethoxy)pyrimidin-2-yl, 5-(difluoromethyl)pyrazin-2-yl, 5-(methoxycarbonyl)-4-(trifluoromethyl)pyrimidin-2-yl, 5-(trifluoromethoxy)pyrazin-2-yl, 5-(trifluoromethyl)-1,3,4-thiadiazol-2-yl, 5-(trifluoromethyl)pyrazin-2-yl, 5-(trifluoromethyl) pyridin-2-yl, 5-(trifluoromethyl)pyrimidin-2-yl, 5-acetylpyrazin-2-yl, 5-bromo-4-(methylsulfanyl)pyrimidin-2-yl, 5-chloropyrazin-2-yl, 5-chloropyrimidin-2-yl, 5-cyano-3-methylpyrazin-2-yl, 5-cyanopyrazin-2-yl, 5-cyanopyridin-2-yl, 5-cyanopyrimidin-2-yl, 5-fluoropyrimidin-2-yl, 5-methoxypyrimidin-2-yl, 5-nitropyridin-2-yl, 6-(trifluoromethyl)pyrazin-2-yl, 6-(trifluoromethyl)pyridazin-3-yl, 6-(trifluoromethyl)pyridin-3-yl, 6-cyanopyrazin-2-yl, 6-fluoroquinoxalin-2-yl, 6-methoxy-3-nitropyridin-2-yl, 6-methoxypyrazin-2-yl, 6-methoxyquinoxalin-2-yl, and quinoxalin-2-yl.

In some embodiments, R¹ is 2-cyano-4-(trifluoromethyl)phenyl.

In some embodiments, R¹ is 3-(trifluoromethyl)pyridin-2-yl.

In some embodiments, R¹ is 3,4,5-trifluorophenyl.

In some embodiments, R¹ is 3,6-dimethylpyrazin-2-yl.

In some embodiments, R¹ is 3-cyano-4-(trifluoromethoxy)phenyl.

In some embodiments, R¹ is 3-cyano-4-fluorophenyl.

In some embodiments, R¹ is 3-cyano-5-fluorophenyl.

In some embodiments, R¹ is 4-(trifluoromethoxy)phenyl.

In some embodiments, R¹ is 4-(trifluoromethyl)phenyl.

In some embodiments, R¹ is 4-cyano-2,5-difluorophenyl.

In some embodiments, R¹ is 4-cyano-2-fluorophenyl.

In some embodiments, R¹ is 4-cyano-6-(trifluoromethyl)pyridin-3-yl.

In some embodiments, R¹ is 4-methylpyrimidin-2-yl.

In some embodiments, R¹ is 5-(difluoromethoxy)pyrimidin-2-yl.

In some embodiments, R¹ is 5-(difluoromethyl)pyrazin-2-yl.

In some embodiments, R¹ is 5-(methoxycarbonyl)-4-(trifluoromethyl)pyrimidin-2-yl.

In some embodiments, R¹ is 5-(trifluoromethoxy)pyrazin-2-yl.

In some embodiments, R¹ is 5-(trifluoromethyl)-1,3,4-thiadiazol-2-yl.

In some embodiments, R¹ is 5-(trifluoromethyl)pyrazin-2-yl.

In some embodiments, R¹ is 5-(trifluoromethyl)pyridin-2-yl.

In some embodiments, R¹ is 5-(trifluoromethyl)pyrimidin-2-yl.

In some embodiments, R¹ is 5-acetylpyrazin-2-yl.

In some embodiments, R¹ is 5-bromo-4-(methylsulfanyl)pyrimidin-2-yl.

In some embodiments, R¹ is 5-chloropyrazin-2-yl.

In some embodiments, R¹ is 5-chloropyrimidin-2-yl.

In some embodiments, R¹ is 5-cyano-3-methylpyrazin-2-yl.

In some embodiments, R¹ is 5-cyanopyrazin-2-yl.

In some embodiments, R¹ is 5-cyanopyridin-2-yl.

In some embodiments, R¹ is 5-cyanopyrimidin-2-yl.

In some embodiments, R¹ is 5-fluoropyrimidin-2-yl.

In some embodiments, R¹ is 5-methoxypyrimidin-2-yl.

In some embodiments, R¹ is 5-nitropyridin-2-yl.

In some embodiments, R¹ is 6-(trifluoromethyl)pyrazin-2-yl.

In some embodiments, R¹ is 6-(trifluoromethyl)pyridazin-3-yl.

In some embodiments, R¹ is 6-(trifluoromethyl)pyridin-3-yl.

In some embodiments, R¹ is 6-cyanopyrazin-2-yl.

In some embodiments, R¹ is 6-fluoroquinoxalin-2-yl.

In some embodiments, R¹ is 6-methoxy-3-nitropyridin-2-yl.

In some embodiments, R¹ is 6-methoxypyrazin-2-yl.

In some embodiments, R¹ is 6-methoxyquinoxalin-2-yl.

In some embodiments, R¹ is quinoxalin-2-yl.

### The R² Group

In some embodiments, R² is selected from: C₆-C₁₀ aryl, C₁-C₆ alkyl, C₃-C₇ cycloalkyl, C₅-C₈ bicycloalkanyl, C₆-C₈ bicycloalkenyl, 5-10 membered heteroaryl, and heterocyclyl; each optionally substituted with one or more groups selected from: C₁-C₄ alkyl, C₁-C₆ alkoxy, carbamoyl, cyano, C₁-C₆ haloalkoxy, C₁-C₆ sulfonyl, and halogen.

In some embodiments, R² is selected from: C₆-C₁₀ aryl, C₁-C₆ alkyl, C₃-C₇ cycloalkyl, C₅-C₈ bicycloalkanyl, C₆-C₈ bicycloalkenyl, 5-10 membered heteroaryl, and heterocyclyl; each optionally substituted with one, two, three, or four groups, wherein the groups can be selected from any of the lists of groups associated with R² as described herein, *supra* and *infra.* In some embodiments, R² is selected from: C₆-C₁₀ aryl, C₁-C₆ alkyl, C₃-C₇ cycloalkyl, C₅-C₈ bicycloalkanyl, C₆-C₈ bicycloalkenyl, 5-10 membered heteroaryl, and heterocyclyl; each optionally substituted with one, two, or three groups, wherein the groups can be selected from any of the lists of groups associated with R² as described herein, *supra* and *infra.* In some embodiments, R² is selected from: C₆-C₁₀ aryl, C₁-C₆ alkyl, C₃-C₇ cycloalkyl, C₅-C₈ bicycloalkanyl, C₆-C₈ bicycloalkenyl, 5-10 membered heteroaryl, and heterocyclyl; each optionally substituted with one or two groups, wherein the groups can be selected from any of the lists of groups associated with R² as described herein, *supra* and *infra.* In some embodiments, R² is selected from: C₆-C₁₀ aryl, C₁-C₆ alkyl, C₃-C₇ cycloalkyl, C₅-C₈ bicycloalkanyl, C₆-C₈ bicycloalkenyl, 5-10 membered heteroaryl, and heterocyclyl; each optionally substituted with one group, wherein the group can be selected from any of the lists of groups associated with R² as described herein, *supra* and *infra.*

In some embodiments, R² is selected from: C₆-C₁₀ aryl, C₁-C₆ alkyl, C₃-C₇ cycloalkyl, C₆-C₈ bicycloalkenyl, 5-10 membered heteroaryl, and heterocyclyl, each optionally substituted with one or more groups selected from: C₁-C₄ alkyl, C₁-C₆ alkoxy, carbamoyl, cyano, and halogen.

In some embodiments, R² is selected from: C₆-C₁₀ aryl, C₁-C₆ alkyl, C₃-C₇ cycloalkyl, C₆-C₈ bicycloalkenyl, 5-10 membered heteroaryl, and heterocyclyl, each optionally substituted with one or more groups selected from: carbamoyl, cyano, fluoro, methoxy, methyl, difluoromethoxy, and methylsulfonyl.

In some embodiments, R² is selected from: C₆-C₁₀ aryl, C₁-C₆ alkyl, C₃-C₇ cycloalkyl, C₆-C₈ bicycloalkenyl, 5-10 membered heteroaryl, and heterocyclyl, each optionally substituted with one or more groups selected from: carbamoyl, cyano, fluoro, methoxy, and methyl.

In some embodiments, R² is selected from: 1*H*-indolyl, 2,2-dimethylpropyl, 2-methylpropyl, 3,3-dimethylbutyl, bicyclo[2.2.1]heptenyl, butyl, cyclohexyl, cyclopropyl, oxanyl, and phenyl, each optionally substituted with one or more groups selected from: C₁-C₄ alkyl, C₁-C₆ alkoxy, carbamoyl, cyano, and halogen.

In some embodiments, R² is selected from: 1*H*-indol-5-yl, 2,2-dimethylpropyl, 2-methylpropyl, 3,3-dimethylbutyl, bicyclo[2.2.1]hept-5-en-2-yl, butyl, cyclohexyl, cyclopropyl, oxan-4-yl, and phenyl, each optionally substituted with one or more groups selected from: carbamoyl, cyano, fluoro, methoxy, and methyl.

In some embodiments, R² is selected from: 1*H*-indol-5-yl, 2,2-dimethylcyclopropyl, 2,2-dimethylpropyl, 2,6-difluorophenyl, 2-cyanophenyl, 2-fluorophenyl, 3,3-dimethylbutyl, 3,4-difluorophenyl, 3-carbamoylphenyl, 3-cyano-4-fluorophenyl, 3-fluorophenyl, 4,4,4-trifluorobutyl, 4-carbamoylphenyl, 4-cyanophenyl, 4-fluorophenyl, 4-methoxyphenyl, bicyclo[2.2.1]hept-5-en-2-yl, cyclohexyl, cyclopropyl, oxan-4-yl, and phenyl.

In some embodiments, R² is 1*H*-indol-5-yl.

In some embodiments, R² is 2,2-dimethylcyclopropyl.

In some embodiments, R² is 2,2-dimethylpropyl.

In some embodiments, R² is 2,6-difluorophenyl.

In some embodiments, R² is 2-cyanophenyl.

In some embodiments, R² is 2-fluorophenyl.

In some embodiments, R² is 3,3-dimethylbutyl.

In some embodiments, R² is 3,4-difluorophenyl.

In some embodiments, R² is 3-carbamoylphenyl.

In some embodiments, R² is 3-cyano-4-fluorophenyl.

In some embodiments, R² is 3-fluorophenyl.

In some embodiments, R² is 4,4,4-trifluorobutyl.

In some embodiments, R² is 4-carbamoylphenyl.

In some embodiments, R² is 4-cyanophenyl.

In some embodiments, R² is 4-fluorophenyl.

In some embodiments, R² is 4-methoxyphenyl.

In some embodiments, R² is bicyclo[2.2.1]hept-5-en-2-yl.

In some embodiments, R² is cyclohexyl.

In some embodiments, R² is cyclopropyl.

In some embodiments, R² is oxan-4-yl.

In some embodiments, R² is phenyl.

### The R³ Group

In some embodiments, R³ is selected from H and C₁-C₄ alkyl.

In some embodiments, R³ is H.

In some embodiments, R³ is C₁-C₄ alkyl.

In some embodiments, R³ is methyl.

### The R⁴ Group

In some embodiments, R⁴ is selected from H and C₁-C₄ alkyl.

In some embodiments, R⁴ is H.

In some embodiments, R⁴ is C₁-C₄ alkyl.

In some embodiments, R⁴ is methyl.

### Certain Combinations

### The Group Z and R² Together

In some embodiments, Z and R² together form a group selected from: (1*H*-indol-5-yl)methyl, (2,2-dimethylcyclopropyl)methyl, (2,6-difluorophenyl)methyl, (2-cyanophenyl)methyl, (2-fluorophenyl)methyl, (3,4-difluorophenyl)methyl, (3-carbamoylphenyl)methyl, (3-cyano-4-fluorophenyl)methyl, (3-fluorophenyl)methyl, (4-carbamoylphenyl)methyl, (4-cyanophenyl)methyl, (4-fluorophenyl)methyl, (4-methoxyphenyl)methyl, (bicyclo[2.2.1]hept-5-en-2-yl)methyl, (cyclohexyl)methyl, (cyclopropyl)methyl, (oxan-4-yl)methyl, 2,2-dimethylpropyl, 2-methylpropyl, 3,3-dimethylbutyl, 4,4,4-trifluorobutyl, and benzyl.

In some embodiments, Z and R² together is (1*H*-indol-5-yl)methyl.

In some embodiments, Z and R² together is (2,2-dimethylcyclopropyl)methyl.

In some embodiments, Z and R² together is (2,6-difluorophenyl)methyl.

In some embodiments, Z and R² together is (2-cyanophenyl)methyl.

In some embodiments, Z and R² together is (2-fluorophenyl)methyl.

In some embodiments, Z and R² together is (3,4-difluorophenyl)methyl.

In some embodiments, Z and R² together is (3-carbamoylphenyl)methyl.

In some embodiments, Z and R² together is (3-cyano-4-fluorophenyl)methyl.

In some embodiments, Z and R² together is (3-fluorophenyl)methyl.

In some embodiments, Z and R² together is (4-carbamoylphenyl)methyl.

In some embodiments, Z and R² together is (4-cyanophenyl)methyl.

In some embodiments, Z and R² together is (4-fluorophenyl)methyl.

In some embodiments, Z and R² together is (4-methoxyphenyl)methyl.

In some embodiments, Z and R² together is (bicyclo[2.2.1]hept-5-en-2-yl)methyl.

In some embodiments, Z and R² together is (cyclohexyl)methyl.

In some embodiments, Z and R² together is (cyclopropyl)methyl.

In some embodiments, Z and R² together is (oxan-4-yl)methyl.

In some embodiments, Z and R² together is 2,2-dimethylpropyl.

In some embodiments, Z and R² together is 2-methylpropyl.

In some embodiments, Z and R² together is 3,3-dimethylbutyl.

In some embodiments, Z and R² together is 4,4,4-trifluorobutyl.

In some embodiments, Z and R² together is benzyl.

### The R³ and R⁴ Groups

In some embodiments, R³ and R⁴ are each H.

One aspect of the present invention pertains to compounds of Formula (**IIa**) or a pharmaceutically acceptable salt thereof: 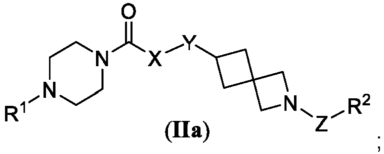 wherein: X, Y, Z, R¹, and R² all have the same definitions as described herein, *supra* and *infra.*

In some embodiments, R³ is C₁-C₄ alkyl; and R⁴ is H.

In some embodiments, R³ is methyl; and R⁴ is H.

One aspect of the present invention pertains to compounds of Formula (**IIc**) or a pharmaceutically acceptable salt thereof: 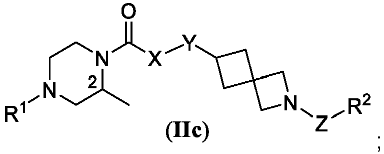 wherein: X, Y, Z, R¹, and R² all have the same definitions as described herein, *supra* and *infra.*

In some embodiments, the stereochemistry in Formula (**IIc**) for C(2) carbon is (*R*).

In some embodiments, the stereochemistry in Formula (**IIc**) for C(2) carbon is (*S*).

One aspect of the present invention pertains to compounds of Formula (**IIe**) or a pharmaceutically acceptable salt thereof: 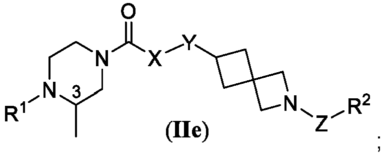 wherein: X, Y, Z, R¹, and R² all have the same definitions as described herein, *supra* and *infra.*

In some embodiments, the stereochemistry in Formula (**IIe**) for the C(3) carbon is (*R*)*.*

In some embodiments, the stereochemistry in Formula (**IIe**) for the C(3) carbon is (S).

In some embodiments, R³ and R⁴ are each C₁-C₄ alkyl.

In some embodiments, R³ and R⁴ are each methyl.

One aspect of the present invention pertains to compounds of Formula (**IIg**) or a pharmaceutically acceptable salt thereof: 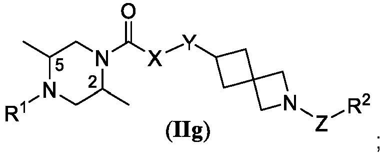 wherein: X, Y, Z, R¹, and R² all have the same definitions as described herein, *supra* and *infra.*

In some embodiments, the stereochemistry in Formula (**IIg**) for the C(2) carbon is (*R*) and the C(5) carbon is (*S*).

In some embodiments, the stereochemistry in Formula (**IIg**) for the C(2) carbon is (*R*) and the C(5) carbon is (*R*).

In some embodiments, the stereochemistry in Formula (**IIg**) for the C(2) carbon is (*S*) and the C(5) carbon is (*R*).

In some embodiments, the stereochemistry in Formula (**IIg**) for the C(2) carbon is (S) and the C(5) carbon is (*S*).

One aspect of the present invention pertains to compounds of Formula **(IIi)** or a pharmaceutically acceptable salt thereof: 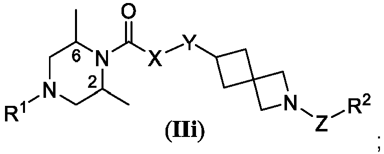 wherein: X, Y, Z, R¹, and R² all have the same definitions as described herein, *supra* and *infra.*

In some embodiments, the stereochemistry in Formula **(IIi)** for the C(2) carbon is (*R*) and the C(6) carbon is (*R*).

In some embodiments, the stereochemistry in Formula **(IIi)** for the C(2) carbon is (*S*) and the C(6) carbon is (*S*).

In some embodiments, the stereochemistry in Formula **(IIi)** for the C(2) carbon is (*R*) and the C(6) carbon is (*S*).

One aspect of the present invention pertains to compounds of Formula **(IIk)** or a pharmaceutically acceptable salt thereof: 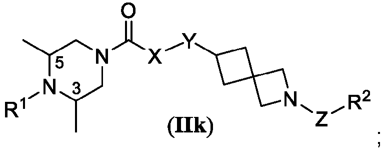 wherein: X, Y, Z, R¹, and R² all have the same definitions as described herein, *supra* and *infra.*

In some embodiments, the stereochemistry in Formula **(IIk)** for the C(3) carbon is (*R*) and the C(5) carbon is (*R*).

In some embodiments, the stereochemistry in Formula **(IIk)** for the C(3) carbon is (*S*) and the C(5) carbon is (*S*).

In some embodiments, R³ and R⁴ taken together form a bridging group selected from: CH₂, CH₂-CH₂, and CH₂-O-CH₂.

In some embodiments, R³ and R⁴ taken together form a bridging group that is CH₂.

One aspect of the present invention pertains to compounds of Formula **(Ia-4)** or a pharmaceutically acceptable salt thereof: 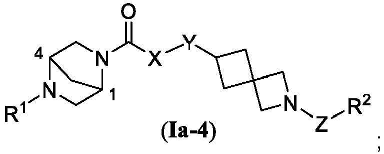 wherein: X, Y, Z, R¹, and R² all have the same definitions as described herein, *supra* and *infra.*

In some embodiments, the stereochemistry in Formula **(Ia-4)** for the C(1) carbon is (*R*) and the C(4) carbon is (*R*).

In some embodiments, the stereochemistry in Formula **(Ia-4)** for the C(1) carbon is (*S*) and the C(4) carbon is (*S*).

One aspect of the present invention pertains to compounds of Formula **(Ia-5)** or a pharmaceutically acceptable salt thereof: 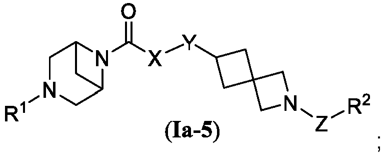 wherein: X, Y, Z, R¹, and R² all have the same definitions as described herein, *supra* and *infra.*

In some embodiments, R³ and R⁴ taken together form a bridging group that is CH₂-CH₂.

One aspect of the present invention pertains to compounds of Formula **(Ia-7)** or a pharmaceutically acceptable salt thereof: 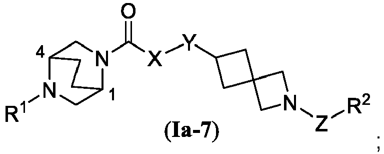 wherein: X, Y, Z, R¹, and R² all have the same definitions as described herein, *supra* and *infra.*

In some embodiments, the stereochemistry in Formula **(Ia-7)** for the C(1) carbon is (*R*) and the C(4) carbon is (*R*).

In some embodiments, the stereochemistry in Formula **(Ia-7)** for the C(1) carbon is (*S*) and the C(4) carbon is (*S*).

One aspect of the present invention pertains to compounds of Formula **(Ia-8)** or a pharmaceutically acceptable salt thereof: 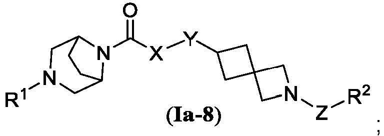 wherein: X, Y, Z, R¹, and R² all have the same definitions as described herein, *supra* and *infra.*

In some embodiments, R³ and R⁴ taken together form a bridging group that is CH₂-O-CH₂.

One aspect of the present invention pertains to compounds of Formula **(Ia-10)** or a pharmaceutically acceptable salt thereof: 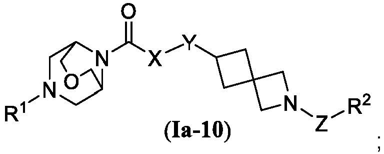 wherein: X, Y, Z, R¹, and R² all have the same definitions as described herein, *supra* and *infra.*

One aspect of the present invention pertains to compounds of Formula **(Ia-11)** or a pharmaceutically acceptable salt thereof: 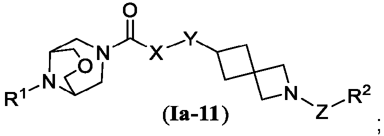 wherein: X, Y, Z, R¹, and R² all have the same definitions as described herein, *supra* and *infra.*

### Certain Combinations

One aspect of the present invention pertains to compounds of Formula **(IIIa):** 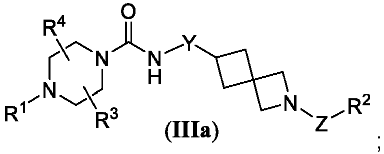 or a pharmaceutically acceptable salt thereof:
wherein:
Y is CH₂ or absent;
Z is CH₂ or absent;
R¹ is C₆-C₁₀ aryl or 5-10 membered heteroaryl, each optionally substituted with one or more groups selected from: C₁-C₆ alkoxy, C₁-C₆ haloalkoxy, C₁-C₆ haloalkyl, cyano, and halogen;
R² is selected from: C₆-C₁₀ aryl, C₁-C₆ alkyl, C₃-C₇ cycloalkyl, C₆-C₈ bicycloalkenyl, 5-10 membered heteroaryl, and heterocyclyl, each optionally substituted with one or more groups selected from: C₁-C₄ alkyl, C₁-C₆ alkoxy, carbamoyl, cyano, and halogen; and
R³ and R⁴ are each independently selected from H and C₁-C₄ alkyl, and R³ and R⁴ are bonded to different ethylene groups of the piperazine ring.

One aspect of the present invention pertains to compounds of Formula **(IIIa):** 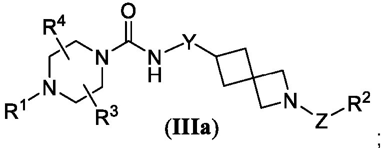 or a pharmaceutically acceptable salt thereof:
wherein:
Y is CH₂ or absent;
Z is CH₂ or absent;
R¹ is selected from: phenyl, pyrazinyl, pyridazinyl, pyridinyl, pyrimidinyl, and thiadiazolyl, each optionally substituted with one or more groups selected from: chloro, cyano, difluoromethoxy, difluoromethyl, fluoro, methoxy, trifluoromethoxy, and trifluoromethyl;
R² is selected from: 1*H*-indolyl, 2,2-dimethylpropyl, 2-methylpropyl, 3,3-dimethylbutyl, bicyclo[2.2.1]heptenyl, butyl, cyclohexyl, cyclopropyl, oxanyl, and phenyl; each optionally substituted with one or more groups selected from: carbamoyl, cyano, fluoro, methoxy, and methyl; and
R³ and R⁴ are each independently selected from H and methyl, and R³ and R⁴ are bonded to different ethylene groups of the piperazine ring.

One aspect of the present invention pertains to compounds of Formula **(IIIa):** 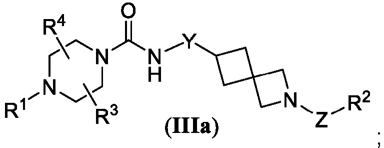 or a pharmaceutically acceptable salt thereof:
wherein:
Y is CH₂ or absent;
R¹ is selected from: 3,4,5-trifluorophenyl, 3-cyano-4-(trifluoromethoxy)phenyl, 3-cyano-4-fluorophenyl, 4-(trifluoromethoxy)phenyl, 4-(trifluoromethyl)phenyl, 4-cyano-2-fluorophenyl, 5-(difluoromethoxy)pyrimidin-2-yl, 5-(difluoromethyl)pyrazin-2-yl, 5-(trifluoromethyl)-1,3,4-thiadiazol-2-yl, 5-(trifluoromethyl)pyrazin-2-yl, 5-(trifluoromethyl) pyridin-2-yl, 5-(trifluoromethyl)pyrimidin-2-yl, 5-chloropyrazin-2-yl, 5-chloropyrimidin-2-yl, 5-cyanopyrazin-2-yl, 5-cyanopyridin-2-yl, 5-cyanopyrimidin-2-yl, 5-methoxypyrimidin-2-yl, 6-(trifluoromethyl)pyrazin-2-yl, 6-(trifluoromethyl)pyridazin-3-yl, and 6-cyanopyrazin-2-yl;
Z-R² taken together is selected from: (1*H*-indol-5-yl)methyl, (2,2-dimethylcyclopropyl)methyl, (2,6-difluorophenyl)methyl, (2-cyanophenyl)methyl, (2-fluorophenyl)methyl, (3,4-difluorophenyl)methyl, (3-carbamoylphenyl)methyl, (3-cyano-4-fluorophenyl)methyl, (3-fluorophenyl)methyl, (4-carbamoylphenyl)methyl, (4-cyanophenyl)methyl, (4-fluorophenyl)methyl, (4-methoxyphenyl)methyl, (bicyclo[2.2.1]hept-5-en-2-yl)methyl, (cyclohexyl)methyl, (cyclopropyl)methyl, (oxan-4-yl)methyl, 2,2-dimethylpropyl, 2-methylpropyl, 3,3-dimethylbutyl, 4,4,4-trifluorobutyl, and benzyl; and
R³ and R⁴ are each independently selected from H and methyl, and R³ and R⁴ are bonded to different ethylene groups of the piperazine ring.

One aspect of the present invention pertains to compounds of Formula (**IIIc**): 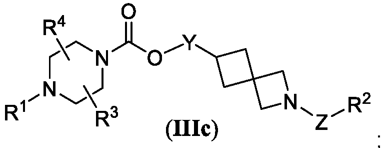 or a pharmaceutically acceptable salt thereof:
wherein:
Y is CH₂ or absent;
Z is CH₂ or absent;
R¹ is C₆-C₁₀ aryl or 5-10 membered heteroaryl, each optionally substituted with one or more groups selected from: C₁-C₆ alkoxy, C₁-C₆ alkyl, C₁-C₆ alkylcarbonyl, C₁-C₆ haloalkoxy, C₁-C₆ haloalkyl, cyano, halogen, nitro, C₁-C₆ alkoxycarbonyl, and C₁-C₆ alkylsulfanyl;
R² is selected from: C₆-C₁₀ aryl, C₁-C₆ alkyl, C₃-C₇ cycloalkyl, and C₆-C₈ bicycloalkenyl, each optionally substituted with one or more groups selected from: C₁-C₄ alkyl and halogen; and
R³ and R⁴ are each independently selected from H and C₁-C₄ alkyl, and R³ and R⁴ are bonded to different ethylene groups of the piperazine ring.

One aspect of the present invention pertains to compounds of Formula (**IIIc**): 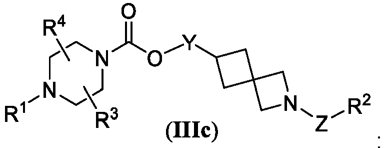 or a pharmaceutically acceptable salt thereof:
wherein:
Y is CH₂ or absent;
Z is CH₂ or absent;
R¹ is selected from: phenyl, pyrazinyl, pyridazinyl, pyridinyl, pyrimidinyl, and quinoxalinyl, each optionally substituted with one or more groups selected from: acetyl, bromo, cyano, fluoro, methoxy, methyl, nitro, trifluoromethoxy, trifluoromethyl, methoxycarbonyl, and methylsulfanyl;
R² is selected from: 2,2-dimethylpropyl, 3,3-dimethylbutyl, bicyclo[2.2.1]hept-5-en-2-yl, cyclohexyl, cyclopropyl, and phenyl, each optionally substituted with one or more groups selected from: methyl and fluoro; and
R³ and R⁴ are each independently selected from H and methyl, and R³ and R⁴ are bonded to different ethylene groups of the piperazine ring.

One aspect of the present invention pertains to compounds of Formula (**IIIc**): 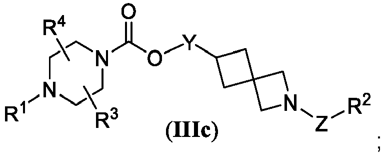 or a pharmaceutically acceptable salt thereof:
wherein:
Y is CH₂ or absent;
R¹ is selected from: 2-cyano-4-(trifluoromethyl)phenyl, 3-(trifluoromethyl)pyridin-2-yl, 3,4,5-trifluorophenyl, 3,6-dimethylpyrazin-2-yl, 3-cyano-4-(trifluoromethoxy)phenyl, 3-cyano-5-fluorophenyl, 4-(trifluoromethoxy)phenyl, 4-(trifluoromethyl)phenyl, 4-cyano-2,5-difluorophenyl, 4-cyano-2-fluorophenyl, 4-cyano-6-(trifluoromethyl)pyridin-3-yl, 4-methylpyrimidin-2-yl, 5-(methoxycarbonyl)-4-(trifluoromethyl)pyrimidin-2-yl, 5-(trifluoromethoxy)pyrazin-2-yl, 5-(trifluoromethyl)pyrazin-2-yl, 5-(trifluoromethyl)pyridin-2-yl, 5-(trifluoromethyl)pyrimidin-2-yl, 5-acetylpyrazin-2-yl, 5-bromo-4-(methylsulfanyl)pyrimidin-2-yl, 5-cyano-3-methylpyrazin-2-yl, 5-cyanopyrazin-2-yl, 5-cyanopyrimidin-2-yl, 5-fluoropyrimidin-2-yl, 5-nitropyridin-2-yl, 6-(trifluoromethyl)pyrazin-2-yl, 6-(trifluoromethyl) pyridazin-3-yl, 6-(trifluoromethyl)pyridin-3-yl, 6-fluoroquinoxalin-2-yl, 6-methoxy-3-nitropyridin-2-yl, 6-methoxypyrazin-2-yl, 6-methoxyquinoxalin-2-yl, and quinoxalin-2-yl;
Z-R² taken together is selected from: (2,2-dimethylcyclopropyl)methyl, (2,6-difluorophenyl)methyl, (bicyclo[2.2.1]hept-5-en-2-yl)methyl, (cyclohexyl)methyl, 2,2-dimethylpropyl, 3,3-dimethylbutyl, and benzyl; and
R³ and R⁴ are each independently selected from H and methyl, and R³ and R⁴ are bonded to different ethylene groups of the piperazine ring.

One aspect of the present invention pertains to compounds of Formula **(IIi):** 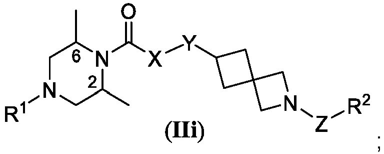 or a pharmaceutically acceptable salt thereof:
wherein:
X is O or NH;
Y is CH₂ or absent;
Z is CH₂ or absent;
R¹ is C₆-C₁₀ aryl or 5-10 membered heteroaryl, each optionally substituted with one or more groups selected from: C₁-C₆ alkoxy, C₁-C₆ alkoxycarbonyl, C₁-C₆ alkyl, C₁-C₆ alkylcarbonyl, C₁-C₆ alkylsulfanyl, C₁-C₆ haloalkoxy, C₁-C₆ haloalkyl, cyano, halogen, and nitro; and
R² is selected from: C₆-C₁₀ aryl, C₁-C₆ alkyl, C₃-C₇ cycloalkyl, C₆-C₈ bicycloalkenyl, 5-10 membered heteroaryl, and heterocyclyl, each optionally substituted with one or more groups selected from: C₁-C₄ alkyl, C₁-C₆ alkoxy, carbamoyl, cyano, and halogen.

In some embodiments, X is O.

In some embodiments, X is NH.

In some embodiments, Y is CH₂.

In some embodiments, Y is absent.

In some embodiments, R¹ is 6-membered heteroaryl.

In some embodiments, R¹ is phenyl, pyridinyl, pyrazinyl, or pyrimidinyl.

In some embodiments, Z is CH₂.

In some embodiments, Z is absent.

In some embodiments, R² is C₆-C₁₀ aryl. In a further embodiment, R² is phenyl.

In some embodiments, the stereochemistry in Formula **(IIi)** for the C(2) carbon is (*R*) and the C(6) carbon is (*R*).

In some embodiments, the stereochemistry in Formula **(IIi)** for the C(2) carbon is (*S*) and the C(6) carbon is (*S*).

In some embodiments, the stereochemistry in Formula **(IIi)** for the C(2) carbon is (*R*) and the C(6) carbon is (*S*).

In some embodiments, R¹ is 6-membered heteroaryl and R² is C₆-C₁₀ aryl.

In some embodiments, R¹ is phenyl, pyridinyl, pyrazinyl, or pyrimidinyl, and R² is C₆-C₁₀ aryl.

In some embodiments, R¹ is 6-membered heteroaryl and X is O.

In some embodiments, R¹ is phenyl, pyridyl, pyrazinyl, or pyrimidinyl, and X is O. In a further embodiment, R² is C₆-C₁₀ aryl.

In some embodiments, R¹ is 6-membered heteroaryl and X is NH.

In some embodiments, R¹ is phenyl, pyridyl, pyrazinyl or pyrimidinyl, and X is NH. In a further embodiment, R² is C₆-C₁₀ aryl.

In some embodiments, R¹ is 6-membered heteroaryl, X is O, Z is CH₂, and R² is C₆-C₁₀ aryl. In a further embodiment, R² is phenyl.

In some embodiments, R¹ is phenyl, pyridyl, pyrazinyl or pyrimidinyl, X is O, Z is CH₂, and R² is C₆-C₁₀ aryl. In a further embodiment, R² is phenyl.

In some embodiments, R¹ is 6-membered heteroaryl, X is NH, Z is CH₂, and R² is C₆-C₁₀ aryl. In a further embodiment, R² is phenyl.

In some embodiments, R¹ is phenyl, pyridinyl, pyrazinyl, or pyrimidinyl, X is NH, Z is CH₂, and R² is C₆-C₁₀ aryl. In a further embodiment, R² is phenyl.

In some embodiments, R¹ is phenyl, pyridinyl, pyrazinyl, or pyrimidinyl, X is O, Z is CH₂, R² is C₆-C₁₀ aryl, and stereochemistry for the C(2) carbon is (*R*) and the C(6) carbon is (*R*)*.* In a further embodiment, R² is phenyl.

In some embodiments, R¹ is phenyl, pyridinyl, pyrazinyl, or pyrimidinyl, X is NH, Z is CH₂, R² is C₆-C₁₀ aryl, and stereochemistry for the C(2) carbon is (*R*) and the C(6) carbon is (*R*)*.* In a further embodiment, R² is phenyl.

In some embodiments, R¹ is phenyl, pyridinyl, pyrazinyl, or pyrimidinyl, X is O, Z is CH₂, R² is C₆-C₁₀ aryl, and stereochemistry for the C(2) carbon is (*R*) and the C(6) carbon is (*S*)*.* In a further embodiment, R² is phenyl.

In some embodiments, R¹ is phenyl, pyridinyl, pyrazinyl, or pyrimidinyl, X is NH, Z is CH₂, R² is C₆-C₁₀ aryl, and stereochemistry for the C(2) carbon is (*R*) and the C(6) carbon is (*S*)*.* In a further embodiment, R² is phenyl.

In some embodiments, R¹ is phenyl, pyridinyl, pyrazinyl, or pyrimidinyl, X is O, Z is CH₂, R² is C₆-C₁₀ aryl, and stereochemistry for the C(2) carbon is (*S*) and the C(6) carbon is (*S*)*.* In a further embodiment, R² is phenyl.

In some embodiments, R¹ is phenyl, pyridinyl, pyrazinyl, or pyrimidinyl, X is NH, Z is CH₂, R² is C₆-C₁₀ aryl, and stereochemistry for the C(2) carbon is (*S*) and the C(6) carbon is (*S*)*.* In a further embodiment, R² is phenyl.

One aspect of the present invention pertains to compounds of Formula (Ili): 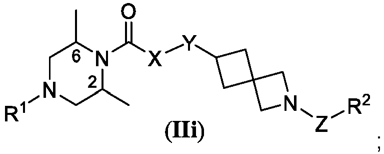 or a pharmaceutically acceptable salt thereof:
wherein:
X is O or NH;
Y is CH₂ or absent;
Z is CH₂ or absent;
R¹ is selected from: phenyl, pyrazinyl, pyridazinyl, pyridinyl, pyrimidinyl, and quinoxalinyl, each optionally substituted with one or more groups selected from: acetyl, bromo, chloro, cyano, difluoromethoxy, difluoromethyl, fluoro, methoxy, methoxycarbonyl, methyl, methylsulfanyl, nitro, trifluoromethoxy, and trifluoromethyl; and
R² is selected from: 1*H*-indolyl, 2,2-dimethylpropyl, 2-methylpropyl, 3,3-dimethylbutyl, bicyclo[2.2.1]heptenyl, butyl, cyclohexyl, cyclopropyl, oxanyl, and phenyl, each optionally substituted with one or more groups selected from: carbamoyl, cyano, fluoro, methoxy, and methyl.

In some embodiments, X is O.

In some embodiments, X is NH.

In some embodiments, Y is CH₂.

In some embodiments, Y is absent.

In some embodiments, R¹ is phenyl, pyridinyl, pyrazinyl, or pyrimidinyl.

In some embodiments, Z is CH₂.

In some embodiments, Z is absent.

In some embodiments, R² is phenyl.

In some embodiments, the stereochemistry in Formula **(IIi)** for the C(2) carbon is (*R*) and the C(6) carbon is (*R*).

In some embodiments, the stereochemistry in Formula **(IIi)** for the C(2) carbon is (*S*) and the C(6) carbon is (*S*).

In some embodiments, the stereochemistry in Formula **(IIi)** for the C(2) carbon is (*R*) and the C(6) carbon is (*S*).

In some embodiments, R¹ is phenyl, pyridinyl, pyrazinyl, or pyrimidinyl, and R² is phenyl.

In some embodiments, R¹ is phenyl, pyridinyl, pyrazinyl, or pyrimidinyl, and X is O. In a further embodiment, R² is phenyl.

In some embodiments, R¹ is phenyl, pyridinyl, pyrazinyl, or pyrimidinyl, and X is NH. In a further embodiment, R² is phenyl.

In some embodiments, R¹ is phenyl, pyridinyl, pyrazinyl, or pyrimidinyl, X is O, and Z is CH₂. In a further embodiment, R² is phenyl.

In some embodiments, R¹ is phenyl, pyridinyl, pyrazinyl, or pyrimidinyl, X is NH, and Z is CH₂. In a further embodiment, R² is phenyl.

In some embodiments, R¹ is phenyl, pyridyl, pyrazinyl, or pyrimidinyl, X is O, Z is CH₂, and stereochemistry for the C(2) carbon is (*R*) and the C(6) carbon is (*R*). In a further embodiment, R² is phenyl.

In some embodiments, R¹ is phenyl, pyridyl, pyrazinyl, or pyrimidinyl, X is NH, Z is CH₂, and stereochemistry for the C(2) carbon is (*R*) and the C(6) carbon is (*R*). In a further embodiment, R² is phenyl.

In some embodiments, R¹ is phenyl, pyridinyl, pyrazinyl, or pyrimidinyl, X is O, Z is CH₂, and stereochemistry for the C(2) carbon is (*R*) and the C(6) carbon is (*S*). In a further embodiment, R² is phenyl.

In some embodiments, R¹ is phenyl, pyridinyl, pyrazinyl, or pyrimidinyl, X is NH, Z is CH₂, and stereochemistry for the C(2) carbon is (*R*) and the C(6) carbon is (*S*). In a further embodiment, R² is phenyl.

In some embodiments, R¹ is phenyl, pyridinyl, pyrazinyl, or pyrimidinyl, X is O, Z is CH₂, and stereochemistry for the C(2) carbon is (*S*) and the C(6) carbon is (*S*). In a further embodiment, R² is phenyl.

In some embodiments, R¹ is phenyl, pyridinyl, pyrazinyl, or pyrimidinyl, X is NH, Z is CH₂, and stereochemistry for the C(2) carbon is (*S*) and the C(6) carbon is (*S*). In a further embodiment, R² is phenyl.

One aspect of the present invention pertains to compounds of Formula (Ili): 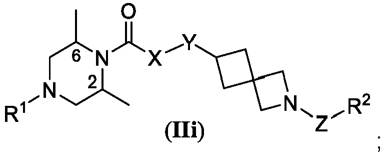 or a pharmaceutically acceptable salt thereof:
wherein:
X is O or NH;
Y is CH₂ or absent;
R¹ is selected from: 3-(trifluoromethyl)pyridin-2-yl, 3,4,5-trifluorophenyl, 4-(trifluoromethyl)phenyl, 4-cyano-6-(trifluoromethyl)pyridin-3-yl, 4-methylpyrimidin-2-yl, 5-(difluoromethoxy)pyrimidin-2-yl, 5-(difluoromethyl)pyrazin-2-yl, 5-(methoxycarbonyl)-4-(trifluoromethyl)pyrimidin-2-yl, 5-(trifluoromethoxy)pyrazin-2-yl, 5-(trifluoromethyl)pyrazin-2-yl, 5-(trifluoromethyl)pyrimidin-2-yl, 5-acetylpyrazin-2-yl, 5-bromo-4-(methylsulfanyl)pyrimidin-2-yl, 5-chloropyrazin-2-yl, 5-chloropyrimidin-2-yl, 5-cyano-3-methylpyrazin-2-yl, 5-cyanopyrazin-2-yl, 5-cyanopyridin-2-yl, 5-cyanopyrimidin-2-yl, 5-fluoropyrimidin-2-yl, 5-methoxypyrimidin-2-yl, 5-nitropyridin-2-yl, 6-(trifluoromethyl)pyrazin-2-yl, 6-(trifluoromethyl)pyridazin-3-yl, 6-(trifluoromethyl)pyridin-3-yl, 6-cyanopyrazin-2-yl, 6-fluoroquinoxalin-2-yl, 6-methoxy-3-nitropyridin-2-yl, and quinoxalin-2-yl; and
Z-R² taken together is selected from: (1H-indol-5-yl)methyl, (2,2-dimethylcyclopropyl)methyl, (2,6-difluorophenyl)methyl, (2-cyanophenyl)methyl, (2-fluorophenyl)methyl, (3,4-difluorophenyl)methyl, (3-carbamoylphenyl)methyl, (3-cyano-4-fluorophenyl)methyl, (3-fluorophenyl)methyl, (4-carbamoylphenyl)methyl, (4-cyanophenyl)methyl, (4-fluorophenyl)methyl, (4-methoxyphenyl)methyl, (bicyclo[2.2.1]hept-5-en-2-yl)methyl, (cyclohexyl)methyl, (cyclopropyl)methyl, (oxan-4-yl)methyl, 2,2-dimethylpropyl, 2-methylpropyl, 3,3-dimethylbutyl, 4,4,4-trifluorobutyl, and benzyl.

In some embodiments, X is O.

In some embodiments, X is NH.

In some embodiments, Y is CH₂.

In some embodiments, Y is absent.

In some embodiments, Z-R² is benzyl.

In some embodiments, the stereochemistry in Formula (Ili) for the C(2) carbon is (*R*) and the C(6) carbon is (*R*).

In some embodiments, the stereochemistry in Formula (Ili) for the C(2) carbon is (*S*) and the C(6) carbon is (*S*).

In some embodiments, the stereochemistry in Formula (Ili) for the C(2) carbon is (*R*) and the C(6) carbon is (*S*).

In some embodiments, X is O and Z-R² is benzyl.

In some embodiments, X is NH and Z-R² is benzyl.

In some embodiments, X is O, Z-R² is benzyl, and stereochemistry for the C(2) carbon is (*R*) and the C(6) carbon is (*R*).

In some embodiments, X is NH, Z-R² is benzyl, and stereochemistry for the C(2) carbon is (*R*) and the C(6) carbon is (*R*).

In some embodiments, X is O, Z-R² is benzyl, and stereochemistry for the C(2) carbon is (*R*) and the C(6) carbon is (*S*).

In some embodiments, X is NH, Z-R² is benzyl, and stereochemistry for the C(2) carbon is (*R*) and the C(6) carbon is (*S*).

In some embodiments, X is O, Z-R² is benzyl, and stereochemistry for the C(2) carbon is (*S*) and the C(6) carbon is (*S*).

In some embodiments, X is NH, Z-R² is benzyl, and stereochemistry for the C(2) carbon is (*S*) and the C(6) carbon is (*S*).

One aspect of the present invention pertains to compounds of Formula (**IIg**): 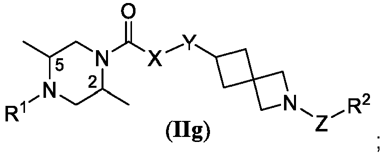 or a pharmaceutically acceptable salt thereof:
wherein:
X is O or NH;
Y is CH₂ or absent;
Z is CH₂ or absent;
R¹ is C₆-C₁₀ aryl or 5-10 membered heteroaryl, each optionally substituted with one or more groups selected from: C₁-C₆ alkoxy, C₁-C₆ alkoxycarbonyl, C₁-C₆ alkyl, C₁-C₆ alkylcarbonyl, C₁-C₆ alkylsulfanyl, C₁-C₆ haloalkoxy, C₁-C₆ haloalkyl, cyano, halogen, and nitro; and
R² is selected from: C₆-C₁₀ aryl, C₁-C₆ alkyl, C₃-C₇ cycloalkyl, C₆-C₈ bicycloalkenyl, 5-10 membered heteroaryl, and heterocyclyl, each optionally substituted with one or more groups selected from: C₁-C₄ alkyl, C₁-C₆ alkoxy, carbamoyl, cyano, and halogen.

In some embodiments, X is O.

In some embodiments, X is NH.

In some embodiments, Y is CH₂.

In some embodiments, Y is absent.

In some embodiments, R¹ is 6-membered heteroaryl.

In some embodiments, R¹ is phenyl, pyridinyl, pyrazinyl, or pyrimidinyl.

In some embodiments, Z is CH₂.

In some embodiments, Z is absent.

In some embodiments, R² is C₆-C₁₀ aryl. In a further embodiment, R² is phenyl.

In some embodiments, the stereochemistry in Formula (**IIg**) for the C(2) carbon is (*R*) and the C(5) carbon is (*S*).

In some embodiments, the stereochemistry in Formula (**IIg**) for the C(2) carbon is (*R*) and the C(5) carbon is (*R*).

In some embodiments, the stereochemistry in Formula (**IIg**) for the C(2) carbon is (*S*) and the C(5) carbon is (*R*).

In some embodiments, the stereochemistry in Formula (**IIg**) for the C(2) carbon is (*S*) and the C(5) carbon is (*S*).

In some embodiments, R¹ is 6-membered heteroaryl and R² is C₆-C₁₀ aryl.

In some embodiments, R¹ is phenyl. pyridinyl, pyrazinyl, or pyrimidinyl, and R² is C₆-C₁₀ aryl.

In some embodiments, R¹ is 6-membered heteroaryl and X is O.

In some embodiments, R¹ is phenyl, pyridyl, pyrazinyl, or pyrimidinyl, X is O. In a further embodiment, R² is C₆-C₁₀ aryl.

In some embodiments, R¹ is 6-membered heteroaryl and X is NH.

In some embodiments, R¹ is phenyl, pyridyl, pyrazinyl, or pyrimidinyl, X is NH. In a further embodiment, R² is C₆-C₁₀ aryl.

In some embodiments, R¹ is 6-membered heteroaryl, X is O, Z is CH₂, and R² is C₆-C₁₀ aryl. In a further embodiment, R² is phenyl.

In some embodiments, R¹ is phenyl, pyridyl, pyrazinyl, or pyrimidinyl, X is O, Z is CH₂, and R² is C₆-C₁₀ aryl. In a further embodiment, R² is phenyl.

In some embodiments, R¹ is 6-membered heteroaryl, X is NH, Z is CH₂, and R² is C₆-C₁₀ aryl. In a further embodiment, R² is phenyl.

In some embodiments, R¹ is phenyl, pyridinyl, pyrazinyl, or pyrimidinyl, X is NH, Z is CH₂, and R² is C₆-C₁₀ aryl. In a further embodiment, R² is phenyl.

In some embodiments, R¹ is phenyl, pyridinyl, pyrazinyl, or pyrimidinyl, X is O, Z is CH₂, R² is C₆-C₁₀ aryl, and stereochemistry for the C(2) carbon is (*R*) and the C(5) carbon is (*R*)*.* In a further embodiment, R² is phenyl.

In some embodiments, R¹ is phenyl, pyridinyl, pyrazinyl or pyrimidinyl, X is NH, Z is CH₂, R² is C₆-C₁₀ aryl, and stereochemistry for the C(2) carbon is (*R*) and the C(5) carbon is (*R*)*.* In a further embodiment, R² is phenyl.

In some embodiments, R¹ is phenyl, pyridinyl, pyrazinyl, or pyrimidinyl, X is O, Z is CH₂, R² is C₆-C₁₀ aryl, and stereochemistry for the C(2) carbon is (*R*) and the C(5) carbon is (*S*)*.* In a further embodiment, R² is phenyl.

In some embodiments, R¹ is phenyl, pyridinyl, pyrazinyl, or pyrimidinyl, X is NH, Z is CH₂, R² is C₆-C₁₀ aryl, and stereochemistry for the C(2) carbon is (*R*) and the C(5) carbon is (*S*)*.* In a further embodiment, R² is phenyl.

In some embodiments, R¹ is phenyl, pyridinyl, pyrazinyl, or pyrimidinyl, X is O, Z is CH₂, R² is C₆-C₁₀ aryl, and stereochemistry for the C(2) carbon is (*S*) and the C(5) carbon is (*R*)*.* In a further embodiment, R² is phenyl.

In some embodiments, R¹ is phenyl, pyridinyl, pyrazinyl, or pyrimidinyl, X is NH, Z is CH₂, R² is C₆-C₁₀ aryl, and stereochemistry for the C(2) carbon is (*S*) and the C(5) carbon is (*R*)*.* In a further embodiment, R² is phenyl.

In some embodiments, R¹ is phenyl, pyridinyl, pyrazinyl, or pyrimidinyl, X is O, Z is CH₂, R² is C₆-C₁₀ aryl, and stereochemistry for the C(2) carbon is (*S*) and the C(5) carbon is (*S*)*.* In a further embodiment, R² is phenyl.

In some embodiments, R¹ is phenyl, pyridinyl, pyrazinyl, or pyrimidinyl, X is NH, Z is CH₂, R² is C₆-C₁₀ aryl, and stereochemistry for the C(2) carbon is (*S*) and the C(5) carbon is (*S*)*.* In a further embodiment, R² is phenyl.

One aspect of the present invention pertains to compounds of Formula (**IIg**): 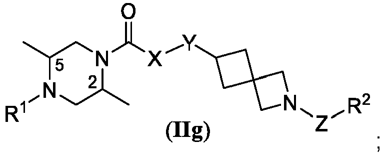 or a pharmaceutically acceptable salt thereof:
wherein:
X is O or NH;
Y is CH₂ or absent;
Z is CH₂ or absent;
R¹ is selected from: phenyl, pyrazinyl, pyridazinyl, pyridinyl, pyrimidinyl, and quinoxalinyl, each optionally substituted with one or more groups selected from: acetyl, bromo, chloro, cyano, difluoromethoxy, difluoromethyl, fluoro, methoxy, methoxycarbonyl, methyl, methylsulfanyl, nitro, trifluoromethoxy, and trifluoromethyl; and
R² is selected from: 1*H*-indolyl, 2,2-dimethylpropyl, 2-methylpropyl, 3,3-dimethylbutyl, bicyclo[2.2.1]heptenyl, butyl, cyclohexyl, cyclopropyl, oxanyl, and phenyl, each optionally substituted with one or more groups selected from: carbamoyl, cyano, fluoro, methoxy, and methyl.

In some embodiments, X is O.

In some embodiments, X is NH.

In some embodiments, Y is CH₂.

In some embodiments, Y is absent.

In some embodiments, R¹ is phenyl, pyridinyl, pyrazinyl, or pyrimidinyl.

In some embodiments, Z is CH₂.

In some embodiments, Z is absent.

In some embodiments, R² is phenyl.

In some embodiments, the stereochemistry in Formula (**IIg**) for the C(2) carbon is (*R*) and the C(5) carbon is (*S*).

In some embodiments, the stereochemistry in Formula (**IIg**) for the C(2) carbon is (*R*) and the C(5) carbon is (*R*).

In some embodiments, the stereochemistry in Formula (**IIg**) for the C(2) carbon is (*S*) and the C(5) carbon is (*R*).

In some embodiments, the stereochemistry in Formula (**IIg**) for the C(2) carbon is (*S*) and the C(5) carbon is (*S*).

In some embodiments, R¹ is phenyl, pyridinyl, pyrazinyl, or pyrimidinyl, and R² is phenyl.

In some embodiments, R¹ is phenyl, pyridinyl, pyrazinyl, or pyrimidinyl, and X is O.

In some embodiments, R¹ is phenyl, pyridinyl, pyrazinyl, or pyrimidinyl, and X is NH.

In some embodiments, R¹ is phenyl, pyridinyl, pyrazinyl, or pyrimidinyl, X is O, and Z is CH₂. In a further embodiment, R² is phenyl.

In some embodiments, R¹ is phenyl, pyridinyl, pyrazinyl, or pyrimidinyl, X is NH, and Z is CH₂. In a further embodiment, R² is phenyl.

In some embodiments, R¹ is phenyl, pyridyl, pyrazinyl, or pyrimidinyl, X is O, Z is CH₂, and stereochemistry for the C(2) carbon is (*R*) and the C(5) carbon is (*R*). In a further embodiment, R² is phenyl.

In some embodiments, R¹ is phenyl, pyridyl, pyrazinyl, or pyrimidinyl, X is NH, Z is CH₂, and stereochemistry for the C(2) carbon is (*R*) and the C(5) carbon is (*R*). In a further embodiment, R² is phenyl.

In some embodiments, R¹ is phenyl, pyridinyl, pyrazinyl, or pyrimidinyl, X is O, Z is CH₂, and stereochemistry for the C(2) carbon is (*R*) and the C(5) carbon is (*S*). In a further embodiment, R² is phenyl.

In some embodiments, R¹ is phenyl, pyridinyl, pyrazinyl, or pyrimidinyl, X is NH, Z is CH₂, and stereochemistry for the C(2) carbon is (*R*) and the C(5) carbon is (*S*). In a further embodiment, R² is phenyl.

In some embodiments, R¹ is phenyl, pyridinyl, pyrazinyl, or pyrimidinyl, X is O, Z is CH₂, and stereochemistry for the C(2) carbon is (*S*) and the C(5) carbon is (*S*). In a further embodiment, R² is phenyl.

In some embodiments, R¹ is phenyl, pyridinyl, pyrazinyl, or pyrimidinyl, X is NH, Z is CH₂, and stereochemistry for the C(2) carbon is (*S*) and the C(5) carbon is (*S*). In a further embodiment, R² is phenyl.

In some embodiments, R¹ is phenyl, pyridinyl, pyrazinyl, or pyrimidinyl, X is O, Z is CH₂, and stereochemistry for the C(2) carbon is (*S*) and the C(5) carbon is (*R*). In a further embodiment, R² is phenyl.

In some embodiments, R¹ is phenyl, pyridinyl, pyrazinyl, or pyrimidinyl, X is NH, Z is CH₂, and stereochemistry for the C(2) carbon is (*S*) and the C(5) carbon is (*R*). In a further embodiment, R² is phenyl.

One aspect of the present invention pertains to compounds of Formula (**IIg**): 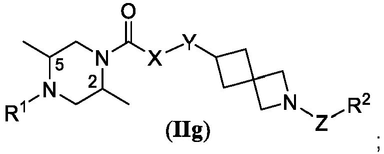 or a pharmaceutically acceptable salt thereof:
wherein:
X is O or NH;
Y is CH₂ or absent;
R¹ is selected from: 3-(trifluoromethyl)pyridin-2-yl, 3,4,5-trifluorophenyl, 4-(trifluoromethyl)phenyl, 4-cyano-6-(trifluoromethyl)pyridin-3-yl, 4-methylpyrimidin-2-yl, 5-(difluoromethoxy)pyrimidin-2-yl, 5-(difluoromethyl)pyrazin-2-yl, 5-(methoxycarbonyl)-4-(trifluoromethyl)pyrimidin-2-yl, 5-(trifluoromethoxy)pyrazin-2-yl, 5-(trifluoromethyl)pyrazin-2-yl, 5-(trifluoromethyl)pyrimidin-2-yl, 5-acetylpyrazin-2-yl, 5-bromo-4-(methylsulfanyl)pyrimidin-2-yl, 5-chloropyrazin-2-yl, 5-chloropyrimidin-2-yl, 5-cyano-3-methylpyrazin-2-yl, 5-cyanopyrazin-2-yl, 5-cyanopyridin-2-yl, 5-cyanopyrimidin-2-yl, 5-fluoropyrimidin-2-yl, 5-methoxypyrimidin-2-yl, 5-nitropyridin-2-yl, 6-(trifluoromethyl)pyrazin-2-yl, 6-(trifluoromethyl)pyridazin-3-yl, 6-(trifluoromethyl)pyridin-3-yl, 6-cyanopyrazin-2-yl, 6-fluoroquinoxalin-2-yl, 6-methoxy-3-nitropyridin-2-yl, and quinoxalin-2-yl; and
Z-R² taken together is selected from: (1*H*-indol-5-yl)methyl, (2,2-dimethylcyclopropyl)methyl, (2,6-difluorophenyl)methyl, (2-cyanophenyl)methyl, (2-fluorophenyl)methyl, (3,4-difluorophenyl)methyl, (3-carbamoylphenyl)methyl, (3-cyano-4-fluorophenyl)methyl, (3-fluorophenyl)methyl, (4-carbamoylphenyl)methyl, (4-cyanophenyl)methyl, (4-fluorophenyl)methyl, (4-methoxyphenyl)methyl, (bicyclo[2.2.1]hept-5-en-2-yl)methyl, (cyclohexyl)methyl, (cyclopropyl)methyl, (oxan-4-yl)methyl, 2,2-dimethylpropyl, 2-methylpropyl, 3,3-dimethylbutyl, 4,4,4-trifluorobutyl, and benzyl.

In some embodiments, X is O.

In some embodiments, X is NH.

In some embodiments, Y is CH₂.

In some embodiments, Y is absent.

In some embodiments, Z-R² is benzyl.

In some embodiments, the stereochemistry in Formula (**IIg**) for the C(2) carbon is (*R*) and the C(5) carbon is (*S*).

In some embodiments, the stereochemistry in Formula (**IIg**) for the C(2) carbon is (*R*) and the C(5) carbon is (*R*).

In some embodiments, the stereochemistry in Formula (**IIg**) for the C(2) carbon is (*S*) and the C(5) carbon is (*R*).

In some embodiments, the stereochemistry in Formula (**IIg**) for the C(2) carbon is (*S*) and the C(5) carbon is (*S*).

In some embodiments, X is O and Z-R² is benzyl.

In some embodiments, X is NH and Z-R² is benzyl.

In some embodiments, X is O, Z-R² is benzyl, and stereochemistry for the C(2) carbon is (*R*) and the C(5) carbon is (*R*).

In some embodiments, X is NH, Z-R² is benzyl, and stereochemistry for the C(2) carbon is (*R*) and the C(5) carbon is (*R*).

In some embodiments, X is O, Z-R² is benzyl, and stereochemistry for the C(2) carbon is (*R*) and the C(5) carbon is (*S*).

In some embodiments, X is NH, Z-R² is benzyl, and stereochemistry for the C(2) carbon is (*R*) and the C(5) carbon is (*S*).

In some embodiments, X is O, Z-R² is benzyl, and stereochemistry for the C(2) carbon is (*S*) and the C(5) carbon is (*R*).

In some embodiments, X is NH, Z-R² is benzyl, and stereochemistry for the C(2) carbon is (*S*) and the C(5) carbon is (*R*).

In some embodiments, X is O, Z-R² is benzyl, and stereochemistry for the C(2) carbon is (*S*) and the C(5) carbon is (*S*).

In some embodiments, X is NH, Z-R² is benzyl, and stereochemistry for the C(2) carbon is (*S*) and the C(5) carbon is (*S*).

Some embodiments of the present invention include every combination of one or more compounds and pharmaceutically acceptable salts thereof selected from the following compounds in **TABLE A,** wherein the Compound Number and the Chemical Name associated with each compound is used elsewhere in this disclosure. **TABLE A** provide certain compounds of the present invention.

**TABLE A**

| Cmpd . No. | Chemical Structure | Chemical Name |
|---|---|---|
| 1 | 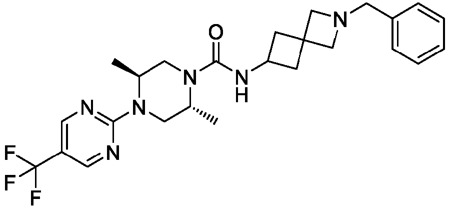 | (2*R*,5*S*)-*N-*{2-benzyl-2-azaspiro[3.3]heptan-6-yl}-2,5-dimethyl-4-[5-(trifluoromethyl)pyrimidin-2-yl]piperazine-1-carboxamide |
| 2 | 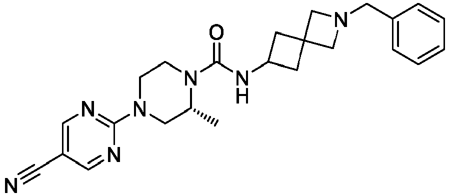 | (2*R*)-*N*-{2-benzyl-2-azaspiro[3.3]heptan-6-yl}-4-(5-cyanopyrimidi n-2-yl)-2-methylpiperazine-1-carboxamide |
| 3 | 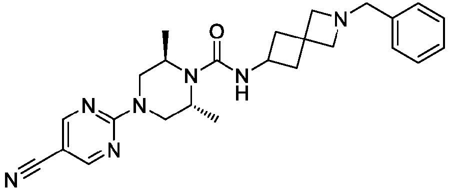 | (2*R*,6*R*)-*N*-{2-benzyl-2-azaspiro[3.3]heptan-6-yl}-4-(5-cyanopyrimidi n-2-yl)-2,6-dimethylpiperazine-1-carboxamide |
| 4 | 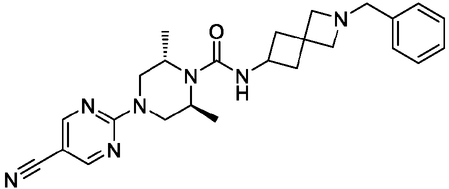 | (2*S*,6*S*)-*N*-{2-benzyl-2-azaspiro[3.3]heptan-6-yl}-4-(5-cyanopyrimidi n-2-yl)-2,6-dimethylpiperazine-1-carboxamide |
| 5 | 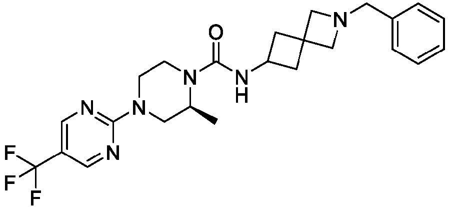 | (2*S*)-*N*-{2-benzyl-2-azaspiro[3.3]heptan-6-yl}-2-methyl-4-[5-(trifluoromethyl)pyrimidin-2-yl]piperazine-1-carboxamide |
| 6 | 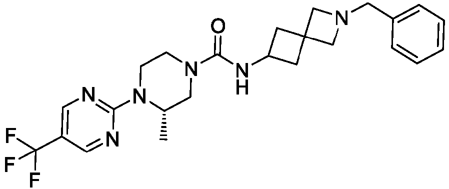 | (3*S*)-*N*-{2-benzyl-2-azaspiro[3.3]heptan-6-yl}-3-methyl-4-[5-(trifluoromethyl)pyrimidin-2-yl]piperazine-1-carboxamide |
| 7 | 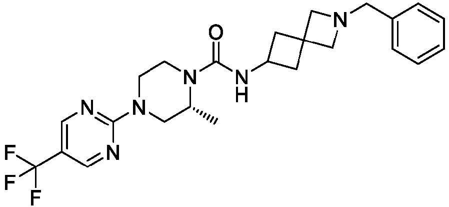 | (2*R*)-*N*-{2-benzyl-2-azaspiro[3.3]heptan-6-yl}-2-methyl-4-[5-(trifluoromethyl)pyrimidin-2-yl]piperazine-1-carboxamide |
| 8 | 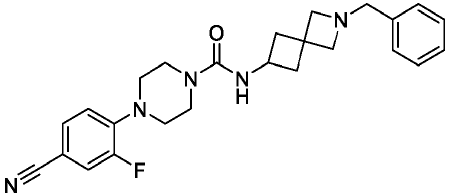 | *N*-{2-benzyl-2-azaspiro[3.3]heptan-6-yl}-4-(4-cyano-2-fluorophenyl)piperazine-1-carboxamide |
| 9 | 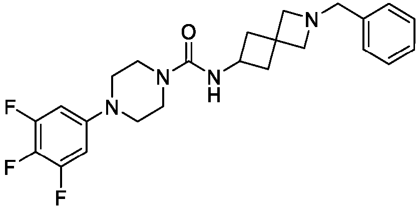 | *N*-{2-benzyl-2-azaspiro[3.3]heptan-6-yl}-4-(3,4,5-trifluorophenyl)piperazine-1-carboxamide |
| 10 | 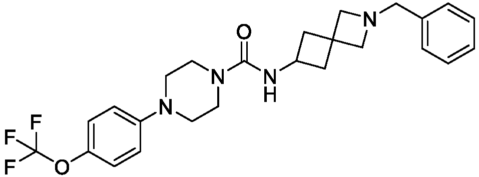 | *N*-{2-benzyl-2-azaspiro[3.3]heptan-6-yl}-4-[4-(trifluoromethoxy)phenyl]pip erazine-1-carboxamide |
| 11 | 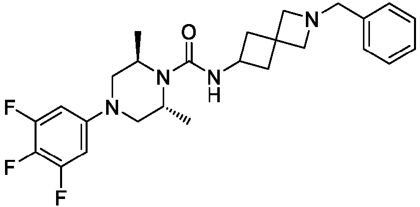 | (2*R*,6*R*)-*N*-{2-benzyl-2-azaspiro[3.3]heptan-6-yl}-2,6-dimethyl-4-(3,4,5-trifluorophenyl)piperazine-1-carboxamide |
| 12 | 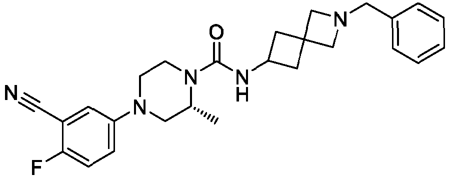 | (2*R*)-*N*-{2-benzyl-2-azaspiro[3.3]heptan-6-yl}-4-(3-cyano-4-fluorophenyl)-2-methylpiperazine-1-carboxamide |
| 13 | 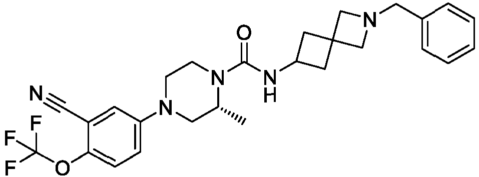 | (2*R*)-*N*-{2-benzyl-2-azaspiro[3.3]heptan-6-yl}-4-[3-cyano-4-(trifluoromethoxy)phenyl]-2-methylpiperazine-1-carboxamide |
| 14 | 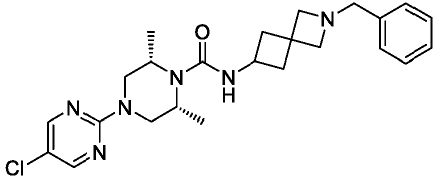 | (2*R*,6*S*)-*N*-{2-benzyl-2-azaspiro[3.3]heptan-6-yl}-4-(5-chloropyrimidin-2-yl)-2,6-dimethylpiperazine-1-carboxamide |
| 15 | 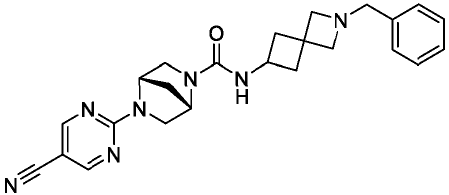 | (1*S*,4*S*)-*N*-{2-benzyl-2-azaspiro[3.3]heptan-6-yl}-5-(5-cyanopyrimidin-2-yl)-2,5-diazabicyclo[2.2.1]heptane-2-carboxamide |
| 16 | 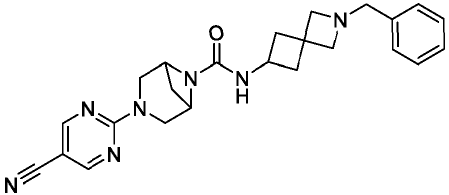 | *N*-{2-benzyl-2-azaspiro[3.3]heptan-6-yl}-3-(5-cyanopyrimidin-2-yl)-3,6-diazabicyclo[3.1.1]heptane-6-carboxamide |
| 17 | 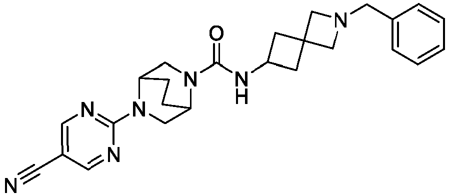 | *N*-{2-benzyl-2-azaspiro[3.3]heptan-6-yl}-5-(5-cyanopyrimidi n-2-yl)-2,5-diazabicyclo[2.2.2]octane-2-carboxamide |
| 18 | 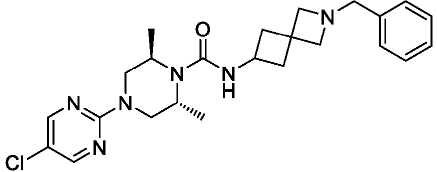 | (2*R*,6*R*)-*N*-{2-benzyl-2-azaspiro[3.3]heptan-6-yl}-4-(5-chloropyrimidin-2-yl)-2,6-dimethylpiperazine-1-carboxamide |
| 19 | 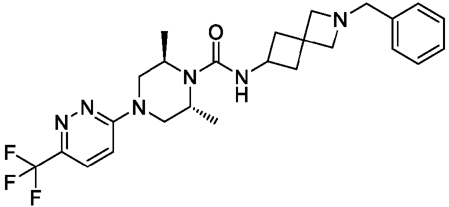 | (2*R*,6*R*)-*N*-{2-benzyl-2-azaspiro[3.3]heptan-6-yl}-2,6-dimethyl-4-[6-(trifluoromethyl)pyridazin-3-yl]piperazine-1-carboxamide |
| 20 | 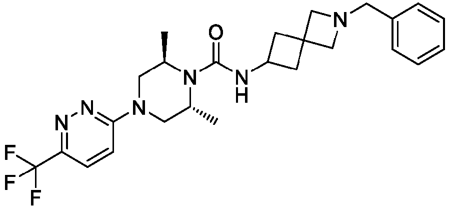 | (2*R*,6*R*)-*N-*{2-benzyl-2-azaspiro[3.3]heptan-6-yl}-4-(5-cyanopyrazin-2-yl)-2,6-dimethylpiperazine-1-carboxamide |
| 21 | 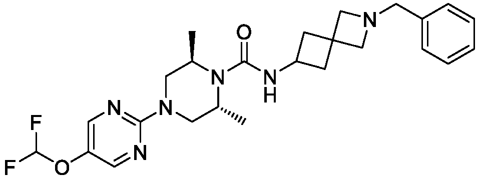 | (2*R*,6*R*)-*N*-{2-benzyl-2-azaspiro[3.3]heptan-6-yl}-4-[5-(difluoromethoxy)pyrimidin-2-yl]-2,6-dimethylpiperazine-1-carboxamide |
| 22 | 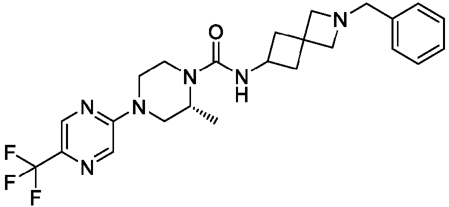 | (2*R*)-*N*-{2-benzyl-2-azaspiro[3.3]heptan-6-yl}-2-methyl-4-[5-(trifluoromethyl) pyrazin-2-yl]piperazine-1-carboxamide |
| 23 | 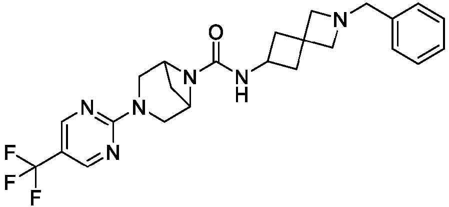 | *N*-{2-benzyl-2-azaspiro[3.3]heptan-6-yl}-3-[5-(trifluoromethyl)pyrimidin-2-yl]-3,6-diazabicyclo[3.1.1]heptane-6-carboxamide |
| 24 | 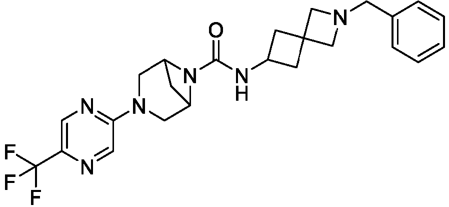 | *N*-{2-benzyl-2-azaspiro[3.3]heptan-6-yl}-3-[5-(trifluoromethyl)pyrazin-2-yl]-3,6-diazabicyclo[3.1.1]heptane-6-carboxamide |
| 25 | 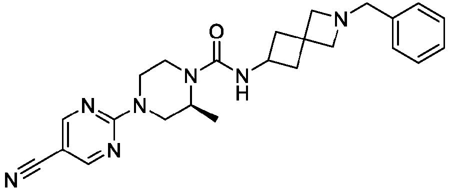 | (2*S*)-*N-*{2-benzyl-2-azaspiro[3.3]heptan-6-yl}-4-(5-cyanopyrimidi n-2-yl)-2-methylpiperazine-1-carboxamide |
| 26 | 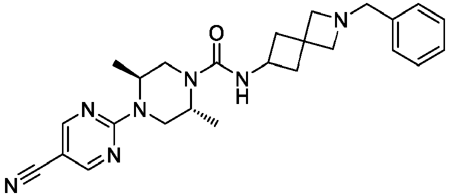 | (2*R*,5*S*)-*N*-{2-benzyl-2-azaspiro[3.3]heptan-6-yl}-4-(5-cyanopyrimidi n-2-yl)-2,5-dimethylpiperazine-1-carboxamide |
| 27 | 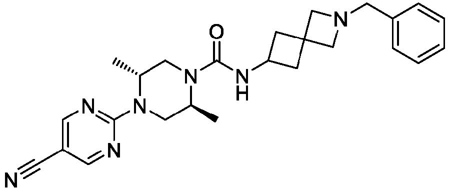 | (2*S*,5*R*)-*N*-{2-benzyl-2-azaspiro[3.3]heptan-6-yl}-4-(5-cyanopyrimidi n-2-yl)-2,5-dimethylpiperazine-1-carboxamide |
| 28 | 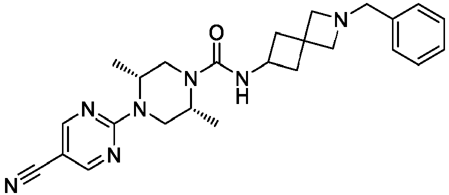 | (2*R*,5*R*)-*N*-{2-benzyl-2-azaspiro[3.3]heptan-6-yl}-4-(5-cyanopyrimidi n-2-yl)-2,5-dimethylpiperazine-1-carboxamide |
| 29 | 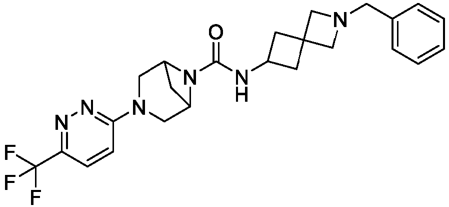 | *N*-{2-benzyl-2-azaspiro[3.3]heptan-6-yl}-3-[6-(trifluoromethyl)pyridazin-3-yl]-3,6-diazabicyclo[3.1.1]heptane-6-carboxamide |
| 30 | 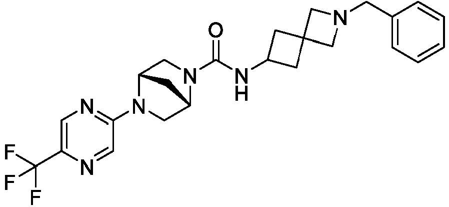 | (1*S*,4*S*)-*N*-{2-benzyl-2-azaspiro[3.3]heptan-6-yl}-5-[5-(trifluoromethyl)pyrazin-2-yl]-2,5-diazabicyclo[2.2.1]heptane-2-carboxamide |
| 31 | 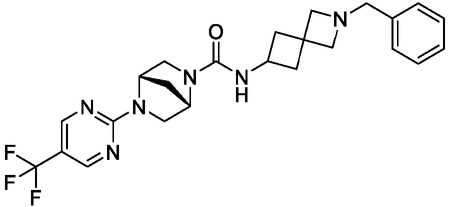 | (1*S*,4*S*)-*N*-{2-benzyl-2-azaspiro[3.3]heptan-6-yl}-5-[5-(trifluoromethyl)pyrimidin-2-yl]-2,5-diazabicyclo[2.2.1]heptane-2-carboxamide |
| 32 | 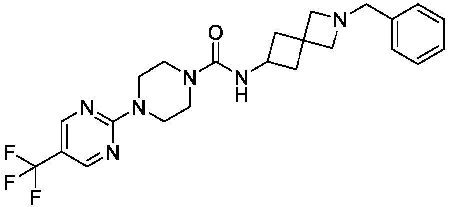 | *N*-{2-benzyl-2-azaspiro[3.3]heptan-6-yl}-4-[5-(trifluoromethyl)pyrimidin-2-yl]piperazine-1-carboxamide |
| 33 | 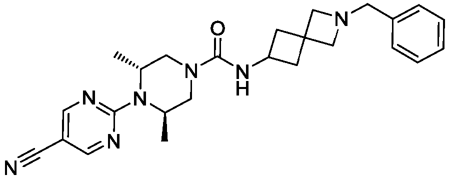 | (3*R*,5*R*)-*N*-{2-benzyl-2-az*as*piro[3.3]heptan-6-yl}-4-(5-cyanopyrimidin-2-yl)-3,5-dimethylpiperazine-1-carboxamide |
| 34 | 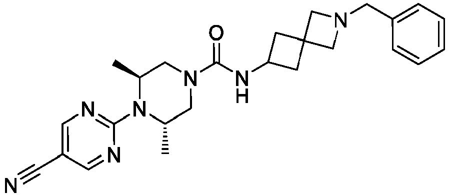 | (3*S*,5*S*)-*N*-{2-benzyl-2-azaspiro[3.3]heptan-6-yl}-4-(5-cyanopyrimidin-2-yl)-3,5-dimethylpiperazine-1-carboxamide |
| 35 | 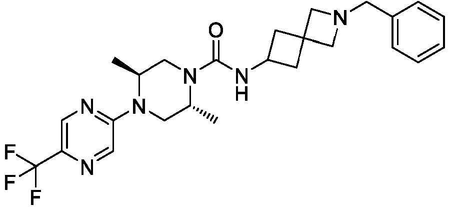 | (2*R*,5*S*)-*N*-{2-benzyl-2-azaspiro[3.3]heptan-6-yl}-2,5-dimethyl-4-[5-(trifluoromethyl)pyrazin-2-yl]piperazine-1-carboxamide |
| 36 | 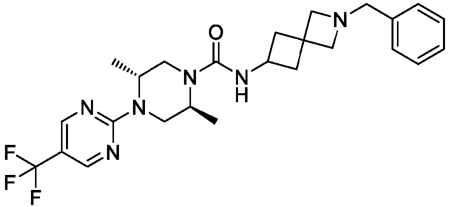 | (2*S*,5*R*)-*N*-{2-benzyl-2-azaspiro[3.3]heptan-6-yl}-2,5-dimethyl-4-[5-(trifluoromethyl)pyrimidin-2-yl]piperazine-1-carboxamide |
| 37 | 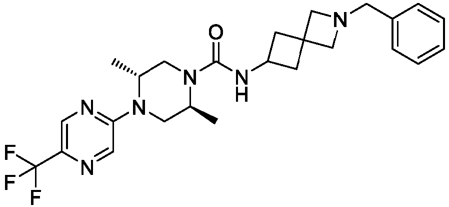 | (2*S*,5*R*)-*N*-{2-benzyl-2-azaspiro[3.3]heptan-6-yl}-2,5-dimethyl-4-[5-(trifluoromethyl)pyrazin-2-yl]piperazine-1-carboxamide |
| 38 | 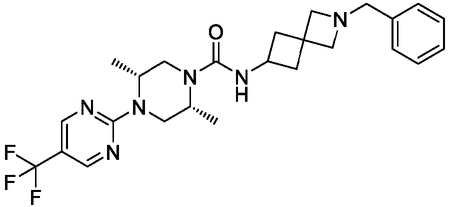 | (2*R*,5*R*)-*N*-{2-benzyl-2-azaspiro[3.3]heptan-6-yl}-2,5-dimethyl-4-[5-(trifluoromethyl)pyrimidin-2-yl]piperazine-1-carboxamide |
| 39 | 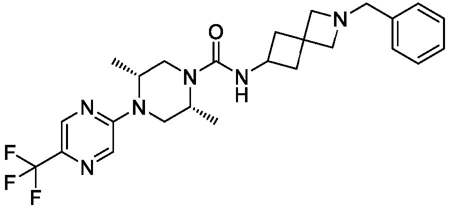 | (2*R*,5*R*)-*N*-{2-benzyl-2-azaspiro[3.3]heptan-6-yl}-2,5-dimethyl-4-[5-(trifluoromethyl)pyrazin-2-yl]piperazine-1-carboxamide |
| 40 | 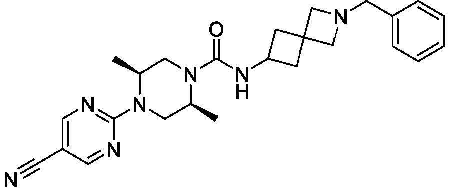 | (2*S*,5*S*)-*N*-{2-benzyl-2-azaspiro[3.3]heptan-6-yl}-4-(5-cyanopyrimidi n-2-yl)-2,5-dimethylpiperazine-1-carboxamide |
| 41 | 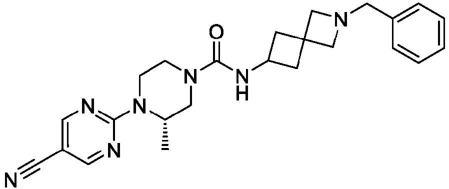 | (3*S*)-*N*-{2-benzyl-2-azaspiro[3.3]heptan-6-yl}-4-(5-cyanopyrimidi n-2-yl)-3-methylpiperazine-1-carboxamide |
| 42 | 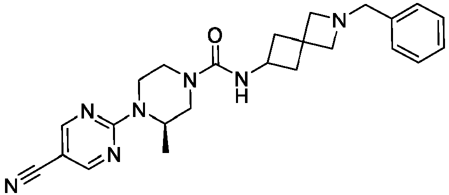 | (3*R*)-*N*-{2-benzyl-2-azaspiro[3.3]heptan-6-yl}-4-(5-cyanopyrimidi n-2-yl)-3-methylpiperazine-1-carboxamide |
| 43 | 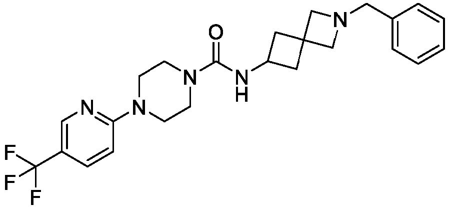 | *N*-{2-benzyl-2-azaspiro[3.3]heptan-6-yl}-4-[5-(trifluoromethyl)pyridin-2-yl]piperazine-1-carboxamide |
| 44 | 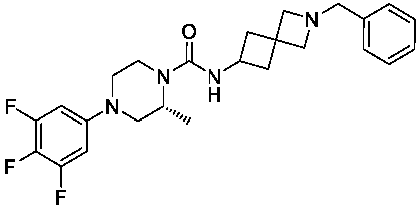 | (2*R*)-*N*-{2-benzyl-2-azaspiro[3.3]heptan-6-yl}-2-methyl-4-(3,4,5-trifluorophenyl)piperazine-1-carboxamide |
| 45 | 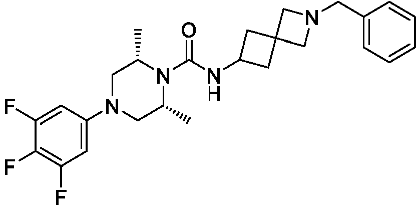 | (2*R*,6*S*)-*N*-{2-benzyl-2-azaspiro[3.3]heptan-6-yl}-2,6-dimethyl-4-(3,4,5-trifluorophenyl)piperazine-1-carboxamide |
| 46 | 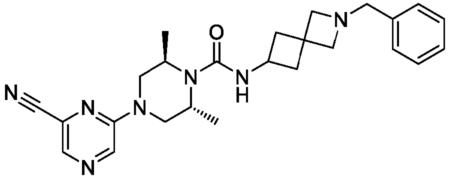 | (2*R*,6*R*)-*N*-{2-benzyl-2-azaspiro[3.3]heptan-6-yl}-4-(6-cyanopyrazin-2-yl)-2,6-dimethylpiperazine-1-carboxamide |
| 47 | 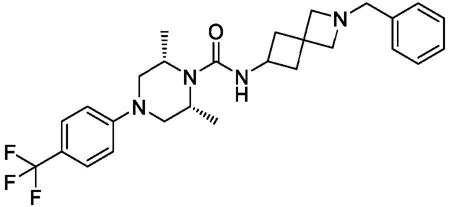 | (2*R*,6*S*)-*N*-{2-benzyl-2-azaspiro[3.3]heptan-6-yl}-2,6-dimethyl-4-[4-(trifluoromethyl)phenyl]piper azine-1-carboxamide |
| 48 | 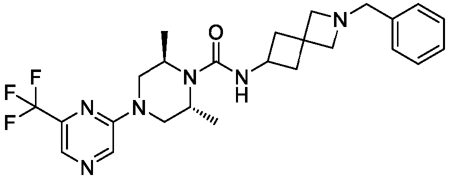 | (2*R*,6*R*)-*N*-{2-benzyl-2-azaspiro[3.3]heptan-6-yl}-2,6-dimethyl-4-[6-(trifluoromethyl)pyrazin-2-yl]piperazine-1-carboxamide |
| 49 | 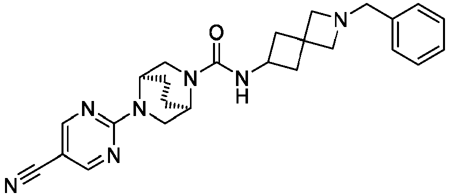 | (1*R*,4*R*)-*N*-{2-benzyl-2-azaspiro[3.3]heptan-6-yl}-5-(5-cyanopyrimidin-2-yl)-2,5-diazabicyclo[2.2.2]octane-2-carboxamide |
| 50 | 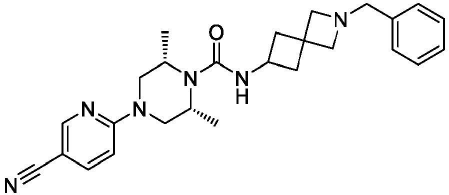 | (2*R*,6*S*)-*N*-{2-benzyl-2-azaspiro[3.3]heptan-6-yl}-4-(5-cyanopyridin-2-yl)-2,6-dimethylpiperazine-1-carboxamide |
| 51 | 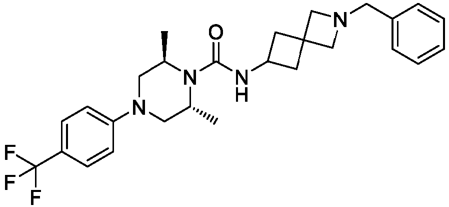 | (2*R*,6*R*)-*N*-{2-benzyl-2-azaspiro[3.3]heptan-6-yl}-2,6-dimethyl-4-[4-(trifluoromethyl)phenyl]piper azine-1-carboxamide |
| 52 | 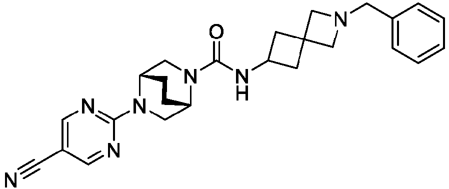 | (1*S*,4*S*)-*N*-{2-benzyl-2-azaspiro[3.3]heptan-6-yl}-5-(5-cyanopyrimidin-2-yl)-2,5-diazabicyclo[2.2.2]octane-2-carboxamide |
| 53 | 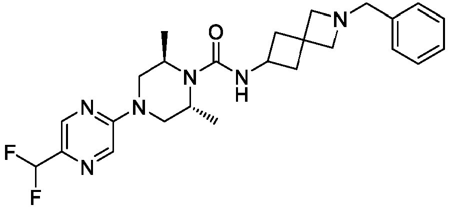 | (2*R*,6*R*)-*N*-{2-benzyl-2-azaspiro[3.3]heptan-6-yl}-4-[5-(difluoromethyl)pyrazin-2-yl]-2,6-dimethylpiperazine-1-carboxamide |
| 54 | 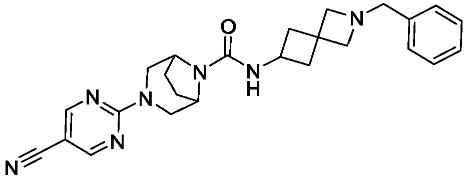 | *N*-{2-benzyl-2-azaspiro[3.3]heptan-6-yl}-3-(5-cyanopyrimidin-2-yl)-3,8-diazabicyclo[3.2.1]octane-8-carboxamide |
| 55 | 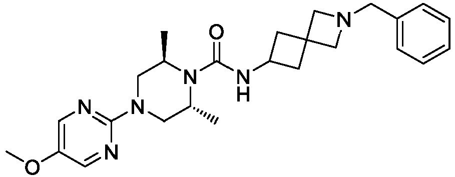 | (2*R*,6*R*)-*N*-{2-benzyl-2-azaspiro[3.3]heptan-6-yl}-4-(5-methoxypyrimidin-2-yl)-2,6-dimethylpiperazine-1-carboxamide |
| 56 | 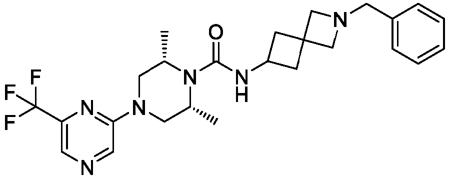 | (2*R*,6*S*)-*N*-{2-benzyl-2-azaspiro[3.3]heptan-6-yl}-2,6-dimethyl-4-[6-(trifluoromethyl)pyrazin-2-yl]piperazine-1-carboxamide |
| 57 | 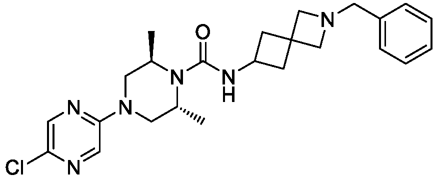 | (2*R*,6*R*)-*N*-{2-benzyl-2-azaspiro[3.3]heptan-6-yl}-4-(5-chloropyrazin-2-yl)-2,6-dimethylpiperazine-1-carboxamide |
| 58 | 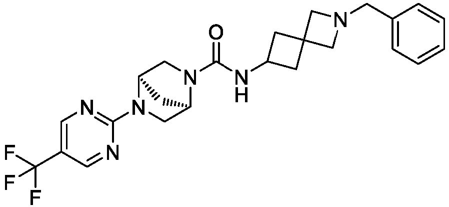 | (1*R*,4*R*)-*N*-{2-benzyl-2-azaspiro[3.3]heptan-6-yl}-5-[5-(trifluoromethyl)pyrimidin-2-yl]-2,5-diazabicyclo[2.2.1]heptane-2-carboxamide |
| 59 | 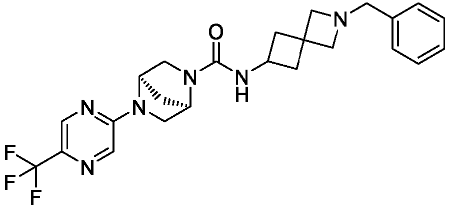 | (1*R*,4*R*)-*N*-{2-benzyl-2-azaspiro[3.3]heptan-6-yl}-5-[5-(trifluoromethyl)pyrazin-2-yl]-2,5-diazabicyclo[2.2.1]heptane-2-carboxamide |
| 60 | 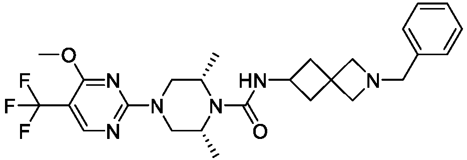 | (2R,6S)-N-{2-benzyl-2-azaspiro[3.3]heptan-6-yl}-4-[4-methoxy-5-(trifluoromethyl)pyrimidin-2-yl]-2,6-dimethylpiperazine-1-carboxamide |
| 61 | 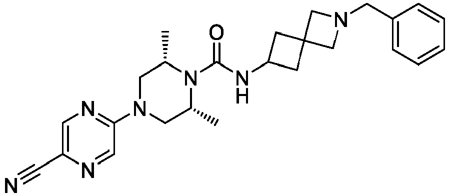 | (2*R*,6*S*)-*N*-{2-benzyl-2-azaspiro[3.3]heptan-6-yl}-4-(5-cyanopyrazin-2-yl)-2,6-dimethylpiperazine-1-carboxamide |
| 62 | 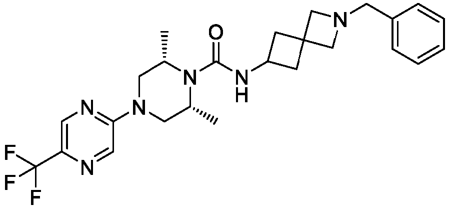 | (2*R*,6*S*)-*N*-{2-benzyl-2-azaspiro[3.3]heptan-6-yl}-2,6-dimethyl-4-[5-(trifluoromethyl)pyrazin-2-yl]piperazine-1-carboxamide |
| 63 | 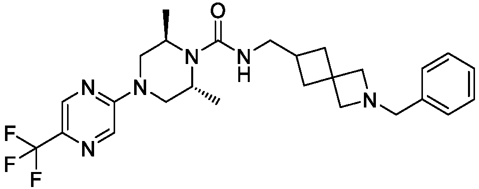 | (2*R*,6*R*)-*N*-({2-benzyl-2-azaspiro[3.3]heptan-6-yl}methyl)-2,6-dimethyl-4-[5-(trifluoromethyl)pyrazin-2-yl]piperazine-1-carboxamide |
| 64 | 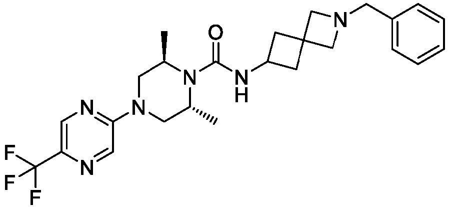 | (2*R*,6*R*)-*N*-{2-benzyl-2-azaspiro[3.3]heptan-6-yl}-2,6-dimethyl-4-[5-(trifluoromethyl)pyrazin-2-yl]piperazine-1-carboxamide |
| 65 | 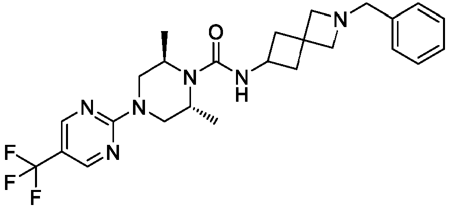 | (2*R*,6*R*)-*N*-{2-benzyl-2-azaspiro[3.3]heptan-6-yl}-2,6-dimethyl-4-[5-(trifluoromethyl)pyrimidin-2-yl]piperazine-1-carboxamide |
| 66 | 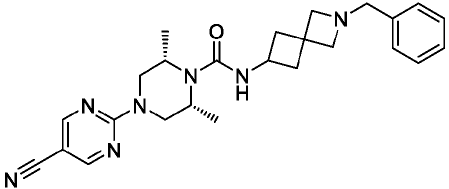 | (2*R*,6*S*)-*N*-{2-benzyl-2-azaspiro[3.3]heptan-6-yl}-4-(5-cyanopyrimidi n-2-yl)-2,6-dimethylpiperazine-1-carboxamide |
| 67 | 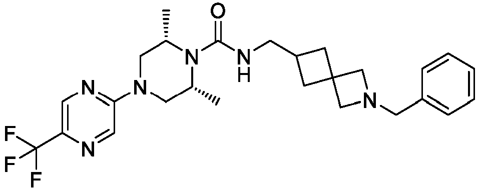 | (2*R*,6*S*)-*N*-({2-benzyl-2-azaspiro[3.3]heptan-6-yl}methyl)-2,6-dimethyl-4-[5-(trifluoromethyl)pyrazin-2-yl]piperazine-1-carboxamide |
| 68 | 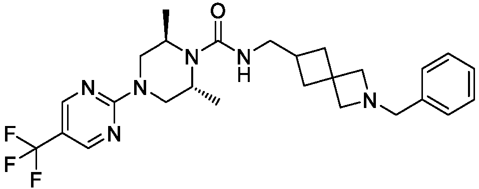 | (2*R*,6*R*)-*N*-({2-benzyl-2-azaspiro[3.3]heptan-6-yl}methyl)-2,6-dimethyl-4-[5-(trifluoromethyl)pyrimidin-2-yl]piperazine-1-carboxamide |
| 69 | 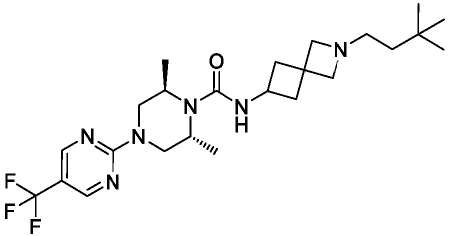 | (2*R*,6*R*)-*N*-[2-(3,3-dimethylbutyl)-2-azaspiro[3.3]heptan-6-yl]-2,6-dimethyl-4-[5-(trifluoromethyl)pyrimidin-2-yl]piperazine-1-carboxamide |
| 70 | 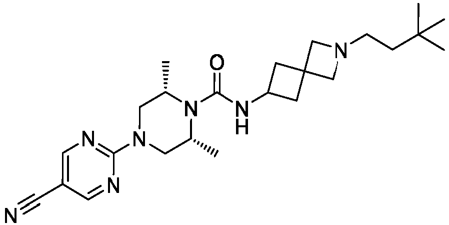 | (2*R*,6*S*)-4-(5-cyanopyrimidin-2-yl)-*N*-[2-(3,3-dimethylbutyl)-2-azaspiro[3.3]heptan-6-yl]-2,6-dimethylpiperazine-1-carboxamide |
| 71 | 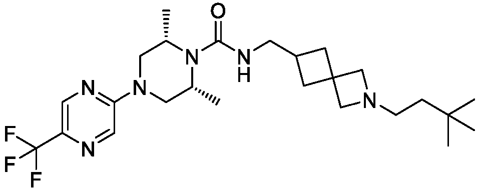 | (2*R*,6*S*)-*N*-{[2-(3,3-dimethylbutyl)-2-azaspiro[3.3]heptan-6-yl]methyl}-2,6-dimethyl-4-[5-(trifluoromethyl)pyrazin-2-yl]piperazine-1-carboxamide |
| 72 | 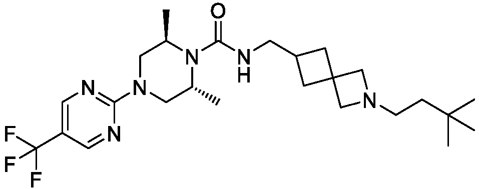 | (2*R*,6*R*)-*N*-{[2-(3,3-dimethylbutyl)-2-azaspiro[3.3]heptan-6-yl]methyl}-2,6-dimethyl-4-[5-(trifluoromethyl)pyrimidin-2-yl]piperazine-1-carboxamide |
| 73 | 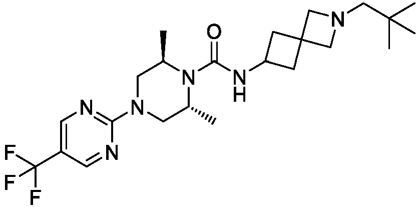 | (2*R,*6*R*)-*N*-[2-(2,2-dimethylpropyl)-2-azaspiro[3.3]heptan-6-yl]-2,6-dimethyl-4-[5-(trifluoromethyl)pyrimidin-2-yl]piperazine-1-carboxamide |
| 74 | 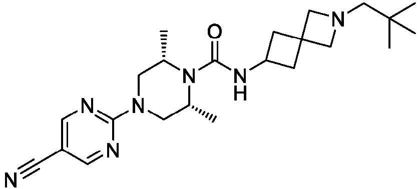 | (2*R*,6*S*)-4-(5-cyanopyrimidin-2-yl)-*N*-[2-(2,2-dimethylpropyl)-2-azaspiro[3.3]heptan-6-yl]-2,6-dimethylpiperazine-1-carboxamide |
| 75 | 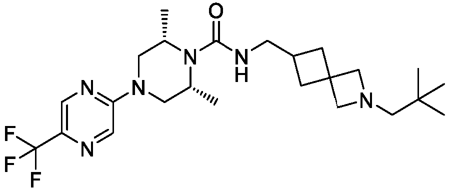 | (2*R*,6*S*)-*N*-{[2-(2,2-dimethylpropyl)-2-azaspiro[3.3]heptan-6-yl]methyl}-2,6-dimethyl-4-[5-(trifluoromethyl)pyrazin-2-yl]piperazine-1-carboxamide |
| 76 | 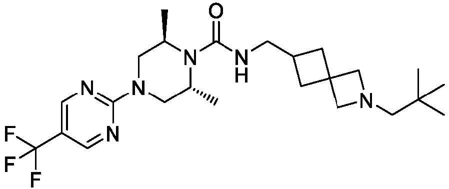 | (2*R*,6*R*)-*N*-{[2-(2,2-dimethylpropyl)-2-azaspiro[3.3]heptan-6-yl]methyl}-2,6-dimethyl-4-[5-(trifluoromethyl)pyrimidin-2-yl]piperazine-1-carboxamide |
| 77 | 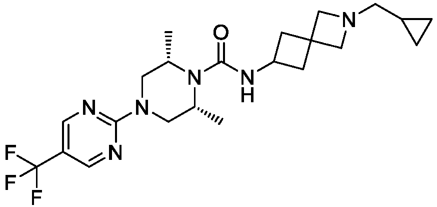 | (2*R*,6*S*)-*N*-[2-(cyclopropylmethyl)-2-azaspiro[3.3]heptan-6-yl]-2,6-dimethyl-4-[5-(trifluoromethyl)pyrimidin-2-yl]piperazine-1-carboxamide |
| 78 | 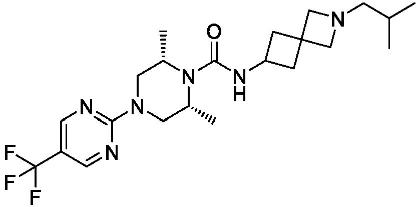 | (2*R*,6*S*)-2,6-dimethyl-*N*-[2-(2-methylpropyl)-2-azaspiro[3.3]heptan-6-yl]-4-[5-(trifluoromethyl)pyrimidin-2-yl]piperazine-1-carboxamide |
| 79 | 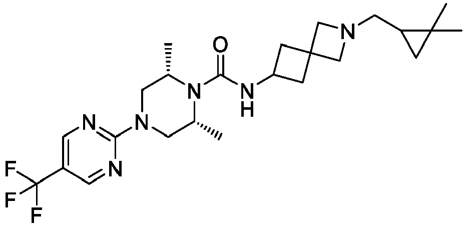 | (2*R*,6*S*)-*N*-{2-[(2,2-dimethylcyclopropyl)methyl]-2-azaspiro[3.3]heptan-6-yl}-2,6-dimethyl-4-[5-(trifluoromethyl)pyrimidin-2-yl]piperazine-1-carboxamide |
| 80 | 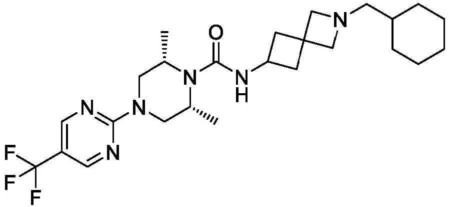 | (2*R*,6*S*)-*N*-[2-(cyclohexylmethyl)-2-azaspiro[3.3]heptan-6-yl]-2,6-dimethyl-4-[5-(trifluoromethyl)pyrimidin-2-yl]piperazine-1-carboxamide |
| 81 | 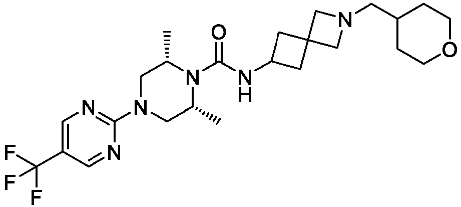 | (2*R*,6*S*)-2,6-dimethyl-*N*-[2-(oxan-4-ylmethyl)-2-azaspiro[3.3]heptan-6-yl]-4-[5-(trifluoromethyl)pyrimidin-2-yl]piperazine-1-carboxamide |
| 82 | 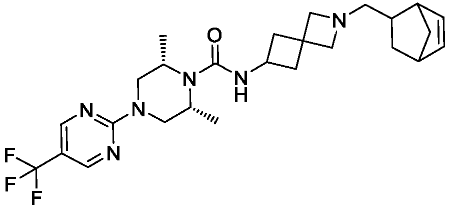 | (2*R*,6*S*)-*N*-(2-{bicyclo[2.2.1]hept-5-en-2-ylmethyl}-2-azaspiro[3.3]heptan-6-yl)-2,6-dimethyl-4-[5-(trifluoromethyl)pyrimidin-2-yl]piperazine-1-carboxamide |
| 83 | 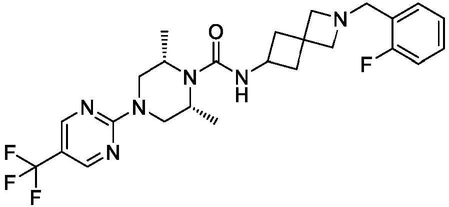 | (2*R*,6*S*)-*N*-{2-[(2-fluorophenyl)methyl]-2-azaspiro[3.3]heptan-6-yl}-2,6-dimethyl-4-[5-(trifluoromethyl)pyrimidin-2-yl]piperazine-1-carboxamide |
| 84 | 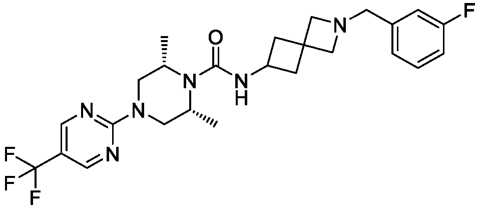 | (2*R*,6*S*)-*N*-{2-[(3-fluorophenyl)methyl]-2-azaspiro[3.3]heptan-6-yl}-2,6-dimethyl-4-[5-(trifluoromethyl)pyrimidin-2-yl]piperazine-1-carboxamide |
| 85 | 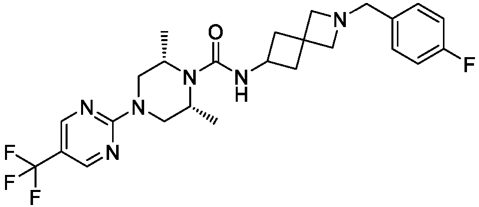 | (2*R*,6*S*)-*N*-{2-[(4-fluorophenyl)methyl]-2-azaspiro[3.3]heptan-6-yl}-2,6-dimethyl-4-[5-(trifluoromethyl)pyrimidin-2-yl]piperazine-1-carboxamide |
| 86 | 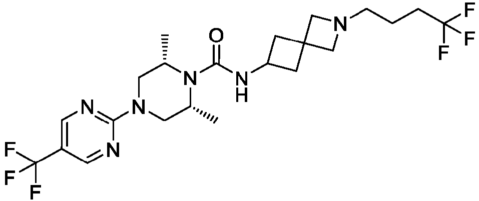 | (2*R*,6*S*)-2,6-dimethyl-*N*-[2-(4,4,4-trifluorobutyl)-2-azaspiro[3.3]heptan-6-yl]-4-[5-(trifluoromethyl)pyrimidin-2-yl]piperazine-1-carboxamide |
| 87 | 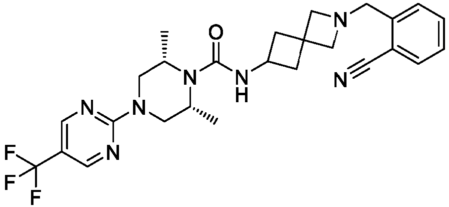 | (2*R*,6*S*)-*N*-{2-[(2-cyanophenyl)methyl]-2-azaspiro[3.3]heptan-6-yl}-2,6-dimethyl-4-[5-(trifluoromethyl)pyrimidin-2-yl]piperazine-1-carboxamide |
| 88 | 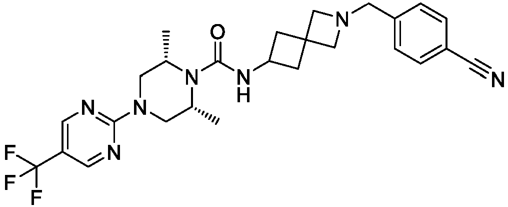 | (2*R*,6*S*)-*N*-{2-[(4-cyanophenyl)methyl]-2-azaspiro[3.3]heptan-6-yl}-2,6-dimethyl-4-[5-(trifluoromethyl)pyrimidin-2-yl]piperazine-1-carboxamide |
| 89 | 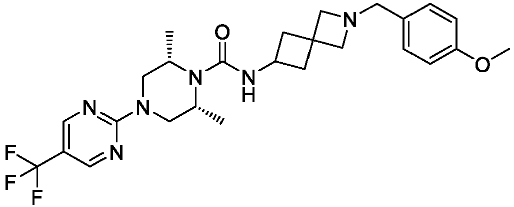 | (2*R,*6*S*)-*N*-{2-[(4-methoxyphenyl)methyl]-2-azaspiro[3.3]heptan-6-yl}-2,6-dimethyl-4-[5-(trifluoromethyl)pyrimidin-2-yl]piperazine-1-carboxamide |
| 90 | 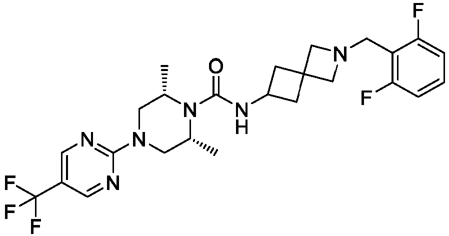 | (2*R*,6*S*)-*N*-{2-[(2,6-difluorophenyl)methyl]-2-azaspiro[3.3]heptan-6-yl}-2,6-dimethyl-4-[5-(trifluoromethyl)pyrimidin-2-yl]piperazine-1-carboxamide |
| 91 | 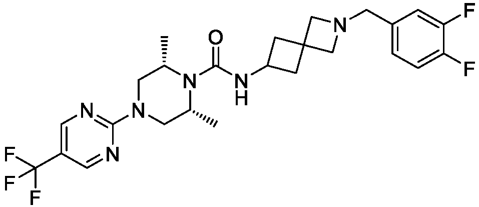 | (2*R*,6*S*)-*N*-{2-[(3,4-difluorophenyl)methyl]-2-azaspiro[3.3]heptan-6-yl}-2,6-dimethyl-4-[5-(trifluoromethyl)pyrimidin-2-yl]piperazine-1-carboxamide |
| 92 | 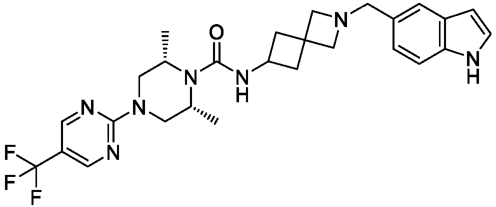 | (2*R*,6*S*)-*N*-[2-(1*H*-indol-5-ylmethyl)-2-azaspiro[3.3]heptan-6-yl]-2,6-dimethyl-4-[5-(trifluoromethyl)pyrimidin-2-yl]piperazine-1-carboxamide |
| 93 | 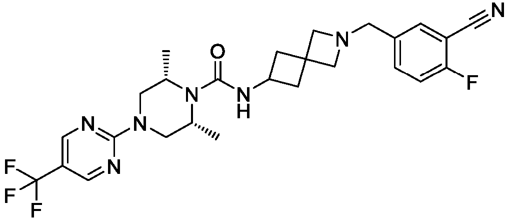 | (2*R*,6*S*)-*N*-{2-[(3-cyano-4-fluorophenyl)methyl]-2-azaspiro[3.3]heptan-6-yl}-2,6-dimethyl-4-[5-(trifluoromethyl)pyrimidin-2-yl]piperazine-1-carboxamide |
| 94 | 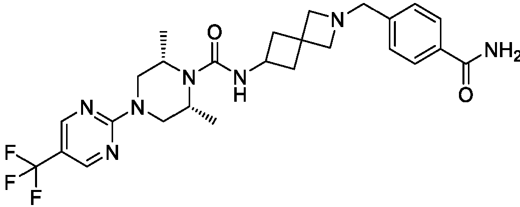 | (2*R*,6*S*)-*N*-{2-[(4-carbamoylphenyl)methyl]-2-azaspiro[3.3]heptan-6-yl}-2,6-dimethyl-4-[5-(trifluoromethyl)pyrimidin-2-yl]piperazine-1-carboxamide |
| 95 | 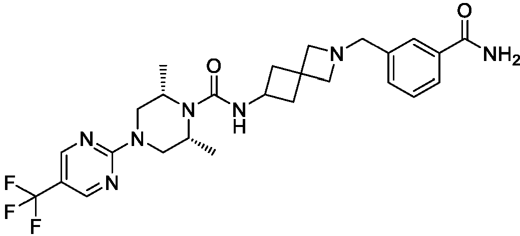 | (2*R*,6*S*)-*N*-{2-[(3-carbamoylphenyl)methyl]-2-azaspiro[3.3]heptan-6-yl}-2,6-dimethyl-4-[5-(trifluoromethyl)pyrimidin-2-yl]piperazine-1-carboxamide |
| 96 | 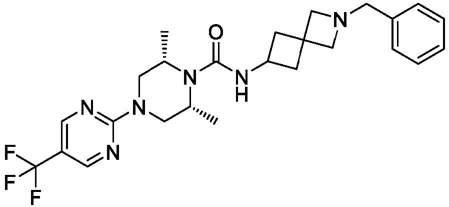 | (2*R*,6*S*)-*N*-{2-benzyl-2-azaspiro[3.3]heptan-6-yl}-2,6-dimethyl-4-[5-(trifluoromethyl)pyrimidin-2-yl]piperazine-1-carboxamide |
| 97 | 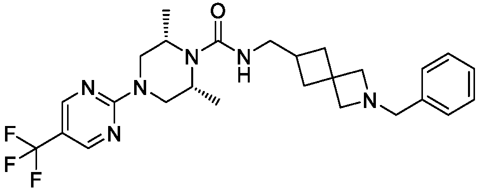 | (2R,6S)-N-({2-benzyl-2-azaspiro[3.3]heptan-6-yl}methyl)-2,6-dimethyl-4-[5-(trifluoromethyl)pyrimidin-2-yl]piperazine-1-carboxamide |
| 98 | 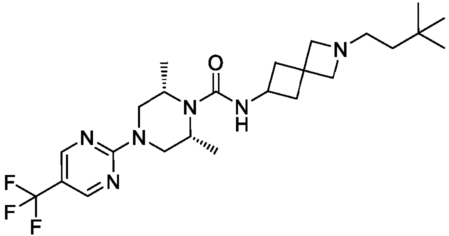 | (2*R*,6*S*)-*N*-[2-(3,3-dimethylbutyl)-2-azaspiro[3.3]heptan-6-yl]-2,6-dimethyl-4-[5-(trifluoromethyl)pyrimidin-2-yl]piperazine-1-carboxamide |
| 99 | 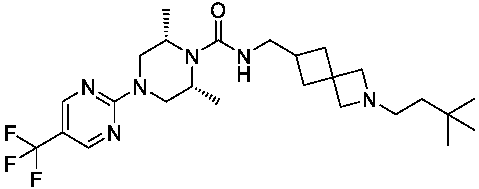 | (2*R*,6*S*)-*N*-{[2-(3,3-dimethylbutyl)-2-azaspiro[3.3]heptan-6-yl]methyl}-2,6-dimethyl-4-[5-(trifluoromethyl)pyrimidin-2-yl]piperazine-1-carboxamide |
| 100 | 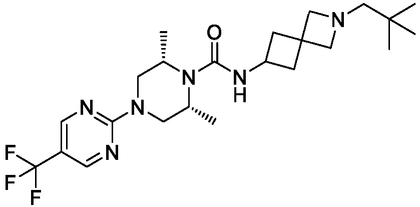 | (2*R*,6*S*)-*N*-[2-(2,2-dimethylpropyl)-2-azaspiro[3.3]heptan-6-yl]-2,6-dimethyl-4-[5-(trifluoromethyl)pyrimidin-2-yl]piperazine-1-carboxamide |
| 101 | 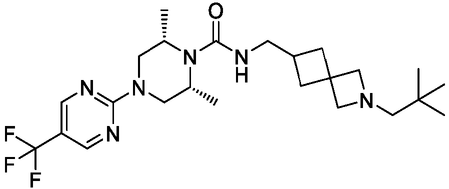 | (2*R*,6*S*)-*N*-{[2-(2,2-dimethylpropyl)-2-azaspiro[3.3]heptan-6-yl]methyl}-2,6-dimethyl-4-[5-(trifluoromethyl)pyrimidin-2-yl]piperazine-1-carboxamide |
| 102 | 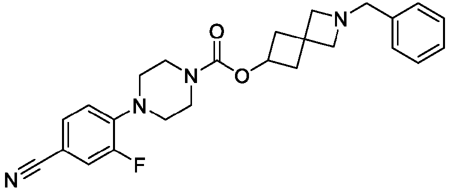 | 2-benzyl-2-azaspiro[3.3]heptan-6-yl 4-(4-cyano-2-fluorophenyl)piperazine-1-carboxylate |
| 103 | 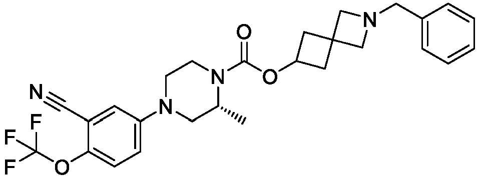 | 2-benzyl-2-azaspiro[3.3]heptan-6-yl (2*R*)-4-[3-cyano-4-(trifluoromethoxy)phenyl]-2-methylpiperazine-1-carboxylate |
| 104 | 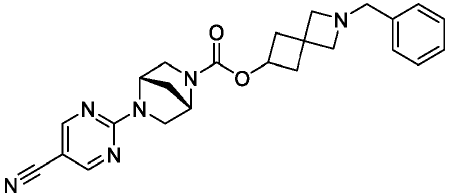 | 2-benzyl-2-azaspiro[3.3]heptan-6-yl (1*S*,4*S*)-5-(5-cyanopyrimidin-2-yl)-2,5-diazabicyclo[2.2.1]heptane-2-carboxylate |
| 105 | 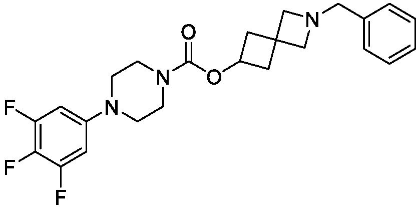 | 2-benzyl-2-azaspiro[3.3]heptan-6-yl 4-(3,4,5-trifluorophenyl)piperazine-1-carboxylate |
| 106 | 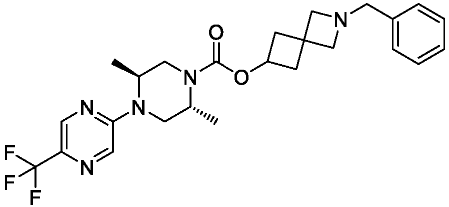 | 2-benzyl-2-azaspiro[3.3]heptan-6-yl (2*R*,5*S*)-2,5-dimethyl-4-[5-(trifluoromethyl)pyrazin-2-yl]piperazine-1-carboxylate |
| 107 | 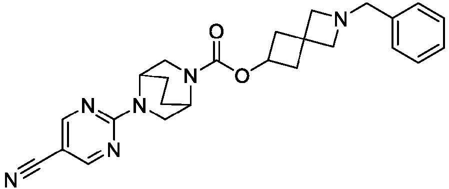 | 2-benzyl-2-azaspiro[3.3]heptan-6-yl 5-(5-cyanopyrimidin-2-yl)-2,5-diazabicyclo[2.2.2]octane-2-carboxylate |
| 108 | 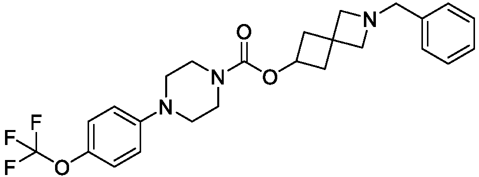 | 2-benzyl-2-azaspiro[3.3]heptan-6-yl 4-[4-(trifluoromethoxy)phenyl]pip erazine-1-carboxylate |
| 109 | 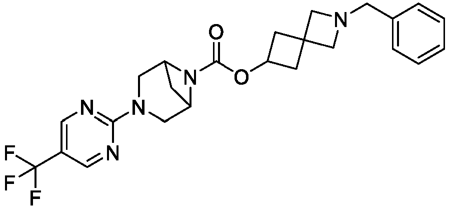 | 2-benzyl-2-azaspiro[3.3]heptan-6-yl 3-[5-(trifluoromethyl)pyrimidin-2-yl]-3,6-diazabicyclo[3.1.1]heptane-6-carboxylate |
| 110 | 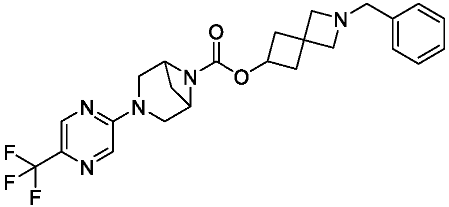 | 2-benzyl-2-azaspiro[3.3]heptan-6-yl 3-[5-(trifluoromethyl)pyrazin-2-yl]-3,6-diazabicyclo[3.1.1]heptane-6-carboxylate |
| 111 | 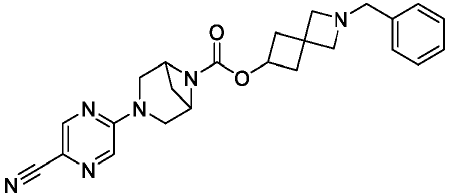 | 2-benzyl-2-azaspiro[3.3]heptan-6-yl 3-(5-cyanopyrazin-2-yl)-3,6-diazabicyclo[3.1.1]heptane-6-carboxylate |
| 112 | 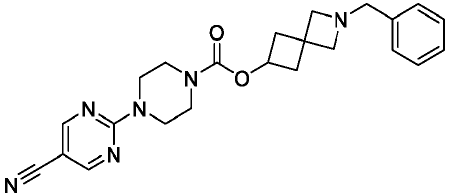 | 2-benzyl-2-azaspiro[3.3]heptan-6-yl 4-(5-cyanopyrimidin-2-yl)piperazine-1-carboxylate |
| 113 | 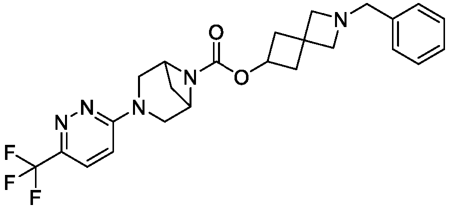 | 2-benzyl-2-azaspiro[3.3]heptan-6-yl 3-[6-(trifluoromethyl)pyridazin-3-yl]-3,6-diazabicyclo[3.1.1]heptane-6-carboxylate |
| 114 | 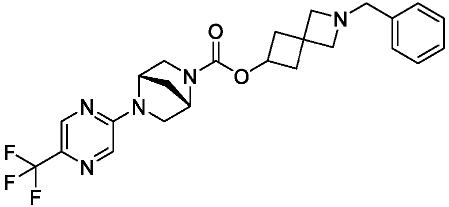 | 2-benzyl-2-azaspiro[3.3]heptan-6-yl (1*S*,4*S*)-5-[5-(trifluoromethyl)pyrazin-2-yl]-2,5-diazabicyclo[2.2.1]heptane-2-carboxylate |
| 115 | 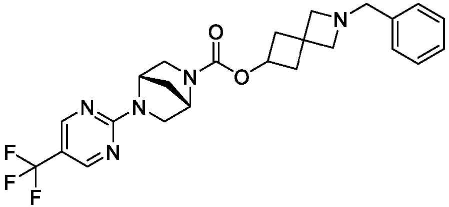 | 2-benzyl-2-azaspiro[3.3]heptan-6-yl (1*S*,4*S*)-5-[5-(trifluoromethyl)pyrimidin-2-yl]-2,5-diazabicyclo[2.2.1]heptane-2-carboxylate |
| 116 | 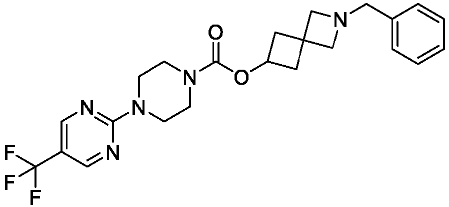 | 2-benzyl-2-azaspiro[3.3]heptan-6-yl 4-[5-(trifluoromethyl)pyrimidin-2-yl]piperazine-1-carboxylate |
| 117 | 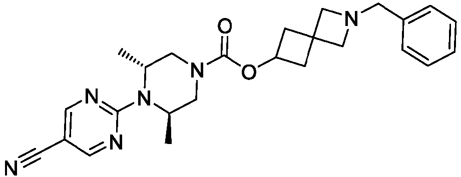 | 2-benzyl-2-azaspiro[3.3]heptan-6-yl (3*R*,5*R*)-4-(5-cyanopyrimidin-2-yl)-3,5-dimethylpiperazine-1-carboxylate |
| 118 | 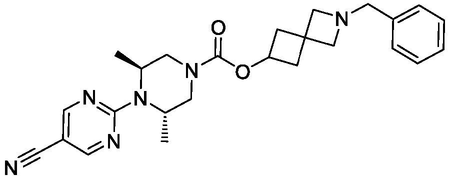 | 2-benzyl-2-azaspiro[3.3]heptan-6-yl (3*S*,5*S*)-4-(5-cyanopyrimidin-2-yl)-3,5-dimethylpiperazine-1-carboxylate |
| 119 | 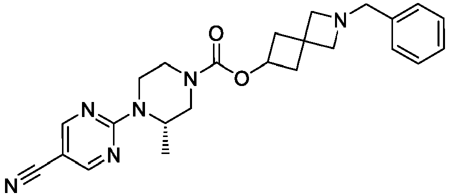 | 2-benzyl-2-azaspiro[3.3]heptan-6-yl (3*S*)-4-(5-cyanopyrimidin-2-yl)-3-methylpiperazine-1-carboxylate |
| 120 | 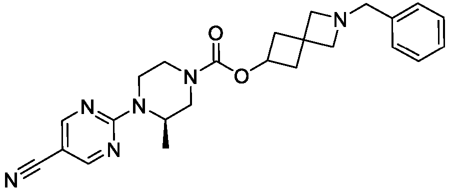 | 2-benzyl-2-azaspiro[3.3]heptan-6-yl (3*R*)-4-(5-cyanopyrimidin-2-yl)-3-methylpiperazine-1-carboxylate |
| 121 | 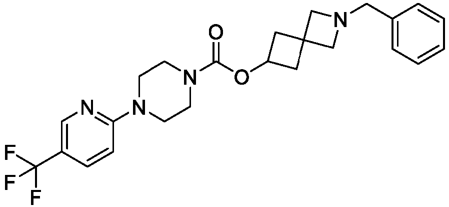 | 2-benzyl-2-azaspiro[3.3]heptan-6-yl 4-[5-(trifluoromethyl)pyridin-2-yl]piperazine-1-carboxylate |
| 122 | 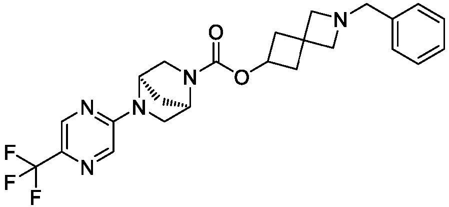 | 2-benzyl-2-azaspiro[3.3]heptan-6-yl (1*R*,4*R*)-5-[5-(trifluoromethyl)pyrazin-2-yl]-2,5-diazabicyclo[2.2.1]heptane-2-carboxylate |
| 123 | 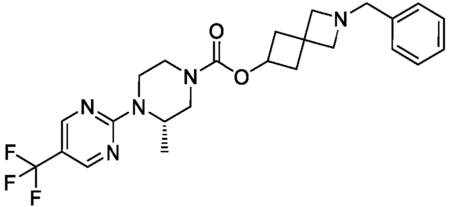 | 2-benzyl-2-azaspiro[3.3]heptan-6-yl (3*S*)-3-methyl-4-[5-(trifluoromethyl)pyrimidin-2-yl]piperazine-1-carboxylate |
| 124 | 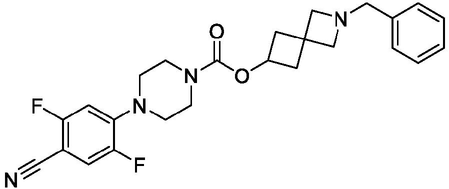 | 2-benzyl-2-azaspiro[3.3]heptan-6-yl 4-(4-cyano-2,5-difluorophenyl)piperazine-1-carboxylate |
| 125 | 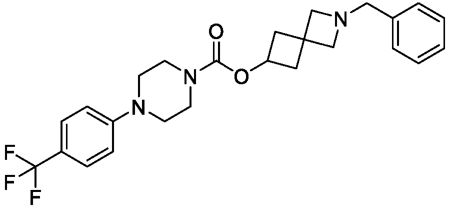 | 2-benzyl-2-azaspiro[3.3]heptan-6-yl 4-[4-(trifluoromethyl)phenyl]piper azine-1-carboxylate |
| 126 | 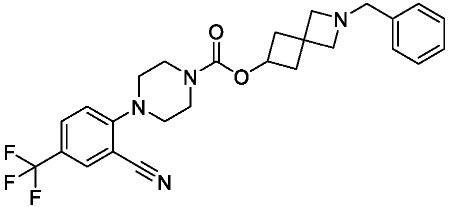 | 2-benzyl-2-azaspiro[3.3]heptan-6-yl 4-[2-cyano-4-(trifluoromethyl)phenyl]piper azine-1-carboxylate |
| 127 | 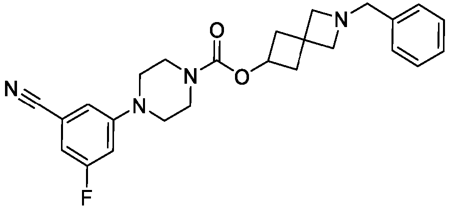 | 2-benzyl-2-azaspiro[3.3]heptan-6-yl 4-(3-cyano-5-fluorophenyl)piperazine-1-carboxylate |
| 128 | 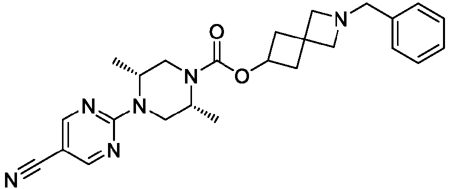 | 2-benzyl-2-azaspiro[3.3]heptan-6-yl (2*R*,5*R*)-4-(5-cyanopyrimidin-2-yl)-2,5-dimethylpiperazine-1-carboxylate |
| 129 | 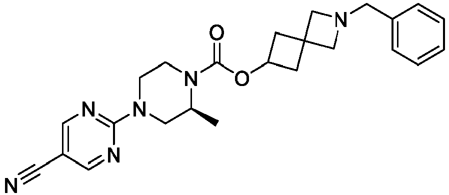 | 2-benzyl-2-azaspiro[3.3]heptan-6-yl (2*S*)-4-(5-cyanopyrimidin-2-yl)-2-methylpiperazine-1-carboxylate |
| 130 | 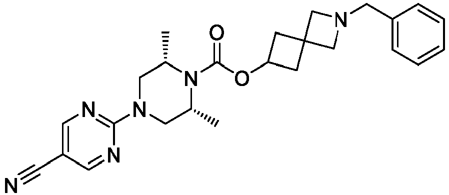 | 2-benzyl-2-azaspiro[3.3]heptan-6-yl (2*R*,6*S*)-4-(5-cyanopyrimidin-2-yl)-2,6-dimethylpiperazine-1-carboxylate |
| 131 | 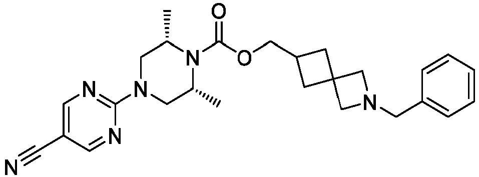 | {2-benzyl-2-azaspiro[3.3]heptan-6-yl}methyl (2*R*,6*S*)-4-(5-cyanopyrimidin-2-yl)-2,6-dimethylpiperazine-1-carboxylate |
| 132 | 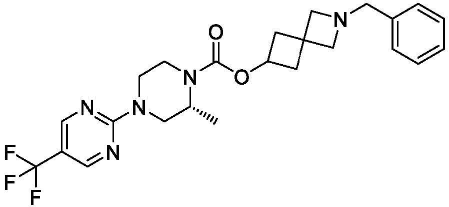 | 2-benzyl-2-azaspiro[3.3]heptan-6-yl (2*R*)-2-methyl-4-[5-(trifluoromethyl)pyrimidin-2-yl]piperazine-1-carboxylate |
| 133 | 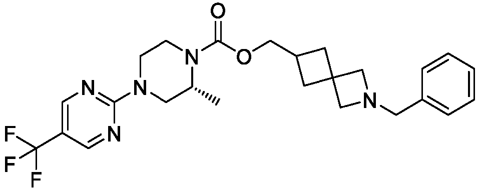 | {2-benzyl-2-azaspiro[3.3]heptan-6-yl}methyl (2*R*)-2-methyl-4-[5-(trifluoromethyl)pyrimidin-2-yl]piperazine-1-carboxylate |
| 134 | 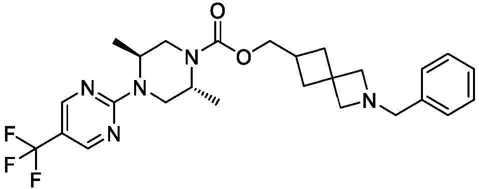 | {2-benzyl-2-azaspiro[3.3]heptan-6-yl}methyl (2*R*,5*S*)-2,5-dimethyl-4-[5-(trifluoromethyl)pyrimidin-2-yl]piperazine-1-carboxylate |
| 135 | 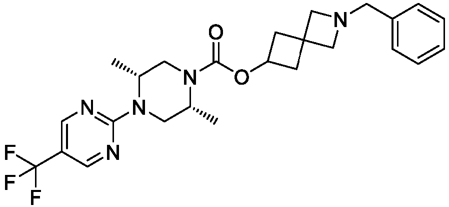 | 2-benzyl-2-azaspiro[3.3]heptan-6-yl (2*R*,5*R*)-2,5-dimethyl-4-[5-(trifluoromethyl)pyrimidin-2-yl]piperazine-1-carboxylate |
| 136 | 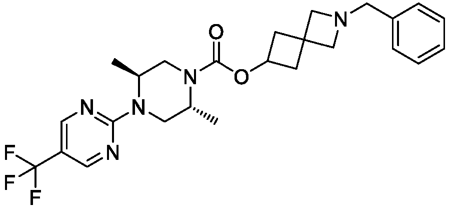 | 2-benzyl-2-azaspiro[3.3]heptan-6-yl (2*R*,5*S*)-2,5-dimethyl-4-[5-(trifluoromethyl)pyrimidin-2-yl]piperazine-1-carboxylate |
| 137 | 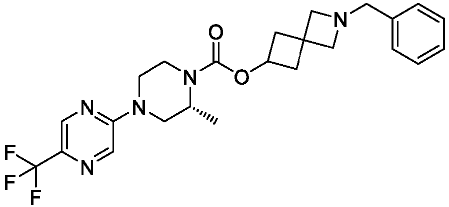 | 2-benzyl-2-azaspiro[3.3]heptan-6-yl (2*R*)-2-methyl-4-[5-(trifluoromethyl)pyrazin-2-yl]piperazine-1-carboxylate |
| 138 | 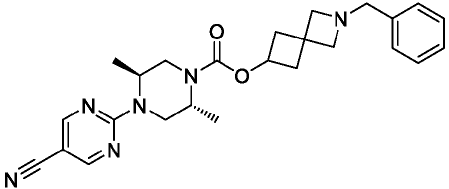 | 2-benzyl-2-azaspiro[3.3]heptan-6-yl (2*R*,5*S*)-4-(5-cyanopyrimidin-2-yl)-2,5-dimethylpiperazine-1-carboxylate |
| 139 | 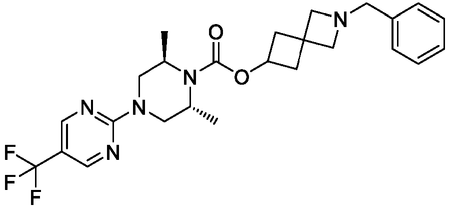 | 2-benzyl-2-azaspiro[3.3]heptan-6-yl (2*R*,6*R*)-2,6-dimethyl-4-[5-(trifluoromethyl)pyrimidin-2-yl]piperazine-1-carboxylate |
| 140 | 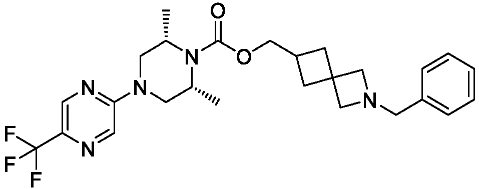 | {2-benzyl-2-azaspiro[3.3]heptan-6-yl}methyl (2*R*,6*S*)-2,6-dimethyl-4-[5-(trifluoromethyl)pyrazin-2-yl]piperazine-1-carboxylate |
| 141 | 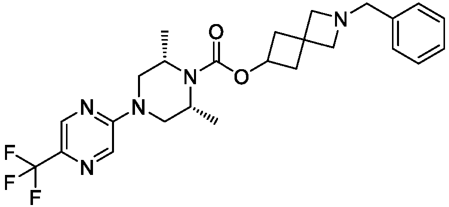 | 2-benzyl-2-azaspiro[3.3]heptan-6-yl (2*R*,6*S*)-2,6-dimethyl-4-[5-(trifluoromethyl)pyrazin-2-yl]piperazine-1-carboxylate |
| 142 | 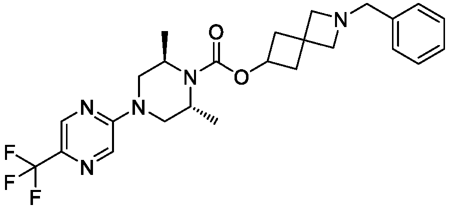 | 2-benzyl-2-azaspiro[3.3]heptan-6-yl (2*R*,6*R*)-2,6-dimethyl-4-[5-(trifluoromethyl)pyrazin-2-yl]piperazine-1-carboxylate |
| 143 | 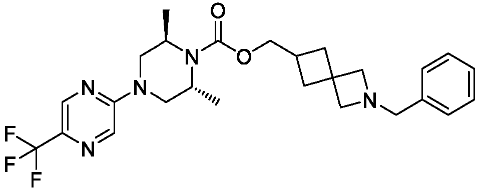 | {2-benzyl-2-azaspiro[3.3]heptan-6-yl}methyl (2*R*,6*R*)-2,6-dimethyl-4-[5-(trifluoromethyl)pyrazin-2-yl]piperazine-1-carboxylate |
| 144 | 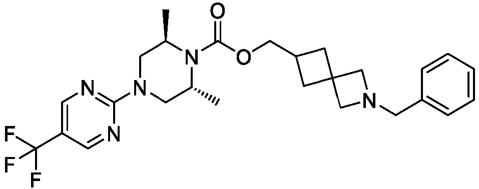 | {2-benzyl-2-azaspiro[3.3]heptan-6-yl}methyl (2*R*,6*R*)-2,6-dimethyl-4-[5-(trifluoromethyl)pyrimidin-2-yl]piperazine-1-carboxylate |
| 145 | 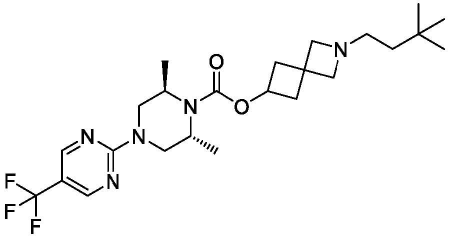 | 2-(3,3-dimethylbutyl)-2-azaspiro[3.3]heptan-6-yl (2*R*,6*R*)-2,6-dimethyl-4-[5-(trifluoromethyl)pyrimidin-2-yl]piperazine-1-carboxylate |
| 146 | 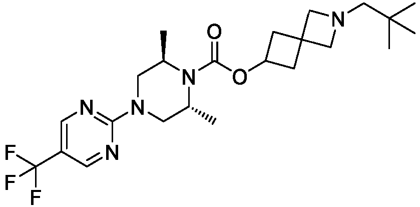 | 2-(2,2-dimethylpropyl)-2-azaspiro[3.3]heptan-6-yl (2*R*,6*R*)-2,6-dimethyl-4-[5-(trifluoromethyl)pyrimidin-2-yl]piperazine-1-carboxylate |
| 147 | 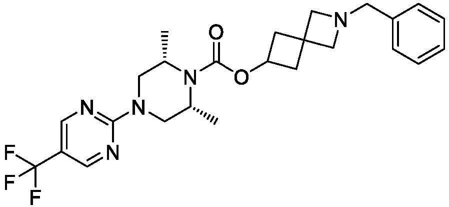 | 2-benzyl-2-azaspiro[3.3]heptan-6-yl (2*R*,6*S*)-2,6-dimethyl-4-[5-(trifluoromethyl)pyrimidin-2-yl]piperazine-1-carboxylate |
| 148 | 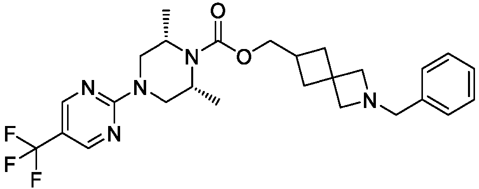 | {2-benzyl-2-azaspiro[3.3]heptan-6-yl}methyl (2*R*,6*S*)-2,6-dimethyl-4-[5-(trifluoromethyl)pyrimidin-2-yl]piperazine-1-carboxylate |
| 149 | 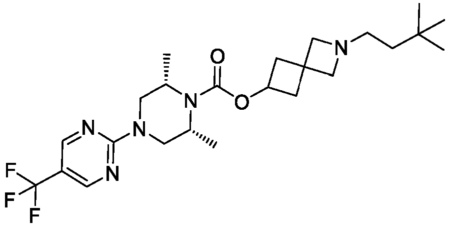 | 2-(3,3-dimethylbutyl)-2-azaspiro[3.3]heptan-6-yl (2*R*,6*S*)-2,6-dimethyl-4-[5-(trifluoromethyl)pyrimidin-2-yl]piperazine-1-carboxylate |
| 150 | 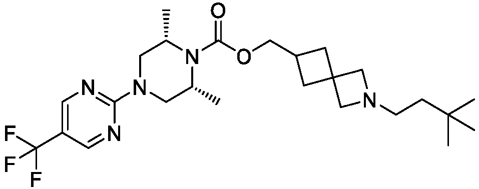 | [2-(3,3-dimethylbutyl)-2-azaspiro[3.3]heptan-6-yl]methyl (2*R*,6*S*)-2,6-dimethyl-4-[5-(trifluoromethyl)pyrimidin-2-yl]piperazine-1-carboxylate |
| 151 | 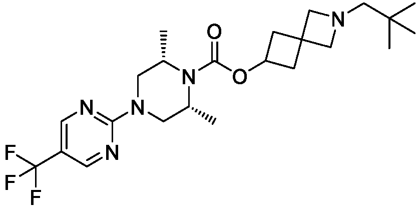 | 2-(2,2-dimethylpropyl)-2-azaspiro[3.3]heptan-6-yl (2*R*,6*S*)-2,6-dimethyl-4-[5-(trifluoromethyl)pyrimidin-2-yl]piperazine-1-carboxylate |
| 152 | 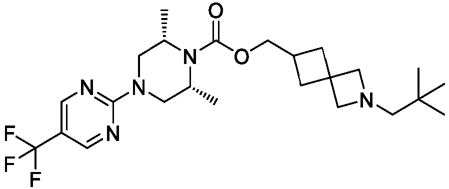 | [2-(2,2-dimethylpropyl)-2-azaspiro[3.3]heptan-6-yl]methyl (2*R*,6*S*)-2,6-dimethyl-4-[5-(trifluoromethyl)pyrimidin-2-yl]piperazine-1-carboxylate |
| 153 | 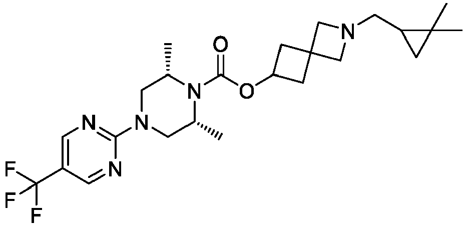 | 2-[(2,2-dimethylcyclopropyl)methyl]-2-azaspiro[3.3]heptan-6-yl (2*R*,6*S*)-2,6-dimethyl-4-[5-(trifluoromethyl)pyrimidin-2-yl]piperazine-1-carboxylate |
| 154 | 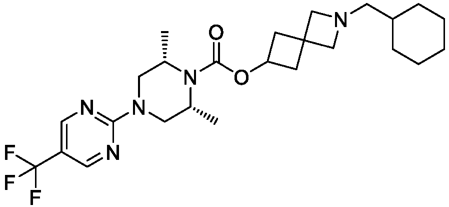 | 2-(cyclohexylmethyl)-2-azaspiro[3.3]heptan-6-yl (2*R*,6*S*)-2,6-dimethyl-4-[5-(trifluoromethyl)pyrimidin-2-yl]piperazine-1-carboxylate |
| 155 | 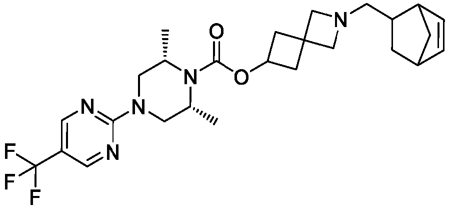 | 2-{bicyclo[2.2.1]hept-5-en-2-ylmethyl}-2-azaspiro[3.3]heptan-6-yl (2*R*,6*S*)-2,6-dimethyl-4-[5-(trifluoromethyl)pyrimidin-2-yl]piperazine-1-carboxylate |
| 156 | 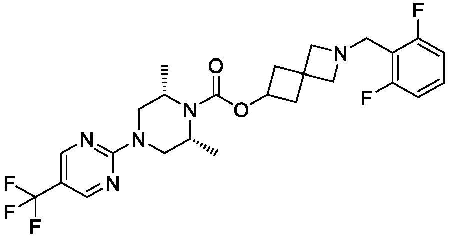 | 2-[(2,6-difluorophenyl)methyl]-2-azaspiro[3.3]heptan-6-yl (2*R*,6*S*)-2,6-dimethyl-4-[5-(trifluoromethyl)pyrimidin-2-yl]piperazine-1-carboxylate |
| 157 | 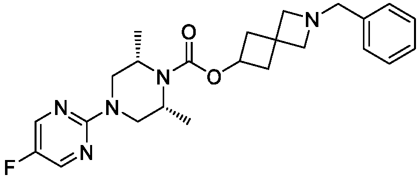 | 2-benzyl-2-azaspiro[3.3]heptan-6-yl (2*R*,6*S*)-4-(5-fluoropyrimidin-2-yl)-2,6-dimethylpiperazine-1-carboxylate |
| 158 | 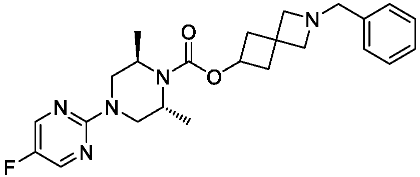 | 2-benzyl-2-azaspiro[3.3]heptan-6-yl (2*R*,6*R*)-4-(5-fluoropyrimidin-2-yl)-2,6-dimethylpiperazine-1-carboxylate |
| 159 | 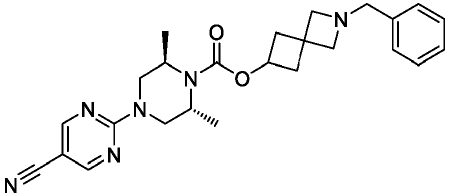 | 2-benzyl-2-azaspiro[3.3]heptan-6-yl (2*R*,6*R*)-4-(5-cyanopyrimidin-2-yl)-2,6-dimethylpiperazine-1-carboxylate |
| 160 | 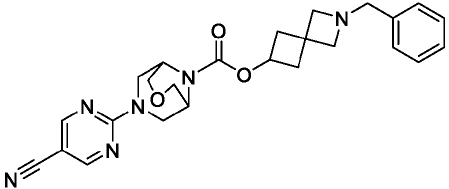 | 2-benzyl-2-azaspiro[3.3]heptan-6-yl 7-(5-cyanopyrimidin-2-yl)-3-oxa-7,9-diazabicyclo[3.3.1]nonane-9-carboxylate |
| 161 | 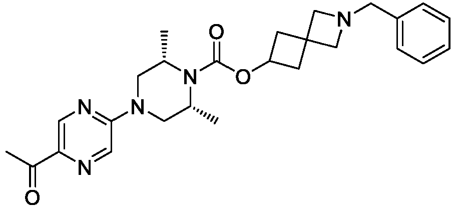 | 2-benzyl-2-azaspiro[3.3]heptan-6-yl (2*R*,6*S*)-4-(5-acetylpyrazin-2-yl)-2,6-dimethylpiperazine-1-carboxylate |
| 162 | 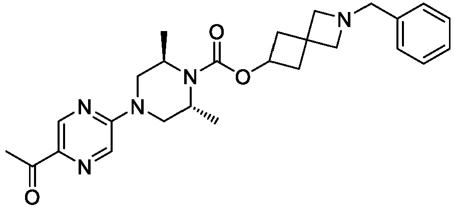 | 2-benzyl-2-azaspiro[3.3]heptan-6-yl (2*R*,6*R*)-4-(5-acetylpyrazin-2-yl)-2,6-dimethylpiperazine-1-carboxylate |
| 163 | 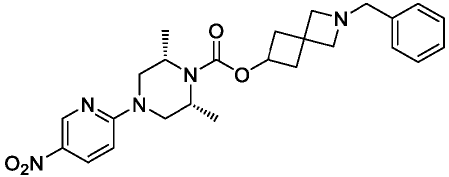 | 2-benzyl-2-azaspiro[3.3]heptan-6-yl (2*R*,6*S*)-2,6-dimethyl-4-(5-nitropyridin-2-yl)piperazine-1-carboxylate |
| 164 | 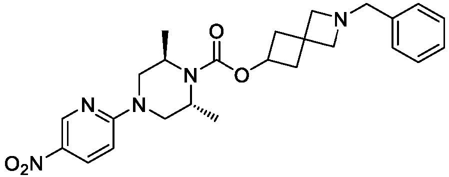 | 2-benzyl-2-azaspiro[3.3]heptan-6-yl (2*R*,6*R*)-2,6-dimethyl-4-(5-nitropyridin-2-yl)piperazine-1-carboxylate |
| 165 | 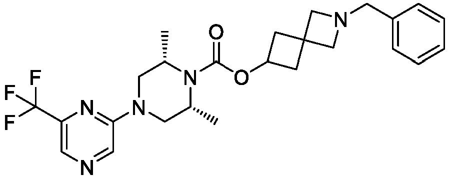 | 2-benzyl-2-azaspiro[3.3]heptan-6-yl (2*R*,6*S*)-2,6-dimethyl-4-[6-(trifluoromethyl)pyrazin-2-yl]piperazine-1-carboxylate |
| 166 | 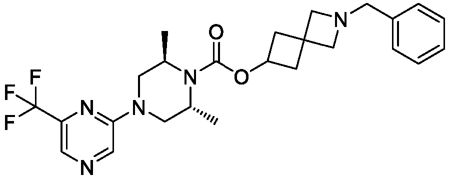 | 2-benzyl-2-azaspiro[3.3]heptan-6-yl (2*R*,6*R*)-2,6-dimethyl-4-[6-(trifluoromethyl)pyrazin-2-yl]piperazine-1-carboxylate |
| 167 | 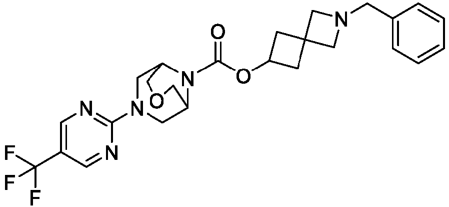 | 2-benzyl-2-azaspiro[3.3]heptan-6-yl 7-[5-(trifluoromethyl)pyrimidin-2-yl]-3-oxa-7,9-diazabicyclo[3.3.1]nonane-9-carboxylate |
| 168 | 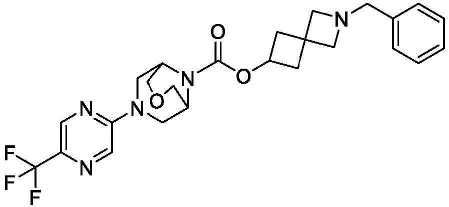 | 2-benzyl-2-azaspiro[3.3]heptan-6-yl 7-[5-(trifluoromethyl)pyrazin-2-yl]-3-oxa-7,9-diazabicyclo[3.3.1]nonane-9-carboxylate |
| 169 | 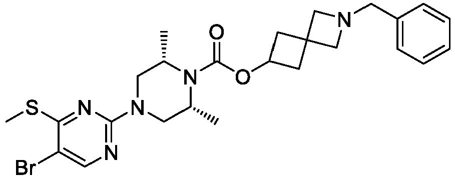 | 2-benzyl-2-azaspiro[3.3]heptan-6-yl (2*R*,6*S*)-4-[5-bromo-4-(methylsulfanyl)pyrimidin-2-yl]-2,6-dimethylpiperazine-1-carboxylate |
| 170 | 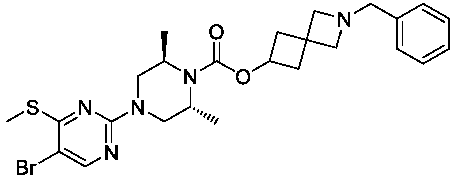 | 2-benzyl-2-azaspiro[3.3]heptan-6-yl (2*R,*6*R*)-4-[5-bromo-4-(methylsulfanyl)pyrimidin-2-yl]-2,6-dimethylpiperazine-1-carboxylate |
| 171 | 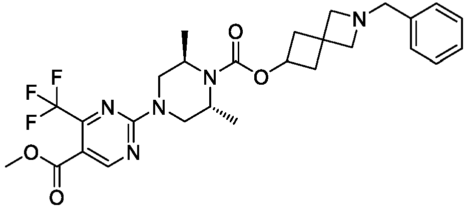 | methyl 2-[(3*R*,5*R*)-4-[({2-benzyl-2-azaspiro[3.3]heptan-6-yl}oxy)carbonyl]-3,5-dimethylpiperazin-1-yl]-4-(trifluoromethyl)pyrimidine-5-carboxylate |
| 172 | 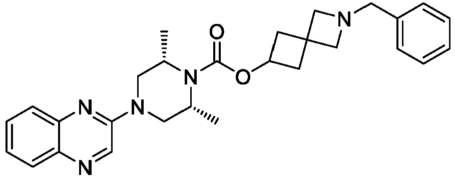 | 2-benzyl-2-azaspiro[3.3]heptan-6-yl (2*R*,6*S*)-2,6-dimethyl-4-(quinoxalin-2-yl)piperazine-1-carboxylate |
| 173 | 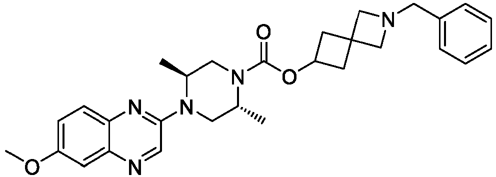 | 2-benzyl-2-azaspiro[3.3]heptan-6-yl (2*R*,5*S*)-4-(6-methoxyquinoxalin-2-yl)-2,5-dimethylpiperazine-1-carboxylate |
| 174 | 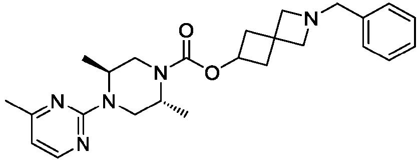 | 2-benzyl-2-azaspiro[3.3]heptan-6-yl (2*R*,5*S*)-2,5-dimethyl-4-(4-methylpyrimidin-2-yl)piperazine-1-carboxylate |
| 175 | 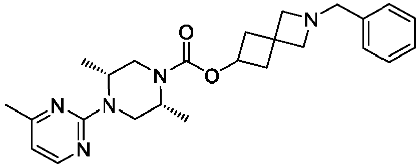 | 2-benzyl-2-azaspiro[3.3]heptan-6-yl (2*R*,5*R*)-2,5-dimethyl-4-(4-methylpyrimidin-2-yl)piperazine-1-carboxylate |
| 176 | 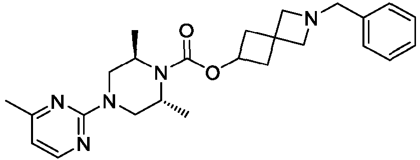 | 2-benzyl-2-azaspiro[3.3]heptan-6-yl (2*R*,6*R*)-2,6-dimethyl-4-(4-methylpyrimidin-2-yl)piperazine-1-carboxylate |
| 177 | 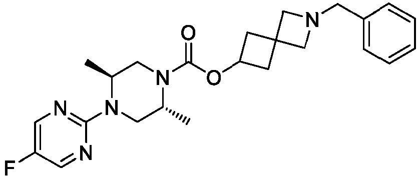 | 2-benzyl-2-azaspiro[3.3]heptan-6-yl (2*R*,5*S*)-4-(5-fluoropyrimidin-2-yl)-2,5-dimethylpiperazine-1-carboxylate |
| 178 | 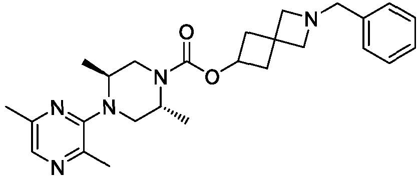 | 2-benzyl-2-azaspiro[3.3]heptan-6-yl (2*R*,5*S*)-4-(3,6-dimethylpyrazin-2-yl)-2,5-dimethylpiperazine-1-carboxylate |
| 179 | 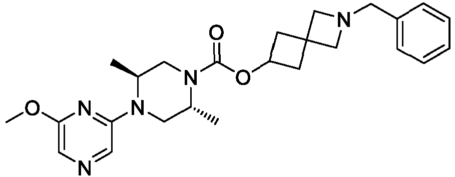 | 2-benzyl-2-azaspiro[3.3]heptan-6-yl (2*R*,5*S*)-4-(6-methoxypyrazin-2-yl)-2,5-dimethylpiperazine-1-carboxylate |
| 180 | 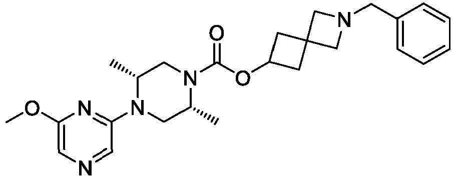 | 2-benzyl-2-azaspiro[3.3]heptan-6-yl (2*R*,5*R*)-4-(6-methoxypyrazin-2-yl)-2,5-dimethylpiperazine-1-carboxylate |
| 181 | 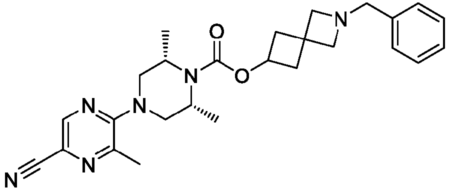 | 2-benzyl-2-azaspiro[3.3]heptan-6-yl (2*R*,6*S*)-4-(5-cyano-3-methylpyrazin-2-yl)-2,6-dimethylpiperazine-1-carboxylate |
| 182 | 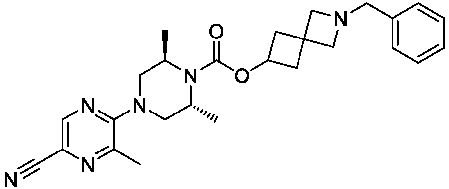 | 2-benzyl-2-azaspiro[3.3]heptan-6-yl (2*R*,6*R*)-4-(5-cyano-3-methylpyrazin-2-yl)-2,6-dimethylpiperazine-1-carboxylate |
| 183 | 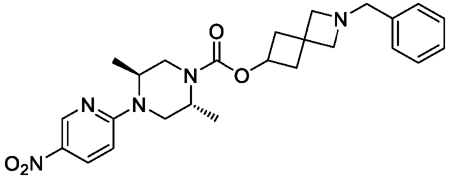 | 2-benzyl-2-azaspiro[3.3]heptan-6-yl (2*R*,5*S*)-2,5-dimethyl-4-(5-nitropyridin-2-yl)piperazine-1-carboxylate |
| 184 | 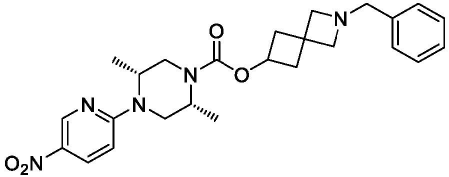 | 2-benzyl-2-azaspiro[3.3]heptan-6-yl (2*R*,5*R*)-2,5-dimethyl-4-(5-nitropyridin-2-yl)piperazine-1-carboxylate |
| 185 | 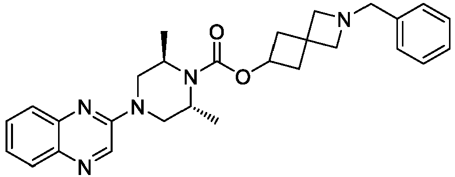 | 2-benzyl-2-azaspiro[3.3]heptan-6-yl (2*R*,6*R*)-2,6-dimethyl-4-(quinoxalin-2-yl)piperazine-1-carboxylate |
| 186 | 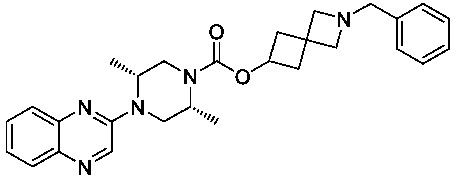 | 2-benzyl-2-azaspiro[3.3]heptan-6-yl (2*R*,5*R*)-2,5-dimethyl-4-(quinoxalin-2-yl)piperazine-1-carboxylate |
| 187 | 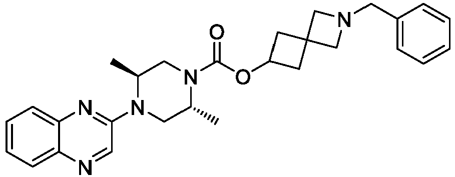 | 2-benzyl-2-azaspiro[3.3]heptan-6-yl (2*R*,5*S*)-2,5-dimethyl-4-(quinoxalin-2-yl)piperazine-1-carboxylate |
| 188 | 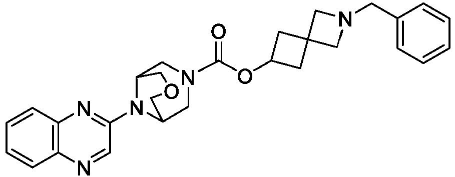 | 2-benzyl-2-azaspiro[3.3]heptan-6-yl 9-(quinoxalin-2-yl)-3-oxa-7,9-diazabicyclo[3.3.1]nonane-7-carboxylate |
| 189 | 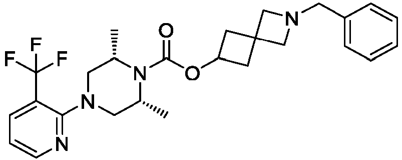 | 2-benzyl-2-azaspiro[3.3]heptan-6-yl (2*R*,6*S*)-2,6-dimethyl-4-[3-(trifluoromethyl)pyridin-2-yl]piperazine-1-carboxylate |
| 190 | 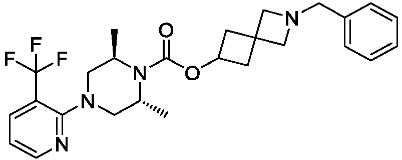 | 2-benzyl-2-azaspiro[3.3]heptan-6-yl (2*R*,6*R*)-2,6-dimethyl-4-[3-(trifluoromethyl)pyridin-2-yl]piperazine-1-carboxylate |
| 191 | 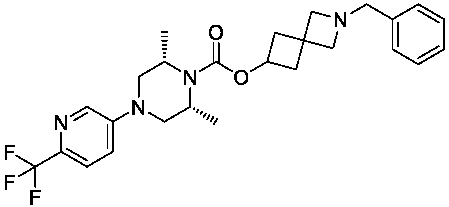 | 2-benzyl-2-azaspiro[3.3]heptan-6-yl (2*R*,6*S*)-2,6-dimethyl-4-[6-(trifluoromethyl)pyridin-3-yl]piperazine-1-carboxylate |
| 192 | 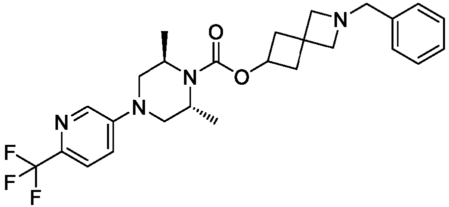 | 2-benzyl-2-azaspiro[3.3]heptan-6-yl (2*R*,6*R*)-2,6-dimethyl-4-[6-(trifluoromethyl)pyridin-3-yl]piperazine-1-carboxylate |
| 193 | 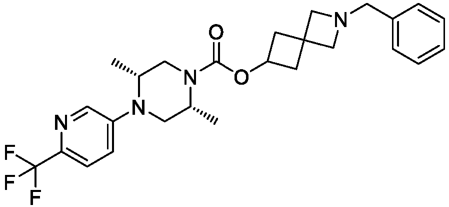 | 2-benzyl-2-azaspiro[3.3]heptan-6-yl (2*R*,5*R*)-2,5-dimethyl-4-[6-(trifluoromethyl)pyridin-3-yl]piperazine-1-carboxylate |
| 194 | 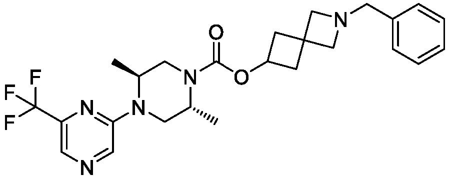 | 2-benzyl-2-azaspiro[3.3]heptan-6-yl (2*R*,5*S*)-2,5-dimethyl-4-[6-(trifluoromethyl)pyrazin-2-yl]piperazine-1-carboxylate |
| 195 | 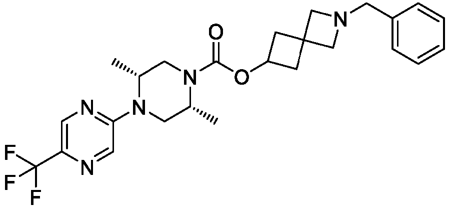 | 2-benzyl-2-azaspiro[3.3]heptan-6-yl (2*R*,5*R*)-2,5-dimethyl-4-[5-(trifluoromethyl)pyrazin-2-yl]piperazine-1-carboxylate |
| 196 | 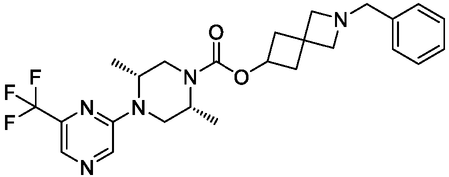 | 2-benzyl-2-azaspiro[3.3]heptan-6-yl (2*R*,5*R*)-2,5-dimethyl-4-[6-(trifluoromethyl)pyrazin-2-yl]piperazine-1-carboxylate |
| 197 | 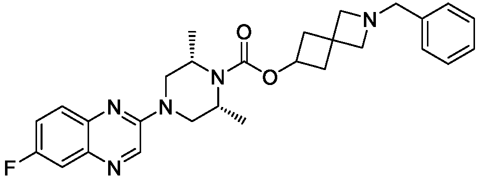 | 2-benzyl-2-azaspiro[3.3]heptan-6-yl (2*R*,6*S*)-4-(6-fluoroquinoxalin-2-yl)-2,6-dimethylpiperazine-1-carboxylate |
| 198 | 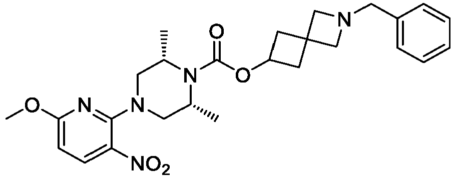 | 2-benzyl-2-azaspiro[3.3]heptan-6-yl (2*R*,6*S*)-4-(6-methoxy-3-nitropyridin-2-yl)-2,6-dimethylpiperazine-1-carboxylate |
| 199 | 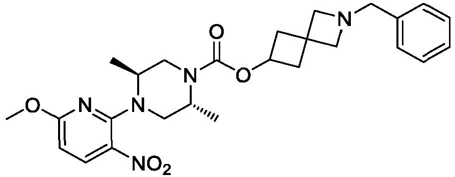 | 2-benzyl-2-azaspiro[3.3]heptan-6-yl (2*R*,5*S*)-4-(6-methoxy-3-nitropyridin-2-yl)-2,5-dimethylpiperazine-1-carboxylate |
| 200 | 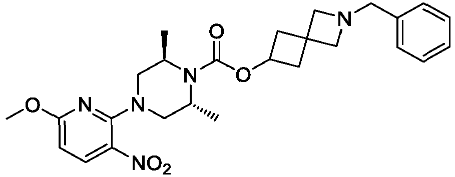 | 2-benzyl-2-azaspiro[3.3]heptan-6-yl (2*R*,6*R*)-4-(6-methoxy-3-nitropyridin-2-yl)-2,6-dimethylpiperazine-1-carboxylate |
| 201 | 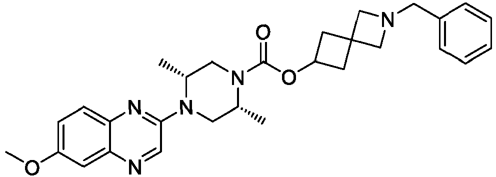 | 2-benzyl-2-azaspiro[3.3]heptan-6-yl (2*R*,5*R*)-4-(6-methoxyquinoxalin-2-yl)-2,5-dimethylpiperazine-1-carboxylate |
| 202 | 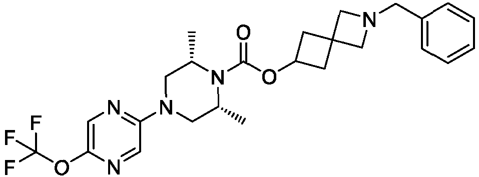 | 2-benzyl-2-azaspiro[3.3]heptan-6-yl (2*R*,6*S*)-2,6-dimethyl-4-[5-(trifluoromethoxy)pyrazin-2-yl]piperazine-1-carboxylate |
| 203 | 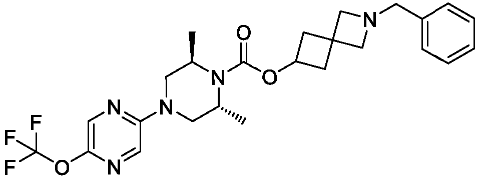 | 2-benzyl-2-azaspiro[3.3]heptan-6-yl (2*R*,6*R*)-2,6-dimethyl-4-[5-(trifluoromethoxy)pyrazin-2-yl]piperazine-1-carboxylate |
| 204 | 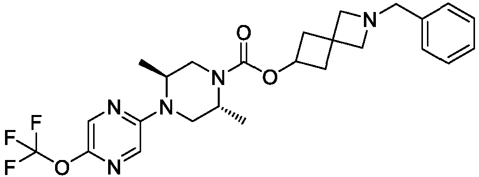 | 2-benzyl-2-azaspiro[3.3]heptan-6-yl (2*R*,5*S*)-2,5-dimethyl-4-[5-(trifluoromethoxy)pyrazin-2-yl]piperazine-1-carboxylate |
| 205 | 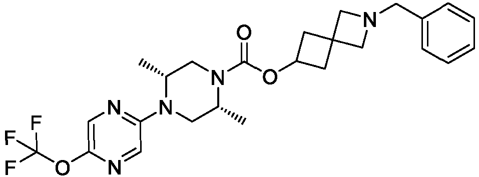 | 2-benzyl-2-azaspiro[3.3]heptan-6-yl (2*R*,5*R*)-2,5-dimethyl-4-[5-(trifluoromethoxy)pyrazin-2-yl]piperazine-1-carboxylate |
| 206 | 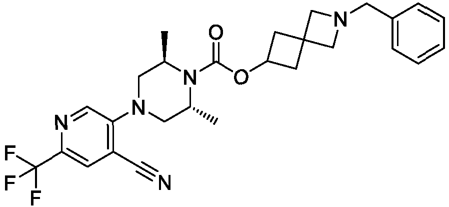 | 2-benzyl-2-azaspiro[3.3]heptan-6-yl (2*R*,6*R*)-4-[4-cyano-6-(trifluoromethyl) pyrid in-3-yl]-2,6-dimethylpiperazine-1-carboxylate |
| 207 | 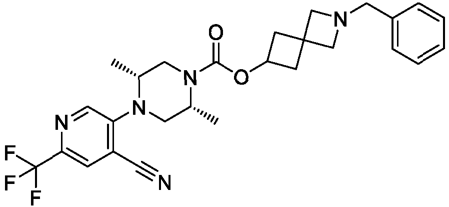 | 2-benzyl-2-azaspiro[3.3]heptan-6-yl (2*R*,5*R*)-4-[4-cyano-6-(trifluoromethyl) pyridin-3-yl]-2,5-dimethylpiperazine-1-carboxylate |
| 208 | 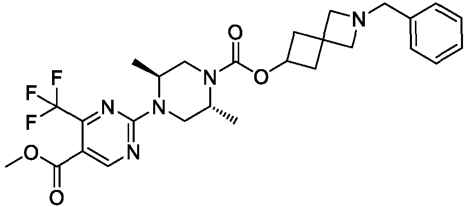 | methyl 2-[(2*S*,5*R*)-4-[({2-benzyl-2-azaspiro[3.3]heptan-6-yl}oxy)carbonyl]-2,5-dimethylpiperazin-1-yl]-4-(trifluoromethyl)pyrimidine-5-carboxylate |
| 209 | 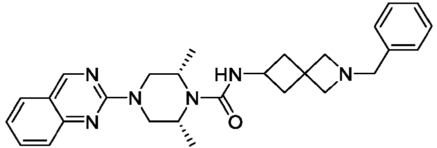 | (2R,6S)-N-{2-benzyl-2-azaspiro[3.3]heptan-6-yl}-2,6-dimethyl-4-(quinazolin-2-yl)piperazine-1-carboxamide |
| 210 | 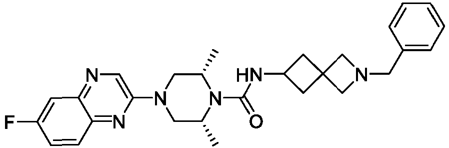 | (2R,6S)-N-{2-benzyl-2-azaspiro[3.3]heptan-6-yl}-4-(6-fluoroquinoxalin-2-yl)-2,6-dimethylpiperazine-1-carboxamide |
| 211 | 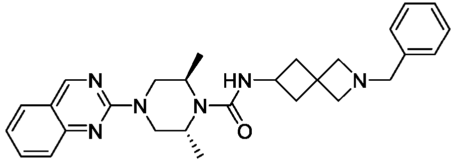 | (2R,6R)-N-{2-benzyl-2-azaspiro[3.3]heptan-6-yl}-2,6-dimethyl-4-(quinazolin-2-yl)piperazine-1-carboxamide |
| 212 | 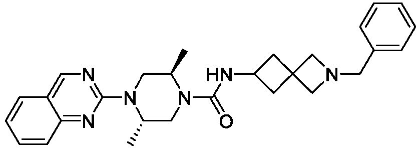 | (2R,5S)-N-{2-benzyl-2-azaspiro[3.3]heptan-6-yl}-2,5-dimethyl-4-(quinazolin-2-yl)piperazine-1-carboxamide |
| 213 | 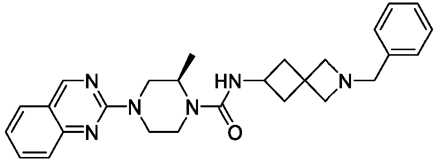 | (2R)-N-{2-benzyl-2-azaspiro[3.3]heptan-6-yl}-2-methyl-4-(quinazolin-2-yl)piperazine-1-carboxamide |
| 214 | 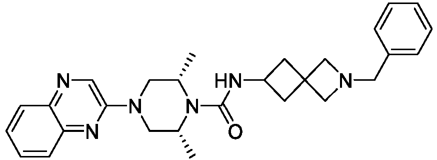 | (2R,6S)-N-{2-benzyl-2-azaspiro[3.3]heptan-6-yl}-2,6-dimethyl-4-(quinoxalin-2-yl)piperazine-1-carboxamide |
| 215 | 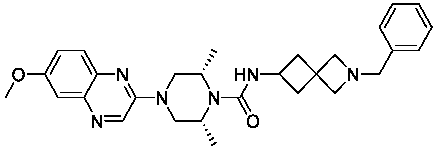 | (2R,6S)-N-{2-benzyl-2-azaspiro[3.3]heptan-6-yl}-4-(6-methoxyquinoxalin-2-yl)-2,6-dimethylpiperazine-1-carboxamide |
| 216 | 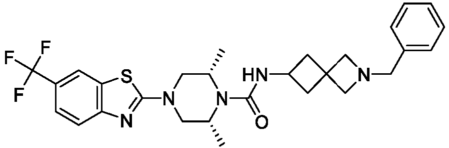 | (2R,6S)-N-{2-benzyl-2-azaspiro[3.3]heptan-6-yl}-2,6-dimethyl-4-[6-(trifluoromethyl)-1,3-benzothiazol-2-yl]piperazine-1-carboxamide |
| 217 | 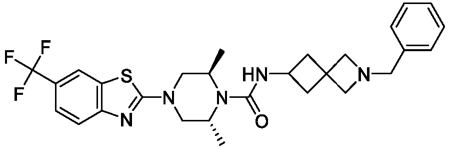 | (2R,6R)-N-{2-benzyl-2-azaspiro[3.3]heptan-6-yl}-2,6-dimethyl-4-[6-(trifluoromethyl)-1,3-benzothiazol-2-yl]piperazine-1-carboxamide |
| 218 | 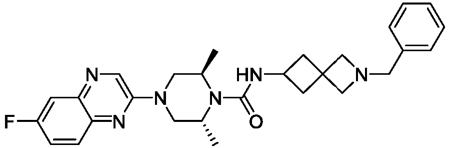 | (2R,6R)-N-{2-benzyl-2-azaspiro[3.3]heptan-6-yl}-4-(6-fluoroquinoxalin-2-yl)-2,6-dimethylpiperazine-1-carboxamide |
| 219 | 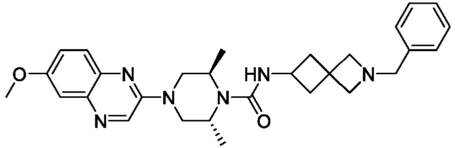 | (2R,6R)-N-{2-benzyl-2-azaspiro[3.3]heptan-6-yl}-4-(6-methoxyquinoxalin-2-yl)-2,6-dimethylpiperazine-1-carboxamide |
| 220 | 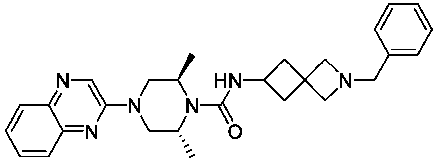 | (2R,6R)-N-{2-benzyl-2-azaspiro[3.3]heptan-6-yl}-2,6-dimethyl-4-(quinoxalin-2-yl)piperazine-1-carboxamide |
| 221 | 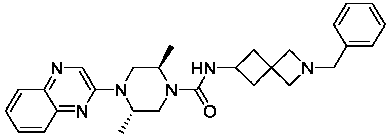 | (2R,5S)-N-{2-benzyl-2-azaspiro[3.3]heptan-6-yl}-2,5-dimethyl-4-(quinoxalin-2-yl)piperazine-1-carboxamide |
| 222 | 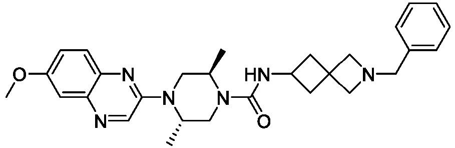 | (2R,5S)-N-{2-benzyl-2-azaspiro[3.3]heptan-6-yl}-4-(6-methoxyquinoxalin-2-yl)-2,5-dimethylpiperazine-1-carboxamide |
| 223 | 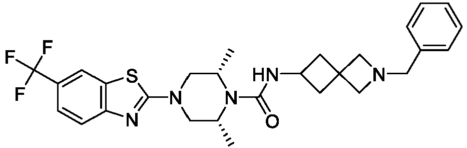 | (2R,6S)-N-{2-benzyl-2-azaspiro[3.3]heptan-6-yl}-2,6-dimethyl-4-[6-(trifluoromethyl)-1,3-benzothiazol-2-yl]piperazine-1-carboxamide |
| 224 | 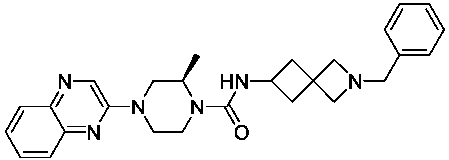 | (2R)-N-{2-benzyl-2-azaspiro[3.3]heptan-6-yl}-2-methyl-4-(quinoxalin-2-yl)piperazine-1-carboxamide |
| 225 | 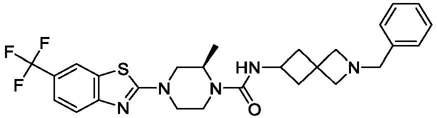 | (2R)-N-{2-benzyl-2-azaspiro[3.3]heptan-6-yl}-2-methyl-4-[6-(trifluoromethyl)-1,3-benzothiazol-2-yl]piperazine-1-carboxamide |
| 226 | 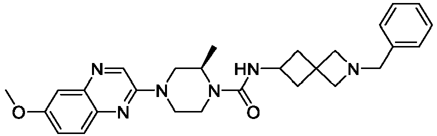 | (2R)-N-{2-benzyl-2-azaspiro[3.3]heptan-6-yl}-4-(6-methoxyquinoxalin-2-yl)-2-methylpiperazine-1-carboxamide |
| 227 | 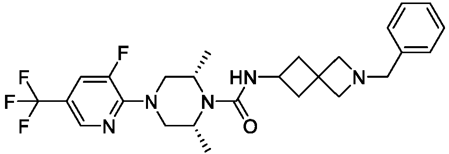 | (2R,6S)-N-{2-benzyl-2-azaspiro[3.3]heptan-6-yl}-4-[3-fluoro-5-(trifluoromethyl) pyridin-2-yl]-2,6-dimethylpiperazine-1-carboxamide |
| 228 | 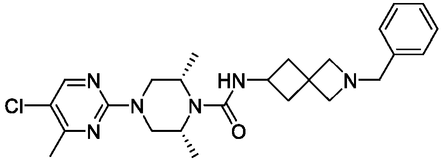 | (2R,6S)-N-{2-benzyl-2-azaspiro[3.3]heptan-6-yl}-4-(5-chloro-4-methylpyrimidin-2-yl)-2,6-dimethylpiperazine-1-carboxamide |
| 229 | 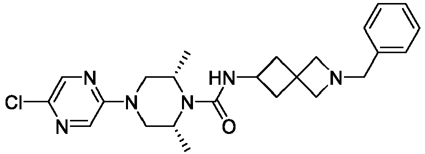 | (2R,6S)-N-{2-benzyl-2-azaspiro[3.3]heptan-6-yl}-4-(5-chloropyrazin-2-yl)-2,6-dimethylpiperazine-1-carboxamide |
| 230 | 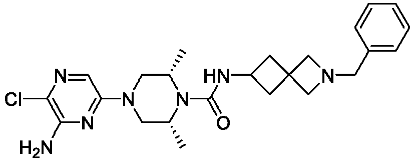 | (2R,6S)-4-(6-amino-5-chloropyrazin-2-yl)-N-{2-benzyl-2-azaspiro[3.3]heptan-6-yl}-2,6-dimethylpiperazine-1-carboxamide |
| 231 | 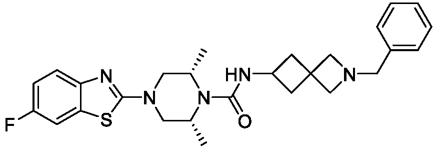 | (2R,6S)-N-{2-benzyl-2-azaspiro[3.3]heptan-6-yl}-4-(6-fluoro-1,3-benzothiazol-2-yl)-2,6-dimethylpiperazine-1-carboxamide |
| 232 | 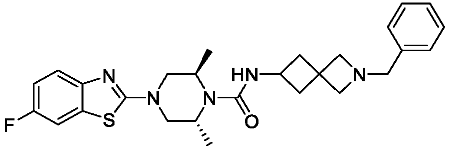 | (2R,6R)-N-{2-benzyl-2-azaspiro[3.3]heptan-6-yl}-4-(6-fluoro-1,3-benzothiazol-2-yl)-2,6-dimethylpiperazine-1-carboxamide |
| 233 | 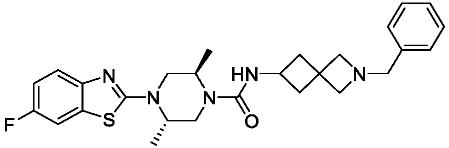 | (2R,5S)-N-{2-benzyl-2-azaspiro[3.3]heptan-6-yl}-4-(6-fluoro-1,3-benzothiazol-2-yl)-2,5-dimethylpiperazine-1-carboxamide |
| 234 | 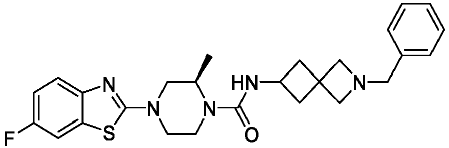 | (2R)-N-{2-benzyl-2-azaspiro[3.3]heptan-6-yl}-4-(6-fluoro-1,3-benzothiazol-2-yl)-2-methylpiperazine-1-carboxamide |
| 235 | 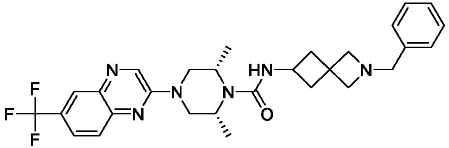 | (2R,6S)-N-{2-benzyl-2-azaspiro[3.3]heptan-6-yl}-2,6-dimethyl-4-[6-(trifluoromethyl)quinoxalin-2-yl]piperazine-1-carboxamide |
| 236 | 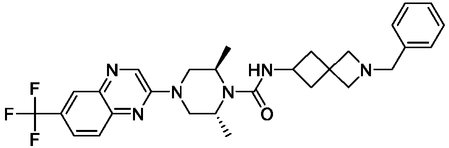 | (2R,6R)-N-{2-benzyl-2-azaspiro[3.3]heptan-6-yl}-2,6-dimethyl-4-[6-(trifluoromethyl)quinoxalin-2-yl]piperazine-1-carboxamide |
| 237 | 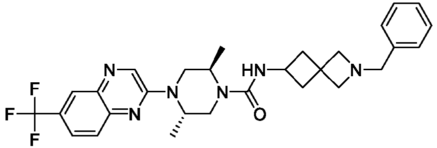 | (2R,5S)-N-{2-benzyl-2-azaspiro[3.3]heptan-6-yl}-2,5-dimethyl-4-[6-(trifluoromethyl)quinoxalin-2-yl]piperazine-1-carboxamide |
| 238 | 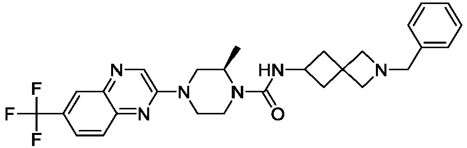 | (2R)-N-{2-benzyl-2-azaspiro[3.3]heptan-6-yl}-2-methyl-4-[6-(trifluoromethyl)quinoxalin-2-yl]piperazine-1-carboxamide |
| 239 | 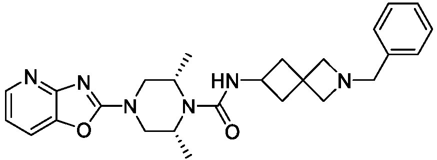 | (2R,6S)-N-{2-benzyl-2-azaspiro[3.3]heptan-6-yl}-2,6-dimethyl-4-{[1,3]oxazolo[4,5-b]pyridin-2-yl}piperazine-1-carboxamide |
| 240 | 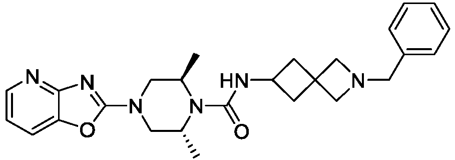 | (2R,6R)-N-{2-benzyl-2-azaspiro[3.3]heptan-6-yl}-2,6-dimethyl-4-{[1,3]oxazolo[4,5-b]pyridin-2-yl}piperazine-1-carboxamide |
| 241 | 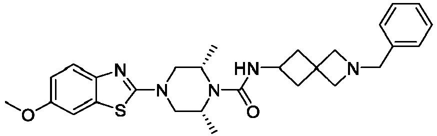 | (2R,6S)-N-{2-benzyl-2-azaspiro[3.3]heptan-6-yl}-4-(6-methoxy-1,3-benzothiazol-2-yl)-2,6-dimethylpiperazine-1-carboxamide |
| 242 | 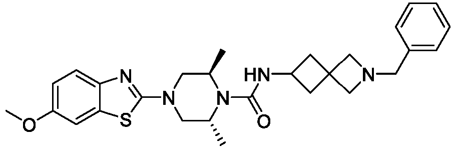 | (2R,6R)-N-{2-benzyl-2-azaspiro[3.3]heptan-6-yl}-4-(6-methoxy-1,3-benzothiazol-2-yl)-2,6-dimethylpiperazine-1-carboxamide |
| 243 | 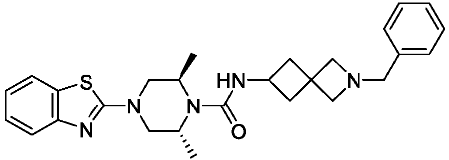 | (2R,6R)-4-(1,3-benzothiazol-2-yl)-N-{2-benzyl-2-azaspiro[3.3]heptan-6-yl}-2,6-dimethylpiperazine-1-carboxamide |
| 244 | 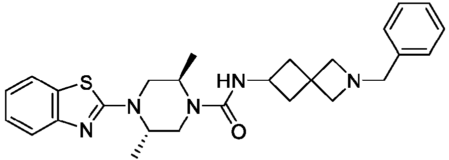 | (2R,5S)-4-(1,3-benzothiazol-2-yl)-N-{2-benzyl-2-azaspiro[3.3]heptan-6-yl}-2,5-dimethylpiperazine-1-carboxamide |
| 245 | 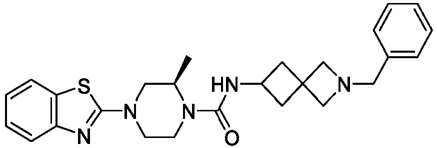 | (2R)-4-(1,3-benzothiazol-2-yl)-N-{2-benzyl-2-azaspiro[3.3]heptan-6-yl}-2-methylpiperazine-1-carboxamide |
| 246 | 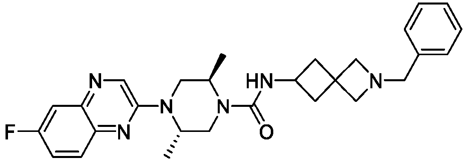 | (2R,5S)-N-{2-benzyl-2-azaspiro[3.3]heptan-6-yl}-4-(6-fluoroquinoxalin-2-yl)-2,5-dimethylpiperazine-1-carboxamide |
| 247 | 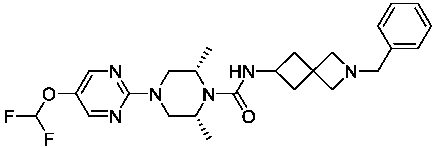 | (2R,6S)-N-{2-benzyl-2-azaspiro[3.3]heptan-6-yl}-4-[5-(difluoromethoxy)pyrimidin-2-yl]-2,6-dimethylpiperazine-1-carboxamide |
| 248 | 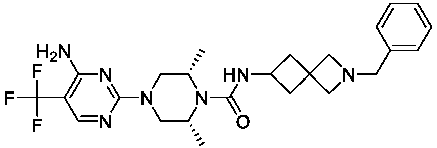 | (2R,6S)-4-[4-amino-5-(trifluoromethyl)pyrimidin-2-yl]-N-{2-benzyl-2-azaspiro[3.3]heptan-6-yl}-2,6-dimethylpiperazine-1-carboxamide |
| 249 | 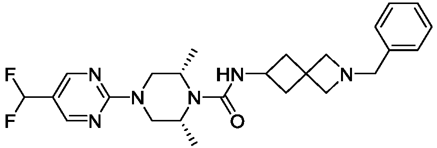 | (2R,6S)-N-{2-benzyl-2-azaspiro[3.3]heptan-6-yl}-4-[5-(difluoromethyl)pyrimidin-2-yl]-2,6-dimethylpiperazine-1-carboxamide |
| 250 | 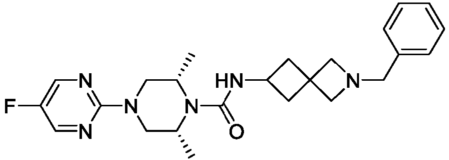 | (2R,6S)-N-{2-benzyl-2-azaspiro[3.3]heptan-6-yl}-4-(5-fluoropyrimidin-2-yl)-2,6-dimethylpiperazine-1-carboxamide |
| 251 | 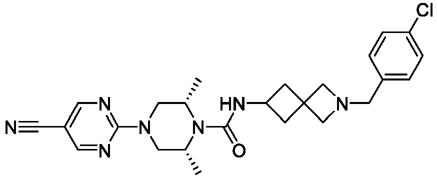 | (2R,6S)-N-{2-[(4-chlorophenyl)methyl]-2-azaspiro[3.3]heptan-6-yl}-4-(5-cyanopyrimidi n-2-yl)-2,6-dimethylpiperazine-1-carboxamide |
| 252 | 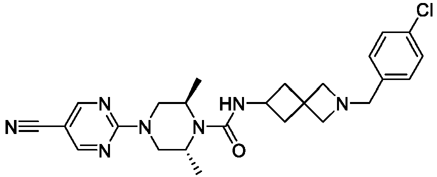 | (2R,6R)-N-{2-[(4-chlorophenyl)methyl]-2-azaspiro[3.3]heptan-6-yl}-4-(5-cyanopyrimidi n-2-yl)-2,6-dimethylpiperazine-1-carboxamide |
| 253 | 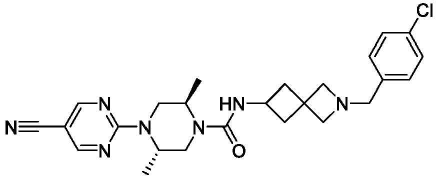 | (2R,5S)-N-{2-[(4-chlorophenyl)methyl]-2-azaspiro[3.3]heptan-6-yl}-4-(5-cyanopyrimidi n-2-yl)-2,5-dimethylpiperazine-1-carboxamide |
| 254 | 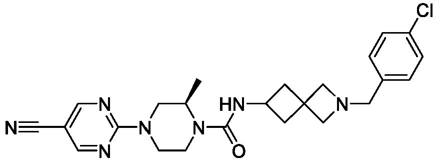 | (2R)-N-{2-[(4-chlorophenyl)methyl]-2-azaspiro[3.3]heptan-6-yl}-4-(5-cyanopyrimidi n-2-yl)-2-methylpiperazine-1-carboxamide |
| 255 | 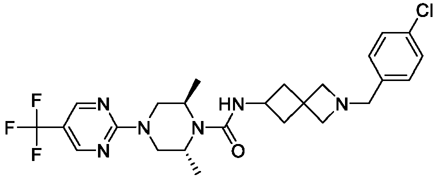 | (2R,6R)-N-{2-[(4-chlorophenyl)methyl]-2-azaspiro[3.3]heptan-6-yl}-2,6-dimethyl-4-[5-(trifluoromethyl)pyrimidin-2-yl]piperazine-1-carboxamide |
| 256 | 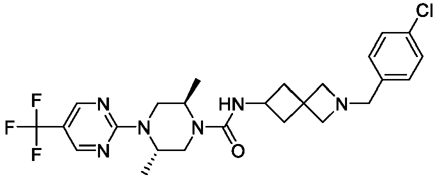 | (2R,5S)-N-{2-[(4-chlorophenyl)methyl]-2-azaspiro[3.3]heptan-6-yl}-2,5-dimethyl-4-[5-(trifluoromethyl)pyrimidin-2-yl]piperazine-1-carboxamide |
| 257 | 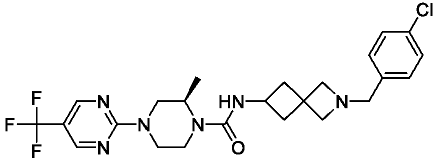 | (2R)-N-{2-[(4-chlorophenyl)methyl]-2-azaspiro[3.3]heptan-6-yl}-2-methyl-4-[5-(trifluoromethyl)pyrimidin-2-yl]piperazine-1-carboxamide |
| 258 | 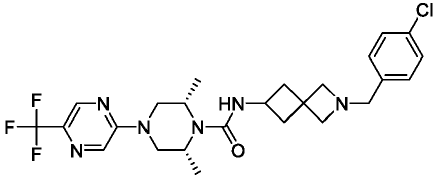 | (2R,6S)-N-{2-[(4-chlorophenyl)methyl]-2-azaspiro[3.3]heptan-6-yl}-2,6-dimethyl-4-[5-(trifluoromethyl)pyrazin-2-yl]piperazine-1-carboxamide |
| 259 | 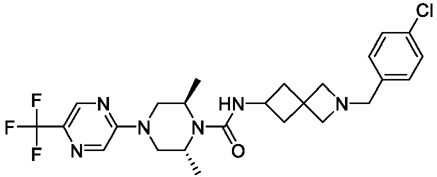 | (2R,6R)-N-{2-[(4-chlorophenyl)methyl]-2-azaspiro[3.3]heptan-6-yl}-2,6-dimethyl-4-[5-(trifluoromethyl)pyrazin-2-yl]piperazine-1-carboxamide |
| 260 | 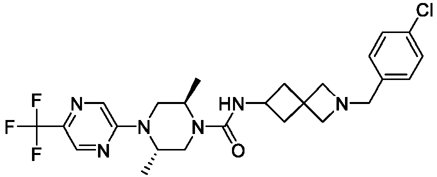 | (2R,5S)-N-{2-[(4-chlorophenyl)methyl]-2-azaspiro[3.3]heptan-6-yl}-2,5-dimethyl-4-[5-(trifluoromethyl)pyrazin-2-yl]piperazine-1-carboxamide |
| 261 | 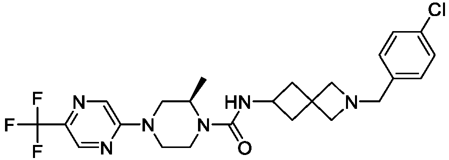 | (2R)-N-{2-[(4-chlorophenyl)methyl]-2-azaspiro[3.3]heptan-6-yl}-2-methyl-4-[5-(trifluoromethyl)pyrazin-2-yl]piperazine-1-carboxamide |
| 262 | 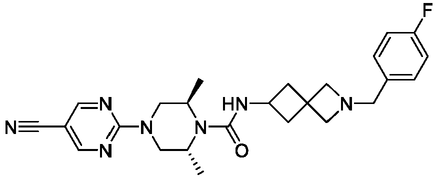 | (2R,6R)-4-(5-cyanopyrimidin-2-yl)-N-{2-[(4-fluorophenyl)methyl]-2-azaspiro[3.3]heptan-6-yl}-2,6-dimethylpiperazine-1-carboxamide |
| 263 | 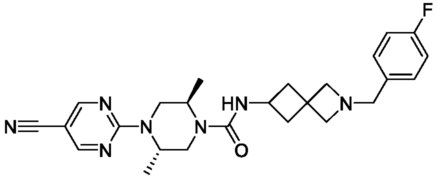 | (2R,5S)-4-(5-cyanopyrimidin-2-yl)-N-{2-[(4-fluorophenyl)methyl]-2-azaspiro[3.3]heptan-6-yl}-2,5-dimethylpiperazine-1-carboxamide |
| 264 | 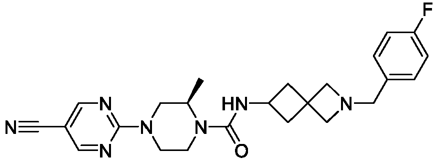 | (2R)-4-(5-cyanopyrimidin-2-yl)-N-{2-[(4-fluorophenyl)methyl]-2-azaspiro[3.3]heptan-6-yl}-2-methylpiperazine-1-carboxamide |
| 265 | 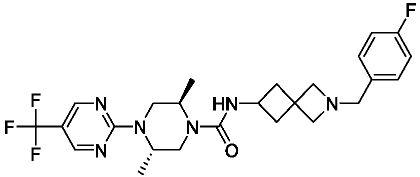 | (2R,5S)-N-{2-[(4-fluorophenyl)methyl]-2-azaspiro[3.3]heptan-6-yl}-2,5-dimethyl-4-[5-(trifluoromethyl)pyrimidin-2-yl]piperazine-1-carboxamide |
| 266 | 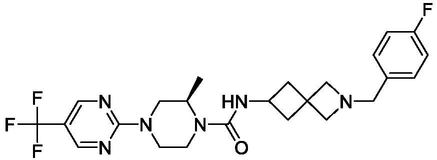 | (2R)-N-{2-[(4-fluorophenyl)methyl]-2-azaspiro[3.3]heptan-6-yl}-2-methyl-4-[5-(trifluoromethyl)pyrimidin-2-yl]piperazine-1-carboxamide |
| 267 | 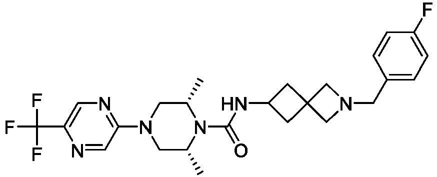 | (2R,6S)-N-{2-[(4-fluorophenyl)methyl]-2-azaspiro[3.3]heptan-6-yl}-2,6-dimethyl-4-[5-(trifluoromethyl)pyrazin-2-yl]piperazine-1-carboxamide |
| 268 | 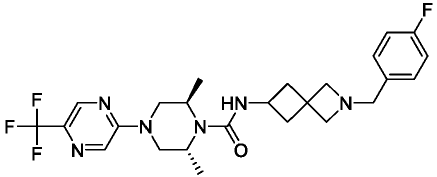 | (2R,6R)-N-{2-[(4-fluorophenyl)methyl]-2-azaspiro[3.3]heptan-6-yl}-2,6-dimethyl-4-[5-(trifluoromethyl)pyrazin-2-yl]piperazine-1-carboxamide |
| 269 | 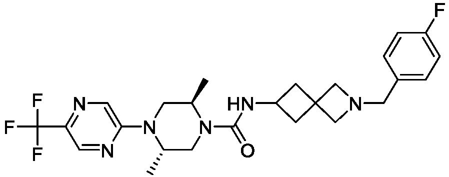 | (2R,5S)-N-{2-[(4-fluorophenyl)methyl]-2-azaspiro[3.3]heptan-6-yl}-2,5-dimethyl-4-[5-(trifluoromethyl)pyrazin-2-yl]piperazine-1-carboxamide |
| 270 | 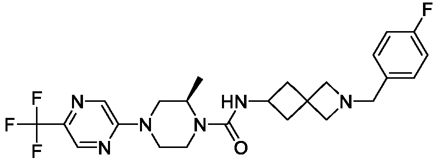 | (2R)-N-{2-[(4-fluorophenyl)methyl]-2-azaspiro[3.3]heptan-6-yl}-2-methyl-4-[5-(trifluoromethyl)pyrazin-2-yl]piperazine-1-carboxamide |
| 271 | 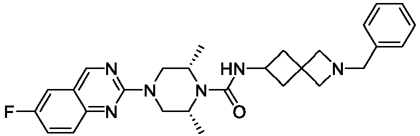 | (2R,6S)-N-{2-benzyl-2-azaspiro[3.3]heptan-6-yl}-4-(6-fluoroquinazolin-2-yl)-2,6-dimethylpiperazine-1-carboxamide |
| 272 | 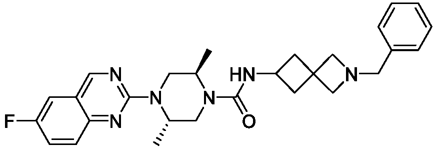 | (2R,5S)-N-{2-benzyl-2-azaspiro[3.3]heptan-6-yl}-4-(6-fluoroquinazolin-2-yl)-2,5-dimethylpiperazine-1-carboxamide |
| 273 | 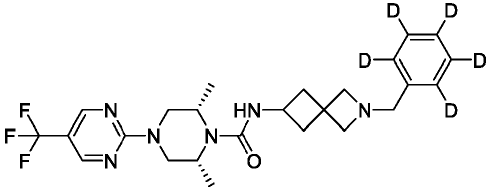 | (2R,6S)-N-{2-[(²H₅)benzyl]-2-azaspiro[3.3]heptan-6-yl}-2,6-dimethyl-4-[5-(trifluoromethyl)pyrimidin-2-yl]piperazine-1-carboxamide |
| 274 | 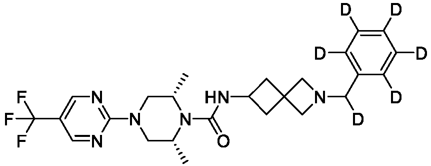 | (2R,6S)-2,6-dimethyl-N-{2-[(²H₅)phenyl(²H₁)methyl]-2-azaspiro[3.3]heptan-6-yl}-4-[5-(trifluoromethyl)pyrimidin-2-yl]piperazine-1-carboxamide |
| 275 | 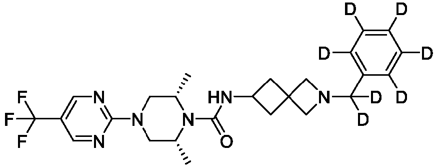 | (2R,6S)-2,6-dimethyl-N-{2-[(²H₅)phenyl(²H₂)methyl]-2-azaspiro[3.3]heptan-6-yl}-4-[5-(trifluoromethyl)pyrimidin-2-yl]piperazine-1-carboxamide |
| 276 | 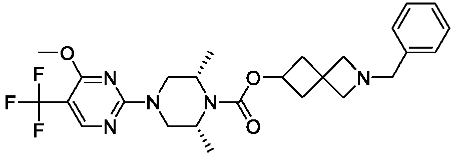 | 2-benzyl-2-azaspiro[3.3]heptan-6-yl (2R,6S)-4-[4-methoxy-5-(trifluoromethyl)pyrimidin-2-yl]-2,6-dimethylpiperazine-1-carboxylate |
| 277 | 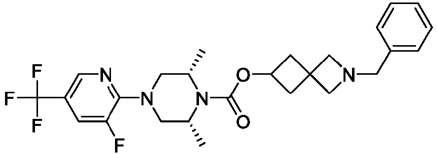 | 2-benzyl-2-azaspiro[3.3]heptan-6-yl (2R,6S)-4-[3-fluoro-5-(trifluoromethyl) pyridin-2-yl]-2,6-dimethylpiperazine-1-carboxylate |
| 278 | 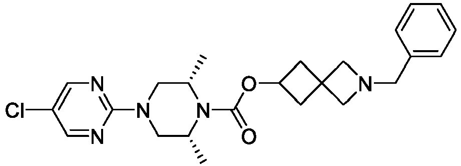 | 2-benzyl-2-azaspiro[3.3]heptan-6-yl (2R,6S)-4-(5-chloropyrimidin-2-yl)-2,6-dimethylpiperazine-1-carboxylate |
| 279 | 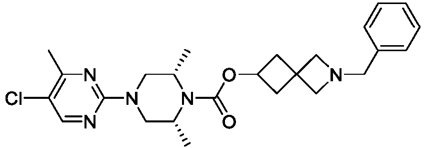 | 2-benzyl-2-azaspiro[3.3]heptan-6-yl (2R,6S)-4-(5-chloro-4-methylpyrimidin-2-yl)-2,6-dimethylpiperazine-1-carboxylate |
| 280 | 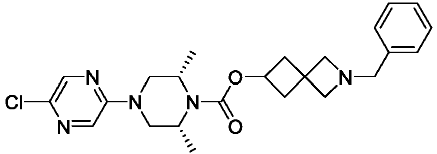 | 2-benzyl-2-azaspiro[3.3]heptan-6-yl (2R,6S)-4-(5-chloropyrazin-2-yl)-2,6-dimethylpiperazine-1-carboxylate |
| 281 | 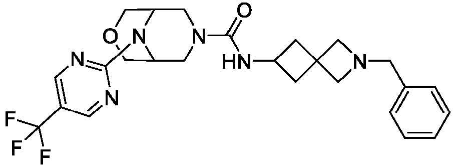 | N-{2-benzyl-2-azaspiro[3.3]heptan-6-yl}-9-[5-(trifluoromethyl)pyrimidin-2-yl]-3-oxa-7,9-diazabicyclo[3.3.1]nonane-7-carboxamide |
| 282 | 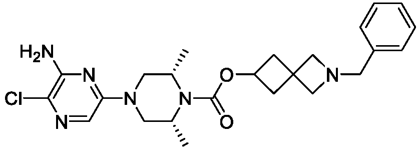 | 2-benzyl-2-azaspiro[3.3]heptan-6-yl (2R,6S)-4-(6-amino-5-chloropyrazin-2-yl)-2,6-dimethylpiperazine-1-carboxylate |
| 283 | 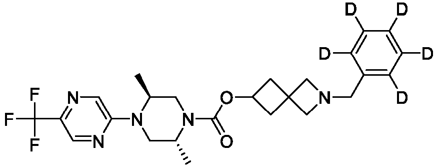 | 2-[(²H₅)benzyl]-2-azaspiro[3.3]heptan-6-yl (2R,5S)-2,5-dimethyl-4-[5-(trifluoromethyl)pyrazin-2-yl]piperazine-1-carboxylate |
| 284 | 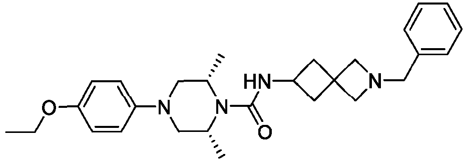 | (2R,6S)-N-{2-benzyl-2-azaspiro[3.3]heptan-6-yl}-4-(4-ethoxyphenyl)-2,6-dimethylpiperazine-1-carboxamide |
| 285 | 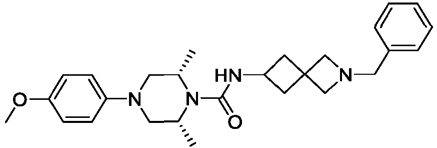 | (2R,6S)-N-{2-benzyl-2-azaspiro[3.3]heptan-6-yl}-4-(4-methoxyphenyl)-2,6-dimethylpiperazine-1-carboxamide |
| 286 | 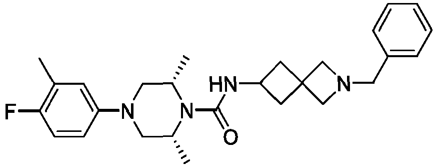 | (2R,6S)-N-{2-benzyl-2-azaspiro[3.3]heptan-6-yl}-4-(4-fluoro-3-methylphenyl)-2,6-dimethylpiperazine-1-carboxamide |
| 287 | 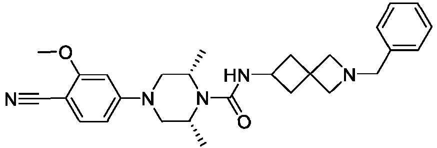 | (2R,6S)-N-{2-benzyl-2-azaspiro[3.3]heptan-6-yl}-4-(4-cyano-3-methoxyphenyl)-2,6-dimethylpiperazine-1-carboxamide |
| 288 | 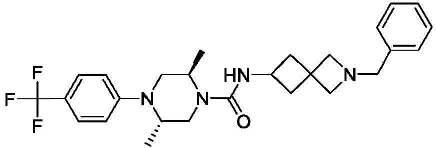 | (2R,5S)-N-{2-benzyl-2-azaspiro[3.3]heptan-6-yl}-2,5-dimethyl-4-[4-(trifluoromethyl)phenyl]piper azine-1-carboxamide |
| 289 | 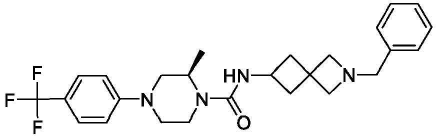 | (2R)-N-{2-benzyl-2-azaspiro[3.3]heptan-6-yl}-2-methyl-4-[4-(trifluoromethyl)phenyl]piper azine-1-carboxamide |
| 290 | 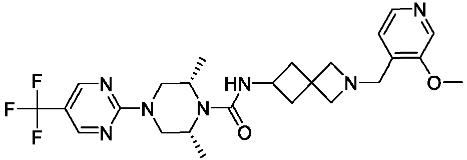 | (2R,6S)-N-{2-[(3-methoxypyridin-4-yl)methyl]-2-azaspiro[3.3]heptan-6-yl}-2,6-dimethyl-4-[5-(trifluoromethyl)pyrimidin-2-yl]piperazine-1-carboxamide |
| 291 | 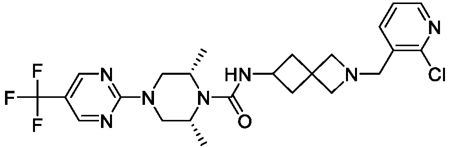 | (2R,6S)-N-{2-[(2-chloropyridin-3-yl)methyl]-2-azaspiro[3.3]heptan-6-yl}-2,6-dimethyl-4-[5-(trifluoromethyl)pyrimidin-2-yl]piperazine-1-carboxamide |
| 292 | 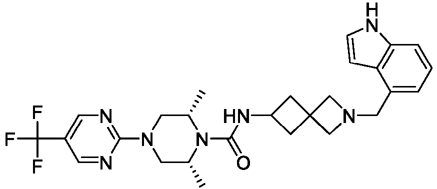 | (2R,6S)-N-[2-(1H-indol-4-ylmethyl)-2-azaspiro[3.3]heptan-6-yl]-2,6-dimethyl-4-[5-(trifluoromethyl)pyrimidin-2-yl]piperazine-1-carboxamide |
| 293 | 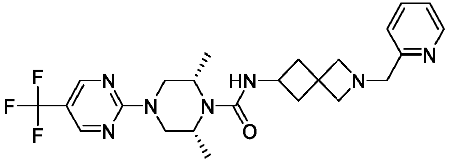 | (2R,6S)-2,6-dimethyl-N-[2-(pyridin-2-ylmethyl)-2-azaspiro[3.3]heptan-6-yl]-4-[5-(trifluoromethyl)pyrimidin-2-yl]piperazine-1-carboxamide |
| 294 | 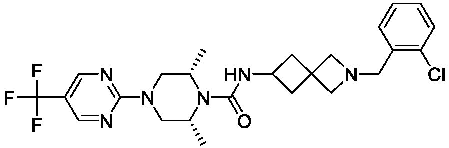 | (2R,6S)-N-{2-[(2-chlorophenyl)methyl]-2-azaspiro[3.3]heptan-6-yl}-2,6-dimethyl-4-[5-(trifluoromethyl)pyrimidin-2-yl]piperazine-1-carboxamide |
| 295 | 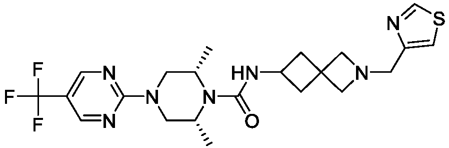 | (2R,6S)-2,6-dimethyl-N-[2-(1,3-thiazol-4-ylmethyl)-2-azaspiro[3.3]heptan-6-yl]-4-[5-(trifluoromethyl)pyrimidin-2-yl]piperazine-1-carboxamide |
| 296 | 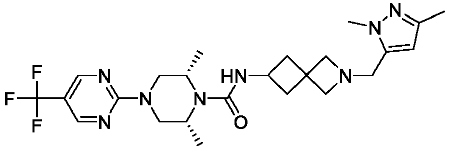 | (2R,6S)-N-{2-[(1,3-dimethyl-1H-pyrazol-5-yl)methyl]-2-azaspiro[3.3]heptan-6-yl}-2,6-dimethyl-4-[5-(trifluoromethyl)pyrimidin-2-yl]piperazine-1-carboxamide |
| 297 | 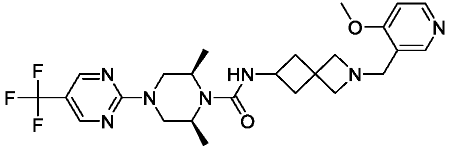 | (2R,6S)-N-{2-[(4-methoxypyridin-3-yl)methyl]-2-azaspiro[3.3]heptan-6-yl}-2,6-dimethyl-4-[5-(trifluoromethyl)pyrimidin-2-yl]piperazine-1-carboxamide |
| 298 | 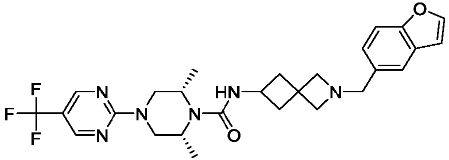 | (2R,6S)-N-[2-(1-benzofuran-5-ylmethyl)-2-azaspiro[3.3]heptan-6-yl]-2,6-dimethyl-4-[5-(trifluoromethyl)pyrimidin-2-yl]piperazine-1-carboxamide |
| 299 | 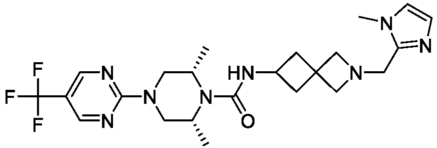 | (2R,6S)-2,6-dimethyl-N-{2-[(1-methyl-1H-imidazol-2-yl)methyl]-2-azaspiro[3.3]heptan-6-yl}-4-[5-(trifluoromethyl)pyrimidin-2-yl]piperazine-1-carboxamide |
| 300 | 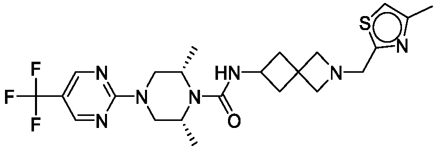 | (2R,6S)-2,6-dimethyl-N-{2-[(4-methyl-1,3-thiazol-2-yl)methyl]-2-azaspiro[3.3]heptan-6-yl}-4-[5-(trifluoromethyl)pyrimidin-2-yl]piperazine-1-carboxamide |
| 301 | 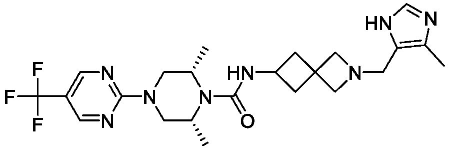 | (2R,6S)-2,6-dimethyl-N-{2-[(4-methyl-1H-imidazol-5-yl)methyl]-2-azaspiro[3.3]heptan-6-yl}-4-[5-(trifluoromethyl)pyrimidin-2-yl]piperazine-1-carboxamide |
| 302 | 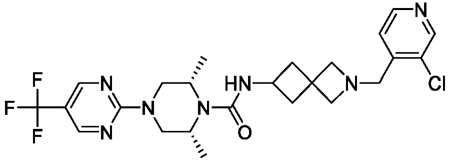 | (2R,6S)-N-{2-[(3-chloropyridin-4-yl)methyl]-2-azaspiro[3.3]heptan-6-yl}-2,6-dimethyl-4-[5-(trifluoromethyl)pyrimidin-2-yl]piperazine-1-carboxamide |
| 303 | 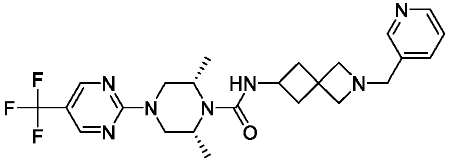 | (2R,6S)-2,6-dimethyl-N-[2-(pyridin-3-ylmethyl)-2-azaspiro[3.3]heptan-6-yl]-4-[5-(trifluoromethyl)pyrimidin-2-yl]piperazine-1-carboxamide |
| 304 | 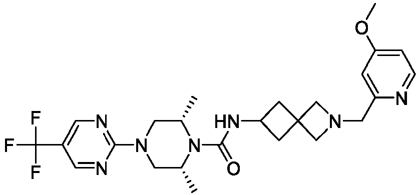 | (2R,6S)-N-{2-[(4-methoxypyridin-2-yl)methyl]-2-azaspiro[3.3]heptan-6-yl}-2,6-dimethyl-4-[5-(trifluoromethyl)pyrimidin-2-yl]piperazine-1-carboxamide |
| 305 | 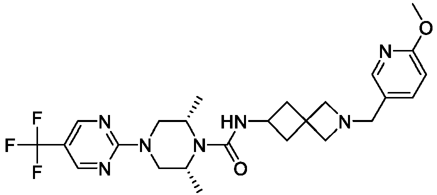 | (2R,6S)-N-{2-[(6-methoxypyridin-3-yl)methyl]-2-azaspiro[3.3]heptan-6-yl}-2,6-dimethyl-4-[5-(trifluoromethyl)pyrimidin-2-yl]piperazine-1-carboxamide |
| 306 | 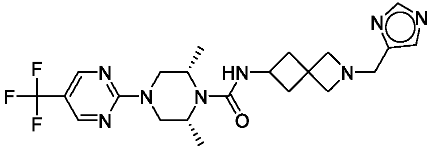 | (2R,6S)-N-[2-(1H-imidazol-4-ylmethyl)-2-azaspiro[3.3]heptan-6-yl]-2,6-dimethyl-4-[5-(trifluoromethyl)pyrimidin-2-yl]piperazine-1-carboxamide |
| 307 | 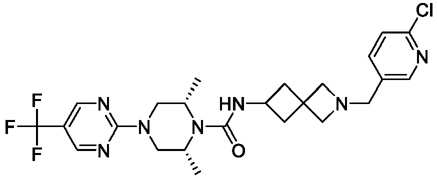 | (2R,6S)-N-{2-[(6-chloropyridin-3-yl)methyl]-2-azaspiro[3.3]heptan-6-yl}-2,6-dimethyl-4-[5-(trifluoromethyl)pyrimidin-2-yl]piperazine-1-carboxamide |
| 308 | 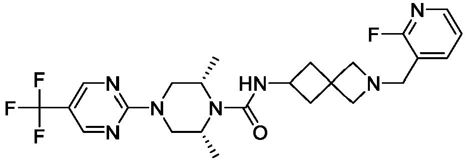 | (2R,6S)-N-{2-[(2-fluoropyridin-3-yl)methyl]-2-azaspiro[3.3]heptan-6-yl}-2,6-dimethyl-4-[5-(trifluoromethyl)pyrimidin-2-yl]piperazine-1-carboxamide |
| 309 | 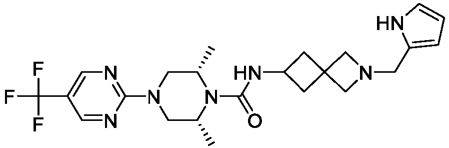 | (2R,6S)-2,6-dimethyl-N-[2-(1H-pyrrol-2-ylmethyl)-2-azaspiro[3.3]heptan-6-yl]-4-[5-(trifluoromethyl)pyrimidin-2-yl]piperazine-1-carboxamide |
| 310 | 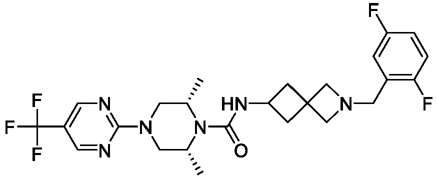 | (2R,6S)-N-{2-[(2,5-difluorophenyl)methyl]-2-azaspiro[3.3]heptan-6-yl}-2,6-dimethyl-4-[5-(trifluoromethyl)pyrimidin-2-yl]piperazine-1-carboxamide |
| 311 | 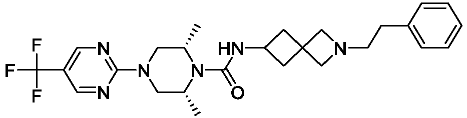 | (2R,6S)-2,6-dimethyl-N-[2-(2-phenylethyl)-2-azaspiro[3.3]heptan-6-yl]-4-[5-(trifluoromethyl)pyrimidin-2-yl]piperazine-1-carboxamide |
| 312 | 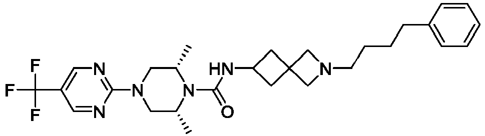 | (2R,6S)-2,6-dimethyl-N-[2-(4-phenylbutyl)-2-azaspiro[3.3]heptan-6-yl]-4-[5-(trifluoromethyl)pyrimidin-2-yl]piperazine-1-carboxamide |
| 313 | 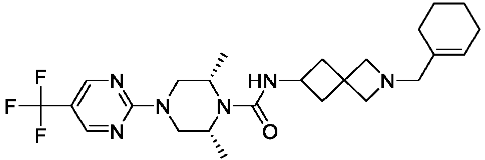 | (2R,6S)-N-[2-(cyclohex-1-en-1-ylmethyl)-2-azaspiro[3.3]heptan-6-yl]-2,6-dimethyl-4-[5-(trifluoromethyl)pyrimidin-2-yl]piperazine-1-carboxamide |
| 314 | 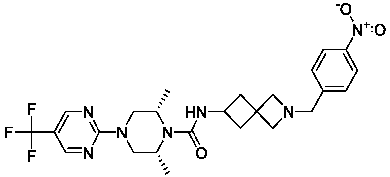 | (2R,6S)-2,6-dimethyl-N-{2-[(4-nitrophenyl)methyl]-2-azaspiro[3.3]heptan-6-yl}-4-[5-(trifluoromethyl)pyrimidin-2-yl]piperazine-1-carboxamide |
| 315 | 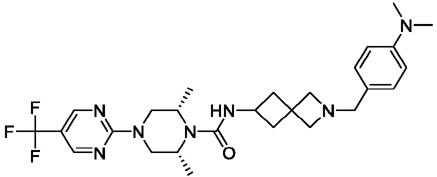 | (2R,6S)-N-(2-{[4-(dimethylamino)phenyl]meth yl}-2-azaspiro[3.3]heptan-6-yl)-2,6-dimethyl-4-[5-(trifluoromethyl)pyrimidin-2-yl]piperazine-1-carboxamide |
| 316 | 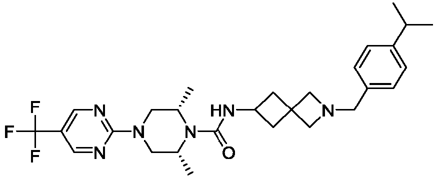 | (2R,6S)-2,6-dimethyl-N-(2-{[4-(propan-2-yl)phenyl]methyl}-2-azaspiro[3.3]heptan-6-yl)-4-[5-(trifluoromethyl)pyrimidin-2-yl]piperazine-1-carboxamide |
| 317 | 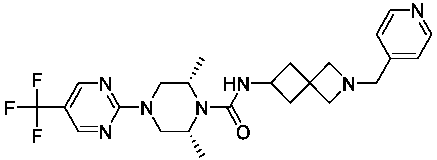 | (2R,6S)-2,6-dimethyl-N-[2-(pyridin-4-ylmethyl)-2-azaspiro[3.3]heptan-6-yl]-4-[5-(trifluoromethyl)pyrimidin-2-yl]piperazine-1-carboxamide |
| 318 | 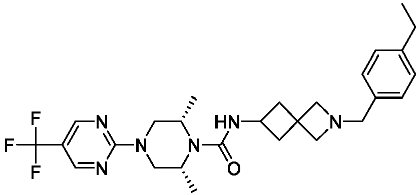 | (2R,6S)-N-{2-[(4-ethylphenyl)methyl]-2-azaspiro[3.3]heptan-6-yl}-2,6-dimethyl-4-[5-(trifluoromethyl)pyrimidin-2-yl]piperazine-1-carboxamide |
| 319 | 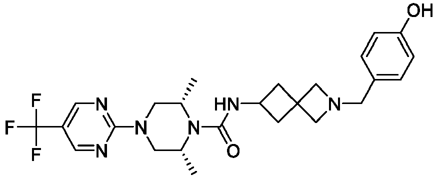 | (2R,6S)-N-{2-[(4-hydroxyphenyl)methyl]-2-azaspiro[3.3]heptan-6-yl}-2,6-dimethyl-4-[5-(trifluoromethyl)pyrimidin-2-yl]piperazine-1-carboxamide |
| 320 | 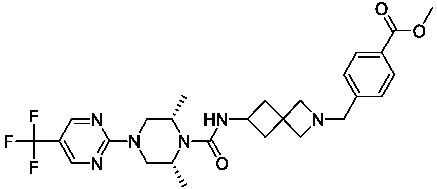 | methyl 4-[(6-{[(2R,6S)-2,6-dimethyl-4-[5-(trifluoromethyl)pyrimidin-2-yl]piperazine-1-carbonyl]amino}-2-azaspiro[3.3]heptan-2-yl)methyl]benzoate |
| 321 | 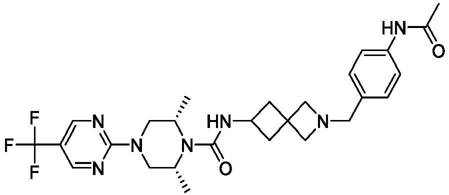 | (2R,6S)-N-{2-[(4-acetamidophenyl)methyl]-2-azaspiro[3.3]heptan-6-yl}-2,6-dimethyl-4-[5-(trifluoromethyl)pyrimidin-2-yl]piperazine-1-carboxamide |
| 322 | 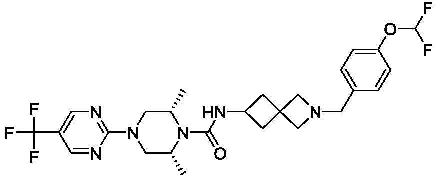 | (2R,6S)-N-(2-{[4-(difluoromethoxy)phenyl]met hyl}-2-azaspiro[3.3]heptan-6-yl)-2,6-dimethyl-4-[5-(trifluoromethyl)pyrimidin-2-yl]piperazine-1-carboxamide |
| 323 | 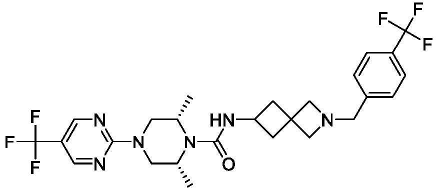 | (2R,6S)-2,6-dimethyl-N-(2-{[4-(trifluoromethyl)phenyl]meth yl}-2-azaspiro[3.3]heptan-6-yl)-4-[5-(trifluoromethyl)pyrimidin-2-yl]piperazine-1-carboxamide |
| 324 | 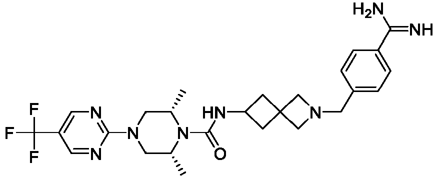 | (2R,6S)-N-{2-[(4-carbamimidoylphenyl)methyl ]-2-azaspiro[3.3]heptan-6-yl}-2,6-dimethyl-4-[5-(trifluoromethyl)pyrimidin-2-yl]piperazine-1-carboxamide |
| 325 | 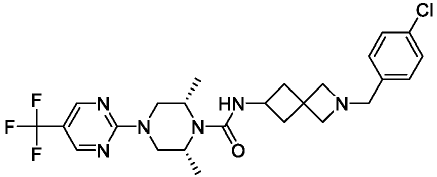 | (2R,6S)-N-{2-[(4-chlorophenyl)methyl]-2-azaspiro[3.3]heptan-6-yl}-2,6-dimethyl-4-[5-(trifluoromethyl)pyrimidin-2-yl]piperazine-1-carboxamide |
| 326 | 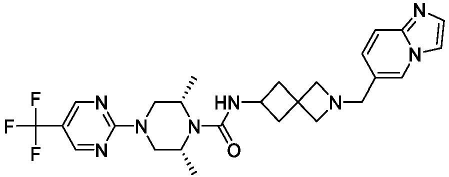 | (2R,6S)-N-(2-{imidazo[1,2-a]pyridin-6-ylmethyl}-2-azaspiro[3.3]heptan-6-yl)-2,6-dimethyl-4-[5-(trifluoromethyl)pyrimidin-2-yl]piperazine-1-carboxamide |
| 327 | 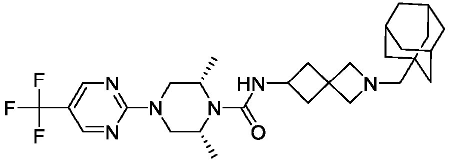 | (2R,6S)-N-[2-(adamantan-1-ylmethyl)-2-azaspiro[3.3]heptan-6-yl]-2,6-dimethyl-4-[5-(trifluoromethyl)pyrimidin-2-yl]piperazine-1-carboxamide |
| 328 | 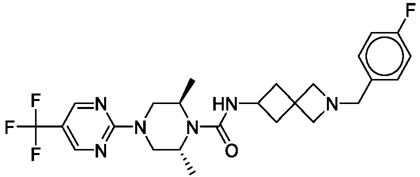 | (2R,6R)-N-{2-[(4-fluorophenyl)methyl]-2-azaspiro[3.3]heptan-6-yl}-2,6-dimethyl-4-[5-(trifluoromethyl)pyrimidin-2-yl]piperazine-1-carboxamide |
| 329 | 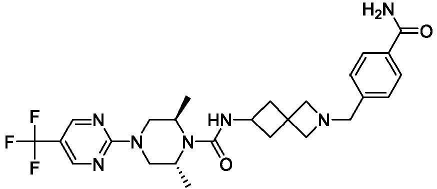 | (2R,6R)-N-{2-[(4-carbamoylphenyl)methyl]-2-azaspiro[3.3]heptan-6-yl}-2,6-dimethyl-4-[5-(trifluoromethyl)pyrimidin-2-yl]piperazine-1-carboxamide |
| 330 | 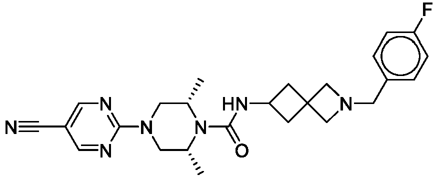 | (2R,6S)-4-(5-cyanopyrimidin-2-yl)-N-{2-[(4-fluorophenyl)methyl]-2-azaspiro[3.3]heptan-6-yl}-2,6-dimethylpiperazine-1-carboxamide |
| 331 | 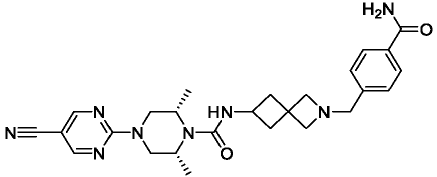 | (2R,6S)-N-{2-[(4-carbamoylphenyl)methyl]-2-azaspiro[3.3]heptan-6-yl}-4-(5-cyanopyrimidi n-2-yl)-2,6-dimethylpiperazine-1-carboxamide |
| 332 | 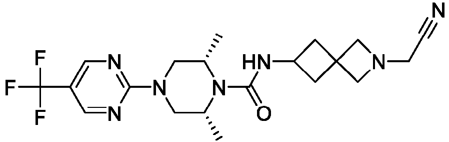 | (2R,6S)-N-[2-(cyanomethyl)-2-azaspiro[3.3]heptan-6-yl]-2,6-dimethyl-4-[5-(trifluoromethyl)pyrimidin-2-yl]piperazine-1-carboxamide |
| 333 | 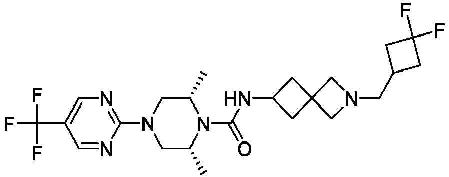 | (2R,6S)-N-{2-[(3,3-difluorocyclobutyl)methyl]-2-azaspiro[3.3]heptan-6-yl}-2,6-dimethyl-4-[5-(trifluoromethyl)pyrimidin-2-yl]piperazine-1-carboxamide |
| 334 | 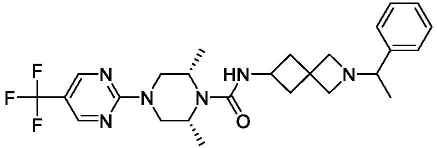 | (2R,6S)-2,6-dimethyl-N-[2-(1-phenylethyl)-2-azaspiro[3.3]heptan-6-yl]-4-[5-(trifluoromethyl)pyrimidin-2-yl]piperazine-1-carboxamide |
| 335 | 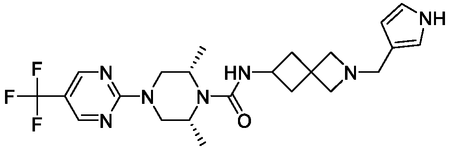 | (2R,6S)-2,6-dimethyl-N-[2-(1 H-pyrrol-3-ylmethyl)-2-azaspiro[3.3]heptan-6-yl]-4-[5-(trifluoromethyl)pyrimidin-2-yl]piperazine-1-carboxamide |
| 336 | 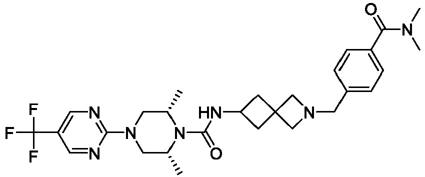 | (2R,6S)-N-(2-{[4-(dimethylcarbamoyl)phenyl] methyl}-2-azaspiro[3.3]heptan-6-yl)-2,6-dimethyl-4-[5-(trifluoromethyl)pyrimidin-2-yl]piperazine-1-carboxamide |
| 337 | 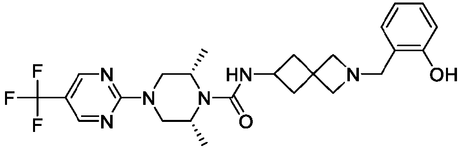 | (2R,6S)-N-{2-[(2-hydroxyphenyl)methyl]-2-azaspiro[3.3]heptan-6-yl}-2,6-dimethyl-4-[5-(trifluoromethyl)pyrimidin-2-yl]piperazine-1-carboxamide |
| 338 | 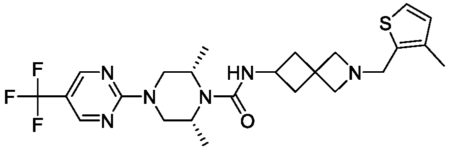 | (2R,6S)-2,6-dimethyl-N-{2-[(3-methylthiophen-2-yl)methyl]-2-azaspiro[3.3]heptan-6-yl}-4-[5-(trifluoromethyl)pyrimidin-2-yl]piperazine-1-carboxamide |
| 339 | 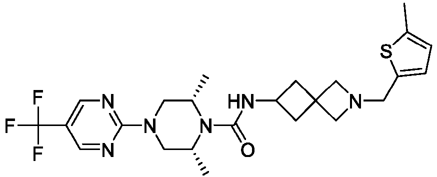 | (2R,6S)-2,6-dimethyl-N-{2-[(5-methylthiophen-2-yl)methyl]-2-azaspiro[3.3]heptan-6-yl}-4-[5-(trifluoromethyl)pyrimidin-2-yl]piperazine-1-carboxamide |
| 340 | 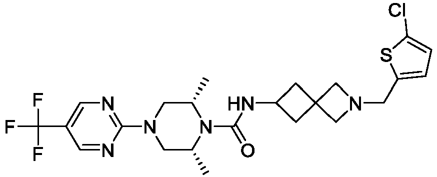 | (2R,6S)-N-{2-[(5-chlorothiophen-2-yl)methyl]-2-azaspiro[3.3]heptan-6-yl}-2,6-dimethyl-4-[5-(trifluoromethyl)pyrimidin-2-yl]piperazine-1-carboxamide |
| 341 | 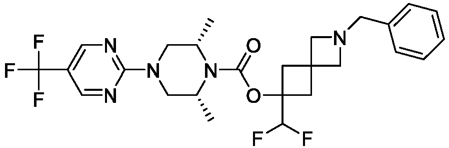 | 2-benzyl-6-(difluoromethyl)-2-azaspiro[3.3]heptan-6-yl (2R,6S)-2,6-dimethyl-4-[5-(trifluoromethyl)pyrimidin-2-yl]piperazine-1-carboxylate |
| 342 | 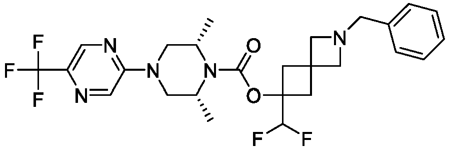 | 2-benzyl-6-(difluoromethyl)-2-azaspiro[3.3]heptan-6-yl (2R,6S)-2,6-dimethyl-4-[5-(trifluoromethyl)pyrazin-2-yl]piperazine-1-carboxylate |
| 343 | 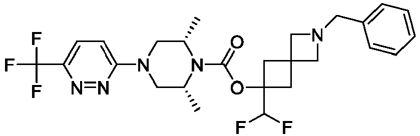 | 2-benzyl-6-(difluoromethyl)-2-azaspiro[3.3]heptan-6-yl (2R,6S)-2,6-dimethyl-4-[6-(trifluoromethyl)pyridazin-3-yl]piperazine-1-carboxylate |
| 344 | 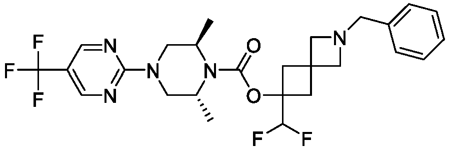 | 2-benzyl-6-(difluoromethyl)-2-azaspiro[3.3]heptan-6-yl (2R,6R)-2,6-dimethyl-4-[5-(trifluoromethyl)pyrimidin-2-yl]piperazine-1-carboxylate |
| 345 | 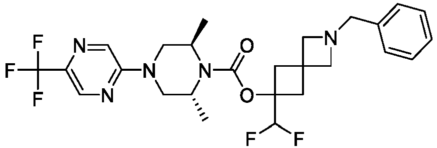 | 2-benzyl-6-(difluoromethyl)-2-azaspiro[3.3]heptan-6-yl (2R,6R)-2,6-dimethyl-4-[5-(trifluoromethyl)pyrazin-2-yl]piperazine-1-carboxylate |
| 346 | 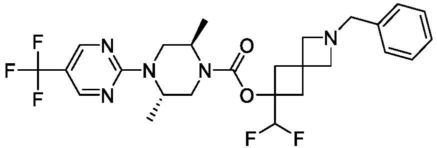 | 2-benzyl-6-(difluoromethyl)-2-azaspiro[3.3]heptan-6-yl (2R,5S)-2,5-dimethyl-4-[5-(trifluoromethyl)pyrimidin-2-yl]piperazine-1-carboxylate |
| 347 | 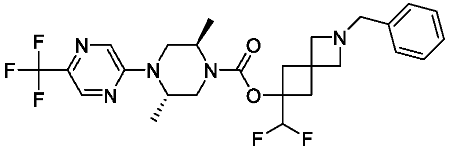 | 2-benzyl-6-(difluoromethyl)-2-azaspiro[3.3]heptan-6-yl (2R,5S)-2,5-dimethyl-4-[5-(trifluoromethyl)pyrazin-2-yl]piperazine-1-carboxylate |
| 348 | 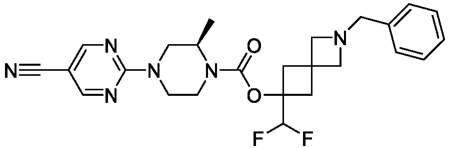 | 2-benzyl-6-(difluoromethyl)-2-azaspiro[3.3]heptan-6-yl (2R)-4-(5-cyanopyrimidin-2-yl)-2-methylpiperazine-1-carboxylate |
| 349 | 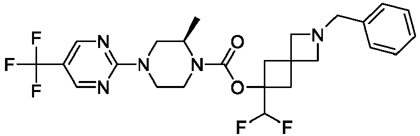 | 2-benzyl-6-(difluoromethyl)-2-azaspiro[3.3]heptan-6-yl (2R)-2-methyl-4-[5-(trifluoromethyl)pyrimidin-2-yl]piperazine-1-carboxylate |
| 350 | 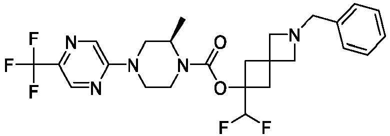 | 2-benzyl-6-(difluoromethyl)-2-azaspiro[3.3]heptan-6-yl (2R)-2-methyl-4-[5-(trifluoromethyl)pyrazin-2-yl]piperazine-1-carboxylate |
| 351 | 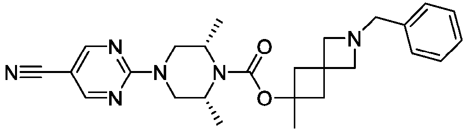 | 2-benzyl-6-methyl-2-azaspiro[3.3]heptan-6-yl (2R,6S)-4-(5-cyanopyrimidin-2-yl)-2,6-dimethylpiperazine-1-carboxylate |
| 352 | 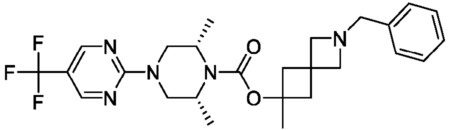 | 2-benzyl-6-methyl-2-azaspiro[3.3]heptan-6-yl (2R,6S)-2,6-dimethyl-4-[5-(trifluoromethyl)pyrimidin-2-yl]piperazine-1-carboxylate |
| 353 | 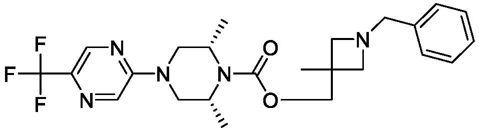 | 2-benzyl-6-methyl-2-azaspiro[3.3]heptan-6-yl (2R,6S)-2,6-dimethyl-4-[5-(trifluoromethyl)pyrazin-2-yl]piperazine-1-carboxylate |
| 354 | 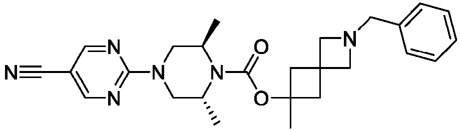 | 2-benzyl-6-methyl-2-azaspiro[3.3]heptan-6-yl (2R,6R)-4-(5-cyanopyrimidin-2-yl)-2,6-dimethylpiperazine-1-carboxylate |
| 355 | 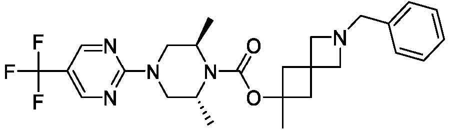 | 2-benzyl-6-methyl-2-azaspiro[3.3]heptan-6-yl (2R,6R)-2,6-dimethyl-4-[5-(trifluoromethyl)pyrimidin-2-yl]piperazine-1-carboxylate |
| 356 | 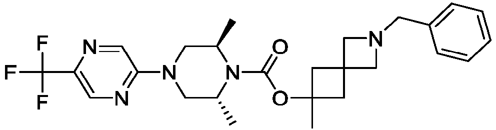 | 2-benzyl-6-methyl-2-azaspiro[3.3]heptan-6-yl (2R,6R)-2,6-dimethyl-4-[5-(trifluoromethyl)pyrazin-2-yl]piperazine-1-carboxylate |
| 357 | 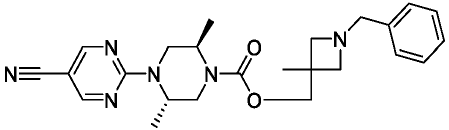 | 2-benzyl-6-methyl-2-azaspiro[3.3]heptan-6-yl (2R,5S)-4-(5-cyanopyrimidin-2-yl)-2,5-dimethylpiperazine-1-carboxylate |
| 358 | 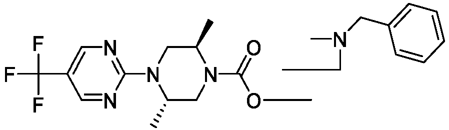 | 2-benzyl-6-methyl-2-azaspiro[3.3]heptan-6-yl (2R,5S)-2,5-dimethyl-4-[5-(trifluoromethyl)pyrimidin-2-yl]piperazine-1-carboxylate |
| 359 | 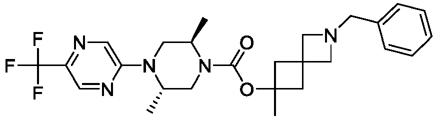 | 2-benzyl-6-methyl-2-azaspiro[3.3]heptan-6-yl (2R,5S)-2,5-dimethyl-4-[5-(trifluoromethyl)pyrazin-2-yl]piperazine-1-carboxylate |
| 360 | 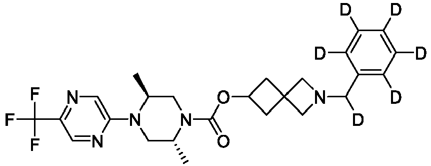 | 2-[(²H₅)phenyl(²H₁)methyl]-2-azaspiro[3.3]heptan-6-yl (2R,5S)-2,5-dimethyl-4-[5-(trifluoromethyl)pyrazin-2-yl]piperazine-1-carboxylate |
| 361 | 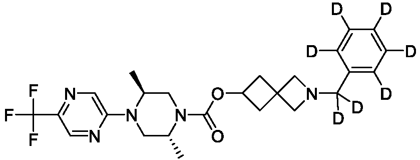 | 2-[(²H₅)phenyl(²H₂)methyl]-2-azaspiro[3.3]heptan-6-yl (2R,5S)-2,5-dimethyl-4-[5-(trifluoromethyl)pyrazin-2-yl]piperazine-1-carboxylate |
| 362 | 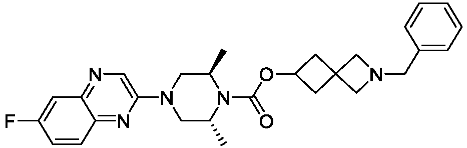 | 2-benzyl-2-azaspiro[3.3]heptan-6-yl (2R,6R)-4-(6-fluoroquinoxalin-2-yl)-2,6-dimethylpiperazine-1-carboxylate |
| 363 | 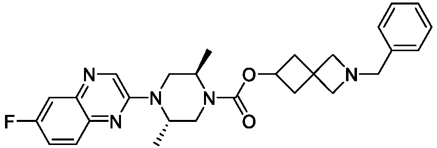 | 2-benzyl-2-azaspiro[3.3]heptan-6-yl (2R,5S)-4-(6-fluoroquinoxalin-2-yl)-2,5-dimethylpiperazine-1-carboxylate |
| 364 | 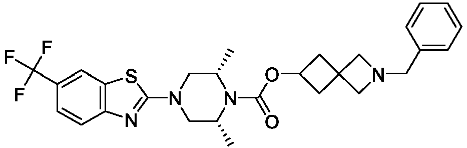 | 2-benzyl-2-azaspiro[3.3]heptan-6-yl (2R,6S)-2,6-dimethyl-4-[6-(trifluoromethyl)-1,3-benzothiazol-2-yl]piperazine-1-carboxylate |
| 365 | 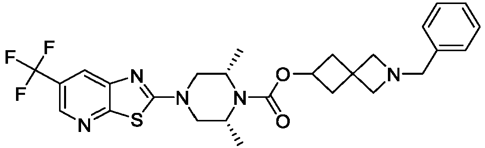 | 2-benzyl-2-azaspiro[3.3]heptan-6-yl (2R,6S)-2,6-dimethyl-4-[6-(trifluoromethyl)-[1,3]thiazolo[5,4-b]pyridin-2-yl]piperazine-1-carboxylate |
| 366 | 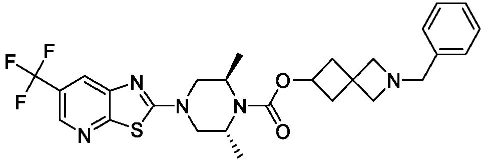 | 2-benzyl-2-azaspiro[3.3]heptan-6-yl (2R,6R)-2,6-dimethyl-4-[6-(trifluoromethyl)-[1,3]thiazolo[5,4-b]pyridin-2-yl]piperazine-1-carboxylate |
| 367 | 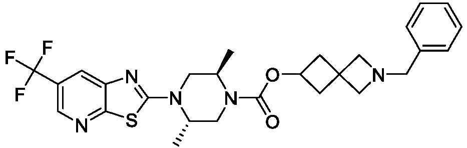 | 2-benzyl-2-azaspiro[3.3]heptan-6-yl (2R,5S)-2,5-dimethyl-4-[6-(trifluoromethyl)-[1,3]thiazolo[5,4-b]pyridin-2-yl]piperazine-1-carboxylate |
| 368 | 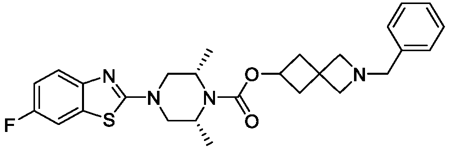 | 2-benzyl-2-azaspiro[3.3]heptan-6-yl (2R,6S)-4-(6-fluoro-1,3-benzothiazol-2-yl)-2,6-dimethylpiperazine-1-carboxylate |
| 369 | 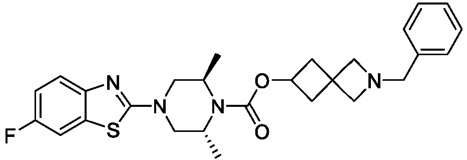 | 2-benzyl-2-azaspiro[3.3]heptan-6-yl (2R,6R)-4-(6-fluoro-1,3-benzothiazol-2-yl)-2,6-dimethylpiperazine-1-carboxylate |
| 370 | 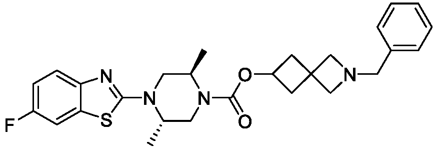 | 2-benzyl-2-azaspiro[3.3]heptan-6-yl (2R,5S)-4-(6-fluoro-1,3-benzothiazol-2-yl)-2,5-dimethylpiperazine-1-carboxylate |
| 371 | 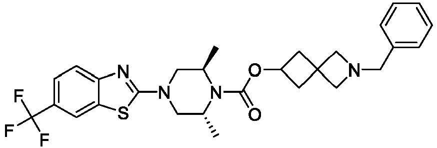 | 2-benzyl-2-azaspiro[3.3]heptan-6-yl (2R,6R)-2,6-dimethyl-4-[6-(trifluoromethyl)-1,3-benzothiazol-2-yl]piperazine-1-carboxylate |
| 372 | 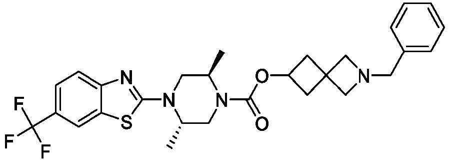 | 2-benzyl-2-azaspiro[3.3]heptan-6-yl (2R,5S)-2,5-dimethyl-4-[6-(trifluoromethyl)-1,3-benzothiazol-2-yl]piperazine-1-carboxylate |
| 373 | 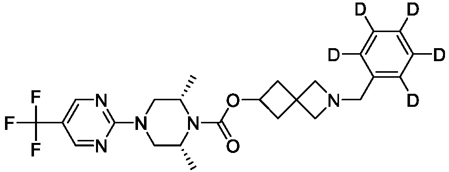 | 2-[(²H₅)benzyl]-2-azaspiro[3.3]heptan-6-yl (2R,6S)-2,6-dimethyl-4-[5-(trifluoromethyl)pyrimidin-2-yl]piperazine-1-carboxylate |
| 374 | 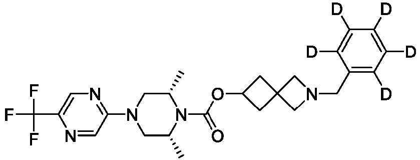 | 2-[(²H₅)benzyl]-2-azaspiro[3.3]heptan-6-yl (2R,6S)-2,6-dimethyl-4-[5-(trifluoromethyl)pyrazin-2-yl]piperazine-1-carboxylate |
| 375 | 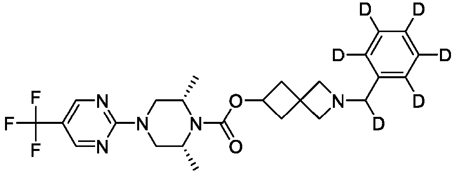 | 2-[(²H₅)phenyl(²H₁)methyl]-2-azaspiro[3.3]heptan-6-yl (2R,6S)-2,6-dimethyl-4-[5-(trifluoromethyl)pyrimidin-2-yl]piperazine-1-carboxylate |
| 376 | 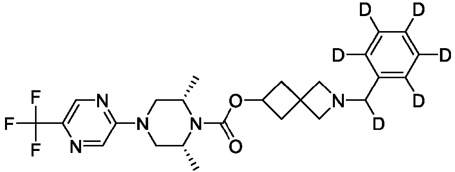 | 2-[(²H₅)phenyl(²H₁)methyl]-2-azaspiro[3.3]heptan-6-yl (2R,6S)-2,6-dimethyl-4-[5-(trifluoromethyl)pyrazin-2-yl]piperazine-1-carboxylate |
| 377 | 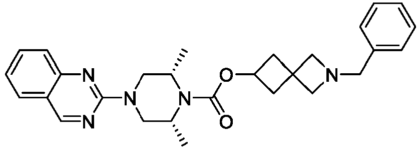 | 2-benzyl-2-azaspiro[3.3]heptan-6-yl (2R,6S)-2,6-dimethyl-4-(quinazolin-2-yl)piperazine-1-carboxylate |
| 378 | 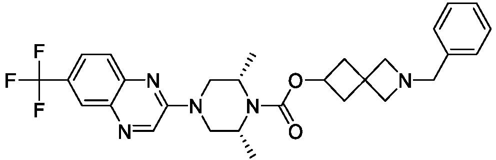 | 2-benzyl-2-azaspiro[3.3]heptan-6-yl (2R,6S)-2,6-dimethyl-4-[6-(trifluoromethyl)quinoxalin-2-yl]piperazine-1-carboxylate |
| 379 | 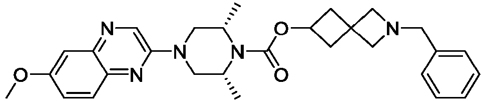 | 2-benzyl-2-azaspiro[3.3]heptan-6-yl (2R,6S)-4-(6-methoxyquinoxalin-2-yl)-2,6-dimethylpiperazine-1-carboxylate |
| 380 | 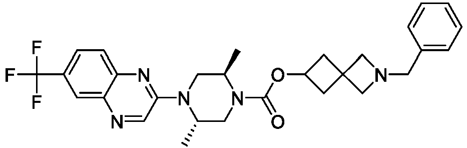 | 2-benzyl-2-azaspiro[3.3]heptan-6-yl (2R,5S)-2,5-dimethyl-4-[6-(trifluoromethyl)quinoxalin-2-yl]piperazine-1-carboxylate |
| 381 | 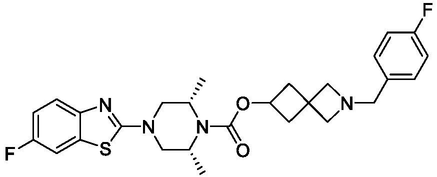 | 2-[(4-fluorophenyl)methyl]-2-azaspiro[3.3]heptan-6-yl (2R,6S)-4-(6-fluoro-1,3-benzothiazol-2-yl)-2,6-dimethylpiperazine-1-carboxylate |
| 382 | 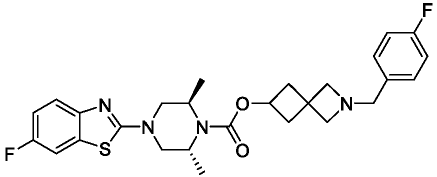 | 2-[(4-fluorophenyl)methyl]-2-azaspiro[3.3]heptan-6-yl (2R,6R)-4-(6-fluoro-1,3-benzothiazol-2-yl)-2,6-dimethylpiperazine-1-carboxylate |
| 383 | 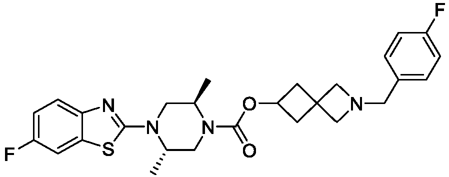 | 2-[(4-fluorophenyl)methyl]-2-azaspiro[3.3]heptan-6-yl (2R,5S)-4-(6-fluoro-1,3-benzothiazol-2-yl)-2,5-dimethylpiperazine-1-carboxylate |
| 384 | 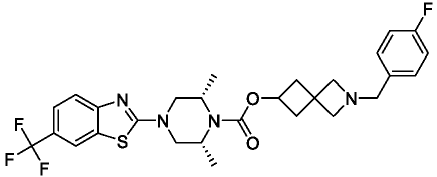 | 2-[(4-fluorophenyl)methyl]-2-azaspiro[3.3]heptan-6-yl (2R,6S)-2,6-dimethyl-4-[6-(trifluoromethyl)-1,3-benzothiazol-2-yl]piperazine-1-carboxylate |
| 385 | 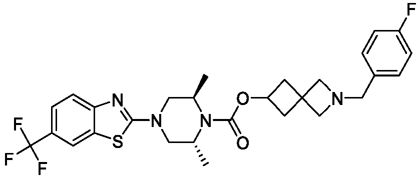 | 2-[(4-fluorophenyl)methyl]-2-azaspiro[3.3]heptan-6-yl (2R,6R)-2,6-dimethyl-4-[6-(trifluoromethyl)-1,3-benzothiazol-2-yl]piperazine-1-carboxylate |
| 386 | 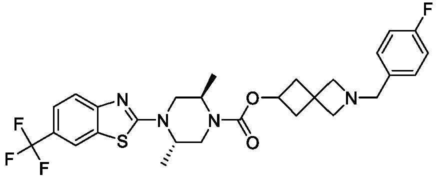 | 2-[(4-fluorophenyl)methyl]-2-azaspiro[3.3]heptan-6-yl (2R,5S)-2,5-dimethyl-4-[6-(trifluoromethyl)-1,3-benzothiazol-2-yl]piperazine-1-carboxylate |
| 387 | 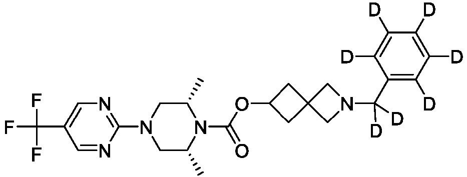 | 2-[(²H₅)phenyl(²H₂)methyl]-2-azaspiro[3.3]heptan-6-yl (2R,6S)-2,6-dimethyl-4-[5-(trifluoromethyl)pyrimidin-2-yl]piperazine-1-carboxylate |
| 388 | 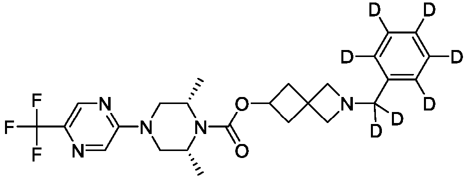 | 2-[(²H₅)phenyl(²H₂)methyl]-2-azaspiro[3.3]heptan-6-yl (2R,6S)-2,6-dimethyl-4-[5-(trifluoromethyl)pyrazin-2-yl]piperazine-1-carboxylate |
| 391 | 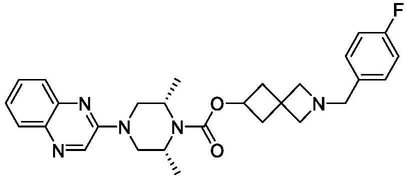 | 2-[(4-fluorophenyl)methyl]-2-azaspiro[3.3]heptan-6-yl (2R,6S)-2,6-dimethyl-4-(quinoxalin-2-yl)piperazine-1-carboxylate |
| 392 | 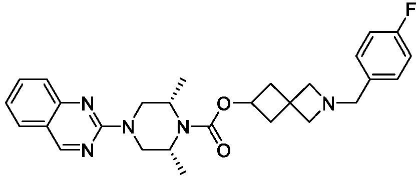 | 2-[(4-fluorophenyl)methyl]-2-azaspiro[3.3]heptan-6-yl (2R,6S)-2,6-dimethyl-4-(quinazolin-2-yl)piperazine-1-carboxylate |
| 393 | 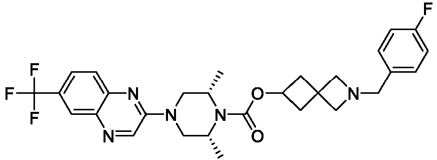 | 2-[(4-fluorophenyl)methyl]-2-azaspiro[3.3]heptan-6-yl (2R,6S)-2,6-dimethyl-4-[6-(trifluoromethyl)quinoxalin-2-yl]piperazine-1-carboxylate |
| 394 | 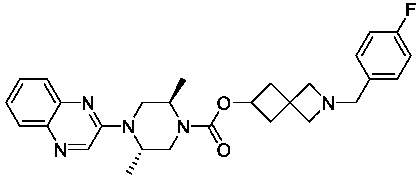 | 2-[(4-fluorophenyl)methyl]-2-azaspiro[3.3]heptan-6-yl (2R,5S)-2,5-dimethyl-4-(quinoxalin-2-yl)piperazine-1-carboxylate |
| 395 | 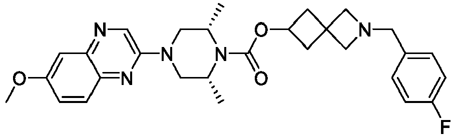 | 2-[(4-fluorophenyl)methyl]-2-azaspiro[3.3]heptan-6-yl (2R,6S)-4-(6-methoxyquinoxalin-2-yl)-2,6-dimethylpiperazine-1-carboxylate |
| 396 | 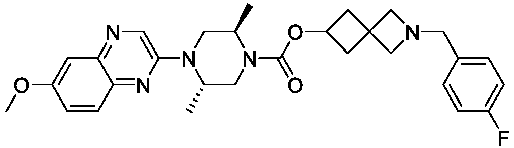 | 2-[(4-fluorophenyl)methyl]-2-azaspiro[3.3]heptan-6-yl (2R,5S)-4-(6-methoxyquinoxalin-2-yl)-2,5-dimethylpiperazine-1-carboxylate |
| 397 | 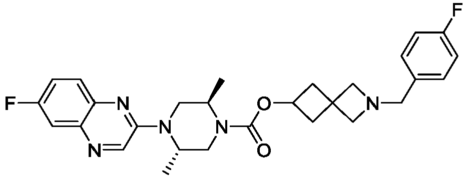 | 2-[(4-fluorophenyl)methyl]-2-azaspiro[3.3]heptan-6-yl (2R,5S)-4-(6-fluoroquinoxalin-2-yl)-2,5-dimethylpiperazine-1-carboxylate |
| 398 | 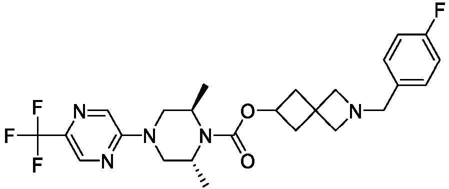 | 2-[(4-fluorophenyl)methyl]-2-azaspiro[3.3]heptan-6-yl (2R,6R)-2,6-dimethyl-4-[5-(trifluoromethyl)pyrazin-2-yl]piperazine-1-carboxylate |
| 399 | 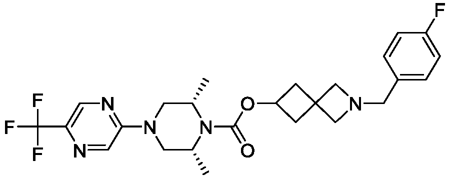 | 2-[(4-fluorophenyl)methyl]-2-azaspiro[3.3]heptan-6-yl (2R,6S)-2,6-dimethyl-4-[5-(trifluoromethyl)pyrazin-2-yl]piperazine-1-carboxylate |
| 400 | 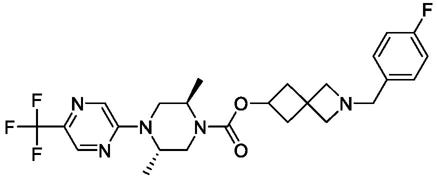 | 2-[(4-fluorophenyl)methyl]-2-azaspiro[3.3]heptan-6-yl (2R,5S)-2,5-dimethyl-4-[5-(trifluoromethyl)pyrazin-2-yl]piperazine-1-carboxylate |
| 401 | 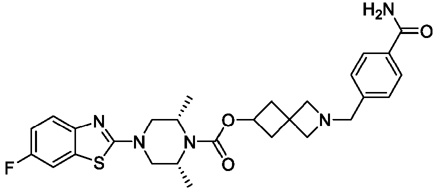 | 2-[(4-carbamoylphenyl)methyl]-2-azaspiro[3.3]heptan-6-yl (2R,6S)-4-(6-fluoro-1,3-benzothiazol-2-yl)-2,6-dimethylpiperazine-1-carboxylate |
| 402 | 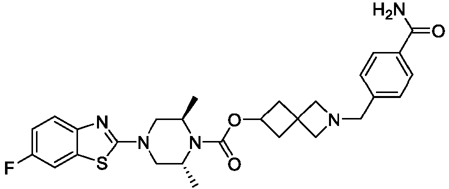 | 2-[(4-carbamoylphenyl)methyl]-2-azaspiro[3.3]heptan-6-yl (2R,6R)-4-(6-fluoro-1,3-benzothiazol-2-yl)-2,6-dimethylpiperazine-1-carboxylate |
| 407 | 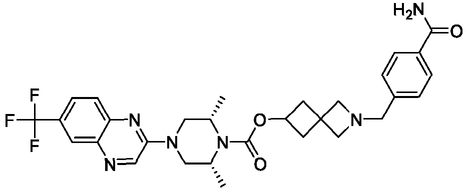 | 2-[(4-carbamoylphenyl)methyl]-2-azaspiro[3.3]heptan-6-yl (2R,6S)-2,6-dimethyl-4-[6-(trifluoromethyl)quinoxalin-2-yl]piperazine-1-carboxylate |
| 408 | 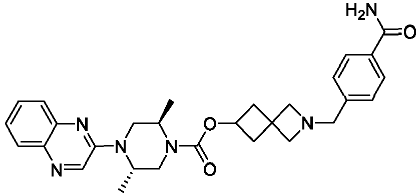 | 2-[(4-carbamoylphenyl)methyl]-2-azaspiro[3.3]heptan-6-yl (2R,5S)-2,5-dimethyl-4-(quinoxalin-2-yl)piperazine-1-carboxylate |
| 409 | 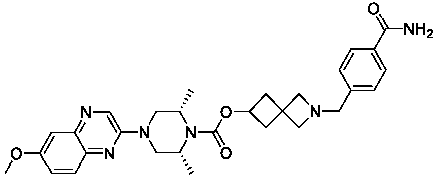 | 2-[(4-carbamoylphenyl)methyl]-2-azaspiro[3.3]heptan-6-yl (2R,6S)-4-(6-methoxyquinoxalin-2-yl)-2,6-dimethylpiperazine-1-carboxylate |
| 410 | 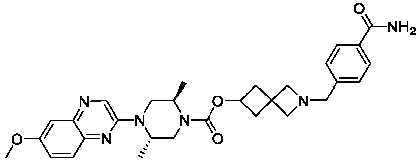 | 2-[(4-carbamoylphenyl)methyl]-2-azaspiro[3.3]heptan-6-yl (2R,5S)-4-(6-methoxyquinoxalin-2-yl)-2,5-dimethylpiperazine-1-carboxylate |
| 411 | 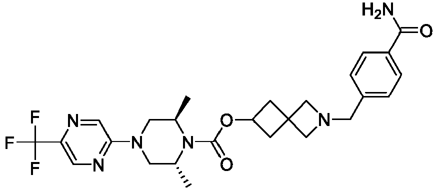 | 2-[(4-carbamoylphenyl)methyl]-2-azaspiro[3.3]heptan-6-yl (2R,6R)-2,6-dimethyl-4-[5-(trifluoromethyl)pyrazin-2-yl]piperazine-1-carboxylate |
| 412 | 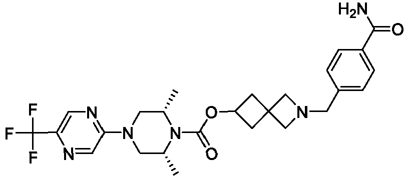 | 2-[(4-carbamoylphenyl)methyl]-2-azaspiro[3.3]heptan-6-yl (2R,6S)-2,6-dimethyl-4-[5-(trifluoromethyl)pyrazin-2-yl]piperazine-1-carboxylate |
| 413 | 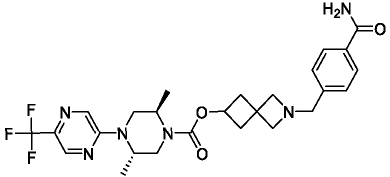 | 2-[(4-carbamoylphenyl)methyl]-2-azaspiro[3.3]heptan-6-yl (2R,5S)-2,5-dimethyl-4-[5-(trifluoromethyl)pyrazin-2-yl]piperazine-1-carboxylate |
| 414 | 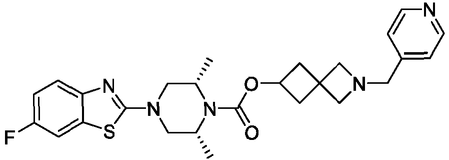 | 2-(pyridin-4-ylmethyl)-2-azaspiro[3.3]heptan-6-yl (2R,6S)-4-(6-fluoro-1,3-benzothiazol-2-yl)-2,6-dimethylpiperazine-1-carboxylate |
| 415 | 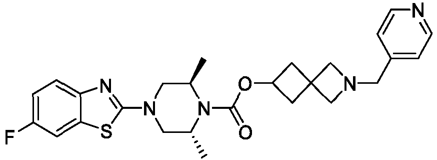 | 2-(pyridin-4-ylmethyl)-2-azaspiro[3.3]heptan-6-yl (2R,6R)-4-(6-fluoro-1,3-benzothiazol-2-yl)-2,6-dimethylpiperazine-1-carboxylate |
| 416 | 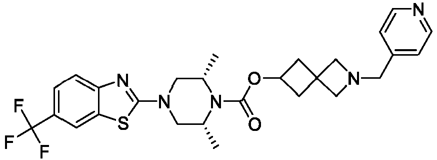 | 2-(pyridin-4-ylmethyl)-2-azaspiro[3.3]heptan-6-yl (2R,6S)-2,6-dimethyl-4-[6-(trifluoromethyl)-1,3-benzothiazol-2-yl]piperazine-1-carboxylate |
| 421 | 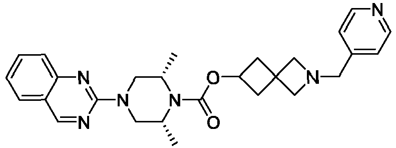 | 2-(pyridin-4-ylmethyl)-2-azaspiro[3.3]heptan-6-yl (2R,6S)-2,6-dimethyl-4-(quinazolin-2-yl)piperazine-1-carboxylate |
| 422 | 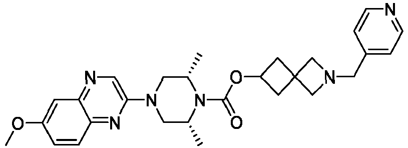 | 2-(pyridin-4-ylmethyl)-2-azaspiro[3.3]heptan-6-yl (2R,6S)-4-(6-methoxyquinoxalin-2-yl)-2,6-dimethylpiperazine-1-carboxylate |
| 423 | 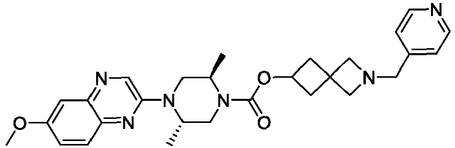 | 2-(pyridin-4-ylmethyl)-2-azaspiro[3.3]heptan-6-yl (2R,5S)-4-(6-methoxyquinoxalin-2-yl)-2,5-dimethylpiperazine-1-carboxylate |
| 424 | 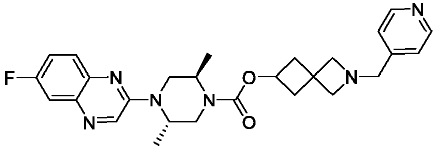 | 2-(pyridin-4-ylmethyl)-2-azaspiro[3.3]heptan-6-yl (2R,5S)-4-(6-fluoroquinoxalin-2-yl)-2,5-dimethylpiperazine-1-carboxylate |
| 425 | 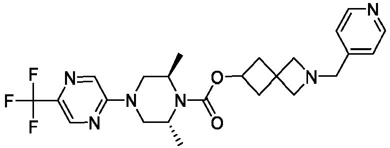 | 2-(pyridin-4-ylmethyl)-2-azaspiro[3.3]heptan-6-yl (2R,6R)-2,6-dimethyl-4-[5-(trifluoromethyl)pyrazin-2-yl]piperazine-1-carboxylate |
| 426 | 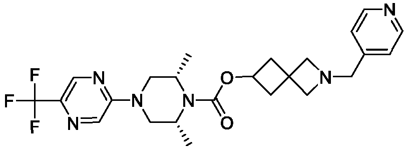 | 2-(pyridin-4-ylmethyl)-2-azaspiro[3.3]heptan-6-yl (2R,6S)-2,6-dimethyl-4-[5-(trifluoromethyl)pyrazin-2-yl]piperazine-1-carboxylate |
| 427 | 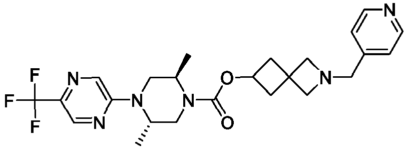 | 2-(pyridin-4-ylmethyl)-2-azaspiro[3.3]heptan-6-yl (2R,5S)-2,5-dimethyl-4-[5-(trifluoromethyl) pyrazin-2-yl]piperazine-1-carboxylate |
| 428 | 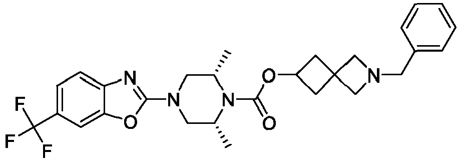 | 2-benzyl-2-azaspiro[3.3]heptan-6-yl (2R,6S)-2,6-dimethyl-4-[6-(trifluoromethyl)-1,3-benzoxazol-2-yl]piperazine-1-carboxylate |
| 429 | 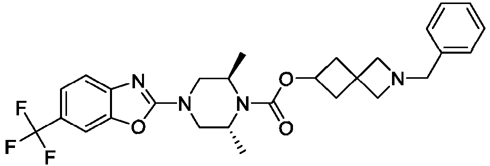 | 2-benzyl-2-azaspiro[3.3]heptan-6-yl (2R,6R)-2,6-dimethyl-4-[6-(trifluoromethyl)-1,3-benzoxazol-2-yl]piperazine-1-carboxylate |
| 430 | 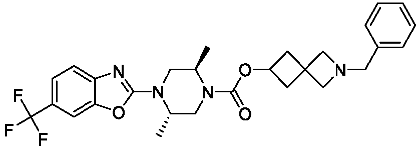 | 2-benzyl-2-azaspiro[3.3]heptan-6-yl (2R,5S)-2,5-dimethyl-4-[6-(trifluoromethyl)-1,3-benzoxazol-2-yl]piperazine-1-carboxylate |
| 431 | 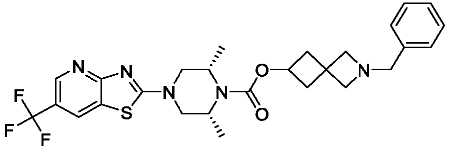 | 2-benzyl-2-azaspiro[3.3]heptan-6-yl (2R,6S)-2,6-dimethyl-4-[6-(trifluoromethyl)-[1,3]thiazolo[4,5-b]pyridin-2-yl]piperazine-1-carboxylate |
| 432 | 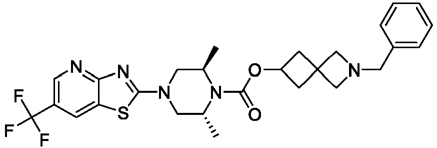 | 2-benzyl-2-azaspiro[3.3]heptan-6-yl (2R,6R)-2,6-dimethyl-4-[6-(trifluoromethyl)-[1,3]thiazolo[4,5-b]pyridin-2-yl]piperazine-1-carboxylate |
| 433 | 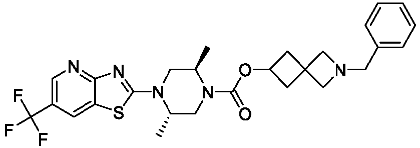 | 2-benzyl-2-azaspiro[3.3]heptan-6-yl (2R,5S)-2,5-dimethyl-4-[6-(trifluoromethyl)-[1,3]thiazolo[4,5-b]pyridin-2-yl]piperazine-1-carboxylate |
| 434 | 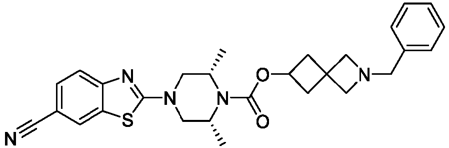 | 2-benzyl-2-azaspiro[3.3]heptan-6-yl (2R,6S)-4-(6-cyano-1,3-benzothiazol-2-yl)-2,6-dimethylpiperazine-1-carboxylate |
| 435 | 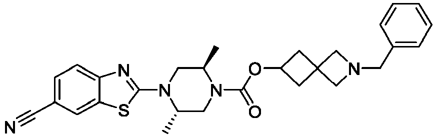 | 2-benzyl-2-azaspiro[3.3]heptan-6-yl (2R,5S)-4-(6-cyano-1,3-benzothiazol-2-yl)-2,5-dimethylpiperazine-1-carboxylate |
| 436 | 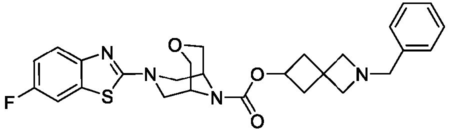 | 2-benzyl-2-azaspiro[3.3]heptan-6-yl 7-(6-fluoro-1,3-benzothiazol-2-yl)-3-oxa-7,9-diazabicyclo[3.3.1]nonane-9-carboxylate |
| 437 | 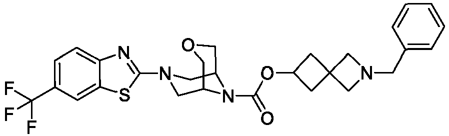 | 2-benzyl-2-azaspiro[3.3]heptan-6-yl 7-[6-(trifluoromethyl)-1,3-benzothiazol-2-yl]-3-oxa-7,9-diazabicyclo[3.3.1]nonane-9-carboxylate |
| 438 | 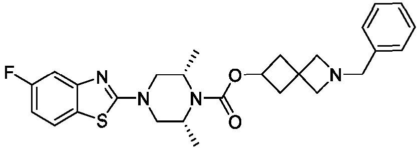 | 2-benzyl-2-azaspiro[3.3]heptan-6-yl (2R,6S)-4-(5-fluoro-1,3-benzothiazol-2-yl)-2,6-dimethylpiperazine-1-carboxylate |
| 439 | 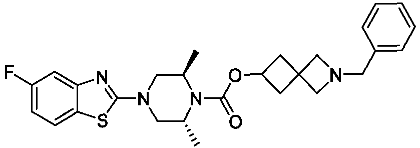 | 2-benzyl-2-azaspiro[3.3]heptan-6-yl (2R,6R)-4-(5-fluoro-1,3-benzothiazol-2-yl)-2,6-dimethylpiperazine-1-carboxylate |
| 440 | 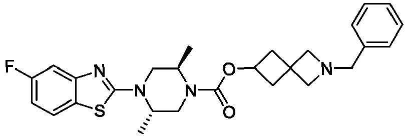 | 2-benzyl-2-azaspiro[3.3]heptan-6-yl (2R,5S)-4-(5-fluoro-1,3-benzothiazol-2-yl)-2,5-dimethylpiperazine-1-carboxylate |
| 441 | 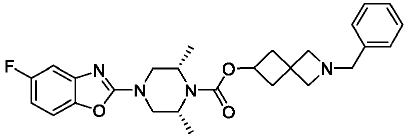 | 2-benzyl-2-azaspiro[3.3]heptan-6-yl (2R,6S)-4-(5-fluoro-1,3-benzoxazol-2-yl)-2,6-dimethylpiperazine-1-carboxylate |
| 442 | 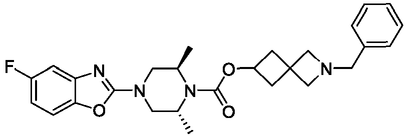 | 2-benzyl-2-azaspiro[3.3]heptan-6-yl (2R,6R)-4-(5-fluoro-1,3-benzoxazol-2-yl)-2,6-dimethylpiperazine-1-carboxylate |
| 443 | 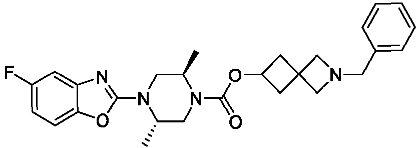 | 2-benzyl-2-azaspiro[3.3]heptan-6-yl (2R,5S)-4-(5-fluoro-1,3-benzoxazol-2-yl)-2,5-dimethylpiperazine-1-carboxylate |
| 444 | 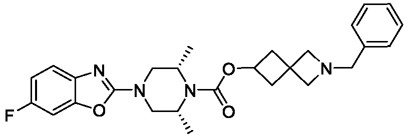 | 2-benzyl-2-azaspiro[3.3]heptan-6-yl (2R,6S)-4-(6-fluoro-1,3-benzoxazol-2-yl)-2,6-dimethylpiperazine-1-carboxylate |
| 445 | 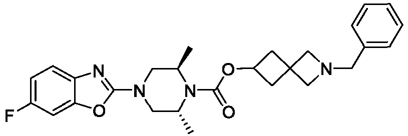 | 2-benzyl-2-azaspiro[3.3]heptan-6-yl (2R,6R)-4-(6-fluoro-1,3-benzoxazol-2-yl)-2,6-dimethylpiperazine-1-carboxylate |
| 446 | 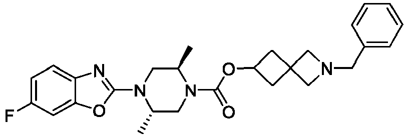 | 2-benzyl-2-azaspiro[3.3]heptan-6-yl (2R,5S)-4-(6-fluoro-1,3-benzoxazol-2-yl)-2,5-dimethylpiperazine-1-carboxylate |
| 447 | 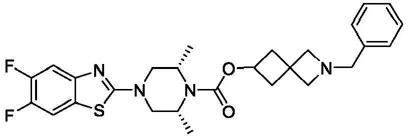 | 2-benzyl-2-azaspiro[3.3]heptan-6-yl (2R,6S)-4-(5,6-difluoro-1,3-benzothiazol-2-yl)-2,6-dimethylpiperazine-1-carboxylate |
| 448 | 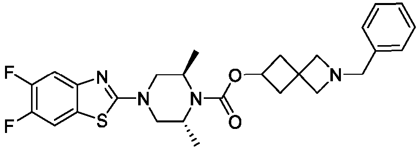 | 2-benzyl-2-azaspiro[3.3]heptan-6-yl (2R,6R)-4-(5,6-difluoro-1,3-benzothiazol-2-yl)-2,6-dimethylpiperazine-1-carboxylate |
| 449 | 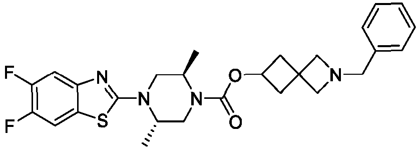 | 2-benzyl-2-azaspiro[3.3]heptan-6-yl (2R,5S)-4-(5,6-difluoro-1,3-benzothiazol-2-yl)-2,5-dimethylpiperazine-1-carboxylate |
| 450 | 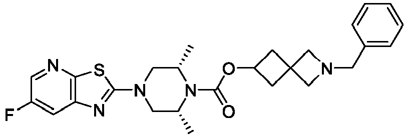 | 2-benzyl-2-azaspiro[3.3]heptan-6-yl (2R,6S)-4-{6-fluoro-[1,3]thiazolo[5,4-b]pyridin-2-yl}-2,6-dimethylpiperazine-1-carboxylate |
| 451 | 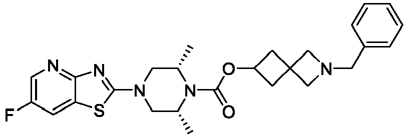 | 2-benzyl-2-azaspiro[3.3]heptan-6-yl (2R,6S)-4-{6-fluoro-[1,3]thiazolo[4,5-b]pyridin-2-yl}-2,6-dimethylpiperazine-1-carboxylate |
| 452 | 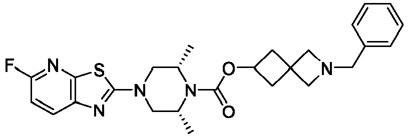 | 2-benzyl-2-azaspiro[3.3]heptan-6-yl (2R,6S)-4-{5-fluoro-[1,3]thiazolo[5,4-b]pyridin-2-yl}-2,6-dimethylpiperazine-1-carboxylate |
| 453 | 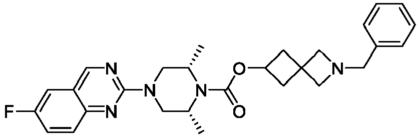 | 2-benzyl-2-azaspiro[3.3]heptan-6-yl (2R,6S)-4-(6-fluoroquinazolin-2-yl)-2,6-dimethylpiperazine-1-carboxylate |
| 454 | 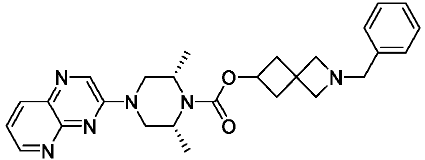 | 2-benzyl-2-azaspiro[3.3]heptan-6-yl (2R,6S)-2,6-dimethyl-4-{pyrido[2,3-b]pyrazin-3-yl}piperazine-1-carboxylate |
| 455 | 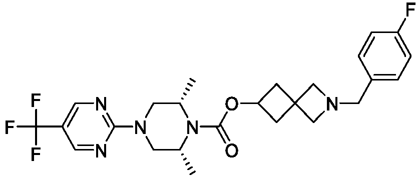 | 2-[(4-fluorophenyl)methyl]-2-azaspiro[3.3]heptan-6-yl (2R,6S)-2,6-dimethyl-4-[5-(trifluoromethyl)pyrimidin-2-yl]piperazine-1-carboxylate |
| 456 | 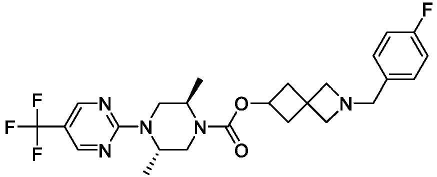 | 2-[(4-fluorophenyl)methyl]-2-azaspiro[3.3]heptan-6-yl (2R,5S)-2,5-dimethyl-4-[5-(trifluoromethyl)pyrimidin-2-yl]piperazine-1-carboxylate |
| 457 | 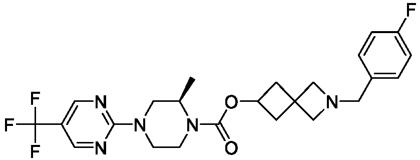 | 2-[(4-fluorophenyl)methyl]-2-azaspiro[3.3]heptan-6-yl (2R)-2-methyl-4-[5-(trifluoromethyl)pyrimidin-2-yl]piperazine-1-carboxylate |
| 458 | 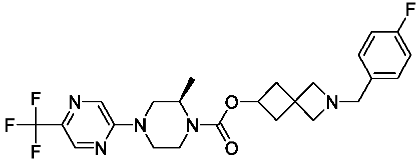 | 2-[(4-fluorophenyl)methyl]-2-azaspiro[3.3]heptan-6-yl (2R)-2-methyl-4-[5-(trifluoromethyl)pyrazin-2-yl]piperazine-1-carboxylate |
| 459 | 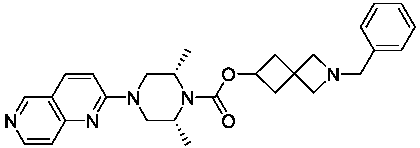 | 2-benzyl-2-azaspiro[3.3]heptan-6-yl (2R,6S)-2,6-dimethyl-4-(1,6-naphthyridin-2-yl)piperazine-1-carboxylate |
| 460 | 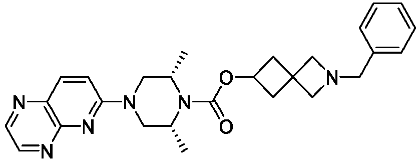 | 2-benzyl-2-azaspiro[3.3]heptan-6-yl (2R,6S)-2,6-dimethyl-4-{pyrido[2,3-b]pyrazin-6-yl}piperazine-1-carboxylate |
| 461 | 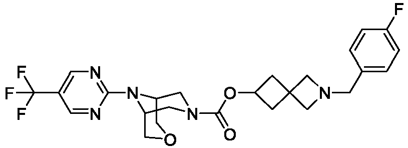 | 2-[(4-fluorophenyl)methyl]-2-azaspiro[3.3]heptan-6-yl 7-[5-(trifluoromethyl)pyrimidin-2-yl]-3-oxa-7,9-diazabicyclo[3.3.1]nonane-9-carboxylate |
| 462 | 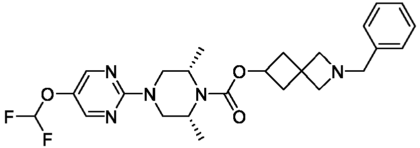 | 2-benzyl-2-azaspiro[3.3]heptan-6-yl (2R,6S)-4-[5-(difluoromethoxy)pyrimidin-2-yl]-2,6-dimethylpiperazine-1-carboxylate |
| 463 | 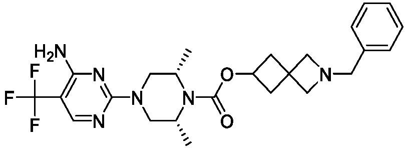 | 2-benzyl-2-azaspiro[3.3]heptan-6-yl (2R,6S)-4-[4-amino-5-(trifluoromethyl)pyrimidin-2-yl]-2,6-dimethylpiperazine-1-carboxylate |
| 464 | 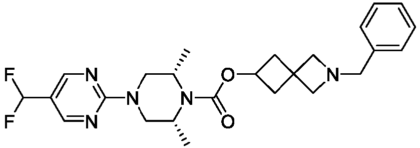 | 2-benzyl-2-azaspiro[3.3]heptan-6-yl (2R,6S)-4-[5-(difluoromethyl) pyrimidin-2-yl]-2,6-dimethylpiperazine-1-carboxylate |
| 465 | 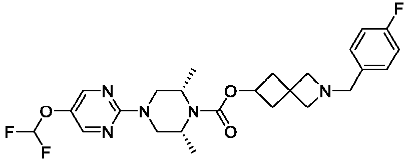 | 2-[(4-fluorophenyl)methyl]-2-azaspiro[3.3]heptan-6-yl (2R,6S)-4-[5-(difluoromethoxy)pyrimidin-2-yl]-2,6-dimethylpiperazine-1-carboxylate |
| 466 | 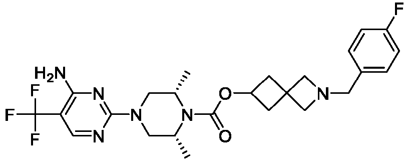 | 2-[(4-fluorophenyl)methyl]-2-azaspiro[3.3]heptan-6-yl (2R,6S)-4-[4-amino-5-(trifluoromethyl)pyrimidin-2-yl]-2,6-dimethylpiperazine-1-carboxylate |
| 467 | 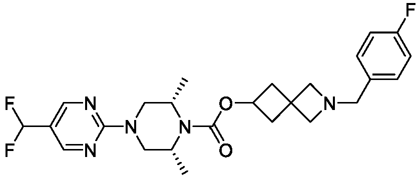 | 2-[(4-fluorophenyl)methyl]-2-azaspiro[3.3]heptan-6-yl (2R,6S)-4-[5-(difluoromethyl) pyrimidin-2-yl]-2,6-dimethylpiperazine-1-carboxylate |
| 468 | 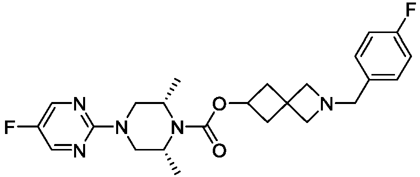 | 2-[(4-fluorophenyl)methyl]-2-azaspiro[3.3]heptan-6-yl (2R,6S)-4-(5-fluoropyrimidin-2-yl)-2,6-dimethylpiperazine-1-carboxylate |
| 469 | 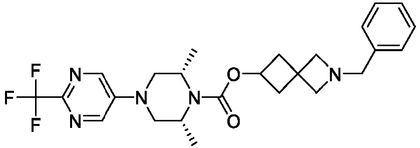 | 2-benzyl-2-azaspiro[3.3]heptan-6-yl (2R,6S)-2,6-dimethyl-4-[2-(trifluoromethyl)pyrimidin-5-yl]piperazine-1-carboxylate |
| 471 | 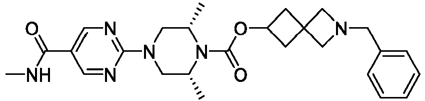 | 2-benzyl-2-azaspiro[3.3]heptan-6-yl (2R,6S)-2,6-dimethyl-4-[5-(methylcarbamoyl)pyrimidin-2-yl]piperazine-1-carboxylate |
| 472 | 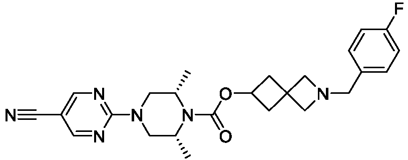 | 2-[(4-fluorophenyl)methyl]-2-azaspiro[3.3]heptan-6-yl (2R,6S)-4-(5-cyanopyrimidin-2-yl)-2,6-dimethylpiperazine-1-carboxylate |
| 473 | 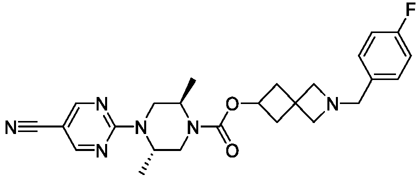 | 2-[(4-fluorophenyl)methyl]-2-azaspiro[3.3]heptan-6-yl (2R,5S)-4-(5-cyanopyrimidin-2-yl)-2,5-dimethylpiperazine-1-carboxylate |
| 474 | 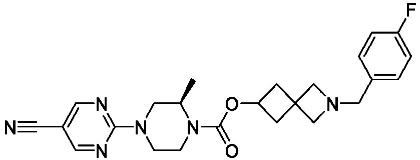 | 2-[(4-fluorophenyl)methyl]-2-azaspiro[3.3]heptan-6-yl (2R)-4-(5-cyanopyrimidin-2-yl)-2-methylpiperazine-1-carboxylate |
| 475 | 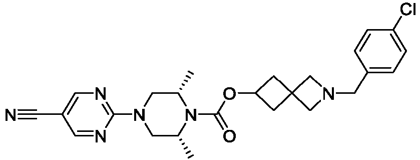 | 2-[(4-chlorophenyl)methyl]-2-azaspiro[3.3]heptan-6-yl (2R,6S)-4-(5-cyanopyrimidin-2-yl)-2,6-dimethylpiperazine-1-carboxylate |
| 476 | 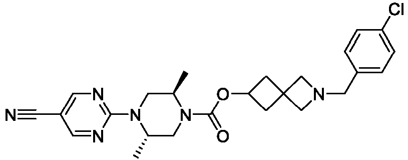 | 2-[(4-chlorophenyl)methyl]-2-azaspiro[3.3]heptan-6-yl (2R,5S)-4-(5-cyanopyrimidin-2-yl)-2,5-dimethylpiperazine-1-carboxylate |
| 477 | 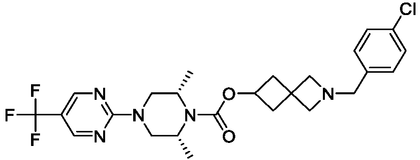 | 2-[(4-chlorophenyl)methyl]-2-azaspiro[3.3]heptan-6-yl (2R,6S)-2,6-dimethyl-4-[5-(trifluoromethyl)pyrimidin-2-yl]piperazine-1-carboxylate |
| 478 | 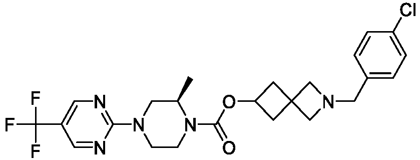 | 2-[(4-chlorophenyl)methyl]-2-azaspiro[3.3]heptan-6-yl (2R)-2-methyl-4-[5-(trifluoromethyl)pyrimidin-2-yl]piperazine-1-carboxylate |
| 479 | 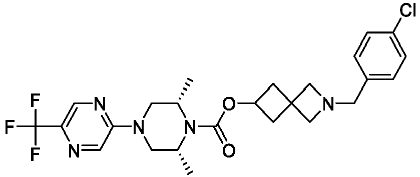 | 2-[(4-chlorophenyl)methyl]-2-azaspiro[3.3]heptan-6-yl (2R,6S)-2,6-dimethyl-4-[5-(trifluoromethyl)pyrazin-2-yl]piperazine-1-carboxylate |
| 480 | 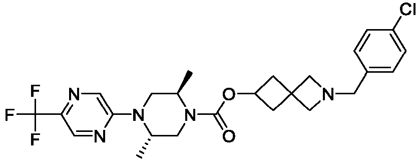 | 2-[(4-chlorophenyl)methyl]-2-azaspiro[3.3]heptan-6-yl (2R,5S)-2,5-dimethyl-4-[5-(trifluoromethyl)pyrazin-2-yl]piperazine-1-carboxylate |
| 481 | 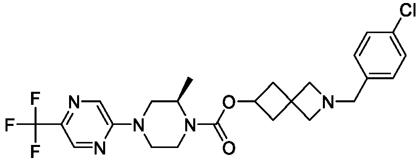 | 2-[(4-chlorophenyl)methyl]-2-azaspiro[3.3]heptan-6-yl (2R)-2-methyl-4-[5-(trifluoromethyl)pyrazin-2-yl]piperazine-1-carboxylate |
| 482 | 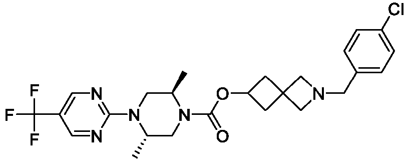 | 2-[(4-chlorophenyl)methyl]-2-azaspiro[3.3]heptan-6-yl (2R,5S)-2,5-dimethyl-4-[5-(trifluoromethyl)pyrimidin-2-yl]piperazine-1-carboxylate |
| 483 | 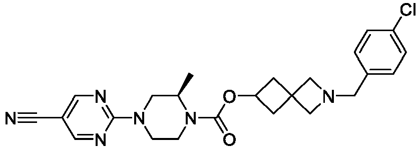 | 2-[(4-chlorophenyl)methyl]-2-azaspiro[3.3]heptan-6-yl (2R)-4-(5-cyanopyrimidin-2-yl)-2-methylpiperazine-1-carboxylate |
| 484 | 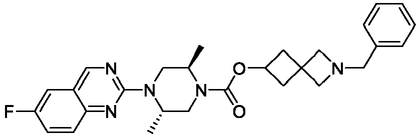 | 2-benzyl-2-azaspiro[3.3]heptan-6-yl (2R,5S)-4-(6-fluoroquinazolin-2-yl)-2,5-dimethylpiperazine-1-carboxylate |
| 487 | 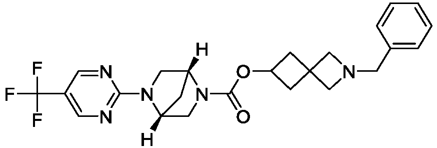 | 2-benzyl-2-azaspiro[3.3]heptan-6-yl (1 R,4R)-5-[5-(trifluoromethyl)pyrimidin-2-yl]-2,5-diazabicyclo[2.2.1]heptane-2-carboxylate |
| 489 | 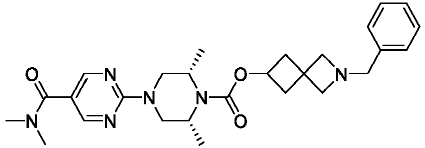 | 2-benzyl-2-azaspiro[3.3]heptan-6-yl (2R,6S)-4-[5-(dimethylcarbamoyl)pyrimidi n-2-yl]-2,6-dimethylpiperazine-1-carboxylate |
| 495 | 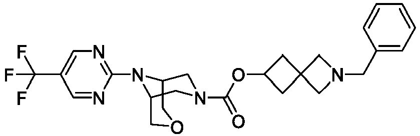 | 2-benzyl-2-azaspiro[3.3]heptan-6-yl 9-[5-(trifluoromethyl)pyrimidin-2-yl]-3-oxa-7,9-diazabicyclo[3.3.1]nonane-7-carboxylate |
| 496 | 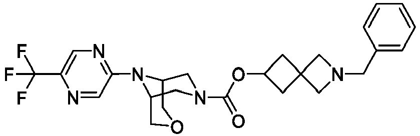 | 2-benzyl-2-azaspiro[3.3]heptan-6-yl 9-[5-(trifluoromethyl)pyrazin-2-yl]-3-oxa-7,9-diazabicyclo[3.3.1]nonane-7-carboxylate |
| 497 | 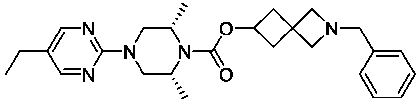 | 2-benzyl-2-azaspiro[3.3]heptan-6-yl (2R,6S)-4-(5-ethylpyrimidin-2-yl)-2,6-dimethylpiperazine-1-carboxylate |
| 498 | 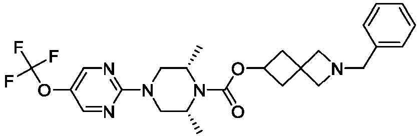 | 2-benzyl-2-azaspiro[3.3]heptan-6-yl (2R,6S)-2,6-dimethyl-4-[5-(trifluoromethoxy)pyrimidin-2-yl]piperazine-1-carboxylate |
| 499 | 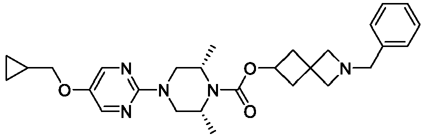 | 2-benzyl-2-azaspiro[3.3]heptan-6-yl (2R,6S)-4-[5-(cyclopropylmethoxy)pyrimid in-2-yl]-2,6-dimethylpiperazine-1-carboxylate |
| 500 | 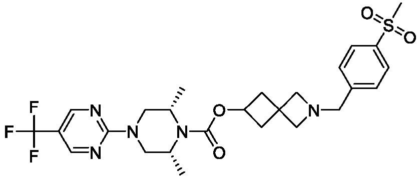 | 2-[(4-methanesulfonylphenyl)met hyl]-2-azaspiro[3.3]heptan-6-yl (2R,6S)-2,6-dimethyl-4-[5-(trifluoromethyl)pyrimidin-2-yl]piperazine-1-carboxylate |
| 501 | 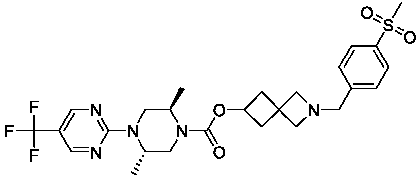 | 2-[(4-methanesulfonylphenyl)met hyl]-2-azaspiro[3.3]heptan-6-yl (2R,5S)-2,5-dimethyl-4-[5-(trifluoromethyl)pyrimidin-2-yl]piperazine-1-carboxylate |
| 502 | 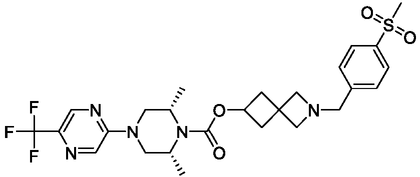 | 2-[(4-methanesulfonylphenyl)met hyl]-2-azaspiro[3.3]heptan-6-yl (2R,6S)-2,6-dimethyl-4-[5-(trifluoromethyl)pyrazin-2-yl]piperazine-1-carboxylate |
| 503 | 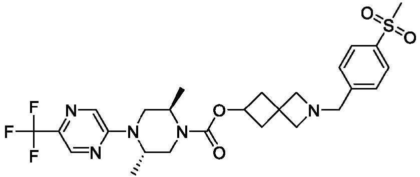 | 2-[(4-methanesulfonylphenyl)met hyl]-2-azaspiro[3.3]heptan-6-yl (2R,5S)-2,5-dimethyl-4-[5-(trifluoromethyl)pyrazin-2-yl]piperazine-1-carboxylate |
| 504 | 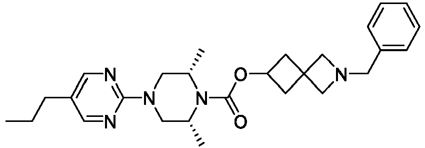 | 2-benzyl-2-azaspiro[3.3]heptan-6-yl (2R,6S)-2,6-dimethyl-4-(5-propylpyrimidin-2-yl)piperazine-1-carboxylate |
| 505 | 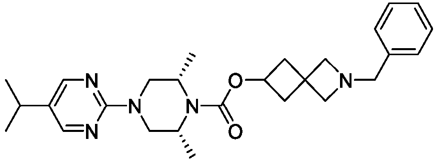 | 2-benzyl-2-azaspiro[3.3]heptan-6-yl (2R,6S)-2,6-dimethyl-4-[5-(propan-2-yl)pyrimidin-2-yl]piperazine-1-carboxylate |
| 506 | 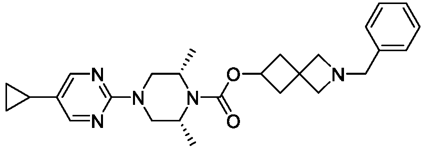 | 2-benzyl-2-azaspiro[3.3]heptan-6-yl (2R,6S)-4-(5-cyclopropylpyrimidin-2-yl)-2,6-dimethylpiperazine-1-carboxylate |
| 508 | 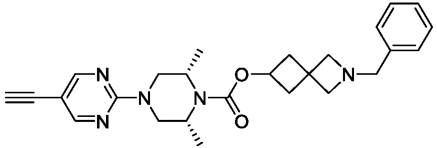 | 2-benzyl-2-azaspiro[3.3]heptan-6-yl (2R,6S)-4-(5-ethynylpyrimidin-2-yl)-2,6-dimethylpiperazine-1-carboxylate |
| 511 | 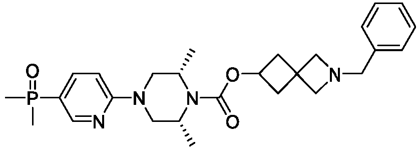 | 2-benzyl-2-azaspiro[3.3]heptan-6-yl (2R,6S)-4-[5-(dimethylphosphoryl)pyridin-2-yl]-2,6-dimethylpiperazine-1-carboxylate |
| 514 | 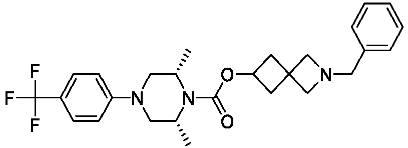 | 2-benzyl-2-azaspiro[3.3]heptan-6-yl (2R,6S)-2,6-dimethyl-4-[4-(trifluoromethyl)phenyl]piper azine-1-carboxylate |
| 523 | 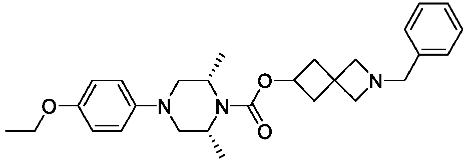 | 2-benzyl-2-azaspiro[3.3]heptan-6-yl (2R,6S)-4-(4-ethoxyphenyl)-2,6-dimethylpiperazine-1-carboxylate |
| 524 | 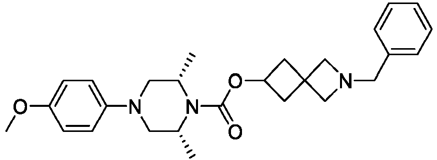 | 2-benzyl-2-azaspiro[3.3]heptan-6-yl (2R,6S)-4-(4-methoxyphenyl)-2,6-dimethylpiperazine-1-carboxylate |
| 525 | 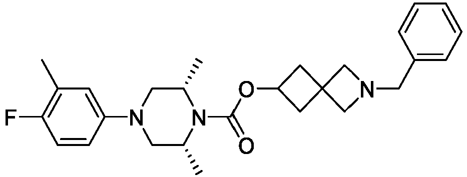 | 2-benzyl-2-azaspiro[3.3]heptan-6-yl (2R,6S)-4-(4-fluoro-3-methylphenyl)-2,6-dimethylpiperazine-1-carboxylate |
| 526 | 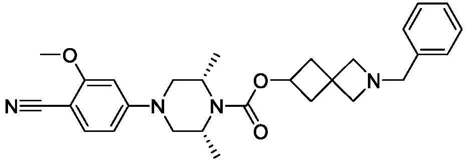 | 2-benzyl-2-azaspiro[3.3]heptan-6-yl (2R,6S)-4-(4-cyano-3-methoxyphenyl)-2,6-dimethylpiperazine-1-carboxylate |
| 527 | 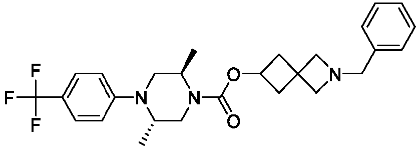 | 2-benzyl-2-azaspiro[3.3]heptan-6-yl (2R,5S)-2,5-dimethyl-4-[4-(trifluoromethyl)phenyl]piper azine-1-carboxylate |
| 528 | 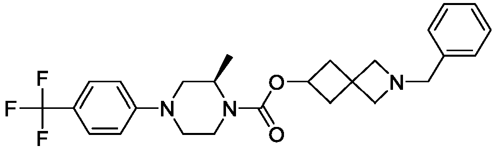 | 2-benzyl-2-azaspiro[3.3]heptan-6-yl (2R)-2-methyl-4-[4-(trifluoromethyl)phenyl]piper azine-1-carboxylate |
| 529 | 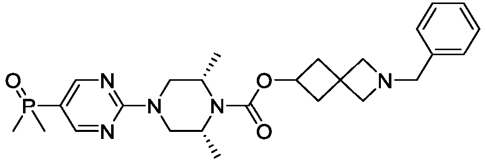 | 2-benzyl-2-azaspiro[3.3]heptan-6-yl (2R,6S)-4-[5-(dimethylphosphoryl)pyrimid in-2-yl]-2,6-dimethylpiperazine-1-carboxylate |
| 530 | 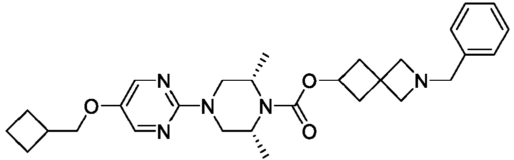 | 2-benzyl-2-azaspiro[3.3]heptan-6-yl (2R,6S)-4-[5-(cyclobutylmethoxy)pyrimidin-2-yl]-2,6-dimethylpiperazine-1-carboxylate |

Additionally, chemical genera of the present invention and individual compounds, for example those compounds found in **TABLE A,** encompass all possible stereoisomers and all pharmaceutically acceptable salts thereof.

The compounds described herein can be asymmetric (e.g., having one or more stereocenters). All stereoisomers, such as enantiomers and diastereomers, are intended unless otherwise indicated. It is understood that, in any compound described herein having one or more chiral centers, if an absolute stereochemistry is not expressly indicated, then each center may independently be the (R)-configuration, or the (S)-configuration, or a mixture thereof. Thus, the compounds provided herein may be enantiomerically pure, enantiomerically enriched, a racemic mixture, diastereomerically pure, diastereomerically enriched, or a stereoisomeric mixture. Preparation of enantiomerically pure or enantiomerically enriched forms may be accomplished by resolution of racemic mixtures or by using enantiomerically pure or enriched starting materials or by stereoselective or stereospecific synthesis. Stereochemical definitions are available in E.L. Eliel, S.H. Wilen & L.N. Mander, Stereochemistry of Organic Compounds, John Wiley & Sons, Inc., New York, NY, 1994. In some embodiments, where the compound of the invention is chiral or otherwise includes one or more stereocenters, the compound can be prepared with an enantiomeric excess or diastereomeric excess of greater than about 75%, greater than about 80%, greater than about 85%, greater than about 90%, greater than about 95%, or greater than about 99%.

Resolution of racemic mixtures of compounds can be carried out by any of numerous methods known in the art. An example method includes fractional recrystallizaion using a chiral resolving organic acid with a racemic compound containing a basic group. Suitable resolving agents for fractional recrystallization methods are, for example, optically active acids, such as the D and L forms of tartaric acid, diacetyltartaric acid, dibenzoyltartaric acid, mandelic acid, malic acid, lactic acid or the various optically active camphorsulfonic acids. Other chiral resolving agents suitable for fractional crystallization methods include stereoisomerically pure forms of methylbenzylamine *(e.g., S* and R forms, or diastereomerically pure forms), 2-phenylglycinol, norephedrine, ephedrine, N-methylephedrine, cyclohexylethylamine, 1,2-diaminocyclohexane, and the like. Similarly, fractional recrystallization using a chiral resolving base can be utilized with a racemic compound containing a basic group.

Resolution of racemic mixtures can also be carried out by elution on a column packed with an optically active resolving agent (e.g., dinitrobenzoylphenylglycine). A suitable elution solvent composition can be determined by one skilled in the art.

In some embodiments, a compound of the invention can be prepared having at least about 5%, at least about 10%, at least about 20%, at least about 30%, at least about 40%, at least about 50%, at least about 60%, at least about 70%, at least about 80%, at least about 90%, at least about 95%, at least about 99%, or at least about 99.9% enantiomeric excess, or an enantiomeric excess within a range defined by any of the preceding numbers.

In addition, it is understood that, when a compound described herein contain one or more double bond(s) (*e.g*., C=C, C=N, and the like) or other centers of geometric asymmetry, and unless specified otherwise, it is understood that the compound includes both E and Z geometric isomers (*e.g*., cis or trans). Cis and trans geometric isomers of the compounds described herein may be isolated as a mixture of isomers or as separated isomeric form.

The compounds described herein also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, enamine - imine pairs, and annular forms where a proton can occupy two or more positions of a heterocyclic system, for example, 1*H*- and 3*H*-imidazole, *1H-,* 2*H*- and 4*H-*1,2,4-triazole, 1*H*- and 2*H*- isoindole, and 1*H*- and 2*H*-pyrazole. Tautomeric forms can be in equilibrium or sterically locked into one form by appropriate substitution.

The compounds of the present invention and their pharmaceutically acceptable salts can be found together with other substances such as water and solvents, for example, in the form of hydrates or solvates. When in the solid-state, the compounds described herein and salts thereof may occur in various forms and may, e.g., take the form of solvates, including hydrates. The compounds may be in any solid-state form, such as a crystalline form, amorphous form, solvated form, *etc.* and unless clearly indicated otherwise, reference in the specification to compounds and salts thereof should be understood as reading on any solid-state form of the compound.

The compounds described herein may be used in a neutral form, such as, a free acid or free base form. Alternatively, the compounds may be used in the form of acid or base addition salts. The term "pharmaceutically acceptable salt" refers to salts of a compound having an acidic or basic moiety which are not biologically or otherwise undesirable for use in a pharmaceutical. In many cases, the compounds disclosed herein are capable of forming acid and/or base salts by virtue of the presence of an acidic or basic moiety (e.g. amino and/or carboxyl groups or groups similar thereto). Pharmaceutically acceptable acid addition salts can be formed by combining a compound having a basic moiety with inorganic acids and organic acids. Inorganic acids which may be used to prepare salts include, for example, hydrochloric acid, hydrobromic acid, sulfuric acid, nitric acid, phosphoric acid, and the like. Organic acids which may be used to prepare salts include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed by combining a compound having an acidic moiety with inorganic and organic bases. Inorganic bases which may be used to prepare salts include, for example, sodium, potassium, lithium, ammonium, calcium, magnesium, iron, zinc, manganese, aluminum hydroxides, carbonates, bicarbonates, phosphates, and the like; particularly preferred are the ammonium, potassium, sodium, calcium, and magnesium hydroxides, carbonates, bicarbonates, or phosphates. Organic bases from which may be used to prepare salts include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. Generally, such salts can be prepared by reacting the free acid or base forms of these compounds with at least a stoichiometric amount of the appropriate base or acid in water or in an organic solvent, or in a mixture of the two; generally, non-aqueous media like ether, ethyl acetate, alcohols (e.g., methanol, ethanol, iso-propanol, or butanol) or acetonitrile (ACN). Lists of suitable salts are found in WO 87/05297; Johnston et al., published September 11, 1987; Remington's Pharmaceutical Sciences, 17th ed., Mack Publishing Company, Easton, Pa., 1985, p. 1418; and J. Pharm. Sci., 66, 2 (1977);. A reference for the preparation and selection of pharmaceutical salts of the present disclosure is P. H. Stahl & C. G. Wermuth, Handbook of Pharmaceutical Salts, Verlag Helvetica Chimica Acta, Zurich, 2002.

In some embodiments, the compounds described herein, or salts thereof, are substantially isolated. The phrase "substantially isolated" refers to the compound that is at least partially or substantially separated from the environment in which it was formed or detected. Partial separation can include, for example, a composition enriched in the compounds of the invention. Substantial separation can include compositions containing at least about 50%, at least about 60%, at least about 65%, at least about 70%, at least about 75%, at least about 80%, at least about 85%, at least about 90%, at least about 95%, at least about 97%, or at least about 99% by weight of the compounds of the invention, or salt thereof.

### POLYMORPHS AND PSEUDOPOLYMORPHS

Polymorphism is the ability of a single-component substance to exist as two or more crystalline phases that have different arrangements and/or conformations of the molecules in the crystal lattice. Polymorphs show the same properties in the liquid or gaseous state, but they behave differently in the solid-state.

Besides single-component polymorphs, compounds (*e.g*., drugs) can also exist as salts and other multicomponent crystalline phases. For example, solvates and hydrates may contain a compound as a host and either solvent or water molecules, respectively, as guests. Analogously, when the guest compound is a solid at room temperature, the resulting form is often called a cocrystal. Salts, solvates, hydrates, and cocrystals may show polymorphism as well. Crystalline phases that share the same compound host, but differ with respect to their guests, may be referred to as pseudopolymorphs of one another.

Solvates contain molecules of the solvent of crystallization in a definite crystal lattice. Solvates, in which the solvent of crystallization is water, are termed hydrates. Because water is a constituent of the atmosphere, hydrates of drugs may be formed rather easily.

By way of example, Stahly published a polymorph screen of 245 compounds consisting of a "wide variety of structural types" that revealed about 90% of them exhibited multiple solid forms. Overall, approximately half of the compounds were polymorphic, often having one to three forms. About one-third of the compounds formed hydrates, and about one-third formed solvates. Data from cocrystal screens of 64 compounds showed that 60% formed cocrystals other than hydrates or solvates. (G. P. Stahly, Crystal Growth & Design (2007), 7(6), 1007-1026).

### ISOTOPES

The compounds disclosed and described herein allow atoms at each position of the compound independently to have: 1) an isotopic distribution for a chemical element in proportional amounts to those usually found in nature or 2) an isotopic distribution in proportional amounts different to those usually found in nature unless the context clearly dictates otherwise. A particular chemical element has an atomic number defined by the number of protons within the atom's nucleus. Each atomic number identifies a specific element, but not the isotope; an atom of a given element may have a wide range in its number of neutrons. The number of both protons and neutrons in the nucleus is the atom's mass number, and each isotope of a given element has a different mass number. A compound wherein one or more atoms have an isotopic distribution for a chemical element in proportional amounts different to those usually found in nature is commonly referred to as being an isotopically-labeled compound. Each chemical element as represented in a compound structure may include any isotopic distribution of said element. For example, in a compound structure a hydrogen atom may be explicitly disclosed or understood to be present in the compound. At any position of the compound that a hydrogen atom may be present, the hydrogen atom can be an isotopic distribution of hydrogen, including but not limited to protium (¹H) and deuterium (²H) in proportional amounts to those usually found in nature and in proportional amounts different to those usually found in nature. Thus, reference herein to a compound encompasses all potential isotopic distributions for each atom unless the context clearly dictates otherwise. Examples of isotopes include isotopes of hydrogen, carbon, nitrogen, oxygen, phosphorous, sulfur, fluorine, chlorine, bromine, and iodine. As one of skill in the art would appreciate, any of the compounds as disclosed and described herein may include radioactive isotopes. Accordingly, also contemplated is use of compounds as disclosed and described herein, wherein one or more atoms have an isotopic distribution different to those usually found in nature, such as having ²H or ³H in greater proportion, or ¹¹C, ¹³C, or ¹⁴C in greater proportion than found in nature. By way of general example, and without limitation, isotopes of hydrogen include protium (¹H), deuterium (²H), and tritium (³H). Isotopes of carbon include carbon-11 (¹¹C), carbon-12 (¹²C), carbon-13 (¹³C), and carbon-14 (¹⁴C). Isotopes of nitrogen include nitrogen-13 (¹³N), nitrogen-14 (¹⁴N) and nitrogen-15 (¹⁵N). Isotopes of oxygen include oxygen-14 (¹⁴O), oxygen-15 (¹⁵O), oxygen-16 (¹⁶O), oxygen-17 (¹⁷O), and oxygen-18 (¹⁸O). Isotope of fluorine include fluorine-17 (¹⁷F), fluorine-18 (¹⁸F) and fluorine-19 (¹⁹F). Isotopes of phosphorous include phosphorus-31 (³¹P), phosphorus-32 (³²P), phosphorus-33 (³³P), phosphorus-34 (³⁴P), phosphorus-35 (³⁵P) and phosphorus-36 (³⁶P). Isotopes of sulfur include sulfur-32 (³²S), sulfur-33 (³³S), sulfur-34 (³⁴S), sulfur-35 (³⁵S), sulfur-36 (³⁶S) and sulfur-38 (³⁸S). Isotopes of chlorine include chlorine-35 (³⁵Cl), chlorine-36 (³⁶Cl) and chlorine-37 (³⁷Cl). Isotopes of bromine include bromine-75 (⁷⁵Br), bromine-76 (⁷⁶Br), bromine-77 (⁷⁷Br), bromine-79 (⁷⁹Br), bromine-81 (⁸¹Br) and bromine-82 (⁸²Br). Isotopes of iodine include iodine-123 (¹²³I), iodine-124 (¹²⁴I), iodine-125 (¹²⁵I), iodine-131 (¹³¹I) and iodine-135 (¹³⁵I). In some embodiments, atoms at every position of the compound have an isotopic distribution for each chemical element in proportional amounts to those usually found in nature. In some embodiments, an atom in one position of the compound has an isotopic distribution for a chemical element in proportional amounts different to those usually found in nature (remainder atoms having an isotopic distribution for a chemical element in proportional amounts to those usually found in nature). In some embodiments, atoms in at least two positions of the compound independently have an isotopic distribution for a chemical element in proportional amounts different to those usually found in nature (remainder atoms having an isotopic distribution for a chemical element in proportional amounts to those usually found in nature). In some embodiments, atoms in at least three positions of the compound independently have an isotopic distribution for a chemical element in proportional amounts different to those usually found in nature (remainder atoms having an isotopic distribution for a chemical element in proportional amounts to those usually found in nature). In some embodiments, atoms in at least four positions of the compound independently have an isotopic distribution for a chemical element in proportional amounts different to those usually found in nature (remainder atoms having an isotopic distribution for a chemical element in proportional amounts to those usually found in nature). In some embodiments, atoms in at least five positions of the compound independently have an isotopic distribution for a chemical element in proportional amounts different to those usually found in nature (remainder atoms having an isotopic distribution for a chemical element in proportional amounts to those usually found in nature). In some embodiments, atoms in at least six positions of the compound independently have an isotopic distribution for a chemical element in proportional amounts different to those usually found in nature (remainder atoms having an isotopic distribution for a chemical element in proportional amounts to those usually found in nature).

Certain compounds, for example those having incorporated radioactive isotopes such as ³H and ¹⁴C, are also useful in drug or substrate tissue distribution assays. Tritium (³H) and carbon-14 (¹⁴C) isotopes are particularly preferred for their ease of preparation and detectability. Compounds with isotopes such as deuterium (²H) in proportional amounts greater than usually found in nature may afford certain therapeutic advantages resulting from greater metabolic stability, such as, for example, increased *in vivo* half-life or reduced dosage requirements. Isotopically-labeled compounds can generally be prepared by performing procedures routinely practiced in the chemical art. Methods are readily available to measure such isotope perturbations or enrichments, such as, mass spectrometry, and for isotopes that are radio-isotopes additional methods are available, such as, radio-detectors used in connection with HPLC or GC.

As used herein, "isotopic variant" means a compound that contains an unnatural proportion of an isotope at one or more of the atoms that constitute such a compound. In certain embodiments, an "isotopic variant" of a compound contains unnatural proportions of one or more isotopes, including, but not limited to, protium (¹H), deuterium (²H), tritium (³H), carbon-11 (¹¹C), carbon-12 (¹²C), carbon-13 (¹³C), carbon-14 (¹⁴C), nitrogen-13 (¹³N), nitrogen-14 (¹⁴N), nitrogen-15 (¹⁵N), oxygen-14 (¹⁴O), oxygen-15 (¹⁵O), oxygen-16 (¹⁶O), oxygen-17 (¹⁷O), oxygen-18 (¹⁸O), fluorine-17 (¹⁷F), fluorine-18 (¹⁸F), phosphorus-31 (³¹P), phosphorus-32 (³²P), phosphorus-33 (³³P), sulfur-32 (³²S), sulfur-33 (³³S), sulfur-34 (³⁴S), sulfur-35 (³⁵S), sulfur-36 (³⁶S), chlorine-35 (³⁵Cl), chlorine-36 (³⁶Cl), chlorine-37 (³⁷Cl), bromine-79 (⁷⁹Br), bromine-81 (⁸¹Br), iodine-123 (¹²³I), iodine-125 (¹²⁵I), iodine-127 (¹²⁷I), iodine-129 (¹²⁹I), and iodine-131 (¹³¹I). In certain embodiments, an "isotopic variant" of a compound is in a stable form, that is, non-radioactive. In certain embodiments, an "isotopic variant" of a compound contains unnatural proportions of one or more isotopes, including, but not limited to, hydrogen (¹H), deuterium (²H), carbon-12 (¹²C), carbon-13 (¹³C), nitrogen-14 (¹⁴N), nitrogen-15 (¹⁵N), oxygen-16 (¹⁶O), oxygen-17 (¹⁷O), and oxygen-18 (¹⁸O). In certain embodiments, an "isotopic variant" of a compound is in an unstable form, that is, radioactive. In certain embodiments, an "isotopic variant" of a compound of the invention contains unnatural proportions of one or more isotopes, including, but not limited to, tritium (³H), carbon-11 (¹¹C), carbon-14 (¹⁴C), nitrogen-13 (¹³N), oxygen-14 (¹⁴O), and oxygen-15 (¹⁵O). It will be understood that, in a compound as provided herein, any hydrogen can include ²H as the major isotopic form, as example, or any carbon include be 13C as the major isotopic form, as example, or any nitrogen can include ¹⁵N as the major isotopic form, as example, and any oxygen can include ¹⁸O as the major isotopic form, as example. In certain embodiments, an "isotopic variant" of a compound contains an unnatural proportion of deuterium (³H).

With regard to the compounds provided herein, when a particular atomic position is designated as having deuterium or "D" or "d", it is understood that the abundance of deuterium at that position is substantially greater than the natural abundance of deuterium, which is about 0.015%. A position designated as having deuterium typically has a minimum isotopic enrichment factor of, in certain embodiments, at least 3500 (52.5% deuterium incorporation), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation) at each designated deuterium position.

Synthetic methods for incorporating radio-isotopes into organic compounds are applicable to compounds of the invention and are well known in the art. These synthetic methods, for example, incorporating activity levels of tritium into target molecules, are as follows:
A. Catalytic Reduction with Tritium Gas: This procedure normally yields high specific activity products and requires halogenated or unsaturated precursors.
B. Reduction with Sodium Borohydride [³H]: This procedure is rather inexpensive and requires precursors containing reducible functional groups such as aldehydes, ketones, lactones, esters and the like.
C. Reduction with Lithium Aluminum Hydride [³H]: This procedure offers products at almost theoretical specific activities. It also requires precursors containing reducible functional groups such as aldehydes, ketones, lactones, esters and the like.
D. Tritium Gas Exposure Labeling: This procedure involves exposing precursors containing exchangeable protons to tritium gas in the presence of a suitable catalyst.
E. N-Methylation using Methyl Iodide [³H]: This procedure is usually employed to prepare O-methyl or N-methyl (³H) products by treating appropriate precursors with high specific activity methyl iodide (³H). This method in general allows for higher specific activity, such as for example, about 70-90 Ci/mmol.

Synthetic methods for incorporating activity levels of ¹²⁵I into target molecules include:
A. Sandmeyer and like reactions: This procedure transforms an aryl amine or a heteroaryl amine into a diazonium salt, such as a diazonium tetrafluoroborate salt and subsequently to ¹²⁵I labeled compound using Na¹²⁵I. A representative procedure was reported by Zhu, G-D. and co-workers in J. Org. Chem., 2002, 67, 943-948.
B. Ortho ¹²⁵lodination of phenols: This procedure allows for the incorporation of ¹²⁵I at the ortho position of a phenol as reported by Collier, T. L. and co-workers in J. Labelled Compd. Radiopharm., 1999, 42, S264-S266.
C. Aryl and heteroaryl bromide exchange with ¹²⁵I: This method is generally a two step process. The first step is the conversion of the aryl or heteroaryl bromide to the corresponding tri-alkyltin intermediate using for example, a Pd catalyzed reaction *[i.e.* Pd(Ph₃P)₄] or through an aryl or heteroaryl lithium, in the presence of a tri-alkyltinhalide or hexaalkylditin [e.g., (CH₃)₃SnSn(CH₃)₃]. A representative procedure was reported by Le Bas, M.-D. and co-workers in J. Labelled Compd. Radiopharm., 2001, 44, S280-S282.

A radiolabeled form of a compound of the invention can be used in a screening assay to identify/evaluate compounds. In general terms, a newly synthesized or identified compound (*i.e.,* test compound) can be evaluated for its ability to reduce binding of a radiolabeled form of a compound disclosed herein to the M₄ receptor. The ability of a test compound to compete with a radiolabeled form of a compound of the invention for the binding to the M₄ receptor correlates to its binding affinity.

### DISORDERS, USES, AND METHODS OF TREATMENT

The compounds disclosed and described herein are muscarinic receptor antagonists. Accordingly, the compounds and salts or their polymorphs thereof can be used in methods of antagonizing a muscarinic receptor (e.g., muscarinic receptor 4) by contacting the receptor. In some embodiments, the compounds and salts or polymorphs thereof can be used in methods of antagonizing muscarinic receptor 4 (i.e., M₄) in a patient in need thereof by administering an effective amount of a compound or salt thereof. In some embodiments, the contacting is in vivo. In some embodiments, the contacting is ex vivo.

The compounds provided herein can be selective. As used herein, the term "selective" is meant that the compound antagonizes the M₄ receptor with greater affinity or potency, compared to at least one other muscarinic receptor (e.g., M₁, M₂, M₃, and/or M₅). In some embodiments, selectivity is at least about 2-fold, 3-fold, 5-fold, 10-fold, 20-fold, 50-fold, or 100-fold over at least one other muscarinic receptor as measured by the assays described herein.

Methods are provided herein for treating or preventing (i.e., reducing the likelihood of occurrence) a neurological disease/disorder or symptom, including but not limited to Tourette's syndrome (TS), Alzheimer's Disease (AD), schizophrenia, Lewy Body Dementia (LBD), cognitive deficits associated with schizophrenia, Parkinson's Disease, parkinsonism, tremor, dyskinesias, excessive daytime sleepiness, dystonia, chorea, levodopa induced dyskinesia, attention deficit hyperactivity disorder (ADHD), cerebral palsy, progressive supranuclear palsy (PSP), Multiple System Atrophy (MSA), Huntington's disease (HD), and chorea associate with Huntington's disease. While some of these diseases/disorders or symptoms are considered cognitive disorders (e.g., Alzheimer's Disease), and other diseases are considered neurological movement diseases/disorders, several have both cognitive and movement deficiencies or conditions associated with them (e.g., Parkinson's Disease, Huntington's disease).

The effectiveness of a muscarinic receptor antagonist, such as a M₄ antagonist, with respect to treating a neurological condition, disease or disorder or symptom described herein can readily be determined by a person skilled in the medical and clinical arts. One or any combination of diagnostic methods appropriate for the particular disease or disorder or symptom, which methods are well known to a person skilled in the art, including physical examination, patient self-assessment, assessment and monitoring of clinical symptoms, performance of analytical tests and methods, including clinical laboratory tests, physical tests, and exploratory surgery, for example, may be used for monitoring the health status of the subject and the effectiveness of the antagonist. The effects of the methods of treatment described herein can be analyzed using techniques known in the art, such as comparing symptoms of patients suffering from or at risk of a particular disease or disorder that have received the pharmaceutical composition comprising an antagonist to those patients who were not treated with the antagonist or who received a placebo treatment.

The compounds disclosed herein (and pharmaceutically acceptable salts or polymorphs thereof) are useful in the treatment or prevention of several diseases, disorders, conditions, or symptoms. One of skill in the art will recognize that when a disease, disorder, or symptom, or a method of treatment or prevention, is disclosed herein, such disclosure encompasses second medical uses (e.g., a compound or a pharmaceutically acceptable salt or a polymorph thereof for use in the treatment of the disease, disorder or symptom, use of a compound or a pharmaceutically acceptable salt or a polymorph thereof for the treatment of the disease, disorder or symptom, and use of a compound or a pharmaceutically acceptable salt or a polymorph thereof in the manufacture of a medicament for the treatment of the disease, disorder, or symptom).

In some embodiments, the compounds disclosed herein (and pharmaceutically acceptable salts or polymorphs thereof) are useful for the treatment or prevention of a disease, disorder or a symptom. In some embodiments, the compounds disclosed herein (and pharmaceutically acceptable salts or polymorphs thereof) are useful for the treatment or prevention of a subtype of a disease, disorder, or a symptom. In some embodiments, the compounds disclosed herein (and pharmaceutically acceptable salts or polymorphs thereof) are useful for the treatment or prevention of a symptom of a disease or disorder.

Provided herein are methods for treating or preventing a neurological disease, disorder, or symptom with a compound of the present invention (and pharmaceutically acceptable salts or polymorphs thereof). In some embodiments are methods for treating a neurological disease, disorder, or symptom with a compound of the present invention (and pharmaceutically acceptable salts or polymorphs thereof). In some embodiments are methods for preventing a neurological disease, disorder, or symptom with a compound of the present invention (and pharmaceutically acceptable salts or polymorphs thereof).

Provided herein are compounds of the present invention (and pharmaceutically acceptable salts or polymorphs thereof) that are useful for treating or preventing a neurological disease, disorder, or symptom. Provided herein are compounds of the present invention (and pharmaceutically acceptable salts or polymorphs thereof) that are useful for treating a neurological disease, disorder, or symptom. Provided herein are compounds of the present invention (and pharmaceutically acceptable salts or polymorphs thereof) that are useful for preventing a neurological disease, disorder, or symptom associated with M₄ activity.

One aspect of the present invention relates to methods for treating or preventing a neurological disease, disorder, or symptom in an individual, comprising administering to the individual in need thereof, a therapeutically effective amount of a compound according of the present invention or a pharmaceutically acceptable salt thereof; a pharmaceutical product of the present invention; or a pharmaceutical composition of the present invention.

One aspect of the present invention relates to methods for treating or preventing a muscarinic receptor 4 (M₄) mediated disease, disorder or symptom in an individual, comprising administering to said individual in need thereof, a therapeutically effective amount of a compound according of the present invention or a pharmaceutically acceptable salt or polymorph thereof; a pharmaceutical product of the present invention; or a pharmaceutical composition of the present invention.

One aspect of the present invention relates to uses of a compound of the present invention or a pharmaceutically acceptable salt or polymorph thereof in the manufacture of a medicament for treating or preventing a neurological disease, disorder, or symptom in an individual.

One aspect of the present invention relates to uses of a compound of the present invention or a pharmaceutically acceptable salt or polymorph thereof in the manufacture of a medicament for treating or preventing a muscarinic receptor 4 (M₄) mediated disease, disorder or symptom in an individual.

One aspect of the present invention relates to compounds of the present invention or a pharmaceutically acceptable salt or polymorph thereof; pharmaceutical products of the present invention; or pharmaceutical compositions of the present invention; for use in a method of treatment or prophylaxis of the human or animal body by therapy.

One aspect of the present invention relates to compounds of the present invention or a pharmaceutically acceptable salt or polymorph thereof; pharmaceutical products of the present invention; or pharmaceutical compositions of the present invention; for use in a method for treating or preventing a neurological disease, disorder, or symptom in an individual.

One aspect of the present invention relates to compounds of the present invention or a pharmaceutically acceptable salt or polymorph thereof; pharmaceutical products of the present invention; or pharmaceutical compositions of the present invention; for use in a method for treating or preventing a muscarinic receptor 4 (M₄) mediated neurological disease, disorder, or symptom in an individual.

One aspect of the present invention relates to use of a compound, a pharmaceutically acceptable salt, or a crystalline form thereof for treatment of a neurological disease, disorder or symptom in a patient.

One aspect of the present invention relates to use of a compound, a pharmaceutically acceptable salt, or a crystalline form thereof for manufacture of a medicament for treating a neurological disease, disorder or symptom in a patient. In some embodiments, the neurological disease, disorder, or symptom is selected from Tourette's syndrome (TS), Alzheimer's Disease (AD), schizophrenia, Lewy Body Dementia (LBD), cognitive deficits associated with schizophrenia, Parkinson's Disease, parkinsonism, tremor, dyskinesias, excessive daytime sleepiness, dystonia, chorea, levodopa induced dyskinesia, attention deficit hyperactivity disorder (ADHD), cerebral palsy, progressive supranuclear palsy (PSP), Multiple System Atrophy (MSA), Huntington's disease (HD), and chorea associate with Huntington's disease.

In some embodiments, the neurological disease, disorder, or symptom is Tourette's syndrome (TS).

In some embodiments, the neurological disease, disorder, or symptom is schizophrenia.

In some embodiments, the neurological disease, disorder, or symptom is progressive supranuclear palsy.

In some embodiments, the neurological disease, disorder, or symptom is tremor. In some further embodiments, the neurological disease, disorder, or symptom is parkinsonian tremor.

In some embodiments, the neurological disease, disorder, or symptom is parkinsonism. In some further embodiments, the parkinsonism is drug induced parkinsonism. In some further embodiments, one or more symptoms of parkinsonism is selected from tremor, bradykinesia, rigidity, and postural instability.

In some embodiments, the neurological disease, disorder, or symptom is Parkinson's disease (PD).

In some embodiments, the neurological disease, disorder, or symptom is Lewy body dementia (LBD).

In some embodiments, the neurological disease, disorder, or symptom is levodopa induced dyskinesia.

In some embodiments, the neurological disease, disorder, or symptom is Huntington's disease (HD).

In some embodiments, the neurological disease, disorder, or symptom is excessive daytime sleepiness.

In some embodiments, the neurological disease, disorder, or symptom is dystonia. In some embodiments, the dystonia is generalized dystonia. In some further embodiments, the generalized dystonia is Oppenheim's dystonia or DYT1 dystonia. In some other further embodiments, the generalized dystonia is non-DYT1 generalized dystonia. In some embodiments, the dystonia is focal dystonia. In some embodiments, the dystonia is caused by infections. In some embodiments, the dystonia is caused by birth injury. In a further embodiment, the birth injury is cerebral palsy.

In some embodiments, the neurological disease, disorder, or symptom is dyskinesias.

In some embodiments, the neurological disease, disorder, or symptom is cognitive deficits associated with schizophrenia.

In some embodiments, the neurological disease, disorder, or symptom is chorea.

In some embodiments, the neurological disease, disorder, or symptom is chorea associated with Huntington's disease (HD).

In some embodiments, the neurological disease, disorder, or symptom is cerebral palsy.

In some embodiments, the neurological disease, disorder, or symptom is attention deficit hyperactivity disorder (ADHD).

In some embodiments, the neurological disease, disorder, or symptom is Alzheimer's disease (AD).

As used herein, the term "subject" refers to any animal, including mammals, preferably mice, rats, other rodents, rabbits, dogs, cats, swine, cattle, sheep, horses, or primates, and most preferably humans. In the context of a clinical trial or screening or activity experiment the subject may be a healthy volunteer or healthy participant without an underlying M4 mediated disorder or condition or a volunteer or participant that has received a diagnosis for a disorder or condition in need of medical treatment as determined by a health care professional. In the context outside of a clinical trial a subject under the care of a health care professional who has received a diagnosis for a disorder or condition is typically described as a patient.

The term "pediatric subject" as used herein refers to a subject under the age of 21 years at the time of diagnosis or treatment. The term "pediatric" can be further divided into various subpopulations including: neonates (from birth through the first month of life); infants (1 month up to two years of age); children (two years of age up to 12 years of age); and adolescents (12 years of age through 21 years of age (up to, but not including, the twenty-second birthday)) see *e.g.,* Berhman et al., Textbook of Pediatrics, 15th Ed. Philadelphia: W.B. Saunders Company, 1996; Rudolph et al., Rudolph's Pediatrics, 21st Ed. New York: McGraw-Hill, 2002; and Avery et al., Pediatric Medicine, 2nd Ed. Baltimore: Williams & Wilkins; 1994.

As used herein, the terms "treat" and "treatment" refer to medical management of a disease, disorder, symptom, or condition of a subject (i.e., patient) (see, e.g., Stedman's Medical Dictionary). In general, an appropriate dose and treatment regimen provide the M4 antagonist in an amount sufficient to provide therapeutic and/or prophylactic benefit. The term "treat" or "treatment" includes slowing, retarding, reducing, or reversing a disease, disorder, or an undesired physiological change or a symptom associated with the disease or disorder. The term "treat" or "treatment" also includes preventing, slowing, or retarding the expansion or severity of such disease, disorder, or symptom. As discussed herein, effectiveness of the treatment by the one or more M4 antagonists may include beneficial or desired clinical results that comprise, but are not limited to, abatement, lessening, or alleviation of symptoms that result from or are associated with the disease or disorder to be treated; decreased occurrence of symptoms associated with the disease or disorder to be treated; improved quality of life; longer disease-free status (i.e., decreasing the likelihood or the propensity that a subject will present symptoms on the basis of which a diagnosis of a disease or disorder is made); diminishment of extent of disease or disorder; stabilized (i.e., not worsening) state of disease or disorder; delay or slowing of disease or disorder progression; amelioration or palliation of the disease or disorder state; and remission (whether partial or total), whether detectable or undetectable; and/or overall survival.

The term "treat" and "treatment" can also mean prolonging survival when compared to expected survival if a subject were not receiving treatment. Subjects in need of treatment include those who already have the disease or disorder as well as subjects prone to have or at risk of developing the disease or disorder, and those in which the disease, condition, disorder, or symptom is to be prevented (i.e., decreasing the likelihood of occurrence or recurrence of the disease or disorder).

The term "preventing," as used herein, means the prevention of the onset, recurrence or spread, in whole or in part, of the disease or condition as described herein, or a symptom thereof.

The term "administration" or "administering" refers to a method of giving a dosage of a compound or pharmaceutical formulation to a vertebrate or invertebrate, including a mammal, a bird, a fish, or an amphibian. The preferred method of administration can vary depending on various factors, e.g., the components of the pharmaceutical formulation, the site of the disease, and the severity of the disease.

As used herein, "therapeutically effective amount" is an amount of the compound of the invention, or a pharmaceutically acceptable salt thereof, or an amount of a pharmaceutical composition comprising the compound of the invention, or a pharmaceutically acceptable salt thereof, which is sufficient to achieve the desired effect and can vary according to the nature and severity of the disease condition, and the potency of the compound. A therapeutic effect is the relief, to some extent, of one or more of the symptoms of the disease, and can include curing a disease. "Curing" means that the symptoms of active disease are eliminated. However, certain long-term or permanent effects of the disease can exist even after a cure is obtained (such as, *e.g*., extensive tissue damage).

### PHARMACEUTICAL COMPOSITIONS, FORMULATION, AND DOSAGE FORMS

The present disclosure further provides for compositions comprising any of the compounds of the present invention as disclosed and described herein (*e.g*., a compound of Formula (la), including specific compounds described herein) or pharmaceutically acceptable salts thereof, and an excipient such as a pharmaceutically acceptable excipient for use in the methods for treating M₄ mediated diseases or disorders, such as a neurological diseases or disorders. A pharmaceutically acceptable excipient is a physiologically and pharmaceutically suitable non-toxic and inactive material or ingredient that does not interfere with the activity of the drug substance; an excipient also may be called a carrier. The formulation methods and excipients described herein are exemplary and are in no way limiting. Pharmaceutically acceptable excipients are well known in the pharmaceutical art and described, for example, in Rowe et al., Handbook of Pharmaceutical Excipients: A Comprehensive Guide to Uses, Properties, and Safety, 5th Ed., 2006, and in Remington: The Science and Practice of Pharmacy (Gennaro, 21 st Ed. Mack Pub. Co., Easton, PA (2005)). Exemplary pharmaceutically acceptable excipients include sterile saline and phosphate buffered saline at physiological pH. Preservatives, stabilizers, dyes, buffers, and the like may be provided in the pharmaceutical composition. In addition, antioxidants and suspending agents may also be used.

For compositions formulated as liquid solutions, acceptable carriers and/or diluents include saline and sterile water, and may optionally include antioxidants, buffers, bacteriostats and other common additives. The compositions can also be formulated as pills, capsules, granules, or tablets which contain, in addition to an M₄ antagonist, diluents, dispersing and surface active agents, binders, and lubricants. One skilled in this art may further formulate the M₄ antagonist in an appropriate manner, and in accordance with accepted practices, such as those disclosed in Remington, *supra.*

Methods of administration include systemic administration of an M₄ antagonist described herein, preferably in the form of a pharmaceutical composition as discussed above. As used herein, systemic administration includes oral and parenteral methods of administration. For oral administration, suitable pharmaceutical compositions include powders, granules, pills, tablets, and capsules as well as liquids, syrups, suspensions, and emulsions. These compositions may also include flavorants, preservatives, suspending, thickening and emulsifying agents, and other pharmaceutically acceptable additives. For parental administration, the compounds of the present invention (or pharmaceutically acceptable salts thereof) can be prepared in aqueous injection solutions which may contain, in addition to the M₄ antagonist, buffers, antioxidants, bacteriostats, and other additives commonly employed in such solutions.

Pharmaceutical preparations for oral administration can be obtained by any suitable method, typically by uniformly mixing the compound(s) with liquids or finely divided solid carriers, or both, in the required proportions and then, if necessary, processing the mixture, after adding suitable auxiliaries, if desired, forming the resulting mixture into a desired shape to obtain tablets or dragee cores.

Conventional excipients, such as binding agents, fillers, adjuvant, carrier, acceptable wetting agents, tabletting lubricants and disintegrants may be used in tablets and capsules for oral administration. Liquid preparations for oral administration may be in the form of solutions, emulsions, aqueous or oily suspensions and syrups. Alternatively, the oral preparations may be in the form of dry powder that can be reconstituted with water or another suitable liquid vehicle before use. Additional additives such as suspending or emulsifying agents, non-aqueous vehicles (including edible oils), preservatives and flavorings and colorants may be added to the liquid preparations. Parenteral dosage forms may be prepared by dissolving the compound of the invention in a suitable liquid vehicle and filter sterilizing the solution before lyophilization, or simply filling and sealing an appropriate vial or ampule.

As used herein, **"drug substance",** defined in the context of a "pharmaceutical composition," refers to a component of a pharmaceutical composition such as any one of the compounds as disclosed and described herein that provides the primary pharmacological effect, as opposed to an "inactive ingredient" which would generally be recognized as providing no therapeutic benefit.

As used herein, an **"excipient"** refers to a substance that is added to a composition to provide, without limitation, bulk, consistency, stability, binding ability, lubrication, disintegrating ability, *etc.,* to the composition. A **"diluent"** is a type of excipient and refers to an ingredient in a pharmaceutical composition that lacks pharmacological activity but may be pharmaceutically necessary or desirable. For example, a diluent may be used to increase the bulk of a potent drug whose mass is too small for manufacture and/or administration. It may also be a liquid for the dissolution of a drug to be administered by injection, ingestion, or inhalation. A pharmaceutically acceptable excipient is a physiologically and pharmaceutically suitable non-toxic and inactive material or ingredient that does not interfere with the activity of the drug substance. Pharmaceutically acceptable excipients are well known in the pharmaceutical art and described, for example, in Rowe et al., Handbook of Pharmaceutical Excipients: A Comprehensive Guide to Uses, Properties, and Safety, 5th Ed., 2006, and in Remington: The Science and Practice of Pharmacy (Gennaro, 21 st Ed. Mack Pub. Co., Easton, PA (2005)). Preservatives, stabilizers, dyes, buffers, and the like may be provided in the pharmaceutical composition. In addition, antioxidants and suspending agents may also be used. For compositions formulated as liquid solutions, acceptable carriers and/or diluents include saline and sterile water, and may optionally include antioxidants, buffers, bacteriostats and other common additives. In some embodiments, the diluents may be a buffered aqueous solution such as, without limitation, phosphate buffered saline. The compositions can also be formulated as capsules, granules, or tablets which contain, in addition to a compound as disclosed and described herein, diluents, dispersing and surface-active agents, binders, and lubricants. One skilled in this art may further formulate a compound as disclosed and described herein in an appropriate manner, and in accordance with accepted practices, such as those disclosed in Remington, *supra.*

One aspect of the present invention relates to methods for preparing a pharmaceutical composition comprising the step of admixing a compound according of the present invention or a pharmaceutically acceptable salt thereof, and a pharmaceutically acceptable carrier.

In making pharmaceutical compositions comprising compounds of the present invention, or pharmaceutically acceptable salts thereof, the drug substance is typically mixed (*i.e.,* admixed) with an excipient, diluted by an excipient or enclosed within such a carrier in the form of, for example, a capsule, sachet, paper, or other container. When the excipient serves as a diluent, it can be a solid, semi-solid, or liquid material, which acts as a vehicle, carrier, or medium for the drug substance. Thus, the compositions can be in the form of tablets, powders, lozenges, sachets, cachets, elixirs, suspensions, emulsions, solutions, syrups, aerosols (as a solid or in a liquid medium), ointments, soft and hard gelatin capsules, suppositories, sterile injectable solutions, and sterile packaged powders.

For preparing solid form pharmaceutical compositions such as powders, tablets, capsules, cachets, suppositories and dispersible granules an excipient can be one or more substances which may also act as diluents, flavoring agents, solubilizers, lubricants, suspending agents, binders, preservatives, tablet disintegrating agents, or an encapsulating material. Also included are solid form preparations which are intended to be converted, shortly before use, to liquid form preparations for oral administration. Such liquid forms include solutions, suspensions and emulsions. These preparations may contain, in addition to the drug substance, colorants, flavors, stabilizers, buffers, artificial and natural sweeteners, dispersants, thickeners, solubilizing agents and the like.

For preparing suppositories, a low melting wax, such as an admixture of fatty acid glycerides or cocoa butter, is first melted and the drug substance is dispersed homogeneously therein, as by stirring. The molten homogenous mixture is then poured into convenient sized molds, allowed to cool and thereby to solidify.

Formulations suitable for vaginal administration may be presented as pessaries, tampons, creams, gels, pastes, foams or sprays containing in addition to the drug substance such carriers as are known in the art to be appropriate.

Liquid form preparations include solutions, suspensions and emulsions, for example, water or water-propylene glycol solutions. For example, parenteral injection liquid preparations can be formulated as solutions in aqueous polyethylene glycol solution. Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid find use in the preparation of injectables.

The pharmaceutical compositions may take such forms as suspensions, solutions, or emulsions in oily or aqueous vehicles and may contain formulatory agents such as suspending, stabilizing and/or dispersing agents. Alternatively, the pharmaceutical compositions may be in powder form, obtained by aseptic isolation of sterile solid or by lyophilization from solution, for constitution with a suitable vehicle, e.g., sterile, pyrogen-free water, before use.

The pharmaceutical compositions may be formulated as an aqueous solution, an aqua-alcoholic solution, a solid suspension, an emulsion, a liposomal suspension, or a freeze-dried powder for reconstitution. Such pharmaceutical compositions may be administered directly or as an admixture for further dilution/reconstitution. Route of administration includes intravenous bolus, intravenous infusion, irrigation, and instillation. Suitable solvents include water, alcohols, PEG, propylene glycol, and lipids; pH adjustments using an acid, e.g., HCl or citric acid, can be used to increase solubility and resulting compositions subjected to suitable sterilization procedures know in the art, such as, aseptic filtration. In some embodiments, the pH of the aqueous solution is about 2.0 to about 4.0. In some embodiments, the pH of the aqueous solution is about 2.5 to about 3.5.

Aqueous formulations suitable for oral use can be prepared by dissolving or suspending the drug substance in water and adding suitable colorants, flavors, stabilizing and thickening agents, as desired.

Aqueous suspensions suitable for oral use can be made by dispersing the finely divided drug substance in water with viscous material, such as natural or synthetic gums, resins, methylcellulose, sodium carboxymethylcellulose, or other well-known suspending agents.

For topical administration to the epidermis the compounds of the present invention, or pharmaceutically acceptable salts thereof may be formulated as gels, ointments, creams or lotions, or as a transdermal patch. Also, formulations suitable for topical administration in the mouth include lozenges comprising drug substance in a flavored base, usually sucrose and acacia or tragacanth; pastilles comprising the drug substance in an inert base such as gelatin and glycerin or sucrose and acacia; and mouthwashes comprising the drug substance in a suitable liquid carrier. Ointments and creams may, for example, be formulated with an aqueous or oily base with the addition of suitable thickening and/or gelling agents. Lotions may be formulated with an aqueous or oily base and will in general also contain one or more emulsifying agents, stabilizing agents, dispersing agents, suspending agents, thickening agents, or coloring agents. In some embodiments, topical formulations can contain one or more conventional carriers. In some embodiments, ointments can contain water and one or more hydrophobic carriers selected from, for example, liquid paraffin, polyoxyethylene alkyl ether, propylene glycol, white vaseline, and the like. Carrier compositions of creams can be based on water in combination with glycerol and one or more other components, *e.g*., glycerinemonostearate, PEG-glycerinemonostearate and cetylstearyl alcohol. Gels can be formulated using isopropyl alcohol and water, suitably in combination with other components such as, for example, glycerol, hydroxyethyl cellulose, and the like.

Solutions or suspensions may be applied directly to the nasal cavity by conventional means, for example with a dropper, pipette or spray. The formulations may be provided in single or multi-dose form. In the latter case of a dropper or pipette, this may be achieved by the patient administering an appropriate, predetermined volume of the solution or suspension. In the case of a spray, this may be achieved for example by means of a metering atomizing spray pump.

Administration to the respiratory tract may also be achieved by means of an aerosol formulation provided in a pressurized pack with a suitable propellant. If the compounds of the present invention, or pharmaceutically acceptable salts thereof or pharmaceutical compositions comprising them are administered as aerosols, for example as nasal aerosols or by inhalation, this can be carried out, for example, using a spray, a nebulizer, a pump nebulizer, an inhalation apparatus, a metered inhaler or a dry powder inhaler. Pharmaceutical forms for administration of the compounds of the present invention (or pharmaceutically acceptable salts thereof) as an aerosol can be prepared by processes well known to the person skilled in the art. For their preparation, for example, solutions or dispersions of the compounds of the present invention (or pharmaceutically acceptable salts thereof) in water, water/alcohol mixtures or suitable saline solutions can be employed using customary additives, for example benzyl alcohol or other suitable preservatives, absorption enhancers for increasing the bioavailability, solubilizers, dispersants and others and, if appropriate, customary propellants, for example include carbon dioxide, CFCs, such as, dichlorodifluoromethane, trichlorofluoromethane, or dichlorotetrafluoroethane; and the like. The aerosol may conveniently also contain a surfactant such as lecithin. The dose of drug may be controlled by provision of a metered valve.

Alternatively, the pharmaceutical composition may be provided in the form of a dry powder, for example, a powder mix of the compound in a suitable, powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, *e.g*., gelatin, or blister packs from which the powder may be administered by means of an inhaler.

The compounds of the present invention, or pharmaceutically acceptable salts thereof may also be administered via a rapid dissolving or a slow release composition, wherein the composition includes a biodegradable rapid dissolving or slow release carrier (such as a polymer carrier and the like). Rapid dissolving or slow release carriers are well known in the art and are used to form complexes that capture therein compounds of the present invention, or pharmaceutically acceptable salts thereof and either rapidly or slowly degrade/dissolve in a suitable environment (*e.g.,* aqueous, acidic, basic, *etc*.)*.*

The pharmaceutical preparations are preferably in unit dosage forms. In such form, the preparation is subdivided into unit doses containing appropriate quantities of the drug substance. The unit dosage form can be a packaged preparation, the package containing discrete quantities of preparation, such as packeted tablets, capsules and powders in vials or ampoules. Also, the unit dosage form can be a capsule, tablet, cachet, or lozenge itself, or it can be the appropriate number of any of these in packaged form.

Tablets or capsules for oral administration and liquids for intravenous administration are preferred compositions.

The compositions can be formulated in a unit dosage form, each dosage containing the drug substance or equivalent mass of the drug substance. The term "unit dosage forms" refers to physically discrete units of a formulation suitable as unitary dosages for human subjects and other mammals, each unit containing a predetermined quantity of drug substance calculated to produce the desired therapeutic effect, in association with a suitable excipient, as described herein.

The compositions described herein can be formulated to provide immediate and/or timed release (also called extended release, sustained release, controlled release, or slow release) of the drug substance after administration to a subject by employing procedures known in the art. For example, the tablets including compounds of the present invention, or pharmaceutically acceptable salts thereof, can be coated or otherwise compounded to provide a dosage form affording the advantage of prolonged action. For example, the tablet can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms including the drug substance can be incorporated for administration orally or by injection include aqueous solutions, suitably flavored syrups, aqueous or oil suspensions, and flavored emulsions with edible oils such as cottonseed oil, sesame oil, coconut oil, or peanut oil, and similar excipients.

The pharmaceutical compositions described herein can be sterilized by conventional sterilization techniques, or may be sterile filtered. Aqueous solutions can be packaged for use as is, or lyophilized, the lyophilized preparation being combined with a sterile aqueous carrier prior to administration. The pH of the compound preparations is typically between 3 and 11, more preferably from 5 to 9 and most preferably from 7 to 8. It will be understood that use of certain of the foregoing excipients may result in the formation of pharmaceutically acceptable salts.

Compositions for inhalation or insufflation include solutions and suspensions in pharmaceutically acceptable, aqueous or organic solvents, or mixtures thereof, and powders. The liquid or solid compositions may contain suitable excipients as described herein. In some embodiments, the compositions are administered by the oral or nasal respiratory route for local or systemic effect. Compositions can be nebulized by use of inert gases. Nebulized solutions may be breathed directly from the nebulizing device or the nebulizing device can be attached to a face masks tent, or intermittent positive pressure breathing machine. Solution, suspension, or powder compositions can be administered orally or nasally from devices which deliver the formulation in an appropriate manner.

The compositions may, if desired, be presented in a pack or dispenser device which may contain one or more-unit dosage forms containing the drug substance. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions that can include a compound described herein formulated in a compatible pharmaceutical carrier may also be prepared, placed in an appropriate container, and labeled for treatment of an indicated condition.

As used herein, a "dose" or "dosage" refers to the measured quantity of drug substance to be taken at one time by a patient. In certain embodiments, wherein the drug substance is not a free base or free acid, the quantity is the molar equivalent to the corresponding amount of free base or free acid.

For preparing solid compositions such as tablets, the drug substance may be mixed with an excipient to form a solid preformulation composition containing a homogeneous mixture of components. When referring to these preformulation compositions as homogeneous, the drug substance is typically dispersed evenly throughout the composition so that the composition can be readily subdivided into equally effective unit dosage forms such as tablets and capsules.

Kits with unit doses of one or more of the compounds described herein, usually in oral or injectable doses, are provided. Such kits may include a container containing the unit dose, an informational package insert describing the use and attendant benefits of the drugs in treating pathological condition of interest, and optionally an appliance or device for delivery of the composition.

### DOSING SCHEDULE / AMOUNT

Compounds of the present invention or a pharmaceutically acceptable salt thereof, may be effective over a wide dosage range and is generally administered in a therapeutically effective amount. It will be understood, however, that the amount of the compound actually administered will usually be determined by a physician, according to the relevant circumstances, including the condition to be treated, the chosen route of administration, the actual compound administered, the age, weight, and response of the individual subject, the severity of the subject's symptoms, and the like.

The amount of compound or composition administered to a subject will also vary depending upon what is being administered, the purpose of the administration, such as prophylaxis or therapy, the state of the subject, the manner of administration, and the like. In therapeutic applications, compositions can be administered to a subject already suffering from a disease in an amount sufficient to cure or at least partially arrest the symptomology and/or pathology of the disease and its complications. Therapeutically effective doses will depend on the disease condition being treated as well as by the judgment of the attending clinician depending upon factors such as the severity of the disease, the age, weight and general condition of the subject, and the like.

The desired dose may conveniently be presented in a single dose or presented as divided doses administered at appropriate intervals, for example, as two, three, four, or more sub-doses per day. The sub-dose itself may be further divided, e.g., into a number of discrete loosely spaced administrations. The daily dose can be divided, especially when relatively large amounts are administered as deemed appropriate, into several, for example two, three, or four-part administrations. If appropriate, depending on individual behavior, it may be necessary to deviate upward or downward from the daily dose indicated.

It will be apparent to those skilled in the art that the dosage forms described herein may comprise, as the drug substance, either a compound described herein or pharmaceutically acceptable salt, solvate, or hydrate thereof. Typical procedures for making and identifying suitable hydrates and solvates, outside those mentioned herein, are well known to those in the art; see for example, pages 202-209 of K.J. Guillory, "Generation of Polymorphs, Hydrates, Solvates, and Amorphous Solids," in: Polymorphism in Pharmaceutical Solids, ed. Harry G. Britain, Vol. 95, Marcel Dekker, Inc., New York, 1999. Accordingly, one aspect of the present invention pertains to methods of administering hydrates and solvates of compounds described herein and/or their pharmaceutical acceptable salts, that can be isolated and characterized by methods known in the art, such as, thermogravimetric analysis (TGA), TGA-mass spectroscopy, TGA-Infrared spectroscopy, powder X-ray diffraction (PXRD), Karl Fisher titration, high resolution X-ray diffraction, and the like.

### EXAMPLES

### SYNTHESES OF COMPOUNDS OF THE PRESENT INVENTION

Detailed compound synthesis methods are described in the Examples provided herein. A person having ordinary skill in the chemical art would be able to make a compound of Formula (la) and the formulae related thereto, including specific compounds described herein, by these methods or similar methods or other methods practiced by a person skilled in the art. In general, starting components are commercially available chemicals and may be obtained from commercial sources or may be made according to organic synthesis techniques known to those skilled in this art, starting from commercially available chemicals and/or from compounds described in the chemical literature. The compounds described herein, *supra* and *infra,* are named according to MarvinSketch 18.24.0 or ChemDraw Professional 18.2.0.48. In certain instances, when common names are used it is understood that these common names would be recognized by those skilled in the art.

In general, the compounds used in the reactions described herein may be made according to organic synthesis techniques known to those skilled in this art, starting from commercially available chemicals and/or from compounds described in the chemical literature. "Commercially available chemicals" may be obtained from standard commercial sources including Acros Organics (Pittsburgh PA), Aldrich Chemical (Milwaukee WI, including Sigma Chemical and Fluka), Apin Chemicals Ltd. (Milton Park UK), Avocado Research (Lancashire U.K.), BDH Inc. (Toronto, Canada), Bionet (Cornwall, U.K.), Chemservice Inc. (West Chester PA), Crescent Chemical Co. (Hauppauge NY), Eastman Organic Chemicals, Eastman Kodak Company (Rochester NY), Fisher Scientific Co. (Pittsburgh PA), Fisons Chemicals (Leicestershire UK), Frontier Scientific (Logan UT), ICN Biomedicals, Inc. (Costa Mesa CA), Key Organics (Cornwall U.K.), Lancaster Synthesis (Windham NH), Maybridge Chemical Co. Ltd. (Cornwall U.K.), Parish Chemical Co. (Orem UT), Pfaltz & Bauer, Inc. (Waterbury CN), Polyorganix (Houston TX), Pierce Chemical Co. (Rockford IL), Riedel de Haen AG (Hanover, Germany), Spectrum Quality Product, Inc. (New Brunswick, NJ), TCI America (Portland OR), Trans World Chemicals, Inc. (Rockville MD), and Wako Chemicals USA, Inc. (Richmond VA).

Certain intermediates are commercially available or can be prepared according to the methods provided herein, examples include, 2,5-difluoro-4-(piperazin-1-yl)benzonitrile, 3-fluoro-4-(piperazin-1-yl)benzonitrile, 1-(5-(trifluoromethyl)pyridin-2-yl)piperazine, 2-(piperazin-1-yl)-5-(trifluoromethyl)benzonitrile, 1-(4-(trifluoromethyl)phenyl)piperazine, 2-azaspiro[3.3]heptan-6-ol, *tert-*butyl *N*-{2-azaspiro[3.3]heptan-6-yl}carbamate, *tert*-butyl ((2-azaspiro[3.3]heptan-6-yl)methyl)carbamate, and *tert*-butyl 6-(hydroxymethyl)-2-azaspiro[3.3]heptane-2-carboxylate.

Methods known to one of ordinary skill in the art may be identified through various reference books and databases. Suitable reference books and treatise that detail the synthesis of reactants useful in the preparation of compounds of the present disclosure, or provide references to articles that describe the preparation, include for example, Synthetic Organic Chemistry, John Wiley & Sons, Inc., New York; S. R. Sandler et al., Organic Functional Group Preparations, 2nd Ed., Academic Press, New York, 1983; H. O. House, Modern Synthetic Reactions, 2nd Ed., W. A. Benjamin, Inc. Menlo Park, Calif. 1972; T. L. Gilchrist, Heterocyclic Chemistry, 2nd Ed., John Wiley & Sons, New York, 1992; J. March, Advanced Organic Chemistry: Reactions, Mechanisms and Structure, 4th Ed., Wiley Interscience, New York, 1992. Additional suitable reference books and treatise that detail the synthesis of reactants useful in the preparation of compounds of the present disclosure, or provide references to articles that describe the preparation, include for example, Fuhrhop, J. and Penzlin G. Organic Synthesis: Concepts, Methods, Starting Materials, Second, Revised and Enlarged Edition (1994) John Wiley & Sons ISBN: 3 527-29074-5; Hoffman, R.V. Organic Chemistry, An Intermediate Text (1996) Oxford University Press, ISBN 0-19-509618-5; Larock, R. C. Comprehensive Organic Transformations: A Guide to Functional Group Preparations, 2nd Edition (1999) Wiley-VCH, ISBN: 0-471-19031-4; March, J. Advanced Organic Chemistry: Reactions, Mechanisms, and Structure, 4th Edition (1992) John Wiley & Sons, ISBN: 0-471-60180-2; Otera, J. (editor) Modern Carbonyl Chemistry, (2000) Wiley-VCH, ISBN: 3-527-29871-1; Patai, S., Patai's 1992 Guide to the Chemistry of Functional Groups, (1992) Interscience ISBN: 0-471-93022-9; Quin, L.D. et al. A Guide to Organophosphorus Chemistry, (2000) Wiley-Interscience, ISBN: 0-471-31824-8; Solomons, T. W. G. Organic Chemistry, 7th Edition (2000) John Wiley & Sons, ISBN: 0-471-19095-0; Stowell, J.C., Intermediate Organic Chemistry, 2nd Edition (1993) Wiley-Interscience, ISBN: 0-471-57456-2; Industrial Organic Chemicals: Starting Materials and Intermediates: An Ullmann's Encyclopedia, (1999) John Wiley & Sons, ISBN: 3-527-29645-X, in 8 volumes; Organic Reactions, (1942-2019) John Wiley & Sons, in over 95 volumes; and Chemistry of Functional Groups, John Wiley & Sons, in hardcover volumes (86) and electronic volumes (26).

Specific and analogous reactants may also be identified through the indices of known chemicals prepared by the Chemical Abstract Service of the American Chemical Society, which are available in most public and university libraries, as well as through on-line databases (the American Chemical Society, Washington, D.C., may be contacted for more details). Chemicals that are known but not commercially available in catalogs may be prepared by custom chemical synthesis houses according to known methods, where many of the standard chemical supply houses (*e.g.,* those listed above) provide custom synthesis services.

### CERTAIN ABBREVIATIONS

The specification includes numerous abbreviations, whose definitions are listed in the following Table:

| **Abbreviation** | **Definition** |
|---|---|
| ACN or CH₃CN | Acetonitrile |
| BOC | *tert*-Butyloxycarbonyl |
| CDI | 1,1'-Carbonyldiimidazole |
| EtOAc | Ethyl acetate |
| DBU | 1,8-Diazabicyclo[5.4.0]undec-7-ene |
| DCC | Dicyclohexylcarbodiimide |
| DCE | Dichloroethane |
| DCM | Dichloromethane or methylene chloride |
| de | Diastereomeric excess |
| DIPEA | *N*,*N*-Diisopropylethylamine |
| DMSO | Dimethylsulfoxide |
| DMSO-*d*₆ | Dimethylsulfoxide-*d*₆ |
| ee | Enantiomeric excess |
| HPLC | High-performance liquid chromatography |
| KHMDS | Potassium bis(trimethylsilyl)amide |
| LCMS | Liquid chromatography-mass spectrometry |
| min. | Minute(s) |
| NH₄Cl | Ammonium chloride |
| Pd(PPh₃)₄ | Palladium-tetrakis(triphenylphosphine) |
| TEA | Triethylamine |
| TFA | Trifluoroacetic acid |
| THF | Tetrahydrofuran |

The following examples are included to demonstrate embodiments of the disclosure. However, those of skill in the art should, in light of the present disclosure, appreciate that many changes can be made in the specific embodiments which are disclosed and still obtain a like or similar result without departing from the scope of the disclosure.

Analytical HPLC analyses were performed on an LC-MS system with a UV Detector (Dionex^{™} UVD 170u UV/VIS Detector), Corona array detector (Thermo^{™} Veo^{™} RS), and mass spectrometer (Dionex MSQ Plus^{™}). Reverse-phase preparative HPLC purifications were performed on an LCMS system C18 Kinetix 5µ 100 A 150x21.2 mm column by Phenomenex using ACN/water gradient containing 0.05% TFA. All final compounds were analyzed by analytical HPLC and peaks were monitored at 210, 254 and 280 nM for purity. ¹H was recorded in an appropriate NMR solvent, such as, DMSO-*d*₆, on a Bruker 400 MHz spectrometer equipped with a Broad Band NMR probe. The ¹H chemical signals are given in parts per million (ppm) with the residual solvent signal used as reference. The chemical shifts are expressed in ppm (*δ*) and coupling constants (*J*) are reported in hertz (Hz). Reactions were performed under an atmosphere of dry nitrogen unless otherwise stated.

### Example 1: Preparation of Intermediate 2-Benzyl-2-azaspiro[3.3]heptan-6-amine (Intermediate 1A).

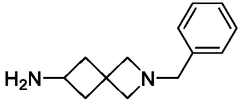

To a solution of *tert-butyl N*-{2-azaspiro[3.3]heptan-6-yl}carbamate (2.5 g, 11.8 mmol, 1.0 eq) in dichloroethane (100 mL) was added benzaldehyde (1.8 mL, 17.7 mmol, 1.5 eq) followed by sodium triacetoxyborohydride (7.5 g, 35.4 mmol, 3.0 eq). The resulting mixture was stirred at room temperature overnight. The formed suspension was carefully diluted and stirred with sat. NaHCO₃ until the evolution of hydrogen ceased. The aqueous mixture was extracted with DCM. The combined organic layers were washed with brine, dried over MgSO₄, filtered to remove solid and concentrated *in vacuo.* Silica gel column (80 g) was loaded using DCM and run with an increasing gradient of MeOH (0-15%) in DCM over 25 min to provide the Boc-protected intermediate:

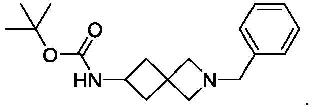

The isolated Boc-protected intermediate was re-dissolved in DCM (35 mL), treated with TFA (5 mL) and stirred at room temperature overnight. Additional TFA (2.5 mL) was added and the mixture stirred until completion. The reaction was carefully quenched with sat. NaHCO₃, brought to pH > 10 with 2M NaOH and extracted with 5:1 DCM:2-propanol. The combined organic layers were dried over MgSO₄, filtered to remove solid and concentrated *in vacuo* to provide 2-benzyl-2-azaspiro[3.3]heptan-6-amine, (**Intermediate 1A,** 2.3 g, 11.4 mmol, 97% over 2 steps) as a yellow liquid.

Other useful intermediates to prepare compounds of the present invention were made using substantially the same procedures described above, including:

| **Intermediate** | **Chemical Structure** | **Chemical Name** |
|---|---|---|
| **1B** | 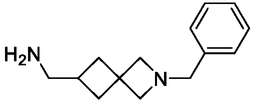 | {2-benzyl-2-azaspiro[3.3]heptan-6-yl}methanamine |
| **1C** | 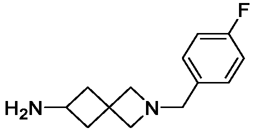 | 2-[(4-fluorophenyl)methyl]-2-azaspiro[3.3]heptan-6-amine |
| **1D** | 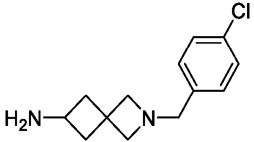 | 2-[(4-chlorophenyl)methyl]-2-azaspiro[3.3]heptan-6-amine |

### Example 2: Preparation of 2-[(2S,5R)-2,5-Dimethylpiperazin-1-yl]-5-(trifluoromethyl)pyrimidine (Intermediate 2A)

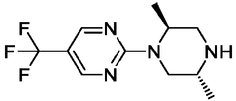

To a solid mixture of *tert*-butyl (2*R*,5*S*)-2,5-dimethylpiperazine-1-carboxylate (0.43 g, 2.0 mmol, 1.0 eq) and 2-chloro-5-(trifluoromethyl)pyrimidine (0.36 g, 2.0 mmol, 1.0 eq) was added ACN (5 mL) followed by triethylamine (1.1 mL, 8.0 mmol, 4.0 eq). The resulting mixture was stirred at 75 °C overnight. The formed suspension was concentrated *in vacuo,* re-dissolved in dioxane (6 mL), treated with 4M HCl in dioxane (1.5 mL) and stirred at 50 °C overnight. The formed suspension was concentrated *in vacuo,* diluted with sat. NaHCO₃. The aqueous mixture was extracted with DCM. The combined organic layers were dried over MgSO₄, filtered to remove solid and concentrated *in vacuo* to provide 2-[(2*S*,5*R*)-2,5-dimethylpiperazin-1-yl]-5-(trifluoromethyl)pyrimidine (**Intermediate 2A,** 0.33 g, 1.3 mmol, 65% over 2 steps) as a semi-solid.

Other intermediates useful in the preparations of compounds of the present invention were made using substantially the same procedures described above, including:

| **Intermediate** | **Chemical Structure** | **Chemical Name** |
|---|---|---|
| **2B** | 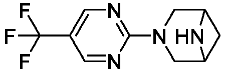 | 3-[5-(trifluoromethyl)pyrimidin-2-yl]-3,6-diazabicyclo[3.1.1]heptane |
| **2C** | 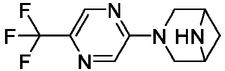 | 3-[5-(trifluoromethyl)pyrazin-2-yl]-3,6-diazabicyclo[3.1.1]heptane |
| **2D** | 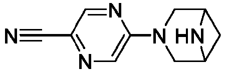 | 5-{3,6-diazabicyclo[3.1.1]heptan-3-yl}pyrazine-2-carbonitrile |
| **2E** | 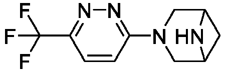 | 3-[6-(trifluoromethyl)pyridazin-3-yl]-3,6-diazabicyclo[3.1.1]heptane |
| **2F** | 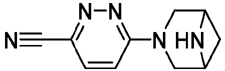 | 6-{3,6-diazabicyclo[3.1.1]heptan-3-yl}pyridazine-3-carbonitrile |
| **2G** | 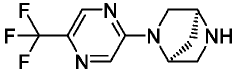 | (1*S*,4*S*)-2-[5-(trifluoromethyl)pyrazin-2-yl]-2,5-diazabicyclo[2.2.1]heptane |
| **2H** | 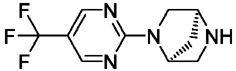 | (1*S*,4*S*)-2-[5-(trifluoromethyl)pyrimidin-2-yl]-2,5-diazabicyclo[2.2.1]heptane |
| **2I** | 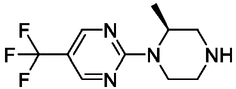 | 2-[(2*S*)-2-methylpiperazin-1-yl]-5-(trifluoromethyl)pyrimidine |
| **2J** | 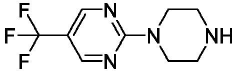 | 2-(piperazin-1-yl)-5-(trifluoromethyl)pyrimidine |
| **2K** | 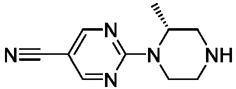 | 2-[(2*R*)-2-methylpiperazin-1-yl]pyrimidine-5-carbonitrile |
| **2L** | 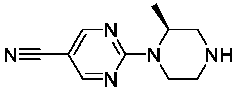 | 2-[(2*S*)-2-methylpiperazin-1-yl]pyrimidine-5-carbonitrile |
| **2M** | 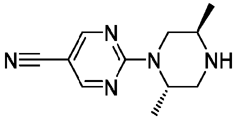 | 2-[(2S,5*R*)-2,5-dimethylpiperazin-1-yl]pyrimidine-5-carbonitrile |
| **2N** | 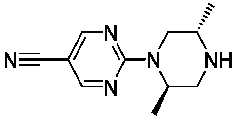 | 2-[(2*R*,5*S*)-2,5-dimethylpiperazin-1-yl]pyrimidine-5-carbonitrile |
| **2O** | 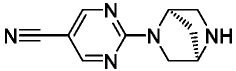 | 2-[(1*R*,4*R*)-2,5-diazabicyclo[2.2.1]heptan-2-yl]pyrimidine-5-carbonitrile |
| **2P** | 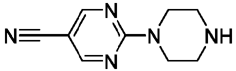 | 2-(piperazin-1-yl)pyrimidine-5-carbonitrile |
| **2Q** | 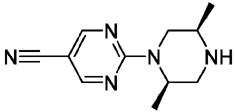 | 2-[(2*R*,5*R*)-2,5-dimethylpiperazin-1-yl]pyrimidine-5-carbonitrile |
| **2R** | 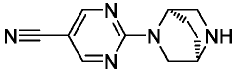 | 2-[(1*R*,4*R*)-2,5-diazabicyclo[2.2.2]octan-2-yl]pyrimidine-5-carbonitrile |
| **2S** | 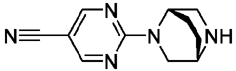 | 2-[(1*S*,4*S*)-2,5-diazabicyclo[2.2.2]octan-2-yl]pyrimidine-5-carbonitrile |
| **2T** | 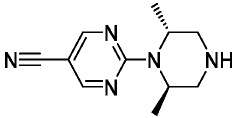 | 2-[(2*R*,6*R*)-2,6-dimethylpiperazin-1-yl]pyrimidine-5-carbonitrile |
| **2U** | 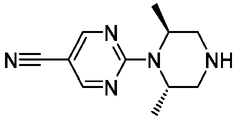 | 2-[(2*S*,6*S*)-2,6-dimethylpiperazin-1-yl]pyrimidine-5-carbonitrile |
| **2V** | 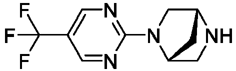 | (1*R*,4*R*)-2-[5-(trifluoromethyl)pyrimidin-2-yl]-2,5-diazabicyclo[2.2.1]heptane |
| **2W** | 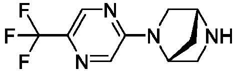 | (1*R*,4*R*)-2-[5-(trifluoromethyl)pyrazin-2-yl]-2,5-diazabicyclo[2.2.1]heptane |
| **2X** | 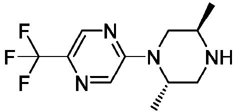 | 2-[(2*S*,5*R*)-2,5-dimethylpiperazin-1-yl]-5-(trifluoromethyl)pyrazine |
| **2Y** | 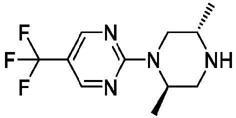 | 2-[(2*R*,5*S*)-2,5-dimethylpiperazin-1-yl]-5-(trifluoromethyl)pyrimidine |
| **2Z** | 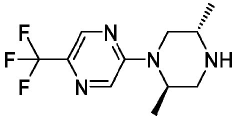 | 2-[(2*R*,5*S*)-2,5-dimethylpiperazin-1-yl]-5-(trifluoromethyl)pyrazine |
| **2AA** | 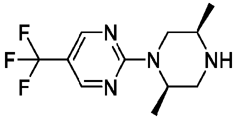 | 2-[(2*R*,5*R*)-2,5-dimethylpiperazin-1-yl]-5-(trifluoromethyl)pyrimidine |
| **2AB** | 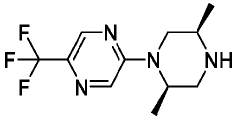 | 2-[(2*R*,5*R*)-2,5-dimethylpiperazin-1-yl]-5-(trifluoromethyl)pyrazine |
| **2AC** | 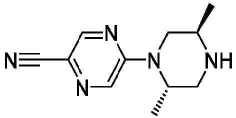 | 5-[(2*S*,5*R*)-2,5-dimethylpiperazin-1-yl]pyrazine-2-carbonitrile |
| **2AD** | 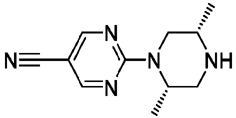 | 2-[(2*S*,5*S*)-2,5-dimethylpiperazin-1-yl]pyrimidine-5-carbonitrile |
| **2AF** | 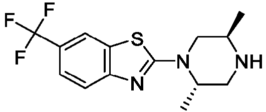 | 2-[(2S,5R)-2,5-dimethylpiperazin-1-yl]-6-(trifluoromethyl)-1,3-benzothiazole |
| **2AG** | 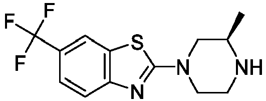 | 2-[(3R)-3-methylpiperazin-1-yl]-6-(trifluoromethyl)-1,3-benzothiazole |
| **2AJ** | 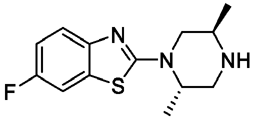 | 2-[(2S,5R)-2,5-dimethylpiperazin-1-yl]-6-fluoro-1,3-benzothiazole |
| **2AK** | 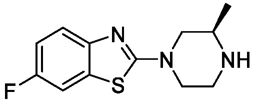 | 6-fluoro-2-[(3R)-3-methylpiperazin-1-yl]-1,3-benzothiazole |
| **2AL** | 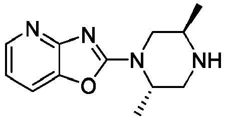 | (2S,5R)-2,5-dimethyl-1-{[1,3]oxazolo[4,5-b]pyridin-2-yl}piperazine |
| **2AM** | 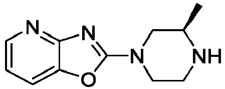 | (3R)-3-methyl-1-{[1,3]oxazolo[4,5-b]pyridin-2-yl}piperazine |
| **2AN** | 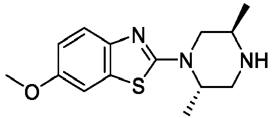 | 2-[(2S,5R)-2,5-dimethylpiperazin-1-yl]-6-methoxy-1,3-benzothiazole |
| **2AO** | 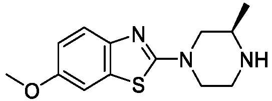 | 6-methoxy-2-[(3R)-3-methylpiperazin-1-yl]-1,3-benzothiazole |
| **2AP** | 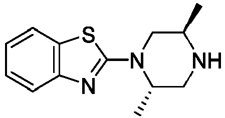 | 2-[(2S,5R)-2,5-dimethylpiperazin-1-yl]-1,3-benzothiazole |
| **2AQ** | 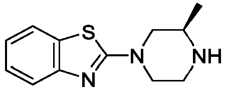 | 2-[(3R)-3-methylpiperazin-1-yl]-1,3-benzothiazole |
| **2AR** | 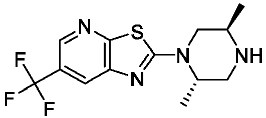 | (2S,5R)-2,5-dimethyl-1-[6-(trifluoromethyl)-[1,3]thiazolo[5,4-b]pyridin-2-yl]piperazine |
| **2AS** | 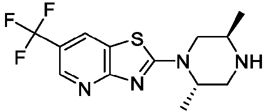 | (2S,5R)-2,5-dimethyl-1-[6-(trifluoromethyl)-[1,3]thiazolo[4,5-b]pyridin-2-yl]piperazine |
| **2AT** | 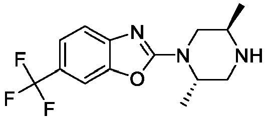 | 2-[(2S,5R)-2,5-dimethylpiperazin-1-yl]-6-(trifluoromethyl)-1,3-benzoxazole |
| **2AU** | 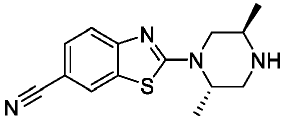 | 2-[(2S,5R)-2,5-dimethylpiperazin-1-yl]-1,3-benzothiazole-6-carbonitrile |
| **2AV** | 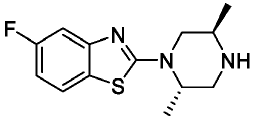 | 2-[(2S,5R)-2,5-dimethylpiperazin-1-yl]-5-fluoro-1,3-benzothiazole |
| **2AW** | 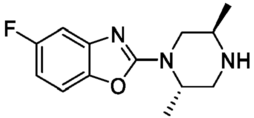 | 2-[(2S,5R)-2,5-dimethylpiperazin-1-yl]-5-fluoro-1,3-benzoxazole |
| **2AX** | 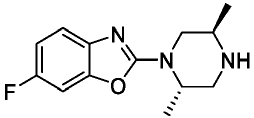 | 2-[(2S,5R)-2,5-dimethylpiperazin-1-yl]-6-fluoro-1,3-benzoxazole |
| **2AY** | 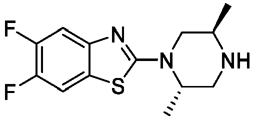 | 2-[(2S,5R)-2,5-dimethylpiperazin-1-yl]-5,6-difluoro-1,3-benzothiazole |
| **2BJ** | 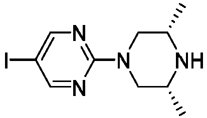 | 2-((3R,5S)-3,5-dimethylpiperazin-1-yl)-5-iodopyrimidine |

### Preparation of Intermediate 2-((3R,5S)-3,5-dimethylpiperazin-1-yl)-N,N-dimethylpyrimidine-5-carboxamide hydrochloride (2BK).

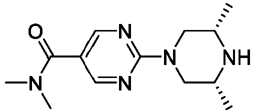

A solution containing (2*R*,6*S*)-2,6-dimethylpiperazine (0.40 g, 1.9 mmol, 1.0 eq), 2-chloropyrimidine-5-carboxylic acid (0.30 g, 1.9 mmol, 1.0 eq), and TEA (0.53 mL, 3.8 mmol, 2.0 eq) in 1,4-dioxanes (12) was heated in a microwave reactor for 30 min at 150 °C. The resulting mixture was acidified with 6 N HCl to pH 2 and product back extracted with ethyl acetate. The combined organics were dried over anhydrous magnesium sulfate, filtered to remove solid, and concentrated *in vacuo* to provide the substituted piperazine as a beige solid (0.31 g, 0.92 mmol, 49% crude):

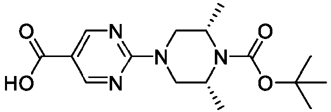

To a solution of the substituted piperazine intermediate (0.20 g, 0.60 mmol, 1.0 eq), 1-[bis(dimethylamino)methylene]-1*H*-1,2,3-triazolo[4,5-*b*]pyridinium 3-oxid hexafluorophosphate (0.23 g, 0.60 mmol, 1.0 eq), and dimethylamine hydrochloride (0.049 g, 0.60 mmol, 1.0 eq), in dry DCM (10.0 mL) was added DIPEA (0.42 mL, 1.2 mmol, 2.0 eq). The resulting solution was stirred at room temperature overnight. The reaction was concentrated *in vacuo.* Silica gel column (12 g) was loaded using DCM and run with an increasing gradient of ethyl acetate (0-100%) in hexanes over 8 min then 100% ethyl acetate for 5 min to provide the Boc-protected intermediate:

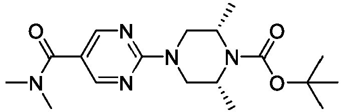

The isolated Boc-protected intermediate was dissolved in 1,4-dioxanes (2 mL), treated with 4 N HCl in 1,4-dioxanes (2 mL), and stirred at room temperature overnight. The reaction was diluted with diethyl ether and the resulting solid was collected by vacuum filtration to provide 2-((3*R*,5*S*)-3,5-dimethylpiperazin-1-yl)-*N*,*N*-dimethylpyrimidine-5-carboxamide hydrochloride (2BK, 0.14 g, 0.47 mmol, 78% over 2 steps) as a white powder.

### Example 3: Preparation of 2-[(3R,5S)-3,5-Dimethylpiperazin-1-yl]-5-(trifluoromethyl)pyrimidine (Intermediate 3A).

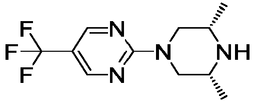

To a solution of (2R,6S)-2,6-dimethylpiperazine (1.1 g, 10 mmol, 1.0 eq) and 2-chloro-5-(trifluoromethyl)pyrimidine (1.8 g, 10 mmol, 1.0 eq) in ACN (40 mL) was added triethylamine (5.6 mL, 40 mmol, 4.0 eq). The resulting mixture was stirred at room temperature overnight. The formed suspension was filtered to remove triethylamine hydrochloride and concentrated *in vacuo* to provide 2-[(3*R*,5*S*)-3,5-dimethylpiperazin-1-yl]-5-(trifluoromethyl)pyrimidine (**Intermediate 3A,** 2.6 g, 10 mmol) as a light orange solid in quantitative yield.

Other intermediates useful in the preparation compounds of the present invention were made using substantially the same procedures as described above (*i.e.,* room temperature overnight), however heat may be necessary. Intermediates prepared by this procedure include:

| **Intermediate** | **Chemical Structure** | **Chemical Name** |
|---|---|---|
| **3B** | 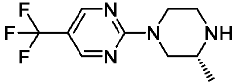 | 2-[(3*R*)-3-methylpiperazin-1-yl]-5-(trifluoromethyl)pyrimidine |
| **3C** | 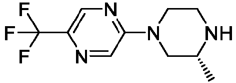 | 2-[(3*R*)-3-methylpiperazin-1-yl]-5-(trifluoromethyl)pyrazine |
| **3D** | 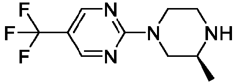 | 2-[(3*S*)-3-methylpiperazin-1-yl]-5-(trifluoromethyl)pyrimidine |
| **3E** | 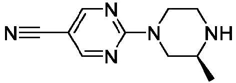 | 2-[(3*S*)-3-methylpiperazin-1-yl]pyrimidine-5-carbonitrile |
| **3F** | 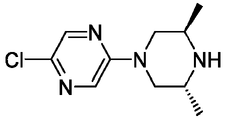 | 2-chloro-5-[(3*R*,5*R*)-3,5-dimethylpiperazin-1-yl]pyrazine |
| **3G** | 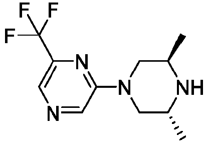 | 2-[(3*R*,5*R*)-3,5-dimethylpiperazin-1-yl]-6-(trifluoromethyl)pyrazine |
| **3H** | 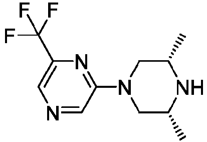 | 2-[(3*R*,5*S*)-3,5-dimethylpiperazin-1-yl]-6-(trifluoromethyl)pyrazine |
| **3I** | 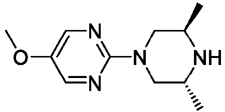 | 2-[(3*R*,5*R*)-3,5-dimethylpiperazin-1-yl]-5-methoxypyrimidine |
| **3J** | 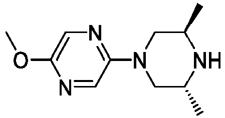 | 2-[(3*R*,5*R*)-3,5-dimethylpiperazin-1-yl]-5-methoxypyrazine |
| **3K** | 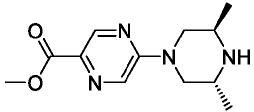 | methyl 5-[(3*R*,5*R*)-3,5-dimethylpiperazin-1-yl]pyrazine-2-carboxylate |
| **3L** | 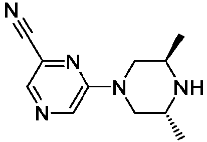 | 6-[(3*R*,5*R*)-3,5-dimethylpiperazin-1-yl]pyrazine-2-carbonitrile |
| **3M** | 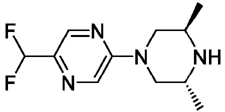 | 2-(difluoromethyl)-5-[(3*R*,5*R*)-3,5-dimethylpiperazin-1-yl]pyrazine |
| **3N** | 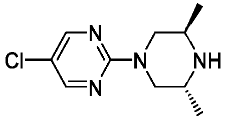 | 5-chloro-2-((3*R*,5*R*)-3,5-dimethylpiperazin-1-yl)pyrimidine |
| **3O** | 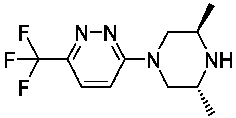 | 3-((3*R*,5*R*)-3,5-dimethylpiperazin-1-yl)-6-(trifluoromethyl)pyridazine |
| **3P** | 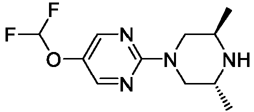 | 5-(difluoromethoxy)-2-((3*R*,5*R*)-3,5-dimethylpiperazin-1-yl)pyrimidine |
| **3Q** | 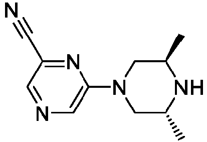 | 6-((3*R*,5*R*)-3,5-dimethylpiperazin-1-yl)pyrazine-2-carbonitrile |
| **3R** | 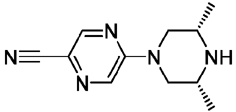 | 5-((3*R*,5*S*)-3,5-dimethylpiperazin-1-yl)pyrazine-2-carbonitrile |
| **3S** | 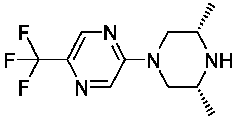 | 2-((3*R*,5*S*)-3,5-dimethylpiperazin-1-yl)-5-(trifluoromethyl)pyrazine |
| **3T** | 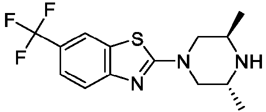 | 2-[(3*R*,5*R*)-3,5-dimethylpiperazin-1-yl]-6-(trifluoromethyl)-1,3-benzothiazole |
| **3U** | 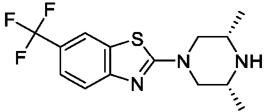 | 2-[(3*R*,5*S*)-3,5-dimethylpiperazin-1-yl]-6-(trifluoromethyl)-1,3-benzothiazole |
| **3V** | 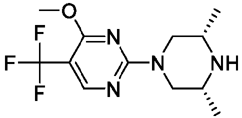 | 2-((3*S*,5*R*)-3,5-dimethylpiperazin-1-yl)-4-methoxy-5-(trifluoromethyl)pyrimidine |
| **3W** | 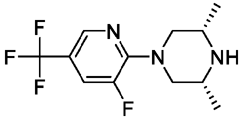 | (3*R*,5*S*)-1-(3-fluoro-5-(trifluoromethyl)pyridin-2-yl)-3,5-dimethylpiperazine |
| **3X** | 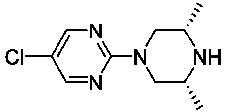 | 5-chloro-2-((3R,5S)-3,5-dimethylpiperazin-1-yl)pyrimidine |
| **3Y** | 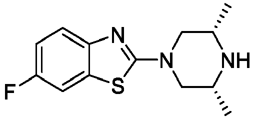 | 2-[(3R,5S)-3,5-dimethylpiperazin-1-yl]-6-fluoro-1,3-benzothiazole |
| **3Z** | 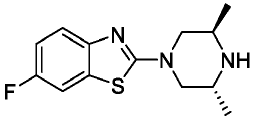 | 2-[(3R,5R)-3,5-dimethylpiperazin-1-yl]-6-fluoro-1,3-benzothiazole |
| **3AA** | 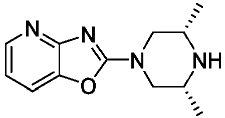 | (3R,5S)-3,5-dimethyl-1-{[1,3]oxazolo[4,5-b]pyridin-2-yl}piperazine |
| **3AB** | 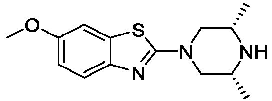 | 2-[(3R,5S)-3,5-dimethylpiperazin-1-yl]-6-methoxy-1,3-benzothiazole |
| **3AC** | 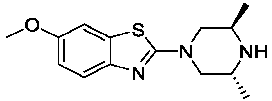 | 2-[(3R,5R)-3,5-dimethylpiperazin-1-yl]-6-methoxy-1,3-benzothiazole |
| **3AD** | 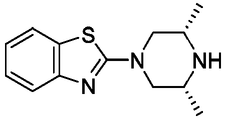 | 2-[(3R,5S)-3,5-dimethylpiperazin-1-yl]-1,3-benzothiazole |
| **3AE** | 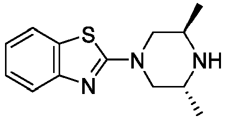 | 2-[(3R,5R)-3,5-dimethylpiperazin-1-yl]-1,3-benzothiazole |
| **3AF** | 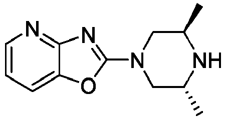 | (3R,5R)-3,5-dimethyl-1-{[1,3]oxazolo[4,5-b]pyridin-2-yl}piperazine |
| **3AG** | 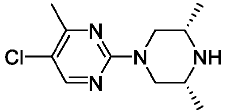 | 5-chloro-2-((3R,5S)-3,5-dimethylpiperazin-1-yl)-4-methylpyrimidine |
| **3AH** | 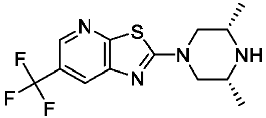 | (3R,5S)-3,5-dimethyl-1-[6-(trifluoromethyl)-[1,3]thiazolo[5,4-b]pyridin-2-yl]piperazine |
| **3AI** | 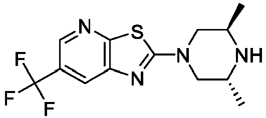 | (3R,5R)-3,5-dimethyl-1-[6-(trifluoromethyl)-[1,3]thiazolo[5,4-b]pyridin-2-yl]piperazine |
| **3AJ** | 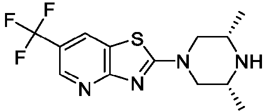 | (3R,5S)-3,5-dimethyl-1-[6-(trifluoromethyl)-[1,3]thiazolo[4,5-b]pyridin-2-yl]piperazine |
| **3AK** | 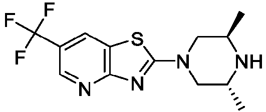 | (3R,5R)-3,5-dimethyl-1-[6-(trifluoromethyl)-[1,3]thiazolo[4,5-b]pyridin-2-yl]piperazine |
| **3AL** | 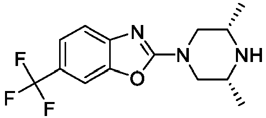 | 2-[(3R,5S)-3,5-dimethylpiperazin-1-yl]-6-(trifluoromethyl)-1,3-benzoxazole |
| **3AM** | 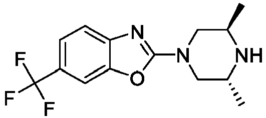 | 2-[(3R,5R)-3,5-dimethylpiperazin-1-yl]-6-(trifluoromethyl)-1,3-benzoxazole |
| **3AN** | 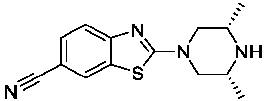 | 2-[(3R,5S)-3,5-dimethylpiperazin-1-yl]-1,3-benzothiazole-6-carbonitrile |
| **3AO** | 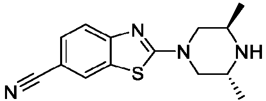 | 2-[(3R,5R)-3,5-dimethylpiperazin-1-yl]-1,3-benzothiazole-6-carbonitrile |
| **3AP** | 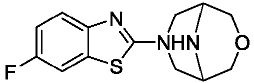 | 7-(6-fluoro-1,3-benzothiazol-2-yl)-3-oxa-7,9-diazabicyclo[3.3.1]nonane |
| **3AQ** | 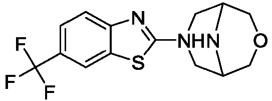 | 7-[6-(trifluoromethyl)-1,3-benzothiazol-2-yl]-3-oxa-7,9-diazabicyclo[3.3.1]nonane |
| **3AR** | 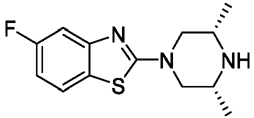 | 2-[(3R,5S)-3,5-dimethylpiperazin-1-yl]-5-fluoro-1,3-benzothiazole |
| **3AS** | 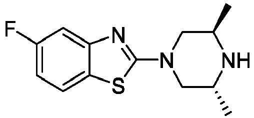 | 2-[(3R,5R)-3,5-dimethylpiperazin-1-yl]-5-fluoro-1,3-benzothiazole |
| **3AT** | 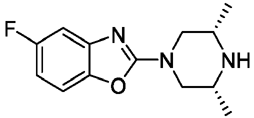 | 2-[(3R,5S)-3,5-dimethylpiperazin-1-yl]-5-fluoro-1,3-benzoxazole |
| **3AU** | 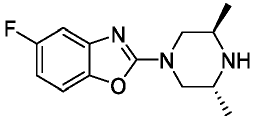 | 2-[(3R,5R)-3,5-dimethylpiperazin-1-yl]-5-fluoro-1,3-benzoxazole |
| **3AV** | 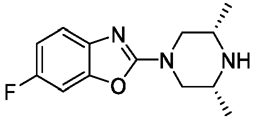 | 2-[(3R,5S)-3,5-dimethylpiperazin-1-yl]-6-fluoro-1,3-benzoxazole |
| **3AW** | 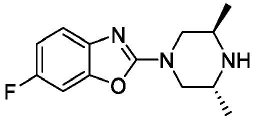 | 2-[(3R,5R)-3,5-dimethylpiperazin-1-yl]-6-fluoro-1,3-benzoxazole |
| **3AX** | 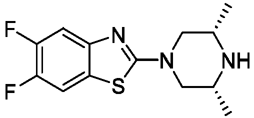 | 2-[(3R,5S)-3,5-dimethylpiperazin-1-yl]-5,6-difluoro-1,3-benzothiazole |
| **3AY** | 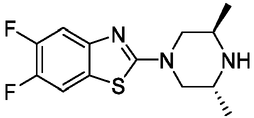 | 2-[(3R,5R)-3,5-dimethylpiperazin-1-yl]-5,6-difluoro-1,3-benzothiazole |
| **3AZ** | 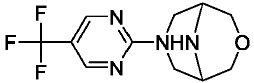 | 7-[5-(trifluoromethyl)pyrimidin-2-yl]-3-oxa-7,9-diazabicyclo[3.3.1]nonane |
| **3BA** | 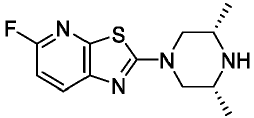 | (3R,5S)-1-{5-fluoro-[1,3]thiazolo[5,4-b]pyridin-2-yl}-3,5-dimethylpiperazine |
| **3BB** | 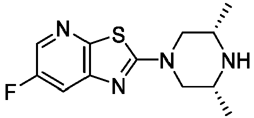 | (3R,5S)-1-{6-fluoro-[1,3]thiazolo[5,4-b]pyridin-2-yl}-3,5-dimethylpiperazine |
| **3BC** | 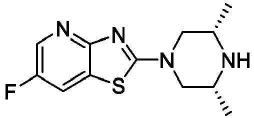 | (3R,5S)-1-{6-fluoro-[1,3]thiazolo[4,5-b]pyridin-2-yl}-3,5-dimethylpiperazine |
| **3BD** | 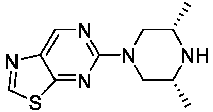 | (3R,5S)-3,5-dimethyl-1-{[1,3]thiazolo[5,4-d]pyrimidin-5-yl}piperazine |
| **3BE** | 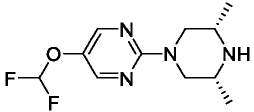 | 5-(difluoromethoxy)-2-[(3R,5S)-3,5-dimethylpiperazin-1-yl]pyrimidine |
| **3BF** | 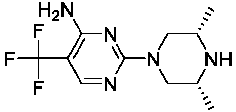 | 2-[(3R,5S)-3,5-dimethylpiperazin-1-yl]-5-(trifluoromethyl)pyrimidin-4-amine |
| **3BG** | 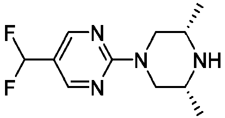 | 5-(difluoromethyl)-2-[(3R,5S)-3,5-dimethylpiperazin-1-yl]pyrimidine |
| **3BH** | 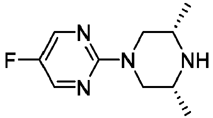 | 2-[(3R,5S)-3,5-dimethylpiperazin-1-yl]-5-fluoropyrimidine |
| **3BI** | 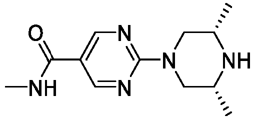 | 2-[(3R,5S)-3,5-dimethylpiperazin-1-yl]-N-methylpyrimidine-5-carboxamide |
| **3BJ** | 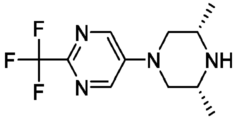 | 5-[(3R,5S)-3,5-dimethylpiperazin-1-yl]-2-(trifluoromethyl)pyrimidine |
| **3BK** | 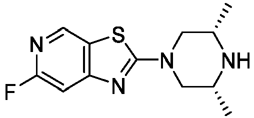 | (3R,5S)-1-{6-fluoro-[1,3]thiazolo[5,4-c]pyridin-2-yl}-3,5-dimethylpiperazine |
| **3BL** | 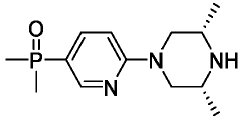 | (3R,5S)-1-[5-(dimethylphosphoryl)pyridin-2-yl]-3,5-dimethylpiperazine |
| **3BM** | 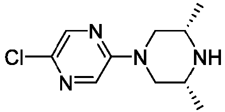 | 2-chloro-5-((3R,5S)-3,5-dimethylpiperazin-1-yl)pyrazine |
| **3BN** | 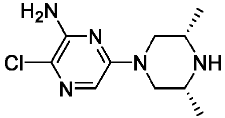 | 3-chloro-6-((3R,5S)-3,5-dimethylpiperazin-1-yl)pyrazin-2-amine |
| **3BR** | 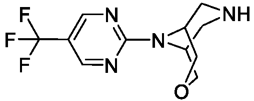 | 9-[5-(trifluoromethyl)pyrimidin-2-yl]-3-oxa-7,9-diazabicyclo[3.3.1]nonane |
| **3BS** | 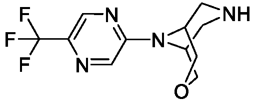 | 9-[5-(trifluoromethyl)pyrazin-2-yl]-3-oxa-7,9-diazabicyclo[3.3.1]nonane |
| **3BT** | 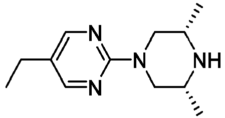 | 2-[(3R,5S)-3,5-dimethylpiperazin-1-yl]-5-ethylpyrimidine |
| **3BU** | 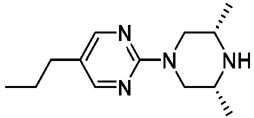 | 2-[(3R,5S)-3,5-dimethylpiperazin-1-yl]-5-propylpyrimidine |
| **3BV** | 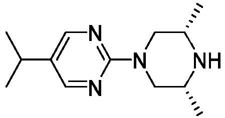 | 2-[(3R,5S)-3,5-dimethylpiperazin-1-yl]-5-(propan-2-yl)pyrimidine |
| **3BW** | 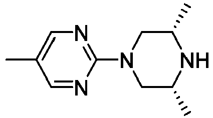 | 2-[(3R,5S)-3,5-dimethylpiperazin-1-yl]-5-methylpyrimidine |
| **3BX** | 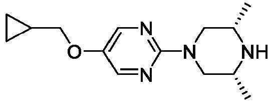 | 5-(cyclopropylmethoxy)-2-[(3R,5S)-3,5-dimethylpiperazin-1-yl]pyrimidine |
| **3BY** | 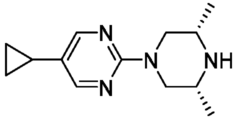 | 5-cyclopropyl-2-[(3R,5S)-3,5-dimethylpiperazin-1-yl]pyrimidine |
| **3BZ** | 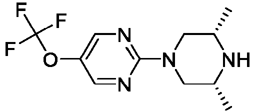 | 2-[(3R,5S)-3,5-dimethylpiperazin-1-yl]-5-(trifluoromethoxy)pyrimidine |
| **3CC** | 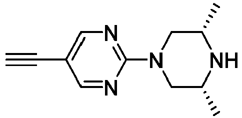 | 2-[(3R,5S)-3,5-dimethylpiperazin-1-yl]-5-ethynylpyrimidine |
| **3CE** | 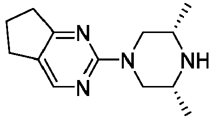 | (3R,5S)-1-{5H,6H,7H-cyclopenta[d]pyrimidin-2-yl}-3,5-dimethylpiperazine |

### Example 4: Preparation of 1-[3-(Trifluoromethyl)pyridin-4-yl]piperazine (Intermediate 4A).

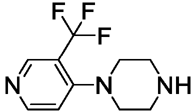

To a solution of *tert*-butyl piperazine-1-carboxylate (0.41 g, 2.2 mmol, 1.0 eq) and 4-bromo-3-(trifluoromethyl)pyridine hydrobromide (0.68 g, 2.2 mmol, 1.0 eq) in dioxane (12 mL) was added sodium *tert*-butoxide (0.63 g, 6.6 mmol, 3.0 eq), racemic 2,2'-bis(diphenylphosphino)-1,1'-binaphthyl (41 mg, 0.07 mmol, 0.03 eq), and lastly tris(dibenzylideneacetone)dipalladium(0) (201 mg, 0.22 mmol, 0.10 eq). The resulting mixture was heated to 95 °C overnight. Subsequently, the mixture was cooled, diluted with EtOAc, passed thru a pad of celite^{®} and concentrated *in vacuo.* The crude material was purified by silica gel column (40 g) using DCM and run with an increasing gradient of EtOAc (0-50%) in hexanes over 20 min. The isolated material was dissolved in DCM (2 mL) and treated with a solution of 4M HCl in dioxane (1 mL) and stirred at room temperature overnight. The formed suspension was filtered and washed with DCM to provide the hydrochloride salt of 1-[3-(trifluoromethyl)pyridin-4-yl]piperazine (**Intermediate 4A,** 0.32 g, 1.2 mmol, 55% over two steps) isolated as a light yellow solid.

Other intermediates useful in the preparations of compounds of the present invention were made using substantially the same procedures described above, including:

| **Intermediate** | **Chemical Structure** | **Chemical Name** |
|---|---|---|
| **4B** | 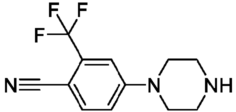 | 4-(piperazin-1-yl)-2-(trifluoromethyl)benzonitrile |
| **4C** | 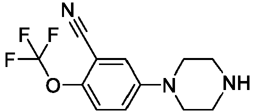 | 5-(piperazin-1-yl)-2-(trifluoromethoxy)benzonitrile |
| **4D** | 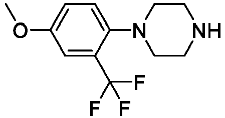 | 1-[4-methoxy-2-(trifluoromethyl)phenyl]piperazine |
| **4E** | 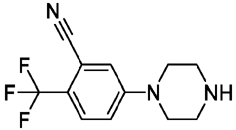 | 5-(piperazin-1-yl)-2-(trifluoromethyl)benzonitrile |
| **4F** | 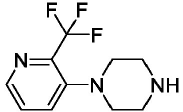 | 1-[2-(trifluoromethyl)pyridin-3-yl]piperazine |
| **4G** | 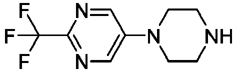 | 5-(piperazin-1-yl)-2-(trifluoromethyl)pyrimidine |

Other intermediates useful in the preparations of compounds of the present invention were made using substantially the same procedures as described above except by replacing catalyst 2,2'-bis(diphenylphosphino)-1,1'-binaphthyl and tris(dibenzylideneacetone)dipalladium(0) with RuPhos-G3-Palladacycle, including:

| **Intermediate** | **Chemical Structure** | **Chemical Name** |
|---|---|---|
| **4H** | 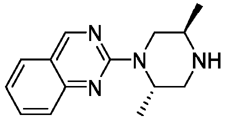 | 2-[(2S,5R)-2,5-dimethylpiperazin-1-yl]quinazoline |
| **4I** | 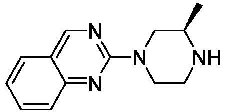 | 2-[(3R)-3-methylpiperazin-1-yl]quinazoline |
| **4J** | 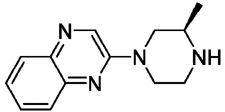 | 2-[(3R)-3-methylpiperazin-1-yl]quinoxaline |
| **4K** | 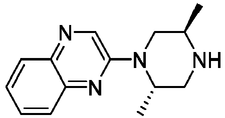 | 2-[(2S,5R)-2,5-dimethylpiperazin-1-yl]quinoxaline |
| **4L** | 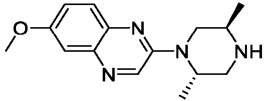 | 2-[(2S,5R)-2,5-dimethylpiperazin-1-yl]-6-methoxyquinoxaline |
| **4M** | 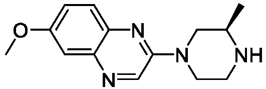 | 6-methoxy-2-[(3R)-3-methylpiperazin-1-yl]quinoxaline |
| **4N** | 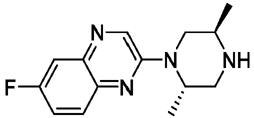 | 2-[(2S,5R)-2,5-dimethylpiperazin-1-yl]-6-fluoroquinoxaline |
| **4O** | 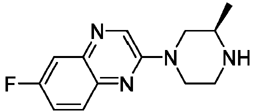 | 6-fluoro-2-[(3R)-3-methylpiperazin-1-yl]quinoxaline |
| **4P** | 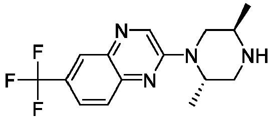 | 2-[(2S,5R)-2,5-dimethylpiperazin-1-yl]-6-(trifluoromethyl)quinoxaline |
| **4Q** | 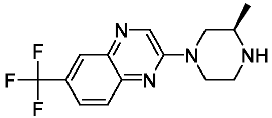 | 2-[(3R)-3-methylpiperazin-1-yl]-6-(trifluoromethyl)quinoxaline |
| **4T** | 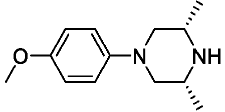 | (3R,5S)-1-(4-methoxyphenyl)-3,5-dimethylpiperazine |
| **4U** | 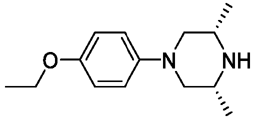 | (3R,5S)-1-(4-ethoxyphenyl)-3,5-dimethylpiperazine |
| **4V** | 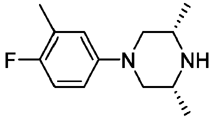 | (3R,5S)-1-(4-fluoro-3-methylphenyl)-3,5-dimethylpiperazine |
| **4W** | 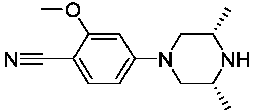 | 4-[(3R,5S)-3,5-dimethylpiperazin-1-yl]-2-methoxybenzonitrile |
| **4X** | 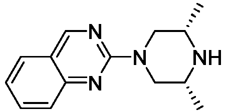 | 2-[(3R,5S)-3,5-dimethylpiperazin-1-yl]quinazoline |

### Example 5: Preparation of (3R,5R)-3,5-Dimethyl-1-[4-(trifluoromethyl)phenyl]piperazine (Intermediate 5A).

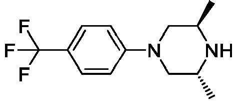

To a solution of (2*R*,6*R*)-2,6-dimethylpiperazine dihydrochloride (1.0 g, 5.3 mmol, 1.0 eq) and 1-bromo-4-(trifluoromethyl)benzene (1.2 g, 5.3 mmol, 1.0 eq) in dioxane (12 mL) was added sodium *tert*-butoxide (1.5 g, 16 mmol, 3.0 eq), racemic 2,2'-*bis*(diphenylphosphino)-1,1'-binaphthyl (0.10 g, 0.16 mmol, 0.03 eq), and lastly tris(dibenzylideneacetone)dipalladium(0) (1.5 g, 1.6 mmol, 0.10 eq). The resulting mixture was heated to 95 °C overnight. Subsequently, the mixture was cooled, diluted with EtOAc, passed thru a pad of celite^{®} and concentrated *in vacuo.* The crude material was purified by silica gel column (80 g) using DCM and run with an increasing gradient of methanol (0-20%) in DCM over 20 min to provide (3*R*,5*R*)-3,5-dimethyl-1-[4-(trifluoromethyl)phenyl]piperazine **(Intermediate 5A,** 0.55 g, 2.1 mmol, 40%) isolated as a brown oil.

Other intermediates useful in the preparations of compounds of the present invention were made using substantially the same procedures described above, including:

| **Intermediate** | **Chemical Structure** | **Chemical Name** |
|---|---|---|
| **5B** | 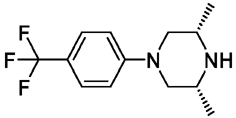 | (3*R*,5*S*)-3,5-dimethyl-1-[4-(trifluoromethyl)phenyl]piperazine |
| **5C** | 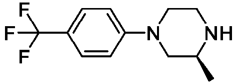 | (3*S*)-3-methyl-1-[4-(trifluoromethyl)phenyl]piperazine |
| **5D** | 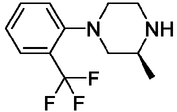 | (3*S*)-3-methyl-1-[2-(trifluoromethyl)phenyl]piperazine |
| **5E** | 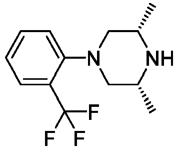 | (3*R*,5*S*)-3,5-dimethyl-1-[2-(trifluoromethyl)phenyl]piperazine |
| **5F** | 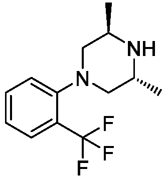 | (3*R*,5*R*)-3,5-dimethyl-1-[2-(trifluoromethyl)phenyl]piperazine |
| **5G** | 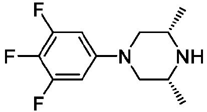 | (3*R*,5*S*)-3,5-dimethyl-1-(3,4,5-trifluorophenyl)piperazine |
| **5H** | 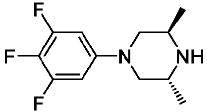 | (3*R*,5*R*)-3,5-dimethyl-1-(3,4,5-trifluorophenyl)piperazine |

Other intermediates useful in the preparations of compounds of the present invention were made using substantially the same procedures as described above except by replacing catalyst 2,2'-bis(diphenylphosphino)-1,1'-binaphthyl and tris(dibenzylideneacetone)dipalladium(0) with RuPhos-G3-Palladacycle, including:

| **Intermediate** | **Chemical Structure** | **Chemical Name** |
|---|---|---|
| **5I** | 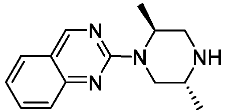 | 2-[(2*S*,5*R*)-2,5-dimethylpiperazin-1-yl]quinazoline |
| **5J** | 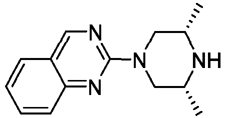 | 2-[(3*R*,5*S*)-3,5-dimethylpiperazin-1-yl]quinazoline |
| **5K** | 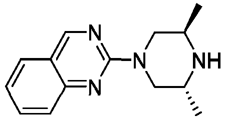 | 2-[(3*R*,5*R*)-3,5-dimethylpiperazin-1-yl]quinazoline |
| **5L** | 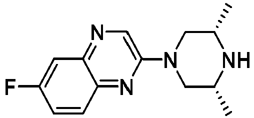 | 2-[(3*R*,5*S*)-3,5-dimethylpiperazin-1-yl]-6-fluoroquinoxaline |
| **5M** | 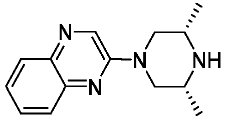 | 2-[(3R,5S)-3,5-dimethylpiperazin-1-yl]quinoxaline |
| **5N** | 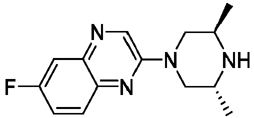 | 2-[(3R,5R)-3,5-dimethylpiperazin-1-yl]-6-fluoroquinoxaline |
| **5O** | 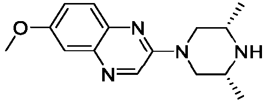 | 2-[(3R,5S)-3,5-dimethylpiperazin-1-yl]-6-methoxyquinoxaline |
| **5P** | 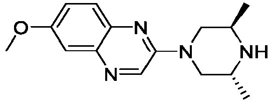 | 2-[(3R,5R)-3,5-dimethylpiperazin-1-yl]-6-methoxyquinoxaline |
| **5Q** | 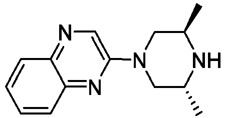 | 2-[(3R,5R)-3,5-dimethylpiperazin-1-yl]quinoxaline |
| **5R** | 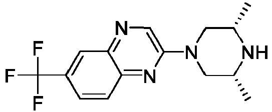 | 2-[(3R,5S)-3,5-dimethylpiperazin-1-yl]-6-(trifluoromethyl)quinoxaline |
| **5S** | 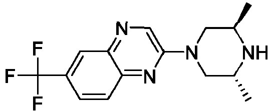 | 2-[(3R,5R)-3,5-dimethylpiperazin-1-yl]-6-(trifluoromethyl)quinoxaline |
| **5T** | 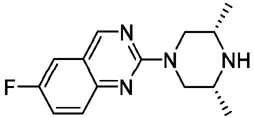 | 2-[(3R,5S)-3,5-dimethylpiperazin-1-yl]-6-fluoroquinazoline |
| **5U** | 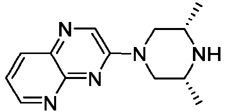 | (3R,5S)-3,5-dimethyl-1-{pyrido[2,3-b]pyrazin-3-yl}piperazine |
| **5V** | 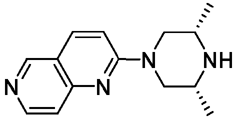 | 2-[(3R,5S)-3,5-dimethylpiperazin-1-yl]-1,6-naphthyridine |
| **5W** | 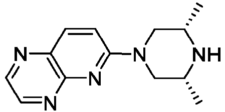 | (3R,5S)-3,5-dimethyl-1-{pyrido[2,3-b]pyrazin-6-yl}piperazine |
| **5X** | 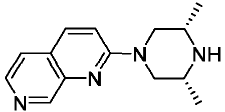 | 2-[(3R,5S)-3,5-dimethylpiperazin-1-yl]-1,7-naphthyridine |
| **5Y** | 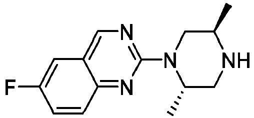 | 2-[(2S,5R)-2,5-dimethylpiperazin-1-yl]-6-fluoroquinazoline |
| **5Z** | 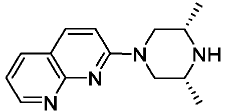 | 2-[(3R,5S)-3,5-dimethylpiperazin-1-yl]-1,8-naphthyridine |
| **5AA** | 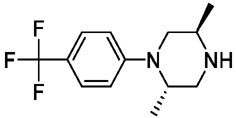 | (2S,5R)-2,5-dimethyl-1-[4-(trifluoromethyl)phenyl]piperazine |
| **5AB** | 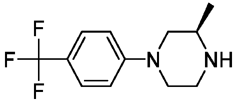 | (3R)-3-methyl-1-[4-(trifluoromethyl)phenyl]piperazine |

### Example 6: Preparation of (2R,5S)-N-{2-Benzyl-2-azaspiro[3.3]heptan-6-yl}-2,5-dimethyl-4-[5-(trifluoromethyl)pyrimidin-2-yl]piperazine-1-carboxamide (Compound 1).

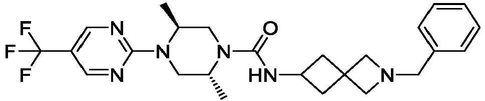

To a solution of 2-benzyl-2-azaspiro[3.3]heptan-6-amine (**Intermediate 1A**, 0.26 g, 1.3 mmol) in dry ACN (6.5 mL) was added carbonyldiimidazole (0.21 g, 1.3 mmol). The resulting mixture was stirred at room temperature for 1 hr. To an aliquot of the mixture (0.1 mL, 0.015 mmol, 1 eq) was added a solution of 2-[(2S,5R)-2,5-dimethylpiperazin-1-yl]-5-(trifluoromethyl)pyrimidine (**Intermediate 2A**, 5.8 mg, 0.022 mmol, 1.5 eq) and DIPEA (15 µL) in dry ACN (0.3 mL) and stirred at room temperature overnight (additional aliquots were used to prepare the remaining compounds listed in **TABLE 1** using the appropriate secondary amine in place of 2-[(2*S*,5*R*)-2,5-dimethylpiperazin-1-yl]-5-(trifluoromethyl)pyrimidine). Subsequently, the mixture was diluted to a total volume of 1 mL using MeOH and submitted directly for preparative chromatography yielding (2*R*,5*S*)-N-{2-benzyl-2-azaspiro[3.3]heptan-6-yl}-2,5-dimethyl-4-[5-(trifluoromethyl)pyrimidin-2-yl]piperazine-1-carboxamide (**Compound 1**).

The table below provides the observed (Obs) ion m/z ratio for the title compound (**Compound 1,** first compound listed in **TABLE 1**) and other compounds of the present invention that were prepared in substantially the same manner as described in this example.

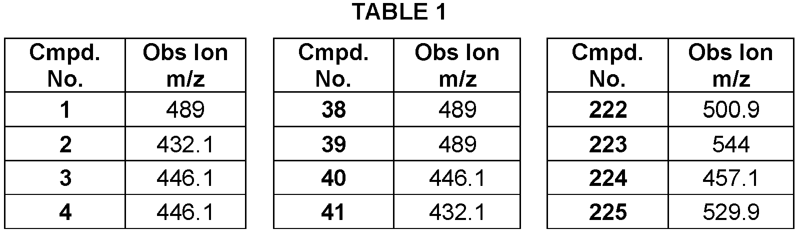

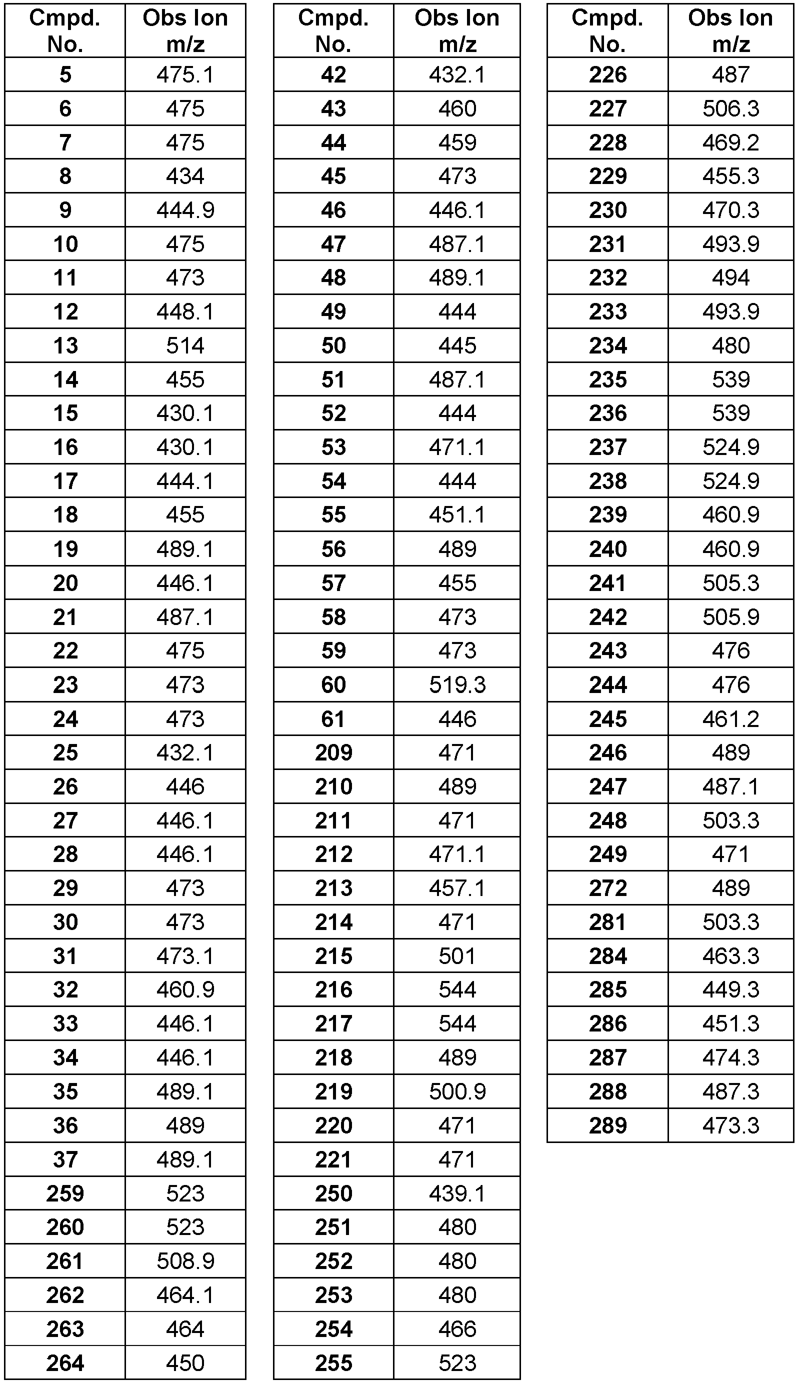

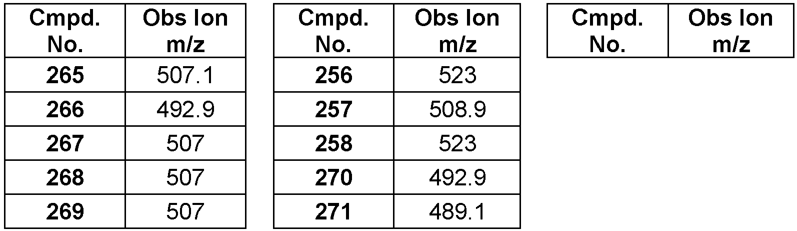

### Example 7: Preparation of (2R,6S)-2,6-Dimethyl-4-[5-(trifluoromethyl)pyrazin-2-yl]piperazine-1-carbonyl chloride (Intermediate 7A).

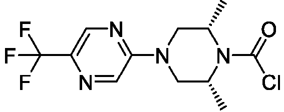

To a suspension of 2-[(3*R*,5*S*)-3,5-dimethylpiperazin-1-yl]-5-(trifluoromethyl)pyrazine (2.6 g, 10 mmol, 1.0 eq) in ACN (70 mL) cooled to 0 °C was added triphosgene (3.6 g, 12 mmol, 1.2 eq) followed by pyridine (1.2 mL, 15 mmol, 1.5 eq) dropwise. The resulting mixture was allowed to warm to room temperature and stirred overnight. The resulting light orange suspension was concentrated *in vacuo* to afford an orange solid. The solid was re-suspended in EtOAc, stirred rapidly for 30 min and filtered. The filtrate was concentrated to dryness *in vacuo* to provide (2*R*,6*S*)-2,6-dimethyl-4-[5-(trifluoromethyl)pyrazin-2-yl]piperazine-1-carbonyl chloride (**Intermediate 7A,** 1.9 g, 5.9 mmol, 59%) as a light orange solid which was used without further purification.

Other intermediates useful in the preparations of compounds of the present invention were made using substantially the same procedures described above, including:

| **Intermediate** | **Chemical Structure** | **Chemical Name** |
|---|---|---|
| **7B** | 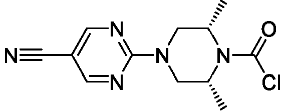 | (2*R*,6*S*)-4-(5-cyanopyrimidin-2-yl)-2,6-dimethylpiperazine-1-carbonyl chloride |
| **7C** | 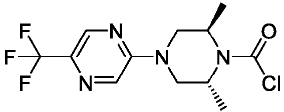 | (2*R*,6*R*)-2,6-dimethyl-4-[5-(trifluoromethyl)pyrazin-2-yl]piperazine-1-carbonyl chloride |
| **7D** | 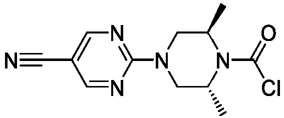 | (2*R*,6*R*)-4-(5-cyanopyrimidin-2-yl)-2,6-dimethylpiperazine-1-carbonyl chloride |
| **7E** | 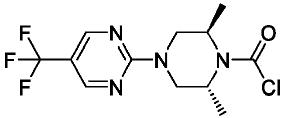 | (2*R*,6*R*)-2,6-dimethyl-4-[5-(trifluoromethyl)pyrimidin-2-yl]piperazine-1-carbonyl chloride |
| **7F** | 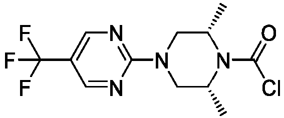 | (2*R*,6*S*)-2,6-dimethyl-4-[5-(trifluoromethyl)pyrimidin-2-yl]piperazine-1-carbonyl chloride |
| **7G** | 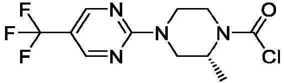 | (2*R*)-2-methyl-4-[5-(trifluoromethyl)pyrimidin-2-yl]piperazine-1-carbonyl chloride |
| **7H** | 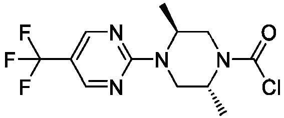 | (2*R*,5*S*)-2,5-dimethyl-4-[5-(trifluoromethyl)pyrimidin-2-yl]piperazine-1-carbonyl chloride |
| **7I** | 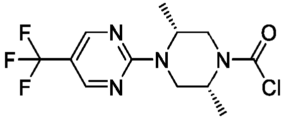 | (2*R*,5*R*)-2,5-dimethyl-4-[5-(trifluoromethyl)pyrimidin-2-yl]piperazine-1-carbonyl chloride |
| **7J** | 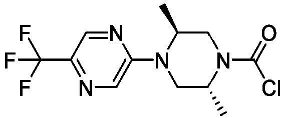 | (2*R*,5*S*)-2,5-dimethyl-4-[5-(trifluoromethyl)pyrazin-2-yl]piperazine-1-carbonyl chloride |
| **7K** | 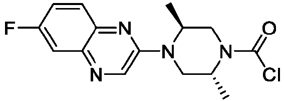 | (2R,5S)-4-(6-fluoroquinoxalin-2-yl)-2,5-dimethylpiperazine-1-carbonyl chloride |
| **7L** | 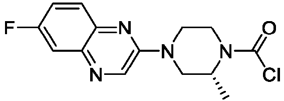 | (2R)-4-(6-fluoroquinoxalin-2-yl)-2-methylpiperazine-1-carbonyl chloride |
| **7M** | 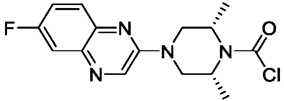 | (2R,6S)-4-(6-fluoroquinoxalin-2-yl)-2,6-dimethylpiperazine-1-carbonyl chloride |
| **7N** | 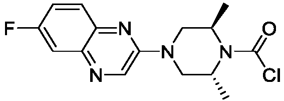 | (2R,6R)-4-(6-fluoroquinoxalin-2-yl)-2,6-dimethylpiperazine-1-carbonyl chloride |
| **7Q** | 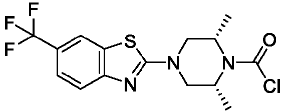 | (2R,6S)-2,6-dimethyl-4-[6-(trifluoromethyl)-1,3-benzothiazol-2-yl]piperazine-1-carbonyl chloride |
| **7R** | 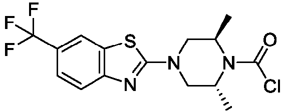 | (2R,6R)-2,6-dimethyl-4-[6-(trifluoromethyl)-1,3-benzothiazol-2-yl]piperazine-1-carbonyl chloride |
| **7S** | 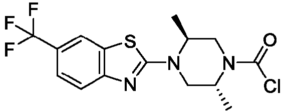 | (2R,5S)-2,5-dimethyl-4-[6-(trifluoromethyl)-1,3-benzothiazol-2-yl]piperazine-1-carbonyl chloride |
| **7T** | 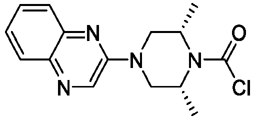 | (2R,6S)-2,6-dimethyl-4-(quinoxalin-2-yl)piperazine-1-carbonyl chloride |
| **7V** | 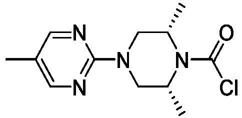 | (2R,6S)-2,6-dimethyl-4-(5-methylpyrimidin-2-yl)piperazine-1-carbonyl chloride |
| **7W** | 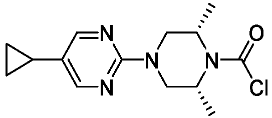 | (2R,6S)-4-(5-cyclopropylpyrimidin-2-yl)-2,6-dimethylpiperazine-1-carbonyl chloride |

### Example 8: Preparation of (2R,6S)-N-{2-Benzyl-2-azaspiro[3.3]heptan-6-yl}-2,6-dimethyl-4-[5-(trifluoromethyl)pyrazin-2-yl]piperazine-1-carboxamide (Compound 62).

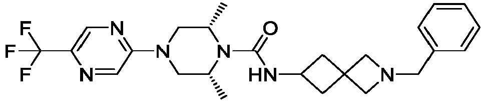

To a solid mixture of (2*R*,6*S*)-2,6-dimethyl-4-[5-(trifluoromethyl)pyrazin-2-yl]piperazine-1-carbonyl chloride (**Intermediate 7A**, 0.30 g, 0.93 mmol, 1.0 eq) and *tert*-butyl 6-amino-2-azaspiro[3.3]heptane-2-carboxylate (0.20 g, 0.93 mmol, 1.0 eq) was added ACN (4 mL) followed by DIPEA (0.61 mL, 3.7 mmol, 4.0 eq). The resulting mixture was stirred at 50 °C overnight. Subsequently, the mixture was concentrated *in vacuo.* The crude material was purified by silica gel column (24 g) using DCM and run with an increasing gradient of EtOAc (0-100%) in hexanes over 25 min to provide the Boc-protected intermediate:

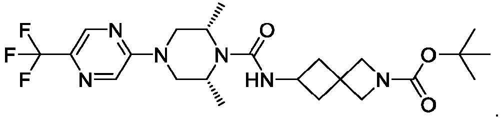

The isolated Boc-protected intermediate was re-dissolved in DCM (4 mL), treated with TFA (1 mL) and stirred at room temperature overnight. The mixture was concentrated *in vacuo,* re-dissolved in MeOH and combined with MP-carbonate resin to remove TFA. The resin was removed by filtration and the filtrate concentrated to provide the free amine (59 mg, 0.15 mmol, 16% over 2 steps) as a clear film.

To a portion of free amine (8.0 mg, 0.020 mmol, 1.0 eq) in ethanol (0.5 mL) was added benzaldehyde (4.0 µL, 0.040 mmol, 2.0 eq), sodium cyanoborohydride (2.5 mg, 0.040 mmol, 2.0 eq) followed by acetic acid (1.1 µL, 0.020 mmol, 1.0 eq). The resulting mixture was stirred at room temperature for 2 hrs. Subsequently, the mixture was diluted to a total volume of 1 mL using MeOH and submitted directly for preparative chromatography yielding (2*R*,6S)-*N*-{2-benzyl-2-azaspiro[3.3]heptan-6-yl}-2,6-dimethyl-4-[5-(trifluoromethyl)pyrazin-2-yl]piperazine-1-carboxamide (**Compound 62**).

The table below provides the observed (Obs) ion m/z ratio for the title compound (**Compound 62**, first compound listed in **TABLE 2**) and other compounds of the present invention that were prepared in substantially the same manner as described in this example.

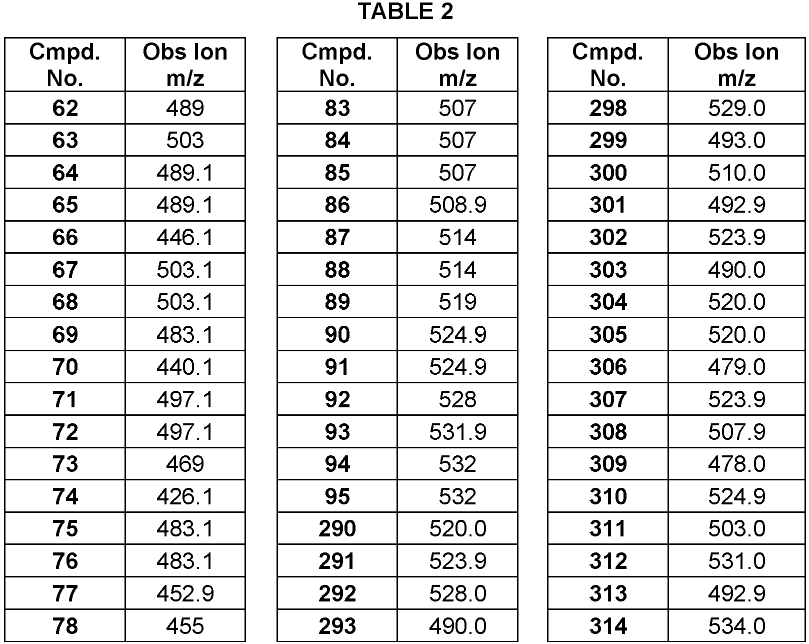

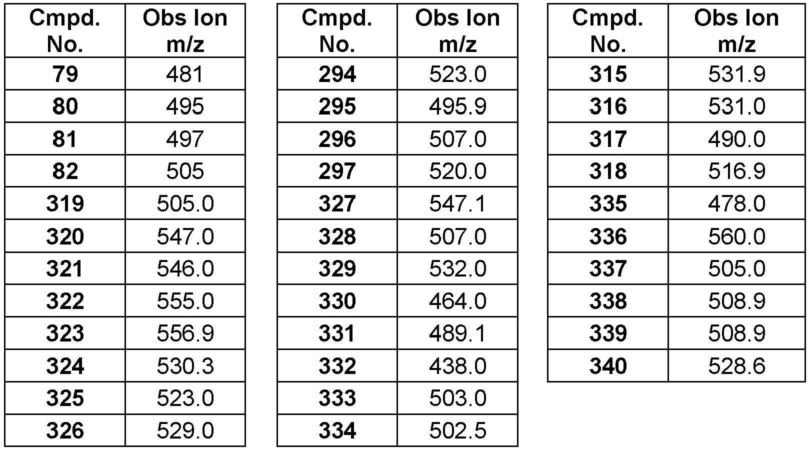

### Example 9: Preparation of (2R,6S)-N-{2-Benzyl-2-azaspiro[3.3]heptan-6-yl}-2,6-dimethyl-4-[5-(trifluoromethyl)pyrimidin-2-yl]piperazine-1-carboxamide (Compound 96).

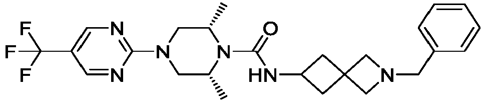

To a solution of triphosgene (0.12 g, 0.40 mmol, 0.4 eq) in DCM (2 mL) was added a solution of 2-[(3*R*,5*S*)-3,5-dimethylpiperazin-1-yl]-5-(trifluoromethyl)pyrimidine (**Intermediate 3A**, 0.26 g, 1.0 mmol, 1.0 eq) and TEA (0.42 mL, 3.0 mmol, 3.0 eq) in DCM (2 mL) dropwise. After stirring for 10 min, the mixture was treated with a solution of *tert*-butyl 6-amino-2-azaspiro[3.3]heptane-2-carboxylate (0.23 g, 1.1 mmol, 1.1 eq) in DCM (2 mL) with TEA (0.42 mL, 3.0 mmol, 3.0 eq) and the resulting mixture was stirred at room temperature overnight. The resulting suspension was concentrated *in vacuo.* The crude material was purified by silica gel column (24 g) using DCM and run with an increasing gradient of EtOAc (5-100%) in hexanes over 25 min to provide the Boc-protected intermediate (*i*.*e*., *tert*-butyl 6-((2*R*,6*S*)-2,6-dimethyl-4-(5-(trifluoromethyl)pyrimidin-2-yl)piperazine-1-carboxamido)-2-azaspiro[3.3]heptane-2-carboxylate):

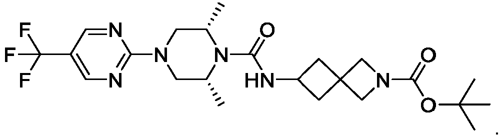

The isolated Boc-protected intermediate was re-dissolved in DCM (3 mL), treated with TFA (0.5 mL) and stirred at room temperature overnight. The reaction was carefully quenched with sat. NaHCO₃ and extracted with 5:1 DCM:2-propanol. The combined organic layers were dried over MgSO₄, filtered to remove solid and concentrated *in vacuo* to provide the free amine (32 mg, 0.08 mmol, 20% over 2 steps) as a clear film.

To a portion of free amine (10 mg, 0.025 mmol, 1.0 eq) in dichloroethane (0.5 mL) was added benzaldehyde (3.7 µL, 0.037 mmol, 1.5 eq) followed by sodium triacetoxyborohydride (16 mg, 0.075 mmol, 3.0 eq). The resulting mixture was stirred at room temperature overnight. Subsequently, the mixture was diluted to a total volume of 1 mL using MeOH and submitted directly for preparative chromatography yielding (2*R*,6*S*)-*N*-{2-benzyl-2-azaspiro[3.3]heptan-6-yl}-2,6-dimethyl-4-[5-(trifluoromethyl)pyrimidin-2-yl]piperazine-1-carboxamide (**Compound 96**). ¹H NMR (400 MHz, DMSO-*d*₆): δ (ppm) 8.71 (s, 2H), 7.33-7.20 (m, 5H), 6.50 (d, *J =* 7.1 Hz, 1H), 4.58 (d, *J* = 12.8 Hz, 2H), 4.23 (m, 2H), 4.04 (m, 1H), 3.50 (s, 2H), 3.15 (m, 4H), 3.06 (s, 2H), 2.31 (m, 2H), 2.04 (m, 2H), 1.05 (d, *J* = 6.8, 6H).

The table below provides the observed (Obs) ion m/z ratio for the title compound (**Compound 96**, the first compound listed in **TABLE 3**) and other compounds of the present invention that were prepared in substantially the same manner as described in this example. Deuterated compounds were prepared as described above with the following exception: sodium cyanoborohydride or sodium cyanoborodeuteride in place of sodium triacetoxyborohydride.

**TABLE 3**

| **Cmpd. No.** | **Obs Ion m/z** |
|---|---|
| **96** | 489.1 |
| **97** | 503.1 |
| **98** | 483.1 |
| **99** | 497.1 |
| **100** | 469 |
| **101** | 483.1 |
| **273** | 494.4 |
| **274** | 495.3 |
| **275** | 496.2 |

The hydrochloride salt for **Compound 96** was prepared by dissolving the free base (2.2 g, 4.5 mmol, 1.0 eq) in methyl *tert*-butyl ether (1.0 L) and adding a solution of 2.0 M HCl in diethyl ether (2.4 mL, 4.7 mmol, 1.05 mmol) dropwise with stirring. The resulting milky suspension was concentrated to dryness, re-suspended in pentane (1.0 L) and rapidly stirred overnight. The resulting white powder was collected by vacuum filtration under a nitrogen atmosphere, ground with mortar and pestle, and dried under high vacuum to remove any residual solvent to provide the HCl salt of **Compound 96**. ¹H NMR (400 MHz, DMSO-*d*₆): δ (ppm) 10.92 (s, 1H), 8.71 (s, 2H), 7.52 (m, 2H), 7.44 (m, 3H), 6.57 (d, *J* = 7.2 Hz, 1H), 4.59 (d, *J* = 13.2 Hz, 2H), 4.29 (d, *J* = 6.2 Hz, 2H), 4.22 (m, 2H), 4.12-4.00 (m, 4H), 3.91 (m, 1H), 3.16 (dd, *J* = 12.7, 4.2 Hz, 2H), 2.56 (m, 1H), 2.44 (m, 1H), 2.18 (m, 2H), 1.05 (d, J = 6.8, 6H).

HCl salts of **Compound 26, Compound 35**, and **Compound 106**, were prepared in substantially the same manner as described in the above example for preparing the HCl salt of **Compound 96**.

### Example 10: Preparation of 2-benzyl-2-azaspiro[3.3]heptan-6-ol (Intermediate 10A)

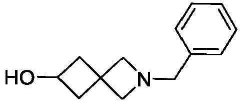

To a suspension of 2-azaspiro[3.3]heptan-6-ol hydrochloride (4.6 g, 30.7 mmol, 1.0 eq) in dichloroethane (160 mL) was added benzaldehyde (4.6 mL, 46.0 mmol, 1.5 eq) followed by sodium triacetoxyborohydride (32 g, 153 mmol, 5.0 eq). The resulting mixture was stirred at room temperature overnight. The formed suspension was carefully diluted and stirred with sat. NaHCO₃ until the evolution of hydrogen ceased. The aqueous mixture was extracted with 5:1 DCM:2-propanol. The combined organic layers were dried over MgSO₄, filtered to remove solid and concentrated *in vacuo.* The crude material was purified by silica gel column (120 g) using DCM and run with an increasing gradient of MeOH (0-20%) in DCM over 20 min, flushing with 50% MeOH to provide 2-benzyl-2-azaspiro[3.3]heptan-6-ol (**Intermediate 10A**, 6.1 g, 30.0 mmol, 98%) as an orange liquid.

Other intermediates useful in the preparations of compounds of the present invention were made using substantially the same procedures described above, including:

| **Intermediate** | **Chemical Structure** | **Chemical Name** |
|---|---|---|
| **10B** | 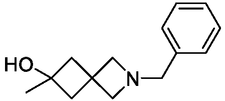 | 2-benzyl-6-methyl-2-azaspiro[3.3]heptan-6-ol |
| **10C** | 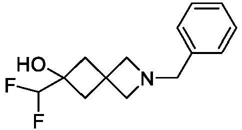 | 2-benzyl-6-(difluoromethyl)-2-azaspiro[3.3]heptan-6-ol |
| **10D** | 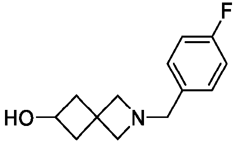 | 2-[(4-fluorophenyl)methyl]-2-azaspiro[3.3]heptan-6-ol |
| **10E** | 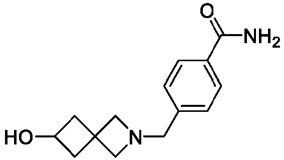 | 4-({6-hydroxy-2-azaspiro[3.3]heptan-2-yl}methyl)benzamide |
| **10F** | 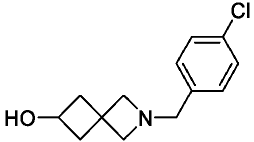 | 2-[(4-chlorophenyl)methyl]-2-azaspiro[3.3]heptan-6-ol |
| **10G** | 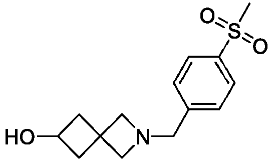 | 2-[(4-methanesulfonylphenyl)methyl]-2-azaspiro[3.3]heptan-6-ol |

### Example 11: Preparation of 2-benzyl-2-azaspiro[3.3]heptan-6-yl 4-(4-cyano-2-fluorophenyl)piperazine-1-carboxylate (Compound 102).

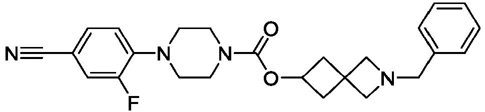

To a solution of 2-benzyl-2-azaspiro[3.3]heptan-6-ol (**Intermediate 10A**) (0.10 g, 0.49 mmol, 1.0 eq) in dry ACN (2.5 mL) was added carbonyldiimidazole (0.08 g, 0.49 mmol, 1.0 eq). The resulting mixture was stirred at room temperature for 1 hr. To an aliquot of the resulting solution (0.5 mL, 0.10 mmol, 1 eq) was added 3-fluoro-4-(piperazin-1-yl)benzonitrile (20 mg, 0.20 mmol, 1.0 eq) and DIPEA (100 µL) in dry ACN (0.3 mL) and the reaction stirred at 80 °C overnight (additional aliquots were used to prepare the remaining compounds listed in **TABLE 4** using the appropriate secondary amine in place of 3-fluoro-4-(piperazin-1-yl)benzonitrile). The reaction mixture was diluted to a total volume of 1 mL using MeOH and submitted directly for preparative chromatography yielding 2-benzyl-2-azaspiro[3.3]heptan-6-yl 4-(4-cyano-2-fluorophenyl)piperazine-1-carboxylate (**Compound 102**).

The table below provides the observed (Obs) ion m/z ratio for the title compound (**Compound 102,** the first compound listed in **TABLE 4**) and other compounds of the present invention that were prepared in substantially the same manner as described in this example.

**TABLE 4**

| **Cmpd. No.** | **Obs Ion m/z** |
|---|---|
| **102** | 435 |
| **103** | 515 |
| **104** | 431 |
| **105** | 446 |
| **106** | 490 |
| **107** | 444.9 |
| **108** | 476 |
| **109** | 474 |
| **110** | 474 |
| **111** | 431 |
| **112** | 419 |
| **113** | 474 |
| **114** | 474 |
| **115** | 474 |
| **116** | 462.1 |
| **117** | 447 |
| **118** | 447.1 |
| **119** | 433 |
| **120** | 433.1 |
| **121** | 460.9 |
| **122** | 474 |
| **123** | 476 |
| **124** | 452.9 |
| **125** | 460 |
| **126** | 484.9 |
| **127** | 435 |
| **128** | 447.1 |
| **129** | 433 |

**Compound 106**: ¹H NMR (400 MHz, DMSO-d6): δ 8.50 (s, 1H), 8.42 (s, 1H), 7.32 - 7.18 (m, 5H), 4.79 (t, *J =* 7.0 Hz, 1H), 4.68 (s, 1H), 4.30 (s, 1H), 4.14 (d, *J =* 13.9 Hz, 1H), 3.73 - 3.64 (m, 1H), 3.49 (s, 2H), 3.39 (dt, *J* = 14.3, 4.9 Hz, 2H), 3.13 (d, *J* = 17.0 Hz, 4H), 2.50 - 2.42 (m, 2H), 2.19 - 2.09 (m, 2H), 1.12 (dd, *J* = 14.6, 6.5 Hz, 6H).

### Example 12: Preparation of 2-Benzyl-2-azaspiro[3.3]heptan-6-yl (2R,6S)-4-(5-cyanopyrimidin-2-yl)-2,6-dimethylpiperazine-1-carboxylate (Compound 130).

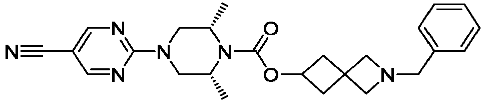

To a solid mixture of 2-benzyl-2-azaspiro[3.3]heptan-6-ol (**Intermediate 10A**, 22 mg, 0.11 mmol, 1.0 eq) and KHMDS (33 mg, 0.16 mmol, 1.5 eq) was added dry THF (0.5 mL). The resulting mixture was stirred at room temperature for 10 min and then treated with (2*R*,6*S*)-4-(5-cyanopyrimidin-2-yl)-2,6-dimethylpiperazine-1-carbonyl chloride (**Intermediate 7B**) (30 mg, 0.11 mmol, 1.0 eq). The resulting mixture was stirred at room temperature overnight. The mixture was diluted to a total volume of 1 mL using MeOH, filtered and submitted directly for preparative chromatography yielding 2-benzyl-2-azaspiro[3.3]heptan-6-yl (2*R*,6*S*)-4-(5-cyanopyrimidin-2-yl)-2,6-dimethylpiperazine-1-carboxylate (**Compound 130**).

The table below provides the observed (Obs) ion m/z ratio for the title compound (**Compound 130**, the first compound listed in **TABLE 5**) and other compounds of the present invention that were prepared in substantially the same manner as described in this example.

**TABLE 5**

| **Cmpd. No.** | **Obs Ion m/z** |
|---|---|
| **130** | 447.1 |
| **131** | 461 |
| **132** | 476 |
| **133** | 490 |
| **134** | 504 |
| **135** | 490 |
| **341** | 540.3 |
| **342** | 540 |
| **343** | 539.9 |
| **344** | 539.9 |
| **345** | 539.9 |
| **346** | 539.9 |
| **347** | 540.3 |
| **348** | 483 |
| **349** | 526 |
| **350** | 526 |
| **351** | 461 |
| **352** | 504 |
| **353** | 504 |
| **354** | 461 |
| **355** | 504 |
| **356** | 504 |
| **357** | 460.9 |
| **358** | 504 |
| **359** | 504 |

### Example 13: Preparation of 2-Benzyl-2-azaspiro[3.3]heptan-6-yl (2R,5S)-2,5-dimethyl-4-[5-(trifluoromethyl)pyrimidin-2-yl]piperazine-1-carboxylate (Compound 136).

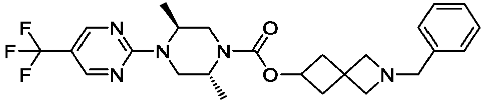

To a solution of triphosgene (11 mg, 0.04 mmol, 0.4 eq) in DCM (0.25 mL) was added a solution of 2-[(2*S*,5*R*)-2,5-dimethylpiperazin-1-yl]-5-(trifluoromethyl)pyrimidine (**Intermediate 2A,** 26 mg, 0.1 mmol, 1.0 eq) and TEA (42 µL, 0.3 mmol, 3.0 eq) in DCM (0.5 mL) dropwise. At the same time, a solution of 2-benzyl-2-azaspiro[3.3]heptan-6-ol (**Intermediate 10A,** 20 mg, 0.10 mmol, 1.0 eq) in THF (0.5 mL) was treated with KHMDS (30 mg, 0.15 mmol, 1.5 eq) and the resulting mixture stirred at room temperature for 10 min affording a suspension. Subsequently, the suspension was added in one portion to the DCM mixture and the resulting mixture stirred at room temperature overnight. The mixture was filtered and submitted directly for preparative chromatography yielding 2-benzyl-2-azaspiro[3.3]heptan-6-yl (2*R*,5*S*)-2,5-dimethyl-4-[5-(trifluoromethyl)pyrimidin-2-yl]piperazine-1-carboxylate (**Compound 136**).

The table below provides the observed (Obs) ion m/z ratio for the title compound (**Compound 136,** the first compound listed in **TABLE 6**) and other compounds of the present invention that were prepared in substantially the same manner as described in this example.

**TABLE 6**

| **Cmpd. No.** | **Obs Ion m/z** |
|---|---|
| **136** | 490.1 |
| **137** | 476 |
| **138** | 447 |

### Example 14: Preparation of 2-Benzyl-2-azaspiro[3.3]heptan-6-yl (2R,6R)-2,6-dimethyl-4-[5-(trifluoromethyl)pyrimidin-2-yl]piperazine-1-carboxylate (Compound 139).

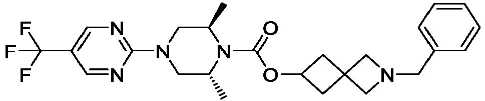

To a solution of *tert-butyl* 6-hydroxy-2-azaspiro[3.3]heptane-2-carboxylate (0.22 g, 1.0 mmol, 1.0 eq) in THF (3 mL) was added KHMDS (0.24 g, 1.2 mmol, 1.2 eq). The resulting mixture was stirred at room temperature for 10 min. Subsequently, the suspension was added in one portion to a suspension of (2*R*,6*R*)-2,6-dimethyl-4-[5-(trifluoromethyl)pyrimidin-2-yl]piperazine-1-carbonyl chloride (**Intermediate 7E,** 0.32 g, 1.0 mmol, 1.0 eq) in THF (2 mL) and the resulting mixture stirred at room temperature overnight affording a suspension. The suspension was concentrated *in vacuo.* The crude material was purified by silica gel column (24 g) using DCM and run with an increasing gradient of EtOAc (5-70%) in hexanes over 25 min to provide the Boc-protected intermediate (*i.e., tert*-butyl 6-(((2*R*,6*R*)-2,6-dimethyl-4-(5-(trifluoromethyl)pyrimidin-2-yl)piperazine-1-carbonyl)oxy)-2-azaspiro[3.3]heptane-2-carboxylate).

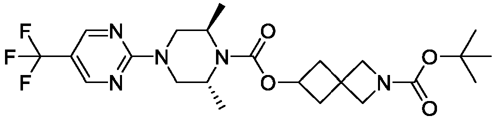

The isolated Boc-protected intermediate was re-dissolved in DCM (3 mL), treated with TFA (0.50 mL) and stirred at room temperature overnight. The reaction was carefully quenched with sat. NaHCO₃ and extracted with 5:1 DCM:2-propanol. The combined organic layers were dried over MgSO₄, filtered to remove solid and concentrated *in vacuo* to provide the free amine (40 mg, 0.10 mmol, 10% over 2 steps) as a clear film.

To a portion of free amine (10 mg, 0.025 mmol, 1.0 eq) in dichloroethane (0.5 mL) was added benzaldehyde (3.7 µL, 0.037 mmol, 1.5 eq) followed by sodium triacetoxyborohydride (16 mg, 0.075 mmol, 3.0 eq). The resulting mixture was stirred at room temperature overnight. The mixture was diluted to a total volume of 1 mL using MeOH and submitted directly for preparative chromatography yielding 2-benzyl-2-azaspiro[3.3]heptan-6-yl (2*R*,6*R*)-2,6-dimethyl-4-[5-(trifluoromethyl)pyrimidin-2-yl]piperazine-1-carboxylate (**Compound 139**).

The table below provides the observed (Obs) ion m/z ratio for the title compound (Compound 139, the first compound listed in **TABLE 7**) and other compounds of the present invention that were prepared in substantially the same manner as described in this example.

**TABLE 7**

| **Cmpd. No.** | **Obs Ion m/z** |
|---|---|
| **139** | 490.1 |
| **140** | 504 |
| **141** | 490 |
| **142** | 490 |
| **143** | 504 |
| **144** | 504 |
| **145** | 484.1 |
| **146** | 470 |

**Compound 141:** ¹H NMR (400 MHz, DMSO-d6): δ 8.54 - 8.45 (m, 2H), 7.34 - 7.18 (m, 5H), 4.80 (p, *J* = 7.1 Hz, 1H), 4.41 (d, *J* = 13.4 Hz, 2H), 4.26 - 4.15 (m, 2H), 3.51 (s, 2H), 3.23 (dd, *J* = 13.4, 4.6 Hz, 2H), 3.17 (s, 2H), 3.13 (s, 2H), 2.52 - 2.44 (m, 2H), 2.16 - 2.06 (m, 2H), 1.13 (d, *J* = 6.9 Hz, 6H).

### Example 15: Preparation of 2-Benzyl-2-azaspiro[3.3]heptan-6-yl (2R,6S)-2,6-dimethyl-4-[5-(trifluoromethyl)pyrimidin-2-yl]piperazine-1-carboxylate (Compound 147).

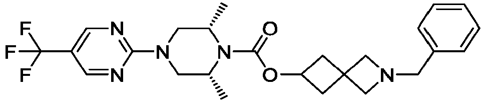

To a solution of triphosgene (0.12 g, 0.40 mmol, 0.4 eq) in DCM (1.5 mL) was added a solution of 2-[(3*R*,5*S*)-3,5-dimethylpiperazin-1-yl]-5-(trifluoromethyl)pyrimidine (**Intermediate 2A,** 0.26 g, 1.0 mmol, 1.0 eq) and TEA (0.42 mL, 3.0 mmol, 3.0 eq) in DCM (1.5 mL) dropwise. At the same time, to a solution of *tert*-butyl 6-hydroxy-2-azaspiro[3.3]heptane-2-carboxylate (0.26 g, 1.2 mmol, 1.2 eq) in THF (3 mL) was added KHMDS (0.24 g, 1.2 mmol, 1.2 eq) and the resulting mixture stirred at room temperature for 10 min affording a suspension. Subsequently, the suspension was added in one portion to the DCM mixture and the resulting mixture stirred at room temperature overnight affording a suspension. The suspension was concentrated *in vacuo.* The crude material was purified by silica gel column (24 g) using DCM and run with an increasing gradient of EtOAc (5-60%) in hexanes over 25 min to provide the Boc-protected intermediate (*tert*-butyl 6-(((2*R*,6*S*)-2,6-dimethyl-4-(5-(trifluoromethyl)pyrimidin-2-yl)piperazine-1-carbonyl)oxy)-2-azaspiro[3.3]heptane-2-carboxylate).

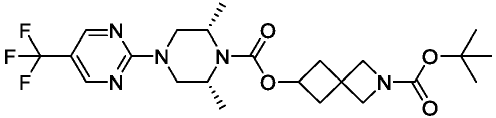

The isolated Boc-protected intermediate was re-dissolved in DCM (4 mL), treated with TFA (0.75 mL) and stirred at room temperature overnight. The reaction was carefully quenched with sat. NaHCO3 and extracted with 5:1 DCM:2-propanol. The combined organic layers were dried over MgSO₄, filtered to remove solid and concentrated *in vacuo* to provide the free amine (64 mg, 0.16 mmol, 16% over 2 steps) as a clear film.

To a portion of free amine (10 mg, 0.025 mmol, 1.0 eq) in dichloroethane (0.5 mL) was added benzaldehyde (3.7 µL, 0.037 mmol, 1.5 eq) followed by sodium triacetoxyborohydride (16 mg, 0.075 mmol, 3.0 eq). The resulting mixture was stirred at room temperature overnight. The mixture was diluted to a total volume of 1 mL using MeOH and submitted directly for preparative chromatography yielding 2-benzyl-2-azaspiro[3.3]heptan-6-yl (2*R*,6*S*)-2,6-dimethyl-4-[5-(trifluoromethyl)pyrimidin-2-yl]piperazine-1-carboxylate (**Compound 147**).

The table below provides the observed (Obs) ion m/z ratio for the title compound (**Compound 147,** the first compound listed in **TABLE 8**) and other compounds of the present invention that were prepared in substantially the same manner as described in this example. Deuterated compounds were prepared as described above with the following exception: sodium cyanoborohydride or sodium cyanoborodeuteride in place of sodium triacetoxyborohydride.

**TABLE 8**

| **Cmpd. No.** | **Obs Ion m/z** |
|---|---|
| **147** | 490 |
| **148** | 504.1 |
| **149** | 484 |
| **150** | 498.1 |
| **361** | 497.3 |
| **375** | 496.3 |
| **388** | 497.2 |
| **151** | 470.1 |
| **152** | 484.1 |
| **153** | 482.1 |
| **283** | 495.3 |
| **373** | 495.3 |
| **376** | 496.3 |
| **154** | 496.1 |
| **155** | 506.1 |
| **156** | 526 |
| **360** | 496.3 |
| **374** | 495.3 |
| **387** | 497.2 |

The hydrochloride salt for **Compound 147** was prepared by dissolving the free base (0.67 g, 1.4 mmol, 1.0 eq) in methylene chloride (25 mL) and adding a solution of 2.0 M HCl in diethyl ether (0.77 mL, 1.5 mmol, 1.1 eq) dropwise with stirring. The resulting solution was concentrated to dryness, re-suspended in pentane (75 mL) and rapidly stirred overnight. The resulting white powder was collected by vacuum filtration under a nitrogen atmosphere, ground with mortar and pestle, and dried under high vacuum to remove any residual solvent to provide the HCl salt of **Compound 147**. ¹H NMR (400 MHz, DMSO-*d*₆): & (ppm) 10.42 (s, 1H), 8.71 (s, 2H), 7.48 - 7.44 (m, 5H), 4.83 (tt, *J* = 7.2, 7.1 Hz, 1H), 4.61 (d, *J* = 13.2 Hz, 2H), 4.32 (d, *J* = 5.7 Hz, 2H), 4.24 - 3.96 (m, 6H), 3.20 (dd, *J* = 13.3, 4.4 Hz, 2H), 2.72 - 2.67 (m, 1H), 2.62 - 2.56 (m, 1H), 2.34 - 2.22 (m, 2H), 1.11 (d, *J =* 6.9 Hz, 6H).

### Example 16: Preparation of 2-Benzyl-2-azaspiro[3.3]heptan-6-yl (2R,6S)-2,6-dimethylpiperazine-1-carboxylate (Intermediate 16A).

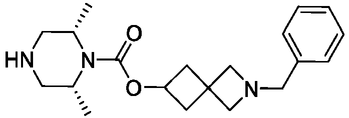

To a flame-dried flask under nitrogen was added triphosgene (1.66 g, 5.60 mmol, 0.4 eq) followed by anhydrous DCM (93 mL). The solution was cooled to 0 °C before dropwise addition of *tert*-butyl (3*R*,5*S*)-3,5-dimethylpiperazine-1-carboxylate (3.00 g, 14.00 mmol, 1.0 eq) and pyridine (1.48 mL, 14.00 eq, 1.0 eq) in anhydrous DCM (31 mL). The reaction was allowed stir at room temperature for 1 h then diluted with water and DCM. The organic layer was dried over anhydrous magnesium sulfate and concentrated *in vacuo* to provide *tert*-butyl (3*R*,5*S*)-4-(carbonochloridoyl)-3,5-dimethylpiperazine-1-carboxylate (2.50 g, 9.03 mmol, 65% yield) as a yellow oil. The carbamoyl chloride was used without further purification.

A solution containing 2-benzyl-2-azaspiro[3.3]heptan-6-ol (**Intermediate 10A,** 1.84 g, 9.03 mmol, 1.0 eq) and KHDMS (3.61 g, 18.10 mmol, 2.0 eq) in anhydrous THF (22 mL) was stirred for 15 min at room temperature. To the solution was added (3R,5S)-4-(carbonochloridoyl)-3,5-dimethylpiperazine-1-carboxylate (2.50 g, 9.03 mmol, 1.0 eq) in anhydrous ACN (22 mL) and the reaction was stirred overnight at 50 °C. Upon completion, the reaction was concentrated *in vacuo.* The crude material was dry loaded with DCM and silica gel. Silica gel column (80 g) was dry loaded and run with an increasing gradient of EtOAc (0-100%) in hexanes over 25 min then 100% EtOAc for 15 min to provide 1-{2-benzyl-2-azaspiro[3.3]heptan-6-yl} 4-*tert*-butyl (2*R*,6*S*)-2,6-dimethylpiperazine-1,4-dicarboxylate (2.45 g, 5.52 mmol, 61% yield).

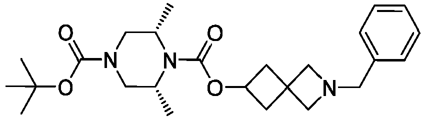

A solution of 1-{2-benzyl-2-azaspiro[3.3]heptan-6-yl} 4-*tert*-butyl (2*R*,6*S*)-2,6-dimethylpiperazine-1,4-dicarboxylate (2.45 g, 5.52 mmol, 1.0 eq) in anhydrous DCM (54 mL) was cooled to 0 °C before addition of TFA (6.0 mL, 78.43 mmol, 14.2 eq). The reaction was then allowed to warm to room temperature and stirred overnight. The reaction was then partially concentrated *in vacuo* and made basic with saturated sodium carbonate. The free amine was extracted with copious amounts of DCM. The combined organics were dried over anhydrous magnesium sulfate and concentrated *in vacuo* to provide 2-benzyl-2-azaspiro[3.3]heptan-6-yl (2*R*,6*S*)-2,6-dimethylpiperazine-1-carboxylate (**Intermediate 16A,** 1.89 g, 5.50 mmol, 99% yield) as colorless oil.

Other intermediates useful in the preparations of compounds of the present invention were made using substantially the same procedures described above, including:

| **Intermediate** | **Chemical Structure** | **Chemical Name** |
|---|---|---|
| **16B** | 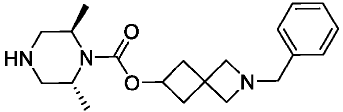 | 2-benzyl-2-azaspiro[3.3]heptan-6-yl (2*R*,6*R*)-2,6-dimethylpiperazine-1-carboxylate |
| **16C** | 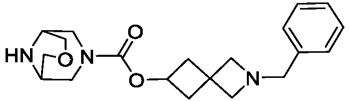 | 2-benzyl-2-azaspiro[3.3]heptan-6-yl 3-oxa-7,9-diazabicyclo[3.3.1]nonane-7-carboxylate |

### Example 17: Preparation of 2-benzyl-2-azaspiro[3.3]heptan-6-yl (2R,6S)-4-(5-fluoropyrimidin-2-yl)-2,6-dimethylpiperazine-1-carboxylate (Compound 157).

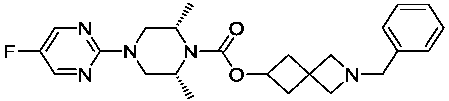

A solution containing 2-benzyl-2-azaspiro[3.3]heptan-6-yl (2*R*,6*S*)-2,6-dimethylpiperazine-1-carboxylate (**Intermediate 16A,** 10 mg, 0.029 mmol, 1.0 eq), 2-chloro-5-fluoropyrimidine (5.8 mg, 0.044 mmol, 1.5 eq), and DIPEA (20 µL, 0.12 mmol, 4.0 eq) in dry ACN (400 µL) was heated at 50 °C overnight. The reaction mixture was then diluted to a total volume of 1 mL using MeOH and submitted directly for preparative chromatography yielding 2-benzyl-2-azaspiro[3.3]heptan-6-yl (2*R*,6*S*)-4-(5-fluoropyrimidin-2-yl)-2,6-dimethylpiperazine-1-carboxylate (**Compound 157**).

The table below provides the observed (Obs) ion m/z ratio for the title compound (**Compound 157**, the first compound listed in **TABLE 9**) and other compounds of the present invention that were prepared in substantially the same manner as described in this example.

**TABLE 9**

| **Cmpd. No.** | **Obs Ion m/z** |
|---|---|
| **157** | 440 |
| **158** | 440.1 |
| **159** | 447 |
| **160** | 460.9 |
| **161** | 464 |
| **162** | 464 |
| **163** | 466 |
| **164** | 466.1 |
| **165** | 490 |
| **166** | 490 |
| **167** | 504 |
| **168** | 504 |
| **169** | 547.9 |
| **170** | 547.9 |
| **171** | 548 |

### Example 18: Preparation of 2-benzyl-2-azaspiro[3.3]heptan-6-yl (2R,6S)-2,6-dimethyl-4-(quinoxalin-2-yl)piperazine-1-carboxylate (Compound 172).

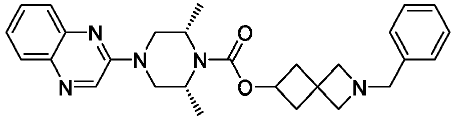

A solution containing 2-benzyl-2-azaspiro[3.3]heptan-6-yl (2*R*,6*S*)-2,6-dimethylpiperazine-1-carboxylate (**Intermediate 16A,** 10 mg, 0.029 mmol, 1.0 eq), 2-chloroquinoxaline (4.8 mg, 0.029 mmol, 1.0 eq), and sodium *tert*-butoxide (7.0 mg, 0.073 mmol, 2.5 eq) in degassed toluene (300 µL) was purged with nitrogen before addition of RhuPhos Pd Generation 2 (2.2 mg, 0.0029 mmol, 0.1 eq). The mixture was heated at 90 °C for 2 h before cooling to room temperature. The reaction mixture was then diluted to a total volume of 1 mL using MeOH and submitted directly for preparative chromatography yielding 2-benzyl-2-azaspiro[3.3]heptan-6-yl (2*R*,6*S*)-2,6-dimethyl-4-(quinoxalin-2-yl)piperazine-1-carboxylate (**Compound 172**).

The table below provides the observed (Obs) ion m/z ratio for the title compound (**Compound 172,** the first compound listed in **TABLE 10**) and other compounds of the present invention that were prepared in substantially the same manner as described in this example.

**TABLE 10**

| **Cmpd. No.** | **Obs Ion m/z** |
|---|---|
| **172** | 472 |
| **173** | 501.3 |
| **174** | 436 |
| **175** | 436 |
| **176** | 436.1 |
| **177** | 440.4 |
| **178** | 450.1 |
| **179** | 452 |
| **180** | 452 |
| **181** | 460.9 |
| **182** | 460.9 |
| **183** | 467.1 |
| **184** | 467.1 |
| **185** | 472 |
| **186** | 472 |
| **187** | 472.1 |
| **188** | 486 |
| **189** | 489 |
| **190** | 489 |
| **191** | 489 |
| **192** | 489 |
| **193** | 489 |
| **194** | 490 |
| **195** | 490 |
| **196** | 490 |
| **197** | 490 |
| **198** | 496 |
| **199** | 496 |
| **200** | 496 |
| **201** | 502.1 |
| **202** | 506 |
| **203** | 506 |
| **204** | 506 |
| **205** | 506 |
| **206** | 514 |
| **207** | 514 |
| **208** | 547.9 |

### Example 18A: Preparation 2-benzyl-2-azaspiro[3.3]heptan-6-yl (2R,6R)-4-(6-fluoroquinoxalin-2-yl)-2,6-dimethylpiperazine-1-carboxylate (Compound 362).

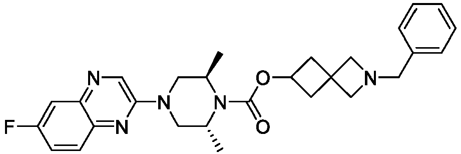

To a solution of 2-benzyl-2-azaspiro[3.3]heptan-6-ol (0.10 g, 0.49 mmol, 1.0 eq) in dry DCM (0.24 mL) was added *N*,*N'*-disuccinimidyl carbonate (0.14 g, 0.54 mmol, 1.1 eq). The reaction was stirred at room temperature overnight. To an aliquot of the resulting solution (0.049 mL, 0.10 mmol, 1.0 eq) was added 2-((3R,5R)-3,5-dimethylpiperazin-1-yl)-6-fluoroquinoxaline hydrochloride (0.031 g, 0.10 mmol, 1.0 eq), 4-DMAP (0.024 g, 0.20 mmol, 2.0 eq), and TEA (0.028 mL, 0.20 mmol, 2.0 eq) in dry DCM (0.049 mL) and reaction stirred at room temperature for 2 h then at 40 °C overnight if incomplete (additional aliquots were used to prepare the remaining compounds using the appropriate secondary amine hydrochloride in place of 2-((3R,5R)-3,5-dimethylpiperazin-1-yl)-6-fluoroquinoxaline hydrochloride). The reaction mixture was diluted to a total volume of 1 mL using MeOH and submitted directly for preparative chromatography yielding 2-benzyl-2-azaspiro[3.3]heptan-6-yl (2R,6R)-4-(6-fluoroquinoxalin-2-yl)-2,6-dimethylpiperazine-1-carboxylate (**Compound 362**).

Other carbamate compounds were prepared in a similar fashion as shown in examples 14, 15, 17, 18, and 18A. The table below provides the observed (Obs) ion m/z ratio for the title compounds.

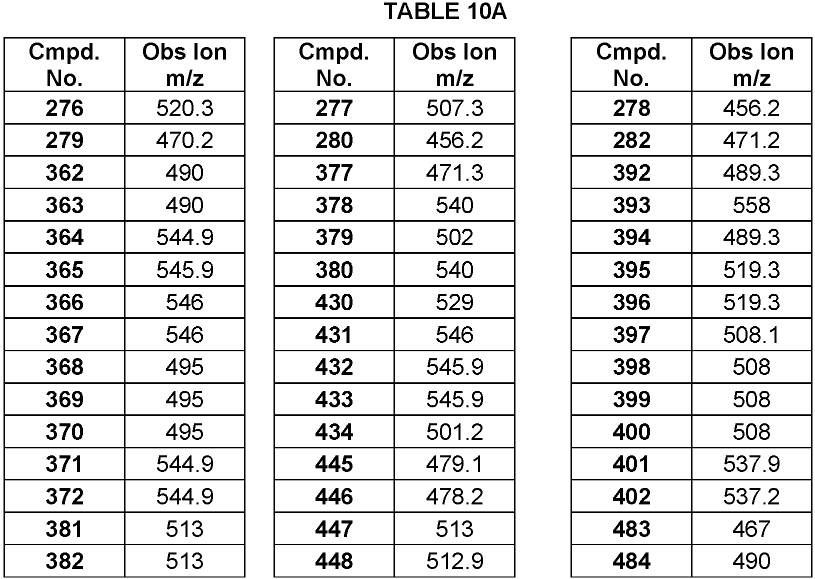

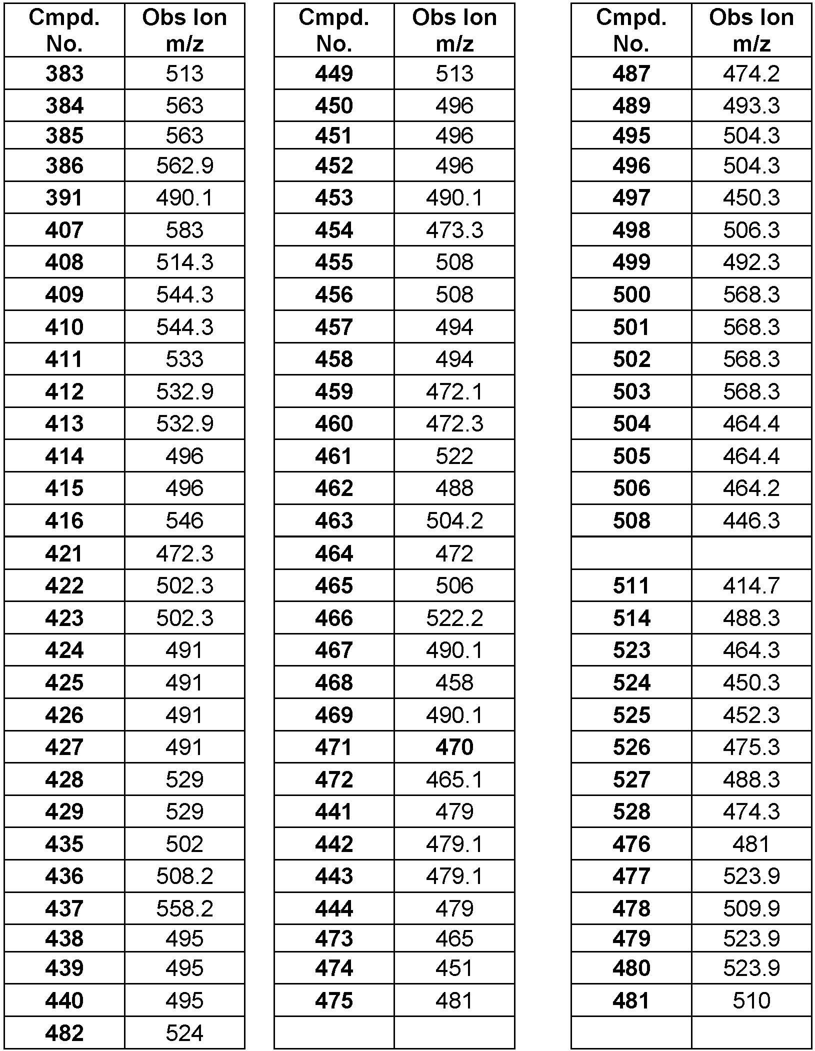

### Example 18B: Preparation of 2-benzyl-2-azaspiro[3.3]heptan-6-yl (2R,6S)-4-(5-(dimethylphosphoryl)pyrimidin-2-yl)-2,6-dimethylpiperazine-1-carboxylate (Compound 529).

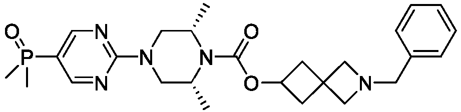

Following Example 18A, 2-benzyl-2-azaspiro[3.3]heptan-6-yl (2*R*,6*S*)-4-(5-iodopyrimidin-2-yl)-2,6-dimethylpiperazine-1-carboxylate was prepared:

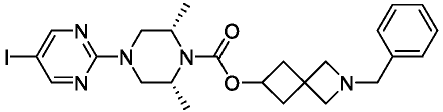

To a solution of the above 2-benzyl-2-azaspiro[3.3]heptan-6-yl (2*R*,6*S*)-4-(5-iodopyrimidin-2-yl)-2,6-dimethylpiperazine-1-carboxylate (0.020 g, 0.037 mmol, 1.0 eq) and (methylphosphonoyl)methane (3.0 µL, 0.037 mmol, 1.0 eq) in degassed 1,4-dioxanes (0.20 mL) was added a solution of tris(dibenzylideneacetone)dipalladium(0) (3.4 mg, 0.0037 mmol, 0.10 eq), 4,5-bis(diphenylphosphino)-9,9-dimethylxanthene (0.64 mg, 0.0011 mmol, 0.030 eq), and TEA (5.7 µL, 0.041 mmol, 1.1 eq) in degassed 1,4-dioxanes (0.20 mL). The resulting reaction was stirred at room temperature overnight. The reaction mixture was diluted to a total volume of 1 mL using MeOH and submitted directly for preparative chromatography yielding 2-benzyl-2-azaspiro[3.3]heptan-6-yl (2*R*,6*S*)-4-(5-(dimethylphosphoryl)pyrimidin-2-yl)-2,6-dimethylpiperazine-1-carboxylate (**Compound 529**). The observed ion m/z ratio for the title compound is 498.3.

### Example 18C: Preparation of 2-benzyl-2-azaspiro[3.3]heptan-6-yl (2R,6S)-4-[5-(cyclobutylmethoxy)pyrimidin-2-yl]-2,6-dimethylpiperazine-1-carboxylate (Compound 530).

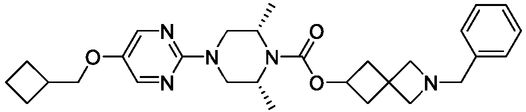

To a solution containing 2-benzyl-2-azaspiro[3.3]heptan-6-yl (2*R*,6*S*)-4-(5-iodopyrimidin-2-yl)-2,6-dimethylpiperazine-1-carboxylate (0.020 g, 0.037 mmol, 1.0 eq), cyclobutylmethanol (0.031 mg, 0.037 mmol, 1.0 eq), and cesium carbonate (0.018 g, 0.056 mmol, 1.5 eq) in degassed 1,4-dioxanes (0.50 mL) was added Cul (0.18 mg, 2.5 mol%) and 1,10-phenanthroline (1.3 mg, 20 mol%). The resulting reaction was stirred at room temperature for 72 h. The reaction mixture was diluted to a total volume of 1 mL using MeOH and submitted directly for preparative chromatography yielding 2-benzyl-2-azaspiro[3.3]heptan-6-yl (2R,6S)-4-[5-(cyclobutylmethoxy)pyrimidin-2-yl]-2,6-dimethylpiperazine-1-carboxylate (**Compound 530**). The observed ion m/z ratio for the title compound is 506.4.

### Example 19: Binding Assay.

Binding affinity (Kᵢ) for the compounds was measured by inhibition of radioligand binding to membranes from CHO cells expressing human M₄ receptor. Membranes were prepared by nitrogen cavitation and differential centrifugation as previously described (Hoare et al., Mol. Pharmacol. 2003 Mar; 63(3): 751-65). The radioligand employed was tritiated *N-*methylscopolamine, used at a concentration of 1.5 nM. A dose-response of twelve concentrations of compound was used, ranging from 10 µM to 32 pM. The assay buffer was 50 mM HEPES, 100 mM NaCl, 5 mM MgCl₂, 1 mM ethylenediaminetetraacetic acid, pH-adjusted to pH 7.4. Membranes, radioligand and compound were incubated together for 90 minutes at 37 °C, in a total volume of 150 µL in a 96-well plate. Receptor-bound radioligand was then collected by harvesting the assay over glass fiber filters pretreated with polyethylenimine to trap the cell membranes, using rapid vacuum filtration. Harvesting and radioactivity counting was conducted as previously described (see, *e.g.,* Hoare et al., Mol. Pharmacol. 2003 63(3):751-65); Erratum at Mol. Pharmacol. 2005 Jul; 68(1): 260).

Binding affinities of certain exemplified compounds, which are described in the examples and listed in the tables above, are less than 1 µM against the M₄ receptor. More specifically, specificity for the M₄ receptor for each of the compounds listed in **TABLE 11** is as follows: (1) "+" means the compound had a Kᵢ against the M₄ receptor of less than 1 µM (1,000 nM) but greater or equal to 100 nM; (2) "++" means the compound had a Kᵢ against the M₄ receptor of less than 100 nM but greater or equal to 10 nM; and (3) "+++" means that the compound had a Kᵢ against the M₄ receptor of less than 10 nM.

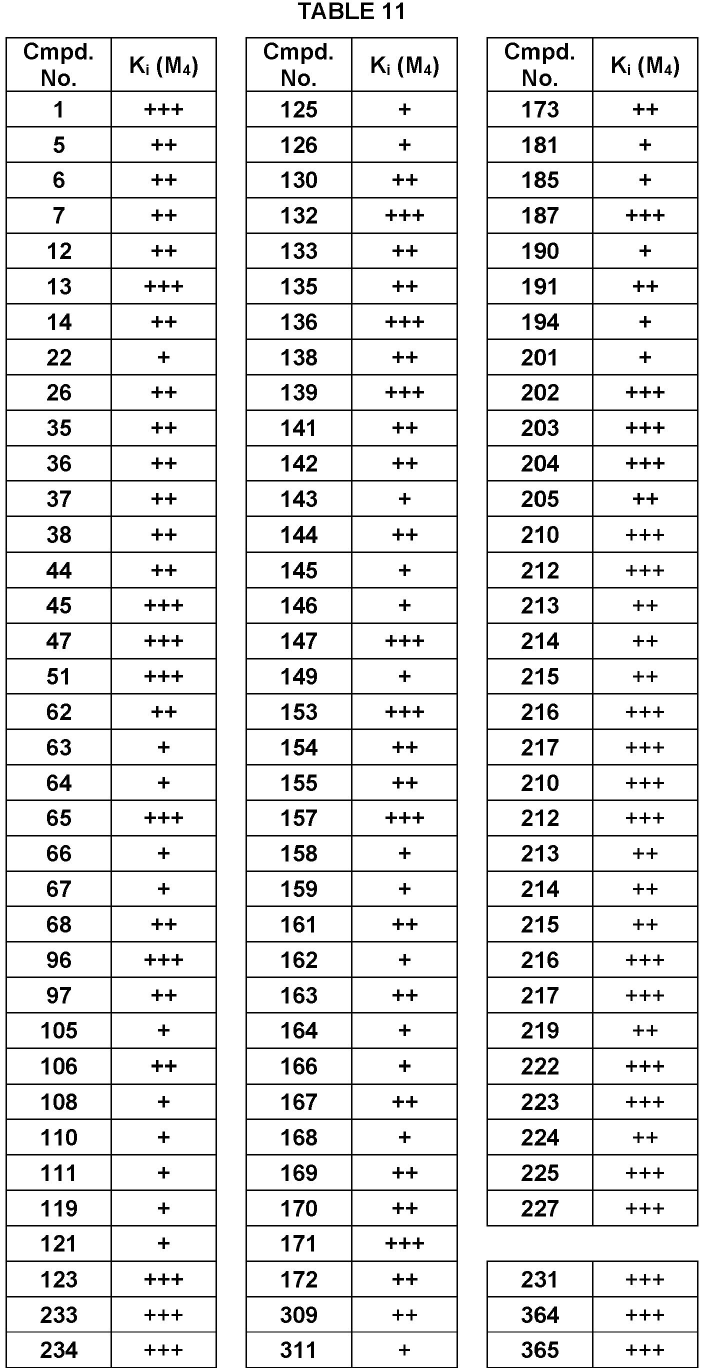

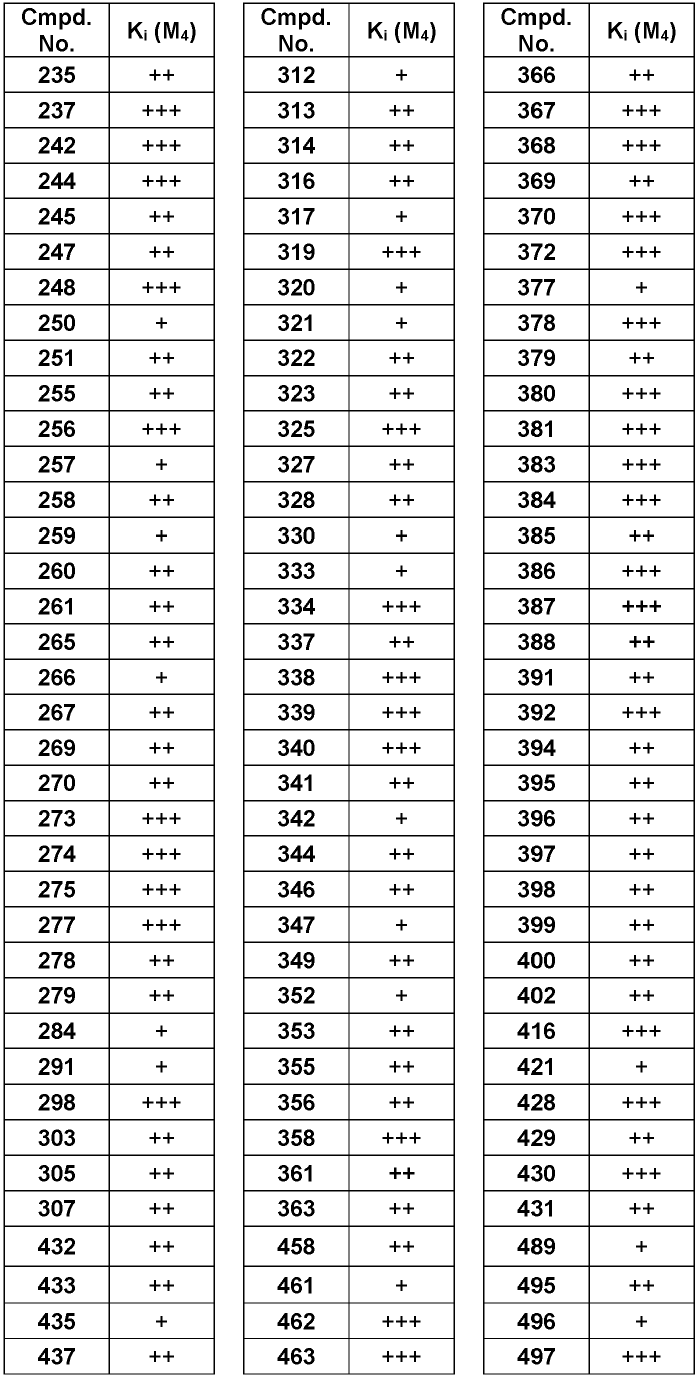

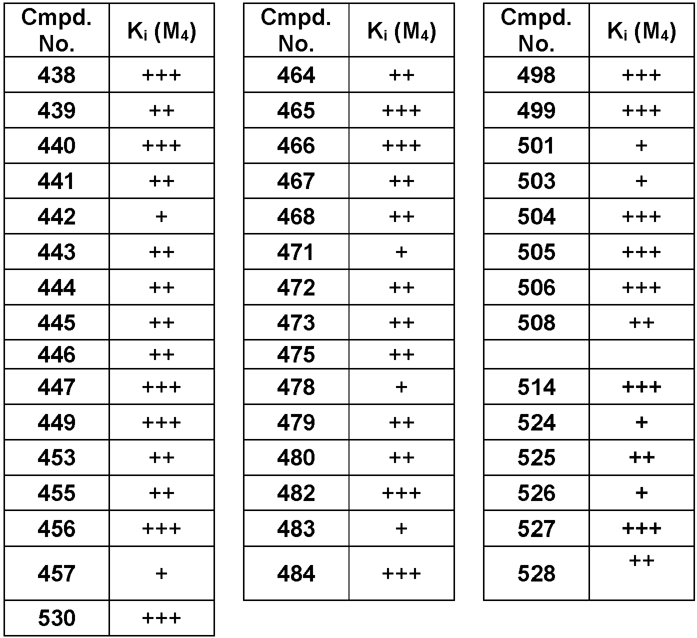

### Example 20: Functional assay.

Functional antagonism of acetylcholine responses was evaluated using a fluorescence-based functional calcium assay. Acetylcholine binding to the muscarinic receptors activates G-proteins. Human muscarinic 4 receptor (CHRM4) was stably expressed in CHO-K1 cells and a promiscuous Gα16 construct is co-transfected. This cell line was commercially available through PerkinElmer (product number ES-213-A). Following ligand binding, activation of the Gα16 subunit induces the release of calcium from the endoplasmic reticulum. Prior to ligand screening, the receptor-expressing cells were loaded with a fluorescent calcium indicator, FLIPR Calcium 6 (Molecular Devices). Antagonist activity of the compounds was determined as the EC₅₀ for inhibition of the acetylcholine response. The assay buffer used was a 1:1 solution of buffer (1X Hank's balanced salt solution plus 20 mM HEPES buffer, pH 7.4) and cell medium (Ham's F-12, 10% FBS, 0.4 mg/mL Geneticin, 0.25 mg/mL Zeocin). The day before the assay, 4 X 103 cells per well were seeded into an assay plate in 25 µL of medium and allowed to incubate overnight at 37°C and 5% CO2. The following day, 25 µL of Calcium 6 dye was added to each well and incubated for two additional hours at 37°C and 5% CO₂. The test compound (a dose-response of eleven concentrations ranging from 10 µM to 100 pM) was added to the cells to a final DMSO concentration of 0.56% v/v. One hour later, acetylcholine to a final concentration of 100 nM was added by the instrument and calcium flux-dependent fluorescence measured in real time. The concentration of acetylcholine used was that which stimulates 80% of the maximal response. IC₅₀ values for each compound listed in **TABLE 12** are as follows: (1) "+" means the compound had a IC₅₀ value of less than 1 µM (1,000 nM) but greater or equal to 100 nM; (2) "++" means the compound had a IC₅₀ value of less than 100 nM but greater or equal to 10 nM; and (3) "+++" means that the compound had a IC₅₀ value against the M₄ receptor of less than 10 nM.

**TABLE 12**

| **Cpd. No.** | **IC₅₀ (M₄)** |
|---|---|
| **96** | **+++** |
| **106** | **+++** |
| **141** | **+++** |
| **147** | **+++** |

### Example 21: Electrophysiology Assay.

Adult (>8 weeks) female Lister hooded rats (Harlan, UK) are killed by decapitation and the brain is removed and placed into ice-cold oxygenated sucrose Krebs' medium containing (mM): sucrose (202), KCI (2), KH₂PO₄ (1.25), MgSO₄ (10), CaCl₂ (0.5), NaHCO₃ (26), glucose (10). The brain is hemisected along the midline and 300 µM parasagittal slices are prepared with an oscillating microtome (Integraslice; Campden Instruments Ltd., Loughborough, UK). Slices are then transferred to a recovery chamber at room temperature containing oxygenated Krebs' solution (mM): NaCl (124), KCI (2), KH₂PO₄ (1.25), MgSO₄ (1), CaCl₂ (2), NaHCO₃ (26), glucose (10). Following at least 1 hour of recovery, individual slices are transferred to an interface recording chamber where they are perfused with Krebs' solution (33 °C). Extracellular field potential recordings are made with an Axoprobe 1A amplifier (Axon Instruments Ltd., USA) via a Krebs'-filled glass micropipette (resistance 2-5 MΩ) positioned in the stratum radiatum of the CA1, digitized (10kHz) via a CED1401 interface and stored on a computer with Spike2 software (Cambridge Electronic Design Ltd., Cambridge, UK). Field excitatory postsynaptic potential (fEPSP) responses are evoked (pair of 0.02ms pulses, separated by 40 ms; applied every 10s; adjusted to approximately 60% of the maximal spike-free response) by a bipolar stimulating electrode positioned in the stratum radiatum near the CA3-CA1 border.

The cholinergic agonist carbachol (aza-acetylcholine, resistant to degradation by acetylcholinesterase) is used to stimulate muscarinic receptors. The M1 muscarinic receptor is blocked using 5 µM VU0255035, a selective M1 antagonist. The resulting inhibitory signal is primarily M₄-mediated, based on its sensitivity to the M₄ activator VU010010. The effect of M₄ antagonists on this M₄-mediated inhibition of fEPSPs is measured by adding compound 20 minutes prior to application of carbachol.

### Example 22: 6-OHDA Surgical Lesion and Behavioral Testing Procedures.

6-OHDA Lesion protocol: Male Sprague-Dawley rats are anesthetized with isoflurane and placed into the stereotaxic frame. Thirty minutes prior the injection of 6-OHDA, rats received desipramine (15 mg/kg, i.p.) to prevent the entry of the toxin into the noradrenergic cells. A unilateral lesion is induced by injections of 6-OHDA (8 µg/4 µL/site/rat; flow rate 1 µL/min; dissolved in 0.9% NaCl with 0.02% ascorbic acid) or vehicle into the left and right medial forebrain bundle at the following coordinates: AP -4.4. mm; L ±1.2 mm; V -7.8 mm relative to Bregma (Paxinos and Watson, 2007). The rats are allowed to recover for 14 days and are then tested for locomotor activity induced by novelty (placing the rat in a new cage, 30 min) and for contraversive (contralateral) rotational behavior induced by apomorphine (0.2 mg/kg, s.c.).

Experimental animal selection criteria: Only the rats with activity higher than 5 turns/min. following apomorphine treatment are enrolled in the study; rats not fulfilling the criteria are excluded from the study (typically 20%). Turning activity is then recorded for each group once per week for four consecutive weeks.

### Example 23: Haloperidol-induced Catalepsy.

Young adult male, Sprague-Dawley (SD) rats (175-200 grams) from Envigo; Indianapolis, IN are used. Upon arrival, rats are housed 3 per cage in ventilated cages and acclimated for at least 7 days prior to testing. Animals are maintained at a 12/12 h light/dark cycle (lights on at 06.00) with room temperature maintained at 22 ± 1°C with the relative humidity maintained at approximately 50%. Food and water are provided ad libitum. Animals are randomly assigned across the treatment groups. The experiments are conducted during the animal's light cycle phase.

The bar test is used to assess catalepsy. The front paws of the rats are placed on a horizontal metal bar raised 6" above a Plexiglas platform and time is recorded for up to 60 seconds per trial. The test ends when the animal's front paws returned to the platform or after 60 seconds. The test is repeated three times and the average of the three trials is reported as the intensity index of catalepsy. Rats are brought to the experimental room for at least 1 hr to acclimate to the experimental room conditions prior to testing. Rats are injected vehicle or compound and catalepsy is assessed 30 and 60 min. following haloperidol injection. Data is analyzed by analysis of variance (ANOVA) followed by Dunnett's post-hoc comparisons.

Various modifications of the embodiments, in addition to those described herein, will be apparent to those skilled in the art from the foregoing description.

## Claims

1. A compound selected from Formula (la): 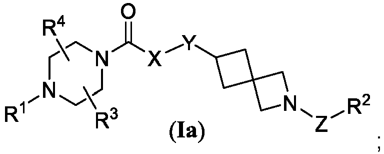 or a pharmaceutically acceptable salt thereof:
wherein:
X is O or NH;
Y is CH₂ or absent;
Z is C₁-C₄ alkylene or absent;
R¹ is C₆-C₁₀ aryl or 5-10 membered heteroaryl, each optionally substituted with one or more groups selected from: C₁-C₆ alkoxy, C₁-C₆ alkoxycarbonyl, C₁-C₆ alkyl, C₁-C₆ alkylamino, C₁-C₆ alkylcarbonyl, C₁-C₆ alkylcarbamoyl, C₁-C₆ alkylsulfanyl, C₂-C₆ dialkylamino, C₂-C₆ dialkyl carbamoyl, C₁-C₆ sulfinyl, C₃-C₁₀ cycloalkyl, C₁-C₆ haloalkoxy, C₁-C₆ haloalkyl, amino, carbamoyl, cyano, halogen, and nitro;
R² is selected from: C₆-C₁₀ aryl, C₁-C₆ alkyl, C₃-C₇ cycloalkyl, C₅-C₈ bicycloalkanyl, C₆-C₈ bicycloalkenyl, 5-10 membered heteroaryl, and heterocyclyl; each optionally substituted with one or more groups selected from: C₁-C₄ alkyl, C₁-C₆ alkoxy, C₁-C₆ haloalkoxy, C₁-C₆ sulfonyl, carbamoyl, cyano, and halogen; and
a) R³ and R⁴ are each independently selected from H and C₁-C₄ alkyl; or
b) R³ and R⁴ taken together form a bridging group selected from: CH₂, CH₂-CH₂, and CH₂-O-CH₂; and
c) R³ and R⁴ are bonded to different ethylene groups of the piperazine ring; or a compound selected from the group consisting of:
(2R,6S)-N-[2-(cyclohex-1-en-1-ylmethyl)-2-azaspiro[3.3]heptan-6-yl]-2,6-dimethyl-4-[5-(trifluoromethyl)pyrimidin-2-yl]piperazine-1-carboxamide;
(2R,6S)-2,6-dimethyl-N-{2-[(4-nitrophenyl)methyl]-2-azaspiro[3.3]heptan-6-yl}-4-[5-(trifluoromethyl)pyrimidin-2-yl]piperazine-1-carboxamide;
(2R,6S)-N-{2-[(4-hydroxyphenyl)methyl]-2-azaspiro[3.3]heptan-6-yl}-2,6-dimethyl-4-[5-(trifluoromethyl)pyrimidin-2-yl]piperazine-1-carboxamide;
methyl 4-[(6-{[(2R,6S)-2,6-dimethyl-4-[5-(trifluoromethyl)pyrimidin-2-yl]piperazine-1-carbonyl]amino}-2-azaspiro[3.3]heptan-2-yl)methyl]benzoate;
(2R,6S)-N-{2-[(4-acetamidophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-(2-{[4-(trifluoromethyl)phenyl]methyl}-2-azaspiro[3.3]heptan-6-yl)-4-[5-(trifluoromethyl)pyrimidin-2-yl]piperazine-1-carboxamide;
(2R,6S)-N-{2-[(4-carbamimidoylphenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-[2-(adamantan-1-ylmethyl)-2-azaspiro[3.3]heptan-6-yl]-2,6-dimethyl-4-[5-(trifluoromethyl)pyrimidin-2-yl]piperazine-1-carboxamide;
(2R,6S)-N-(2-{[4-(dimethylcarbamoyl)phenyl]methyl}-2-azaspiro[3.3]heptan-6-yl)-2,6-dimethyl-4-[5-(trifluoromethyl)pyrimidin-2-yl]piperazine-1-carboxamide;
2-benzyl-6-(difluoromethyl)-2-azaspiro[3.3]heptan-6-yl (2R,6S)-2,6-dimethyl-4-[5-(trifluoromethyl)pyrimidin-2-yl]piperazine-1-carboxylate;
2-benzyl-6-(difluoromethyl)-2-azaspiro[3.3]heptan-6-yl (2R,6S)-2,6-dimethyl-4-[5-(trifluoromethyl)pyrazin-2-yl]piperazine-1-carboxylate;
2-benzyl-6-(difluoromethyl)-2-azaspiro[3.3]heptan-6-yl (2R,6S)-2,6-dimethyl-4-[6-(trifluoromethyl)pyridazin-3-yl]piperazine-1-carboxylate;
2-benzyl-6-(difluoromethyl)-2-azaspiro[3.3]heptan-6-yl (2R,6R)-2,6-dimethyl-4-[5-(trifluoromethyl)pyrimidin-2-yl]piperazine-1-carboxylate;
2-benzyl-6-(difluoromethyl)-2-azaspiro[3.3]heptan-6-yl (2R,6R)-2,6-dimethyl-4-[5-(trifluoromethyl)pyrazin-2-yl]piperazine-1-carboxylate;
2-benzyl-6-(difluoromethyl)-2-azaspiro[3.3]heptan-6-yl (2R,5S)-2,5-dimethyl-4-[5-(trifluoromethyl)pyrimidin-2-yl]piperazine-1-carboxylate;
2-benzyl-6-(difluoromethyl)-2-azaspiro[3.3]heptan-6-yl (2R,5S)-2,5-dimethyl-4-[5-(trifluoromethyl)pyrazin-2-yl]piperazine-1-carboxylate;
2-benzyl-6-(difluoromethyl)-2-azaspiro[3.3]heptan-6-yl (2R)-4-(5-cyanopyrimidin-2-yl)-2-methylpiperazine-1-carboxylate;
2-benzyl-6-(difluoromethyl)-2-azaspiro[3.3]heptan-6-yl (2R)-2-methyl-4-[5-(trifluoromethyl)pyrimidin-2-yl]piperazine-1-carboxylate;
2-benzyl-6-(difluoromethyl)-2-azaspiro[3.3]heptan-6-yl (2R)-2-methyl-4-[5-(trifluoromethyl)pyrazin-2-yl]piperazine-1-carboxylate;
2-benzyl-6-methyl-2-azaspiro[3.3]heptan-6-yl (2R,6S)-4-(5-cyanopyrimidin-2-yl)-2,6-dimethylpiperazine-1-carboxylate;
2-benzyl-6-methyl-2-azaspiro[3.3]heptan-6-yl (2R,6S)-2,6-dimethyl-4-[5-(trifluoromethyl)pyrimidin-2-yl]piperazine-1-carboxylate;
2-benzyl-6-methyl-2-azaspiro[3.3]heptan-6-yl (2R,6S)-2,6-dimethyl-4-[5-(trifluoromethyl)pyrazin-2-yl]piperazine-1-carboxylate;
2-benzyl-6-methyl-2-azaspiro[3.3]heptan-6-yl (2R,6R)-4-(5-cyanopyrimidin-2-yl)-2,6-dimethylpiperazine-1-carboxylate;
2-benzyl-6-methyl-2-azaspiro[3.3]heptan-6-yl (2R,6R)-2,6-dimethyl-4-[5-(trifluoromethyl)pyrimidin-2-yl]piperazine-1-carboxylate;
2-benzyl-6-methyl-2-azaspiro[3.3]heptan-6-yl (2R,6R)-2,6-dimethyl-4-[5-(trifluoromethyl)pyrazin-2-yl]piperazine-1-carboxylate;
2-benzyl-6-methyl-2-azaspiro[3.3]heptan-6-yl (2R,5S)-4-(5-cyanopyrimidin-2-yl)-2,5-dimethylpiperazine-1-carboxylate;
2-benzyl-6-methyl-2-azaspiro[3.3]heptan-6-yl (2R,5S)-2,5-dimethyl-4-[5-(trifluoromethyl)pyrimidin-2-yl]piperazine-1-carboxylate;
2-benzyl-6-methyl-2-azaspiro[3.3]heptan-6-yl (2R,5S)-2,5-dimethyl-4-[5-(trifluoromethyl)pyrazin-2-yl]piperazine-1-carboxylate;
2-benzyl-2-azaspiro[3.3]heptan-6-yl (2R,6S)-4-(5-ethynylpyrimidin-2-yl)-2,6-dimethylpiperazine-1-carboxylate;
2-benzyl-2-azaspiro[3.3]heptan-6-yl (2R,6S)-4-[5-(dimethylphosphoryl)pyridin-2-yl]-2,6-dimethylpiperazine-1-carboxylate; and
2-benzyl-2-azaspiro[3.3]heptan-6-yl (2R,6S)-4-[5-(dimethylphosphoryl) pyrimidin-2-yl]-2,6-dimethylpiperazine-1-carboxylate;
or a pharmaceutically acceptable salt thereof.

2. The compound according to claim 1, or a pharmaceutically acceptable salt thereof, wherein:
R¹ is selected from: 1,3,4-thiadiazol-2-yl, phenyl, pyrazin-2-yl, pyridazin-3-yl, pyridin-2-yl, pyridin-3-yl, pyrimidin-2-yl, and quinoxalin-2-yl;
each optionally substituted with one or more groups selected from: acetyl, bromo, chloro, cyano, difluoromethoxy, difluoromethyl, fluoro, methoxy, methoxycarbonyl, methyl, methylsulfanyl, nitro, trifluoromethoxy, and trifluoromethyl.

3. The compound according to claim 1, or a pharmaceutically acceptable salt thereof, wherein:
R¹ is selected from: 2-cyano-4-(trifluoromethyl)phenyl, 3-(trifluoromethyl)pyridin-2-yl, 3,4,5-trifluorophenyl, 3,6-dimethylpyrazin-2-yl, 3-cyano-4-(trifluoromethoxy)phenyl, 3-cyano-4-fluorophenyl, 3-cyano-5-fluorophenyl, 4-(trifluoromethoxy)phenyl, 4-(trifluoromethyl)phenyl, 4-cyano-2,5-difluorophenyl, 4-cyano-2-fluorophenyl, 4-cyano-6-(trifluoromethyl)pyridin-3-yl, 4-methylpyrimidin-2-yl, 5-(difluoromethoxy)pyrimidin-2-yl, 5-(difluoromethyl)pyrazin-2-yl, 5-(methoxycarbonyl)-4-(trifluoromethyl)pyrimidin-2-yl, 5-(trifluoromethoxy)pyrazin-2-yl, 5-(trifluoromethyl)-1,3,4-thiadiazol-2-yl, 5-(trifluoromethyl)pyrazin-2-yl, 5-(trifluoromethyl)pyridin-2-yl, 5-(trifluoromethyl)pyrimidin-2-yl, 5-acetylpyrazin-2-yl, 5-bromo-4-(methylsulfanyl)pyrimidin-2-yl, 5-chloropyrazin-2-yl, 5-chloropyrimidin-2-yl, 5-cyano-3-methylpyrazin-2-yl, 5-cyanopyrazin-2-yl, 5-cyanopyridin-2-yl, 5-cyanopyrimidin-2-yl, 5-fluoropyrimidin-2-yl, 5-methoxypyrimidin-2-yl, 5-nitropyridin-2-yl, 6-(trifluoromethyl)pyrazin-2-yl, 6-(trifluoromethyl)pyridazin-3-yl, 6-(trifluoromethyl)pyridin-3-yl, 6-cyanopyrazin-2-yl, 6-fluoroquinoxalin-2-yl, 6-methoxy-3-nitropyridin-2-yl, 6-methoxypyrazin-2-yl, 6-methoxyquinoxalin-2-yl, and quinoxalin-2-yl.

4. The compound according to any one of claims 1-3, or a pharmaceutically acceptable salt thereof, wherein:
R² is selected from: 1H-indolyl, 2,2-dimethylpropyl, 2-methylpropyl, 3,3-dimethylbutyl, bicyclo[2.2.1]heptenyl, butyl, cyclohexyl, cyclopropyl, oxanyl, and phenyl;
each optionally substituted with one or more groups selected from: C₁-C₄ alkyl, C₁-C₆ alkoxy, carbamoyl, cyano, and halogen.

5. The compound according to any one of claims 1-4, wherein the compound is of Formula (**IIg**): 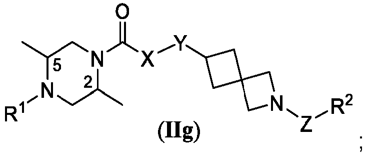 or a pharmaceutically acceptable salt thereof.

6. The compound according to any one of claims 1-4, wherein the compound is of Formula (**IIi**): 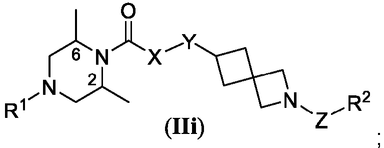 or a pharmaceutically acceptable salt thereof.

7. The compound according to any one of claims 1-4, wherein the compound is of Formula (Ilk): 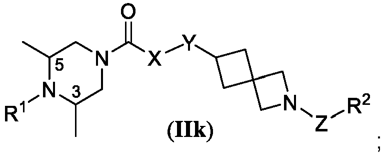 or a pharmaceutically acceptable salt thereof.

8. The compound according to claim 6, or a pharmaceutically acceptable salt thereof, wherein:
the stereochemistry in Formula (**IIi**) for the C(2) carbon is (R) and the C(6) carbon is (R).

9. The compound according to claim 6, or a pharmaceutically acceptable salt thereof, wherein:
the stereochemistry in Formula (**IIi**) for the C(2) carbon is (S) and the C(6) carbon is (S).

10. The compound according to claim 6, or a pharmaceutically acceptable salt thereof, wherein:
the stereochemistry in Formula (**IIi**) for the C(2) carbon is (R) and the C(6) carbon is (S).

11. The compound according to claim 1, which is selected from the following compounds, or a pharmaceutically acceptable salt thereof:
(2R,5S)-N-{2-benzyl-2-azaspiro[3.3]heptan-6-yl}-2,5-dimethyl-4-[5-(trifluoromethyl)pyrimidin-2-yl]piperazine-1-carboxamide;
(2R)-N-{2-benzyl-2-azaspiro[3.3]heptan-6-yl}-4-(5-cyanopyrimidin-2-yl)-2-methylpiperazine-1-carboxamide;
(2R,6R)-N-{2-benzyl-2-azaspiro[3.3]heptan-6-yl}-4-(5-cyanopyrimidin-2-yl)-2,6-dimethylpiperazine-1-carboxamide;
(2S,6S)-N-{2-benzyl-2-azaspiro[3.3]heptan-6-yl}-4-(5-cyanopyrimidin-2-yl)-2,6-dimethylpiperazine-1-carboxamide;
(2S)-N-{2-benzyl-2-azaspiro[3.3]heptan-6-yl}-2-methyl-4-[5-(trifluoromethyl)pyrimidin-2-yl]piperazine-1-carboxamide;
(3S)-N-{2-benzyl-2-azaspiro[3.3]heptan-6-yl}-3-methyl-4-[5-(trifluoromethyl)pyrimidin-2-yl]piperazine-1-carboxamide;
(2R)-N-{2-benzyl-2-azaspiro[3.3]heptan-6-yl}-2-methyl-4-[5-(trifluoromethyl)pyrimidin-2-yl]piperazine-1-carboxamide;
N-{2-benzyl-2-azaspiro[3.3]heptan-6-yl}-4-(4-cyano-2-fluorophenyl)piperazine-1-carboxamide;
N-{2-benzyl-2-azaspiro[3.3]heptan-6-yl}-4-(3,4,5-trifluorophenyl)piperazine-1-carboxamide;
N-{2-benzyl-2-azaspiro[3.3]heptan-6-yl}-4-[4-(trifluoromethoxy)phenyl]piperazine-1-carboxamide;
(2R,6R)-N-{2-benzyl-2-azaspiro[3.3]heptan-6-yl}-2,6-dimethyl-4-(3,4,5-trifluorophenyl)piperazine-1-carboxamide;
(2R)-N-{2-benzyl-2-azaspiro[3.3]heptan-6-yl}-4-(3-cyano-4-fluorophenyl)-2-methylpiperazine-1-carboxamide;
(2R)-N-{2-benzyl-2-azaspiro[3.3]heptan-6-yl}-4-[3-cyano-4-(trifluoromethoxy)phenyl]-2-methylpiperazine-1-carboxamide;
(2R,6S)-N-{2-benzyl-2-azaspiro[3.3]heptan-6-yl}-4-(5-chloropyrimidin-2-yl)-2,6-dimethylpiperazine-1-carboxamide;
(1S,45)-N-{2-benzyl-2-azaspiro[3.3]heptan-6-yl}-5-(5-cyanopyrimidin-2-yl)-2,5-diazabicyclo[2.2.1]heptane-2-carboxamide;
N-{2-benzyl-2-azaspiro[3.3]heptan-6-yl}-3-(5-cyanopyrimidin-2-yl)-3,6-diazabicyclo[3.1.1]heptane-6-carboxamide;
N-{2-benzyl-2-azaspiro[3.3]heptan-6-yl}-5-(5-cyanopyrimidin-2-yl)-2,5-diazabicyclo[2.2.2]octane-2-carboxamide;
(2R,6R)-N-{2-benzyl-2-azaspiro[3.3]heptan-6-yl}-4-(5-chloropyrimidin-2-yl)-2,6-dimethylpiperazine-1-carboxamide;
(2R,6R)-N-{2-benzyl-2-azaspiro[3.3]heptan-6-yl}-2,6-dimethyl-4-[6-(trifluoromethyl)pyridazin-3-yl]piperazine-1-carboxamide;
(2R,6R)-N-{2-benzyl-2-azaspiro[3.3]heptan-6-yl}-4-(5-cyanopyrazin-2-yl)-2,6-dimethylpiperazine-1-carboxamide;
(2R,6R)-N-{2-benzyl-2-azaspiro[3.3]heptan-6-yl}-4-[5-(difluoromethoxy)pyrimidin-2-yl]-2,6-dimethylpiperazine-1-carboxamide;
(2R)-N-{2-benzyl-2-azaspiro[3.3]heptan-6-yl}-2-methyl-4-[5-(trifluoromethyl)pyrazin-2-yl]piperazine-1-carboxamide;
N-{2-benzyl-2-azaspiro[3.3]heptan-6-yl}-3-[5-(trifluoromethyl)pyrimidin-2-yl]-3,6-diazabicyclo[3.1.1]heptane-6-carboxamide;
N-{2-benzyl-2-azaspiro[3.3]heptan-6-yl}-3-[5-(trifluoromethyl)pyrazin-2-yl]-3,6-diazabicyclo[3.1.1]heptane-6-carboxamide;
(2S)-N-{2-benzyl-2-azaspiro[3.3]heptan-6-yl}-4-(5-cyanopyrimidin-2-yl)-2-methylpiperazine-1-carboxamide;
(2R,5S)-N-{2-benzyl-2-azaspiro[3.3]heptan-6-yl}-4-(5-cyanopyrimidin-2-yl)-2,5-dimethylpiperazine-1-carboxamide;
(2S,5R)-N-{2-benzyl-2-azaspiro[3.3]heptan-6-yl}-4-(5-cyanopyrimidin-2-yl)-2,5-dimethylpiperazine-1-carboxamide;
(2R,5R)-N-{2-benzyl-2-azaspiro[3.3]heptan-6-yl}-4-(5-cyanopyrimidin-2-yl)-2,5-dimethylpiperazine-1-carboxamide;
N-{2-benzyl-2-azaspiro[3.3]heptan-6-yl}-3-[6-(trifluoromethyl)pyridazin-3-yl]-3,6-diazabicyclo[3.1.1]heptane-6-carboxamide;
(1S,4S)-N-{2-benzyl-2-azaspiro[3.3]heptan-6-yl}-5-[5-(trifluoromethyl)pyrazin-2-yl]-2,5-diazabicyclo[2.2.1]heptane-2-carboxamide;
(1S,4S)-N-{2-benzyl-2-azaspiro[3.3]heptan-6-yl}-5-[5-(trifluoromethyl)pyrimidin-2-yl]-2,5-diazabicyclo[2.2.1]heptane-2-carboxamide;
N-{2-benzyl-2-azaspiro[3.3]heptan-6-yl}-4-[5-(trifluoromethyl)pyrimidin-2-yl]piperazine-1-carboxamide;
(3R,5R)-N-{2-benzyl-2-azaspiro[3.3]heptan-6-yl}-4-(5-cyanopyrimidin-2-yl)-3,5-dimethylpiperazine-1-carboxamide;
(3S,5S)-N-{2-benzyl-2-azaspiro[3.3]heptan-6-yl}-4-(5-cyanopyrimidin-2-yl)-3,5-dimethylpiperazine-1-carboxamide;
(2R,5S)-N-{2-benzyl-2-azaspiro[3.3]heptan-6-yl}-2,5-dimethyl-4-[5-(trifluoromethyl)pyrazin-2-yl]piperazine-1-carboxamide;
(2S,5R)-N-{2-benzyl-2-azaspiro[3.3]heptan-6-yl}-2,5-dimethyl-4-[5-(trifluoromethyl)pyrimidin-2-yl]piperazine-1-carboxamide;
(2S,5R)-N-{2-benzyl-2-azaspiro[3.3]heptan-6-yl}-2,5-dimethyl-4-[5-(trifluoromethyl)pyrazin-2-yl]piperazine-1-carboxamide;
(2R,5R)-N-{2-benzyl-2-azaspiro[3.3]heptan-6-yl}-2,5-dimethyl-4-[5-(trifluoromethyl)pyrimidin-2-yl]piperazine-1-carboxamide;
(2R,5R)-N-{2-benzyl-2-azaspiro[3.3]heptan-6-yl}-2,5-dimethyl-4-[5-(trifluoromethyl)pyrazin-2-yl]piperazine-1-carboxamide;
(2S,5S)-N-{2-benzyl-2-azaspiro[3.3]heptan-6-yl}-4-(5-cyanopyrimidin-2-yl)-2,5-dimethylpiperazine-1-carboxamide;
(3S)-N-{2-benzyl-2-azaspiro[3.3]heptan-6-yl}-4-(5-cyanopyrimidin-2-yl)-3-methylpiperazine-1-carboxamide;
(3R)-N-{2-benzyl-2-azaspiro[3.3]heptan-6-yl}-4-(5-cyanopyrimidin-2-yl)-3-methylpiperazine-1-carboxamide;
N-{2-benzyl-2-azaspiro[3.3]heptan-6-yl}-4-[5-(trifluoromethyl)pyridin-2-yl]piperazine-1-carboxamide;
(2R)-N-{2-benzyl-2-azaspiro[3.3]heptan-6-yl}-2-methyl-4-(3,4,5-trifluorophenyl)piperazine-1-carboxamide;
(2R,6S)-N-{2-benzyl-2-azaspiro[3.3]heptan-6-yl}-2,6-dimethyl-4-(3,4,5-trifluorophenyl)piperazine-1-carboxamide;
(2R,6R)-N-{2-benzyl-2-azaspiro[3.3]heptan-6-yl}-4-(6-cyanopyrazin-2-yl)-2,6-dimethylpiperazine-1-carboxamide;
(2R,6S)-N-{2-benzyl-2-azaspiro[3.3]heptan-6-yl}-2,6-dimethyl-4-[4-(trifluoromethyl)phenyl]piperazine-1-carboxamide;
(2R,6R)-N-{2-benzyl-2-azaspiro[3.3]heptan-6-yl}-2,6-dimethyl-4-[6-(trifluoromethyl)pyrazin-2-yl]piperazine-1-carboxamide;
(1R,4R)-N-{2-benzyl-2-azaspiro[3.3]heptan-6-yl}-5-(5-cyanopyrimidin-2-yl)-2,5-diazabicyclo[2.2.2]octane-2-carboxamide;
(2R,6S)-N-{2-benzyl-2-azaspiro[3.3]heptan-6-yl}-4-(5-cyanopyridin-2-yl)-2,6-dimethylpiperazine-1-carboxamide;
(2R,6R)-N-{2-benzyl-2-azaspiro[3.3]heptan-6-yl}-2,6-dimethyl-4-[4-(trifluoromethyl)phenyl]piperazine-1-carboxamide;
(1S,4S)-N-{2-benzyl-2-azaspiro[3.3]heptan-6-yl}-5-(5-cyanopyrimidin-2-yl)-2,5-diazabicyclo[2.2.2]octane-2-carboxamide;
(2R,6R)-N-{2-benzyl-2-azaspiro[3.3]heptan-6-yl}-4-[5-(difluoromethyl)pyrazin-2-yl]-2,6-dimethylpiperazine-1-carboxamide;
N-{2-benzyl-2-azaspiro[3.3]heptan-6-yl}-3-(5-cyanopyrimidin-2-yl)-3,8-diazabicyclo[3.2.1]octane-8-carboxamide;
(2R,6R)-N-{2-benzyl-2-azaspiro[3.3]heptan-6-yl}-4-(5-methoxypyrimidin-2-yl)-2,6-dimethylpiperazine-1-carboxamide;
(2R,6S)-N-{2-benzyl-2-azaspiro[3.3]heptan-6-yl}-2,6-dimethyl-4-[6-(trifluoromethyl)pyrazin-2-yl]piperazine-1-carboxamide;
(2R,6R)-N-{2-benzyl-2-azaspiro[3.3]heptan-6-yl}-4-(5-chloropyrazin-2-yl)-2,6-dimethylpiperazine-1-carboxamide;
(1R,4R)-N-{2-benzyl-2-azaspiro[3.3]heptan-6-yl}-5-[5-(trifluoromethyl)pyrimidin-2-yl]-2,5-diazabicyclo[2.2.1]heptane-2-carboxamide;
(1R,4R)-N-{2-benzyl-2-azaspiro[3.3]heptan-6-yl}-5-[5-(trifluoromethyl)pyrazin-2-yl]-2,5-diazabicyclo[2.2.1]heptane-2-carboxamide;
(2R,6S)-N-{2-benzyl-2-azaspiro[3.3]heptan-6-yl}-4-[4-methoxy-5-(trifluoromethyl)pyrimidin-2-yl]-2,6-dimethylpiperazine-1-carboxamide;
(2R,6S)-N-{2-benzyl-2-azaspiro[3.3]heptan-6-yl}-4-(5-cyanopyrazin-2-yl)-2,6-dimethylpiperazine-1-carboxamide;
(2R,6S)-N-{2-benzyl-2-azaspiro[3.3]heptan-6-yl}-2,6-dimethyl-4-[5-(trifluoromethyl)pyrazin-2-yl]piperazine-1-carboxamide;
(2R,6R)-N-({2-benzyl-2-azaspiro[3.3]heptan-6-yl}methyl)-2,6-dimethyl-4-[5-(trifluoromethyl)pyrazin-2-yl]piperazine-1-carboxamide;
(2R,6R)-N-{2-benzyl-2-azaspiro[3.3]heptan-6-yl}-2,6-dimethyl-4-[5-(trifluoromethyl)pyrazin-2-yl]piperazine-1-carboxamide;
(2R,6R)-N-{2-benzyl-2-azaspiro[3.3]heptan-6-yl}-2,6-dimethyl-4-[5-(trifluoromethyl)pyrimidin-2-yl]piperazine-1-carboxamide;
(2R,6S)-N-{2-benzyl-2-azaspiro[3.3]heptan-6-yl}-4-(5-cyanopyrimidin-2-yl)-2,6-dimethylpiperazine-1-carboxamide;
(2R,6S)-N-({2-benzyl-2-azaspiro[3.3]heptan-6-yl}methyl)-2,6-dimethyl-4-[5-(trifluoromethyl)pyrazin-2-yl]piperazine-1-carboxamide;
(2R,6R)-N-({2-benzyl-2-azaspiro[3.3]heptan-6-yl}methyl)-2,6-dimethyl-4-[5-(trifluoromethyl)pyrimidin-2-yl]piperazine-1-carboxamide;
(2R,6R)-N-[2-(3,3-dimethylbutyl)-2-azaspiro[3.3]heptan-6-yl]-2,6-dimethyl-4-[5-(trifluoromethyl)pyrimidin-2-yl]piperazine-1-carboxamide;
(2R,6S)-4-(5-cyanopyrimidin-2-yl)-N-[2-(3,3-dimethylbutyl)-2-azaspiro[3.3]heptan-6-yl]-2,6-dimethylpiperazine-1-carboxamide;
(2R,6S)-N-{[2-(3,3-dimethylbutyl)-2-azaspiro[3.3]heptan-6-yl]methyl}-2,6-dimethyl-4-[5-(trifluoromethyl)pyrazin-2-yl]piperazine-1-carboxamide;
(2R,6R)-N-{[2-(3,3-dimethylbutyl)-2-azaspiro[3.3]heptan-6-yl]methyl}-2,6-dimethyl-4-[5-(trifluoromethyl)pyrimidin-2-yl]piperazine-1-carboxamide;
(2R,6R)-N-[2-(2,2-dimethylpropyl)-2-azaspiro[3.3]heptan-6-yl]-2,6-dimethyl-4-[5-(trifluoromethyl)pyrimidin-2-yl]piperazine-1-carboxamide;
(2R,6S)-4-(5-cyanopyrimidin-2-yl)-N-[2-(2,2-dimethylpropyl)-2-azaspiro[3.3]heptan-6-yl]-2,6-dimethylpiperazine-1-carboxamide;
(2R,6S)-N-{[2-(2,2-dimethylpropyl)-2-azaspiro[3.3]heptan-6-yl]methyl}-2,6-dimethyl-4-[5-(trifluoromethyl)pyrazin-2-yl]piperazine-1-carboxamide;
(2R,6R)-N-{[2-(2,2-dimethylpropyl)-2-azaspiro[3.3]heptan-6-yl]methyl}-2,6-dimethyl-4-[5-(trifluoromethyl)pyrimidin-2-yl]piperazine-1-carboxamide;
(2R,6S)-N-[2-(cyclopropylmethyl)-2-azaspiro[3.3]heptan-6-yl]-2,6-dimethyl-4-[5-(trifluoromethyl)pyrimidin-2-yl]piperazine-1-carboxamide;
(2R,6S)-2,6-dimethyl-N-[2-(2-methylpropyl)-2-azaspiro[3.3]heptan-6-yl]-4-[5-(trifluoromethyl)pyrimidin-2-yl]piperazine-1-carboxamide;
(2R,6S)-N-{2-[(2,2-dimethylcyclopropyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-[2-(cyclohexylmethyl)-2-azaspiro[3.3]heptan-6-yl]-2,6-dimethyl-4-[5-(trifluoromethyl)pyrimidin-2-yl]piperazine-1-carboxamide;
(2R,6S)-2,6-dimethyl-N-[2-(oxan-4-ylmethyl)-2-azaspiro[3.3]heptan-6-yl]-4-[5-(trifluoromethyl)pyrimidin-2-yl]piperazine-1-carboxamide;
(2R,6S)-N-(2-{bicyclo[2.2.1]hept-5-en-2-ylmethyl}-2-azaspiro[3.3]heptan-6-yl)-2,6-dimethyl-4-[5-(trifluoromethyl)pyrimidin-2-yl]piperazine-1-carboxamide;
(2R,6S)-N-{2-[(2-fluorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(3-fluorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(4-fluorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-[2-(4,4,4-trifluorobutyl)-2-azaspiro[3.3]heptan-6-yl]-4-[5-(trifluoromethyl)pyrimidin-2-yl]piperazine-1-carboxamide;
(2R,6S)-N-{2-[(2-cyanophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(4-cyanophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(4-methoxyphenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(2,6-difluorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(3,4-difluorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-[2-(1H-indol-5-ylmethyl)-2-azaspiro[3.3]heptan-6-yl]-2,6-dimethyl-4-[5-(trifluoromethyl)pyrimidin-2-yl]piperazine-1-carboxamide;
(2R,6S)-N-{2-[(3-cyano-4-fluorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(4-carbamoylphenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(3-carbamoylphenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-benzyl-2-azaspiro[3.3]heptan-6-yl}-2,6-dimethyl-4-[5-(trifluoromethyl)pyrimidin-2-yl]piperazine-1-carboxamide;
(2R,6S)-N-({2-benzyl-2-azaspiro[3.3]heptan-6-yl}methyl)-2,6-dimethyl-4-[5-(trifluoromethyl)pyrimidin-2-yl]piperazine-1-carboxamide;
(2R,6S)-N-[2-(3,3-dimethylbutyl)-2-azaspiro[3.3]heptan-6-yl]-2,6-dimethyl-4-[5-(trifluoromethyl)pyrimidin-2-yl]piperazine-1-carboxamide;
(2R,6S)-N-{[2-(3,3-dimethylbutyl)-2-azaspiro[3.3]heptan-6-yl]methyl}-2,6-dimethyl-4-[5-(trifluoromethyl)pyrimidin-2-yl]piperazine-1-carboxamide;
(2R,6S)-N-[2-(2,2-dimethylpropyl)-2-azaspiro[3.3]heptan-6-yl]-2,6-dimethyl-4-[5-(trifluoromethyl)pyrimidin-2-yl]piperazine-1-carboxamide;
(2R,6S)-N-{[2-(2,2-dimethylpropyl)-2-azaspiro[3.3]heptan-6-yl]methyl}-2,6-dimethyl-4-[5-(trifluoromethyl)pyrimidin-2-yl]piperazine-1-carboxamide;
2-benzyl-2-azaspiro[3.3]heptan-6-yl 4-(4-cyano-2-fluorophenyl)piperazine-1-carboxylate;
2-benzyl-2-azaspiro[3.3]heptan-6-yl (2R)-4-[3-cyano-4-(trifluoromethoxy)phenyl]-2-methylpiperazine-1-carboxylate;
2-benzyl-2-azaspiro[3.3]heptan-6-yl (1S,4S)-5-(5-cyanopyrimidin-2-yl)-2,5-diazabicyclo[2.2.1]heptane-2-carboxylate;
2-benzyl-2-azaspiro[3.3]heptan-6-yl 4-(3,4,5-trifluorophenyl)piperazine-1-carboxylate;
2-benzyl-2-azaspiro[3.3]heptan-6-yl (2R,5S)-2,5-dimethyl-4-[5-(trifluoromethyl)pyrazin-2-yl]piperazine-1-carboxylate;
2-benzyl-2-azaspiro[3.3]heptan-6-yl 5-(5-cyanopyrimidin-2-yl)-2,5-diazabicyclo[2.2.2]octane-2-carboxylate;
2-benzyl-2-azaspiro[3.3]heptan-6-yl 4-[4-(trifluoromethoxy)phenyl]piperazine-1-carboxylate;
2-benzyl-2-azaspiro[3.3]heptan-6-yl 3-[5-(trifluoromethyl)pyrimidin-2-yl]-3,6-diazabicyclo[3.1.1]heptane-6-carboxylate;
2-benzyl-2-azaspiro[3.3]heptan-6-yl 3-[5-(trifluoromethyl)pyrazin-2-yl]-3,6-diazabicyclo[3.1.1]heptane-6-carboxylate;
2-benzyl-2-azaspiro[3.3]heptan-6-yl 3-(5-cyanopyrazin-2-yl)-3,6-diazabicyclo[3.1.1]heptane-6-carboxylate;
2-benzyl-2-azaspiro[3.3]heptan-6-yl 4-(5-cyanopyrimidin-2-yl)piperazine-1-carboxylate;
2-benzyl-2-azaspiro[3.3]heptan-6-yl 3-[6-(trifluoromethyl)pyridazin-3-yl]-3,6-diazabicyclo[3.1.1]heptane-6-carboxylate;
2-benzyl-2-azaspiro[3.3]heptan-6-yl (1S,4S)-5-[5-(trifluoromethyl)pyrazin-2-yl]-2,5-diazabicyclo[2.2.1]heptane-2-carboxylate;
2-benzyl-2-azaspiro[3.3]heptan-6-yl (1S,4S)-5-[5-(trifluoromethyl)pyrimidin-2-yl]-2,5-diazabicyclo[2.2.1]heptane-2-carboxylate;
2-benzyl-2-azaspiro[3.3]heptan-6-yl 4-[5-(trifluoromethyl)pyrimidin-2-yl]piperazine-1-carboxylate;
2-benzyl-2-azaspiro[3.3]heptan-6-yl (3R,5R)-4-(5-cyanopyrimidin-2-yl)-3,5-dimethylpiperazine-1-carboxylate;
2-benzyl-2-azaspiro[3.3]heptan-6-yl (3S,5S)-4-(5-cyanopyrimidin-2-yl)-3,5-dimethylpiperazine-1-carboxylate;
2-benzyl-2-azaspiro[3.3]heptan-6-yl (3S)-4-(5-cyanopyrimidin-2-yl)-3-methylpiperazine-1-carboxylate;
2-benzyl-2-azaspiro[3.3]heptan-6-yl (3R)-4-(5-cyanopyrimidin-2-yl)-3-methylpiperazine-1-carboxylate;
2-benzyl-2-azaspiro[3.3]heptan-6-yl 4-[5-(trifluoromethyl)pyridin-2-yl]piperazine-1-carboxylate;
2-benzyl-2-azaspiro[3.3]heptan-6-yl (1R,4R)-5-[5-(trifluoromethyl)pyrazin-2-yl]-2,5-diazabicyclo[2.2.1]heptane-2-carboxylate;
2-benzyl-2-azaspiro[3.3]heptan-6-yl (3S)-3-methyl-4-[5-(trifluoromethyl)pyrimidin-2-yl]piperazine-1-carboxylate;
2-benzyl-2-azaspiro[3.3]heptan-6-yl 4-(4-cyano-2,5-difluorophenyl)piperazine-1-carboxylate;
2-benzyl-2-azaspiro[3.3]heptan-6-yl 4-[4-(trifluoromethyl)phenyl]piperazine-1-carboxylate;
2-benzyl-2-azaspiro[3.3]heptan-6-yl 4-[2-cyano-4-(trifluoromethyl)phenyl]piperazine-1-carboxylate;
2-benzyl-2-azaspiro[3.3]heptan-6-yl 4-(3-cyano-5-fluorophenyl)piperazine-1-carboxylate;
2-benzyl-2-azaspiro[3.3]heptan-6-yl (2R,5R)-4-(5-cyanopyrimidin-2-yl)-2,5-dimethylpiperazine-1-carboxylate;
2-benzyl-2-azaspiro[3.3]heptan-6-yl (2S)-4-(5-cyanopyrimidin-2-yl)-2-methylpiperazine-1-carboxylate;
2-benzyl-2-azaspiro[3.3]heptan-6-yl (2R,6S)-4-(5-cyanopyrimidin-2-yl)-2,6-dimethylpiperazine-1-carboxylate;
{2-benzyl-2-azaspiro[3.3]heptan-6-yl}methyl (2R,6S)-4-(5-cyanopyrimidin-2-yl)-2,6-dimethylpiperazine-1-carboxylate;
2-benzyl-2-azaspiro[3.3]heptan-6-yl (2R)-2-methyl-4-[5-(trifluoromethyl)pyrimidin-2-yl]piperazine-1-carboxylate;
{2-benzyl-2-azaspiro[3.3]heptan-6-yl}methyl (2R)-2-methyl-4-[5-(trifluoromethyl)pyrimidin-2-yl]piperazine-1-carboxylate;
{2-benzyl-2-azaspiro[3.3]heptan-6-yl}methyl (2R,5S)-2,5-dimethyl-4-[5-(trifluoromethyl)pyrimidin-2-yl]piperazine-1-carboxylate;
2-benzyl-2-azaspiro[3.3]heptan-6-yl (2R,5R)-2,5-dimethyl-4-[5-(trifluoromethyl)pyrimidin-2-yl]piperazine-1-carboxylate;
2-benzyl-2-azaspiro[3.3]heptan-6-yl (2R,5S)-2,5-dimethyl-4-[5-(trifluoromethyl)pyrimidin-2-yl]piperazine-1-carboxylate;
2-benzyl-2-azaspiro[3.3]heptan-6-yl (2R)-2-methyl-4-[5-(trifluoromethyl)pyrazin-2-yl]piperazine-1-carboxylate;
2-benzyl-2-azaspiro[3.3]heptan-6-yl (2R,5S)-4-(5-cyanopyrimidin-2-yl)-2,5-dimethylpiperazine-1-carboxylate;
2-benzyl-2-azaspiro[3.3]heptan-6-yl (2R,6R)-2,6-dimethyl-4-[5-(trifluoromethyl)pyrimidin-2-yl]piperazine-1-carboxylate;
{2-benzyl-2-azaspiro[3.3]heptan-6-yl}methyl (2R,6S)-2,6-dimethyl-4-[5-(trifluoromethyl)pyrazin-2-yl]piperazine-1-carboxylate;
2-benzyl-2-azaspiro[3.3]heptan-6-yl (2R,6S)-2,6-dimethyl-4-[5-(trifluoromethyl)pyrazin-2-yl]piperazine-1-carboxylate;
2-benzyl-2-azaspiro[3.3]heptan-6-yl (2R,6R)-2,6-dimethyl-4-[5-(trifluoromethyl)pyrazin-2-yl]piperazine-1-carboxylate;
{2-benzyl-2-azaspiro[3.3]heptan-6-yl}methyl (2R,6R)-2,6-dimethyl-4-[5-(trifluoromethyl)pyrazin-2-yl]piperazine-1-carboxylate;
{2-benzyl-2-azaspiro[3.3]heptan-6-yl}methyl (2R,6R)-2,6-dimethyl-4-[5-(trifluoromethyl)pyrimidin-2-yl]piperazine-1-carboxylate;
2-(3,3-dimethylbutyl)-2-azaspiro[3.3]heptan-6-yl (2R,6R)-2,6-dimethyl-4-[5-(trifluoromethyl)pyrimidin-2-yl]piperazine-1-carboxylate;
2-(2,2-dimethylpropyl)-2-azaspiro[3.3]heptan-6-yl (2R,6R)-2,6-dimethyl-4-[5-(trifluoromethyl)pyrimidin-2-yl]piperazine-1-carboxylate;
2-benzyl-2-azaspiro[3.3]heptan-6-yl (2R,6S)-2,6-dimethyl-4-[5-(trifluoromethyl)pyrimidin-2-yl]piperazine-1-carboxylate;
{2-benzyl-2-azaspiro[3.3]heptan-6-yl}methyl (2R,6S)-2,6-dimethyl-4-[5-(trifluoromethyl)pyrimidin-2-yl]piperazine-1-carboxylate;
2-(3,3-dimethylbutyl)-2-azaspiro[3.3]heptan-6-yl (2R,6S)-2,6-dimethyl-4-[5-(trifluoromethyl)pyrimidin-2-yl]piperazine-1-carboxylate;
[2-(3,3-dimethylbutyl)-2-azaspiro[3.3]heptan-6-yl]methyl (2R,6S)-2,6-dimethyl-4-[5-(trifluoromethyl)pyrimidin-2-yl]piperazine-1-carboxylate;
2-(2,2-dimethylpropyl)-2-azaspiro[3.3]heptan-6-yl (2R,6S)-2,6-dimethyl-4-[5-(trifluoromethyl)pyrimidin-2-yl]piperazine-1-carboxylate;
[2-(2,2-dimethylpropyl)-2-azaspiro[3.3]heptan-6-yl]methyl (2R,6S)-2,6-dimethyl-4-[5-(trifluoromethyl)pyrimidin-2-yl]piperazine-1-carboxylate;
2-[(2,2-dimethylcyclopropyl)methyl]-2-azaspiro[3.3]heptan-6-yl (2R,6S)-2,6-dimethyl-4-[5-(trifluoromethyl)pyrimidin-2-yl]piperazine-1-carboxylate;
2-(cyclohexylmethyl)-2-azaspiro[3.3]heptan-6-yl (2R,6S)-2,6-dimethyl-4-[5-(trifluoromethyl)pyrimidin-2-yl]piperazine-1-carboxylate;
2-{bicyclo[2.2.1]hept-5-en-2-ylmethyl}-2-azaspiro[3.3]heptan-6-yl (2R,6S)-2,6-dimethyl-4-[5-(trifluoromethyl)pyrimidin-2-yl]piperazine-1-carboxylate;
2-[(2,6-difluorophenyl)methyl]-2-azaspiro[3.3]heptan-6-yl (2R,6S)-2,6-dimethyl-4-[5-(trifluoromethyl)pyrimidin-2-yl]piperazine-1-carboxylate;
2-benzyl-2-azaspiro[3.3]heptan-6-yl (2R,6S)-4-(5-fluoropyrimidin-2-yl)-2,6-dimethylpiperazine-1-carboxylate;
2-benzyl-2-azaspiro[3.3]heptan-6-yl (2R,6R)-4-(5-fluoropyrimidin-2-yl)-2,6-dimethylpiperazine-1-carboxylate;
2-benzyl-2-azaspiro[3.3]heptan-6-yl (2R,6R)-4-(5-cyanopyrimidin-2-yl)-2,6-dimethylpiperazine-1-carboxylate;
2-benzyl-2-azaspiro[3.3]heptan-6-yl 7-(5-cyanopyrimidin-2-yl)-3-oxa-7,9-diazabicyclo[3.3.1]nonane-9-carboxylate;
2-benzyl-2-azaspiro[3.3]heptan-6-yl (2R,6S)-4-(5-acetylpyrazin-2-yl)-2,6-dimethylpiperazine-1-carboxylate;
2-benzyl-2-azaspiro[3.3]heptan-6-yl (2R,6R)-4-(5-acetylpyrazin-2-yl)-2,6-dimethylpiperazine-1-carboxylate;
2-benzyl-2-azaspiro[3.3]heptan-6-yl (2R,6S)-2,6-dimethyl-4-(5-nitropyridin-2-yl)piperazine-1-carboxylate;
2-benzyl-2-azaspiro[3.3]heptan-6-yl (2R,6R)-2,6-dimethyl-4-(5-nitropyridin-2-yl)piperazine-1-carboxylate;
2-benzyl-2-azaspiro[3.3]heptan-6-yl (2R,6S)-2,6-dimethyl-4-[6-(trifluoromethyl)pyrazin-2-yl]piperazine-1-carboxylate;
2-benzyl-2-azaspiro[3.3]heptan-6-yl (2R,6R)-2,6-dimethyl-4-[6-(trifluoromethyl)pyrazin-2-yl]piperazine-1-carboxylate;
2-benzyl-2-azaspiro[3.3]heptan-6-yl 7-[5-(trifluoromethyl)pyrimidin-2-yl]-3-oxa-7,9-diazabicyclo[3.3.1]nonane-9-carboxylate;
2-benzyl-2-azaspiro[3.3]heptan-6-yl 7-[5-(trifluoromethyl)pyrazin-2-yl]-3-oxa-7,9-diazabicyclo[3.3.1]nonane-9-carboxylate;
2-benzyl-2-azaspiro[3.3]heptan-6-yl (2R,6S)-4-[5-bromo-4-(methylsulfanyl)pyrimidin-2-yl]-2,6-dimethylpiperazine-1-carboxylate;
2-benzyl-2-azaspiro[3.3]heptan-6-yl (2R,6R)-4-[5-bromo-4-(methylsulfanyl)pyrimidin-2-yl]-2,6-dimethylpiperazine-1-carboxylate;
methyl 2-[(3R,5R)-4-[({2-benzyl-2-azaspiro[3.3]heptan-6-yl}oxy)carbonyl]-3,5-dimethylpiperazin-1-yl]-4-(trifluoromethyl)pyrimidine-5-carboxylate;
2-benzyl-2-azaspiro[3.3]heptan-6-yl (2R,6S)-2,6-dimethyl-4-(quinoxalin-2-yl)piperazine-1-carboxylate;
2-benzyl-2-azaspiro[3.3]heptan-6-yl (2R,5S)-4-(6-methoxyquinoxalin-2-yl)-2,5-dimethylpiperazine-1-carboxylate;
2-benzyl-2-azaspiro[3.3]heptan-6-yl (2R,5S)-2,5-dimethyl-4-(4-methylpyrimidin-2-yl)piperazine-1-carboxylate;
2-benzyl-2-azaspiro[3.3]heptan-6-yl (2R,5R)-2,5-dimethyl-4-(4-methylpyrimidin-2-yl)piperazine-1-carboxylate;
2-benzyl-2-azaspiro[3.3]heptan-6-yl (2R,6R)-2,6-dimethyl-4-(4-methylpyrimidin-2-yl)piperazine-1-carboxylate;
2-benzyl-2-azaspiro[3.3]heptan-6-yl (2R,5S)-4-(5-fluoropyrimidin-2-yl)-2,5-dimethylpiperazine-1-carboxylate;
2-benzyl-2-azaspiro[3.3]heptan-6-yl (2R,5S)-4-(3,6-dimethylpyrazin-2-yl)-2,5-dimethylpiperazine-1-carboxylate;
2-benzyl-2-azaspiro[3.3]heptan-6-yl (2R,5S)-4-(6-methoxypyrazin-2-yl)-2,5-dimethylpiperazine-1-carboxylate;
2-benzyl-2-azaspiro[3.3]heptan-6-yl (2R,5R)-4-(6-methoxypyrazin-2-yl)-2,5-dimethylpiperazine-1-carboxylate;
2-benzyl-2-azaspiro[3.3]heptan-6-yl (2R,6S)-4-(5-cyano-3-methylpyrazin-2-yl)-2,6-dimethylpiperazine-1-carboxylate;
2-benzyl-2-azaspiro[3.3]heptan-6-yl (2R,6R)-4-(5-cyano-3-methylpyrazin-2-yl)-2,6-dimethylpiperazine-1-carboxylate;
2-benzyl-2-azaspiro[3.3]heptan-6-yl (2R,5S)-2,5-dimethyl-4-(5-nitropyridin-2-yl)piperazine-1-carboxylate;
2-benzyl-2-azaspiro[3.3]heptan-6-yl (2R,5R)-2,5-dimethyl-4-(5-nitropyridin-2-yl)piperazine-1-carboxylate;
2-benzyl-2-azaspiro[3.3]heptan-6-yl (2R,6R)-2,6-dimethyl-4-(quinoxalin-2-yl)piperazine-1-carboxylate;
2-benzyl-2-azaspiro[3.3]heptan-6-yl (2R,5R)-2,5-dimethyl-4-(quinoxalin-2-yl)piperazine-1-carboxylate;
2-benzyl-2-azaspiro[3.3]heptan-6-yl (2R,5S)-2,5-dimethyl-4-(quinoxalin-2-yl)piperazine-1-carboxylate;
2-benzyl-2-azaspiro[3.3]heptan-6-yl 9-(quinoxalin-2-yl)-3-oxa-7,9-diazabicyclo[3.3.1]nonane-7-carboxylate;
2-benzyl-2-azaspiro[3.3]heptan-6-yl (2R,6S)-2,6-dimethyl-4-[3-(trifluoromethyl)pyridin-2-yl]piperazine-1-carboxylate;
2-benzyl-2-azaspiro[3.3]heptan-6-yl (2R,6R)-2,6-dimethyl-4-[3-(trifluoromethyl)pyridin-2-yl]piperazine-1-carboxylate;
2-benzyl-2-azaspiro[3.3]heptan-6-yl (2R,6S)-2,6-dimethyl-4-[6-(trifluoromethyl)pyridin-3-yl]piperazine-1-carboxylate;
2-benzyl-2-azaspiro[3.3]heptan-6-yl (2R,6R)-2,6-dimethyl-4-[6-(trifluoromethyl)pyridin-3-yl]piperazine-1-carboxylate;
2-benzyl-2-azaspiro[3.3]heptan-6-yl (2R,5R)-2,5-dimethyl-4-[6-(trifluoromethyl)pyridin-3-yl]piperazine-1-carboxylate;
2-benzyl-2-azaspiro[3.3]heptan-6-yl (2R,5S)-2,5-dimethyl-4-[6-(trifluoromethyl)pyrazin-2-yl]piperazine-1-carboxylate;
2-benzyl-2-azaspiro[3.3]heptan-6-yl (2R,5R)-2,5-dimethyl-4-[5-(trifluoromethyl)pyrazin-2-yl]piperazine-1-carboxylate;
2-benzyl-2-azaspiro[3.3]heptan-6-yl (2R,5R)-2,5-dimethyl-4-[6-(trifluoromethyl)pyrazin-2-yl]piperazine-1-carboxylate;
2-benzyl-2-azaspiro[3.3]heptan-6-yl (2R,6S)-4-(6-fluoroquinoxalin-2-yl)-2,6-dimethylpiperazine-1-carboxylate;
2-benzyl-2-azaspiro[3.3]heptan-6-yl (2R,6S)-4-(6-methoxy-3-nitropyridin-2-yl)-2,6-dimethylpiperazine-1-carboxylate;
2-benzyl-2-azaspiro[3.3]heptan-6-yl (2R,5S)-4-(6-methoxy-3-nitropyridin-2-yl)-2,5-dimethylpiperazine-1-carboxylate;
2-benzyl-2-azaspiro[3.3]heptan-6-yl (2R,6R)-4-(6-methoxy-3-nitropyridin-2-yl)-2,6-dimethylpiperazine-1-carboxylate;
2-benzyl-2-azaspiro[3.3]heptan-6-yl (2R,5R)-4-(6-methoxyquinoxalin-2-yl)-2,5-dimethylpiperazine-1-carboxylate;
2-benzyl-2-azaspiro[3.3]heptan-6-yl (2R,6S)-2,6-dimethyl-4-[5-(trifluoromethoxy)pyrazin-2-yl]piperazine-1-carboxylate;
2-benzyl-2-azaspiro[3.3]heptan-6-yl (2R,6R)-2,6-dimethyl-4-[5-(trifluoromethoxy)pyrazin-2-yl]piperazine-1-carboxylate;
2-benzyl-2-azaspiro[3.3]heptan-6-yl (2R,5S)-2,5-dimethyl-4-[5-(trifluoromethoxy)pyrazin-2-yl]piperazine-1-carboxylate;
2-benzyl-2-azaspiro[3.3]heptan-6-yl (2R,5R)-2,5-dimethyl-4-[5-(trifluoromethoxy)pyrazin-2-yl]piperazine-1-carboxylate;
2-benzyl-2-azaspiro[3.3]heptan-6-yl (2R,6R)-4-[4-cyano-6-(trifluoromethyl)pyridin-3-yl]-2,6-dimethylpiperazine-1-carboxylate;
2-benzyl-2-azaspiro[3.3]heptan-6-yl (2R,5R)-4-[4-cyano-6-(trifluoromethyl)pyridin-3-yl]-2,5-dimethylpiperazine-1-carboxylate;
methyl 2-[(2S,5R)-4-[({2-benzyl-2-azaspiro[3.3]heptan-6-yl}oxy)carbonyl]-2,5-dimethylpiperazin-1-yl]-4-(trifluoromethyl)pyrimidine-5-carboxylate;
(2R,6S)-N-{2-benzyl-2-azaspiro[3.3]heptan-6-yl}-2,6-dimethyl-4-(quinazolin-2-yl)piperazine-1-carboxamide;
(2R,6S)-N-{2-benzyl-2-azaspiro[3.3]heptan-6-yl}-4-(6-fluoroquinoxalin-2-yl)-2,6-dimethylpiperazine-1-carboxamide;
(2R,6R)-N-{2-benzyl-2-azaspiro[3.3]heptan-6-yl}-2,6-dimethyl-4-(quinazolin-2-yl)piperazine-1-carboxamide;
(2R,5S)-N-{2-benzyl-2-azaspiro[3.3]heptan-6-yl}-2,5-dimethyl-4-(quinazolin-2-yl)piperazine-1-carboxamide;
(2R)-N-{2-benzyl-2-azaspiro[3.3]heptan-6-yl}-2-methyl-4-(quinazolin-2-yl)piperazine-1-carboxamide;
(2R,6S)-N-{2-benzyl-2-azaspiro[3.3]heptan-6-yl}-2,6-dimethyl-4-(quinoxalin-2-yl)piperazine-1-carboxamide;
(2R,6S)-N-{2-benzyl-2-azaspiro[3.3]heptan-6-yl}-4-(6-methoxyquinoxalin-2-yl)-2,6-dimethylpiperazine-1-carboxamide;
(2R,6S)-N-{2-benzyl-2-azaspiro[3.3]heptan-6-yl}-2,6-dimethyl-4-[6-(trifluoromethyl)-1,3-benzothiazol-2-yl]piperazine-1-carboxamide;
(2R,6R)-N-{2-benzyl-2-azaspiro[3.3]heptan-6-yl}-2,6-dimethyl-4-[6-(trifluoromethyl)-1,3-benzothiazol-2-yl]piperazine-1-carboxamide;
(2R,6R)-N-{2-benzyl-2-azaspiro[3.3]heptan-6-yl}-4-(6-fluoroquinoxalin-2-yl)-2,6-dimethylpiperazine-1-carboxamide;
(2R,6R)-N-{2-benzyl-2-azaspiro[3.3]heptan-6-yl}-4-(6-methoxyquinoxalin-2-yl)-2,6-dimethylpiperazine-1-carboxamide;
(2R,6R)-N-{2-benzyl-2-azaspiro[3.3]heptan-6-yl}-2,6-dimethyl-4-(quinoxalin-2-yl)piperazine-1-carboxamide;
(2R,5S)-N-{2-benzyl-2-azaspiro[3.3]heptan-6-yl}-2,5-dimethyl-4-(quinoxalin-2-yl)piperazine-1-carboxamide;
(2R,5S)-N-{2-benzyl-2-azaspiro[3.3]heptan-6-yl}-4-(6-methoxyquinoxalin-2-yl)-2,5-dimethylpiperazine-1-carboxamide;
(2R,6S)-N-{2-benzyl-2-azaspiro[3.3]heptan-6-yl}-2,6-dimethyl-4-[6-(trifluoromethyl)-1,3-benzothiazol-2-yl]piperazine-1-carboxamide;
(2R)-N-{2-benzyl-2-azaspiro[3.3]heptan-6-yl}-2-methyl-4-(quinoxalin-2-yl)piperazine-1-carboxamide;
(2R)-N-{2-benzyl-2-azaspiro[3.3]heptan-6-yl}-2-methyl-4-[6-(trifluoromethyl)-1,3-benzothiazol-2-yl]piperazine-1-carboxamide;
(2R)-N-{2-benzyl-2-azaspiro[3.3]heptan-6-yl}-4-(6-methoxyquinoxalin-2-yl)-2-methylpiperazine-1-carboxamide;
(2R,6S)-N-{2-benzyl-2-azaspiro[3.3]heptan-6-yl}-4-[3-fluoro-5-(trifluoromethyl)pyridin-2-yl]-2,6-dimethylpiperazine-1-carboxamide;
(2R,6S)-N-{2-benzyl-2-azaspiro[3.3]heptan-6-yl}-4-(5-chloro-4-methylpyrimidin-2-yl)-2,6-dimethylpiperazine-1-carboxamide;
(2R,6S)-N-{2-benzyl-2-azaspiro[3.3]heptan-6-yl}-4-(5-chloropyrazin-2-yl)-2,6-dimethylpiperazine-1-carboxamide;
(2R,6S)-4-(6-amino-5-chloropyrazin-2-yl)-N-{2-benzyl-2-azaspiro[3.3]heptan-6-yl}-2,6-dimethylpiperazine-1-carboxamide;
(2R,6S)-N-{2-benzyl-2-azaspiro[3.3]heptan-6-yl}-4-(6-fluoro-1,3-benzothiazol-2-yl)-2,6-dimethylpiperazine-1-carboxamide;
(2R,6R)-N-{2-benzyl-2-azaspiro[3.3]heptan-6-yl}-4-(6-fluoro-1,3-benzothiazol-2-yl)-2,6-dimethylpiperazine-1-carboxamide;
(2R,5S)-N-{2-benzyl-2-azaspiro[3.3]heptan-6-yl}-4-(6-fluoro-1,3-benzothiazol-2-yl)-2,5-dimethylpiperazine-1-carboxamide;
(2R)-N-{2-benzyl-2-azaspiro[3.3]heptan-6-yl}-4-(6-fluoro-1,3-benzothiazol-2-yl)-2-methylpiperazine-1-carboxamide;
(2R,6S)-N-{2-benzyl-2-azaspiro[3.3]heptan-6-yl}-2,6-dimethyl-4-[6-(trifluoromethyl)quinoxalin-2-yl]piperazine-1-carboxamide;
(2R,6R)-N-{2-benzyl-2-azaspiro[3.3]heptan-6-yl}-2,6-dimethyl-4-[6-(trifluoromethyl)quinoxalin-2-yl]piperazine-1-carboxamide;
(2R,5S)-N-{2-benzyl-2-azaspiro[3.3]heptan-6-yl}-2,5-dimethyl-4-[6-(trifluoromethyl)quinoxalin-2-yl]piperazine-1-carboxamide;
(2R)-N-{2-benzyl-2-azaspiro[3.3]heptan-6-yl}-2-methyl-4-[6-(trifluoromethyl)quinoxalin-2-yl]piperazine-1-carboxamide;
(2R,6S)-N-{2-benzyl-2-azaspiro[3.3]heptan-6-yl}-2,6-dimethyl-4-{[1,3]oxazolo[4,5-b]pyridin-2-yl}piperazine-1-carboxamide;
(2R,6R)-N-{2-benzyl-2-azaspiro[3.3]heptan-6-yl}-2,6-dimethyl-4-{[1,3]oxazolo[4,5-b]pyridin-2-yl}piperazine-1-carboxamide;
(2R,6S)-N-{2-benzyl-2-azaspiro[3.3]heptan-6-yl}-4-(6-methoxy-1,3-benzothiazol-2-yl)-2,6-dimethylpiperazine-1-carboxamide;
(2R,6R)-N-{2-benzyl-2-azaspiro[3.3]heptan-6-yl}-4-(6-methoxy-1,3-benzothiazol-2-yl)-2,6-dimethylpiperazine-1-carboxamide;
(2R,6R)-4-(1,3-benzothiazol-2-yl)-N-{2-benzyl-2-azaspiro[3.3]heptan-6-yl}-2,6-dimethylpiperazine-1-carboxamide;
(2R,5S)-4-(1,3-benzothiazol-2-yl)-N-{2-benzyl-2-azaspiro[3.3]heptan-6-yl}-2,5-dimethylpiperazine-1-carboxamide;
(2R)-4-(1,3-benzothiazol-2-yl)-N-{2-benzyl-2-azaspiro[3.3]heptan-6-yl}-2-methylpiperazine-1-carboxamide;
(2R,5S)-N-{2-benzyl-2-azaspiro[3.3]heptan-6-yl}-4-(6-fluoroquinoxalin-2-yl)-2,5-dimethylpiperazine-1-carboxamide;
(2R,6S)-N-{2-benzyl-2-azaspiro[3.3]heptan-6-yl}-4-[5-(difluoromethoxy)pyrimidin-2-yl]-2,6-dimethylpiperazine-1-carboxamide;
(2R,6S)-4-[4-amino-5-(trifluoromethyl)pyrimidin-2-yl]-N-{2-benzyl-2-azaspiro[3.3]heptan-6-yl}-2,6-dimethylpiperazine-1-carboxamide;
(2R,6S)-N-{2-benzyl-2-azaspiro[3.3]heptan-6-yl}-4-[5-(difluoromethyl)pyrimidin-2-yl]-2,6-dimethylpiperazine-1-carboxamide;
(2R,6S)-N-{2-benzyl-2-azaspiro[3.3]heptan-6-yl}-4-(5-fluoropyrimidin-2-yl)-2,6-dimethylpiperazine-1-carboxamide;
(2R,6S)-N-{2-[(4-chlorophenyl)methyl]-2-azaspiro[3.3]heptan-6-yl}-4-(5-cyanopyrimidin-2-yl)-2,6-dimethylpiperazine-1-carboxamide;
(2R,6R)-N-{2-[(4-chlorophenyl)methyl]-2-azaspiro[3.3]heptan-6-yl}-4-(5-cyanopyrimidin-2-yl)-2,6-dimethylpiperazine-1-carboxamide;
(2R,5S)-N-{2-[(4-chlorophenyl)methyl]-2-azaspiro[3.3]heptan-6-yl}-4-(5-cyanopyrimidin-2-yl)-2,5-dimethylpiperazine-1-carboxamide;
(2R)-N-{2-[(4-chlorophenyl)methyl]-2-azaspiro[3.3]heptan-6-yl}-4-(5-cyanopyrimidin-2-yl)-2-methylpiperazine-1-carboxamide;
(2R,6R)-N-{2-[(4-chlorophenyl)methyl]-2-azaspiro[3.3]heptan-6-yl}-2,6-dimethyl-4-[5-(trifluoromethyl)pyrimidin-2-yl]piperazine-1-carboxamide;
(2R,5S)-N-{2-[(4-chlorophenyl)methyl]-2-azaspiro[3.3]heptan-6-yl}-2,5-dimethyl-4-[5-(trifluoromethyl)pyrimidin-2-yl]piperazine-1-carboxamide;
(2R)-N-{2-[(4-chlorophenyl)methyl]-2-azaspiro[3.3]heptan-6-yl}-2-methyl-4-[5-(trifluoromethyl)pyrimidin-2-yl]piperazine-1-carboxamide;
(2R,6S)-N-{2-[(4-chlorophenyl)methyl]-2-azaspiro[3.3]heptan-6-yl}-2,6-dimethyl-4-[5-(trifluoromethyl)pyrazin-2-yl]piperazine-1-carboxamide;
(2R,6R)-N-{2-[(4-chlorophenyl)methyl]-2-azaspiro[3.3]heptan-6-yl}-2,6-dimethyl-4-[5-(trifluoromethyl)pyrazin-2-yl]piperazine-1-carboxamide;
(2R,5S)-N-{2-[(4-chlorophenyl)methyl]-2-azaspiro[3.3]heptan-6-yl}-2,5-dimethyl-4-[5-(trifluoromethyl)pyrazin-2-yl]piperazine-1-carboxamide;
(2R)-N-{2-[(4-chlorophenyl)methyl]-2-azaspiro[3.3]heptan-6-yl}-2-methyl-4-[5-(trifluoromethyl)pyrazin-2-yl]piperazine-1-carboxamide;
(2R,6R)-4-(5-cyanopyrimidin-2-yl)-N-{2-[(4-fluorophenyl)methyl]-2-azaspiro[3.3]heptan-6-yl}-2,6-dimethylpiperazine-1-carboxamide;
(2R,5S)-4-(5-cyanopyrimidin-2-yl)-N-{2-[(4-fluorophenyl)methyl]-2-azaspiro[3.3]heptan-6-yl}-2,5-dimethylpiperazine-1-carboxamide;
(2R)-4-(5-cyanopyrimidin-2-yl)-N-{2-[(4-fluorophenyl)methyl]-2-azaspiro[3.3]heptan-6-yl}-2-methylpiperazine-1-carboxamide;
(2R,5S)-N-{2-[(4-fluorophenyl)methyl]-2-azaspiro[3.3]heptan-6-yl}-2,5-dimethyl-4-[5-(trifluoromethyl)pyrimidin-2-yl]piperazine-1-carboxamide;
(2R)-N-{2-[(4-fluorophenyl)methyl]-2-azaspiro[3.3]heptan-6-yl}-2-methyl-4-[5-(trifluoromethyl)pyrimidin-2-yl]piperazine-1-carboxamide;
(2R,6S)-N-{2-[(4-fluorophenyl)methyl]-2-azaspiro[3.3]heptan-6-yl}-2,6-dimethyl-4-[5-(trifluoromethyl)pyrazin-2-yl]piperazine-1-carboxamide;
(2R,6R)-N-{2-[(4-fluorophenyl)methyl]-2-azaspiro[3.3]heptan-6-yl}-2,6-dimethyl-4-[5-(trifluoromethyl)pyrazin-2-yl]piperazine-1-carboxamide;
(2R,5S)-N-{2-[(4-fluorophenyl)methyl]-2-azaspiro[3.3]heptan-6-yl}-2,5-dimethyl-4-[5-(trifluoromethyl)pyrazin-2-yl]piperazine-1-carboxamide;
(2R)-N-{2-[(4-fluorophenyl)methyl]-2-azaspiro[3.3]heptan-6-yl}-2-methyl-4-[5-(trifluoromethyl)pyrazin-2-yl]piperazine-1-carboxamide;
(2R,6S)-N-{2-benzyl-2-azaspiro[3.3]heptan-6-yl}-4-(6-fluoroquinazolin-2-yl)-2,6-dimethylpiperazine-1-carboxamide;
(2R,5S)-N-{2-benzyl-2-azaspiro[3.3]heptan-6-yl}-4-(6-fluoroquinazolin-2-yl)-2,5-dimethylpiperazine-1-carboxamide;
(2R,6S)-N-{2-[(²H₅)benzyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-{2-[(²H₅)phenyl(²H₁)methyl]-2-azaspiro[3.3]heptan-6-yl}-4-[5-(trifluoromethyl)pyrimidin-2-yl]piperazine-1-carboxamide;
(2R,6S)-2,6-dimethyl-N-{2-[(²H₅)phenyl(²H₂)methyl]-2-azaspiro[3.3]heptan-6-yl}-4-[5-(trifluoromethyl)pyrimidin-2-yl]piperazine-1-carboxamide;
2-benzyl-2-azaspiro[3.3]heptan-6-yl (2R,6S)-4-[4-methoxy-5-(trifluoromethyl)pyrimidin-2-yl]-2,6-dimethylpiperazine-1-carboxylate;
2-benzyl-2-azaspiro[3.3]heptan-6-yl (2R,6S)-4-[3-fluoro-5-(trifluoromethyl)pyridin-2-yl]-2,6-dimethylpiperazine-1-carboxylate;
2-benzyl-2-azaspiro[3.3]heptan-6-yl (2R,6S)-4-(5-chloropyrimidin-2-yl)-2,6-dimethylpiperazine-1-carboxylate;
2-benzyl-2-azaspiro[3.3]heptan-6-yl (2R,6S)-4-(5-chloro-4-methylpyrimidin-2-yl)-2,6-dimethylpiperazine-1-carboxylate;
2-benzyl-2-azaspiro[3.3]heptan-6-yl (2R,6S)-4-(5-chloropyrazin-2-yl)-2,6-dimethylpiperazine-1-carboxylate;
N-{2-benzyl-2-azaspiro[3.3]heptan-6-yl}-9-[5-(trifluoromethyl)pyrimidin-2-yl]-3-oxa-7,9-diazabicyclo[3.3.1]nonane-7-carboxamide;
2-benzyl-2-azaspiro[3.3]heptan-6-yl (2R,6S)-4-(6-amino-5-chloropyrazin-2-yl)-2,6-dimethylpiperazine-1-carboxylate;
2-[(²H₅)benzyl]-2-azaspiro[3.3]heptan-6-yl (2R,5S)-2,5-dimethyl-4-[5-(trifluoromethyl)pyrazin-2-yl]piperazine-1-carboxylate;
(2R,6S)-N-{2-benzyl-2-azaspiro[3.3]heptan-6-yl}-4-(4-ethoxyphenyl)-2,6-dimethylpiperazine-1-carboxamide;
(2R,6S)-N-{2-benzyl-2-azaspiro[3.3]heptan-6-yl}-4-(4-methoxyphenyl)-2,6-dimethylpiperazine-1-carboxamide;
(2R,6S)-N-{2-benzyl-2-azaspiro[3.3]heptan-6-yl}-4-(4-fluoro-3-methylphenyl)-2,6-dimethylpiperazine-1-carboxamide;
(2R,6S)-N-{2-benzyl-2-azaspiro[3.3]heptan-6-yl}-4-(4-cyano-3-methoxyphenyl)-2,6-dimethylpiperazine-1-carboxamide;
(2R,5S)-N-{2-benzyl-2-azaspiro[3.3]heptan-6-yl}-2,5-dimethyl-4-[4-(trifluoromethyl)phenyl]piperazine-1-carboxamide;
(2R)-N-{2-benzyl-2-azaspiro[3.3]heptan-6-yl}-2-methyl-4-[4-(trifluoromethyl)phenyl]piperazine-1-carboxamide;
(2R,6S)-N-{2-[(3-methoxypyridin-4-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(2-chloropyridin-3-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-[2-(1H-indol-4-ylmethyl)-2-azaspiro[3.3]heptan-6-yl]-2,6-dimethyl-4-[5-(trifluoromethyl)pyrimidin-2-yl]piperazine-1-carboxamide;
(2R,6S)-2,6-dimethyl-N-[2-(pyridin-2-ylmethyl)-2-azaspiro[3.3]heptan-6-yl]-4-[5-(trifluoromethyl)pyrimidin-2-yl]piperazine-1-carboxamide;
(2R,6S)-N-{2-[(2-chlorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-[2-(1,3-thiazol-4-ylmethyl)-2-azaspiro[3.3]heptan-6-yl]-4-[5-(trifluoromethyl)pyrimidin-2-yl]piperazine-1-carboxamide;
(2R,6S)-N-{2-[(1,3-dimethyl-1H-pyrazol-5-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(4-methoxypyridin-3-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-[2-(1-benzofuran-5-ylmethyl)-2-azaspiro[3.3]heptan-6-yl]-2,6-dimethyl-4-[5-(trifluoromethyl)pyrimidin-2-yl]piperazine-1-carboxamide;
(2R,6S)-2,6-dimethyl-N-{2-[(1-methyl-1H-imidazol-2-yl)methyl]-2-azaspiro[3.3]heptan-6-yl}-4-[5-(trifluoromethyl)pyrimidin-2-yl]piperazine-1-carboxamide;
(2R,6S)-2,6-dimethyl-N-{2-[(4-methyl-1,3-thiazol-2-yl)methyl]-2-azaspiro[3.3]heptan-6-yl}-4-[5-(trifluoromethyl)pyrimidin-2-yl]piperazine-1-carboxamide;
(2R,6S)-2,6-dimethyl-N-{2-[(4-methyl-1H-imidazol-5-yl)methyl]-2-azaspiro[3.3]heptan-6-yl}-4-[5-(trifluoromethyl)pyrimidin-2-yl]piperazine-1-carboxamide;
(2R,6S)-N-{2-[(3-chloropyridin-4-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-[2-(pyridin-3-ylmethyl)-2-azaspiro[3.3]heptan-6-yl]-4-[5-(trifluoromethyl)pyrimidin-2-yl]piperazine-1-carboxamide;
(2R,6S)-N-{2-[(4-methoxypyridin-2-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(6-methoxypyridin-3-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-[2-(1H-imidazol-4-ylmethyl)-2-azaspiro[3.3]heptan-6-yl]-2,6-dimethyl-4-[5-(trifluoromethyl)pyrimidin-2-yl]piperazine-1-carboxamide;
(2R,6S)-N-{2-[(6-chloropyridin-3-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(2-fluoropyridin-3-yl)methyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-[2-(1H-pyrrol-2-ylmethyl)-2-azaspiro[3.3]heptan-6-yl]-4-[5-(trifluoromethyl)pyrimidin-2-yl)piperazine-1-carboxamide;
(2R,6S)-N-{2-[(2,5-difluorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-[2-(2-phenylethyl)-2-azaspiro[3.3]heptan-6-yl]-4-[5-(trifluoromethyl)pyrimidin-2-yl]piperazine-1-carboxamide;
(2R,6S)-2,6-dimethyl-N-[2-(4-phenylbutyl)-2-azaspiro[3.3]heptan-6-yl]-4-[5-(trifluoromethyl)pyrimidin-2-yl]piperazine-1-carboxamide;
(2R,6S)-N-[2-(cyclohex-1-en-1-ylmethyl)-2-azaspiro[3.3]heptan-6-yl]-2,6-dimethyl-4-[5-(trifluoromethyl)pyrimidin-2-yl]piperazine-1-carboxamide;
(2R,6S)-2,6-dimethyl-N-{2-[(4-nitrophenyl)methyl]-2-azaspiro[3.3]heptan-6-yl}-4-[5-(trifluoromethyl)pyrimidin-2-yl]piperazine-1-carboxamide;
(2R,6S)-N-(2-{[4-(dimethylamino)phenyl]methyl}-2-azaspiro[3.3]heptan-6-yl)-2,6-dimethyl-4-[5-(trifluoromethyl)pyrimidin-2-yl]piperazine-1-carboxamide;
(2R,6S)-2,6-dimethyl-N-(2-{[4-(propan-2-yl)phenyl]methyl}-2-azaspiro[3.3]heptan-6-yl)-4-[5-(trifluoromethyl)pyrimidin-2-yl]piperazine-1-carboxamide;
(2R,6S)-2,6-dimethyl-N-[2-(pyridin-4-ylmethyl)-2-azaspiro[3.3]heptan-6-yl]-4-[5-(trifluoromethyl)pyrimidin-2-yl]piperazine-1-carboxamide;
(2R,6S)-N-{2-[(4-ethylphenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(4-hydroxyphenyl)methyl]-2-azaspiro[3.3]heptan-6-yl}-2,6-dimethyl-4-[5-(trifluoromethyl)pyrimidin-2-yl]piperazine-1-carboxamide;
methyl 4-[(6-{[(2R,6S)-2,6-dimethyl-4-[5-(trifluoromethyl)pyrimidin-2-yl]piperazine-1-carbonyl]amino}-2-azaspiro[3.3]heptan-2-yl)methyl]benzoate;
(2R,6S)-N-{2-[(4-acetamidophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-(2-{[4-(difluoromethoxy)phenyl]methyl}-2-azaspiro[3.3]heptan-6-yl)-2,6-dimethyl-4-[5-(trifluoromethyl)pyrimidin-2-yl]piperazine-1-carboxamide;
(2R,6S)-2,6-dimethyl-N-(2-{[4-(trifluoromethyl)phenyl]methyl}-2-azaspiro[3.3]heptan-6-yl)-4-[5-(trifluoromethyl)pyrimidin-2-yl]piperazine-1-carboxamide;
(2R,6S)-N-{2-[(4-carbamimidoylphenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-{2-[(4-chlorophenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-N-(2-{imidazo[1,2-a]pyridin-6-ylmethyl}-2-azaspiro[3.3]heptan-6-yl)-2,6-dimethyl-4-[5-(trifluoromethyl)pyrimidin-2-yl]piperazine-1-carboxamide;
(2R,6S)-N-[2-(adamantan-1-ylmethyl)-2-azaspiro[3.3]heptan-6-yl]-2,6-dimethyl-4-[5-(trifluoromethyl)pyrimidin-2-yl]piperazine-1-carboxamide;
(2R,6R)-N-{2-[(4-fluorophenyl)methyl]-2-azaspiro[3.3]heptan-6-yl}-2,6-dimethyl-4-[5-(trifluoromethyl)pyrimidin-2-yl]piperazine-1-carboxamide;
(2R,6R)-N-{2-[(4-carbamoylphenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-4-(5-cyanopyrimidin-2-yl)-N-{2-[(4-fluorophenyl)methyl]-2-azaspiro[3.3]heptan-6-yl}-2,6-dimethylpiperazine-1-carboxamide;
(2R,6S)-N-{2-[(4-carbamoylphenyl)methyl]-2-azaspiro[3.3]heptan-6-yl}-4-(5-cyanopyrimidin-2-yl)-2,6-dimethylpiperazine-1-carboxamide;
(2R,6S)-N-[2-(cyanomethyl)-2-azaspiro[3.3]heptan-6-yl]-2,6-dimethyl-4-[5-(trifluoromethyl)pyrimidin-2-yl]piperazine-1-carboxamide;
(2R,6S)-N-{2-[(3,3-difluorocyclobutyl)methyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-[2-(1-phenylethyl)-2-azaspiro[3.3]heptan-6-yl]-4-[5-(trifluoromethyl)pyrimidin-2-yl]piperazine-1-carboxamide;
(2R,6S)-2,6-dimethyl-N-[2-(1H-pyrrol-3-ylmethyl)-2-azaspiro[3.3]heptan-6-yl]-4-[5-(trifluoromethyl)pyrimidin-2-yl]piperazine-1-carboxamide;
(2R,6S)-N-(2-{[4-(dimethylcarbamoyl)phenyl]methyl}-2-azaspiro[3.3]heptan-6-yl)-2,6-dimethyl-4-[5-(trifluoromethyl)pyrimidin-2-yl]piperazine-1-carboxamide;
(2R,6S)-N-{2-[(2-hydroxyphenyl)methyl]-2-azaspiro[3.3]heptan-6-yl}-2,6-dimethyl-4-[5-(trifluoromethyl)pyrimidin-2-yl]piperazine-1-carboxamide;
(2R,6S)-2,6-dimethyl-N-{2-[(3-methylthiophen-2-yl)methyl]-2-azaspiro[3.3]heptan-6-yl}-4-[5-(trifluoromethyl)pyrimidin-2-yl]piperazine-1-carboxamide;
(2R,6S)-2,6-dimethyl-N-{2-[(5-methylthiophen-2-yl)methyl]-2-azaspiro[3.3]heptan-6-yl}-4-[5-(trifluoromethyl)pyrimidin-2-yl]piperazine-1-carboxamide;
(2R,6S)-N-{2-[(5-chlorothiophen-2-yl)methyl]-2-azaspiro[3.3]heptan-6-yl}-2,6-dimethyl-4-[5-(trifluoromethyl)pyrimidin-2-yl]piperazine-1-carboxamide;
2-benzyl-6-(difluoromethyl)-2-azaspiro[3.3]heptan-6-yl (2R,6S)-2,6-dimethyl-4-[5-(trifluoromethyl)pyrimidin-2-yl]piperazine-1-carboxylate;
2-benzyl-6-(difluoromethyl)-2-azaspiro[3.3]heptan-6-yl (2R,6S)-2,6-dimethyl-4-[5-(trifluoromethyl)pyrazin-2-yl]piperazine-1-carboxylate;
2-benzyl-6-(difluoromethyl)-2-azaspiro[3.3]heptan-6-yl (2R,6S)-2,6-dimethyl-4-[6-(trifluoromethyl)pyridazin-3-yl]piperazine-1-carboxylate;
2-benzyl-6-(difluoromethyl)-2-azaspiro[3.3]heptan-6-yl (2R,6R)-2,6-dimethyl-4-[5-(trifluoromethyl)pyrimidin-2-yl]piperazine-1-carboxylate;
2-benzyl-6-(difluoromethyl)-2-azaspiro[3.3]heptan-6-yl (2R,6R)-2,6-dimethyl-4-[5-(trifluoromethyl)pyrazin-2-yl]piperazine-1-carboxylate;
2-benzyl-6-(difluoromethyl)-2-azaspiro[3.3]heptan-6-yl (2R,5S)-2,5-dimethyl-4-[5-(trifluoromethyl)pyrimidin-2-yl]piperazine-1-carboxylate;
2-benzyl-6-(difluoromethyl)-2-azaspiro[3.3]heptan-6-yl (2R,5S)-2,5-dimethyl-4-[5-(trifluoromethyl)pyrazin-2-yl]piperazine-1-carboxylate;
2-benzyl-6-(difluoromethyl)-2-azaspiro[3.3]heptan-6-yl (2R)-4-(5-cyanopyrimidin-2-yl)-2-methylpiperazine-1-carboxylate;
2-benzyl-6-(difluoromethyl)-2-azaspiro[3.3]heptan-6-yl (2R)-2-methyl-4-[5-(trifluoromethyl)pyrimidin-2-yl]piperazine-1-carboxylate;
2-benzyl-6-(difluoromethyl)-2-azaspiro[3.3]heptan-6-yl (2R)-2-methyl-4-[5-(trifluoromethyl)pyrazin-2-yl]piperazine-1-carboxylate;
2-benzyl-6-methyl-2-azaspiro[3.3]heptan-6-yl (2R,6S)-4-(5-cyanopyrimidin-2-yl)-2,6-dimethylpiperazine-1-carboxylate;
2-benzyl-6-methyl-2-azaspiro[3.3]heptan-6-yl (2R,6S)-2,6-dimethyl-4-[5-(trifluoromethyl)pyrimidin-2-yl]piperazine-1-carboxylate;
2-benzyl-6-methyl-2-azaspiro[3.3]heptan-6-yl (2R,6S)-2,6-dimethyl-4-[5-(trifluoromethyl)pyrazin-2-yl]piperazine-1-carboxylate;
2-benzyl-6-methyl-2-azaspiro[3.3]heptan-6-yl (2R,6R)-4-(5-cyanopyrimidin-2-yl)-2,6-dimethylpiperazine-1-carboxylate;
2-benzyl-6-methyl-2-azaspiro[3.3]heptan-6-yl (2R,6R)-2,6-dimethyl-4-[5-(trifluoromethyl)pyrimidin-2-yl]piperazine-1-carboxylate;
2-benzyl-6-methyl-2-azaspiro[3.3]heptan-6-yl (2R,6R)-2,6-dimethyl-4-[5-(trifluoromethyl)pyrazin-2-yl]piperazine-1-carboxylate;
2-benzyl-6-methyl-2-azaspiro[3.3]heptan-6-yl (2R,5S)-4-(5-cyanopyrimidin-2-yl)-2,5-dimethylpiperazine-1-carboxylate;
2-benzyl-6-methyl-2-azaspiro[3.3]heptan-6-yl (2R,5S)-2,5-dimethyl-4-[5-(trifluoromethyl)pyrimidin-2-yl]piperazine-1-carboxylate;
2-benzyl-6-methyl-2-azaspiro[3.3]heptan-6-yl (2R,5S)-2,5-dimethyl-4-[5-(trifluoromethyl)pyrazin-2-yl]piperazine-1-carboxylate;
2-[(²H₅)phenyl(²H₁)methyl]-2-azaspiro[3.3]heptan-6-yl (2R,5S)-2,5-dimethyl-4-[5-(trifluoromethyl)pyrazin-2-yl]piperazine-1-carboxylate;
2-[(²H₅)phenyl(²H₂)methyl]-2-azaspiro[3.3]heptan-6-yl (2R,5S)-2,5-dimethyl-4-[5-(trifluoromethyl)pyrazin-2-yl]piperazine-1-carboxylate;
2-benzyl-2-azaspiro[3.3]heptan-6-yl (2R,6R)-4-(6-fluoroquinoxalin-2-yl)-2,6-dimethylpiperazine-1-carboxylate;
2-benzyl-2-azaspiro[3.3]heptan-6-yl (2R,5S)-4-(6-fluoroquinoxalin-2-yl)-2,5-dimethylpiperazine-1-carboxylate;
2-benzyl-2-azaspiro[3.3]heptan-6-yl (2R,6S)-2,6-dimethyl-4-[6-(trifluoromethyl)-1,3-benzothiazol-2-yl]piperazine-1-carboxylate;
2-benzyl-2-azaspiro[3.3]heptan-6-yl (2R,6S)-2,6-dimethyl-4-[6-(trifluoromethyl)-[1,3]thiazolo[5,4-b]pyridin-2-yl]piperazine-1-carboxylate;
2-benzyl-2-azaspiro[3.3]heptan-6-yl (2R,6R)-2,6-dimethyl-4-[6-(trifluoromethyl)-[1,3]thiazolo[5,4-b]pyridin-2-yl]piperazine-1-carboxylate;
2-benzyl-2-azaspiro[3.3]heptan-6-yl (2R,5S)-2,5-dimethyl-4-[6-(trifluoromethyl)-[1,3]thiazolo[5,4-b]pyridin-2-yl]piperazine-1-carboxylate;
2-benzyl-2-azaspiro[3.3]heptan-6-yl (2R,6S)-4-(6-fluoro-1,3-benzothiazol-2-yl)-2,6-dimethylpiperazine-1-carboxylate;
2-benzyl-2-azaspiro[3.3]heptan-6-yl (2R,6R)-4-(6-fluoro-1,3-benzothiazol-2-yl)-2,6-dimethylpiperazine-1-carboxylate;
2-benzyl-2-azaspiro[3.3]heptan-6-yl (2R,5S)-4-(6-fluoro-1,3-benzothiazol-2-yl)-2,5-dimethylpiperazine-1-carboxylate;
2-benzyl-2-azaspiro[3.3]heptan-6-yl (2R,6R)-2,6-dimethyl-4-[6-(trifluoromethyl)-1,3-benzothiazol-2-yl]piperazine-1-carboxylate;
2-benzyl-2-azaspiro[3.3]heptan-6-yl (2R,5S)-2,5-dimethyl-4-[6-(trifluoromethyl)-1,3-benzothiazol-2-yl]piperazine-1-carboxylate;
2-[(²H₅)benzyl]-2-azaspiro[3.3]heptan-6-yl (2R,6S)-2,6-dimethyl-4-[5-(trifluoromethyl)pyrimidin-2-yl]piperazine-1-carboxylate;
2-[(²H₅)benzyl]-2-azaspiro[3.3]heptan-6-yl (2R,6S)-2,6-dimethyl-4-[5-(trifluoromethyl)pyrazin-2-yl]piperazine-1-carboxylate;
2-[(²H₅)phenyl(²H₁)methyl]-2-azaspiro[3.3]heptan-6-yl (2R,6S)-2,6-dimethyl-4-[5-(trifluoromethyl)pyrimidin-2-yl]piperazine-1-carboxylate;
2-[(²H₅)phenyl(²H₁)methyl]-2-azaspiro[3.3]heptan-6-yl (2R,6S)-2,6-dimethyl-4-[5-(trifluoromethyl)pyrazin-2-yl]piperazine-1-carboxylate;
2-benzyl-2-azaspiro[3.3]heptan-6-yl (2R,6S)-2,6-dimethyl-4-(quinazolin-2-yl)piperazine-1-carboxylate;
2-benzyl-2-azaspiro[3.3]heptan-6-yl (2R,6S)-2,6-dimethyl-4-[6-(trifluoromethyl)quinoxalin-2-yl]piperazine-1-carboxylate;
2-benzyl-2-azaspiro[3.3]heptan-6-yl (2R,6S)-4-(6-methoxyquinoxalin-2-yl)-2,6-dimethylpiperazine-1-carboxylate;
2-benzyl-2-azaspiro[3.3]heptan-6-yl (2R,5S)-2,5-dimethyl-4-[6-(trifluoromethyl)quinoxalin-2-yl]piperazine-1-carboxylate;
2-[(4-fluorophenyl)methyl]-2-azaspiro[3.3]heptan-6-yl (2R,6S)-4-(6-fluoro-1,3-benzothiazol-2-yl)-2,6-dimethylpiperazine-1-carboxylate;
2-[(4-fluorophenyl)methyl]-2-azaspiro[3.3]heptan-6-yl (2R,6R)-4-(6-fluoro-1,3-benzothiazol-2-yl)-2,6-dimethylpiperazine-1-carboxylate;
2-[(4-fluorophenyl)methyl]-2-azaspiro[3.3]heptan-6-yl (2R,5S)-4-(6-fluoro-1,3-benzothiazol-2-yl)-2,5-dimethylpiperazine-1-carboxylate;
2-[(4-fluorophenyl)methyl]-2-azaspiro[3.3]heptan-6-yl (2R,6S)-2,6-dimethyl-4-[6-(trifluoromethyl)-1,3-benzothiazol-2-yl]piperazine-1-carboxylate;
2-[(4-fluorophenyl)methyl]-2-azaspiro[3.3]heptan-6-yl (2R,6R)-2,6-dimethyl-4-[6-(trifluoromethyl)-1,3-benzothiazol-2-yl]piperazine-1-carboxylate;
2-[(4-fluorophenyl)methyl]-2-azaspiro[3.3]heptan-6-yl (2R,5S)-2,5-dimethyl-4-[6-(trifluoromethyl)-1,3-benzothiazol-2-yl]piperazine-1-carboxylate;
2-[(²H₅)phenyl(²H₂)methyl]-2-azaspiro[3.3]heptan-6-yl (2R,6S)-2,6-dimethyl-4-[5-(trifluoromethyl)pyrimidin-2-yl]piperazine-1-carboxylate;
2-[(²H₅)phenyl(²H₂)methyl]-2-azaspiro[3.3]heptan-6-yl (2R,6S)-2,6-dimethyl-4-[5-(trifluoromethyl)pyrazin-2-yl]piperazine-1-carboxylate;
2-[(4-fluorophenyl)methyl]-2-azaspiro[3.3]heptan-6-yl (2R,6S)-2,6-dimethyl-4-(quinoxalin-2-yl)piperazine-1-carboxylate;
2-[(4-fluorophenyl)methyl]-2-azaspiro[3.3]heptan-6-yl (2R,6S)-2,6-dimethyl-4-(quinazolin-2-yl)piperazine-1-carboxylate;
2-[(4-fluorophenyl)methyl]-2-azaspiro[3.3]heptan-6-yl (2R,6S)-2,6-dimethyl-4-[6-(trifluoromethyl)quinoxalin-2-yl]piperazine-1-carboxylate;
2-[(4-fluorophenyl)methyl]-2-azaspiro[3.3]heptan-6-yl (2R,5S)-2,5-dimethyl-4-(quinoxalin-2-yl)piperazine-1-carboxylate;
2-[(4-fluorophenyl)methyl]-2-azaspiro[3.3]heptan-6-yl (2R,6S)-4-(6-methoxyquinoxalin-2-yl)-2,6-dimethylpiperazine-1-carboxylate;
2-[(4-fluorophenyl)methyl]-2-azaspiro[3.3]heptan-6-yl (2R,5S)-4-(6-methoxyquinoxalin-2-yl)-2,5-dimethylpiperazine-1-carboxylate;
2-[(4-fluorophenyl)methyl]-2-azaspiro[3.3]heptan-6-yl (2R,5S)-4-(6-fluoroquinoxalin-2-yl)-2,5-dimethylpiperazine-1-carboxylate;
2-[(4-fluorophenyl)methyl]-2-azaspiro[3.3]heptan-6-yl (2R,6R)-2,6-dimethyl-4-[5-(trifluoromethyl)pyrazin-2-yl]piperazine-1-carboxylate;
2-[(4-fluorophenyl)methyl]-2-azaspiro[3.3]heptan-6-yl (2R,6S)-2,6-dimethyl-4-[5-(trifluoromethyl)pyrazin-2-yl]piperazine-1-carboxylate;
2-[(4-fluorophenyl)methyl]-2-azaspiro[3.3]heptan-6-yl (2R,5S)-2,5-dimethyl-4-[5-(trifluoromethyl)pyrazin-2-yl]piperazine-1-carboxylate;
2-[(4-carbamoylphenyl)methyl]-2-azaspiro[3.3]heptan-6-yl (2R,6S)-4-(6-fluoro-1,3-benzothiazol-2-yl)-2,6-dimethylpiperazine-1-carboxylate;
2-[(4-carbamoylphenyl)methyl]-2-azaspiro[3.3]heptan-6-yl (2R,6R)-4-(6-fluoro-1,3-benzothiazol-2-yl)-2,6-dimethylpiperazine-1-carboxylate;
2-[(4-carbamoylphenyl)methyl]-2-azaspiro[3.3]heptan-6-yl (2R,6S)-2,6-dimethyl-4-[6-(trifluoromethyl)quinoxalin-2-yl]piperazine-1-carboxylate;
2-[(4-carbamoylphenyl)methyl]-2-azaspiro[3.3]heptan-6-yl (2R,5S)-2,5-dimethyl-4-(quinoxalin-2-yl)piperazine-1-carboxylate;
2-[(4-carbamoylphenyl)methyl]-2-azaspiro[3.3]heptan-6-yl (2R,6S)-4-(6-methoxyquinoxalin-2-yl)-2,6-dimethylpiperazine-1-carboxylate;
2-[(4-carbamoylphenyl)methyl]-2-azaspiro[3.3]heptan-6-yl (2R,5S)-4-(6-methoxyquinoxalin-2-yl)-2,5-dimethylpiperazine-1-carboxylate;
2-[(4-carbamoylphenyl)methyl]-2-azaspiro[3.3]heptan-6-yl (2R,6R)-2,6-dimethyl-4-[5-(trifluoromethyl)pyrazin-2-yl]piperazine-1-carboxylate;
2-[(4-carbamoylphenyl)methyl]-2-azaspiro[3.3]heptan-6-yl (2R,6S)-2,6-dimethyl-4-[5-(trifluoromethyl)pyrazin-2-yl]piperazine-1-carboxylate;
2-[(4-carbamoylphenyl)methyl]-2-azaspiro[3.3]heptan-6-yl (2R,5S)-2,5-dimethyl-4-[5-(trifluoromethyl)pyrazin-2-yl]piperazine-1-carboxylate;
2-(pyridin-4-ylmethyl)-2-azaspiro[3.3]heptan-6-yl (2R,6S)-4-(6-fluoro-1,3-benzothiazol-2-yl)-2,6-dimethylpiperazine-1-carboxylate;
2-(pyridin-4-ylmethyl)-2-azaspiro[3.3]heptan-6-yl (2R,6R)-4-(6-fluoro-1,3-benzothiazol-2-yl)-2,6-dimethylpiperazine-1-carboxylate;
2-(pyridin-4-ylmethyl)-2-azaspiro[3.3]heptan-6-yl (2R,6S)-2,6-dimethyl-4-[6-(trifluoromethyl)-1,3-benzothiazol-2-yl]piperazine-1-carboxylate;
2-(pyridin-4-ylmethyl)-2-azaspiro[3.3]heptan-6-yl (2R,6S)-2,6-dimethyl-4-(quinazolin-2-yl)piperazine-1-carboxylate;
2-(pyridin-4-ylmethyl)-2-azaspiro[3.3]heptan-6-yl (2R,6S)-4-(6-methoxyquinoxalin-2-yl)-2,6-dimethylpiperazine-1-carboxylate;
2-(pyridin-4-ylmethyl)-2-azaspiro[3.3]heptan-6-yl (2R,5S)-4-(6-methoxyquinoxalin-2-yl)-2,5-dimethylpiperazine-1-carboxylate;
2-(pyridin-4-ylmethyl)-2-azaspiro[3.3]heptan-6-yl (2R,5S)-4-(6-fluoroquinoxalin-2-yl)-2,5-dimethylpiperazine-1-carboxylate;
2-(pyridin-4-ylmethyl)-2-azaspiro[3.3]heptan-6-yl (2R,6R)-2,6-dimethyl-4-[5-(trifluoromethyl)pyrazin-2-yl]piperazine-1-carboxylate;
2-(pyridin-4-ylmethyl)-2-azaspiro[3.3]heptan-6-yl (2R,6S)-2,6-dimethyl-4-[5-(trifluoromethyl)pyrazin-2-yl]piperazine-1-carboxylate;
2-(pyridin-4-ylmethyl)-2-azaspiro[3.3]heptan-6-yl (2R,5S)-2,5-dimethyl-4-[5-(trifluoromethyl)pyrazin-2-yl]piperazine-1-carboxylate;
2-benzyl-2-azaspiro[3.3]heptan-6-yl (2R,6S)-2,6-dimethyl-4-[6-(trifluoromethyl)-1,3-benzoxazol-2-yl]piperazine-1-carboxylate;
2-benzyl-2-azaspiro[3.3]heptan-6-yl (2R,6R)-2,6-dimethyl-4-[6-(trifluoromethyl)-1,3-benzoxazol-2-yl]piperazine-1-carboxylate;
2-benzyl-2-azaspiro[3.3]heptan-6-yl (2R,5S)-2,5-dimethyl-4-[6-(trifluoromethyl)-1,3-benzoxazol-2-yl]piperazine-1-carboxylate;
2-benzyl-2-azaspiro[3.3]heptan-6-yl (2R,6S)-2,6-dimethyl-4-[6-(trifluoromethyl)-[1,3]thiazolo[4,5-b]pyridin-2-yl]piperazine-1-carboxylate;
2-benzyl-2-azaspiro[3.3]heptan-6-yl (2R,6R)-2,6-dimethyl-4-[6-(trifluoromethyl)-[1,3]thiazolo[4,5-b]pyridin-2-yl]piperazine-1-carboxylate;
2-benzyl-2-azaspiro[3.3]heptan-6-yl (2R,5S)-2,5-dimethyl-4-[6-(trifluoromethyl)-[1,3]thiazolo[4,5-b]pyridin-2-yl]piperazine-1-carboxylate;
2-benzyl-2-azaspiro[3.3]heptan-6-yl (2R,6S)-4-(6-cyano-1,3-benzothiazol-2-yl)-2,6-dimethylpiperazine-1-carboxylate;
2-benzyl-2-azaspiro[3.3]heptan-6-yl (2R,5S)-4-(6-cyano-1,3-benzothiazol-2-yl)-2,5-dimethylpiperazine-1-carboxylate;
2-benzyl-2-azaspiro[3.3]heptan-6-yl 7-(6-fluoro-1,3-benzothiazol-2-yl)-3-oxa-7,9-diazabicyclo[3.3.1]nonane-9-carboxylate;
2-benzyl-2-azaspiro[3.3]heptan-6-yl 7-[6-(trifluoromethyl)-1,3-benzothiazol-2-yl]-3-oxa-7,9-diazabicyclo[3.3.1]nonane-9-carboxylate;
2-benzyl-2-azaspiro[3.3]heptan-6-yl (2R,6S)-4-(5-fluoro-1,3-benzothiazol-2-yl)-2,6-dimethylpiperazine-1-carboxylate;
2-benzyl-2-azaspiro[3.3]heptan-6-yl (2R,6R)-4-(5-fluoro-1,3-benzothiazol-2-yl)-2,6-dimethylpiperazine-1-carboxylate;
2-benzyl-2-azaspiro[3.3]heptan-6-yl (2R,5S)-4-(5-fluoro-1,3-benzothiazol-2-yl)-2,5-dimethylpiperazine-1-carboxylate;
2-benzyl-2-azaspiro[3.3]heptan-6-yl (2R,6S)-4-(5-fluoro-1,3-benzoxazol-2-yl)-2,6-dimethylpiperazine-1-carboxylate;
2-benzyl-2-azaspiro[3.3]heptan-6-yl (2R,6R)-4-(5-fluoro-1,3-benzoxazol-2-yl)-2,6-dimethylpiperazine-1-carboxylate;
2-benzyl-2-azaspiro[3.3]heptan-6-yl (2R,5S)-4-(5-fluoro-1,3-benzoxazol-2-yl)-2,5-dimethylpiperazine-1-carboxylate;
2-benzyl-2-azaspiro[3.3]heptan-6-yl (2R,6S)-4-(6-fluoro-1,3-benzoxazol-2-yl)-2,6-dimethylpiperazine-1-carboxylate;
2-benzyl-2-azaspiro[3.3]heptan-6-yl (2R,6R)-4-(6-fluoro-1,3-benzoxazol-2-yl)-2,6-dimethylpiperazine-1-carboxylate;
2-benzyl-2-azaspiro[3.3]heptan-6-yl (2R,5S)-4-(6-fluoro-1,3-benzoxazol-2-yl)-2,5-dimethylpiperazine-1-carboxylate;
2-benzyl-2-azaspiro[3.3]heptan-6-yl (2R,6S)-4-(5,6-difluoro-1,3-benzothiazol-2-yl)-2,6-dimethylpiperazine-1-carboxylate;
2-benzyl-2-azaspiro[3.3]heptan-6-yl (2R,6R)-4-(5,6-difluoro-1,3-benzothiazol-2-yl)-2,6-dimethylpiperazine-1-carboxylate;
2-benzyl-2-azaspiro[3.3]heptan-6-yl (2R,5S)-4-(5,6-difluoro-1,3-benzothiazol-2-yl)-2,5-dimethylpiperazine-1-carboxylate;
2-benzyl-2-azaspiro[3.3]heptan-6-yl (2R,6S)-4-{6-fluoro-[1,3]thiazolo[5,4-b]pyridin-2-yl}-2,6-dimethylpiperazine-1-carboxylate;
2-benzyl-2-azaspiro[3.3]heptan-6-yl (2R,6S)-4-{6-fluoro-[1,3]thiazolo[4,5-b]pyridin-2-yl}-2,6-dimethylpiperazine-1-carboxylate;
2-benzyl-2-azaspiro[3.3]heptan-6-yl (2R,6S)-4-{5-fluoro-[1,3]thiazolo[5,4-b]pyridin-2-yl}-2,6-dimethylpiperazine-1-carboxylate;
2-benzyl-2-azaspiro[3.3]heptan-6-yl (2R,6S)-4-(6-fluoroquinazolin-2-yl)-2,6-dimethylpiperazine-1-carboxylate;
2-benzyl-2-azaspiro[3.3]heptan-6-yl (2R,6S)-2,6-dimethyl-4-{pyrido[2,3-b]pyrazin-3-yl}piperazine-1-carboxylate;
2-[(4-fluorophenyl)methyl]-2-azaspiro[3.3]heptan-6-yl (2R,6S)-2,6-dimethyl-4-[5-(trifluoromethyl)pyrimidin-2-yl]piperazine-1-carboxylate;
2-[(4-fluorophenyl)methyl]-2-azaspiro[3.3]heptan-6-yl (2R,5S)-2,5-dimethyl-4-[5-(trifluoromethyl)pyrimidin-2-yl]piperazine-1-carboxylate;
2-[(4-fluorophenyl)methyl]-2-azaspiro[3.3]heptan-6-yl (2R)-2-methyl-4-[5-(trifluoromethyl)pyrimidin-2-yl]piperazine-1-carboxylate;
2-[(4-fluorophenyl)methyl]-2-azaspiro[3.3]heptan-6-yl (2R)-2-methyl-4-[5-(trifluoromethyl)pyrazin-2-yl]piperazine-1-carboxylate;
2-benzyl-2-azaspiro[3.3]heptan-6-yl (2R,6S)-2,6-dimethyl-4-(1,6-naphthyridin-2-yl)piperazine-1-carboxylate;
2-benzyl-2-azaspiro[3.3]heptan-6-yl (2R,6S)-2,6-dimethyl-4-{pyrido[2,3-b]pyrazin-6-yl}piperazine-1-carboxylate;
2-[(4-fluorophenyl)methyl]-2-azaspiro[3.3]heptan-6-yl 7-[5-(trifluoromethyl)pyrimidin-2-yl]-3-oxa-7,9-diazabicyclo[3.3.1]nonane-9-carboxylate;
2-benzyl-2-azaspiro[3.3]heptan-6-yl (2R,6S)-4-[5-(difluoromethoxy)pyrimidin-2-yl]-2,6-dimethylpiperazine-1-carboxylate;
2-benzyl-2-azaspiro[3.3]heptan-6-yl (2R,6S)-4-[4-amino-5-(trifluoromethyl)pyrimidin-2-yl]-2,6-dimethylpiperazine-1-carboxylate;
2-benzyl-2-azaspiro[3.3]heptan-6-yl (2R,6S)-4-[5-(difluoromethyl)pyrimidin-2-yl]-2,6-dimethylpiperazine-1-carboxylate;
2-[(4-fluorophenyl)methyl]-2-azaspiro[3.3]heptan-6-yl (2R,6S)-4-[5-(difluoromethoxy)pyrimidin-2-yl]-2,6-dimethylpiperazine-1-carboxylate;
2-[(4-fluorophenyl)methyl]-2-azaspiro[3.3]heptan-6-yl (2R,6S)-4-[4-amino-5-(trifluoromethyl)pyrimidin-2-yl]-2,6-dimethylpiperazine-1-carboxylate;
2-[(4-fluorophenyl)methyl]-2-azaspiro[3.3]heptan-6-yl (2R,6S)-4-[5-(difluoromethyl)pyrimidin-2-yl]-2,6-dimethylpiperazine-1-carboxylate;
2-[(4-fluorophenyl)methyl]-2-azaspiro[3.3]heptan-6-yl (2R,6S)-4-(5-fluoropyrimidin-2-yl)-2,6-dimethylpiperazine-1-carboxylate;
2-benzyl-2-azaspiro[3.3]heptan-6-yl (2R,6S)-2,6-dimethyl-4-[2-(trifluoromethyl)pyrimidin-5-yl]piperazine-1-carboxylate;
2-benzyl-2-azaspiro[3.3]heptan-6-yl (2R,6S)-2,6-dimethyl-4-[5-(methylcarbamoyl)pyrimidin-2-yl]piperazine-1-carboxylate;
2-[(4-fluorophenyl)methyl]-2-azaspiro[3.3]heptan-6-yl (2R,6S)-4-(5-cyanopyrimidin-2-yl)-2,6-dimethylpiperazine-1-carboxylate;
2-[(4-fluorophenyl)methyl]-2-azaspiro[3.3]heptan-6-yl (2R,5S)-4-(5-cyanopyrimidin-2-yl)-2,5-dimethylpiperazine-1-carboxylate;
2-[(4-fluorophenyl)methyl]-2-azaspiro[3.3]heptan-6-yl (2R)-4-(5-cyanopyrimidin-2-yl)-2-methylpiperazine-1-carboxylate;
2-[(4-chlorophenyl)methyl]-2-azaspiro[3.3]heptan-6-yl (2R,6S)-4-(5-cyanopyrimidin-2-yl)-2,6-dimethylpiperazine-1-carboxylate;
2-[(4-chlorophenyl)methyl]-2-azaspiro[3.3]heptan-6-yl (2R,5S)-4-(5-cyanopyrimidin-2-yl)-2,5-dimethylpiperazine-1-carboxylate;
2-[(4-chlorophenyl)methyl]-2-azaspiro[3.3]heptan-6-yl (2R,6S)-2,6-dimethyl-4-[5-(trifluoromethyl)pyrimidin-2-yl]piperazine-1-carboxylate;
2-[(4-chlorophenyl)methyl]-2-azaspiro[3.3]heptan-6-yl (2R)-2-methyl-4-[5-(trifluoromethyl)pyrimidin-2-yl]piperazine-1-carboxylate;
2-[(4-chlorophenyl)methyl]-2-azaspiro[3.3]heptan-6-yl (2R,6S)-2,6-dimethyl-4-[5-(trifluoromethyl)pyrazin-2-yl]piperazine-1-carboxylate;
2-[(4-chlorophenyl)methyl]-2-azaspiro[3.3]heptan-6-yl (2R,5S)-2,5-dimethyl-4-[5-(trifluoromethyl)pyrazin-2-yl]piperazine-1-carboxylate;
2-[(4-chlorophenyl)methyl]-2-azaspiro[3.3]heptan-6-yl (2R)-2-methyl-4-[5-(trifluoromethyl)pyrazin-2-yl]piperazine-1-carboxylate;
2-[(4-chlorophenyl)methyl]-2-azaspiro[3.3]heptan-6-yl (2R,5S)-2,5-dimethyl-4-[5-(trifluoromethyl)pyrimidin-2-yl]piperazine-1-carboxylate;
2-[(4-chlorophenyl)methyl]-2-azaspiro[3.3]heptan-6-yl (2R)-4-(5-cyanopyrimidin-2-yl)-2-methylpiperazine-1-carboxylate;
2-benzyl-2-azaspiro[3.3]heptan-6-yl (2R,5S)-4-(6-fluoroquinazolin-2-yl)-2,5-dimethylpiperazine-1-carboxylate;
2-benzyl-2-azaspiro[3.3]heptan-6-yl (1R,4R)-5-[5-(trifluoromethyl)pyrimidin-2-yl]-2,5-diazabicyclo[2.2.1]heptane-2-carboxylate;
2-benzyl-2-azaspiro[3.3]heptan-6-yl (2R,6S)-4-[5-(dimethylcarbamoyl)pyrimidin-2-yl]-2,6-dimethylpiperazine-1-carboxylate;
2-benzyl-2-azaspiro[3.3]heptan-6-yl 9-[5-(trifluoromethyl)pyrimidin-2-yl]-3-oxa-7,9-diazabicyclo[3.3.1]nonane-7-carboxylate;
2-benzyl-2-azaspiro[3.3]heptan-6-yl 9-[5-(trifluoromethyl)pyrazin-2-yl]-3-oxa-7,9-diazabicyclo[3.3.1]nonane-7-carboxylate;
2-benzyl-2-azaspiro[3.3]heptan-6-yl (2R,6S)-4-(5-ethylpyrimidin-2-yl)-2,6-dimethylpiperazine-1-carboxylate;
2-benzyl-2-azaspiro[3.3]heptan-6-yl (2R,6S)-2,6-dimethyl-4-[5-(trifluoromethoxy)pyrimidin-2-yl]piperazine-1-carboxylate;
2-benzyl-2-azaspiro[3.3]heptan-6-yl (2R,6S)-4-[5-(cyclopropylmethoxy)pyrimidin-2-yl]-2,6-dimethylpiperazine-1-carboxylate;
2-[(4-methanesulfonylphenyl)methyl]-2-azaspiro[3.3]heptan-6-yl (2R,6S)-2,6-dimethyl-4-[5-(trifluoromethyl)pyrimidin-2-yl]piperazine-1-carboxylate;
2-[(4-methanesulfonylphenyl)methyl]-2-azaspiro[3.3]heptan-6-yl (2R,5S)-2,5-dimethyl-4-[5-(trifluoromethyl)pyrimidin-2-yl]piperazine-1-carboxylate;
2-[(4-methanesulfonylphenyl)methyl]-2-azaspiro[3.3]heptan-6-yl (2R,6S)-2,6-dimethyl-4-[5-(trifluoromethyl)pyrazin-2-yl]piperazine-1-carboxylate;
2-[(4-methanesulfonylphenyl)methyl]-2-azaspiro[3.3]heptan-6-yl (2R,5S)-2,5-dimethyl-4-[5-(trifluoromethyl)pyrazin-2-yl]piperazine-1-carboxylate;
2-benzyl-2-azaspiro[3.3]heptan-6-yl (2R,6S)-2,6-dimethyl-4-(5-propylpyrimidin-2-yl)piperazine-1-carboxylate;
2-benzyl-2-azaspiro[3.3]heptan-6-yl (2R,6S)-2,6-dimethyl-4-[5-(propan-2-yl)pyrimidin-2-yl]piperazine-1-carboxylate;
2-benzyl-2-azaspiro[3.3]heptan-6-yl (2R,6S)-4-(5-cyclopropylpyrimidin-2-yl)-2,6-dimethylpiperazine-1-carboxylate;
2-benzyl-2-azaspiro[3.3]heptan-6-yl (2R,6S)-4-(5-ethynylpyrimidin-2-yl)-2,6-dimethylpiperazine-1-carboxylate;
2-benzyl-2-azaspiro[3.3]heptan-6-yl (2R,6S)-4-[5-(dimethylphosphoryl)pyridin-2-yl]-2,6-dimethylpiperazine-1-carboxylate;
2-benzyl-2-azaspiro[3.3]heptan-6-yl (2R,6S)-2,6-dimethyl-4-[4-(trifluoromethyl)phenyl]piperazine-1-carboxylate;
2-benzyl-2-azaspiro[3.3]heptan-6-yl (2R,6S)-4-(4-ethoxyphenyl)-2,6-dimethylpiperazine-1-carboxylate;
2-benzyl-2-azaspiro[3.3]heptan-6-yl (2R,6S)-4-(4-methoxyphenyl)-2,6-dimethylpiperazine-1-carboxylate;
2-benzyl-2-azaspiro[3.3]heptan-6-yl (2R,6S)-4-(4-fluoro-3-methylphenyl)-2,6-dimethylpiperazine-1-carboxylate;
2-benzyl-2-azaspiro[3.3]heptan-6-yl (2R,6S)-4-(4-cyano-3-methoxyphenyl)-2,6-dimethylpiperazine-1-carboxylate;
2-benzyl-2-azaspiro[3.3]heptan-6-yl (2R,5S)-2,5-dimethyl-4-[4-(trifluoromethyl)phenyl]piperazine-1-carboxylate;
2-benzyl-2-azaspiro[3.3]heptan-6-yl (2R)-2-methyl-4-[4-(trifluoromethyl)phenyl]piperazine-1-carboxylate;
2-benzyl-2-azaspiro[3.3]heptan-6-yl (2R,6S)-4-[5-(dimethylphosphoryl)pyrimidin-2-yl]-2,6-dimethylpiperazine-1-carboxylate; and
2-benzyl-2-azaspiro[3.3]heptan-6-yl (2R,6S)-4-[5-(cyclobutylmethoxy)pyrimidin-2-yl]-2,6-dimethylpiperazine-1-carboxylate.

12. A pharmaceutical composition comprising a compound according to any one of claims 1-11, or a pharmaceutically acceptable salt thereof, and a pharmaceutically acceptable carrier.

13. The compound or salt according to any one of claims 1-11, for use in a method for treating or preventing a muscarinic receptor 4 (M₄) mediated disease, disorder, or symptom in an individual.

14. The compound or salt according to any one of claims 1-11, for use in a method for treating or preventing a neurological disease, disorder, or symptom in an individual;
wherein the disease, disorder, or symptom is selected from: Tourette's syndrome (TS), Alzheimer's Disease (AD), schizophrenia, Lewy Body Dementia (LBD), cognitive deficits associated with schizophrenia, Parkinson's Disease, parkinsonism, tremor, dyskinesias, excessive daytime sleepiness, dystonia, chorea, levodopa induced dyskinesia, attention deficit hyperactivity disorder (ADHD), cerebral palsy, progressive supranuclear palsy (PSP), Multiple System Atrophy (MSA), Huntington's disease (HD), and chorea associate with Huntington's disease.

15. The compound or salt for use according to claim 14, wherein:
the disease, disorder, or symptom is parkinsonism, tremor, or dystonia.

## Patentansprüche

1. Verbindung, ausgewählt aus Formel (la): 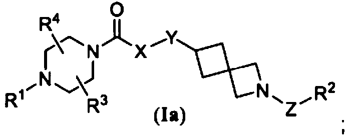 oder ein pharmazeutisch annehmbares Salz davon:
worin:
X O oder NH ist;
Y CH₂ ist oder fehlt;
Z C₁₋₄-Alkylen ist oder fehlt;
R¹ C₆₋₁₀-Aryl oder 5- bis 10-gliedriges Heteroaryl ist, die jeweils gegebenenfalls mit einer oder mehreren Gruppen substituiert sind, die aus den folgenden ausgewählt sind: C₁₋₆-Alkoxy, C₁₋₆-Alkoxycarbonyl, C₁₋₆-Alkyl, C₁₋₆-Alkylamino, C₁₋₆-Alkylcarbonyl, C₁₋₆-Alkylcarbamoyl, C₁₋₆-Alkylsulfanyl, C₂₋₆-Dialkylamino, C₂₋₆-Dialkylcarbamoyl, C₁₋₆-Sulfinyl, C₃₋₁₀-Cycloalkyl, C₁₋₆-Halogenalkoxy, C₁₋₆-Halogenalkyl, Amino, Carbamoyl, Cyano, Halogen und Nitro;
R² aus Folgendem ausgewählt ist: C₆₋₁₀-Aryl, C₁₋₆-Alkyl, C₃₋₇-Cycloalkyl, C₅₋₈-Bicycloalkanyl, C₆₋₈-Bicycloalkenyl, 5- bis 10-gliedrigem Heteroaryl und Heterocyclyl; die jeweils gegebenenfalls mit einer oder mehreren Gruppen substituiert ist, die aus den folgenden ausgewählt sind: C₁₋₄-Alkyl, C₁₋₆-Alkoxy, C₁₋₆-Halogenalkoxy, C₁₋₆-Sulfonyl, Carbamoyl, Cyano und Halogen; und
a) R³ und R⁴ jeweils unabhängig aus H und C₁₋₄-Alkyl ausgewählt sind; oder
b) R³ und R⁴ zusammen eine Brückengruppe bilden die ausgewählt ist aus: CH₂, CH₂-CH₂ und CH₂-O-CH₂; und
c) R³ und R⁴ an unterschiedliche Ethylengruppen des Piperazinrings gebunden sind;
oder eine Verbindung, die aus der aus den folgenden bestehenden Gruppe ausgewählt ist:
(2R,6S)-N-[2-(Cyclohex-1-en-1-ylmethyl)-2-azaspiro[3.3]heptan-6-yl]-2,6-dimethyl-4-[5-(trifluormethyl)pyrimidin-2-yl]piperazin-1-carboxamid;
(2R,6S)-2,6-Dimethyl-N-{2-[(4-nitrophenyl)methyl]-2-azaspiro[3.3]heptan-6-yl}-4-[5-(trifluormethyl)pyrimidin-2-yl]piperazin-1-carboxamid;
(2R,6S)-N-(2-[(4-Hydroxyphenyl)methyl]-2-azaspiro[3.3]heptan-6-yl)-2,6-dimethyl-4-[5-(trifluormethyl)pyrimidin-2-yl]piperazin-1-carboxamid;
Methyl-4-[(6-{[(2R,6S)-2,6-dimethyl-4-[5-(trifluormethyl)pyrimidin-2-yl]piperazin-1-carbonyl]-amino}-2-azaspiro[3.3]heptan-2-yl)methyl]benzoat;
(2R,6S)-N-(2-[(4-Acetamidophenyl)methyl]-2-azaspiro[3.3]heptan-6-yl}-2,6-dimethyl-4-[5-(trifluormethyl)pyrimidin-2-yl]piperazin-1-carboxamid;
(2R,6S)-2,6-Dimethyl-N-(2-{[4-(trifluormethyl)phenyl]methyl)-2-azaspiro[3.3]heptan-6-yl)-4-[5-(trifluormethyl)pyrimidin-2-yl]piperazin-1-carboxamid;
(2R,6S)-N-{2-[(4-Carbamimidoylphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-[2-(Adamantan-1-ylmethyl)-2-azaspiro[3.3]heptan-6-yl]-2,6-dimethyl-4-[5-(trifluormethyl)pyrimidin-2-yl]piperazin-1-carboxamid;
(2R,6S)-N-(2-{[4-(Dimethylcarbamoyl)phenyl]methyl}-2-azaspiro[3.3]heptan-6-yl)-2,6-dimethyl-4-[5-(trifluormethyl)pyrimidin-2-yl]piperazin-1-carboxamid;
2-Benzyl-6-(difluormethyl)-2-azaspiro[3.3]heptan-6-yl-(2R,6S)-2,6-dimethyl-4-[5-(trifluormethyl)pyrimidin-2-yl]piperazin-1-carboxylat;
2-Benzyl-6-(difluormethyl)-2-azaspiro[3.3]heptan-6-yl-(2R,6S)-2,6-dimethyl-4-[5-(trifluormethyl)pyrazin-2-yl]piperazin-1-carboxylat;
2-Benzyl-6-(difluormethyl)-2-azaspiro[3.3]heptan-6-yl-(2R,6S)-2,6-dimethyl-4-[6-(trifluormethyl)pyridazin-3-yl]piperazin-1-carboxylat;
2-Benzyl-6-(difluormethyl)-2-azaspiro[3.3]heptan-6-yl-(2R,6R)-2,6-dimethyl-4-[5-(trifluormethyl)pyrimidin-2-yl]piperazin-1-carboxylat;
2-Benzyl-6-(difluormethyl)-2-azaspiro[3.3]heptan-6-yl-(2R,6R)-2,6-dimethyl-4-[5-(trifluormethyl)pyrazin-2-yl]piperazin-1-carboxylat;
2-Benzyl-6-(difluormethyl)-2-azaspiro[3.3]heptan-6-yl-(2R,5S)-2,5-dimethyl-4-[5-(trifluormethyl)pyrimidin-2-yl]piperazin-1-carboxylat;
2-Benzyl-6-(difluormethyl)-2-azaspiro[3.3]heptan-6-yl-(2R,5S)-2,5-dimethyl-4-[5-(trifluormethyl)pyrazin-2-yl]piperazin-1-carboxylat;
2-Benzyl-6-(difluormethyl)-2-azaspiro[3.3]heptan-6-yl-(2R)-4-(5-cyanopyrimidin-2-yl)-2-methylpiperazin-1-carboxylat;
2-Benzyl-6-(difluormethyl)-2-azaspiro[3.3]heptan-6-yl-(2R)-2-methyl-4-[5-(trifluormethyl)-pyrimidin-2-yl]piperazin-1-carboxylat;
2-Benzyl-6-(difluormethyl)-2-azaspiro[3.3]heptan-6-yl-(2R)-2-methyl-4-[5-(trifluormethyl)-pyrazin-2-yl]piperazin-1-carboxylat;
2-Benzyl-6-(difluormethyl)-2-azaspiro[3.3]heptan-6-yl-(2R)-4-(5-cyanopyrimidin-2-yl)-2,6-dimethylpiperazin-1-carboxylat;
2-Benzyl-6-methyl-2-azaspiro[3.3]heptan-6-yl-(2R,6S)-2,6-dimethyl-4-[5-(trifluormethyl)-pyrimidin-2-yl]piperazin-1-carboxylat;
2-Benzyl-6-methyl-2-azaspiro[3.3]heptan-6-yl-(2R,6S)-2,6-dimethyl-4-[5-(trifluormethyl)-pyrazin-2-yl]piperazin-1-carboxylat;
2-Benzyl-6-methyl-2-azaspiro[3.3]heptan-6-yl-(2R,6R)-4-(5-cyanopyrimidin-2-yl)-2,6-dimethylpiperazin-1-carboxylat;
2-Benzyl-6-methyl-2-azaspiro[3.3]heptan-6-yl-(2R,6R)-2,6-dimethyl-4-[5-(trifluormethyl)-pyrimidin-2-yl]piperazin-1-carboxylat;
2-Benzyl-6-methyl-2-azaspiro[3.3]heptan-6-yl-(2R,6R)-2,6-dimethyl-4-[5-(trifluormethyl)-pyrazin-2-yl]piperazin-1-carboxylat;
2-Benzyl-6-methyl-2-azaspiro[3.3]heptan-6-yl-(2R,5S)-4-(5-cyanopyrimidin-2-yl)-2,5-dimethylpiperazin-1-carboxylat;
2-Benzyl-6-methyl-2-azaspiro[3.3]heptan-6-yl-(2R,5S)-2,5-dimethyl-4-[5-(trifluormethyl)-pyrimidin-2-yl]piperazin-1-carboxylat;
2-Benzyl-6-methyl-2-azaspiro[3.3]heptan-6-yl-(2R,5S)-2,5-dimethyl-4-[5-(trifluormethyl)-pyrazin-2-yl]piperazin-1-carboxylat;
2-Benzyl-2-azaspiro[3.3]heptan-6-yl-(2R,6S)-4-(5-ethinylpyrimidin-2-yl)-2,6-dimethyl-piperazin-1-carboxylat;
2-Benzyl-2-azaspiro[3.3]heptan-6-yl-(2R,6S)-4-[5-(dimethylphosphoryl)pyridin-2-yl]-2,6-dimethylpiperazin-1-carboxylat; und
2-Benzyl-2-azaspiro[3.3]heptan-6-yl-(2R,6S)-4-[5-(dimethylphosphoryl)pyrimidin-2-yl]-2,6-dimethylpiperazin-1-carboxylat;
oder ein pharmazeutisch annehmbares Salz davon.

2. Verbindung nach Anspruch 1 oder ein pharmazeutisch annehmbares Salz davon, worin:
R¹ aus Folgendem ausgewählt ist: 1,3,4-Thiadiazol-2-yl, Phenyl, Pyrazin-2-yl, Pyridazin-3-yl, Pyridin-2-yl, Pyrimidin-2-yl und Chinoxalin-2-yl;
die jeweils gegebenenfalls mit einer oder mehreren Gruppen substituiert sind, die aus den folgenden ausgewählt sind: Acetyl, Brom, Chlor, Cyano, Difluormethoxy, Difluormethyl, Fluor, Methoxy, Methoxycarbonyl, Methyl, Methylsulfanyl, Nitro, Trifluormethoxy und Trifluormethyl.

3. Verbindung nach Anspruch 1 oder ein pharmazeutisch annehmbares Salz davon, worin:
R¹ aus Folgendem ausgewählt ist: 2-Cyano-4-(trifluormethyl)phenyl, 3-(Trifluormethyl)pyridin-2-yl, 3,4,5-Trifluorphenyl, 3,6-Dimethylpyrazin-2-yl, 3-Cyano-4-(trifluormethoxy)phenyl, 3-Cyano-4-fluorphenyl, 3-Cyano-5-fluorphenyl, 4-(Trifluormethoxy)phenyl, 4-(Trifluormethyl)phenyl, 4-Cyano-2,5-difluorphenyl, 4-Cyano-2-fluorphenyl, 4-Cyano-6-(trifluormethyl)pyridin-3-yl, 4-Methylpyrimidin-2-yl, 5-(Difluormethoxy)pyrimidin-2-yl, 5-(Difluormethyl)pyrazin-2-yl, 5-(Methoxycarbonyl)-4-(trifluormethyl)pyrimidin-2-yl, 5-(Trifluormethoxy)pyrazin-2-yl, 5-(Trifluormethyl)-1,3,4-thiadiazol-2-yl, 5-(Trifluormethyl)-pyrazin-2-yl, 5-(Trifluormethyl)pyridin-2-yl, 5-(Trifluormethyl)pyrimidin-2-yl, 5-Acetylpyrazin-2-yl, 5-Brom-4-(methylsulfanyl)pyrimidin-2-yl, 5-Chlorpyrazin-2-yl, 5-Chlorpyrimidin-2-yl, 5-Cyano-3-methylpyrazin-2-yl, 5-Cyanopyrazin-2-yl, 5-Cyanopyridin-2-yl, 5-Cyanopyrimidin-2-yl, 5-Fluorpyrimidin-2-yl, 5-Methoxypyrimidin-2-yl, 5-Nitropyridin-2-yl, 6-(Trifluormethyl)-pyrazin-2-yl, 6-(Trifluormethyl)pyridazin-3-yl, 6-(Trifluormethyl)pyridin-3-yl, 6-Cyanopyrazin-2-yl, 6- Fluorchinoxalin-2-yl, 6-Methoxy-3-nitropyridin-2-yl, 6-Methoxypyrazin-2-yl, 6-Methoxy-chinoxalin-2-yl und Chinoxalin-2-yl.

4. Verbindung nach einem der Ansprüche 1 bis 3 oder ein pharmazeutisch annehmbares Salz davon, worin:
R² aus Folgendem ausgewählt ist: 1H-Indolyl, 2,2-Dimethylpropyl, 2-Methylpropyl, 3,3- Dimethylbutyl, Bicyclo[2.2.1]heptenyl, Butyl, Cyclohexyl, Cyclopropyl, Oxanyl und Phenyl;
die jeweils gegebenenfalls mit einer oder mehreren Gruppen substituiert sind, die aus den folgenden ausgewählt sind: C₁₋₄-Alkyl, C₁₋₆-Alkoxy, Carbamoyl, Cyano und Halogen.

5. Verbindung nach einem der Ansprüche 1 bis 4, wobei die Verbindung die Formel (IIg) aufweist: 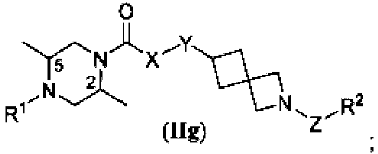 oder ein pharmazeutisch annehmbares Salz davon.

6. Verbindung nach einem der Ansprüche 1 bis 4, wobei die Verbindung die Formel (Ili) aufweist: 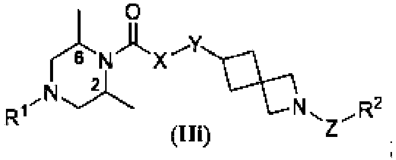 oder ein pharmazeutisch annehmbares Salz davon.

7. Verbindung nach einem der Ansprüche 1 bis 4, wobei die Verbindung die Formel (IIk) aufweist: 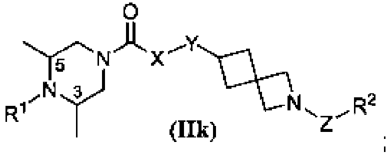 oder ein pharmazeutisch annehmbares Salz davon.

8. Verbindung nach Anspruch 6 oder ein pharmazeutisch annehmbares Salz davon, worin:
die Stereochemie in Formel (Ili) für den C(2)-Kohlenstoff (R) ist und für den C(6)-Kohlenstoff (R) ist.

9. Verbindung nach Anspruch 6 oder ein pharmazeutisch annehmbares Salz davon, worin:
die Stereochemie in Formel (Ili) für den C(2)-Kohlenstoff (S) ist und für den C(6)-Kohlenstoff (S) ist.

10. Verbindung nach Anspruch 6 oder ein pharmazeutisch annehmbares Salz davon, worin:
die Stereochemie in Formel (Ili) für den C(2)-Kohlenstoff (R) ist und für den C(6)-Kohlenstoff (S) ist.

11. Verbindung nach Anspruch 1, die aus den folgenden Verbindungen ausgewählt ist, oder ein pharmazeutisch annehmbares Salz davon:
(2R,5S)-N-{2-Benzyl-2-azaspiro[3.3]heptan-6-yl}-2,5-dimethyl-4-[5-(trifluormethyl)pyrimidin-2-yl]piperazin-1-carboxamid;
(2R)-N-{2-Benzyl-2-azaspiro[3.3]heptan-6-yl}-4-(5-cyanopyrimidin-2-yl)-2-methylpiperazin-1-carboxamid;
(2R,6R)-N-{2-Benzyl-2-azaspiro[3.3]heptan-6-yl}-4-(5-cyanopyrimidin-2-yl)-2,6-dimethyl-piperazin-1-carboxamid;
(25,6S)-N-{2-Benzyl-2-azaspiro[3.3]heptan-6-yl}-4-(5-cyanopyrimidin-2-yl)-2,6-dimethyl-piperazin-1-carboxamid;
(2S)-N-{2-Benzyl-2-azaspiro[3.3]heptan-6-yl}-2-methyl-4-[5-(trifluormethyl)pyrimidin-2-yl]-piperazin-1-carboxamid;
(3S)-N-{2-Benzyl-2-azaspiro[3.3]heptan-6-yl}-3-methyl-4-[5-(trifluormethyl)pyrimidin-2-yl]-piperazin-1-carboxamid;
(2R)-N-{2-Benzyl-2-azaspiro[3.3]heptan-6-yl}-2-methyl-4-[5-(trifluormethyl)pyrimidin-2-yl]-piperazin-1-carboxamid;
N-{2-Benzyl-2-azaspiro[3.3]heptan-6-yl}-4-(4-cyano-2-fluorphenyl)piperazin-1-carboxamid;
N-{2-Benzyl-2-azaspiro[3.3]heptan-6-yl}-4-(3 4,5-trifluorphenyl)piperazin-1-carboxamid;
N-{2-Benzyl-2-azaspiro[3.3]heptan-6-yl}-4-[4-(trifluormethoxy)phenyl]piperazin-1-carboxamid;
(2R,6R)-N-{2-Benzyl-2-azaspiro[3.3]heptan-6-yl}-2 6-dimethyl-4-(3,4,5-trifluorphenyl)-piperazin-1-carboxamid;
(2R)-N-{2-Benzyl-2-azaspiro[3.3]heptan-6-yl}-4-(3-cyano-4-fluorphenyl)-2-methylpiperazin-1-carboxamid;
(2R)-N-{2-Benzyl-2-azaspiro[3.3]heptan-6-yl}-4-[3-cyano-4-(trifluormethoxy)phenyl]-2-methylpiperazin-1-carboxamid;
(2R,6S)-N-{2-Benzyl-2-azaspiro[3.3]heptan-6-yl}-4-(5-chlorpyrimidin-2-yl)-2,6-dimethyl-piperazin-1-carboxamid;
(1S,4S)-N-{2-Benzyl-2-azaspiro[3.3]heptan-6-yl}-5-(5-cyanopyrimidin-2-yl)-2,5-diazabicyclo-[2.2.1]heptan-2-carboxamid;
N-{2-Benzyl-2-azaspiro[3.3]heptan-6-yl}-3-(5-cyanopyrimidin-2-yl)-3,6-diazabicyclo[3.1.1]-heptan-6-carboxamid;
N-{2-Benzyl-2-azaspiro[3.3]heptan-6-yl}-5-(5-cyanopyrimidin-2-yl)-2 ,5-diazabicyclo[2.2.2]-octan-2-carboxamid;
(2R,6R)-N-{2-Benzyl-2-azaspiro[3.3]heptan-6-yl}-4-(5-chlorpyrimidin-2-yl)-2,6-dimethyl-piperazin-1-carboxamid;
(2R,6R)-N-{2-Benzyl-2-azaspiro[3.3]heptan-6-yl}-2,6-dimethyl-4-[6-(trifluormethyl)pyridazin-3-yl]piperazin-1-carboxamid;
(2R,6R)-N-{2-Benzyl-2-azaspiro[3.3]heptan-6-yl}-4-(5-cyanopyrazin-2-yl)-2,6-dimethyl-piperazin-1-carboxamid:
(2R,6R)-N-{2-Benzyl-2-azaspiro[3.3]heptan-6-yl}-4-[5-(difluormethoxy)pyrimidin-2-yl]-2,6-dimethylpiperazin-1-carboxamid;
(2R)-N-{2-Benzyl-2-azaspiro[3.3]heptan-6-yl}-2-methyl-4-[5-(trifluormethyl)pyrazin-2-yl]-piperazin-1-carboxamid;
N-{2-Benzyl-2-azaspiro[3.3]heptan-6-yl}-3-[5-(trifluormethyl)pyrimidin-2-yl]-3,6-diazabicyclo-[3.1.1]heptan-6-carboxamid;
N-{2-Benzyl-2-azaspiro[3.3]heptan-6-yl}-3-[5-(trifluormethyl)pyrazin-2-yl]-3,6-diazabicyclo-[3.1.1]heptan-6-carboxamid;
(2S)-N-{2-Benzyl-2-azaspiro[3.3]heptan-6-yl}-4-(5-cyanopyrimidin-2-yl)-2-methylpiperazin-1-carboxamid;
(2R,5S)-N-{2-Benzyl-2-azaspiro[3.3]heptan-6-yl}-4-(5-cyanopyrimidin-2-yl)-2,5-dimethyl-piperazin-1-carboxamid;
(2S, 5R)-N-{2-Benzyl-2-azaspiro[3.3]heptan-6-yl}-4-(5-cyanopyrimidin-2-yl)-2,5-dimethyl-piperazin-1-carboxamid;
(2R,5R)-N-{2-Benzyl-2-azaspiro[3.3]heptan-6-yl}-4-(5-cyanopyrimidin-2-yl)-2,5-dimethyl-piperazin-1-carboxamid;
N-{2-Benzyl-2-azaspiro[3.3]heptan-6-yl}-3-[6-(trifluormethyl)pyridazin-3-yl]-3,6-diazabicyclo-[3.1.1]heptan-6-carboxamid;
(15,4S)-N-{2-Benzyl-2-azaspiro[3.3]heptan-6-yl}-5-[5-(trifluormethyl)pyrazin-2-yl]-2,5-diaza-bicyclo[2.2.1]heptan-2-carboxamid;
(15,4S)-N-{2-Benzyl-2-azaspiro[3.3]heptan-6-yl}-5-[5-(trifluormethyl)pyrimidin-2-yl]-2,5-diazabicyclo[2.2.1]heptan-2-carboxamid;
N-{2-Benzyl-2-azaspiro[3.3]heptan-6-yl}-4-[5-(trifluormethyl)pyrimidin-2-yl]piperazin-1-carboxamid;
(3R,5R)-N-{2-Benzyl-2-azaspiro[3.3]heptan-6-yl}-4-(5-cyanopyrimidin-2-yl)-3,5-dimethyl-piperazin-1-carboxamid;
(3S,5S)-N-{2-Benzyl-2-azaspiro[3.3]heptan-6-yl}-4-(5-cyanopyrimidin-2-yl)-3,5-dimethyl-piperazin-1-carboxamid;
(2R,5S)-N-{2-Benzyl-2-azaspiro[3.3]heptan-6-yl}-2,5-dimethyl-4-[5-(trifluormethyl)pyrazin-2-yl]piperazin-1-carboxamid;
(2S,5R)-N-{2-Benzyl-2-azaspiro[3.3]heptan-6-yl}-2,5-dimethyl-4-[5-(trifluormethyl)pyrimidin-2-yl]piperazin-1-carboxamid;
(2S,5R)-N-{2-Benzyl-2-azaspiro[3.3]heptan-6-yl}-2,5-dimethyl-4-[5-(trifluormethyl)pyrazin-2-yl]piperazin-1-carboxamid;
(2R,5R)-N-{2-Benzyl-2-azaspiro[3.3]heptan-6-yl}-2,5-dimethyl-4-[5-(trifluormethyl)pyrimidin-2-yl]piperazin-1-carboxamid;
(2R,5R)-N-{2-Benzyl-2-azaspiro[3.3]heptan-6-yl}-2,5-dimethyl-4-[5-(trifluormethyl)pyrazin-2-yl]piperazin-1-carboxamid;
(2S,5S)-N-{2-Benzyl-2-azaspiro[3.3]heptan-6-yl}-4-(5-cyanopyrimidin-2-yl)-2,5-dimethyl-piperazin-1-carboxamid;
(3S)-N-{2-Benzyl-2-azaspiro[3.3]heptan-6-yl}-4-(5-cyanopyrimidin-2-yl)-3-methylpiperazin-1-carboxamid;
(3R)-N-{2-Benzyl-2-azaspiro[3.3]heptan-6-yl}-4-(5-cyanopyrimidin-2-yl)-3-methylpiperazin-1-carboxamid;
N-{2-Benzyl-2-azaspiro[3.3]heptan-6-yl}-4-[5-(trifluormethyl)pyridin-2-yl]piperazin-1-carboxamid;
(2R)-N-{2-Benzyl-2-azaspiro[3.3]heptan-6-yl}-2-methyl-4-(3,4,5-trifluorphenyl)piperazin-1-carboxamid;
(2R,6S)-N-{2-Benzyl-2-azaspiro[3.3]heptan-6-yl}-2,6-dimethyl-4-(3,4,5-trifluorphenyl)-piperazin-1-carboxamid;
(2R,6R)-N-{2-Benzyl-2-azaspiro[3.3]heptan-6-yl}-4-(6-cyanopyrazin-2-yl)-2,6-dimethyl-piperazin-1-carboxamid;
(2R,6S)-N-{2-Benzyl-2-azaspiro[3.3]heptan-6-yl}-2,6-dimethyl-4-[4-(trifluormethyl)phenyl]-piperazin-1-carboxamid;
(2R,6R)-N-{2-Benzyl-2-azaspiro[3.3]heptan-6-yl}-2 6-dimethyl-4-[6-(trifluormethyl)pyrazin-2-yl]piperazin-1-carboxamid;
(1R,4R)-N-{2-Benzyl-2-azaspiro[3.3]heptan-6-yl}-5-(5-cyanopyrimidin-2-yl)-2,5-diazabicyclo-[2.2.2]}octan-2-carboxamid;
(2R,6S)-N-{2-Benzyl-2-azaspiro[3.3]heptan-6-yl}-4-(5-cyanopyridin-2-yl)-2,6-dimethyl-piperazin-1-carboxamid;
(2R,6R)-N-{2-Benzyl-2-azaspiro[3.3]heptan-6-yl}-2 6-dimethyl-4-[4-(trifluormethyl)phenyl]-piperazin-1-carboxamid;
(1S,4S)-N-{2-Benzyl-2-azaspiro[3.3]heptan-6-yl}-5-(5-cyanopyrimidin-2-yl)-2,5-diazabicyclo-[2.2.2]octan-2-carboxamid;
(2R,6R)-N-{2-Benzyl-2-azaspiro[3.3]heptan-6-yl}-4-[5-(difluormethyl)pyrazin-2-yl]-2,6-dimethylpiperazin-1-carboxamid;
N-{2-Benzyl-2-azaspiro[3.3]heptan-6-yl}-3-(5-cyanopyrimidin-2-yl)-3,8-diazabicyclo[3.2.1]-octan-8-carboxamid;
(2R,6R)-N-{2-Benzyl-2-azaspiro[3.3]heptan-6-yl}-4-(5-methoxypyrimidin-2-yl)-2,6-dimethyl-piperazin-1-carboxamid;
(2R,6S)-N-{2-Benzyl-2-azaspiro[3.3]heptan-6-yl}-2,6-dimethyl-4-[6-(trifluormethyl)pyrazin-2-yl]piperazin-1-carboxamid;
(2R,6R)-N-{2-Benzyl-2-azaspiro[3.3]heptan-6-yl}-4-(5-chlorpyrazin-2-yl)-2,6-dimethyl-piperazin-1-carboxamid;
(1R,4R)-N-{2-Benzyl-2-azaspiro[3.3]heptan-6-yl}-5-[5-(trifluormethyl)pyrimidin-2-yl]-2,5-diazabicyclo[2.2.1|heptan-2-carboxamid;
(1R,4R)-N-{2-Benzyl-2-azaspiro[3.3]heptan-6-yl}-5-[5-(trifluormethyl)pyrazin-2-yl]-2,5-diazabicyclo[2.2.1]heptan-2-carboxamid;
(2R,6S)-N-{2-Benzyl-2-azaspiro[3.3]heptan-6-yl}-4-[4-methoxy-5-(trifluormethyl)pyrimidin-2-yl]-2,6-dimethylpiperazin-1-carboxamid;
(2R,6S)-N-{2-Benzyl-2-azaspiro[3.3]heptan-6-yl}-4-(5-cyanopyrazin-2-yl)-2,6-dimethylpiperazin-1-carboxamid;
(2R,6S)-N-{2-Benzyl-2-azaspiro[3.3]heptan-6-yl}-2,6-dimethyl-4-[5-(trifluormethyl)pyrazin-2-yl]piperazin-1-carboxamid;
(2R,6R)-N-({2-Benzyl-2-azaspiro[3.3]heptan-6-yl}methyl)-2,6-dimethyl-4-[5-(trifluormethyl)-pyrazin-2-yl]piperazin-1-carboxamid;
(2R,6R)-N-{2-Benzyl-2-azaspiro[3.3]heptan-6-yl}-2,6-dimethyl-4-[5-(trifluormethyl)pyrazin-2-yl]piperazin-1-carboxamid;
(2R,6R)-N-{2-Benzyl-2-azaspiro[3.3]heptan-6-yl}-2,6-dimethyl-4-[5-(trifluormethyl)pyrimidin-2-yl]piperazin-1-carboxamid;
(2R,6S)-N-{2-Benzyl-2-azaspiro[3.3]heptan-6-yl}-4-(5-cyanopyrimidin-2-yl)-2,6-dimethylpiperazin-1-carboxamid;
(2R,6S)-N-({2-Benzyl-2-azaspiro[3.3]heptan-6-yl}methyl)-2,6-dimethyl-4-[5-(trifluormethyl)-pyrazin-2-yl]piperazin-1-carboxamid;
(2R,6R)-N-({2-Benzyl-2-azaspiro[3.3]heptan-6-yl}methyl)-2,6-dimethyl-4-[5-(trifluormethyl)-pyrimidin-2-yl]piperazin-1-carboxamid;
(2R,6R)-N-[2-(3,3-Dimethylbutyl)-2-azaspiro[3.3]heptan-6-yl]-2 ,6-dimethyl-4-[5-(trifluormethyl)pyrimidin-2-yl]piperazin-1-carboxamid;
(2R,6S)-4-(5-Cyanopyrimidin-2-yl)-N-[2-(3,3-dimethylbutyl)-2-azaspiro[3.3]heptan-6-yl]-2,6-dimethylpiperazin-1-carboxamid;
(2R,6S)-N-{[2-(3,3-Dimethylbutyl)-2-azaspiro[3.3]heptan-6-yl]methy]}-2,6-dimethyl-4-[5-(trifluormethyl)pyrazin-2-yl]piperazin-1-carboxamid;
(2R,6R)-N-{[2-(3,3-Dimethylbutyl)-2-azaspiro[3.3]heptan-6-yl]methyl}-2,6-dimethyl-4-[5-(trifluormethyl)pyrimidin-2-yl]piperazin-1-carboxamid;
(2R,6R)-N-[2-(2,2-Dimethylpropyl)-2-azaspiro[3.3]heptan-6-yl]-2,6-dimethyl-4-[5-(trifluormethyl)pyrimidin-2-yl]piperazin-1-carboxamid;
(2R,6S)-4-(5-Cyanopyrimidin-2-yl)-N-[2-(2,2-dimethylpropyl)-2-azaspiro[3.3]heptan-6-yl]-2,6-dimethylpiperazin-1-carboxamid;
(2R,6S)-N-{[2-(2,2-Dimethylpropyl)-2-azaspiro[3.3]heptan-6-yl]methyl}-2,6-dimethyl-4-[5-(trifluormethyl)pyrazin-2-yl]piperazin-1-carboxamid;
(2R,6R)-N-{[2-(2,2-Dimethylpropyl)-2-azaspiro[3.3 ]|heptan-6-yl]methyl}-2,6-dimethyl-4-[5-(trifluormethyl)pyrimidin-2-yl]piperazin-1-carboxamid;
(2R,6S)-N-[2-(Cyclopropylmethyl)-2-azaspiro[3.3]heptan-6-yl]-2,6-dimethyl-4-[5-(trifluormethyl)pyrimidin-2-yl]piperazin-1-carboxamid;
(2R,6S)-2,6-Dimethyl-N-[2-(2-methylpropyl)-2-azaspiro[3.3]heptan-6-yl]-4-[5-(trifluormethyl)-pyrimidin-2-yl]piperazin-1-carboxamid;
(2R,6S)-N-{2-[(2,2-Dimethylcyclopropyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-[2-(Cyclohexylmethyl)-2-azaspiro[3.3]heptan-6-yl]-2,6-dimethyl-4-[5-(trifluormethyl)pyrimidin-2-yl]piperazin-1-carboxamid;
(2R,6S)-2,6-Dimethyl-N-[2-(oxan-4-ylmethyl)-2-azaspiro[3.3]heptan-6-yl]-4-[5-(trifluormethyl)-pyrimidin-2-yl]piperazin-1-carboxamid;
(2R,6S)-N-(2-{Bicyclo[2.2. 1]hept-5-en-2-ylmethy|}-2-azaspiro[3.3]heptan-6-yl)-2,6-dimethyl-4-[5-(trifluormethyl)pyrimidin-2-yl]piperazin-1-carboxamid;
(2R,6S)-N-{2-[(2-Fluorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(3-Fluorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(4-Fluorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-2,6-Dimethyl-N-[2-(4,4,4-trifluorbutyl)-2-azaspiro[3.3]heptan-6-yl]-4-[5-(trifluormethyl)pyrimidin-2-yl]piperazin-1-carboxamid;
(2R,6S)-N-{2-[(2-Cyanophenyl)methy|]-2-azaspiro[3.3]heptan-6-yl}-2,6-dimethyl-4-[5-(trifluormethyl)pyrimidin-2-yl]piperazin-1-carboxamid;
(2R,6S)-N-{2-[(4-Cyanophenyl)methyl|]-2-azaspiro[3.3]heptan-6-yl}-2,6-dimethyl-4-[5-(trifluormethyl)pyrimidin-2-yl]piperazin-1-carboxamid;
(2R,6S)-N-{2-[(4-Methoxyphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(2,6-Difluorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(3,4-Difluorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-[2-(1H-Indol-5-ylmethyl)-2-azaspiro[3.3]heptan-6-yl]-2,6-dimethyl-4-[5-(trifluormethyl)pyrimidin-2-yl]piperazin-1-carboxamid;
(2R,6S)-N-{2-[(3-Cyano-4-fluorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(4-Carbamoylphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(3-Carbamoylphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-Benzyl-2-azaspiro[3.3]heptan-6-yl}-2,6-dimethyl-4-[5-(trifluormethyl)pyrimidin-2-yl]piperazin-1-carboxamid;
(2R,6S)-N-({2-Benzyl-2-azaspiro[3.3]heptan-6-yl}methyl)-2,6-dimethyl-4-[5-(trifluormethyl)-pyrimidin-2-yl]piperazin-1-carboxamid;
(2R,6S)-N-[2-(3,3-Dimethylbutyl)-2-azaspiro[3.3]heptan-6-yl]-2,6-dimethyl-4-[5-(trifluormethyl)pyrimidin-2-yl]piperazin-1-carboxamid;
(2R,6S)-N-{[2-(3,3-Dimethylbutyl)-2-azaspiro[3.3]heptan-6-yl]methy]}-2,6-dimethyl-4-[5-(trifluormethyl)pyrimidin-2-yl]piperazin-1-carboxamid;
(2R,6S)-N-[2-(2,2-Dimethylpropyl)-2-azaspiro[3.3]heptan-6-yl]-2,6-dimethyl-4-[5-(trifluormethyl)pyrimidin-2-yl]piperazin-1-carboxamid;
(2R,6S)-N-{[2-(2,2-Dimethylpropyl)-2-azaspiro[3.3]heptan-6-yl]methyl}-2,6-dimethyl-4-[5-(trifluormethyl)pyrimidin-2-yl]piperazin-1-carboxamid;
2-Benzyl-2-azaspiro[3.3]heptan-6-yl-4-(4-cyano-2-fluorphenyl)piperazin-1-carboxylat;
2-Benzyl-2-azaspiro[3.3]heptan-6-yl-(2R)-4-[3-cyano-4-(trifluormethoxy)phenyl]-2-methylpiperazin-1-carboxylat;
2-Benzyl-2-azaspiro[3.3]heptan-6-yl-(1 S,4S)-5-(5-cyanopyrimidin-2-yl)-2,5-diazabicyclo-[2.2.1]heptan-2-carboxylat;
2-Benzyl-2-azaspiro[3.3]heptan-6-yl-4-(3,4,5-trifluorphenyl)piperazin-1-carboxylat;
2-Benzyl-2-azaspiro[3.3]heptan-6-yl-(2R,5S)-2,5-dimethyl-4-[5-(trifluormethyl)pyrazin-2-yl]-piperazin-1-carboxylat;
2-Benzyl-2-azaspiro[3.3]heptan-6-yl-5-(5-cyanopyrimidin-2-yl)-2,5-diazabicyclo[2.2.2]octan-2-carboxylat;
2-Benzyl-2-azaspiro[3.3]heptan-6-yl-4-[4-(trifluormethoxy)phenyl]piperazin-1-carboxylat;
2-Benzyl-2-azaspiro[3.3]heptan-6-yl-3-[5-(trifluormethyl)pyrimidin-2-yl]-3,6-diazabicyclo-[3.1.1]heptan-6-carboxylat;
2-Benzyl-2-azaspiro[3.3]heptan-6-yl-3-[5-(trifluormethyl)pyrazin-2-yl]-3,6-diazabicyclo[3.1.1]-heptan-6-carboxylat;
2-Benzyl-2-azaspiro[3.3]heptan-6-yl-3-(5-cyanopyrazin-2-yl)-3,6-diazabicyclo[3.1.1]heptan-6-carboxylat;
2-Benzyl-2-azaspiro[3.3]heptan-6-yl-4-(5-cyanopyrimidin-2-yl)piperazin-1-carboxylat;
2-Benzyl-2-azaspiro[3.3]heptan-6-yl-3-[6-(trifluormethyl)pyridazin-3-yl]-3,6-diazabicyclo-[3.1.1]heptan-6-carboxylat;
2-Benzyl-2-azaspiro[3.3]heptan-6-yl-(1S,4S)-5-[5-(trifluormethyl)pyrazin-2-yl]-2,5-diazabicyclo[2.2.1]heptan-2-carboxylat;
2-Benzyl-2-azaspiro[3.3]heptan-6-yl-(1S,4S)-5-[5-(trifluormethyl)pyrimidin-2-yl]-2,5-diazabicyclo[2.2.1]heptan-2-carboxylat;
2-Benzyl-2-azaspiro[3.3]heptan-6-yl-4-[5-(trifluormethyl)pyrimidin-2-yl]piperazin-1-carboxylat;
2-Benzyl-2-azaspiro[3.3]heptan-6-yl-(3R,5R)-4-(5-cyanopyrimidin-2-yl)-3,5-dimethyl-piperazin-1-carboxylat;
2-Benzyl-2-azaspiro[3.3]heptan-6-yl-(3S,5S)-4-(5-cyanopyrimidin-2-yl)-3,5-dimethylpiperazin-1-carboxylat;
2-Benzyl-2-azaspiro[3.3]heptan-6-yl-(3S)-4-(5-cyanopyrimidin-2-yl)-3-methylpiperazin-1-carboxylat;
2-Benzyl-2-azaspiro[3.3]heptan-6-yl-(3R)-4-(5-cyanopyrimidin-2-yl)-3-methylpiperazin-1-carboxylat;
2-Benzyl-2-azaspiro[3.3]heptan-6-yl-4-[5-(trifluormethyl)pyridin-2-yl]piperazin-1-carboxylat;
2-Benzyl-2-azaspiro[3.3]heptan-6-yl-(1R,4R)-5-[5-(trifluormethyl)pyrazin-2-yl]-2,5-diazabicyclo[2.2.1]heptan-2-carboxylat;
2-Benzyl-2-azaspiro[3.3]heptan-6-yl-(3S)-3-methyl-4-[5-(trifluormethyl)pyrimidin-2-yl]-piperazin-1-carboxylat;
2-Benzyl-2-azaspiro[3.3]heptan-6-yl-4-(4-cyano-2,5-difluorphenyl)piperazin-1-carboxylat;
2-Benzyl-2-azaspiro[3.3]heptan-6-yl-4-[4-(trifluormethyl)phenyl]piperazin-1-carboxylat;
2-Benzyl-2-azaspiro[3.3]heptan-6-yl-4-[2-cyano-4-(trifluormethyl)phenyl]piperazin-1-carboxylat;
2-Benzyl-2-azaspiro[3.3]heptan-6-yl-4-(3-cyano-5-fluorphenyl)piperazin-1-carboxylat;
2-Benzyl-2-azaspiro[3.3]heptan-6-yl-(2R,5R)-4-(5-cyanopyrimidin-2-yl)-2,5-dimethyl-piperazin-1-carboxylat;
2-Benzyl-2-azaspiro[3.3]heptan-6-yl-(2S)-4-(5-cyanopyrimidin-2-yl)-2-methylpiperazin-1-carboxylat;
2-Benzyl-2-azaspiro[3.3]heptan-6-yl-(2R,6S)-4-(5-cyanopyrimidin-2-yl)-2,6-dimethylpiperazin-1-carboxylat;
{2-Benzyl-2-azaspiro[3.3]heptan-6-yl}methyl-(2R,6S)-4-(5-cyanopyrimidin-2-yl)-2,6-dimethyl-piperazin-1-carboxylat;
2-Benzyl-2-azaspiro[3.3]heptan-6-yl-(2R)-2-methyl-4-[5-(trifluormethyl)pyrimidin-2-yl]-piperazin-1-carboxylat;
{2-Benzyl-2-azaspiro[3.3]heptan-6-yl}methyl-(2R)-2-methyl-4-[5-(trifluormethyl)pyrimidin-2-yl]piperazin-1-carboxylat;
{2-Benzyl-2-azaspiro[3.3]heptan-6-yl}methyl-(2R,5S)-2,5-dimethyl-4-[5-(trifluormethyl)-pyrimidin-2-yl]piperazin-1-carboxylat;
2-Benzyl-2-azaspiro[3.3]heptan-6-yl-(2R,5R)-2,5-dimethyl-4-[5-(trifluormethyl)pyrimidin-2-yl]-piperazin-1-carboxylat;
2-Benzyl-2-azaspiro[3.3]heptan-6-yl-(2R,5S)-2,5-dimethyl-4-[5-(trifluormethyl)pyrimidin-2-yl]-piperazin-1-carboxylat;
2-Benzyl-2-azaspiro[3.3]heptan-6-yl-(2R)-2-methyl-4-[5-(trifluormethyl)pyrazin-2-yl]piperazin-1-carboxylat;
2-Benzyl-2-azaspiro[3.3]heptan-6-yl-(2R,5S)-4-(5-cyanopyrimidin-2-yl)-2,5-dimethylpiperazin-1-carboxylat;
2-Benzyl-2-azaspiro[3.3]heptan-6-yl-(2R,6R)-2,6-dimethyl-4-[5-(trifluormethyl)pyrimidin-2-yl]-piperazin-1-carboxylat;
{2-Benzyl-2-azaspiro[3.3]heptan-6-yl}methyl-(2R,6S)-2,6-dimethyl-4-[5-(trifluormethyl)-pyrazin-2-yl]piperazin-1-carboxylat;
2-Benzyl-2-azaspiro[3.3]heptan-6-yl-(2R,6S)-2,6-dimethyl-4-[5-(trifluormethyl)pyrazin-2-yl]-piperazin-1-carboxylat;
2-Benzyl-2-azaspiro[3.3]heptan-6-yl-(2R,6R)-2,6-dimethyl-4-[5-(trifluormethyl)pyrazin-2-yl]-piperazin-1-carboxylat;
{2-Benzyl-2-azaspiro[3.3]heptan-6-yl}methyl-(2R,6R)-2,6-dimethyl-4-[5-(trifluormethyl)-pyrazin-2-yl]piperazin-1-carboxylat;
{2-Benzyl-2-azaspiro[3.3]heptan-6-yl}methyl-(2R,6R)-2,6-dimethyl-4-[5-(trifluormethyl)-pyrimidin-2-yl]piperazin-1-carboxylat;
2-(3,3-Dimethylbutyl)-2-azaspiro[3.3]heptan-6-yl-(2R,6R)-2,6-dimethyl-4-[5-(trifluormethyl)-pyrimidin-2-yl]piperazin-1-carboxylat;
2-(2,2-Dimethylpropyl)-2-azaspiro[3.3]heptan-6-yl-(2R,6R)-2,6-dimethyl-4-[5-(trifluormethyl)-pyrimidin-2-yl]piperazin-1-carboxylat;
2-Benzyl-2-azaspiro[3.3]heptan-6-yl-(2R,6S)-2,6-dimethyl-4-[5-(trifluormethyl)pyrimidin-2-yl]-piperazin-1-carboxylat;
{2-Benzyl-2-azaspiro[3.3]heptan-6-yl}methyl-(2R,6S)-2,6-dimethyl-4-[5-(trifluormethyl)-pyrimidin-2-yl]piperazin-1-carboxylat;
2-(3,3-Dimethylbutyl)-2-azaspiro[3.3]heptan-6-yl-(2R,6S)-2,6-dimethyl-4-[5-(trifluormethyl)-pyrimidin-2-yl]piperazin-1-carboxylat;
[2-(3,3-Dimethylbutyl)-2-azaspiro[3.3]heptan-6-yl]methyl-(2R,6S)-2,6-dimethyl-4-[5-(trifluormethyl)pyrimidin-2-yl]piperazin-1-carboxylat;
2-(2,2-Dimethylpropyl)-2-azaspiro[3.3]heptan-6-yl-(2R,6S)-2,6-dimethyl-4-[5-(trifluormethyl)-pyrimidin-2-yl]piperazin-1-carboxylat;
[2-(2,2-Dimethylpropyl)-2-azaspiro[3.3]heptan-6-yl]methyl-(2R,6S)-2,6-dimethyl-4-[5-(trifluormethyl)pyrimidin-2-yl]piperazin-1-carboxylat;
2-[(2,2-Dimethylcyclopropyl)methyl]-2-azaspiro[3.3]heptan-6-yl-(2R,6S)-2,6-dimethyl-4-[5-(trifluormethyl)pyrimidin-2-yl]piperazin-1-carboxylat;
2-(Cyclohexylmethyl)-2-azaspiro[3.3]heptan-6-yl-(2R,6S)-2,6-dimethyl-4-[5-(trifluormethyl)-pyrimidin-2-yl]piperazin-1-carboxylat;
2-{Bicyclo[2.2.1]nept-5-en-2-ylmethyl}-2-azaspiro[3.3]heptan-6-yl-(2R,6S)-2,6-dimethyl-4-[5-(trifluormethyl)pyrimidin-2-yl]piperazin-1-carboxylat;
2-[(2,6-Difluorphenyl)methyl]-2-azaspiro[3.3]heptan-6-yl-(2R,6S)-2,6-dimethyl-4-[5-(trifluormethyl)pyrimidin-2-yl]piperazin-1-carboxylat;
2-Benzyl-2-azaspiro[3.3]heptan-6-yl-(2R,6S)-4-(5-fluorpyrimidin-2-yl)-2,6-dimethylpiperazin-1-carboxylat;
2-Benzyl-2-azaspiro[3.3]heptan-6-yl-(2R,6R)-4-(5-fluorpyrimidin-2-yl)-2,6-dimethylpiperazin-1-carboxylat;
2-Benzyl-2-azaspiro[3.3]heptan-6-yl-(2R,6R)-4-(5-cyanopyrimidin-2-yl)-2,6-dimethyl-piperazin-1-carboxylat;
2-Benzyl-2-azaspiro[3.3]heptan-6-yl-7-(5-cyanopyrimidin-2-yl)-3-oxa-7,9-diazabicyclo[3.3.1]-nonan-9-carboxylat;
2-Benzyl-2-azaspiro[3.3]heptan-6-yl-(2R,6S)-4-(5-acetylpyrazin-2-yl)-2,6-dimethylpiperazin-1-carboxylat;
2-Benzyl-2-azaspiro[3.3]heptan-6-yl-(2R,6R)-4-(5-acetylpyrazin-2-yl)-2,6-dimethylpiperazin-1-carboxylat;
2-Benzyl-2-azaspiro[3.3]heptan-6-yl-(2R,6S)-2,6-dimethyl-4-(5-nitropyridin-2-yl)piperazin-1-carboxylat;
2-Benzyl-2-azaspiro[3.3]heptan-6-yl-(2R,6R)-2,6-dimethyl-4-(5-nitropyridin-2-yl)piperazin-1-carboxylat;
2-Benzyl-2-azaspiro[3.3]heptan-6-yl-(2R,6S)-2,6-dimethyl-4-[6-(trifluormethyl)pyrazin-2-yl]-piperazin-1-carboxylat;
2-Benzyl-2-azaspiro[3.3]heptan-6-yl-(2R,6R)-2,6-dimethyl-4-[6-(trifluormethyl)pyrazin-2-yl]-piperazin-1-carboxylat;
2-Benzyl-2-azaspiro[3.3]heptan-6-yl-7-[5-(trifluormethyl)pyrimidin-2-yl]-3-oxa-7,9-diazabicyclo[3.3.1]nonan-9-carboxylat;
2-Benzyl-2-azaspiro[3.3]heptan-6-yl-7-[5-(trifluormethyl)pyrazin-2-yl]-3-oxa-7,9-diazabicyclo-[3.3.1]nonan-9-carboxylat;
2-Benzyl-2-azaspiro[3.3]heptan-6-yl-(2R,6S)-4-[5-brom-4-(methylsulfanyl)pyrimidin-2-yl]-2,6-dimethylpiperazin-1-carboxylat;
2-Benzyl-2-azaspiro[3.3]heptan-6-yl-(2R,6R)-4-[5-brom-4-(methylsulfanyl)pyrimidin-2-yl]-2,6-dimethylpiperazin-1-carboxylat;
Methyl-2-[(3R,5R)-4-[({2-benzyl-2-azaspiro[3.3]heptan-6-yl}oxy)carbonyl]-3,5-dimethyl-piperazin-1-yl]-4-(trifluormethyl)pyrimidin-5-carboxylat;
2-Benzyl-2-azaspiro[3.3]heptan-6-yl-(2R,6S)-2,6-dimethyl-4-(chinoxalin-2-yl)piperazin-1-carboxylat;
2-Benzyl-2-azaspiro[3.3]heptan-6-yl-(2R,5S)-4-(6-methoxychinoxalin-2-yl)-2,5-dimethyl-piperazin-1-carboxylat;
2-Benzyl-2-azaspiro[3.3]heptan-6-yl-(2R,5S)-2,5-dimethyl-4-(4-methylpyrimidin-2-yl)-piperazin-1-carboxylat;
2-Benzyl-2-azaspiro[3.3]heptan-6-yl-(2R,5R)-2,5-dimethyl-4-(4-methylpyrimidin-2-yl)-piperazin-1-carboxylat;
2-Benzyl-2-azaspiro[3.3]heptan-6-yl-(2R,6R)-2,6-dimethyl-4-(4-methylpyrimidin-2-yl)-piperazin-1-carboxylat;
2-Benzyl-2-azaspiro[3.3]heptan-6-yl-(2R,5S)-4-(5-fluorpyrimidin-2-yl)-2,5-dimethylpiperazin-1-carboxylat;
2-Benzyl-2-azaspiro[3.3]heptan-6-yl-(2R,5S)-4-(3,6-dimethylpyrazin-2-yl)-2,5-dimethyl-piperazin-1-carboxylat;
2-Benzyl-2-azaspiro[3.3]heptan-6-yl-(2R,5S)-4-(6-methoxypyrazin-2-yl)-2,5-dimethyl-piperazin-1-carboxylat;
2-Benzyl-2-azaspiro[3.3]heptan-6-yl-(2R,5R)-4-(6-methoxypyrazin-2-yl)-2,5-dimethyl-piperazin-1-carboxylat;
2-Benzyl-2-azaspiro[3.3]heptan-6-yl-(2R,6S)-4-(5-cyano-3-methylpyrazin-2-yl)-2,6-dimethyl-piperazin-1-carboxylat;
2-Benzyl-2-azaspiro[3.3]heptan-6-yl-(2R,6R)-4-(5-cyano-3-methylpyrazin-2-yl)-2,6-dimethyl-piperazin-1-carboxylat;
2-Benzyl-2-azaspiro[3.3]heptan-6-yl-(2R,5S)-2,5-dimethyl-4-(5-nitropyridin-2-yl)piperazin-1-carboxylat;
2-Benzyl-2-azaspiro[3.3]heptan-6-yl-(2R,5R)-2,5-dimethyl-4-(5-nitropyridin-2-yl)piperazin-1-carboxylat;
2-Benzyl-2-azaspiro[3.3]heptan-6-yl-(2R,6R)-2,6-dimethyl-4-(chinoxalin-2-yl)piperazin-1-carboxylat;
2-Benzyl-2-azaspiro[3.3]heptan-6-yl-(2R,5R)-2,5-dimethyl-4-(chinoxalin-2-yl)piperazin-1-carboxylat;
2-Benzyl-2-azaspiro[3.3]heptan-6-yl-(2R,5S)-2,5-dimethyl-4-(chinoxalin-2-yl)piperazin-1-carboxylat;
2-Benzyl-2-azaspiro[3.3]heptan-6-yl-9-(chinoxalin-2-yl)-3-oxa- 7,9-diazabicyclo[3.3.1]nonan-7-carboxylat;
2-Benzyl-2-azaspiro[3.3]heptan-6-yl-(2R,6S)-2,6-dimethyl-4-[3-(trifluormethyl)pyridin-2-yl]-piperazin-1-carboxylat;
2-Benzyl-2-azaspiro[3.3]heptan-6-yl-(2R,6R)-2,6-dimethyl-4-[3-(trifluormethyl)pyridin-2-yl]-piperazin-1-carboxylat;
2-Benzyl-2-azaspiro[3.3]heptan-6-yl-(2R,6S)-2,6-dimethyl-4-[6-(trifluormethyl)pyridin-3-yl]-piperazin-1-carboxylat;
2-Benzyl-2-azaspiro[3.3]heptan-6-yl-(2R,6R)-2,6-dimethyl-4-[6-(trifluormethyl)pyridin-3-yl]-piperazin-1-carboxylat;
2-Benzyl-2-azaspiro[3.3]heptan-6-yl-(2R,5R)-2,5-dimethyl-4-[6-(trifluormethyl)pyridin-3-yl]-piperazin-1-carboxylat;
2-Benzyl-2-azaspiro[3.3]heptan-6-yl-(2R,5S)-2,5-dimethyl-4-[6-(trifluormethyl)pyrazin-2-yl]-piperazin-1-carboxylat;
2-Benzyl-2-azaspiro[3.3]heptan-6-yl-(2R,5R)-2,5-dimethyl-4-[5-(trifluormethyl)pyrazin-2-yl]-piperazin-1-carboxylat;
2-Benzyl-2-azaspiro[3.3]heptan-6-yl-(2R,5R)-2,5-dimethyl-4-[6-(trifluormethyl)pyrazin-2-yl]-piperazin-1-carboxylat;
2-Benzyl-2-azaspiro[3.3]heptan-6-yl-(2R,6S)-4-(6-fluorchinoxalin-2-yl)-2,6-dimethylpiperazin-1-carboxylat;
2-Benzyl-2-azaspiro[3.3]heptan-6-yl-(2R,6S)-4-(6-methoxy-3-nitropyridin-2-yl)-2,6-dimethyl-piperazin-1-carboxylat;
2-Benzyl-2-azaspiro[3.3]heptan-6-yl-(2R,5S)-4-(6-methoxy-3-nitropyridin-2-yl)-2,5-dimethyl-piperazin-1-carboxylat;
2-Benzyl-2-azaspiro[3.3]heptan-6-yl-(2R,6R)-4-(6-methoxy-3-nitropyridin-2-yl)-2,6-dimethyl-piperazin-1-carboxylat;
2-Benzyl-2-azaspiro[3.3]heptan-6-yl-(2R,5R)-4-(6-methoxychinoxalin-2-yl)-2,5-dimethyl-piperazin-1-carboxylat;
2-Benzyl-2-azaspiro[3.3]heptan-6-yl-(2R,6S)-2,6-dimethyl-4-[5-(trifluormethoxy)pyrazin-2-yl]-piperazin-1-carboxylat;
2-Benzyl-2-azaspiro[3.3]heptan-6-yl-(2R,6R)-2,6-dimethyl-4-[5-(trifluormethoxy)pyrazin-2-yl]-piperazin-1-carboxylat;
2-Benzyl-2-azaspiro[3.3]heptan-6-yl-(2R,5S)-2,5-dimethyl-4-[5-(trifluormethoxy)pyrazin-2-yl]-piperazin-1-carboxylat;
2-Benzyl-2-azaspiro[3.3]heptan-6-yl-(2R,5R)-2,5-dimethyl-4-[5-(trifluormethoxy)pyrazin-2-yl]-piperazin-1-carboxylat;
2-Benzyl-2-azaspiro[3.3]heptan-6-yl-(2R,6R)-4-[4-cyano-6-(trifluormethyl)pyridin-3-yl]-2,6-dimethylpiperazin-1-carboxylat;
2-Benzyl-2-azaspiro[3.3]heptan-6-yl-(2R,5R)-4-[4-cyano-6-(trifluormethyl)pyridin-3-yl]-2,5-dimethylpiperazin-1-carboxylat;
Methyl-2-[(2S,5R)-4-[({2-benzyl-2-azaspiro[3.3]heptan-6-yl}oxy)carbonyl]-2,5-dimethyl-piperazin-1-yl]-4-(trifluormethyl)pyrimidin-5-carboxylat;
(2R,6S)-N-{2-Benzyl-2-azaspiro[3.3]heptan-6-yl}-2,6-dimethyl-4-(chinazolin-2-yl)piperazin-1-carboxamid;
(2R,6S)-N-{2-Benzyl-2-azaspiro[3.3]heptan-6-yl}-4-(6-fluorchinoxalin-2-yl)-2,6-dimethyl-piperazin-1-carboxamid;
(2R,6R)-N-{2-Benzyl-2-azaspiro[3.3]heptan-6-yl}-2 6-dimethyl-4-(chinazolin-2-yl)piperazin-1-carboxamid;
(2R,5S)-N-{2-Benzyl-2-azaspiro[3.3]heptan-6-yl}-2,5-dimethyl-4-(chinazolin-2-yl)piperazin-1-carboxamid;
(2R)-N-{2-Benzyl-2-azaspiro[3.3]heptan-6-yl}-2-methyl-4-(chinazolin-2-yl)piperazin-1-carboxamid;
(2R,6S)-N-{2-Benzyl-2-azaspiro[3.3]heptan-6-yl}-2,6-dimethyl-4-(chinoxalin-2-yl)piperazin-1-carboxamid;
(2R,6S)-N-{2-Benzyl-2-azaspiro[3.3]heptan-6-yl}-4-(6-methoxychinoxalin-2-yl)-2,6-dimethyl-piperazin-1-carboxamid;
(2R,6S)-N-{2-Benzyl-2-azaspiro[3.3]heptan-6-yl}-2,6-dimethyl-4-[6-(trifluormethyl)-1,3-benzo-thiazol-2-yl]piperazin-1-carboxamid;
(2R,6R)-N-{2-Benzyl-2-azaspiro[3.3]heptan-6-yl}-2,6-dimethyl-4-[6-(trifluormethyl)-1,3-benzo-thiazol-2-yl]piperazin-1-carboxamid;
(2R,6R)-N-{2-Benzyl-2-azaspiro[3.3]heptan-6-yl}-4-(6-fluorchinoxalin-2-yl)-2,6-dimethyl-piperazin-1-carboxamid;
(2R,6R)-N-{2-Benzyl-2-azaspiro[3.3]heptan-6-yl}-4-(6-methoxychinoxalin-2-yl)-2,6-dimethyl-piperazin-1-carboxamid;
(2R,6R)-N-{2-Benzyl-2-azaspiro[3.3]heptan-6-yl}-2,6-dimethyl-4-(chinoxalin-2-yl)piperazin-1-carboxamid;
(2R,5S)-N-{2-Benzyl-2-azaspiro[3.3]heptan-6-yl}-2,5-dimethyl-4-(chinoxalin-2-yl)piperazin-1-carboxamid;
(2R,5S)-N-{2-Benzyl-2-azaspiro[3.3]heptan-6-yl}-4-(6-methoxychinoxalin-2-yl)-2,5-dimethyl-piperazin-1-carboxamid;
(2R,6S)-N-{2-Benzyl-2-azaspiro[3.3]heptan-6-yl}-2,6-dimethyl-4-[6-(trifluormethyl)-1,3-benzo-thiazol-2-yl]piperazin-1-carboxamid;
(2R)-N-{2-Benzyl-2-azaspiro[3.3]heptan-6-yl}-2-methyl-4-(chinoxalin-2-yl)piperazin-1-carboxamid;
(2R)-N-{2-Benzyl-2-azaspiro[3.3]heptan-6-yl}-2-methyl-4-[6-(trifluormethyl)-1,3-benzothiazol-2-yl]piperazin-1-carboxamid;
(2R)-N-{2-Benzyl-2-azaspiro[3.3]heptan-6-yl}-4-(6-methoxychinoxalin-2-yl)-2-methylpiperazin-1-carboxamid;
(2R,6S)-N-{2-Benzyl-2-azaspiro[3.3]heptan-6-yl}-4-[3-fluor-5-(trifluormethyl)pyridin-2-yl]-2,6-dimethylpiperazin-1-carboxamid;
(2R,6S)-N-{2-Benzyl-2-azaspiro[3.3]heptan-6-yl}-4-(5-chlor-4-methylpyrimidin-2-yl)-2,6-dimethylpiperazin-1-carboxamid;
(2R,6S)-N-{2-Benzyl-2-azaspiro[3.3]heptan-6-yl}-4-(5-chlorpyrazin-2-yl)-2,6-dimethyl-piperazin-1-carboxamid;
(2R,6S)-4-(6-Amino-5-chlorpyrazin-2-yl)-N-{2-benzyl-2-azaspiro[3.3]heptan-6-yl}-2,6-dimethylpiperazin-1-carboxamid;
(2R,6S)-N-{2-Benzyl-2-azaspiro[3.3]heptan-6-yl}-4-(6-fluor-1,3-benzothiazol-2-yl)-2,6-dimethylpiperazin-1-carboxamid;
(2R,6R)-N-{2-Benzyl-2-azaspiro[3.3]heptan-6-yl}-4-(6-fluor-1,3-benzothiazol-2-yl)-2,6-dimethylpiperazin-1-carboxamid;
(2R,5S)-N-{2-Benzyl-2-azaspiro[3.3]heptan-6-yl}-4-(6-fluor-1,3-benzothiazol-2-yl)-2,5-dimethylpiperazin-1-carboxamid;
(2R)-N-{2-Benzyl-2-azaspiro[3.3]heptan-6-yl}-4-(6-fluor-1,3-benzothiazol-2-yl)-2-methylpiperazin-1-carboxamid;
(2R,6S)-N-{2-Benzyl-2-azaspiro[3.3]heptan-6-yl}-2,6-dimethyl-4-[6-(trifluormethyl)chinoxalin-2-yl]piperazin-1-carboxamid;
(2R,6R)-N-{2-Benzyl-2-azaspiro[3.3]heptan-6-yl}-2,6-dimethyl-4-[6-(trifluormethyl)chinoxalin-2-yl]piperazin-1-carboxamid;
(2R,5S)-N-{2-Benzyl-2-azaspiro[3.3]heptan-6-yl}-2,5-dimethyl-4-[6-(trifluormethyl)chinoxalin-2-yl]piperazin-1-carboxamid;
(2R)-N-{2-Benzyl-2-azaspiro[3.3]heptan-6-yl}-2-methyl-4-[6-(trifluormethyl)chinoxalin-2-yl]-piperazin-1-carboxamid;
(2R,6S)-N-{2-Benzyl-2-azaspiro[3.3]heptan-6-yl}-2,6-dimethyl-4-{[1,3]oxazolo[4,5-b]pyridin-2-yl}piperazin-1-carboxamid;
(2R,6R)-N-{2-Benzyl-2-azaspiro[3.3]heptan-6-yl}-2,6-dimethyl-4-{[1,3]oxazolo[4,5-b]pyridin-2-yl}piperazin-1-carboxamid;
(2R,6S)-N-{2-Benzyl-2-azaspiro[3.3]heptan-6-yl}-4-(6-methoxy-1,3-benzothiazol-2-yl)-2,6-dimethylpiperazin-1-carboxamid;
(2R,6R)-N-{2-Benzyl-2-azaspiro[3.3]heptan-6-yl}-4-(6-methoxy-1,3-benzothiazol-2-yl)-2,6-dimethylpiperazin-1-carboxamid;
(2R,6R)-4-(1,3-Benzothiazol-2-yl)-N-{2-benzyl-2-azaspiro[3.3]heptan-6-yl}-2,6-dimethyl-piperazin-1-carboxamid;
(2R,5S)-4-(1,3-Benzothiazol-2-yl)-N-{2-benzyl-2-azaspiro[3.3]heptan-6-yl}-2,5-dimethyl-piperazin-1-carboxamid;
(2R)-4-(1,3-Benzothiazol-2-yl)-N-{2-benzyl-2-azaspiro[3.3]heptan-6-yl}-2-methylpiperazin-1-carboxamid;
(2R,5S)-N-{2-Benzyl-2-azaspiro[3.3]heptan-6-yl}-4-(6-fluorchinoxalin-2-yl)-2,5-dimethyl-piperazin-1-carboxamid;
(2R,6S)-N-{2-Benzyl-2-azaspiro[3.3]heptan-6-yl}-4-[5-(difluormethoxy)pyrimidin-2-yl]-2,6-dimethylpiperazin-1-carboxamid;
(2R,6S)-4-[4-Amino-5-(trifluormethyl)pyrimidin-2-yl]-N-{2-benzyl-2-azaspiro[3.3]heptan-6-yl}-2,6-dimethylpiperazin-1-carboxamid;
(2R,6S)-N-{2-Benzyl-2-azaspiro[3.3]heptan-6-yl}-4-[5-(difluormethyl)pyrimidin-2-yl]-2,6-dimethylpiperazin-1-carboxamid;
(2R,6S)-N-{2-Benzyl-2-azaspiro[3.3]heptan-6-yl}-4-(5-fluorpyrimidin-2-yl)-2,6-dimethyl-piperazin-1-carboxamid;
(2R,6S)-N-{2-[(4-Chlorphenyl)methyl]-2-azaspiro[3.3]heptan-6-yl}-4-(5-cyanopyrimidin-2-yl)-2,6-dimethylpiperazin-1-carboxamid;
(2R,6R)-N-{2-[(4-Chlorphenyl)methyl]-2-azaspiro[3.3]heptan-6-yl}-4-(5-cyanopyrimidin-2-yl)-2,6-dimethylpiperazin-1-carboxamid;
(2R,5S)-N-{2-[(4-Chlorphenyl)methyl]-2-azaspiro[3.3]heptan-6-yl}-4-(5-cyanopyrimidin-2-yl)-2,5-dimethylpiperazin-1-carboxamid;
(2R)-N-{2-[(4-Chlorphenyl)methyl]-2-azaspiro[3.3]heptan-6-yl}-4-(5-cyanopyrimidin-2-yl)-2-methylpiperazin-1-carboxamid;
(2R,6R)-N-{2-[(4-Chlorphenyl)methyl]-2-azaspiro[3.3]heptan-6-yl}-2,6-dimethyl-4-[5-(trifluormethyl)pyrimidin-2-yl]piperazin-1-carboxamid;
(2R,5S)-N-{2-[(4-Chlorphenyl)methyl]-2-azaspiro[3.3]heptan-6-yl}-2,5-dimethyl-4-[5-(trifluormethyl)pyrimidin-2-yl]piperazin-1-carboxamid;
(2R)-N-{2-[(4-Chlorphenyl)methyl]-2-azaspiro[3.3]heptan-6-yl}-2-methyl-4-[5-(trifluormethyl)-pyrimidin-2-yl]piperazin-1-carboxamid;
(2R,6S)-N-{2-[(4-Chlorphenyl)methyl]-2-azaspiro[3.3]heptan-6-yl}-2,6-dimethyl-4-[5-(trifluormethyl)pyrazin-2-yl]piperazin-1-carboxamid;
(2R,6R)-N-{2-[(4-Chlorphenyl)methyl]-2-azaspiro[3.3]heptan-6-yl}-2,6-dimethyl-4-[5-(trifluormethyl)pyrazin-2-yl]piperazin-1-carboxamid;
(2R,5S)-N-{2-[(4-Chlorphenyl)methyl]-2-azaspiro[3.3]heptan-6-yl}-2,5-dimethyl-4-[5-(trifluormethyl)pyrazin-2-yl]piperazin-1-carboxamid;
(2R)-N-{2-[(4-Chlorphenyl)methyl]-2-azaspiro[3.3]heptan-6-yl}-2-methyl-4-[5-(trifluormethyl)-pyrazin-2-yl]piperazin-1-carboxamid;
(2R,6R)-4-(5-Cyanopyrimidin-2-yl)-N-{2-[(4-fluorphenyl)methyl]-2-azaspiro[3.3]heptan-6-yl}-2,6-dimethylpiperazin-1-carboxamid;
(2R,5S)-4-(5-Cyanopyrimidin-2-yl)-N-{2-[(4-fluorphenyl)methyl]-2-azaspiro[3.3]heptan-6-yl}-2,5-dimethylpiperazin-1-carboxamid;
(2R)-4-(5-Cyanopyrimidin-2-yl)-N-{2-[(4-fluorphenyl)methyl]-2-azaspiro[3.3]heptan-6-yl}-2-methylpiperazin-1-carboxamid;
(2R,5S)-N-{2-[(4-Fluorphenyl)methyl]-2-azaspiro[3.3]heptan-6-yl}-2,5-dimethyl-4-[5-(trifluormethyl)pyrimidin-2-yl]piperazin-1-carboxamid;
(2R)-N-{2-[(4-Fluorphenyl)methyl]-2-azaspiro[3.3]heptan-6-yl}-2-methyl-4-[5-(trifluormethyl)-pyrimidin-2-yl]piperazin-1-carboxamid;
(2R,6S)-N-{2-[(4-Fluorphenyl)methyl]-2-azaspiro[3.3]heptan-6-yl}-2,6-dimethyl-4-[5-(trifluormethyl)pyrazin-2-yl]piperazin-1-carboxamid;
(2R,6R)-N-{2-[(4-Fluorphenyl)methyl]-2-azaspiro[3.3]heptan-6-yl}-2,6-dimethyl-4-[5-(trifluormethyl)pyrazin-2-yl]piperazin-1-carboxamid;
(2R,5S)-N-{2-[(4-Fluorphenyl)methyl]-2-azaspiro[3.3]heptan-6-yl}-2,5-dimethyl-4-[5-(trifluormethyl) pyrazin-2-yl]piperazin-1-carboxamid;
(2R)-N-{2-[(4-Fluorphenyl)methyl]-2-azaspiro[3.3]heptan-6-yl}-2-methyl-4-[5-(trifluormethyl)-pyrazin-2-yl]piperazin-1-carboxamid;
(2R,6S)-N-{2-Benzyl-2-azaspiro[3.3]heptan-6-yl}-4-(6-fluorchinazolin-2-yl)-2,6-dimethyl-piperazin-1-carboxamid;
(2R,5S)-N-{2-Benzyl-2-azaspiro[3.3]heptan-6-yl}-4-(6-fluorchinazolin-2-yl)-2,5-dimethyl-piperazin-1-carboxamid;
(2R,6S)-N-{2-[(²H₅)Benzyl]-2-azaspiro[3.3]heptan-6-yl}-2,6-dimethyl-4-[5-(trifluormethyl)-pyrimidin-2-yl]piperazin-1-carboxamid;
(2R,6S)-2,6-Dimethyl-N-{2-[(²H₅;)phenyl(²H₁)methyl]-2-azaspiro[3.3]heptan-6-yl}-4-[5-(trifluormethyl)pyrimidin-2-yl]piperazin-1-carboxamid;
(2R,6S)-2,6-Dimethyl-N-{2-[(²H₅)phenyl(²H₂)methyl]-2-azaspiro[3.3]heptan-6-yl}-4-[5-(trifluormethyl)pyrimidin-2-yl]piperazin-1-carboxamid;
2-Benzyl-2-azaspiro[3.3]heptan-6-yl-(2R,6S)-4-[4-methoxy-5-(trifluormethyl)pyrimidin-2-yl]-2,6-dimethylpiperazin-1-carboxylat;
2-Benzyl-2-azaspiro[3.3]heptan-6-yl-(2R,6S)-4-[3-fluor-5-(trifluormethyl)pyridin-2-yl]-2,6-dimethylpiperazin-1-carboxylat;
2-Benzyl-2-azaspiro[3.3]heptan-6-yl-(2R,6S)-4-(5-chlorpyrimidin-2-yl)-2,6-dimethylpiperazin-1-carboxylat;
2-Benzyl-2-azaspiro[3.3]heptan-6-yl-(2R,6S)-4-(5-chlor-4-methylpyrimidin-2-yl)-2,6-dimethyl-piperazin-1-carboxylat;
2-Benzyl-2-azaspiro[3.3]heptan-6-yl-(2R,6S)-4-(5-chlorpyrazin-2-yl)-2,6-dimethylpiperazin-1-carboxylat;
N-{2-Benzyl-2-azaspiro[3.3]heptan-6-yl}-9-[5-(trifluormethyl)pyrimidin-2-yl]-3-oxa-7,9-diaza-bicyclo[3.3.1]nonan-7-carboxamid;
2-Benzyl-2-azaspiro[3.3]heptan-6-yl-(2R,6S)-4-(6-amino-5-chlorpyrazin-2-yl)-2,6-dimethyl-piperazin-1-carboxylat;
2-[(²H₅)Benzyl]-2-azaspiro[3.3]heptan-6-yl-(2R,5S)-2,5-dimethyl-4-[5-(trifluormethyl)pyrazin-2-yl]piperazin-1-carboxylat;
(2R,6S)-N-{2-Benzyl-2-azaspiro[3.3]heptan-6-yl}-4-(4-ethoxyphenyl)-2,6-dimethylpiperazin-1-carboxamid;
(2R,6S)-N-{2-Benzyl-2-azaspiro[3.3]heptan-6-yl}-4-(4-methoxyphenyl)-2,6-dimethylpiperazin-1-carboxamid;
(2R,6S)-N-{2-Benzyl-2-azaspiro[3.3]heptan-6-yl}-4-(4-fluor-3-methylphenyl)-2,6-dimethyl-piperazin-1-carboxamid;
(2R,6S)-N-{2-Benzyl-2-azaspiro[3.3]heptan-6-yl}-4-(4-cyano-3-methoxyphenyl)-2,6-dimethyl-piperazin-1-carboxamid;
(2R,5S)-N-{2-Benzyl-2-azaspiro[3.3]heptan-6-yl}-2,5-dimethyl-4-[4-(trifluormethyl)phenyl]-piperazin-1-carboxamid;
(2R)-N-{2-Benzyl-2-azaspiro[3.3]heptan-6-yl}-2-methyl-4-[4-(trifluormethyl)pheny|]piperazin-1-carboxamid;
(2R,6S)-N-{2-[(3-Methoxypyridin-4-yl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(2-Chlorpyridin-3-yl)methyl]-2-azaspiro[3.3]heptan-6-yl}-2,6-dimethyl-4-[5-(trifluormethyl)pyrimidin-2-yl]piperazin-1-carboxamid;
(2R,6S)-N-[2-(1H-Indol-4-ylmethyl)-2-azaspiro[3.3]heptan-6-yl]-2,6-dimethyl-4-[5-(trifluormethyl)pyrimidin-2-yl]piperazin-1-carboxamid;
(2R,6S)-2,6-Dimethyl-N-[2-(pyridin-2-ylmethyl)-2-azaspiro[3.3]heptan-6-yl]-4-[5-(trifluormethyl)pyrimidin-2-yl]piperazin-1-carboxamid;
(2R,6S)-N-{2-[(2-Chlorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-2,6-Dimethyl-N-[2-(1,3-thiazol-4-ylmethyl)-2-azaspiro[3.3]heptan-6-yl]-4-[5-(trifluormethyl)pyrimidin-2-yl]piperazin-1-carboxamid;
(2R,6S)-N-{2-[(1,3-Dimethyl-1H-pyrazol-5-yl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(4-Methoxypyridin-3-yl)methyl]-2-azaspiro[3.3]heptan-6-yl}-2,6-dimethyl-4-[5-(trifluormethyl)pyrimidin-2-yl]piperazin-1-carboxamid;
(2R,6S)-N-[2-(1-Benzofuran-5-ylmethyl)-2-azaspiro[3.3]heptan-6-yl]-2,6-dimethyl-4-[5-(trifluormethyl)pyrimidin-2-yl]piperazin-1-carboxamid;
(2R,6S)-2,6-Dimethyl-N-{2-[(1-methyl-1H-imidazol-2-yl)methyl]-2-azaspiro[3.3]heptan-6-yl}-4-[5-(trifluormethyl)pyrimidin-2-yl]piperazin-1-carboxamid;
(2R,6S)-2,6-Dimethyl-N-{2-[(4-methyl-1,3-thiazol-2-yl)methyl]-2-azaspiro[3.3]heptan-6-yl}-4-[5-(trifluormethyl)pyrimidin-2-yl]piperazin-1-carboxamid;
(2R,6S)-2,6-Dimethyl-N-{2-[(4-methyl-1H-imidazol-5-yl)methyl]-2-azaspiro[3.3]heptan-6-yl}-4-[5-(trifluormethyl)pyrimidin-2-yl]piperazin-1-carboxamid;
(2R,6S)-N-{2-[(3-Chlorpyridin-4-yl)methyl]-2-azaspiro[3.3]heptan-6-yl}-2,6-dimethyl-4-[5-(trifluormethyl)pyrimidin-2-yl]piperazin-1-carboxamid;
(2R,6S)-2,6-Dimethyl-N-[2-(pyridin-3-ylmethyl)-2-azaspiro[3.3]heptan-6-yl]-4-[5-(trifluormethyl)pyrimidin-2-yl]piperazin-1-carboxamid;
(2R,6S)-N-{2-[(4-Methoxypyridin-2-yl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(6-Methoxypyridin-3-yl)methyl]-2-azaspiro[3.3]heptan-6-yl}-2,6-dimethyl-4-[5-(trifluormethyl)pyrimidin-2-yl]piperazin-1-carboxamid;
(2R,6S)-N-[2-(1H-Imidazol-4-ylmethyl)-2-azaspiro[3.3]heptan-6-yl]-2,6-dimethyl-4-[5-(trifluormethyl)pyrimidin-2-yl]piperazin-1-carboxamid;
(2R,6S)-N-{2-[(6-Chlorpyridin-3-yl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(2-Fluorpyridin-3-yl)methyl]-2-azaspiro[3.3]heptan-6-yl}-2,6-dimethyl-4-[5-(trifluormethyl)pyrimidin-2-yl]piperazin-1-carboxamid;
(2R,6S)-2,6-Dimethyl-N-[2-(1H-pyrrol-2-ylmethyl)-2-azaspiro[3.3]heptan-6-yl]-4-[5-(trifluormethyl)pyrimidin-2-yl]piperazin-1-carboxamid;
(2R,6S)-N-{2-[(2,5-Difluorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-2,6-Dimethyl-N-[2-(2-phenylethyl)-2-azaspiro[3.3]heptan-6-yl]-4-[5-(trifluormethyl)-pyrimidin-2-yl]piperazin-1-carboxamid;
(2R,6S)-2,6-Dimethyl-N-[2-(4-phenylbutyl)-2-azaspiro[3.3]heptan-6-yl]-4-[5-(trifluormethyl)-pyrimidin-2-yl]piperazin-1-carboxamid;
(2R,6S)-N-[2-(Cyclohex-1-en-1-ylmethyl)-2-azaspiro[3.3]heptan-6-yl]-2,6-dimethyl-4-[5-(trifluormethyl)pyrimidin-2-yl]piperazin-1-carboxamid;
(2R,6S)-2,6-Dimethyl-N-{2-[(4-nitrophenyl)methyl]-2-azaspiro[3.3]heptan-6-y]}-4-[5-(trifluormethyl)pyrimidin-2-yl]piperazin-1-carboxamid;
(2R,6S)-N-(2-{[4-(Dimethylamino)phenyl]methyl}-2-azaspiro[3.3]heptan-6-yl)-2,6-dimethyl-4-[5-(trifluormethyl)pyrimidin-2-yl]piperazin-1-carboxamid;
(2R,6S)-2,6-Dimethyl-N-(2-{[4-(propan-2-yl)phenyl]methyl}-2-azaspiro[3.3]heptan-6-yl)-4-[5-(trifluormethyl)pyrimidin-2-yl]piperazin-1-carboxamid;
(2R,6S)-2,6-Dimethyl-N-[2-(pyridin-4-ylmethyl)-2-azaspiro[3.3]heptan-6-yl]-4-[5-(trifluormethyl)pyrimidin-2-yl]piperazin-1-carboxamid;
(2R,6S)-N-{2-[(4-Ethylphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(4-Hydroxyphenyl)methyl]-2-azaspiro[3.3]heptan-6-yl}-2,6-dimethyl-4-[5-(trifluormethyl)pyrimidin-2-yl]piperazin-1-carboxamid;
Methyl-4-[(6-{[(2R,6S)-2,6-dimethyl-4-[5-(trifluormethyl)pyrimidin-2-yl]piperazin-1-carbonyl]-amino}-2-azaspiro[3.3]heptan-2-yl)methyl]benzoat;
(2R,6S)-N-{2-[(4-Acetamidophenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-(2-{[4-(Difluormethoxy)phenyl]methyl}-2-azaspiro[3.3]heptan-6-yl)-2,6-dimethyl-4-[5-(trifluormethyl)pyrimidin-2-yl]piperazin-1-carboxamid;
(2R,6S)-2,6-Dimethyl-N-(2-{[4-(trifluormethyl)phenyl]methyl}-2-azaspiro[3.3]heptan-6-yl)-4-[5-(trifluormethyl)pyrimidin-2-yl]piperazin-1-carboxamid;
(2R,6S)-N-{2-[(4-Carbamimidoylphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-{2-[(4-Chlorphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-N-(2-{Imidazo[1,2-a]pyridin-6-ylmethyl}-2-azaspiro[3.3]heptan-6-yl)-2,6-dimethyl-4-[5-(trifluormethyl)pyrimidin-2-yl]piperazin-1-carboxamid;
(2R,6S)-N-[2-(Adamantan-1-ylmethyl)-2-azaspiro[3.3]heptan-6-yl]-2,6-dimethyl-4-[5-(trifluormethyl)pyrimidin-2-yl]piperazin-1-carboxamid;
(2R,6R)-N-{2-[(4-Fluorphenyl)methyl]-2-azaspiro[3.3]heptan-6-yl}-2,6-dimethyl-4-[5-(trifluormethyl)pyrimidin-2-yl]piperazin-1-carboxamid;
(2R,6R)-N-{2-[(4-Carbamoylphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-4-(5-Cyanopyrimidin-2-yl)-N-{2-[(4-fluorphenyl)methyl]-2-azaspiro[3.3]heptan-6-yl}-2,6-dimethylpiperazin-1-carboxamid;
(2R,6S)-N-{2-[(4-Carbamoylphenyl)methyl]-2-azaspiro[3.3]heptan-6-yl}-4-(5-cyanopyrimidin-2-yl)-2,6-dimethylpiperazin-1-carboxamid;
(2R,6S)-N-[2-(Cyanomethyl)-2-azaspiro[3.3]heptan-6-yl]-2,6-dimethyl-4-[5-(trifluormethyl)-pyrimidin-2-yl]piperazin-1-carboxamid;
(2R,6S)-N-{2-[(3,3-Difluorcyclobutyl)methyl]-2-azaspiro[3.3]heptan-6-yl}-2,6-dimethyl-4-[5-(trifluormethyl)pyrimidin-2-yl]piperazin-1-carboxamid;
(2R,6S)-2,6-Dimethyl-N-[2-(1-phenylethyl)-2-azaspiro[3.3]heptan-6-yl]-4-[5-(trifluormethyl)-pyrimidin-2-yl]piperazin-1-carboxamid;
(2R,6S)-2,6-Dimethyl-N-[2-(1H-pyrrol-3-ylmethyl)-2-azaspiro[3.3]heptan-6-yl]-4-[5-(trifluormethyl)pyrimidin-2-yl]piperazin-1-carboxamid;
(2R,6S)-N-(2-{[4-(Dimethylcarbamoyl)phenyl]methyl}-2-azaspiro[3.3]heptan-6-yl)-2,6-dimethyl-4-[5-(trifluormethyl)pyrimidin-2-yl]piperazin-1-carboxamid;
(2R,6S)-N-{2-[(2-Hydroxyphenyl)methyl]-2-azaspiro[3.3]heptan-6-yl}-2,6-dimethyl-4-[5-(trifluormethyl)pyrimidin-2-yl]piperazin-1-carboxamid;
(2R,6S)-2,6-Dimethyl-N-{2-[(3-methylthiophen-2-yl)methyl]-2-azaspiro[3.3]heptan-6-yl}-4-[5-(trifluormethyl)pyrimidin-2-yl]piperazin-1-carboxamid;
(2R,6S)-2,6-Dimethyl-N-{2-[(5-methylthiophen-2-yl)methyl]-2-azaspiro[3.3]heptan-6-y]}-4-[5-(trifluormethyl)pyrimidin-2-yl]piperazin-1-carboxamid;
(2R,6S)-N-{2-[(5-Chlorthiophen-2-yl)methyl]-2-azaspiro[3.3]heptan-6-yl}-2,6-dimethyl-4-[5-(trifluormethyl)pyrimidin-2-yl]piperazin-1-carboxamid;
2-Benzyl-6-(difluormethyl)-2-azaspiro[3.3]heptan-6-yl-(2R,6S)-2,6-dimethyl-4-[5-(trifluormethyl)pyrimidin-2-yl]piperazin-1-carboxylat;
2-Benzyl-6-(difluormethyl)-2-azaspiro[3.3]heptan-6-yl-(2R,6S)-2,6-dimethyl-4-[5-(trifluormethyl)pyrazin-2-yl]piperazin-1-carboxylat;
2-Benzyl-6-(difluormethyl)-2-azaspiro[3.3]heptan-6-yl-(2R,6S)-2,6-dimethyl-4-[6-(trifluormethyl)pyridazin-3-yl]piperazin-1-carboxylat;
2-Benzyl-6-(difluormethy|)-2-azaspiro[3.3]heptan-6-yl-(2R,6R)-2,6-dimethyl-4-[5-(trifluormethyl)pyrimidin-2-yl]piperazin-1-carboxylat;
2-Benzyl-6-(difluormethy|)-2-azaspiro[3.3]heptan-6-yl-(2R,6R)-2,6-dimethyl-4-[5-(trifluormethyl)pyrazin-2-yl]piperazin-1-carboxylat;
2-Benzyl-6-(difluormethyl)-2-azaspiro[3.3]heptan-6-yl-(2R,5S)-2,5-dimethyl-4-[5-(trifluormethyl)pyrimidin-2-yl]piperazin-1-carboxylat;
2-Benzyl-6-(difluormethyl)-2-azaspiro[3.3]heptan-6-yl-(2R,5S)-2,5-dimethyl-4-[5-(trifluormethyl)pyrazin-2-yl]piperazin-1-carboxylat;
2-Benzyl-6-(difluormethyl|)-2-azaspiro[3.3]heptan-6-yl-(2R)-4-(5-cyanopyrimidin-2-yl)-2-methylpiperazin-1-carboxylat;
2-Benzyl-6-(difluormethyl)-2-azaspiro[3.3]heptan-6-yl-(2R)-2-methyl-4-[5-(trifluormethyl)-pyrimidin-2-yl]piperazin-1-carboxylat;
2-Benzyl-6-(difluormethyl)-2-azaspiro[3.3]heptan-6-yl-(2R)-2-methyl-4-[5-(trifluormethyl)-pyrazin-2-yl]piperazin-1-carboxylat;
2-Benzyl-6-methyl-2-azaspiro[3.3]heptan-6-yl-(2R,6S)-4-(5-cyanopyrimidin-2-yl)-2,6-dimethylpiperazin-1-carboxylat;
2-Benzyl-6-methyl-2-azaspiro[3.3]heptan-6-yl-(2R,6S)-2,6-dimethyl-4-[5-(trifluormethyl)-pyrimidin-2-yl]piperazin-1-carboxylat;
2-Benzyl-6-methyl-2-azaspiro[3.3]heptan-6-yl-(2R,6S)-2,6-dimethyl-4-[5-(trifluormethyl)-pyrazin-2-yl]piperazin-1-carboxylat;
2-Benzyl-6-methyl-2-azaspiro[3.3]heptan-6-yl-(2R,6R)-4-(5-cyanopyrimidin-2-yl)-2,6-dimethylpiperazin-1-carboxylat;
2-Benzyl-6-methyl-2-azaspiro[3.3]heptan-6-yl-(2R,6R)-2,6-dimethyl-4-[5-(trifluormethyl)-pyrimidin-2-yl]piperazin-1-carboxylat;
2-Benzyl-6-methyl-2-azaspiro[3.3]heptan-6-yl-(2R,6R)-2,6-dimethyl-4-[5-(trifluormethyl)-pyrazin-2-yl]piperazin-1-carboxylat;
2-Benzyl-6-methyl-2-azaspiro[3.3]heptan-6-yl-(2R,5S)-4-(5-cyanopyrimidin-2-yl)-2,5-dimethylpiperazin-1-carboxylat;
2-Benzyl-6-methyl-2-azaspiro[3.3]heptan-6-yl-(2R,5S)-2,5-dimethyl-4-[5-(trifluormethyl)-pyrimidin-2-yl]piperazin-1-carboxylat;
2-Benzyl-6-methyl-2-azaspiro[3.3]heptan-6-yl-(2R,5S)-2,5-dimethyl-4-[5-(trifluormethyl)-pyrazin-2-yl]piperazin-1-carboxylat;
2-[(²H₅)Phenyl(²H₁)methyl]-2-azaspiro[3.3]heptan-6-yl-(2R,5S)-2,5-dimethyl-4-[5-(trifluormethyl)pyrazin-2-yl]piperazin-1-carboxylat;
2-[(²H₅)Phenyl(²H₂)methyl]-2-azaspiro[3.3]heptan-6-yl-(2R,5S)-2,5-dimethyl-4-[5-(trifluormethyl)pyrazin-2-yl]piperazin-1-carboxylat;
2-Benzyl-2-azaspiro[3.3]heptan-6-yl-(2R,6R)-4-(6-fluorchinoxalin-2-yl)-2,6-dimethylpiperazin-1-carboxylat;
2-Benzyl-2-azaspiro[3.3]heptan-6-yl-(2R,5S)-4-(6-fluorchinoxalin-2-yl)-2,5-dimethylpiperazin-1-carboxylat;
2-Benzyl-2-azaspiro[3.3]heptan-6-yl-(2R,6S)-2,6-dimethyl-4-[6-(trifluormethyl)-1,3-benzo-thiazol-2-yl]piperazin-1-carboxylat;
2-Benzyl-2-azaspiro[3.3]heptan-6-yl-(2R,6S)-2,6-dimethyl-4-[6-(trifluormethyl)-[1,3]thiazolo-[5,4-b]pyridin-2-yl]piperazin-1-carboxylat;
2-Benzyl-2-azaspiro[3.3]heptan-6-yl-(2R,6R)-2,6-dimethyl-4-[6-(trifluormethyl)-[1,3]thiazolo-[5,4-b]pyridin-2-yl]piperazin-1-carboxylat;
2-Benzyl-2-azaspiro[3.3]heptan-6-yl-(2R,5S)-2,5-dimethyl-4-[6-(trifluormethyl)-[1,3]thiazolo-[5,4-b]pyridin-2-yl]piperazin-1-carboxylat;
2-Benzyl-2-azaspiro[3.3]heptan-6-yl-(2R,6S)-4-(6-fluor-1,3-benzothiazol-2-yl)-2,6-dimethyl-piperazin-1-carboxylat;
2-Benzyl-2-azaspiro[3.3]heptan-6-yl-(2R,6R)-4-(6-fluor-1,3-benzothiazol-2-yl)-2,6-dimethyl-piperazin-1-carboxylat;
2-Benzyl-2-azaspiro[3.3]heptan-6-yl-(2R,5S)-4-(6-fluor-1,3-benzothiazol-2-yl)-2,5-dimethyl-piperazin-1-carboxylat;
2-Benzyl-2-azaspiro[3.3]heptan-6-yl-(2R,6R)-2,6-dimethyl-4-[6-(trifluormethyl)-1,3-benzo-thiazol-2-yl]piperazin-1-carboxylat;
2-Benzyl-2-azaspiro[3.3]heptan-6-yl-(2R,5S)-2,5-dimethyl-4-[6-(trifluormethyl)-1,3-benzo-thiazol-2-yl]piperazin-1-carboxylat;
2-[(²H₅)Benzyl]-2-azaspiro[3.3]heptan-6-yl-(2R,6S)-2,6-dimethyl-4-[5-(trifluormethyl)pyrimidin-2-yl]piperazin-1-carboxylat;
2-[(²H₅)Benzyl]-2-azaspiro[3.3]heptan-6-yl-(2R,6S)-2,6-dimethyl-4-[5-(trifluormethyl)pyrazin-2-yl]piperazin-1-carboxylat;
2-[(²H₅)Phenyl(²H₁)methyl]-2-azaspiro[3.3]heptan-6-yl-(2R,6S)-2,6-dimethyl-4-[5-(trifluormethyl)pyrimidin-2-yl]piperazin-1-carboxylat;
2-[(²H₅)Phenyl(²H₁)methyl]-2-azaspiro[3.3]heptan-6-yl-(2R,6S)-2,6-dimethyl-4-[5-(trifluormethyl)pyrazin-2-yl]piperazin-1-carboxylat;
2-Benzyl-2-azaspiro[3.3]heptan-6-yl-(2R,6S)-2,6-dimethyl-4-(chinazolin-2-yl)piperazin-1-carboxylat;
2-Benzyl-2-azaspiro[3.3]heptan-6-yl-(2R,6S)-2,6-dimethyl-4-[6-(trifluormethyl)chinoxalin-2-yl]piperazin-1-carboxylat;
2-Benzyl-2-azaspiro[3.3]heptan-6-yl-(2R,6S)-4-(6-methoxychinoxalin-2-yl)-2,6-dimethyl-piperazin-1-carboxylat;
2-Benzyl-2-azaspiro[3.3]heptan-6-yl-(2R,5S)-2,5-dimethyl-4-[6-(trifluormethyl)chinoxalin-2-yl]piperazin-1-carboxylat;
2-[(4-Fluorphenyl)methyl]-2-azaspiro[3.3]heptan-6-yl-(2R,6S)-4-(6-fluor-1,3-benzothiazol-2-yl)-2,6-dimethylpiperazin-1-carboxylat;
2-[(4-Fluorphenyl)methyl]-2-azaspiro[3.3]heptan-6-yl-(2R,6R)-4-(6-fluor-1,3-benzothiazol-2-yl)-2,6-dimethylpiperazin-1-carboxylat;
2-[(4-Fluorphenyl)methyl]-2-azaspiro[3.3]heptan-6-yl-(2R,5S)-4-(6-fluor-1,3-benzothiazol-2-yl)-2,5-dimethylpiperazin-1-carboxylat;
2-[(4-Fluorphenyl)methyl]-2-azaspiro[3.3]heptan-6-yl-(2R,6S)-2,6-dimethyl-4-[6-(trifluormethyl)-1,3-benzothiazol-2-yl]piperazin-1-carboxylat;
2-[(4-Fluorphenyl)methyl]-2-azaspiro[3.3]heptan-6-yl-(2R,6R)-2,6-dimethyl-4-[6-(trifluormethyl)-1,3-benzothiazol-2-yl]piperazin-1-carboxylat;
2-[(4-Fluorphenyl)methyl]-2-azaspiro[3.3]heptan-6-yl-(2R,5S)-2,5-dimethyl-4-[6-(trifluormethyl)-1,3-benzothiazol-2-yl]piperazin-1-carboxylat:
2-[(²H₅)Phenyl(²H₂)methyl]-2-azaspiro[3.3]heptan-6-yl-(2R,6S)-2,6-dimethyl-4-[5-(trifluormethyl)pyrimidin-2-yl]piperazin-1-carboxylat;
2-[(²H₅)Phenyl(²H₂)methyl]-2-azaspiro[3.3]heptan-6-yl-(2R,6S)-2,6-dimethyl-4-[5-(trifluormethyl)pyrazin-2-yl]piperazin-1-carboxylat:
2-[(4-Fluorphenyl)methyl]-2-azaspiro[3.3]heptan-6-yl-(2R,6S)-2,6-dimethyl-4-(chinoxalin-2-yl)piperazin-1-carboxylat;
2-[(4-Fluorphenyl)methyl]-2-azaspiro[3.3]heptan-6-yl-(2R,6S)-2,6-dimethyl-4-(chinazolin-2-yl)piperazin-1-carboxylat;
2-[(4-Fluorphenyl)methyl]-2-azaspiro[3.3]heptan-6-yl-(2R,6S)-2,6-dimethyl-4-[6-(trifluormethyl)chinoxalin-2-yl]piperazin-1-carboxylat;
2-[(4-Fluorphenyl)methyl]-2-azaspiro[3.3]heptan-6-yl-(2R,5S)-2,5-dimethyl-4-(chinoxalin-2-yl)piperazin-1-carboxylat;
2-[(4-Fluorphenyl)methyl]-2-azaspiro[3.3]heptan-6-yl-(2R,6S)-4-(6-methoxychinoxalin-2-yl)-2,6-dimethylpiperazin-1-carboxylat;
2-[(4-Fluorphenyl)methyl]-2-azaspiro[3.3]heptan-6-yl-(2R,5S)-4-(6-methoxychinoxalin-2-yl)-2,5-dimethylpiperazin-1-carboxylat;
2-[(4-Fluorphenyl)methyl]-2-azaspiro[3.3]heptan-6-yl-(2R,5S)-4-(6-fluorchinoxalin-2-yl)-2,5-dimethylpiperazin-1-carboxylat;
2-[(4-Fluorphenyl)methyl]-2-azaspiro[3.3]heptan-6-yl-(2R,6R)-2,6-dimethyl-4-[5-(trifluormethyl)pyrazin-2-yl]piperazin-1-carboxylat;
2-[(4-Fluorphenyl)methyl]-2-azaspiro[3.3]heptan-6-yl-(2R,6S)-2,6-dimethyl-4-[5-(trifluormethyl)pyrazin-2-yl]piperazin-1-carboxylat;
2-[(4-Fluorphenyl)methyl]-2-azaspiro[3.3]heptan-6-yl-(2R,5S)-2,5-dimethyl-4-[5-(trifluormethyl)pyrazin-2-yl]piperazin-1-carboxylat;
2-[(4-Carbamoylphenyl)methyl]-2-azaspiro[3.3]heptan-6-yl-(2R,6S)-4-(6-fluor-1,3-benzo-thiazol-2-yl)-2,6-dimethylpiperazin-1-carboxylat;
2-[(4-Carbamoylphenyl)methyl]-2-azaspiro[3.3]heptan-6-yl-(2R,6R)-4-(6-fluor-1,3-benzo-thiazol-2-yl)-2,6-dimethylpiperazin-1-carboxylat;
2-[(4-Carbamoylphenyl)methyl]-2-azaspiro[3.3]heptan-6-yl-(2R,6S)-2,6-dimethyl-4-[6-(trifluormethyl)chinoxalin-2-yl]piperazin-1-carboxylat;
2-[(4-Carbamoylphenyl)methyl]-2-azaspiro[3.3]heptan-6-yl-(2R,5S)-2,5-dimethyl-4-(chinoxalin-2-yl)piperazin-1-carboxylat;
2-[(4-Carbamoylphenyl)methyl]-2-azaspiro[3.3]heptan-6-yl-(2R,6S)-4-(6-methoxychinoxalin-2-yl)-2,6-dimethylpiperazin-1-carboxylat;
2-[(4-Carbamoylphenyl)methyl]-2-azaspiro[3.3]heptan-6-yl-(2R,5S)-4-(6-methoxychinoxalin-2-yl)-2,5-dimethylpiperazin-1-carboxylat;
2-[(4-Carbamoylphenyl)methyl]-2-azaspiro[3.3]heptan-6-yl-(2R,6R)-2,6-dimethyl-4-[5-(trifluormethyl)pyrazin-2-yl]piperazin-1-carboxylat;
2-[(4-Carbamoylphenyl)methyl]-2-azaspiro[3.3]heptan-6-yl-(2R,6S)-2,6-dimethyl-4-[5-(trifluormethyl)pyrazin-2-yl]piperazin-1-carboxylat;
2-[(4-Carbamoylphenyl)methyl]-2-azaspiro[3.3]heptan-6-yl-(2R,5S)-2,5-dimethyl-4-[5-(trifluormethyl)pyrazin-2-yl]piperazin-1-carboxylat;
2-(Pyridin-4-ylmethyl)-2-azaspiro[3.3]heptan-6-yl-(2R,6S)-4-(6-fluor-1,3-benzothiazol-2-yl)-2,6-dimethylpiperazin-1-carboxylat;
2-(Pyridin-4-ylmethyl)-2-azaspiro[3.3]heptan-6-yl-(2R,6R)-4-(6-fluor-1,3-benzothiazol-2-yl)-2,6-dimethylpiperazin-1-carboxylat;
2-(Pyridin-4-ylmethyl)-2-azaspiro[3.3]heptan-6-yl-(2R,6S)-2,6-dimethyl-4-[6-(trifluormethyl)-1,3-benzothiazol-2-yl]piperazin-1-carboxylat;
2-(Pyridin-4-ylmethyl)-2-azaspiro[3.3]heptan-6-yl-(2R,6S)-2,6-dimethyl-4-(chinazolin-2-yl)-piperazin-1-carboxylat;
2-(Pyridin-4-ylmethyl)-2-azaspiro[3.3]heptan-6-yl-(2R,6S)-4-(6-methoxychinoxalin-2-yl)-2,6-dimethylpiperazin-1-carboxylat;
2-(Pyridin-4-ylmethyl)-2-azaspiro[3.3]heptan-6-yl-(2R,5S)-4-(6-methoxychinoxalin-2-yl)-2,5-dimethylpiperazin-1-carboxylat;
2-(Pyridin-4-ylmethyl)-2-azaspiro[3.3]heptan-6-yl-(2R,5S)-4-(6-fluorchinoxalin-2-yl)-2,5-dimethylpiperazin-1-carboxylat;
2-(Pyridin-4-ylmethyl)-2-azaspiro[3.3]heptan-6-yl-(2R,6R)-2,6-dimethyl-4-[5-(trifluormethyl)-pyrazin-2-yl]piperazin-1-carboxylat;
2-(Pyridin-4-ylmethyl)-2-azaspiro[3.3]heptan-6-yl-(2R,6S)-2,6-dimethyl-4-[5-(trifluormethyl)-pyrazin-2-yl]piperazin-1-carboxylat;
2-(Pyridin-4-ylmethyl)-2-azaspiro[3.3]heptan-6-yl-(2R,5S)-2,5-dimethyl-4-[5-(trifluormethyl)-pyrazin-2-yl]piperazin-1-carboxylat;
2-Benzyl-2-azaspiro[3.3]heptan-6-yl-(2R,6S)-2,6-dimethyl-4-[6-(trifluormethyl)-1,3-benzoxazol-2-yl]piperazin-1-carboxylat;
2-Benzyl-2-azaspiro[3.3]heptan-6-yl-(2R,6R)-2,6-dimethyl-4-[6-(trifluormethyl)-1,3-benzoxazol-2-yl]piperazin-1-carboxylat;
2-Benzyl-2-azaspiro[3.3]heptan-6-yl-(2R,5S)-2,5-dimethyl-4-[6-(trifluormethyl)-1,3-benzoxazol-2-yl]piperazin-1-carboxylat;
2-Benzyl-2-azaspiro[3.3]heptan-6-yl-(2R,6S)-2,6-dimethyl-4-[6-(trifluormethyl)-[1,3]thiazolo-[4,5-b]pyridin-2-yl]piperazin-1-carboxylat;
2-Benzyl-2-azaspiro[3.3]heptan-6-yl-(2R,6R)-2,6-dimethyl-4-[6-(trifluormethyl)-[1,3]thiazolo-[4,5-b]pyridin-2-yl]piperazin-1-carboxylat;
2-Benzyl-2-azaspiro[3.3]heptan-6-yl-(2R,5S)-2,5-dimethyl-4-[6-(trifluormethyl)-[1,3]thiazolo-[4,5-b]pyridin-2-yl]piperazin-1-carboxylat;
2-Benzyl-2-azaspiro[3.3]heptan-6-yl-(2R,6S)-4-(6-cyano-1,3-benzothiazol-2-yl)-2,6-dimethyl-piperazin-1-carboxylat;
2-Benzyl-2-azaspiro[3.3]heptan-6-yl-(2R,5S)-4-(6-cyano-1,3-benzothiazol-2-yl)-2,5-dimethyl-piperazin-1-carboxylat;
2-Benzyl-2-azaspiro[3.3]heptan-6-yl-7-(6-fluor-1,3-benzothiazol-2-yl)-3-oxa-7,9-diazabicyclo-[3.3.1]nonan-9-carboxylat;
2-Benzyl-2-azaspiro[3.3]heptan-6-yl-7-[6-(trifluormethyl)-1,3-benzothiazol-2-yl]-3-oxa-7,9-diazabicyclo[3.3.1]nonan-9-carboxylat;
2-Benzyl-2-azaspiro[3.3]heptan-6-yl-(2R,6S)-4-(5-fluor-1,3-benzothiazol-2-yl)-2,6-dimethyl-piperazin-1-carboxylat;
2-Benzyl-2-azaspiro[3.3]heptan-6-yl-(2R,6R)-4-(5-fluor-1,3-benzothiazol-2-yl)-2,6-dimethyl-piperazin-1-carboxylat;
2-Benzyl-2-azaspiro[3.3]heptan-6-yl-(2R,5S)-4-(5-fluor-1,3-benzothiazol-2-yl)-2,5-dimethyl-piperazin-1-carboxylat;
2-Benzyl-2-azaspiro[3.3]heptan-6-yl-(2R,6S)-4-(5-fluor-1,3-benzoxazol-2-yl)-2,6-dimethyl-piperazin-1-carboxylat;
2-Benzyl-2-azaspiro[3.3]heptan-6-yl-(2R,6R)-4-(5-fluor-1,3-benzoxazol-2-yl)-2,6-dimethyl-piperazin-1-carboxylat;
2-Benzyl-2-azaspiro[3.3]heptan-6-yl-(2R,5S)-4-(5-fluor-1,3-benzoxazol-2-yl)-2,5-dimethyl-piperazin-1-carboxylat;
2-Benzyl-2-azaspiro[3.3]heptan-6-yl-(2R,6S)-4-(6-fluor-1,3-benzoxazol-2-yl)-2,6-dimethyl-piperazin-1-carboxylat;
2-Benzyl-2-azaspiro[3.3]heptan-6-yl-(2R,6R)-4-(6-fluor-1,3-benzoxazol-2-yl)-2,6-dimethyl-piperazin-1-carboxylat;
2-Benzyl-2-azaspiro[3.3]heptan-6-yl-(2R,5S)-4-(6-fluor-1,3-benzoxazol-2-yl)-2,5-dimethyl-piperazin-1-carboxylat;
2-Benzyl-2-azaspiro[3.3]heptan-6-yl-(2R,6S)-4-(5,6-difluor-1,3-benzothiazol-2-yl)-2,6-dimethylpiperazin-1-carboxylat;
2-Benzyl-2-azaspiro[3.3]heptan-6-yl-(2R,6R)-4-(5,6-difluor-1,3-benzothiazol-2-yl)-2,6-dimethylpiperazin-1-carboxylat;
2-Benzyl-2-azaspiro[3.3]heptan-6-yl-(2R,5S)-4-(5,6-difluor-1,3-benzothiazol-2-yl)-2,5-dimethylpiperazin-1-carboxylat;
2-Benzyl-2-azaspiro[3.3]heptan-6-yl-(2R,6S)-4-{6-fluor-[1,3]thiazolo[5,4-b]pyridin-2-yl}-2,6-dimethylpiperazin-1-carboxylat;
2-Benzyl-2-azaspiro[3.3]heptan-6-yl-(2R,6S)-4-{6-fluor-[1,3]thiazolo[4,5-b]pyridin-2-yl}-2,6-dimethylpiperazin-1-carboxylat;
2-Benzyl-2-azaspiro[3.3]heptan-6-yl-(2R,6S)-4-{5-fluor-[1,3]thiazolo[5,4-b]pyridin-2-yl}-2,6-dimethylpiperazin-1-carboxylat;
2-Benzyl-2-azaspiro[3.3]heptan-6-yl-(2R,6S)-4-(6-fluorchinazolin-2-yl)-2,6-dimethylpiperazin-1-carboxylat;
2-Benzyl-2-azaspiro[3.3]heptan-6-yl-(2R,6S)-2,6-dimethyl-4-{pyrido[2,3-b]pyrazin-3-yl}-piperazin-1-carboxylat;
2-[(4-Fluorphenyl)methyl]-2-azaspiro[3.3]heptan-6-yl-(2R,6S)-2,6-dimethyl-4-[5-(trifluormethyl)pyrimidin-2-yl]piperazin-1-carboxylat;
2-[(4-Fluorphenyl)methyl]-2-azaspiro[3.3]heptan-6-yl-(2R,5S)-2,5-dimethyl-4-[5-(trifluormethyl)pyrimidin-2-yl]piperazin-1-carboxylat;
2-[(4-Fluorphenyl)methyl]-2-azaspiro[3.3]heptan-6-yl-(2R)-2-methyl-4-[5-(trifluormethyl)-pyrimidin-2-yl]piperazin-1-carboxylat;
2-[(4-Fluorphenyl)methyl]-2-azaspiro[3.3]heptan-6-yl-(2R)-2-methyl-4-[5-(trifluormethyl)-pyrazin-2-yl]piperazin-1-carboxylat;
2-Benzyl-2-azaspiro[3.3]heptan-6-yl-(2R,6S)-2,6-dimethyl-4-(1,6-naphthyridin-2-yl)piperazin-1-carboxylat;
2-Benzyl-2-azaspiro[3.3]heptan-6-yl-(2R,6S)-2,6-dimethyl-4-{pyrido[2,3-b]pyrazin-6-yl}-piperazin-1-carboxylat;
2-[(4-Fluorphenyl)methyl]-2-azaspiro[3.3]heptan-6-yl-7-[5-(trifluormethyl)pyrimidin-2-yl]-3-oxa-7,9-diazabicyclo[3.3.1]nonan-9-carboxylat;
2-Benzyl-2-azaspiro[3.3]heptan-6-yl-(2R,6S)-4-[5-(difluormethoxy)pyrimidin-2-yl]-2,6-dimethylpiperazin-1-carboxylat;
2-Benzyl-2-azaspiro[3.3]heptan-6-yl-(2R,6S)-4-[4-amino-5-(trifluormethyl)pyrimidin-2-yl]-2,6-dimethylpiperazin-1-carboxylat;
2-Benzyl-2-azaspiro[3.3]heptan-6-yl-(2R,6S)-4-[5-(difluormethyl)pyrimidin-2-yl]-2,6-dimethyl-piperazin-1-carboxylat;
2-[(4-Fluorphenyl)methyl]-2-azaspiro[3.3]heptan-6-yl-(2R,6S)-4-[5-(difluormethoxy)pyrimidin-2-yl]-2,6-dimethylpiperazin-1-carboxylat;
2-[(4-Fluorphenyl)methyl]-2-azaspiro[3.3]heptan-6-yl-(2R,6S)-4-[4-amino-5-(trifluormethyl)-pyrimidin-2-yl]-2,6-dimethylpiperazin-1-carboxylat;
2-[(4-Fluorphenyl)methyl]-2-azaspiro[3.3]heptan-6-yl-(2R,6S)-4-[5-(difluormethyl)pyrimidin-2-yl]-2,6-dimethylpiperazin-1-carboxylat;
2-[(4-Fluorphenyl)methyl]-2-azaspiro[3.3]heptan-6-yl-(2R,6S)-4-(5-fluorpyrimidin-2-yl)-2,6-dimethylpiperazin-1-carboxylat;
2-Benzyl-2-azaspiro[3.3]heptan-6-yl-(2R,6S)-2,6-dimethyl-4-[2-(trifluormethyl)pyrimidin-5-yl]-piperazin-1-carboxylat;
2-Benzyl-2-azaspiro[3.3]heptan-6-yl-(2R,6S)-2,6-dimethyl-4-[5-(methylcarbamoyl)pyrimidin-2-yl]piperazin-1-carboxylat;
2-[(4-Fluorphenyl)methyl]-2-azaspiro[3.3]heptan-6-yl-(2R,6S)-4-(5-cyanopyrimidin-2-yl)-2,6-dimethylpiperazin-1-carboxylat;
2-[(4-Fluorphenyl)methyl]-2-azaspiro[3.3]heptan-6-yl-(2R,5S)-4-(5-cyanopyrimidin-2-yl)-2,5-dimethylpiperazin-1-carboxylat;
2-[(4-Fluorphenyl)methyl]-2-azaspiro[3.3]heptan-6-yl-(2R)-4-(5-cyanopyrimidin-2-yl)-2-methylpiperazin-1-carboxylat;
2-[(4-Chlorphenyl)methyl]-2-azaspiro[3.3]heptan-6-yl-(2R,6S)-4-(5-cyanopyrimidin-2-yl)-2,6-dimethylpiperazin-1-carboxylat;
2-[(4-Chlorphenyl)methyl]-2-azaspiro[3.3]heptan-6-yl-(2R,5S)-4-(5-cyanopyrimidin-2-yl)-2,5-dimethylpiperazin-1-carboxylat;
2-[(4-Chlorphenyl)methyl]-2-azaspiro[3.3]heptan-6-yl-(2R,6S)-2,6-dimethyl-4-[5-(trifluormethyl)pyrimidin-2-yl]piperazin-1-carboxylat;
2-[(4-Chlorphenyl)methyl]-2-azaspiro[3.3]heptan-6-yl-(2R)-2-methyl-4-[5-(trifluormethyl)-pyrimidin-2-yl]piperazin-1-carboxylat;
2-[(4-Chlorphenyl)methyl]-2-azaspiro[3.3]heptan-6-yl-(2R,6S)-2,6-dimethyl-4-[5-(trifluormethyl)pyrazin-2-yl]piperazin-1-carboxylat;
2-[(4-Chlorphenyl)methyl]-2-azaspiro[3.3]heptan-6-yl-(2R,5S)-2,5-dimethyl-4-[5-(trifluormethyl)pyrazin-2-yl]piperazin-1-carboxylat;
2-[(4-Chlorphenyl)methyl]-2-azaspiro[3.3]heptan-6-yl-(2R)-2-methyl-4-[5-(trifluormethyl)-pyrazin-2-yl]piperazin-1-carboxylat;
2-[(4-Chlorphenyl)methyl]-2-azaspiro[3.3]heptan-6-yl-(2R,5S)-2,5-dimethyl-4-[5-(trifluormethyl)pyrimidin-2-yl]piperazin-1-carboxylat;
2-[(4-Chlorphenyl)methyl]-2-azaspiro[3.3]heptan-6-yl-(2R)-4-(5-cyanopyrimidin-2-yl)-2-methylpiperazin-1-carboxylat;
2-Benzyl-2-azaspiro[3.3]heptan-6-yl-(2R,5S)-4-(6-fluorchinazolin-2-yl)-2,5-dimethylpiperazin-1-carboxylat;
2-Benzyl-2-azaspiro[3.3]heptan-6-yl-(1R,4R)-5-[5-(trifluormethyl)pyrimidin-2-yl]-2,5-diaza-bicyclo[2.2.1]heptan-2-carboxylat;
2-Benzyl-2-azaspiro[3.3]heptan-6-yl-(2R,6S)-4-[5-(dimethylcarbamoyl)pyrimidin-2-yl]-2,6-dimethylpiperazin-1-carboxylat;
2-Benzyl-2-azaspiro[3.3]heptan-6-yl-9-[5-(trifluormethyl)pyrimidin-2-yl]-3-oxa-7,9-diaza-bicyclo[3.3.1]nonan-7-carboxylat;
2-Benzyl-2-azaspiro[3.3]heptan-6-yl-9-[5-(trifluormethyl)pyrazin-2-yl]-3-oxa-7,9-diazabicyclo-[3.3.1]nonan-7-carboxylat;
2-Benzyl-2-azaspiro[3.3]heptan-6-yl-(2R,6S)-4-(5-ethylpyrimidin-2-yl)-2,6-dimethylpiperazin-1-carboxylat;
2-Benzyl-2-azaspiro[3.3]heptan-6-yl-(2R,6S)-2,6-dimethyl-4-[5-(trifluormethoxy)pyrimidin-2-yl]piperazin-1-carboxylat;
2-Benzyl-2-azaspiro[3.3]heptan-6-yl-(2R,6S)-4-[5-(cyclopropylmethoxy)pyrimidin-2-yl]-2,6-dimethylpiperazin-1-carboxylat;
2-[(4-Methansulfonylphenyl)methyl]-2-azaspiro[3.3]heptan-6-yl-(2R,6S)-2,6-dimethyl-4-[5-(trifluormethyl)pyrimidin-2-yl]piperazin-1-carboxylat;
2-[(4-Methansulfonylphenyl)methyl]-2-azaspiro[3.3]heptan-6-yl-(2R,5S)-2,5-dimethyl-4-[5-(trifluormethyl)pyrimidin-2-yl]piperazin-1-carboxylat;
2-[(4-Methansulfonylophenyl)methyl]-2-azaspiro[3.3]heptan-6-yl-(2R,6S)-2,6-dimethyl-4-[5-(trifluormethyl)pyrazin-2-yl]piperazin-1-carboxylat;
2-[(4-Methansulfonylphenyl)methyl]-2-azaspiro[3.3]heptan-6-yl-(2R,5S)-2,5-dimethyl-4-[5-(trifluormethyl)pyrazin-2-yl]piperazin-1-carboxylat;
2-Benzyl-2-azaspiro[3.3]heptan-6-yl-(2R,6S)-2,6-dimethyl-4-(5-propylpyrimidin-2-yl)-piperazin-1-carboxylat;
2-Benzyl-2-azaspiro[3.3]heptan-6-yl-(2R,6S)-2,6-dimethyl-4-[5-(propan-2-yl)pyrimidin-2-yl]-piperazin-1-carboxylat;
2-Benzyl-2-azaspiro[3.3]heptan-6-yl-(2R,6S)-4-(5-cyclopropylpyrimidin-2-yl)-2,6-dimethyl-piperazin-1-carboxylat;
2-Benzyl-2-azaspiro[3.3]heptan-6-yl-(2R,6S)-4-(5-ethinylpyrimidin-2-yl)-2,6-dimethyl-piperazin-1-carboxylat;
2-Benzyl-2-azaspiro[3.3]heptan-6-yl-(2R,6S)-4-[5-(dimethylphosphoryl)pyridin-2-yl]-2,6-dimethylpiperazin-1-carboxylat;
2-Benzyl-2-azaspiro[3.3]heptan-6-yl-(2R,6S)-2,6-dimethyl-4-[4-(trifluormethyl)phenyl]-piperazin-1-carboxylat;
2-Benzyl-2-azaspiro[3.3]heptan-6-yl-(2R,6S)-4-(4-ethoxyphenyl)-2,6-dimethylpiperazin-1-carboxylat;
2-Benzyl-2-azaspiro[3.3]heptan-6-yl-(2R,6S)-4-(4-methoxyphenyl)-2,6-dimethylpiperazin-1-carboxylat;
2-Benzyl-2-azaspiro[3.3]heptan-6-yl-(2R,6S)-4-(4-fluor-3-methylphenyl)-2,6-dimethyl-piperazin-1-carboxylat;
2-Benzyl-2-azaspiro[3.3]heptan-6-yl-(2R,6S)-4-(4-cyano-3-methoxyphenyl)-2,6-dimethyl-piperazin-1-carboxylat;
2-Benzyl-2-azaspiro[3.3]heptan-6-yl-(2R,5S)-2,5-dimethyl-4-[4-(trifluormethyl)phenyl]-piperazin-1-carboxylat;
2-Benzyl-2-azaspiro[3.3]heptan-6-yl-(2R)-2-methyl-4-[4-(trifluormethyl)phenyl|]piperazin-1-carboxylat;
2-Benzyl-2-azaspiro[3.3]heptan-6-yl-(2R,6S)-4-[5-(dimethylohosphoryl)pyrimidin-2-yl]-2,6-dimethylpiperazin-1-carboxylat; und
2-Benzyl-2-azaspiro[3.3]heptan-6-yl-(2R,6S)-4-[5-(cyclobutylmethoxy)pyrimidin-2-yl]-2,6-dimethylpiperazin-1-carboxylat.

12. Pharmazeutische Zusammensetzung, die eine Verbindung nach einem der Ansprüche 1 bis 11 oder ein pharmazeutisch annehmbares Salz davon und einen pharmazeutisch annehmbaren Träger umfasst.

13. Verbindung oder Salz nach einem der Ansprüche 1 bis 11 zur Verwendung in einem Verfahren zur Behandlung oder Prävention einer durch den Muskarin-Rezeptor 4 (M₄) vermittelten Erkrankung, Störung oder eines Symptoms davon in einem Individuum.

14. Verbindung oder Salz nach einem der Ansprüche 1 bis 11 zur Verwendung in einem Verfahren zur Behandlung oder Prävention einer neurologischen Erkrankung, Störung oder eines Symptoms davon in einem Individuum;
wobei die Erkrankung, Störung oder das Symptom aus den folgenden ausgewählt ist: dem Tourette-Syndrom (TS), der Alzheimer-Krankheit (AD), Schizophrenie, Lewy-Body-Demenz (LBD), kognitiven Defiziten im Zusammenhang mit Schizophrenie, der Parkinson-Krankheit, Parkinsonismus, Tremor, Dyskinesien, übermäßiger Tagesmüdigkeit, Dystonie, Chorea, Levodopa-induzierter Dyskinesie, Aufmerksamkeitsdefizit-/Hyperaktivitätsstörung (ADHD), zerebraler Lähmung, progressiver supranuklearer Lähmung (PSP), multipler Systematrophie (MSA), der Huntington-Krankheit (HD) und Chorea im Zusammenhang mit der Huntington-Krankheit.

15. Verbindung oder Salz zur Verwendung nach Anspruch 14, wobei:
die Erkrankung, die Störung oder das Symptom Parkinsonismus, Tremor oder Dystonie ist.

## Revendications

1. Composé choisi parmi ceux de formule (Ia) : 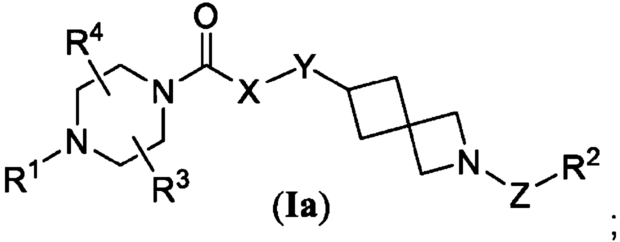
ou un sel pharmaceutiquement acceptable de celui-ci ;
dans lequel :
X est O ou NH ;
Y est CH₂ ou absent ;
Z est un alcylène en C₁-C₄ ou absent ;
R¹ est un aryle en C₆-C₁₀ ou un hétéroaryle à 5 à 10 chaînons, chacun étant éventuellement substitué par un ou plusieurs groupes choisis parmi : alcoxy en C₁-C₆, alcoxycarboxyle en C₁-C₆, alkyle en C₁-C₆, alkylamino en C₁-C₆, alkylcarbonyle en C₁-C₆, alkylcarbamoyle en C₁-C₆, alkylsulfanyle en C₁-C₆, dialkylamino en C₂-C₆, dialkylcarbamoyle en C₂-C₆, sulfinyle en C₁-C₆, cycloalkyle en C₃-C₁₀, halogénoalcoxy en C₁-C₆, halogénalkyle en C₁-C₆, amino, carbamoyle, cyano, halogène et nitro ;
R² est choisi parmi : aryle en C₅-C₁₀, alkyle en C₁-C₆, cycloalkyle en C₃-C₇, bicycloalcanyle en C₅-C₈, bicycloalcényle en C₅-C₈, hétéroaryle à 5 à 10 chaînons et hétérocyclyle ; chacun étant éventuellement substitué par un ou plusieurs groupes choisis parmi : alkyle en C₁-C₄, un alcoxy en C₁-C₆, halogénoalcoxy en C₁-C₆, sulfonyle en C₁-C₆, carbamoyle, cyano et halogène ; et
a) R³ et R⁴ sont chacun indépendamment choisis parmi H et un alkyle en C₁-C₄ ; ou
b) R³ et R⁴, pris ensemble, forment un groupe pontant choisi parmi : CH₂, CH₂-CH₂ et CH₂-O-CH₂ ; et
c) R³ et R⁴ sont liés à des groupes éthylène différents du cycle pipérazine ;
ou un composé choisi dans le groupe constitué par :
(2R,6S)-N-[2-(cyclohex-1-én-1-ylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-2,6-diméthyl-N-{2-[(4-nitrophényl)méthyl]-2-azaspiro[3.3]heptan-6-yl}-4-[5-(trifluorométhyl)pyrimidin-2-yl]pipérazine-1-carboxamide ;
(2R,6S)-N-{2-[(4-hydroxyphényl)méthyl]-2-azaspiro[3.3]heptan-6-yl}-2,6-diméthyl-4-[5-(trifluorométhyl)pyrimidin-2-yl]pipérazine-1-carboxamide ;
4-[(6-{[(2R,6S)-2,6-diméthyl-4-[5-(trifluorométhyl)pyrimidin-2-yl]pipérazine-1-carbonyl]amino}-2-azaspiro[3.3]heptan-2-yl)méthyl]benzoate de méthyle ;
(2R,6S)-N-{2-[(4-acétamidophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-N-(2-{[4-(trifluorométhyl)phényl]méthyl}-2-azaspiro[3.3]heptan-6-yl)-4-[5-(trifluorométhyl)pyrimidin-2-yl]pipérazine-1-carboxamide ;
(2R,6S)-N-{2-[(4-carbamimidoylphényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-[2-(adamantan-1-ylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-N-(2-{[4-(diméthylcarbamoyl)phényl]méthyl}-2-azaspiro[3.3]heptan-6-yl)-2,6-diméthyl-4-[5-(trifluorométhyl)pyrimidin-2-yl]pipérazine-1-carboxamide ;
(2R,6S)-2,6-diméthyl-4-[5-(trifluorométhyl)pyrimidin-2-yl]pipérazine-1-carboxylate de 2-benzyl-6-(difluorométhyl)-2-azaspiro[3.3]heptan-6-yle ;
(2R,6S)-2,6-diméthyl-4-[5-(trifluorométhyl)pyrazin-2-yl]pipérazine-1-carboxylate de 2-benzyl-6-(difluorométhyl)-2-azaspiro[3.3]heptan-6-yle ;
(2R,6S)-2,6-diméthyl-4-[6-(trifluorométhyl)pyridazin-3-yl]pipérazine-1-carboxylate de 2-benzyl-6-(difluorométhyl)-2-azaspiro[3.3]heptan-6-yle ;
(2R,6R)-2,6-diméthyl-4-[5-(trifluorométhyl)pyrimidin-2-yl]pipérazine-1-carboxylate de 2-benzyl-6-(difluorométhyl)-2-azaspiro[3.3]heptan-6-yle ;
(2R,6R)-2,6-diméthyl-4-[5-(trifluorométhyl)pyrazin-2-yl]pipérazine-1-carboxylate de 2-benzyl-6-(difluorométhyl)-2-azaspiro[3.3]heptan-6-yle ;
(2R,5S)-2,5-diméthyl-4-[5-(trifluorométhyl)pyrimidin-2-yl]pipérazine-1-carboxylate de 2-benzyl-6-(difluorométhyl)-2-azaspiro[3.3]heptan-6-yle ;
(2R,5S)-2,5-diméthyl-4-[5-(trifluorométhyl)pyrazin-2-yl]pipérazine-1-carboxylate de 2-benzyl-6-(difluorométhyl)-2-azaspiro[3.3]heptan-6-yle ;
(2R)-4-(5-cyanopyrimidin-2-yl)-2-méthylpipérazine-1-carboxylate de 2-benzyl-6-(difluorométhyl)-2-azaspiro[3.3]heptan-6-yle ;
(2R)-2-méthyl-4-[5-(trifluorométhyl)pyrimidin-2-yl]pipérazine-1-carboxylate de 2-benzyl-6-(difluorométhyl)-2-azaspiro[3.3]heptan-6-yle ;
(2R)-2-méthyl-4-[5-(trifluorométhyl)pyrazin-2-yl]pipérazine-1-carboxylate de 2-benzyl-6-(difluorométhyl)-2-azaspiro[3.3]heptan-6-yle ;
(2R,6S)-4-(5-cyanopyrimidin-2-yl)-2,6-diméthylpipérazine-1-carboxylate de 2-benzyl-6-méthyl-2-azaspiro[3.3]heptan-6-yle ;
(2R,6S)-2,6-diméthyl-4-[5-(trifluorométhyl)pyrimidin-2-yl]pipérazine-1-carboxylate de 2-benzyl-6-méthyl-2-azaspiro[3.3]heptan-6-yle ;
(2R,6S)-2,6-diméthyl-4-[5-(trifluorométhyl)pyrazin-2-yl]pipérazine-1-carboxylate de 2-benzyl-6-méthyl-2-azaspiro[3.3]heptan-6-yle ;
(2R,6R)-4-(5-cyanopyrimidin-2-yl)-2,6-diméthylpipérazine-1-carboxylate de 2-benzyl-6-méthyl-2-azaspiro[3.3]heptan-6-yle ;
(2R,6R)-2,6-diméthyl-4-[5-(trifluorométhyl)pyrimidin-2-yl]pipérazine-1-carboxylate de 2-benzyl-6-méthyl-2-azaspiro[3.3]heptan-6-yle ;
(2R,6R)-2,6-diméthyl-4-[5-(trifluorométhyl)pyrazin-2-yl]pipérazine-1-carboxylate de 2-benzyl-6-méthyl-2-azaspiro[3.3]heptan-6-yle ;
(2R,5S)-4-(5-cyanopyrimidin-2-yl)-2,5-diméthylpipérazine-1-carboxylate de 2-benzyl-6-méthyl-2-azaspiro[3.3]heptan-6-yle ;
(2R,5S)-2,5-diméthyl-4-[5-(trifluorométhyl)pyrimidin-2-yl]pipérazine-1-carboxylate de 2-benzyl-6-méthyl-2-azaspiro[3.3]heptan-6-yle ;
(2R,5S)-2,5-diméthyl-4-[5-(trifluorométhyl)pyrazin-2-yl]pipérazine-1-carboxylate de 2-benzyl-6-méthyl-2-azaspiro[3.3]heptan-6-yle ;
(2R,6S)-4-(5-éthynylpyrimidin-2-yl)-2,6-diméthylpipérazine-1-carboxylate de 2-benzyl-2-azaspiro[3.3]heptan-6-yle ;
(2R,6S)-4-[5-(diméthylphosphoryl)pyridin-2-yl]-2,6-diméthylpipérazine-1-carboxylate de 2-benzyl-2-azaspiro[3.3]heptan-6-yle ; et
(2R,6S)-4-[5-(diméthylphosphoryl)pyrimidin-2-yl]-2,6-diméthylpipérazine-1-carboxylate de benzyl-2-azaspiro[3.3]heptan-6-yle ;
ou un sel pharmaceutiquement acceptable de ceux-ci.

2. Composé selon la revendication 1, ou un sel pharmaceutiquement acceptable de celui-ci, dans lequel :
R¹ est choisi parmi : 1,3,4-thiadiazol-2-yle, phényle, pyrazin-2-yle, pyridazin-3-yle, pyridin-2-yle, pyridin-3-yle, pyrimidin-2-yl et quinoxalin-2-yle ;
chacun étant éventuellement substitué par un ou plusieurs groupes choisis parmi : acétyle, brome, chlore, cyano, difluorométhoxy, difluorométhyle, fluor, méthoxy, méthoxycarbonyle, méthyle, méthylsulfanyle, nitro, trifluorométhoxy et trifluorométhyle.

3. Composé selon la revendication 1, ou un sel pharmaceutiquement acceptable de celui-ci, dans lequel :
R¹ est choisi parmi : 2-cyano-4-(trifluorométhyl)phényle, 3-(trifluorométhyl)pyridin-2-yle, 3,4,5-trifluorophényle, 3,6-diméthylpyrazin-2-yle, cyano-4-(trifluorométhoxy)phényle, 3-cyano-4-fluorophényle, 3-cyano-5-fluorophényle, 4-(trifluorométhoxy)phényle, 4-(trifluorométhyl)phényle, 4-cyano-2,5-difluorophényle, cyano-2-fluorophényle, 4-cyano-6-(trifluorométhyl)pyridin-3-yle, 4-méthylpyrimidin-2-yle, 5-(difluorométhoxy)pyrimidin-2-yle, 5-(difluorométhyl)pyrazin-2-yle, 5-(méthoxycarbonyl)-4-(trifluorométhyl)pyrimidin-2-yle, 5-(trifluorométhoxy)pyrazin-2-yle, 5-(trifluorométhyl)-1,3,4-thiadiazol-2-yle, 5-(trifluorométhyl)pyrazin-2-yle, 5-(trifluorométhyl)pyridin-2-yle, 5-(trifluorométhyl)pyrimidin-2-yle, 5-acétylpyrazin-2-yle, bromo-4-(méthylsulfanyl)pyrimidin-2-yle, 5-chloropyrazin-2-yle, 5-chloropyrimidin-2-yle, 5-cyano-3-méthylpyrazin-2-yle, 5-cyanopyrazin-2-yle, 5-cyanopyridin-2-yle, 5-cyanopyrimidin-2-yle, 5-fluoropyrimidin-2-yle, 5-méthoxypyrimidin-2-yle, 5-nitropyridin-2-yle, 6-(trifluorométhyl)pyrazin-2-yle, 6-(trifluorométhyl)pyridazin-3-yle, 6-(trifluorométhyl)pyridin-3-yle, 6-cyanopyrazin-2-yle, 6-fluoroquinoxalin-2-yle, 6-méthoxy-3-nitropyridin-2-yle, 6-méthoxypyrazin-2-yle, 6-méthoxyquinoxalin-2-yle et quinoxalin-2-yle.

4. Composé selon l'une quelconque des revendications 1 à 3, ou un sel pharmaceutiquement acceptable de celui-ci, dans lequel :
R² est choisi parmi : 1H-indolyle, 2,2-diméthylpropyle, 2-méthylpropyle, 3,3-diméthylbutyle, bicyclo[2.2.1]heptényle, butyle, cyclohexyle, cyclopropyle, oxanyle et phényle ;
chacun étant éventuellement substitué par un ou plusieurs groupes choisis parmi : alkyle en C₁-C₄, alcoxy en C₁-C₆, carbamoyle, cyano et halogène.

5. Composé selon l'une quelconque des revendications 1 à 4, dans lequel Composé est de formule (IIg) : 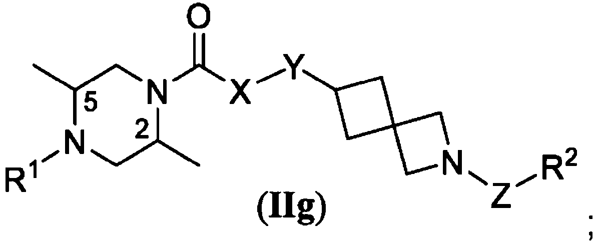 ou un sel pharmaceutiquement acceptable de celui-ci.

6. Composé selon l'une quelconque des revendications 1 à 4, dans lequel Composé est de formule (IIi) : 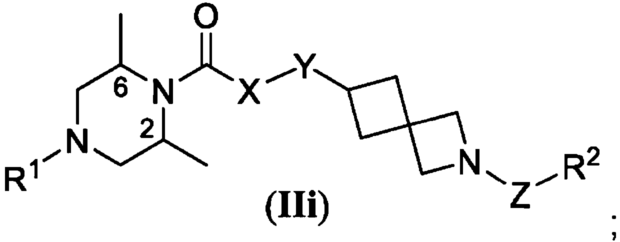 ou un sel pharmaceutiquement acceptable de celui-ci.

7. Composé selon l'une quelconque des revendications 1 à 4, dans lequel Composé est de formule (IIk) : 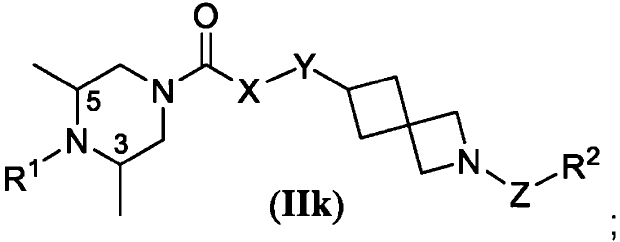 ou un sel pharmaceutiquement acceptable de celui-ci.

8. Composé selon la revendication 6, ou un sel pharmaceutiquement acceptable de celui-ci, dans lequel :
la stéréochimie dans la formule (IIi) pour le carbone C(2) est (R) et pour le carbone C(6) est (R).

9. Composé selon la revendication 6, ou un sel pharmaceutiquement acceptable de celui-ci, dans lequel :
la stéréochimie dans la formule (IIi) pour le carbone C(2) est (S) et pour le carbone C(6) est (S).

10. Composé selon la revendication 6, ou un sel pharmaceutiquement acceptable de celui-ci, dans lequel :
la stéréochimie dans la formule (IIi) pour le carbone C(2) est *(R)* et pour le carbone C(6) est (*S*).

11. Composé selon la revendication 1, qui est choisi parmi les composés suivants, ou un sel pharmaceutiquement acceptable de ceux-ci :
(2R,5S)-N-{2-benzyl-2-azaspiro[3.3]heptan-6-yl}-2,5-diméthyl-4-[5-(trifluorométhyl)pyrimidin-2-yl]pipérazine-1-carboxamide ;
(2R)-N-{2-benzyl-2-azaspiro[3.3]heptan-6-yl}-4-(5-cyanopyrimidin-2-yl)-2-méthylpipérazine-1-carboxamide ;
(2R,6R)-N-{2-benzyl-2-azaspiro[3.3]heptan-6-yl}-4-(5-cyanopyrimidin-2-yl)-2,6-diméthylpipérazine-1-carboxamide ;
(2S,6S)-N-{2-benzyl-2-azaspiro[3.3]heptan-6-yl}-4-(5-cyanopyrimidin-2-yl)-2,6-diméthylpipérazine-1-carboxamide ;
(2S)-N-{2-benzyl-2-azaspiro[3.3]heptan-6-yl}-2-méthyl-4-[5-(trifluorométhyl)pyrimidin-2-yl]pipérazine-1-carboxamide ;
(3S)-N-{2-benzyl-2-azaspiro[3.3]heptan-6-yl}-3-méthyl-4-[5-(trifluorométhyl)pyrimidin-2-yl]pipérazine-1-carboxamide ;
(2R)-N-{2-benzyl-2-azaspiro[3.3]heptan-6-yl}-2-méthyl-4-[5-(trifluorométhyl)pyrimidin-2-yl]pipérazine-1-carboxamide ;
N-{2-benzyl-2-azaspiro[3.3]heptan-6-yl}-4-(4-cyano-2-fluorophényl)pipérazine-1-carboxamide ;
N-{2-benzyl-2-azaspiro[3.3]heptan-6-yl}-4-(3,4,5-trifluorophényl)pipérazine-1-carboxamide ;
N-{2-benzyl-2-azaspiro[3.3]heptan-6-yl}-4-[4-(trifluorométhoxy)phényl]pipérazine-1-carboxamide ;
(2R,6R)-N-{2-benzyl-2-azaspiro[3.3]heptan-6-yl}-2,6-diméthyl-4-(3,4,5-trifluorophényl)pipérazine-1-carboxamide ;
(2R)-N-{2-benzyl-2-azaspiro[3.3]heptan-6-yl}-4-(3-cyano-4-fluorophényl)-2-méthylpipérazine-1-carboxamide ;
(2R)-N-{2-benzyl-2-azaspiro[3.3]heptan-6-yl}-4-[3-cyano-4-(trifluorométhoxy)phényl]-2-méthylpipérazine-1-carboxamide ;
(2R,6S)-N-{2-benzyl-2-azaspiro[3.3]heptan-6-yl}-4-(5-chloropyrimidin-2-yl)-2,6-diméthylpipérazine-1-carboxamide ;
(1S,4S)-N-{2-benzyl-2-azaspiro[3.3]heptan-6-yl}-5-(5-cyanopyrimidin-2-yl)-2,5-diazabicyclo[2.2.1]heptane-2-carboxamide ;
N-{2-benzyl-2-azaspiro[3.3]heptan-6-yl}-3-(5-cyanopyrimidin-2-yl)-3,6-diazabicyclo[3.1.1]heptane-6-carboxamide ;
N-{2-benzyl-2-azaspiro[3.3]heptan-6-yl}-5-(5-cyanopyrimidin-2-yl)-2,5-diazabicyclo[2.2.2]octane-2-carboxamide ;
(2R,6R)-N-{2-benzyl-2-azaspiro[3.3]heptan-6-yl}-4-(5-chloropyrimidin-2-yl)-2,6-diméthylpipérazine-1-carboxamide ;
(2R,6R)-N-{2-benzyl-2-azaspiro[3.3]heptan-6-yl}-2,6-diméthyl-4-[6-(trifluorométhyl)pyridazin-3-yl]pipérazine-1-carboxamide ;
(2R,6R)-N-{2-benzyl-2-azaspiro[3.3]heptan-6-yl}-4-(5-cyanopyrazin-2-yl)-2,6-diméthylpipérazine-1-carboxamide ;
(2R,6R)-N-{2-benzyl-2-azaspiro[3.3]heptan-6-yl}-4-[5-(difluorométhoxy)pyrimidin-2-yl]-2,6-diméthylpipérazine-1-carboxamide ;
(2R)-N-{2-benzyl-2-azaspiro[3.3]heptan-6-yl}-2-méthyl-4-[5-(trifluorométhyl)pyrazin-2-yl]pipérazine-1-carboxamide ;
N-{2-benzyl-2-azaspiro[3.3]heptan-6-yl}-3-[5-(trifluorométhyl)pyrimidin-2-yl]-3,6-diazabicyclo[3.1.1]heptane-6-carboxamide ;
N-{2-benzyl-2-azaspiro[3.3]heptan-6-yl}-3-[5-(trifluorométhyl)pyrazin-2-yl]-3,6-diazabicyclo[3.1.1]heptane-6-carboxamide ;
(2S)-N-{2-benzyl-2-azaspiro[3.3]heptan-6-yl}-4-(5-cyanopyrimidin-2-yl)-2-méthylpipérazine-1-carboxamide ;
(2R,5S)-N-{2-benzyl-2-azaspiro[3.3]heptan-6-yl}-4-(5-cyanopyrimidin-2-yl)-2,5-diméthylpipérazine-1-carboxamide ;
(2S,5R)-N-{2-benzyl-2-azaspiro[3.3]heptan-6-yl}-4-(5-cyanopyrimidin-2-yl)-2,5-diméthylpipérazine-1-carboxamide ;
(2R,5R)-N-{2-benzyl-2-azaspiro[3.3]heptan-6-yl}-4-(5-cyanopyrimidin-2-yl)-2,5-diméthylpipérazine-1-carboxamide ;
N-{2-benzyl-2-azaspiro[3.3]heptan-6-yl}-3-[6-(trifluorométhyl)pyridazin-3-yl]-3,6-diazabicyclo[3.1.1]heptane-6-carboxamide ;
(1S,4S)-N-{2-benzyl-2-azaspiro[3.3]heptan-6-yl}-5-[5-(trifluorométhyl)pyrazin-2-yl]-2,5-diazabicyclo[2.2.1]heptane-2-carboxamide ;
(1S,4S)-N-{2-benzyl-2-azaspiro[3.3]heptan-6-yl}-5-[5-(trifluorométhyl)pyrimidin-2-yl]-2,5-diazabicyclo[2.2.1]heptane-2-carboxamide ;
N-{2-benzyl-2-azaspiro[3.3]heptan-6-yl}-4-[5-(trifluorométhyl)pyrimidin-2-yl]pipérazine-1-carboxamide ;
(3R,5R)-N-{2-benzyl-2-azaspiro[3.3]heptan-6-yl}-4-(5-cyanopyrimidin-2-yl)-3,5-diméthylpipérazine-1-carboxamide ;
(3S,5S)-N-{2-benzyl-2-azaspiro[3.3]heptan-6-yl}-4-(5-cyanopyrimidin-2-yl)-3,5-diméthylpipérazine-1-carboxamide ;
(2R,5S)-N-{2-benzyl-2-azaspiro[3.3]heptan-6-yl}-2,5-diméthyl-4-[5-(trifluorométhyl)pyrazin-2-yl]pipérazine-1-carboxamide ;
(2S,5R)-N-{2-benzyl-2-azaspiro[3.3]heptan-6-yl}-2,5-diméthyl-4-[5-(trifluorométhyl)pyrimidin-2-yl]pipérazine-1-carboxamide ;
(2S,5R)-N-{2-benzyl-2-azaspiro[3.3]heptan-6-yl}-2,5-diméthyl-4-[5-(trifluorométhyl)pyrazin-2-yl]pipérazine-1-carboxamide ;
(2R,5R)-N-{2-benzyl-2-azaspiro[3.3]heptan-6-yl}-2,5-diméthyl-4-[5-(trifluorométhyl)pyrimidin-2-yl]pipérazine-1-carboxamide ;
(2R,5R)-N-{2-benzyl-2-azaspiro[3.3]heptan-6-yl}-2,5-diméthyl-4-[5-(trifluorométhyl)pyrazin-2-yl]pipérazine-1-carboxamide ;
(2S,5S)-N-{2-benzyl-2-azaspiro[3.3]heptan-6-yl}-4-(5-cyanopyrimidin-2-yl)-2,5-diméthylpipérazine-1-carboxamide ;
(3S)-N-{2-benzyl-2-azaspiro[3.3]heptan-6-yl}-4-(5-cyanopyrimidin-2-yl)-3-méthylpipérazine-1-carboxamide ;
(3R)-N-{2-benzyl-2-azaspiro[3.3]heptan-6-yl}-4-(5-cyanopyrimidin-2-yl)-3-méthylpipérazine-1-carboxamide ;
N-{2-benzyl-2-azaspiro[3.3]heptan-6-yl}-4-[5-(trifluorométhyl)pyridin-2-yl]pipérazine-1-carboxamide ;
(2R)-N-{2-benzyl-2-azaspiro[3.3]heptan-6-yl}-2-méthyl-4-(3,4,5-trifluorophényl)pipérazine-1-carboxamide ;
(2R,6S)-N-{2-benzyl-2-azaspiro[3.3]heptan-6-yl}-2,6-diméthyl-4-(3,4,5-trifluorophényl)pipérazine-1-carboxamide ;
(2R,6R)-N-{2-benzyl-2-azaspiro[3.3]heptan-6-yl}-4-(6-cyanopyrazin-2-yl)-2,6-diméthylpipérazine-1-carboxamide ;
(2R,6S)-N-{2-benzyl-2-azaspiro[3.3]heptan-6-yl}-2,6-diméthyl-4-[4-(trifluorométhyl)phényl]pipérazine-1-carboxamide ;
(2R,6R)-N-{2-benzyl-2-azaspiro[3.3]heptan-6-yl}-2,6-diméthyl-4-[6-(trifluorométhyl)pyrazin-2-yl]pipérazine-1-carboxamide ;
(1R,4R)-N-{2-benzyl-2-azaspiro[3.3]heptan-6-yl}-5-(5-cyanopyrimidin-2-yl)-2,5-diazabicyclo[2.2.2]octane-2-carboxamide ;
(2R,6S)-N-{2-benzyl-2-azaspiro[3.3]heptan-6-yl}-4-(5-cyanopyridin-2-yl)-2,6-diméthylpipérazine-1-carboxamide ;
(2R,6R)-N-{2-benzyl-2-azaspiro[3.3]heptan-6-yl}-2,6-diméthyl-4-[4-(trifluorométhyl)phényl]pipérazine-1-carboxamide ;
(1S,4S)-N-{2-benzyl-2-azaspiro[3.3]heptan-6-yl}-5-(5-cyanopyrimidin-2-yl)-2,5-diazabicyclo[2.2.2]octane-2-carboxamide ;
(2R,6R)-N-{2-benzyl-2-azaspiro[3.3]heptan-6-yl}-4-[5-(difluorométhyl)pyrazin-2-yl]-2,6-diméthylpipérazine-1-carboxamide ;
N-{2-benzyl-2-azaspiro[3.3]heptan-6-yl}-3-(5-cyanopyrimidin-2-yl)-3,8-diazabicyclo[3.2.1]octane-8-carboxamide ;
(2R,6R)-N-{2-benzyl-2-azaspiro[3.3]heptan-6-yl}-4-(5-méthoxypyrimidin-2-yl)-2,6-diméthylpipérazine-1-carboxamide ;
(2R,6S)-N-{2-benzyl-2-azaspiro[3.3]heptan-6-yl}-2,6-diméthyl-4-[6-(trifluorométhyl)pyrazin-2-yl]pipérazine-1-carboxamide ;
(2R,6R)-N-{2-benzyl-2-azaspiro[3.3]heptan-6-yl}-4-(5-chloropyrazin-2-yl)-2,6-diméthylpipérazine-1-carboxamide ;
(1R,4R)-N-{2-benzyl-2-azaspiro[3.3]heptan-6-yl}-5-[5-(trifluorométhyl)pyrimidin-2-yl]-2,5-diazabicyclo[2.2.1]heptane-2-carboxamide ;
(1R,4R)-N-{2-benzyl-2-azaspiro[3.3]heptan-6-yl}-5-[5-(trifluorométhyl)pyrazin-2-yl]-2,5-diazabicyclo[2.2.1]heptane-2-carboxamide ;
(2R,6S)-N-{2-benzyl-2-azaspiro[3.3]heptan-6-yl}-4-[4-méthoxy-5-(trifluorométhyl)pyrimidin-2-yl]-2,6-diméthylpipérazine-1-carboxamide ;
(2R,6S)-N-{2-benzyl-2-azaspiro[3.3]heptan-6-yl}-4-(5-cyanopyrazin-2-yl)-2,6-diméthylpipérazine-1-carboxamide ;
(2R,6S)-N-{2-benzyl-2-azaspiro[3.3]heptan-6-yl}-2,6-diméthyl-4-[5-(trifluorométhyl)pyrazin-2-yl]pipérazine-1-carboxamide ;
(2R,6R)-N-({2-benzyl-2-azaspiro[3.3]heptan-6-yl}méthyl)-2,6-diméthyl-4-[5-(trifluorométhyl)pyrazin-2-yl]pipérazine-1-carboxamide ;
(2R,6R)-N-{2-benzyl-2-azaspiro[3.3]heptan-6-yl}-2,6-diméthyl-4-[5-(trifluorométhyl)pyrazin-2-yl]pipérazine-1-carboxamide ;
(2R,6R)-N-{2-benzyl-2-azaspiro[3.3]heptan-6-yl}-2,6-diméthyl-4-[5-(trifluorométhyl)pyrimidin-2-yl]pipérazine-1-carboxamide ;
(2R,6S)-N-{2-benzyl-2-azaspiro[3.3]heptan-6-yl}-4-(5-cyanopyrimidin-2-yl)-2,6-diméthylpipérazine-1-carboxamide ;
(2R,6S)-N-({2-benzyl-2-azaspiro[3.3]heptan-6-yl}méthyl)-2,6-diméthyl-4-[5-(trifluorométhyl)pyrazin-2-yl]pipérazine-1-carboxamide ;
(2R,6R)-N-({2-benzyl-2-azaspiro[3.3]heptan-6-yl}méthyl)-2,6-diméthyl-4-[5-(trifluorométhyl)pyrimidin-2-yl]pipérazine-1-carboxamide ;
(2R,6R)-N-[2-(3,3-diméthylbutyl)-2-azaspiro[3.3]heptan-6-yl]-2,6-diméthyl-4-[5-(trifluorométhyl)pyrimidin-2-yl]pipérazine-1-carboxamide ;
(2R,6S)-4-(5-cyanopyrimidin-2-yl)-N-[2-(3,3-diméthylbutyl)-2-azaspiro[3.3]heptan-6-yl]-2,6-diméthylpipérazine-1-carboxamide ;
(2R,6S)-N-{[2-(3,3-diméthylbutyl)-2-azaspiro[3.3]heptan-6-yl]méthyl}-2,6-diméthyl-4-[5-(trifluorométhyl)pyrazin-2-yl]pipérazine-1-carboxamide ;
(2R,6R)-N-{[2-(3,3-diméthylbutyl)-2-azaspiro[3.3]heptan-6-yl]méthyl}-2,6-diméthyl-4-[5-(trifluorométhyl)pyrimidin-2-yl]pipérazine-1-carboxamide ;
(2R,6R)-N-[2-(2,2-diméthylpropyl)-2-azaspiro[3.3]heptan-6-yl]-2,6-diméthyl-4-[5-(trifluorométhyl)pyrimidin-2-yl]pipérazine-1-carboxamide ;
(2R,6S)-4-(5-cyanopyrimidin-2-yl)-N-[2-(2,2-diméthylpropyl)-2-azaspiro[3.3]heptan-6-yl]-2,6-diméthylpipérazine-1-carboxamide ;
(2R,6S)-N-{[2-(2,2-diméthylpropyl)-2-azaspiro[3.3]heptan-6-yl]méthyl}-2,6-diméthyl-4-[5-(trifluorométhyl)pyrazin-2-yl]pipérazine-1-carboxamide ;
(2R,6R)-N-{[2-(2,2-diméthylpropyl)-2-azaspiro[3.3]heptan-6-yl]méthyl}-2,6-diméthyl-4-[5-(trifluorométhyl)pyrimidin-2-yl]pipérazine-1-carboxamide ;
(2R,6S)-N-[2-(cyclopropylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-2,6-diméthyl-N-[2-(2-méthylpropyl)-2-azaspiro[3.3]heptan-6-yl]-4-[5-(trifluorométhyl)pyrimidin-2-yl]pipérazine-1-carboxamide ;
(2R,6S)-N-{2-[(2,2-diméthylcyclopropyl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-[2-(cyclohexylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-2,6-diméthyl-N-[2-(oxan-4-ylméthyl)-2-azaspiro[3.3]heptan-6-yl]-4-[5-(trifluorométhyl)pyrimidin-2-yl]pipérazine-1-carboxamide ;
(2R,6S)-N-(2-{bicyclo[2.2.1]hept-5-én-2-ylméthyl}-2-azaspiro[3.3]heptan-6-yl)-2,6-diméthyl-4-[5-(trifluorométhyl)pyrimidin-2-yl]pipérazine-1-carboxamide ;
(2R,6S)-N-{2-[(2-fluorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(3-fluorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(4-fluorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-N-[2-(4,4,4-trifluorobutyl)-2-azaspiro[3.3]heptan-6-yl]-4-[5-(trifluorométhyl)pyrimidin-2-yl]pipérazine-1-carboxamide ;
(2R,6S)-N-{2-[(2-cyanophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(4-cyanophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(4-méthoxyphényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(2,6-difluorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(3,4-difluorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-[2-(1H-indol-5-ylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-N-{2-[(3-cyano-4-fluorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(4-carbamoylphényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(3-carbamoylphényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-benzyl-2-azaspiro[3.3]heptan-6-yl}-2,6-diméthyl-4-[5-(trifluorométhyl)pyrimidin-2-yl]pipérazine-1-carboxamide ;
(2R,6S)-N-({2-benzyl-2-azaspiro[3.3]heptan-6-yl}méthyl)-2,6-diméthyl-4-[5-(trifluorométhyl)pyrimidin-2-yl]pipérazine-1-carboxamide ;
(2R,6S)-N-[2-(3,3-diméthylbutyl)-2-azaspiro[3.3]heptan-6-yl]-2,6-diméthyl-4-[5-(trifluorométhyl)pyrimidin-2-yl]pipérazine-1-carboxamide ;
(2R,6S)-N-{[2-(3,3-diméthylbutyl)-2-azaspiro[3.3]heptan-6-yl]méthyl}-2,6-diméthyl-4-[5-(trifluorométhyl)pyrimidin-2-yl]pipérazine-1-carboxamide ;
(2R,6S)-N-[2-(2,2-diméthylpropyl)-2-azaspiro[3.3]heptan-6-yl]-2,6-diméthyl-4-[5-(trifluorométhyl)pyrimidin-2-yl]pipérazine-1-carboxamide ;
(2R,6S)-N-{[2-(2,2-diméthylpropyl)-2-azaspiro[3.3]heptan-6-yl]méthyl}-2,6-diméthyl-4-[5-(trifluorométhyl)pyrimidin-2-yl]pipérazine-1-carboxamide ;
4-(4-cyano-2-fluorophényl)pipérazine-1-carboxylate de 2-benzyl-2-azaspiro[3.3]heptan-6-yle ;
(2R)-4-[3-cyano-4-(trifluorométhoxy)phényl]-2-méthylpipérazine-1-carboxylate de 2-benzyl-2-azaspiro[3.3]heptan-6-yle ;
(1S,4S)-5-(5-cyanopyrimidin-2-yl)-2,5-diazabicyclo[2.2.1]heptane-2-carboxylate de 2-benzyl-2-azaspiro[3.3]heptan-6-yle ;
4-(3,4,5-trifluorophényl)pipérazine-1-carboxylate de 2-benzyl-2-azaspiro[3.3]heptan-6-yle ;
(2R,5S)-2,5-diméthyl-4-[5-(trifluorométhyl)pyrazin-2-yl]pipérazine-1-carboxylate de 2-benzyl-2-azaspiro[3.3]heptan-6-yle ;
5-(5-cyanopyrimidin-2-yl)-2,5-diazabicyclo[2.2.2]octane-2-carboxylate de 2-benzyl-2-azaspiro[3.3]heptan-6-yle ;
4-[4-(trifluorométhoxy)phényl]pipérazine-1-carboxylate de 2-benzyl-2-azaspiro[3.3]heptan-6-yle ;
3-[5-(trifluorométhyl)pyrimidin-2-yl]-3,6-diazabicyclo[3.1.1]heptane-6-carboxylate de 2-benzyl-2-azaspiro[3.3]heptan-6-yle ;
3-[5-(trifluorométhyl)pyrazin-2-yl]-3,6-diazabicyclo[3.1.1]heptane-6-carboxylate de 2-benzyl-2-azaspiro[3.3]heptan-6-yle ;
3-(5-cyanopyrazin-2-yl)-3,6-diazabicyclo[3.1.1]heptane-6-carboxylate de 2-benzyl-2-azaspiro[3.3]heptan-6-yle ;
4-(5-cyanopyrimidin-2-yl)pipérazine-1-carboxylate de 2-benzyl-2-azaspiro[3.3]heptan-6-yle ;
3-[6-(trifluorométhyl)pyridazin-3-yl]-3,6-diazabicyclo[3.1.1]heptane-6-carboxylate de 2-benzyl-2-azaspiro[3.3]heptan-6-yle ;
(1S,4S)-5-[5-(trifluorométhyl)pyrazin-2-yl]-2,5-diazabicyclo[2.2.1]heptane-2-carboxylate de 2-benzyl-2-azaspiro[3.3]heptan-6-yle ;
(1S,4S)-5-[5-(trifluorométhyl)pyrimidin-2-yl]-2,5-diazabicyclo[2.2.1]heptane-2-carboxylate de 2-benzyl-2-azaspiro[3.3]heptan-6-yle ;
4-[5-(trifluorométhyl)pyrimidin-2-yl]pipérazine-1-carboxylate de 2-benzyl-2-azaspiro[3.3]heptan-6-yle ;
(3R,5R)-4-(5-cyanopyrimidin-2-yl)-3,5-diméthylpipérazine-1-carboxylate de 2-benzyl-2-azaspiro[3.3]heptan-6-yle ;
(3S,5S)-4-(5-cyanopyrimidin-2-yl)-3,5-diméthylpipérazine-1-carboxylate de 2-benzyl-2-azaspiro[3.3]heptan-6-yle ;
(3S)-4-(5-cyanopyrimidin-2-yl)-3-méthylpipérazine-1-carboxylate de 2-benzyl-2-azaspiro[3.3]heptan-6-yle ;
(3R)-4-(5-cyanopyrimidin-2-yl)-3-méthylpipérazine-1-carboxylate de 2-benzyl-2-azaspiro[3.3]heptan-6-yle ;
4-[5-(trifluorométhyl)pyridin-2-yl]pipérazine-1-carboxylate de 2-benzyl-2-azaspiro[3.3]heptan-6-yle ;
(1R,4R)-5-[5-(trifluorométhyl)pyrazin-2-yl]-2,5-diazabicyclo[2.2.1]heptane-2-carboxylate de 2-benzyl-2-azaspiro[3.3]heptan-6-yle ;
(3S)-3-méthyl-4-[5-(trifluorométhyl)pyrimidin-2-yl]pipérazine-1-carboxylate de 2-benzyl-2-azaspiro[3.3]heptan-6-yle ;
4-(4-cyano-2,5-difluorophényl)pipérazine-1-carboxylate de 2-benzyl-2-azaspiro[3.3]heptan-6-yle ;
4-[4-(trifluorométhyl)phényl]pipérazine-1-carboxylate de 2-benzyl-2-azaspiro[3.3]heptan-6-yle ;
4-[2-cyano-4-(trifluorométhyl)phényl]pipérazine-1-carboxylate de 2-benzyl-2-azaspiro[3.3]heptan-6-yle ;
4-(3-cyano-5-fluorophényl)pipérazine-1-carboxylate de 2-benzyl-2-azaspiro[3.3]heptan-6-yle ;
(2R,5R)-4-(5-cyanopyrimidin-2-yl)-2,5-diméthylpipérazine-1-carboxylate de 2-benzyl-2-azaspiro[3.3]heptan-6-yle ;
(2S)-4-(5-cyanopyrimidin-2-yl)-2-méthylpipérazine-1-carboxylate de 2-benzyl-2-azaspiro[3.3]heptan-6-yle ;
(2R,6S)-4-(5-cyanopyrimidin-2-yl)-2,6-diméthylpipérazine-1-carboxylate de 2-benzyl-2-azaspiro[3.3]heptan-6-yle ;
(2R,6S)-4-(5-cyanopyrimidin-2-yl)-2,6-diméthylpipérazine-1-carboxylate de {2-benzyl-2-azaspiro[3.3]heptan-6-yl}méthyle ;
(2R)-2-méthyl-4-[5-(trifluorométhyl)pyrimidin-2-yl]pipérazine-1-carboxylate de 2-benzyl-2-azaspiro[3.3]heptan-6-yle ;
(2R)-2-méthyl-4-[5-(trifluorométhyl)pyrimidin-2-yl]pipérazine-1-carboxylate de {2-benzyl-2-azaspiro[3.3]heptan-6-yl}méthyle ;
(2R,5S)-2,5-diméthyl-4-[5-(trifluorométhyl)pyrimidin-2-yl]pipérazine-1-carboxylate de {2-benzyl-2-azaspiro[3.3]heptan-6-yl}méthyle ;
(2R,5R)-2,5-diméthyl-4-[5-(trifluorométhyl)pyrimidin-2-yl]pipérazine-1-carboxylate de 2-benzyl-2-azaspiro[3.3]heptan-6-yle ;
(2R,5S)-2,5-diméthyl-4-[5-(trifluorométhyl)pyrimidin-2-yl]pipérazine-1-carboxylate de 2-benzyl-2-azaspiro[3.3]heptan-6-yle ;
(2R)-2-méthyl-4-[5-(trifluorométhyl)pyrazin-2-yl]pipérazine-1-carboxylate de 2-benzyl-2-azaspiro[3.3]heptan-6-yle ;
(2R,5S)-4-(5-cyanopyrimidin-2-yl)-2,5-diméthylpipérazine-1-carboxylate de 2-benzyl-2-azaspiro[3.3]heptan-6-yle ;
(2R,6R)-2,6-diméthyl-4-[5-(trifluorométhyl)pyrimidin-2-yl]pipérazine-1-carboxylate de 2-benzyl-2-azaspiro[3.3]heptan-6-yle ;
(2R,6S)-2,6-diméthyl-4-[5-(trifluorométhyl)pyrazin-2-yl]pipérazine-1-carboxylate de {2-benzyl-2-azaspiro[3.3]heptan-6-yl}méthyle ;
(2R,6S)-2,6-diméthyl-4-[5-(trifluorométhyl)pyrazin-2-yl]pipérazine-1-carboxylate de 2-benzyl-2-azaspiro[3.3]heptan-6-yle ;
(2R,6R)-2,6-diméthyl-4-[5-(trifluorométhyl)pyrazin-2-yl]pipérazine-1-carboxylate de 2-benzyl-2-azaspiro[3.3]heptan-6-yle ;
(2R,6R)-2,6-diméthyl-4-[5-(trifluorométhyl)pyrazin-2-yl]pipérazine-1-carboxylate de {2-benzyl-2-azaspiro[3.3]heptan-6-yl}méthyle ;
(2R,6R)-2,6-diméthyl-4-[5-(trifluorométhyl)pyrimidin-2-yl]pipérazine-1-carboxylate de {2-benzyl-2-azaspiro[3.3]heptan-6-yl}méthyle ;
(2R,6R)-2,6-diméthyl-4-[5-(trifluorométhyl)pyrimidin-2-yl]pipérazine-1-carboxylate de 2-(3,3-diméthylbutyl)-2-azaspiro[3.3]heptan-6-yle ;
(2R,6R)-2,6-diméthyl-4-[5-(trifluorométhyl)pyrimidin-2-yl]pipérazine-1-carboxylate de 2-(2,2-diméthylpropyl)-2-azaspiro[3.3]heptan-6-yle ;
(2R,6S)-2,6-diméthyl-4-[5-(trifluorométhyl)pyrimidin-2-yl]pipérazine-1-carboxylate de 2-benzyl-2-azaspiro[3.3]heptan-6-yle ;
(2R,6S)-2,6-diméthyl-4-[5-(trifluorométhyl)pyrimidin-2-yl]pipérazine-1-carboxylate de {2-benzyl-2-azaspiro[3.3]heptan-6-yl}méthyle ;
(2R,6S)-2,6-diméthyl-4-[5-(trifluorométhyl)pyrimidin-2-yl]pipérazine-1-carboxylate de 2-(3,3-diméthylbutyl)-2-azaspiro[3.3]heptan-6-yle ;
(2R,6S)-2,6-diméthyl-4-[5-(trifluorométhyl)pyrimidin-2-yl]pipérazine-1-carboxylate de [2-(3,3-diméthylbutyl)-2-azaspiro[3.3]heptan-6-yl]méthyle ;
(2R,6S)-2,6-diméthyl-4-[5-(trifluorométhyl)pyrimidin-2-yl]pipérazine-1-carboxylate de 2-(2,2-diméthylpropyl)-2-azaspiro[3.3]heptan-6-yle ;
(2R,6S)-2,6-diméthyl-4-[5-(trifluorométhyl)pyrimidin-2-yl]pipérazine-1-carboxylate de [2-(2,2-diméthylpropyl)-2-azaspiro[3.3]heptan-6-yl]méthyle ;
(2R,6S)-2,6-diméthyl-4-[5-(trifluorométhyl)pyrimidin-2-yl]pipérazine-1-carboxylate de 2-[(2,2-diméthylcyclopropyl)méthyl]-2-azaspiro[3.3]heptan-6-yle ;
(2R,6S)-2,6-diméthyl-4-[5-(trifluorométhyl)pyrimidin-2-yl]pipérazine-1-carboxylate de 2-(cyclohexylméthyl)-2-azaspiro[3.3]heptan-6-yle ;
(2R,6S)-2,6-diméthyl-4-[5-(trifluorométhyl)pyrimidin-2-yl]pipérazine-1-carboxylate de 2-{bicyclo[2.2.1]hept-5-én-2-ylméthyl}-2-azaspiro[3.3]heptan-6-yle ;
(2R,6S)-2,6-diméthyl-4-[5-(trifluorométhyl)pyrimidin-2-yl]pipérazine-1-carboxylate de 2-[(2,6-difluorophényl)méthyl]-2-azaspiro[3.3]heptan-6-yle ;
(2R,6S)-4-(5-fluoropyrimidin-2-yl)-2,6-diméthylpipérazine-1-carboxylate de 2-benzyl-2-azaspiro[3.3]heptan-6-yle ;
(2R,6R)-4-(5-fluoropyrimidin-2-yl)-2,6-diméthylpipérazine-1-carboxylate de 2-benzyl-2-azaspiro[3.3]heptan-6-yle ;
(2R,6R)-4-(5-cyanopyrimidin-2-yl)-2,6-diméthylpipérazine-1-carboxylate de 2-benzyl-2-azaspiro[3.3]heptan-6-yle ;
7-(5-cyanopyrimidin-2-yl)-3-oxa-7,9-diazabicyclo[3.3.1]nonane-9-carboxylate de 2-benzyl-2-azaspiro[3.3]heptan-6-yle ;
(2R,6S)-4-(5-acétylpyrazin-2-yl)-2,6-diméthylpipérazine-1-carboxylate de 2-benzyl-2-azaspiro[3.3]heptan-6-yle ;
(2R,6R)-4-(5-acétylpyrazin-2-yl)-2,6-diméthylpipérazine-1-carboxylate fr 2-benzyl-2-azaspiro[3.3]heptan-6-yle ;
(2R,6S)-2,6-diméthyl-4-(5-nitropyridin-2-yl)pipérazine-1-carboxylate de 2-benzyl-2-azaspiro[3.3]heptan-6-yle ;
(2R,6R)-2,6-diméthyl-4-(5-nitropyridin-2-yl)pipérazine-1-carboxylate de 2-benzyl-2-azaspiro[3.3]heptan-6-yle ;
(2R,6S)-2,6-diméthyl-4-[6-(trifluorométhyl)pyrazin-2-yl]pipérazine-1-carboxylate de 2-benzyl-2-azaspiro[3.3]heptan-6-yle ;
(2R,6R)-2,6-diméthyl-4-[6-(trifluorométhyl)pyrazin-2-yl]pipérazine-1-carboxylate de 2-benzyl-2-azaspiro[3.3]heptan-6-yle ;
7-[5-(trifluorométhyl)pyrimidin-2-yl]-3-oxa-7,9-diazabicyclo[3.3.1]nonane-9-carboxylate de 2-benzyl-2-azaspiro[3.3]heptan-6-yle ;
7-[5-(trifluorométhyl)pyrazin-2-yl]-3-oxa-7,9-diazabicyclo[3.3.1]nonane-9-carboxylate de 2-benzyl-2-azaspiro[3.3]heptan-6-yle ;
(2R,6S)-4-[5-bromo-4-(méthylsulfanyl)pyrimidin-2-yl]-2,6-diméthylpipérazine-1-carboxylate de 2-benzyl-2-azaspiro[3.3]heptan-6-yle ;
(2R,6R)-4-[5-bromo-4-(méthylsulfanyl)pyrimidin-2-yl]-2,6-diméthylpipérazine-1-carboxylate de 2-benzyl-2-azaspiro[3.3]heptan-6-yle ;
2-[(3R,5R)-4-[({2-benzyl-2-azaspiro[3.3]heptan-6-yl}oxy)carbonyl]-3,5-diméthylpiperazin-1-yl]-4-(trifluorométhyl)pyrimidine-5-carboxylate de méthyle ;
(2R, 6S)-2,6-diméthyl-4-(quinoxalin-2-yl)pipérazine-1-carboxylate de 2-benzyl-2-azaspiro[3.3]heptan-6-yle ;
(2R,5S)-4-(6-méthoxyquinoxalin-2-yl)-2,5-diméthylpipérazine-1-carboxylate de 2-benzyl-2-azaspiro[3.3]heptan-6-yle ;
(2R,5S)-2,5-diméthyl-4-(4-méthylpyrimidin-2-yl)pipérazine-1-carboxylate de 2-benzyl-2-azaspiro[3.3]heptan-6-yle ;
(2R,5R)-2,5-diméthyl-4-(4-méthylpyrimidin-2-yl)pipérazine-1-carboxylate de 2-benzyl-2-azaspiro[3.3]heptan-6-yle ;
(2R,6R)-2,6-diméthyl-4-(4-méthylpyrimidin-2-yl)pipérazine-1-carboxylate de 2-benzyl-2-azaspiro[3.3]heptan-6-yle ;
(2R,5S)-4-(5-fluoropyrimidin-2-yl)-2,5-diméthylpipérazine-1-carboxylate de 2-benzyl-2-azaspiro[3.3]heptan-6-yle ;
(2R,5S)-4-(3,6-diméthylpyrazin-2-yl)-2,5-diméthylpipérazine-1-carboxylate de 2-benzyl-2-azaspiro[3.3]heptan-6-yle ;
(2R,5S)-4-(6-méthoxypyrazin-2-yl)-2,5-diméthylpipérazine-1-carboxylate de 2-benzyl-2-azaspiro[3.3]heptan-6-yle ;
(2R,5R)-4-(6-méthoxypyrazin-2-yl)-2,5-diméthylpipérazine-1-carboxylate de 2-benzyl-2-azaspiro[3.3]heptan-6-yle ;
(2R,6S)-4-(5-cyano-3-méthylpyrazin-2-yl)-2,6-diméthylpipérazine-1-carboxylate de 2-benzyl-2-azaspiro[3.3]heptan-6-yle ;
(2R,6R)-4-(5-cyano-3-méthylpyrazin-2-yl)-2,6-diméthylpipérazine-1-carboxylate de 2-benzyl-2-azaspiro[3.3]heptan-6-yle ;
(2R,5S)-2,5-diméthyl-4-(5-nitropyridin-2-yl)pipérazine-1-carboxylate de 2-benzyl-2-azaspiro[3.3]heptan-6-yle ;
(2R,5R)-2,5-diméthyl-4-(5-nitropyridin-2-yl)pipérazine-1-carboxylate de 2-benzyl-2-azaspiro[3.3]heptan-6-yle ;
(2R,6R)-2,6-diméthyl-4-(quinoxalin-2-yl)pipérazine-1-carboxylate de 2-benzyl-2-azaspiro[3.3]heptan-6-yle ;
(2R,5R)-2,5-diméthyl-4-(quinoxalin-2-yl)pipérazine-1-carboxylate de 2-benzyl-2-azaspiro[3.3]heptan-6-yle ;
(2R,5S)-2,5-diméthyl-4-(quinoxalin-2-yl)pipérazine-1-carboxylate de 2-benzyl-2-azaspiro[3.3]heptan-6-yle ;
9-(quinoxalin-2-yl)-3-oxa-7,9-diazabicyclo[3.3.1]nonane-7-carboxylate de 2-benzyl-2-azaspiro[3.3]heptan-6-yle ;
(2R,6S)-2,6-diméthyl-4-[3-(trifluorométhyl)pyridin-2-yl]pipérazine-1-carboxylate de 2-benzyl-2-azaspiro[3.3]heptan-6-yle ;
(2R,6R)-2,6-diméthyl-4-[3-(trifluorométhyl)pyridin-2-yl]pipérazine-1-carboxylate de 2-benzyl-2-azaspiro[3.3]heptan-6-yle ;
(2R,6S)-2,6-diméthyl-4-[6-(trifluorométhyl)pyridin-3-yl]pipérazine-1-carboxylate de 2-benzyl-2-azaspiro[3.3]heptan-6-yle ;
(2R,6R)-2,6-diméthyl-4-[6-(trifluorométhyl)pyridin-3-yl]pipérazine-1-carboxylate de 2-benzyl-2-azaspiro[3.3]heptan-6-yle ;
(2R,5R)-2,5-diméthyl-4-[6-(trifluorométhyl)pyridin-3-yl]pipérazine-1-carboxylate de 2-benzyl-2-azaspiro[3.3]heptan-6-yle ;
(2R,5S)-2,5-diméthyl-4-[6-(trifluorométhyl)pyrazin-2-yl]pipérazine-1-carboxylate de 2-benzyl-2-azaspiro[3.3]heptan-6-yle ;
(2R,5R)-2,5-diméthyl-4-[5-(trifluorométhyl)pyrazin-2-yl]pipérazine-1-carboxylate de 2-benzyl-2-azaspiro[3.3]heptan-6-yle ;
(2R,5R)-2,5-diméthyl-4-[6-(trifluorométhyl)pyrazin-2-yl]pipérazine-1-carboxylate de 2-benzyl-2-azaspiro[3.3]heptan-6-yle ;
(2R,6S)-4-(6-fluoroquinoxalin-2-yl)-2,6-diméthylpipérazine-1-carboxylate de 2-benzyl-2-azaspiro[3.3]heptan-6-yle ;
(2R,6S)-4-(6-méthoxy-3-nitropyridin-2-yl)-2,6-diméthylpipérazine-1-carboxylate de 2-benzyl-2-azaspiro[3.3]heptan-6-yle ;
(2R,5S)-4-(6-méthoxy-3-nitropyridin-2-yl)-2,5-diméthylpipérazine-1-carboxylate de 2-benzyl-2-azaspiro[3.3]heptan-6-yle ;
(2R,6R)-4-(6-méthoxy-3-nitropyridin-2-yl)-2,6-diméthylpipérazine-1-carboxylate de 2-benzyl-2-azaspiro[3.3]heptan-6-yle ;
(2R,5R)-4-(6-méthoxyquinoxalin-2-yl)-2,5-diméthylpipérazine-1-carboxylate de 2-benzyl-2-azaspiro[3.3]heptan-6-yle ;
(2R,6S)-2,6-diméthyl-4-[5-(trifluorométhoxy)pyrazin-2-yl]pipérazine-1-carboxylate de 2-benzyl-2-azaspiro[3.3]heptan-6-yle ;
(2R,6R)-2,6-diméthyl-4-[5-(trifluorométhoxy)pyrazin-2-yl]pipérazine-1-carboxylate de 2-benzyl-2-azaspiro[3.3]heptan-6-yle ;
(2R,5S)-2,5-diméthyl-4-[5-(trifluorométhoxy)pyrazin-2-yl]pipérazine-1-carboxylate de 2-benzyl-2-azaspiro[3.3]heptan-6-yle ;
(2R,5R)-2,5-diméthyl-4-[5-(trifluorométhoxy)pyrazin-2-yl]pipérazine-1-carboxylate de 2-benzyl-2-azaspiro[3.3]heptan-6-yle ;
(2R,6R)-4-[4-cyano-6-(trifluorométhyl)pyridin-3-yl]-2,6-diméthylpipérazine-1-carboxylatede 2-benzyl-2-azaspiro[3.3]heptan-6-yle ;
(2R,5R)-4-[4-cyano-6-(trifluorométhyl)pyridin-3-yl]-2,5-diméthylpipérazine-1-carboxylate de 2-benzyl-2-azaspiro[3.3]heptan-6-yle ;
2-[(2S,5R)-4-[({2-benzyl-2-azaspiro[3.3]heptan-6-yl}oxy)carbonyl]-2,5-diméthylpiperazin-1-yl]-4-(trifluorométhyl)pyrimidine-5-carboxylate de méthyle ;
(2R,6S)-N-{2-benzyl-2-azaspiro[3.3]heptan-6-yl}-2,6-diméthyl-4-(quinazolin-2-yl)pipérazine-1-carboxamide ;
(2R,6S)-N-{2-benzyl-2-azaspiro[3.3]heptan-6-yl}-4-(6-fluoroquinoxalin-2-yl)-2,6-diméthylpipérazine-1-carboxamide ;
(2R,6R)-N-{2-benzyl-2-azaspiro[3.3]heptan-6-yl}-2,6-diméthyl-4-(quinazolin-2-yl)pipérazine-1-carboxamide ;
(2R,5S)-N-{2-benzyl-2-azaspiro[3.3]heptan-6-yl}-2,5-diméthyl-4-(quinazolin-2-yl)pipérazine-1-carboxamide ;
(2R)-N-{2-benzyl-2-azaspiro[3.3]heptan-6-yl}-2-méthyl-4-(quinazolin-2-yl)pipérazine-1-carboxamide ;
(2R,6S)-N-{2-benzyl-2-azaspiro[3.3]heptan-6-yl}-2,6-diméthyl-4-(quinoxalin-2-yl)pipérazine-1-carboxamide ;
(2R,6S)-N-{2-benzyl-2-azaspiro[3.3]heptan-6-yl}-4-(6-méthoxyquinoxalin-2-yl)-2,6-diméthylpipérazine-1-carboxamide ;
(2R,6S)-N-{2-benzyl-2-azaspiro[3.3]heptan-6-yl}-2,6-diméthyl-4-[6-(trifluorométhyl)-1,3-benzothiazol-2-yl]pipérazine-1-carboxamide ;
(2R,6R)-N-{2-benzyl-2-azaspiro[3.3]heptan-6-yl}-2,6-diméthyl-4-[6-(trifluorométhyl)-1,3-benzothiazol-2-yl]pipérazine-1-carboxamide ;
(2R,6R)-N-{2-benzyl-2-azaspiro[3.3]heptan-6-yl}-4-(6-fluoroquinoxalin-2-yl)-2,6-diméthylpipérazine-1-carboxamide ;
(2R,6R)-N-{2-benzyl-2-azaspiro[3.3]heptan-6-yl}-4-(6-méthoxyquinoxalin-2-yl)-2,6-diméthylpipérazine-1-carboxamide ;
(2R,6R)-N-{2-benzyl-2-azaspiro[3.3]heptan-6-yl}-2,6-diméthyl-4-(quinoxalin-2-yl)pipérazine-1-carboxamide ;
(2R,5S)-N-{2-benzyl-2-azaspiro[3.3]heptan-6-yl}-2,5-diméthyl-4-(quinoxalin-2-yl)pipérazine-1-carboxamide ;
(2R,5S)-N-{2-benzyl-2-azaspiro[3.3]heptan-6-yl}-4-(6-méthoxyquinoxalin-2-yl)-2,5-diméthylpipérazine-1-carboxamide ;
(2R,6S)-N-{2-benzyl-2-azaspiro[3.3]heptan-6-yl}-2,6-diméthyl-4-[6-(trifluorométhyl)-1,3-benzothiazol-2-yl]pipérazine-1-carboxamide ;
(2R)-N-{2-benzyl-2-azaspiro[3.3]heptan-6-yl}-2-méthyl-4-(quinoxalin-2-yl)pipérazine-1-carboxamide ;
(2R)-N-{2-benzyl-2-azaspiro[3.3]heptan-6-yl}-2-méthyl-4-[6-(trifluorométhyl)-1,3-benzothiazol-2-yl]pipérazine-1-carboxamide ;
(2R)-N-{2-benzyl-2-azaspiro[3.3]heptan-6-yl}-4-(6-méthoxyquinoxalin-2-yl)-2-méthylpipérazine-1-carboxamide ;
(2R,6S)-N-{2-benzyl-2-azaspiro[3.3]heptan-6-yl}-4-[3-fluoro-5-(trifluorométhyl)pyridin-2-yl]-2,6-diméthylpipérazine-1-carboxamide ;
(2R,6S)-N-{2-benzyl-2-azaspiro[3.3]heptan-6-yl}-4-(5-chloro-4-méthylpyrimidin-2-yl)-2,6-diméthylpipérazine-1-carboxamide ;
(2R,6S)-N-{2-benzyl-2-azaspiro[3.3]heptan-6-yl}-4-(5-chloropyrazin-2-yl)-2,6-diméthylpipérazine-1-carboxamide ;
(2R,6S)-4-(6-amino-5-chloropyrazin-2-yl)-N-{2-benzyl-2-azaspiro[3.3]heptan-6-yl}-2,6-diméthylpipérazine-1-carboxamide ;
(2R,6S)-N-{2-benzyl-2-azaspiro[3.3]heptan-6-yl}-4-(6-fluoro-1,3-benzothiazol-2-yl)-2,6-diméthylpipérazine-1-carboxamide ;
(2R,6R)-N-{2-benzyl-2-azaspiro[3.3]heptan-6-yl}-4-(6-fluoro-1,3-benzothiazol-2-yl)-2,6-diméthylpipérazine-1-carboxamide ;
(2R,5S)-N-{2-benzyl-2-azaspiro[3.3]heptan-6-yl}-4-(6-fluoro-1,3-benzothiazol-2-yl)-2,5-diméthylpipérazine-1-carboxamide ;
(2R)-N-{2-benzyl-2-azaspiro[3.3]heptan-6-yl}-4-(6-fluoro-1,3-benzothiazol-2-yl)-2-méthylpipérazine-1-carboxamide ;
(2R,6S)-N-{2-benzyl-2-azaspiro[3.3]heptan-6-yl}-2,6-diméthyl-4-[6-(trifluorométhyl)quinoxalin-2-yl]pipérazine-1-carboxamide ;
(2R,6R)-N-{2-benzyl-2-azaspiro[3.3]heptan-6-yl}-2,6-diméthyl-4-[6-(trifluorométhyl)quinoxalin-2-yl]pipérazine-1-carboxamide ;
(2R,5S)-N-{2-benzyl-2-azaspiro[3.3]heptan-6-yl}-2,5-diméthyl-4-[6-(trifluorométhyl)quinoxalin-2-yl]pipérazine-1-carboxamide ;
(2R)-N-{2-benzyl-2-azaspiro[3.3]heptan-6-yl}-2-méthyl-4-[6-(trifluorométhyl)quinoxalin-2-yl]pipérazine-1-carboxamide ;
(2R,6S)-N-{2-benzyl-2-azaspiro[3.3]heptan-6-yl}-2,6-diméthyl-4-{[1,3]oxazolo[4,5-b]pyridin-2-yl}pipérazine-1-carboxamide ;
(2R,6R)-N-{2-benzyl-2-azaspiro[3.3]heptan-6-yl}-2,6-diméthyl-4-{[1,3]oxazolo[4,5-b]pyridin-2-yl}pipérazine-1-carboxamide ;
(2R,6S)-N-{2-benzyl-2-azaspiro[3.3]heptan-6-yl}-4-(6-méthoxy-1,3-benzothiazol-2-yl)-2,6-diméthylpipérazine-1-carboxamide ;
(2R,6R)-N-{2-benzyl-2-azaspiro[3.3]heptan-6-yl}-4-(6-méthoxy-1,3-benzothiazol-2-yl)-2,6-diméthylpipérazine-1-carboxamide ;
(2R,6R)-4-(1,3-benzothiazol-2-yl)-N-{2-benzyl-2-azaspiro[3.3]heptan-6-yl}-2,6-diméthylpipérazine-1-carboxamide ;
(2R,5S)-4-(1,3-benzothiazol-2-yl)-N-{2-benzyl-2-azaspiro[3.3]heptan-6-yl}-2,5-diméthylpipérazine-1-carboxamide ;
(2R)-4-(1,3-benzothiazol-2-yl)-N-{2-benzyl-2-azaspiro[3.3]heptan-6-yl}-2-méthylpipérazine-1-carboxamide ;
(2R,5S)-N-{2-benzyl-2-azaspiro[3.3]heptan-6-yl}-4-(6-fluoroquinoxalin-2-yl)-2,5-diméthylpipérazine-1-carboxamide ;
(2R,6S)-N-{2-benzyl-2-azaspiro[3.3]heptan-6-yl}-4-[5-(difluorométhoxy)pyrimidin-2-yl]-2,6-diméthylpipérazine-1-carboxamide ;
(2R,6S)-4-[4-amino-5-(trifluorométhyl)pyrimidin-2-yl]-N-{2-benzyl-2-azaspiro[3.3]heptan-6-yl}-2,6-diméthylpipérazine-1-carboxamide ;
(2R,6S)-N-{2-benzyl-2-azaspiro[3.3]heptan-6-yl}-4-[5-(difluorométhyl)pyrimidin-2-yl]-2,6-diméthylpipérazine-1-carboxamide ;
(2R,6S)-N-{2-benzyl-2-azaspiro[3.3]heptan-6-yl}-4-(5-fluoropyrimidin-2-yl)-2,6-diméthylpipérazine-1-carboxamide ;
(2R,6S)-N-{2-[(4-chlorophényl)méthyl]-2-azaspiro[3.3]heptan-6-yl}-4-(5-cyanopyrimidin-2-yl)-2,6-diméthylpipérazine-1-carboxamide ;
(2R,6R)-N-{2-[(4-chlorophényl)méthyl]-2-azaspiro[3.3]heptan-6-yl}-4-(5-cyanopyrimidin-2-yl)-2,6-diméthylpipérazine-1-carboxamide ;
(2R,5S)-N-{2-[(4-chlorophényl)méthyl]-2-azaspiro[3.3]heptan-6-yl}-4-(5-cyanopyrimidin-2-yl)-2,5-diméthylpipérazine-1-carboxamide ;
(2R)-N-{2-[(4-chlorophényl)méthyl]-2-azaspiro[3.3]heptan-6-yl}-4-(5-cyanopyrimidin-2-yl)-2-méthylpipérazine-1-carboxamide ;
(2R,6R)-N-{2-[(4-chlorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,5S)-N-{2-[(4-chlorophényl)méthyl]-2-azaspiro[3.3]heptan-6-yl}-2,5-diméthyl-4-[5-(trifluorométhyl)pyrimidin-2-yl]pipérazine-1-carboxamide ;
(2R)-N-{2-[(4-chlorophényl)méthyl]-2-azaspiro[3.3]heptan-6-yl}-2-méthyl-4-[5-(trifluorométhyl)pyrimidin-2-yl]pipérazine-1-carboxamide ;
(2R,6S)-N-{2-[(4-chlorophényl)méthyl]-2-azaspiro[3.3]heptan-6-yl}-2,6-diméthyl-4-[5-(trifluorométhyl)pyrazin-2-yl]pipérazine-1-carboxamide ;
(2R,6R)-N-{2-[(4-chlorophényl)méthyl]-2-azaspiro[3.3]heptan-6-yl}-2,6-diméthyl-4-[5-(trifluorométhyl)pyrazin-2-yl]pipérazine-1-carboxamide ;
(2R,5S)-N-{2-[(4-chlorophényl)méthyl]-2-azaspiro[3.3]heptan-6-yl}-2,5-diméthyl-4-[5-(trifluorométhyl)pyrazin-2-yl]pipérazine-1-carboxamide ;
(2R)-N-{2-[(4-chlorophényl)méthyl]-2-azaspiro[3.3]heptan-6-yl}-2-méthyl-4-[5-(trifluorométhyl)pyrazin-2-yl]pipérazine-1-carboxamide ;
(2R,6R)-4-(5-cyanopyrimidin-2-yl)-N-{2-[(4-fluorophényl)méthyl]-2-azaspiro[3.3]heptan-6-yl}-2,6-diméthylpipérazine-1-carboxamide ;
(2R,5S)-4-(5-cyanopyrimidin-2-yl)-N-{2-[(4-fluorophényl)méthyl]-2-azaspiro[3.3]heptan-6-yl}-2,5-diméthylpipérazine-1-carboxamide ;
(2R)-4-(5-cyanopyrimidin-2-yl)-N-{2-[(4-fluorophényl)méthyl]-2-azaspiro[3.3]heptan-6-yl}-2-méthylpipérazine-1-carboxamide ;
(2R,5S)-N-{2-[(4-fluorophényl)méthyl]-2-azaspiro[3.3]heptan-6-yl}-2,5-diméthyl-4-[5-(trifluorométhyl)pyrimidin-2-yl]pipérazine-1-carboxamide ;
(2R)-N-{2-[(4-fluorophényl)méthyl]-2-azaspiro[3.3]heptan-6-yl}-2-méthyl-4-[5-(trifluorométhyl)pyrimidin-2-yl]pipérazine-1-carboxamide ;
(2R,6S)-N-{2-[(4-fluorophényl)méthyl]-2-azaspiro[3.3]heptan-6-yl}-2,6-diméthyl-4-[5-(trifluorométhyl)pyrazin-2-yl]pipérazine-1-carboxamide ;
(2R, 6R)-N-{2-[(4-fluorophényl)méthyl]-2-azaspiro[3.3]heptan-6-yl}-2,6-diméthyl-4-[5-(trifluorométhyl)pyrazin-2-yl]pipérazine-1-carboxamide ;
(2R,5S)-N-{2-[(4-fluorophényl)méthyl]-2-azaspiro[3.3]heptan-6-yl}-2,5-diméthyl-4-[5-(trifluorométhyl)pyrazin-2-yl]pipérazine-1-carboxamide ;
(2R)-N-{2-[(4-fluorophényl)méthyl]-2-azaspiro[3.3]heptan-6-yl}-2-méthyl-4-[5-(trifluorométhyl)pyrazin-2-yl]pipérazine-1-carboxamide ;
(2R,6S)-N-{2-benzyl-2-azaspiro[3.3]heptan-6-yl}-4-(6-fluoroquinazolin-2-yl)-2,6-diméthylpipérazine-1-carboxamide ;
(2R,5S)-N-{2-benzyl-2-azaspiro[3.3]heptan-6-yl}-4-(6-fluoroquinazolin-2-yl)-2,5-diméthylpipérazine-1-carboxamide ;
(2R,6S)-N-{2-[(²H₅)benzyl]-2-azaspiro[3.3] heptan-6-yl}-2,6-diméthyl-4-[5-(trifluorométhyl)pyrimidin-2-yl]pipérazine-1-carboxamide ;
(2R,6S)-2,6-diméthyl-N-{2-[(²H₅)phényl(2H₁)méthyl]-2-azaspiro[3.3]heptan-6-yl}-4-[5-(trifluorométhyl)pyrimidin-2-yl]pipérazine-1-carboxamide ;
(2R,6S)-2,6-diméthyl-N-{2-[(²H₅)phényl(2H₂)méthyl]-2-azaspiro[3.3]heptan-6-yl}-4-[5-(trifluorométhyl)pyrimidin-2-yl]pipérazine-1-carboxamide ;
(2R,6S)-4-[4-méthoxy-5-(trifluorométhyl)pyrimidin-2-yl]-2,6-diméthylpipérazine-1-carboxylate de 2-benzyl-2-azaspiro[3.3]heptan-6-yle ;
(2R,6S)-4-[3-fluoro-5-(trifluorométhyl)pyridin-2-yl]-2,6-diméthylpipérazine-1-carboxylate de 2-benzyl-2-azaspiro[3.3]heptan-6-yle ;
(2R,6S)-4-(5-chloropyrimidin-2-yl)-2,6-diméthylpipérazine-1-carboxylate de 2-benzyl-2-azaspiro[3.3]heptan-6-yle ;
(2R,6S)-4-(5-chloro-4-méthylpyrimidin-2-yl)-2,6-diméthylpipérazine-1-carboxylate de 2-benzyl-2-azaspiro[3.3]heptan-6-yle ;
(2R,6S)-4-(5-chloropyrazin-2-yl)-2,6-diméthylpipérazine-1-carboxylate de 2-benzyl-2-azaspiro[3.3]heptan-6-yle ;
N-{2-benzyl-2-azaspiro[3.3]heptan-6-yl}-9-[5-(trifluorométhyl)pyrimidin-2-yl]-3-oxa-7,9-diazabicyclo[3.3.1]nonane-7-carboxamide ;
(2R,6S)-4-(6-amino-5-chloropyrazin-2-yl)-2,6-diméthylpipérazine-1-carboxylate de 2-benzyl-2-azaspiro[3.3]heptan-6-yle ;
(2R,5S)-2,5-diméthyl-4-[5-(trifluorométhyl)pyrazin-2-yl]pipérazine-1-carboxylate de 2-[(²H₅)benzyl]-2-azaspiro[3.3]heptan-6-yle ;
(2R,6S)-N-{2-benzyl-2-azaspiro[3.3]heptan-6-yl}-4-(4-éthoxyphényl)-2,6-diméthylpipérazine-1-carboxamide ;
(2R,6S)-N-{2-benzyl-2-azaspiro[3.3]heptan-6-yl}-4-(4-méthoxyphényl)-2,6-diméthylpipérazine-1-carboxamide ;
(2R,6S)-N-{2-benzyl-2-azaspiro[3.3]heptan-6-yl}-4-(4-fluoro-3-méthylphényl)-2,6-diméthylpipérazine-1-carboxamide ;
(2R,6S)-N-{2-benzyl-2-azaspiro[3.3]heptan-6-yl}-4-(4-cyano-3-méthoxyphényl)-2,6-diméthylpipérazine-1-carboxamide ;
(2R,5S)-N-{2-benzyl-2-azaspiro[3.3]heptan-6-yl}-2,5-diméthyl-4-[4-(trifluorométhyl)phényl]pipérazine-1-carboxamide ;
(2R)-N-{2-benzyl-2-azaspiro[3.3]heptan-6-yl}-2-méthyl-4-[4-(trifluorométhyl)phényl]pipérazine-1-carboxamide ;
(2R,6S)-N-{2-[(3-méthoxypyridin-4-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(2-chloropyridin-3-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-[2-(1H-indol-4-ylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-2,6-diméthyl-N-[2-(pyridin-2-ylméthyl)-2-azaspiro[3.3]heptan-6-yl]-4-[5-(trifluorométhyl)pyrimidin-2-yl]pipérazine-1-carboxamide ;
(2R,6S)-N-{2-[(2-chlorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-N-[2-(1,3-thiazol-4-ylméthyl)-2-azaspiro[3.3]heptan-6-yl]-4-[5-(trifluorométhyl)pyrimidin-2-yl]pipérazine-1-carboxamide ;
(2R,6S)-N-{2-[(1,3-diméthyl-1H-pyrazol-5-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(4-méthoxypyridin-3-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-[2-(1-benzofuran-5-ylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-2,6-diméthyl-N-{2-[(1-méthyl-1H-imidazol-2-yl)méthyl]-2-azaspiro[3.3]heptan-6-yl}-4-[5-(trifluorométhyl)pyrimidin-2-yl]pipérazine-1-carboxamide ;
(2R,6S)-2,6-diméthyl-N-{2-[(4-méthyl-1,3-thiazol-2-yl)méthyl]-2-azaspiro[3.3]heptan-6-yl}-4-[5-(trifluorométhyl)pyrimidin-2-yl]pipérazine-1-carboxamide ;
(2R,6S)-2,6-diméthyl-N-{2-[(4-méthyl-1H-imidazol-5-yl)méthyl]-2-azaspiro[3.3]heptan-6-yl}-4-[5-(trifluorométhyl)pyrimidin-2-yl]pipérazine-1-carboxamide ;
(2R,6S)-N-{2-[(3-chloropyridin-4-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-N-[2-(pyridin-3-ylméthyl)-2-azaspiro[3.3]heptan-6-yl]-4-[5-(trifluorométhyl)pyrimidin-2-yl]pipérazine-1-carboxamide ;
(2R,6S)-N-{2-[(4-méthoxypyridin-2-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(6-méthoxypyridin-3-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-[2-(1H-imidazol-4-ylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-N-{2-[(6-chloropyridin-3-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(2-fluoropyridin-3-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-N-[2-(1H-pyrrol-2-ylméthyl)-2-azaspiro[3.3]heptan-6-yl]-4-[5-(trifluorométhyl)pyrimidin-2-yl]pipérazine-1-carboxamide ;
(2R,6S)-N-{2-[(2,5-difluorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-N-[2-(2-phényléthyl)-2-azaspiro[3.3]heptan-6-yl]-4-[5-(trifluorométhyl)pyrimidin-2-yl]pipérazine-1-carboxamide ;
(2R,6S)-2,6-diméthyl-N-[2-(4-phénylbutyl)-2-azaspiro[3.3]heptan-6-yl]-4-[5-(trifluorométhyl)pyrimidin-2-yl]pipérazine-1-carboxamide ;
(2R,6S)-N-[2-(cyclohex-1-én-1-ylméthyl)-2-azaspiro[3.3]heptan-6-yl]-2,6-diméthyl-4-[5-(trifluorométhyl)pyrimidin-2-yl]pipérazine-1-carboxamide ;
(2R,6S)-2,6-diméthyl-N-{2-[(4-nitrophényl)méthyl]-2-azaspiro[3.3]heptan-6-yl}-4-[5-(trifluorométhyl)pyrimidin-2-yl]pipérazine-1-carboxamide ;
(2R,6S)-N-(2-{ [4-(diméthylamino)phényl]méthyl}-2-azaspiro[3.3]heptan-6-yl)-2,6-diméthyl-4-[5-(trifluorométhyl)pyrimidin-2-yl]pipérazine-1-carboxamide ;
(2R,6S)-2,6-diméthyl-N-(2-{[4-(propan-2-yl)phényl]méthyl}-2-azaspiro[3.3]heptan-6-yl)-4-[5-(trifluorométhyl)pyrimidin-2-yl]pipérazine-1-carboxamide ;
(2R,6S)-2,6-diméthyl-N-[2-(pyridin-4-ylméthyl)-2-azaspiro[3.3]heptan-6-yl]-4-[5-(trifluorométhyl)pyrimidin-2-yl]pipérazine-1-carboxamide ;
(2R,6S)-N-{2-[(4-éthylphényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(4-hydroxyphényl)méthyl]-2-azaspiro[3.3]heptan-6-yl}-2,6-diméthyl-4-[5-(trifluorométhyl)pyrimidin-2-yl]pipérazine-1-carboxamide ;
4-[(6-{[(2R,6S)-2,6-diméthyl-4-[5-(trifluorométhyl)pyrimidin-2-yl]pipérazine-1-carbonyl]amino}-2-azaspiro[3.3]heptan-2-yl)méthyl]benzoate de méthyle ;
(2R,6S)-N-{2-[(4-acétamidophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-(2-{ [4-(difluorométhoxy)phényl]méthyl}-2-azaspiro[3.3]heptan-6-yl)-2,6-diméthyl-4-[5-(trifluorométhyl)pyrimidin-2-yl]pipérazine-1-carboxamide ;
(2R,6S)-2,6-diméthyl-N-(2-{[4-(trifluorométhyl)phényl]méthyl}-2-azaspiro[3.3]heptan-6-yl)-4-[5-(trifluorométhyl)pyrimidin-2-yl]pipérazine-1-carboxamide ;
(2R,6S)-N-{2-[(4-carbamimidoylphényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-{2-[(4-chlorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-N-(2-{imidazo[1,2-a]pyridin-6-ylméthyl}-2-azaspiro[3.3]heptan-6-yl)-2,6-diméthyl-4-[5-(trifluorométhyl)pyrimidin-2-yl]pipérazine-1-carboxamide ;
(2R,6S)-N-[2-(adamantan-1-ylméthyl)-2-azaspiro[3.3]heptan-6-yl]-2,6-diméthyl-4-[5-(trifluorométhyl)pyrimidin-2-yl]pipérazine-1-carboxamide ;
(2R,6R)-N-{2-[(4-fluorophényl)méthyl]-2-azaspiro[3.3]heptan-6-yl}-2,6-diméthyl-4-[5-(trifluorométhyl)pyrimidin-2-yl]pipérazine-1-carboxamide ;
(2R,6R)-N-{2-[(4-carbamoylphényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-4-(5-cyanopyrimidin-2-yl)-N-{2-[(4-fluorophényl)méthyl]-2-azaspiro[3.3]heptan-6-yl}-2,6-diméthylpipérazine-1-carboxamide ;
(2R,6S)-N-{2-[(4-carbamoylphényl)méthyl]-2-azaspiro[3.3]heptan-6-yl}-4-(5-cyanopyrimidin-2-yl)-2,6-diméthylpipérazine-1-carboxamide ;
(2R,6S)-N-[2-(cyanométhyl)-2-azaspiro[3.3]heptan-6-yl]-2,6-diméthyl-4-[5-(trifluorométhyl)pyrimidin-2-yl]pipérazine-1-carboxamide ;
(2R,6S)-N-{2-[(3,3-difluorocyclobutyl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-N-[2-(1-phényléthyl)-2-azaspiro[3.3]heptan-6-yl]-4-[5-(trifluorométhyl)pyrimidin-2-yl]pipérazine-1-carboxamide ;
(2R,6S)-2,6-diméthyl-N-[2-(1H-pyrrol-3-ylméthyl)-2-azaspiro[3.3]heptan-6-yl]-4-[5-(trifluorométhyl)pyrimidin-2-yl]pipérazine-1-carboxamide ;
(2R,6S)-N-(2-{ [4-(diméthylcarbamoyl)phényl]méthyl}-2-azaspiro[3.3]heptan-6-yl)-2,6-diméthyl-4-[5-(trifluorométhyl)pyrimidin-2-yl]pipérazine-1-carboxamide ;
(2R,6S)-N-{2-[(2-hydroxyphényl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-N-{2-[(3-méthylthiophen-2-yl)méthyl]-2-azaspiro[3.3]heptan-6-yl}-4-[5-(trifluorométhyl)pyrimidin-2-yl]pipérazine-1-carboxamide ;
(2R,6S)-2,6-diméthyl-N-{2-[(5-méthylthiophen-2-yl)méthyl]-2-azaspiro[3.3]heptan-6-yl}-4-[5-(trifluorométhyl)pyrimidin-2-yl]pipérazine-1-carboxamide ;
(2R,6S)-N-{2-[(5-chlorothiophen-2-yl)méthyl]-2-azaspiro[3.3]heptan-6-yl}-2,6-diméthyl-4-[5-(trifluorométhyl)pyrimidin-2-yl]pipérazine-1-carboxamide ;
(2R,6S)-2,6-diméthyl-4-[5-(trifluorométhyl)pyrimidin-2-yl]pipérazine-1-carboxylate de 2-benzyl-6-(difluorométhyl)-2-azaspiro[3.3]heptan-6-yle ;
(2R,6S)-2,6-diméthyl-4-[5-(trifluorométhyl)pyrazin-2-yl]pipérazine-1-carboxylate de 2-benzyl-6-(difluorométhyl)-2-azaspiro[3.3]heptan-6-yle ;
(2R,6S)-2,6-diméthyl-4-[6-(trifluorométhyl)pyridazin-3-yl]pipérazine-1-carboxylate de 2-benzyl-6-(difluorométhyl)-2-azaspiro[3.3]heptan-6-yle ;
(2R,6R)-2,6-diméthyl-4-[5-(trifluorométhyl)pyrimidin-2-yl]pipérazine-1-carboxylate de 2-benzyl-6-(difluorométhyl)-2-azaspiro[3.3]heptan-6-yle ;
(2R,6R)-2,6-diméthyl-4-[5-(trifluorométhyl)pyrazin-2-yl]pipérazine-1-carboxylate de 2-benzyl-6-(difluorométhyl)-2-azaspiro[3.3]heptan-6-yle ;
(2R,5S)-2,5-diméthyl-4-[5-(trifluorométhyl)pyrimidin-2-yl]pipérazine-1-carboxylate de 2-benzyl-6-(difluorométhyl)-2-azaspiro[3.3]heptan-6-yle ;
(2R,5S)-2,5-diméthyl-4-[5-(trifluorométhyl)pyrazin-2-yl]pipérazine-1-carboxylate de 2-benzyl-6-(difluorométhyl)-2-azaspiro[3.3]heptan-6-yle ;
(2R)-4-(5-cyanopyrimidin-2-yl)-2-méthylpipérazine-1-carboxylate de 2-benzyl-6-(difluorométhyl)-2-azaspiro[3.3]heptan-6-yle ;
(2R)-2-méthyl-4-[5-(trifluorométhyl)pyrimidin-2-yl]pipérazine-1-carboxylate de 2-benzyl-6-(difluorométhyl)-2-azaspiro[3.3]heptan-6-yle ;
(2R)-2-méthyl-4-[5-(trifluorométhyl)pyrazin-2-yl]pipérazine-1-carboxylate de 2-benzyl-6-(difluorométhyl)-2-azaspiro[3.3]heptan-6-yle ;
(2R,6S)-4-(5-cyanopyrimidin-2-yl)-2,6-diméthylpipérazine-1-carboxylate de 2-benzyl-6-méthyl-2-azaspiro[3.3]heptan-6-yle ;
(2R,6S)-2,6-diméthyl-4-[5-(trifluorométhyl)pyrimidin-2-yl]pipérazine-1-carboxylate de 2-benzyl-6-méthyl-2-azaspiro[3.3]heptan-6-yle ;
(2R,6S)-2,6-diméthyl-4-[5-(trifluorométhyl)pyrazin-2-yl]pipérazine-1-carboxylate de 2-benzyl-6-méthyl-2-azaspiro[3.3]heptan-6-yle ;
(2R,6R)-4-(5-cyanopyrimidin-2-yl)-2,6-diméthylpipérazine-1-carboxylate de 2-benzyl-6-méthyl-2-azaspiro[3.3]heptan-6-yle ;
(2R,6R)-2,6-diméthyl-4-[5-(trifluorométhyl)pyrimidin-2-yl]pipérazine-1-carboxylate de 2-benzyl-6-méthyl-2-azaspiro[3.3]heptan-6-yle ;
(2R,6R)-2,6-diméthyl-4-[5-(trifluorométhyl)pyrazin-2-yl]pipérazine-1-carboxylate de 2-benzyl-6-méthyl-2-azaspiro[3.3]heptan-6-yle ;
(2R,5S)-4-(5-cyanopyrimidin-2-yl)-2,5-diméthylpipérazine-1-carboxylate de 2-benzyl-6-méthyl-2-azaspiro[3.3]heptan-6-yle ;
(2R,5S)-2,5-diméthyl-4-[5-(trifluorométhyl)pyrimidin-2-yl]pipérazine-1-carboxylate de 2-benzyl-6-méthyl-2-azaspiro[3.3]heptan-6-yle ;
(2R,5S)-2,5-diméthyl-4-[5-(trifluorométhyl)pyrazin-2-yl]pipérazine-1-carboxylate de 2-benzyl-6-méthyl-2-azaspiro[3.3]heptan-6-yle ;
(2R,5S)-2,5-diméthyl-4-[5-(trifluorométhyl)pyrazin-2-yl]pipérazine-1-carboxylate de 2-[(²H₅)phényl(²H₁)méthyl]-2-azaspiro[3.3]heptan-6-yle ;
(2R,5S)-2,5-diméthyl-4-[5-(trifluorométhyl)pyrazin-2-yl]pipérazine-1-carboxylate de 2-[(²H₅)phényl(²H₂)méthyl]-2-azaspiro[3.3]heptan-6-yle ;
(2R,6R)-4-(6-fluoroquinoxalin-2-yl)-2,6-diméthylpipérazine-1-carboxylate de 2-benzyl-2-azaspiro[3.3]heptan-6-yle ;
(2R,5S)-4-(6-fluoroquinoxalin-2-yl)-2,5-diméthylpipérazine-1-carboxylate de 2-benzyl-2-azaspiro[3.3]heptan-6-yle ;
(2R,6S)-2,6-diméthyl-4-[6-(trifluorométhyl)-1,3-benzothiazol-2-yl]pipérazine-1-carboxylate de 2-benzyl-2-azaspiro[3.3]heptan-6-yle ;
(2R,6S)-2,6-diméthyl-4-[6-(trifluorométhyl)-[1,3]thiazolo[S,4-b]pyridin-2-yl]pipérazine-1-carboxylate de 2-benzyl-2-azaspiro[3.3]heptan-6-yle ;
(2R,6R)-2,6-diméthyl-4-[6-(trifluorométhyl)-[1,3]thiazolo[S,4-b]pyridin-2-yl]pipérazine-1-carboxylate de 2-benzyl-2-azaspiro[3.3]heptan-6-yle ;
(2R,5S)-2,5-diméthyl-4-[6-(trifluorométhyl)-[1,3]thiazolo[S,4-b]pyridin-2-yl]pipérazine-1-carboxylate de 2-benzyl-2-azaspiro[3.3]heptan-6-yle ;
(2R,6S)-4-(6-fluoro-1,3-benzothiazol-2-yl)-2,6-diméthylpipérazine-1-carboxylate de 2-benzyl-2-azaspiro[3.3]heptan-6-yle ;
(2R,6R)-4-(6-fluoro-1,3-benzothiazol-2-yl)-2,6-diméthylpipérazine-1-carboxylate de 2-benzyl-2-azaspiro[3.3]heptan-6-yle ;
(2R,5S)-4-(6-fluoro-1,3-benzothiazol-2-yl)-2,5-diméthylpipérazine-1-carboxylate de 2-benzyl-2-azaspiro[3.3]heptan-6-yle ;
(2R,6R)-2,6-diméthyl-4-[6-(trifluorométhyl)-1,3-benzothiazol-2-yl]pipérazine-1-carboxylate de 2-benzyl-2-azaspiro[3.3]heptan-6-yle ;
(2R,5S)-2,5-diméthyl-4-[6-(trifluorométhyl)-1,3-benzothiazol-2-yl]pipérazine-1-carboxylate de 2-benzyl-2-azaspiro[3.3]heptan-6-yle ;
(2R,6S)-2,6-diméthyl-4-[5-(trifluorométhyl)pyrimidin-2-yl]pipérazine-1-carboxylate de 2-[(²H₅)benzyl]-2-azaspiro[3.3]heptan-6-yle ;
(2R,6S)-2,6-diméthyl-4-[5-(trifluorométhyl)pyrazin-2-yl]pipérazine-1-carboxylate de 2-[(²H₅)benzyl]-2-azaspiro[3.3]heptan-6-yle ;
(2R,6S)-2,6-diméthyl-4-[5-(trifluorométhyl)pyrimidin-2-yl]pipérazine-1-carboxylate de 2-[(²H₅)phényl(2H₁)méthyl]-2-azaspiro[3.3]heptan-6-yle ;
(2R,6S)-2,6-diméthyl-4-[5-(trifluorométhyl)pyrazin-2-yl]pipérazine-1-carboxylate de 2-[(²H₅)phényl(²H₁)méthyl]-2-azaspiro[3.3]heptan-6-yle ;
(2R,6S)-2,6-diméthyl-4-(quinazolin-2-yl)pipérazine-1-carboxylate de 2-benzyl-2-azaspiro[3.3]heptan-6-yle ;
(2R,6S)-2,6-diméthyl-4-[6-(trifluorométhyl)quinoxalin-2-yl]pipérazine-1-carboxylate de 2-benzyl-2-azaspiro[3.3]heptan-6-yle ;
(2R,6S)-4-(6-méthoxyquinoxalin-2-yl)-2,6-diméthylpipérazine-1-carboxylate de 2-benzyl-2-azaspiro[3.3]heptan-6-yle ;
(2R,5S)-2,5-diméthyl-4-[6-(trifluorométhyl)quinoxalin-2-yl]pipérazine-1-carboxylate de 2-benzyl-2-azaspiro[3.3]heptan-6-yle ;
(2R,6S)-4-(6-fluoro-1,3-benzothiazol-2-yl)-2,6-diméthylpipérazine-1-carboxylate de 2-[(4-fluorophényl)méthyl]-2-azaspiro[3.3]heptan-6-yle ;
(2R,6R)-4-(6-fluoro-1,3-benzothiazol-2-yl)-2,6-diméthylpipérazine-1-carboxylate de 2-[(4-fluorophényl)méthyl]-2-azaspiro[3.3]heptan-6-yle ;
(2R,5S)-4-(6-fluoro-1,3-benzothiazol-2-yl)-2,5-diméthylpipérazine-1-carboxylate de 2-[(4-fluorophényl)méthyl]-2-azaspiro[3.3]heptan-6-yle ;
(2R,6S)-2,6-diméthyl-4-[6-(trifluorométhyl)-1,3-benzothiazol-2-yl]pipérazine-1-carboxylate de 2-[(4-fluorophényl)méthyl]-2-azaspiro[3.3]heptan-6-yle ;
(2R,6R)-2,6-diméthyl-4-[6-(trifluorométhyl)-1,3-benzothiazol-2-yl]pipérazine-1-carboxylate de 2-[(4-fluorophényl)méthyl]-2-azaspiro[3.3]heptan-6-yle ;
(2R,5S)-2,5-diméthyl-4-[6-(trifluorométhyl)-1,3-benzothiazol-2-yl]pipérazine-1-carboxylate de 2-[(4-fluorophényl)méthyl]-2-azaspiro[3.3]heptan-6-yle ;
(2R,6S)-2,6-diméthyl-4-[5-(trifluorométhyl)pyrimidin-2-yl] pipérazine-1-carboxylate de 2-[(²H₅)phényl(²H₂)méthyl]-2-azaspiro[3.3]heptan-6-yle ;
(2R,6S)-2,6-diméthyl-4-[5-(triflluorométhyl)pyrazin-2-yl]pipérazine-1-carboxylate de 2-[(²H₅)phényl(²H₂)méthyl]-2-azaspiro[3.3]heptan-6-yle ;
(2R,6S)-2,6-diméthyl-4-(quinoxalin-2-yl)pipérazine-1-carboxylate de 2-[(4-fluorophényl)méthyl]-2-azaspiro[3.3]heptan-6-yle ;
(2R,6S)-2,6-diméthyl-4-(quinazolin-2-yl)pipérazine-1-carboxylate de 2-[(4-fluorophényl)méthyl]-2-azaspiro[3.3]heptan-6-yle ;
(2R,6S)-2,6-diméthyl-4-[6-(trifluorométhyl)quinoxalin-2-yl]pipérazine-1-carboxylate de 2-[(4-fluorophényl)méthyl]-2-azaspiro[3.3]heptan-6-yle ;
(2R,5S)-2,5-diméthyl-4-(quinoxalin-2-yl)pipérazine-1-carboxylate de 2-[(4-fluorophényl)méthyl]-2-azaspiro[3.3]heptan-6-yle ;
(2R,6S)-4-(6-méthoxyquinoxalin-2-yl)-2,6-diméthylpipérazine-1-carboxylate de 2-[(4-fluorophényl)méthyl]-2-azaspiro[3.3]heptan-6-yle ;
(2R,5S)-4-(6-méthoxyquinoxalin-2-yl)-2,5-diméthylpipérazine-1-carboxylate de 2-[(4-fluorophényl)méthyl]-2-azaspiro[3.3]heptan-6-yle ;
(2R,5S)-4-(6-fluoroquinoxalin-2-yl)-2,5-diméthylpipérazine-1-carboxylate de 2-[(4-fluorophényl)méthyl]-2-azaspiro[3.3]heptan-6-yle ;
(2R,6R)-2,6-diméthyl-4-[5-(trifluorométhyl)pyrazin-2-yl]pipérazine-1-carboxylate de 2-[(4-fluorophényl)méthyl]-2-azaspiro[3.3]heptan-6-yle ;
(2R,6S)-2,6-diméthyl-4-[5-(trifluorométhyl)pyrazin-2-yl]pipérazine-1-carboxylate de 2-[(4-fluorophényl)méthyl]-2-azaspiro[3.3]heptan-6-yle ;
(2R,5S)-2,5-diméthyl-4-[5-(trifluorométhyl)pyrazin-2-yl]pipérazine-1-carboxylate de 2-[(4-fluorophényl)méthyl]-2-azaspiro[3.3]heptan-6-yle ;
(2R,6S)-4-(6-fluoro-1,3-benzothiazol-2-yl)-2,6-diméthylpipérazine-1-carboxylate de 2-[(4-carbamoylphényl)méthyl]-2-azaspiro[3.3]heptan-6-yle ;
(2R,6R)-4-(6-fluoro-1,3-benzothiazol-2-yl)-2,6-diméthylpipérazine-1-carboxylate de 2-[(4-carbamoylphényl)méthyl]-2-azaspiro[3.3]heptan-6-yle ;
(2R,6S)-2,6-diméthyl-4-[6-(trifluorométhyl)quinoxalin-2-yl]pipérazine-1-carboxylate de 2-[(4-carbamoylphényl)méthyl]-2-azaspiro[3.3]heptan-6-yle ;
(2R,5S)-2,5-diméthyl-4-(quinoxalin-2-yl)pipérazine-1-carboxylate de 2-[(4-carbamoylphényl)méthyl]-2-azaspiro[3.3]heptan-6-yle ;
(2R,6S)-4-(6-méthoxyquinoxalin-2-yl)-2,6-diméthylpipérazine-1-carboxylate de 2-[(4-carbamoylphényl)méthyl]-2-azaspiro[3.3]heptan-6-yle ;
(2R,5S)-4-(6-méthoxyquinoxalin-2-yl)-2,5-diméthylpipérazine-1-carboxylate de 2-[(4-carbamoylphényl)méthyl]-2-azaspiro[3.3]heptan-6-yle ;
(2R,6R)-2,6-diméthyl-4-[5-(trifluorométhyl)pyrazin-2-yl]pipérazine-1-carboxylate de 2-[(4-carbamoylphényl)méthyl]-2-azaspiro[3.3]heptan-6-yle ;
(2R,6S)-2,6-diméthyl-4-[5-(trifluorométhyl)pyrazin-2-yl]pipérazine-1-carboxylate de 2-[(4-carbamoylphényl)méthyl]-2-azaspiro[3.3]heptan-6-yle ;
(2R,5S)-2,5-diméthyl-4-[5-(trifluorométhyl)pyrazin-2-yl]pipérazine-1-carboxylate de 2-[(4-carbamoylphényl)méthyl]-2-azaspiro[3.3]heptan-6-yle ;
(2R,6S)-4-(6-fluoro-1,3-benzothiazol-2-yl)-2,6-diméthylpipérazine-1-carboxylate de 2-(pyridin-4-ylméthyl)-2-azaspiro[3.3]heptan-6-yle ;
(2R,6R)-4-(6-fluoro-1,3-benzothiazol-2-yl)-2,6-diméthylpipérazine-1-carboxylate de 2-(pyridin-4-ylméthyl)-2-azaspiro[3.3]heptan-6-yle ;
(2R,6S)-2,6-diméthyl-4-[6-(trifluorométhyl)-1,3-benzothiazol-2-yl]pipérazine-1-carboxylate de 2-(pyridin-4-ylméthyl)-2-azaspiro[3.3]heptan-6-yle ;
(2R,6S)-2,6-diméthyl-4-(quinazolin-2-yl)pipérazine-1-carboxylate de 2-(pyridin-4-ylméthyl)-2-azaspiro[3.3]heptan-6-yle ;
(2R,6S)-4-(6-méthoxyquinoxalin-2-yl)-2,6-diméthylpipérazine-1-carboxylate de 2-(pyridin-4-ylméthyl)-2-azaspiro[3.3]heptan-6-yle ;
(2R,5S)-4-(6-méthoxyquinoxalin-2-yl)-2,5-diméthylpipérazine-1-carboxylate de 2-(pyridin-4-ylméthyl)-2-azaspiro[3.3]heptan-6-yle ;
(2R,5S)-4-(6-fluoroquinoxalin-2-yl)-2,5-diméthylpipérazine-1-carboxylate de 2-(pyridin-4-ylméthyl)-2-azaspiro[3.3]heptan-6-yle ;
(2R,6R)-2,6-diméthyl-4-[5-(trifluorométhyl)pyrazin-2-yl]pipérazine-1-carboxylate de 2-(pyridin-4-ylméthyl)-2-azaspiro[3.3]heptan-6-yle ;
(2R,6S)-2,6-diméthyl-4-[5-(trifluorométhyl)pyrazin-2-yl]pipérazine-1-carboxylate de 2-(pyridin-4-ylméthyl)-2-azaspiro[3.3]heptan-6-yle ;
(2R,5S)-2,5-diméthyl-4-[5-(trifluorométhyl)pyrazin-2-yl]pipérazine-1-carboxylate de 2-(pyridin-4-ylméthyl)-2-azaspiro[3.3]heptan-6-yle ;
(2R,6S)-2,6-diméthyl-4-[6-(trifluorométhyl)-1,3-benzoxazol-2-yl]pipérazine-1-carboxylate de 2-benzyl-2-azaspiro[3.3]heptan-6-yle ;
(2R,6R)-2,6-diméthyl-4-[6-(trifluorométhyl)-1,3-benzoxazol-2-yl]pipérazine-1-carboxylate de 2-benzyl-2-azaspiro[3.3]heptan-6-yle ;
(2R,5S)-2,5-diméthyl-4-[6-(trifluorométhyl)-1,3-benzoxazol-2-yl]pipérazine-1-carboxylate de 2-benzyl-2-azaspiro[3.3]heptan-6-yle ;
(2R,6S)-2,6-diméthyl-4-[6-(trifluorométhyl)-[1,3]thiazolo[4,5-b]pyridin-2-yl]pipérazine-1-carboxylate de 2-benzyl-2-azaspiro[3.3]heptan-6-yle ;
(2R,6R)-2,6-diméthyl-4-[6-(trifluorométhyl)-[1,3]thiazolo[4,5-b]pyridin-2-yl]pipérazine-1-carboxylate de 2-benzyl-2-azaspiro[3.3]heptan-6-yle ;
(2R,5S)-2,5-diméthyl-4-[6-(trifluorométhyl)-[1,3]thiazolo[4,5-b]pyridin-2-yl]pipérazine-1-carboxylate de 2-benzyl-2-azaspiro[3.3]heptan-6-yle ;
(2R,6S)-4-(6-cyano-1,3-benzothiazol-2-yl)-2,6-diméthylpipérazine-1-carboxylate de 2-benzyl-2-azaspiro[3.3]heptan-6-yle ;
(2R,5S)-4-(6-cyano-1,3-benzothiazol-2-yl)-2,5-diméthylpipérazine-1-carboxylate de 2-benzyl-2-azaspiro[3.3]heptan-6-yle ;
7-(6-fluoro-1,3-benzothiazol-2-yl)-3-oxa-7,9-diazabicyclo[3.3.1]nonane-9-carboxylate de 2-benzyl-2-azaspiro[3.3]heptan-6-yle ;
7-[6-(trifluorométhyl)-1,3-benzothiazol-2-yl]-3-oxa-7,9-diazabicyclo[3.3.1]nonane-9-carboxylate de 2-benzyl-2-azaspiro[3.3]heptan-6-yle ;
(2R,6S)-4-(5-fluoro-1,3-benzothiazol-2-yl)-2,6-diméthylpipérazine-1-carboxylate de 2-benzyl-2-azaspiro[3.3]heptan-6-yle ;
(2R,6R)-4-(5-fluoro-1,3-benzothiazol-2-yl)-2,6-diméthylpipérazine-1-carboxylate de 2-benzyl-2-azaspiro[3.3]heptan-6-yle ;
(2R,5S)-4-(5-fluoro-1,3-benzothiazol-2-yl)-2,5-diméthylpipérazine-1-carboxylate de 2-benzyl-2-azaspiro[3.3]heptan-6-yle ;
(2R,6S)-4-(5-fluoro-1,3-benzoxazol-2-yl)-2,6-diméthylpipérazine-1-carboxylate de 2-benzyl-2-azaspiro[3.3]heptan-6-yle ;
(2R,6R)-4-(5-fluoro-1,3-benzoxazol-2-yl)-2,6-diméthylpipérazine-1-carboxylate de 2-benzyl-2-azaspiro[3.3]heptan-6-yle ;
(2R,5S)-4-(5-fluoro-1,3-benzoxazol-2-yl)-2,5-diméthylpipérazine-1-carboxylate de 2-benzyl-2-azaspiro[3.3]heptan-6-yle ;
(2R,6S)-4-(6-fluoro-1,3-benzoxazol-2-yl)-2,6-diméthylpipérazine-1-carboxylate de 2-benzyl-2-azaspiro[3.3]heptan-6-yle ;
(2R,6R)-4-(6-fluoro-1,3-benzoxazol-2-yl)-2,6-diméthylpipérazine-1-carboxylate de 2-benzyl-2-azaspiro[3.3]heptan-6-yle ;
(2R,5S)-4-(6-fluoro-1,3-benzoxazol-2-yl)-2,5-diméthylpipérazine-1-carboxylate de 2-benzyl-2-azaspiro[3.3]heptan-6-yle ;
(2R,6S)-4-(5,6-difluoro-1,3-benzothiazol-2-yl)-2,6-diméthylpipérazine-1-carboxylate de 2-benzyl-2-azaspiro[3.3]heptan-6-yle ;
(2R,6R)-4-(5,6-difluoro-1,3-benzothiazol-2-yl)-2,6-diméthylpipérazine-1-carboxylate de 2-benzyl-2-azaspiro[3.3]heptan-6-yle ;
(2R,5S)-4-(5,6-difluoro-1,3-benzothiazol-2-yl)-2,5-diméthylpipérazine-1-carboxylate de 2-benzyl-2-azaspiro[3.3]heptan-6-yle ;
(2R,6S)-4-{6-fluoro-[1,3]thiazolo[5,4-b]pyridin-2-yl}-2,6-diméthylpipérazine-1-carboxylate de 2-benzyl-2-azaspiro[3.3]heptan-6-yle ;
(2R,6S)-4-{6-fluoro-[1,3]thiazolo[4,5-b]pyridin-2-yl}-2,6-diméthylpipérazine-1-carboxylate de 2-benzyl-2-azaspiro[3.3]heptan-6-yle ;
(2R,6S)-4-{5-fluoro-[1,3]thiazolo[5,4-b]pyridin-2-yl}-2,6-diméthylpipérazine-1-carboxylate de 2-benzyl-2-azaspiro[3.3]heptan-6-yle ;
(2R,6S)-4-(6-fluoroquinazolin-2-yl)-2,6-diméthylpipérazine-1-carboxylate de 2-benzyl-2-azaspiro[3.3]heptan-6-yle ;
(2R,6S)-2,6-diméthyl-4-{pyrido[2,3-b]pyrazin-3-yl}pipérazine-1-carboxylate de 2-benzyl-2-azaspiro[3.3]heptan-6-yle ;
(2R,6S)-2,6-diméthyl-4-[5-(trifluorométhyl)pyrimidin-2-yl]pipérazine-1-carboxylate de 2-[(4-fluorophényl)méthyl]-2-azaspiro[3.3]heptan-6-yle ;
(2R,5S)-2,5-diméthyl-4-[5-(trifluorométhyl)pyrimidin-2-yl]pipérazine-1-carboxylate de 2-[(4-fluorophényl)méthyl]-2-azaspiro[3.3]heptan-6-yle ;
(2R)-2-méthyl-4-[5-(trifluorométhyl)pyrimidin-2-yl]pipérazine-1-carboxylate de 2-[(4-fluorophényl)méthyl]-2-azaspiro[3.3]heptan-6-yle ;
(2R)-2-méthyl-4-[5-(trifluorométhyl)pyrazin-2-yl]pipérazine-1-carboxylate de 2-[(4-fluorophényl)méthyl]-2-azaspiro[3.3]heptan-6-yle ;
(2R,6S)-2,6-diméthyl-4-(1,6-naphthyridin-2-yl)pipérazine-1-carboxylate de 2-benzyl-2-azaspiro[3.3]heptan-6-yle ;
(2R,6S)-2,6-diméthyl-4-{pyrido[2,3-b]pyrazin-6-yl}pipérazine-1-carboxylate de 2-benzyl-2-azaspiro[3.3]heptan-6-yle ;
7-[5-(trifluorométhyl)pyrimidin-2-yl]-3-oxa-7,9-diazabicyclo[3.3.1]nonane-9-carboxylate de 2-[(4-fluorophényl)méthyl]-2-azaspiro[3.3]heptan-6-yle ;
(2R,6S)-4-[5-(difluorométhoxy)pyrimidin-2-yl]-2,6-diméthylpipérazine-1-carboxylate de 2-benzyl-2-azaspiro[3.3]heptan-6-yle ;
(2R,6S)-4-[4-amino-5-(trifluorométhyl)pyrimidin-2-yl]-2,6-diméthylpipérazine-1-carboxylate de 2-benzyl-2-azaspiro[3.3]heptan-6-yle ;
(2R,6S)-4-[5-(difluorométhyl)pyrimidin-2-yl]-2,6-diméthylpipérazine-1-carboxylate de 2-benzyl-2-azaspiro[3.3]heptan-6-yle ;
(2R,6S)-4-[5-(difluorométhoxy)pyrimidin-2-yl]-2,6-diméthylpipérazine-1-carboxylate de 2-[(4-fluorophényl)méthyl]-2-azaspiro[3.3]heptan-6-yle ;
(2R,6S)-4-[4-amino-5-(trifluorométhyl)pyrimidin-2-yl]-2,6-diméthylpipérazine-1-carboxylate de 2-[(4-fluorophényl)méthyl]-2-azaspiro[3.3]heptan-6-yle ;
(2R,6S)-4-[5-(difluorométhyl)pyrimidin-2-yl]-2,6-diméthylpipérazine-1-carboxylate de 2-[(4-fluorophényl)méthyl]-2-azaspiro[3.3]heptan-6-yle ;
(2R,6S)-4-(5-fluoropyrimidin-2-yl)-2,6-diméthylpipérazine-1-carboxylate de 2-[(4-fluorophényl)méthyl]-2-azaspiro[3.3]heptan-6-yle ;
(2R,6S)-2,6-diméthyl-4-[2-(trifluorométhyl)pyrimidin-5-yl]pipérazine-1-carboxylate de 2-benzyl-2-azaspiro[3.3]heptan-6-yle ;
(2R,6S)-2,6-diméthyl-4-[5-(méthylcarbamoyl)pyrimidin-2-yl]pipérazine-1-carboxylate de 2-benzyl-2-azaspiro[3.3]heptan-6-yle ;
(2R,6S)-4-(5-cyanopyrimidin-2-yl)-2,6-diméthylpipérazine-1-carboxylate de 2-[(4-fluorophényl)méthyl]-2-azaspiro[3.3]heptan-6-yle ;
(2R,5S)-4-(5-cyanopyrimidin-2-yl)-2, 5-diméthylpipérazine-1-carboxylate de 2-[(4-fluorophényl)méthyl]-2-azaspiro[3.3]heptan-6-yle ;
(2R)-4-(5-cyanopyrimidin-2-yl)-2-méthylpipérazine-1-carboxylate de 2-[(4-fluorophényl)méthyl]-2-azaspiro[3.3]heptan-6-yle ;
(2R,6S)-4-(5-cyanopyrimidin-2-yl)-2,6-diméthylpipérazine-1-carboxylate de 2-[(4-chlorophényl)méthyl]-2-azaspiro[3.3]heptan-6-yle ;
(2R,5S)-4-(5-cyanopyrimidin-2-yl)-2, 5-diméthylpipérazine-1-carboxylate de 2-[(4-chlorophényl)méthyl]-2-azaspiro[3.3]heptan-6-yle ;
(2R,6S)-2,6-diméthyl-4-[5-(trifluorométhyl)pyrimidin-2-yl]pipérazine-1-carboxylate de 2-[(4-chlorophényl)méthyl]-2-azaspiro[3.3]heptan-6-yle ;
(2R)-2-méthyl-4-[5-(trifluorométhyl)pyrimidin-2-yl]pipérazine-1-carboxylate de 2-[(4-chlorophényl)méthyl]-2-azaspiro[3.3]heptan-6-yle ;
(2R,6S)-2,6-diméthyl-4-[5-(trifluorométhyl)pyrazin-2-yl]pipérazine-1-carboxylate de 2-[(4-chlorophényl)méthyl]-2-azaspiro[3.3]heptan-6-yle ;
(2R,5S)-2,5-diméthyl-4-[5-(trifluorométhyl)pyrazin-2-yl]pipérazine-1-carboxylate de 2-[(4-chlorophényl)méthyl]-2-azaspiro[3.3]heptan-6-yle ;
(2R)-2-méthyl-4-[5-(trifluorométhyl)pyrazin-2-yl]pipérazine-1-carboxylate de 2-[(4-chlorophényl)méthyl]-2-azaspiro[3.3]heptan-6-yle ;
(2R,5S)-2,5-diméthyl-4-[5-(trifluorométhyl)pyrimidin-2-yl]pipérazine-1-carboxylate de 2-[(4-chlorophényl)méthyl]-2-azaspiro[3.3]heptan-6-yle ;
(2R)-4-(5-cyanopyrimidin-2-yl)-2-méthylpipérazine-1-carboxylate de 2-[(4-chlorophényl)méthyl]-2-azaspiro[3.3]heptan-6-yle ;
(2R,5S)-4-(6-fluoroquinazolin-2-yl)-2,5-diméthylpipérazine-1-carboxylate de 2-benzyl-2-azaspiro[3.3]heptan-6-yle ;
(1R,4R)-5-[5-(trifluorométhyl)pyrimidin-2-yl]-2,5-diazabicyclo[2.2.1]heptane-2-carboxylate de 2-benzyl-2-azaspiro[3.3]heptan-6-yle ;
(2R,6S)-4-[5-(diméthylcarbamoyl)pyrimidin-2-yl]-2,6-diméthylpipérazine-1-carboxylate de 2-benzyl-2-azaspiro[3.3]heptan-6-yle ;
9-[5-(trifluorométhyl)pyrimidin-2-yl]-3-oxa-7,9-diazabicyclo[3.3.1]nonane-7-carboxylate de 2-benzyl-2-azaspiro[3.3]heptan-6-yle ;
9-[5-(trifluorométhyl)pyrazin-2-yl]-3-oxa-7,9-diazabicyclo[3.3.1]nonane-7-carboxylate de 2-benzyl-2-azaspiro[3.3]heptan-6-yle ;
(2R,6S)-4-(5-éthylpyrimidin-2-yl)-2,6-diméthylpipérazine-1-carboxylate de 2-benzyl-2-azaspiro[3.3]heptan-6-yle ;
(2R,6S)-2,6-diméthyl-4-[5-(trifluorométhoxy)pyrimidin-2-yl]pipérazine-1-carboxylate de 2-benzyl-2-azaspiro[3.3]heptan-6-yle ;
(2R,6S)-4-[5-(cyclopropylméthoxy)pyrimidin-2-yl]-2,6-diméthylpipérazine-1-carboxylate de 2-benzyl-2-azaspiro[3.3]heptan-6-yle ;
(2R,6S)-2,6-diméthyl-4-[5-(trifluorométhyl)pyrimidin-2-yl]pipérazine-1-carboxylate de 2-[(4-méthanesulfonylphényl)méthyl]-2-azaspiro[3.3]heptan-6-yle ;
(2R,5S)-2,5-diméthyl-4-[5-(trifluorométhyl)pyrimidin-2-yl]pipérazine-1-carboxylate de 2-[(4-méthanesulfonylphényl)méthyl]-2-azaspiro[3.3]heptan-6-yle ;
(2R,6S)-2,6-diméthyl-4-[5-(trifluorométhyl)pyrazin-2-yl]pipérazine-1-carboxylate de 2-[(4-méthanesulfonylphényl)méthyl]-2-azaspiro[3.3]heptan-6-yle ;
(2R,5S)-2,5-diméthyl-4-[5-(trifluorométhyl)pyrazin-2-yl]pipérazine-1-carboxylate de 2-[(4-méthanesulfonylphényl)méthyl]-2-azaspiro[3.3]heptan-6-yle ;
(2R,6S)-2,6-diméthyl-4-(5-propylpyrimidin-2-yl)pipérazine-1-carboxylate de 2-benzyl-2-azaspiro[3.3]heptan-6-yle ;
(2R,6S)-2,6-diméthyl-4-[5-(propan-2-yl)pyrimidin-2-yl]pipérazine-1-carboxylate de 2-benzyl-2-azaspiro[3.3]heptan-6-yle ;
(2R,6S)-4-(5-cyclopropylpyrimidin-2-yl)-2,6-diméthylpipérazine-1-carboxylate de 2-benzyl-2-azaspiro[3.3]heptan-6-yle ;
(2R,6S)-4-(5-éthynylpyrimidin-2-yl)-2,6-diméthylpipérazine-1-carboxylate de 2-benzyl-2-azaspiro[3.3]heptan-6-yle ;
(2R,6S)-4-[5-(diméthylphosphoryl)pyridin-2-yl]-2,6-diméthylpipérazine-1-carboxylate de 2-benzyl-2-azaspiro[3.3]heptan-6-yle ;
(2R,6S)-2,6-diméthyl-4-[4-(trifluorométhyl)phényl]pipérazine-1-carboxylate de 2-benzyl-2-azaspiro[3.3]heptan-6-yle ;
(2R,6S)-4-(4-éthoxyphényl)-2,6-diméthylpipérazine-1-carboxylate de 2-benzyl-2-azaspiro[3.3]heptan-6-yle ;
(2R,6S)-4-(4-méthoxyphényl)-2,6-diméthylpipérazine-1-carboxylate de 2-benzyl-2-azaspiro[3.3]heptan-6-yle ;
(2R,6S)-4-(4-fluoro-3-méthylphényl)-2,6-diméthylpipérazine-1-carboxylate de 2-benzyl-2-azaspiro[3.3]heptan-6-yle ;
(2R,6S)-4-(4-cyano-3-méthoxyphényl)-2,6-diméthylpipérazine-1-carboxylate de 2-benzyl-2-azaspiro[3.3]heptan-6-yle ;
(2R,5S)-2,5-diméthyl-4-[4-(trifluorométhyl)phényl]pipérazine-1-carboxylate de 2-benzyl-2-azaspiro[3.3]heptan-6-yle ;
(2R)-2-méthyl-4-[4-(trifluorométhyl)phényl]pipérazine-1-carboxylate de 2-benzyl-2-azaspiro[3.3]heptan-6-yle ;
(2R,6S)-4-[5-(diméthylphosphoryl)pyrimidin-2-yl]-2,6-diméthylpipérazine-1-carboxylate de 2-benzyl-2-azaspiro[3.3]heptan-6-yle ; et
(2R,6S)-4-[5-(cyclobutylméthoxy)pyrimidin-2-yl]-2,6-diméthylpipérazine-1-carboxylate de 2-benzyl-2-azaspiro[3.3]heptan-6-yle.

12. Composition pharmaceutique comprenant un composé selon l'une quelconque des revendications 1 à 11, ou un sel pharmaceutiquement acceptable de celui-ci, et un véhicule pharmaceutiquement acceptable.

13. Composé ou sel selon l'une quelconque des revendications 1 à 11, pour utilisation dans une méthode de traitement ou de prévention d'une maladie, d'un trouble ou d'un symptôme médié par le récepteur muscarinique 4 (M₄) chez un individu.

14. Composé ou sel selon l'une quelconque des revendications 1 à 11, pour utilisation dans une méthode de traitement ou de prévention d'une maladie, d'un trouble ou d'un symptôme neurologique chez un individu ;
dans lequel la maladie, le trouble ou le symptôme est choisi parmi : syndrome de Tourette (TS), maladie d'Alzheimer (MA), schizophrénie, démence à corps de Lewy (DCL), déficits cognitifs associés à la schizophrénie, maladie de Parkinson, parkinsonisme, tremblement, dyskinésies, somnolence diurne excessive, dystonie, chorée, dyskinésie induite par la lévodopa, trouble du déficit de l'attention avec hyperactivité (TDAH), paralysie cérébrale, paralysie supranucléaire progressive (PSP), atrophie multisystématisée (AMS), maladie de Huntington (MH) et chorée associée à la maladie de Huntington.

15. Composé ou sel pour utilisation selon la revendication 14, dans lequel :
la maladie, le trouble ou le symptôme est le parkinsonisme, le tremblement ou la dystonie.
